# EUROPEAN PATENT APPLICATION

(11) **EP 4 129 996 A1**
(43) Date of publication of application: **08.02.2023**
(21) Application number: 21776985.0
(22) Date of filing: 19.03.2021
(51) Int. Cl.: C07D 403/10, C07D 403/14, C07D 471/10, C07D 498/10, C07F 9/6558, A61K 31/5386, A61K 31/675, A61P 35/00

(54) **NOVEL AMINOPYRIMIDINE EGFR INHIBITOR**

(30) Priority: 23.03.2020 CN 202010208625; 18.09.2020 CN 202010984379
(71) Applicant: QILU PHARMACEUTICAL CO., LTD., High Technical Zone Jinan Shandong 250100 (CN)
(72) Inventor: DENG, Wei, Jinan, Shandong 250100 (CN); ZHENG, Shansong, Jinan, Shandong 250100 (CN); CAMPOS, Sebastien Andre, Jinan, Shandong 250100 (CN); YANG, Yingying, Jinan, Shandong 250100 (CN); TIAN, Zhenhua, Jinan, Shandong 250100 (CN); ZHENG, Qingmei, Jinan, Shandong 250100 (CN); WU, Guosheng, Jinan, Shandong 250100 (CN); ZHAO, Zhiwei, Jinan, Shandong 250100 (CN); LI, Leilei, Jinan, Shandong 250100 (CN); FU, Jianmin, Jinan, Shandong 250100 (CN); ZHAO, Shuyong, Jinan, Shandong 250100 (CN)
(74) Representative: Calysta NV
(86) International application number: PCT/CN2021/081868
(87) International publication number: WO 2021/190417

(57) **Abstract**

The present invention provides a novel aminopyrimidine compound as a fourth-generation EGFR (T790M/C797S mutation) selective inhibitor, a pharmaceutical composition comprising the compound, an intermediate useful for preparing the compound, and a method for treating a cell proliferative disease, such as a cancer, by using the compound of the present invention.

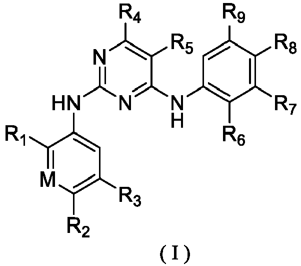

## Description

The present application claims the priorities of Chinese patent application CN202010208625.2 filed on March 23, 2020 and Chinese patent application CN202010984379.X filed on September 18, 2020. The contents of the above Chinese patent applications are incorporated herein by reference in its entireties.

### TECHNICAL FIELD

The present disclosure belongs to the field of pharmaceutical chemistry, and particularly relates to a novel aminopyrimidine compound as a selective EGFR inhibitor, a pharmaceutical composition comprising the compound, an intermediate useful for preparing the compound, and a method for treating a cell proliferative disease, such as a cancer, by using the compound of the present disclosure.

### BACKGROUND

Lung Cancer is the cancer with the highest incidence and mortality, which seriously threatens human health and life. Lung cancer is mainly divided into small cell lung cancer (SCLC) and non-small cell lung cancer (NSCLC), of which about 80 % are NSCLC.

Epidermal growth factor receptor (EGFR) is widely distributed on the surface of mammalian epithelial cells, fibroblasts, glial cells and other cells. The EGFR signaling pathway plays an important role in physiological processes such as cell growth, proliferation and differentiation. EGFR mutation is also the most common type of mutation in NSCLC patients, especially in Asian populations, which can account for 40 % to 50 %. Therefore, EGFR has always been one of the most popular targets in pharmaceutical industry research.

At present, there are first-, second- and third-generation EGFR inhibitors on the market. The first generation is reversible targeted drugs, such as gefitinib, erlotinib, and icotinib. The second-generation is irreversible targeted drugs, such as afatinib and dacomitinib. Although the first- and second-generation targeted drugs are effective, most patients will develop drug resistance after using drugs for 1-2 years. Among patients with EGFR inhibitor resistance, 50 % of the drug resistance is related to the T790M mutation. The third-generation EGFR-targeted drug osimertinib can bind to the T790M mutation site of EGFR-sensitive mutation and inhibit the drug resistance of tumor caused by T790M mutation. Its advent has brought good survival benefits to more lung cancer patients. However, the third-generation targeted drugs also inevitably develop drug resistance, and the main reason for the drug resistance is C797S mutation. The C797S mutation is reflected in the mutation of the cysteine residue into serine. This mutation destroys the binding of the EGFR protein to the third-generation targeted drugs, thereby failing to prevent the unilateral phosphorylation of EGFR and the activation of downstream signaling pathways. At present, there is no mature treatment method for the two main cis-triple mutations after osimertinib drug resistance: del19/T790M/C797S and L858R/T790M/C797S, and the clinical demands are imminent. The present disclosure is based on solving this problem.

### CONTENT OF THE PRESENT INVENTION

The present disclosure provides a compound represented by formula (I'), a stereoisomer thereof, a tautomer thereof, a pharmaceutically acceptable salt thereof, a prodrug thereof, a hydrate thereof, a solvate thereof or an isotope-labeled derivative thereof, 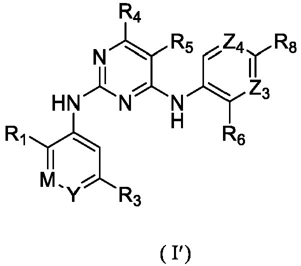 wherein,
R₁ is selected from H, halogen, -CN, C₁₋₆ alkyl, C₁₋₆ heteroalkyl, C₁₋₆ haloalkyl, C₁₋₆ haloalkoxy, C₃₋₆ cycloalkyl, 3-6-membered heterocycloalkyl, C₃₋₆ cycloalkyloxy, 3-6-membered heterocycloalkyloxy, and C₂₋₆ alkenyloxy;
M is selected from N or CR₁₀;
R₁₀ and R₁ may form a 5-8-membered heterocycloalkyl or a 5-7-membered heteroaryl; the 5-8-membered heterocycloalkyl and the 5-7-membered heteroaryl are optionally substituted by one or more R₁₁ groups;
Y is selected from N and CR₂;
R₂ is selected from H, halogen, -CN, -OH, -NH₂, phosphonyl, sulfonyl, aminosulfonyl, aminocarbonyl, carbonylamino, C₁₋₆ alkyl, C₁₋₆ alkoxy, C₁₋₆ haloalkyl , C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, C₃₋₆ heterocycloalkenyl, phenyl and 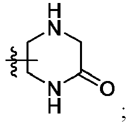 wherein, the -OH, -NH₂, phosphonyl, sulfonyl, aminosulfonyl, aminocarbonyl, carbonylamino, C₁₋₆ alkyl, C₁₋₆ alkoxy, C₁₋₆ haloalkyl , C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, C₃₋₆ heterocycloalkenyl, phenyl and 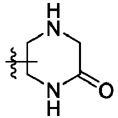 are optionally substituted by one or more R₁₂ groups;
R₃ is selected from -CN, sulfoximino, -L₁-C₆₋₁₀ aryl, -L₁-5-12-membered heteroaryl, -L₁-C₃₋₆ cycloalkenyl and 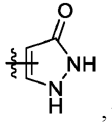 wherein, the -CN, sulfoximino, -L₁-C₆₋₁₀ aryl, -L₁-5-12-membered heteroaryl, -L₁-C₃₋₆ cycloalkenyl and 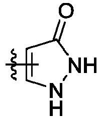 are optionally substituted by one or more R₁₃ groups;
L₁ is independently selected from a linking bond, C₁₋₄ alkyl and C₁₋₄ heteroalkyl, wherein the C₁₋₄ alkyl and C₁₋₄ heteroalkyl are optionally substituted by one or more groups selected from -OH, -NH₂ and halogen;
R₄ and R₅ are each independently selected from H, halogen, -CN, C₁₋₆ alkyl, C₁₋₆ alkoxy, C₁₋₆ haloalkyl, C₁₋₆ haloalkoxy, C₃₋₈ cycloalkyl and 3-8-membered heterocycloalkyl;
or, R₄ and R₅ are cyclized to 4-6-membered cycloalkyl, 4-6-membered heterocycloalkyl, 4-6-membered aryl or 4-6-membered heteroaryl;
R₆ is selected from amino, amido, aminocarbonyl, sulfonyl, thiophosphonyl, phosphonyl, phosphonoamino, sulfonylamino, aminosulfonyl and sulfoximino, wherein the amino, amido, aminocarbonyl, sulfonyl, thiophosphonyl, phosphonyl, phosphonoamino, sulfonylamino, aminosulfonyl and sulfoximino are optionally substituted by one or more R₁₄ groups;
Z₃ is selected from N or CR₇;
Z₄ is selected from N or CR₉;
R₇, R₈ and R₉ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6-membered heterocycloalkyl and 5-7-membered heteroaryl;
or, R₇ and R₈ are cyclized to C₄₋₆ cycloalkyl, 4-6-membered heterocycloalkyl, C₅₋₆ aryl or 5-7-membered heteroaryl;
or, R₈ and R₉ are cyclized to C₄₋₆ cycloalkyl, 4-6-membered heterocycloalkyl, C₅₋₆ aryl or 5-7-membered heteroaryl;
R₁₀ is selected from H, halogen, C₁₋₆ alkyl, C₁₋₆ alkoxy, C₁₋₆ haloalkyl and C₁₋₆ haloalkoxy;
R₁₁ is selected from H, halogen, C₁₋₆ alkyl, C₁₋₆ alkoxy, C₁₋₆ haloalkyl, C₁₋₆ haloalkoxy, -C₀₋₄ alkyl-NRₐR_{b}, -C₁₋₄ alkyl-O-C₁₋₄ alkyl, -C₁₋₄ alkyl-OH, -O-C₁₋₄ alkyl, -C₀₋₄ alkyl-C₃₋₆ cycloalkyl and -C₀₋₄ alkyl-3-6-member heterocycloalkyl;
R₁₂ is selected from H, halogen, -CN, -OH, -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₈ cycloalkyl, 3-14-membered heterocycloalkyl, hydroxy-C₁₋₆ alkyl-, C₃₋₈ cycloalkylalkyl-, C₃₋₈ cycloalkyloxy-, C₃₋₈ heterocycloalkylalkyl-, C₃₋₈ heterocycloalkyloxy-, C₃₋₈ cycloalkyl-C₁₋₆ alkoxy-, C₃₋₈ heterocycloalkyl-C₁₋₆ alkoxy-, C₁₋₆ alkylsulfonyl, C₃₋₆ cycloalkylsulfonyl, NRₐR_{b}CO-, C₁₋₆ alkylcarbonyl, C₃₋₈ cycloalkylcarbonyl, C₁₋₆ alkoxy-C₁₋₆ alkyl- , C₃₋₆ cycloalkenyl, 5-12-membered heteroaryl, C₆₋₁₀ aryl, NRₐR_{b}S(O)₂-, -(CH₂)ₘNRₐR_{b}, -(CH₂)ₘO(CH₂)ₙCH₃ and -O(CH₂)ₘNRₐR_{b}; wherein the Rₐ and R_{b} are independently H, C₁₋₆ alkyl or C₁₋₆ alkoxy, or Rₐ and R_{b} together form a C₃₋₈ cycloalkyl or 3-8-membered heterocycloalkyl; wherein the m and n are independently optionally 0, 1, 2 or 3, the C₃₋₈ cycloalkyl and 3-8-membered heterocycloalkyl are optionally substituted by a group selected from halogen, -OH, -NH₂, -CN, C₁₋₆ alkyl, C₁₋₆ alkoxy, C₁₋₆ haloalkyl and -C₀₋₄ alkyl-O-C₁₋₄ alkyl;
R₁₃ is selected from H, halogen, -CN, C₁₋₆ alkyl, C₁₋₆ heteroalkyl, C₁₋₆ haloalkyl, C₁₋₆ haloalkoxy, C₃₋₈ cycloalkyl, 3-8-membered heterocycloalkyl, C₃₋₆ cycloalkylsulfonyl, 5-6-membered heteroaryl and phenyl;
R₁₄ is selected from H, C₁₋₆ alkyl, C₁₋₆ alkoxy, C₁₋₆ haloalkyl, C₁₋₆ haloalkoxy, C₃₋₈ cycloalkyl and 3-8-membered heterocycloalkyl.

In some embodiments of the present disclosure, in the compound represented by formula (I'), the stereoisomer thereof, the tautomer thereof, the pharmaceutically acceptable salt thereof, the prodrug thereof, the hydrate thereof, the solvate thereof or the isotope-labeled derivative thereof,
R₁ is selected from H, C₁₋₄ alkyl, C₁₋₄ alkoxy, C₁₋₄ haloalkyl and C₁₋₄ haloalkoxy;
M is selected from N or CR₁₀;
R₁₀ and R₁ may form a 5-8-membered heterocycloalkyl and a 5-7-membered heteroaryl; the 5-8-membered heterocycloalkyl and the 5-7-membered heteroaryl are optionally substituted by one or more R₁₁ groups;
Y is selected from N and CR₂;
R₂ is selected from H, halogen, -NH₂, phosphonyl, sulfonyl, aminosulfonyl, aminocarbonyl, C₃₋₁₄ cycloalkyl, 3-14-membered heterocycloalkyl, C₃₋₆ cycloalkenyl, C₃₋₆ heterocycloalkenyl and 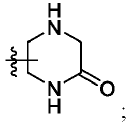 wherein, the -NH₂, phosphonyl, sulfonyl, aminosulfonyl, aminocarbonyl, C₃₋₁₄ cycloalkyl, 3-14-membered heterocycloalkyl, C₃₋₆ cycloalkenyl, C₃₋₆ heterocycloalkenyl and 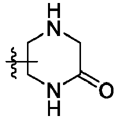 are optionally substituted by one or more R₁₂ groups;
R₃ is selected from -CN, 5-12-membered heteroaryl and 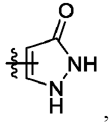 wherein, the -CN, 5-12-membered heteroaryl and 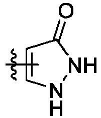 are optionally substituted by one or more R₁₃ groups;
R₄ and R₅ are each independently selected from H, halogen, -CN, C₁₋₄ alkyl, C₁₋₄ haloalkyl, and C₃₋₆ cycloalkyl;
or, R₄ and R₅ are cyclized to 4-6-membered aryl or 4-6-membered heteroaryl;
R₆ is selected from amido, sulfonyl, thiophosphonyl, phosphonyl, sulfonylamino and aminosulfonyl, wherein the amido, sulfonyl, thiophosphonyl, phosphonyl, sulfonylamino and aminosulfonyl are optionally substituted by one or more R₁₄ groups;
Z₃ is selected from CR₇;
Z₄ is selected from CR₉;
R₇, R₈ and R₉ are each independently selected from H and C₁₋₄ alkyl;
or, R₇ and R₈ are cyclized to C₄₋₆ cycloalkyl or 5-7-membered heteroaryl;
or, R₈ and R₉ are cyclized to C₄₋₆ cycloalkyl or 5-7-membered heteroaryl;
R₁₀ is selected from H, halogen and C₁₋₄ alkyl;
R₁₁ is selected from H, C₁₋₄ alkyl, -C₁₋₄ alkyl-NRₐR_{b} and 3-6-membered heterocycloalkyl;
R₁₂ is selected from H, halogen, -OH, -NH₂, C₁₋₆ alkyl, C₁₋₆ haloalkyl, C₃₋₈ cycloalkyl, 3-8-membered heterocycloalkyl, hydroxy-C₁₋₆ alkyl, 6-14-membered spiro heterocyclyl, -C₁₋₄ alkyl-O-C₁₋₄ alkyl, -O-C₁₋₄ alkyl, -C₁₋₄ alkyl-C₃₋₆ cycloalkyl, -O-C₀₋₄ alkyl-C₃₋₆ cycloalkyl, -C₁₋₄ alkyl-3-6-membered heterocycloalkyl, -O-C₀₋₄ alkyl-3-6-membered heterocycloalkyl, -(CH₂)ₘNRₐR_{b}, -O(CH₂)ₘNRₐR_{b}, C₁₋₆ alkylsulfonyl, C₃₋₆ cycloalkylsulfonyl, NRₐR_{b}CO-, C₁₋₆ alkylcarbonyl and C₃₋₆ cycloalkylcarbonyl, wherein the cycloalkyl and heterocycloalkyl are optionally substituted by R_{ab}, and the R_{ab} is selected from H, halogen, -OH, -NH₂, -CN, C₁₋₄ alkyl, C₁₋₄ alkoxy, C₁₋₄ haloalkyl and -C₀₋₄ alkyl-O-C₁₋₄ alkyl;
Rₐ and R_{b} are independently H or C₁₋₄ alkyl;
m is optionally 0, 1, 2 or 3;
R₁₃ is selected from H, halogen, C₁₋₄ alkyl, C₁₋₄ haloalkyl, hydroxy-C₁₋₆ alkyl and C₃₋₆ cycloalkylsulfonyl;
R₁₄ is selected from H, C₁₋₄ alkyl, C₁₋₄ alkoxy and C₃₋₈ cycloalkyl.

In some embodiments of the present disclosure, in the compound represented by formula (I'), the stereoisomer thereof, the tautomer thereof, the pharmaceutically acceptable salt thereof, the prodrug thereof, the hydrate thereof, the solvate thereof or the isotope-labeled derivative thereof, the compound represented by formula (I'), the stereoisomer thereof, the tautomer thereof, the pharmaceutically acceptable salt thereof, the prodrug thereof, the hydrate thereof, the solvate thereof or the isotope-labeled derivative thereof may be a compound represented by formula (I), a stereoisomer thereof, a tautomer thereof, a pharmaceutically acceptable salt thereof, a prodrug thereof, a hydrate thereof, a solvate thereof or an isotope-labeled derivative thereof, 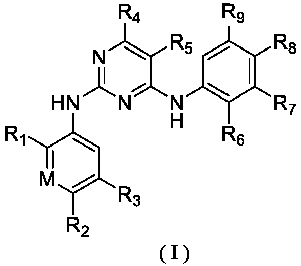 wherein, R₁, R₂, R₃, R₄, R₅, R₆, R₇, R₈, R₉ and M are defined as above.

In some embodiments of the present disclosure, in the compound represented by formula (I), the stereoisomer thereof, the tautomer thereof, the pharmaceutically acceptable salt thereof, the prodrug thereof, the hydrate thereof, the solvate thereof or the isotope-labeled derivative thereof,
R₁ is selected from H, C₁₋₄ alkyl, C₁₋₄ alkoxy, C₁₋₄ haloalkyl and C₁₋₄ haloalkoxy;
M is selected from N or CR₁₀;
R₁₀ and R₁ may form a 5-8-membered heterocycloalkyl, and the 5-8-membered heterocycloalkyl is optionally substituted by one or more R₁₁ groups;
R₂ is selected from H, halogen, -NH₂, phosphonyl, sulfonyl, C₃₋₁₄ cycloalkyl, 3-14-membered heterocycloalkyl, C₃₋₆ cycloalkenyl and C₃₋₆ heterocycloalkenyl; wherein, the -NH₂, phosphonyl, sulfonyl, C₃₋₁₄ cycloalkyl, 3-14-membered heterocycloalkyl, C₃₋₆ cycloalkenyl and C₃₋₆ heterocycloalkenyl are optionally substituted by one or more R₁₂ groups;
R₃ is selected from 5-12-membered heteroaryl optionally substituted by one or more R₁₃ groups;
R₄ and R₅ are each independently selected from H, halogen, -CN, C₁₋₄ alkyl, C₁₋₄ haloalkyl, and C₃₋₆ cycloalkyl;
or, R₄ and R₅ are cyclized to aryl or 4-6-membered heteroaryl;
R₆ is selected from amido, sulfonyl, thiophosphonyl, phosphonyl, sulfonylamino and aminosulfonyl, wherein the amino, amido, sulfonyl, thiophosphonyl, phosphonyl, sulfonylamino and aminosulfonyl are optionally substituted by one or more R₁₄ groups;
R₇, R₈ and R₉ are each independently selected from H and C₁₋₄ alkyl;
or, R₇ and R₈ are cyclized to C₄₋₆ cycloalkyl or 5-7-membered heteroaryl;
or, R₈ and R₉ are cyclized to C₄₋₆ cycloalkyl or 5-7-membered heteroaryl;
R₁₀ is optionally selected from H, halogen and C₁₋₄ alkyl;
R₁₁ is optionally selected from H, C₁₋₄ alkyl, -C₁₋₄ alkyl-NRₐR_{b} and 3-6-membered heterocycloalkyl;
R₁₂ is selected from H, halogen, -OH, -NH₂, C₁₋₆ alkyl, C₁₋₆ haloalkyl, C₃₋₈ cycloalkyl, 3-8-membered heterocycloalkyl, hydroxy-C₁₋₆ alkyl, 6-14-membered spiro heterocyclyl, -C₁₋₄ alkyl-O-C₁₋₄ alkyl, -O-C₁₋₄ alkyl, -C₁₋₄ alkyl-C₃₋₆ cycloalkyl, -O-C₀₋₄ alkyl-C₃₋₆ cycloalkyl, -C₁₋₄ alkyl-3-6-membered heterocycloalkyl, -O-C₀₋₄ alkyl-3-6-membered heterocycloalkyl, -(CH₂)ₘNRₐR_{b}, -O(CH₂)ₘNRₐR_{b}, C₁₋₆ alkylsulfonyl, C₃₋₆ cycloalkylsulfonyl, NRₐR_{b}CO-, C₁₋₆ alkylcarbonyl and C₃₋₆ cycloalkylcarbonyl, wherein the cycloalkyl and heterocycloalkyl are optionally substituted by R_{ab}, and the R_{ab} is selected from H, halogen, -OH, -NH₂, -CN, C₁₋₄ alkyl, C₁₋₄ alkoxy, C₁₋₄ haloalkyl and -C₀₋₄ alkyl-O-C₁₋₄ alkyl; Rₐ and R_{b} are H or C₁₋₄ alkyl; m is optionally 0, 1, 2 or 3;
R₁₃ is selected from H, halogen, C₁₋₄ alkyl, C₁₋₄ haloalkyl and C₃₋₆ cycloalkylsulfonyl;
R₁₄ is selected from H, C₁₋₄ alkyl, C₁₋₄ alkoxy and C₃₋₈ cycloalkyl.

In some embodiments of the present disclosure, the Z₃ is selected from CR₇, and R₇ is defined as above.

In some embodiments of the present disclosure, the Z₄ is selected from CR₉, and R₉ is defined as above.

In some embodiments of the present disclosure, the M is selected from CR₁₀, and R₁₀ is defined as above.

In some embodiments of the present disclosure, the R₁₀ is selected from H, fluorine and methyl.

In some embodiments of the present disclosure, the R₁₀ is selected from H.

In some embodiments of the present disclosure, the R₁₁ is independently selected from H, methyl, -CH₂CH₂N(CH₃)₂ and

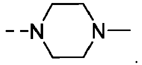

In some embodiments of the present disclosure, the R₁₂ is selected from H, F, -OH, -NH₂, C₁₋₄ alkyl, C₁₋₄ fluoroalkyl, C₁₋₄ alkoxy, -O-C₁₋₄ alkyl-C₃₋₆ cycloalkyl, -O-C₁₋₄ alkyl-NRₐR_{b}, -C₁₋₄ alkyl-O-C₀₋₄ alkyl, 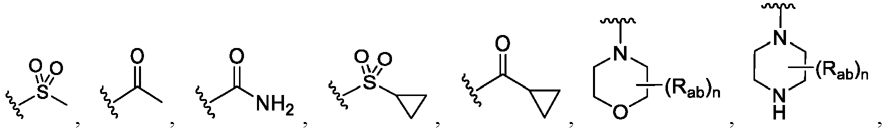 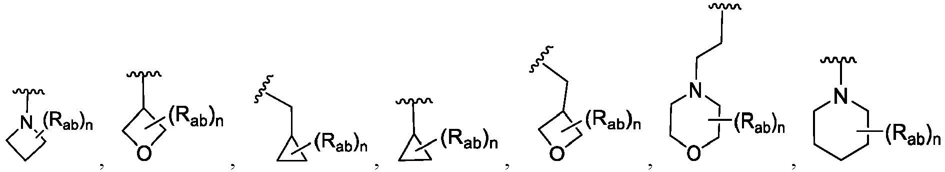 and 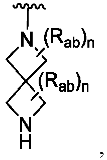 and Rₐ, R_{b}, R_{ab} and n are defined as above.

In some embodiments of the present disclosure, the R₁₂ is selected from H, F, -OH, -NH₂, methyl, ethyl, isopropyl, -CH₂F, -CH₂CH₂F, -CH₂CHF₂, -CH₂CF₃, -CH₂CH₂CHF₂, -CH₂CH₂CF₃, -CH(CH₃)CF₃, - CH(CH₃)CH₂F, -C(CH₃)₂CH₂F, 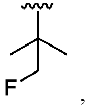 -OCH₃, -OCH(CH₃)CH₃,

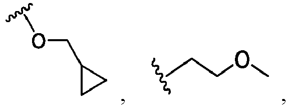

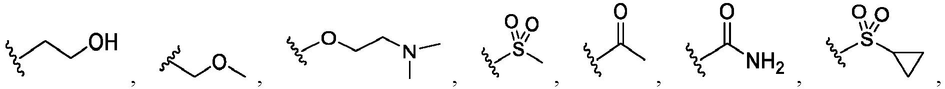

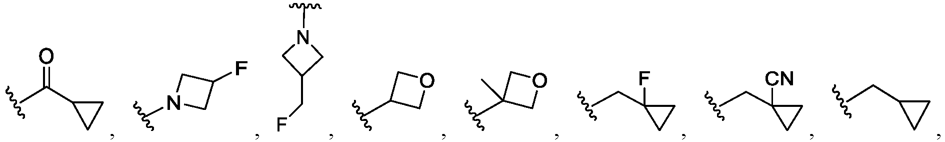

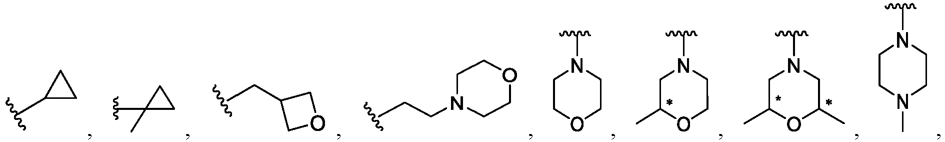

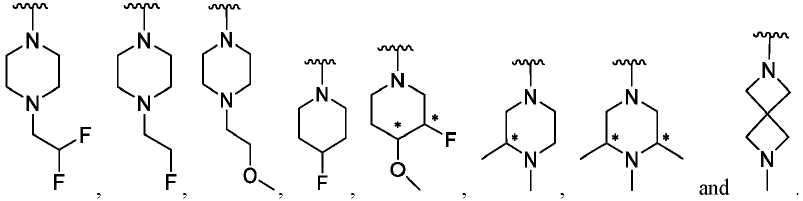

In some embodiments of the present disclosure, the R₁₂ is selected from H, methyl, -CH₂CH₂F, - CH₂CH₂OCH₃, -CH₂CHF₂, -CH₂CF₃, 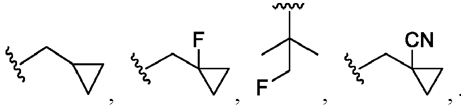 -CH(CH₃)CH₂F, -CH₂CH₂F and

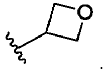

In some embodiments of the present disclosure, the R₁₃ is selected from H, fluorine, chlorine, bromine, methyl, ethyl, n-propyl, isopropyl, difluoromethyl, trifluoromethyl, trichloromethyl, cyclopropyl and

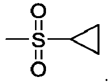

In some embodiments of the present disclosure, the R₁₃ is selected from H, fluorine, methyl, ethyl, difluoromethyl and

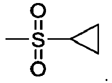

In some embodiments of the present disclosure, the R₁₃ is selected from H, fluorine, methyl, ethyl and difluoromethyl.

In some embodiments of the present disclosure, the R₁₃ is selected from methyl.

In some embodiments of the present disclosure, the R₁₄ is selected from methyl, ethyl, n-propyl, isopropyl, n-butyl, methoxy, ethoxy, n-propoxy, isopropoxy and cyclopropyl.

In some embodiments of the present disclosure, the R₁₄ is selected from methyl, isopropyl, cyclopropyl and ethoxy.

In some embodiments of the present disclosure, the R₁₄ is selected from methyl.

In some embodiments of the present disclosure, the R₁ is selected from H, halogen, -CN, C₁₋₃ alkyl, C₁₋₃ alkoxy, C₁₋₃ haloalkyl and C₁₋₃ haloalkoxy.

In some embodiments of the present disclosure, the R₁ is selected from H, methyl, ethyl, n-propyl, isopropyl, n-butyl, methoxy, ethoxy, n-propoxy, isopropoxy, n-butoxy, trifluoromethyl, trifluoromethoxy, trichloromethyl, trichloromethoxy and 2,2,2-trifluoroethoxy.

In some embodiments of the present disclosure, the R₁ is selected from H, methyl, methoxy, ethoxy and 2,2,2-trifluoroethoxy.

In some embodiments of the present disclosure, the R₁ is selected from methoxy.

In some embodiments of the present disclosure, the R₂ is selected from H, halogen, -CN, -OH, - NH₂, phosphonyl, sulfonyl, C₃₋₇ cycloalkyl, 3-7-membered heterocycloalkyl, C₆₋₁₄ spiro cyclyl, C₆₋₁₄ fused cyclyl, C₆₋₁₄ bridged cyclyl, 6-14-membered spiro heterocyclyl, 6-14-membered bridged heterocyclyl, 6-14-membered fused heterocyclyl and 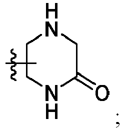 wherein, the -NH₂, phosphonyl, sulfonyl, C₃₋₇ cycloalkyl, 3-7-membered heterocycloalkyl, C₆₋₁₄ spiro cyclyl, C₆₋₁₄ fused ring, C₆₋₁₄ bridged cyclyl, 6-14-membered spiro heterocyclyl, 6-14-membered bridged heterocyclyl, 6-14-membered fused heterocyclyl and 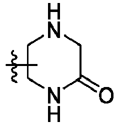 are optionally substituted by one or more R₁₂ groups, and the R₁₂ group is defined as above.

In some embodiments of the present disclosure, the R₂ is selected from H, halogen, -CN, -OH, - NH₂, -NHR₁₂, -NR₁₂R₁₂, 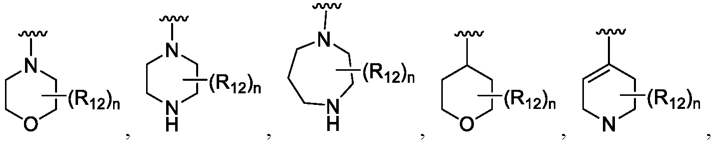 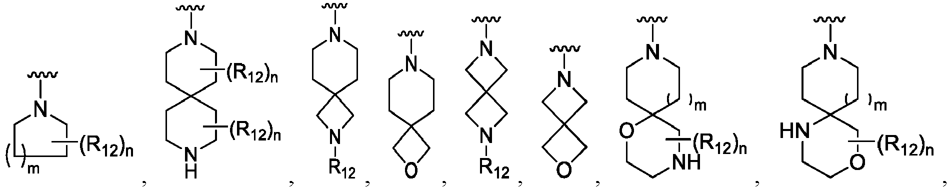 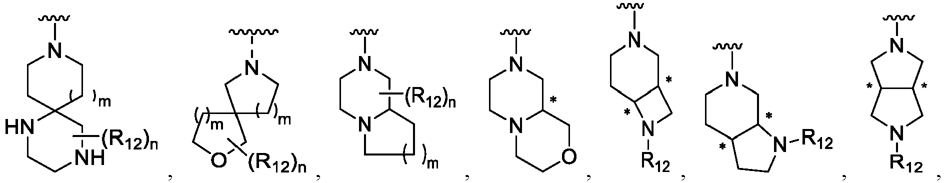 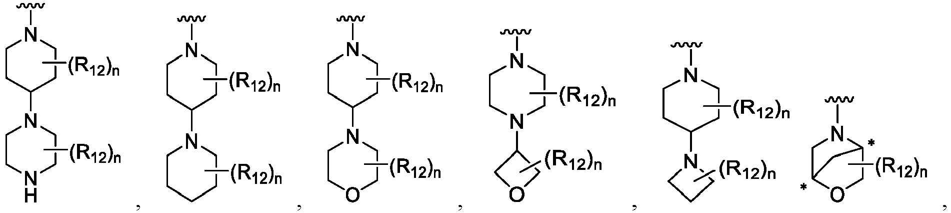 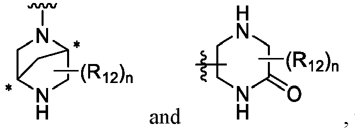 wherein the R₁₂, m and n are defined as above.

In some embodiments of the present disclosure, the R₂ is selected from H, fluorine, chlorine, bromine, iodine, -NH₂,

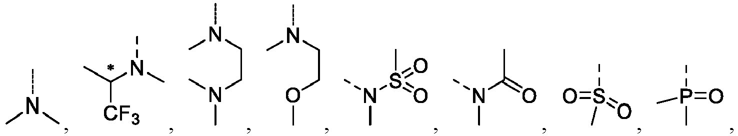

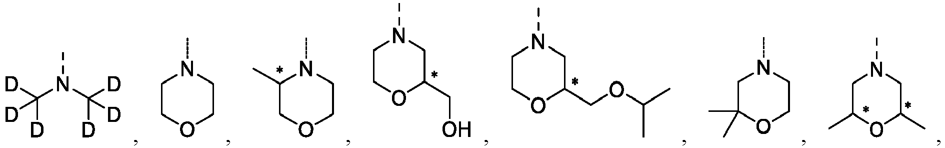

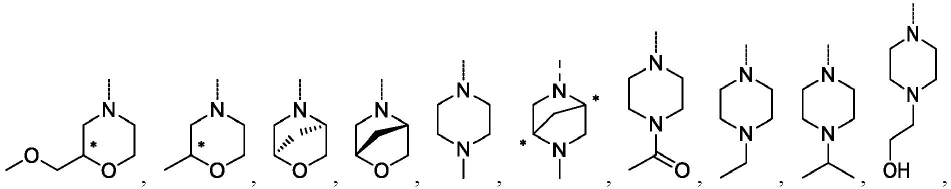

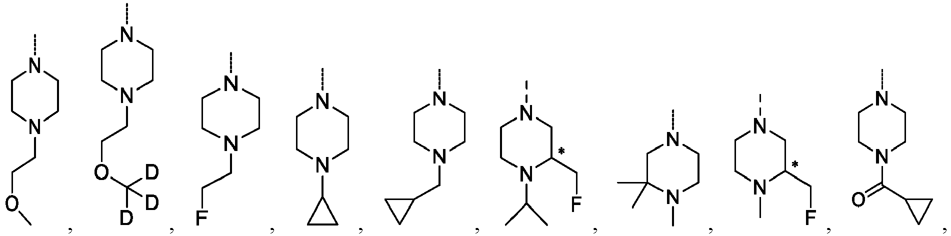

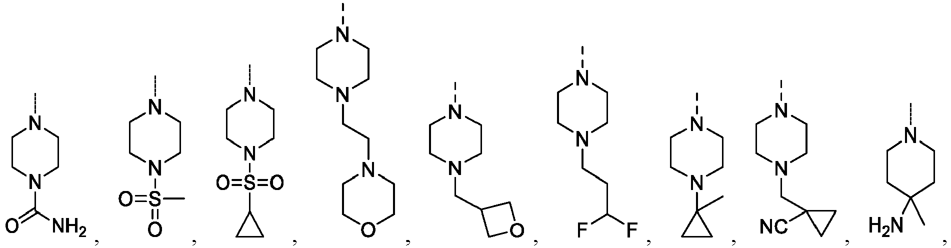

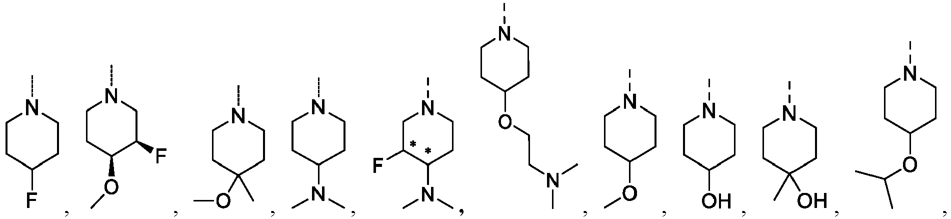

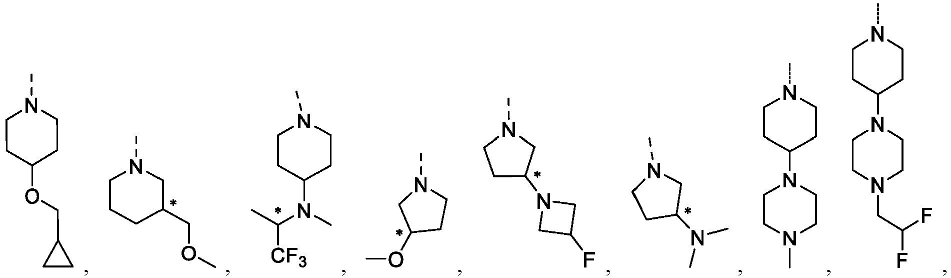

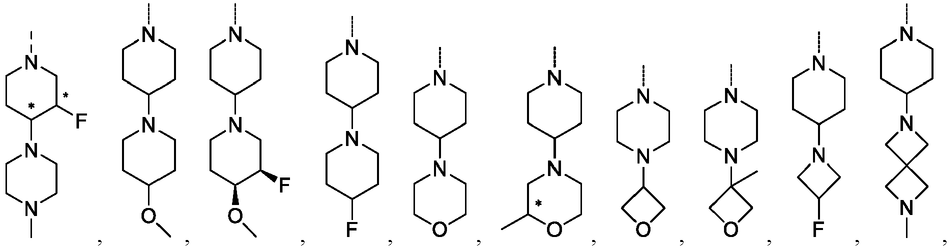

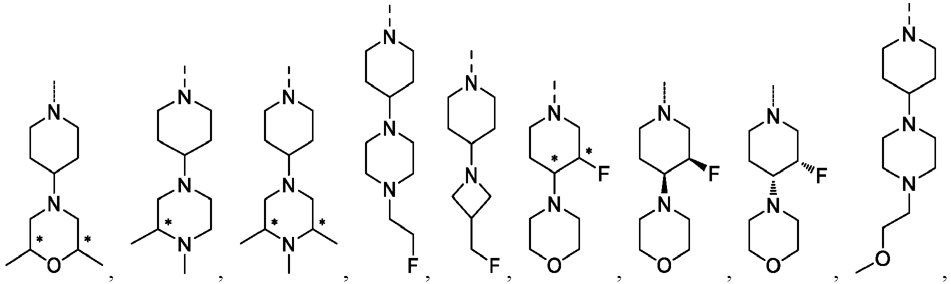

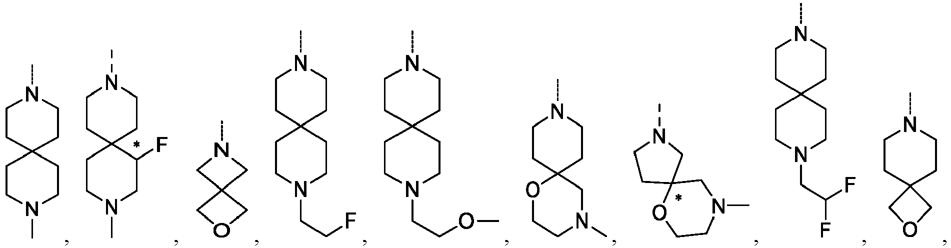

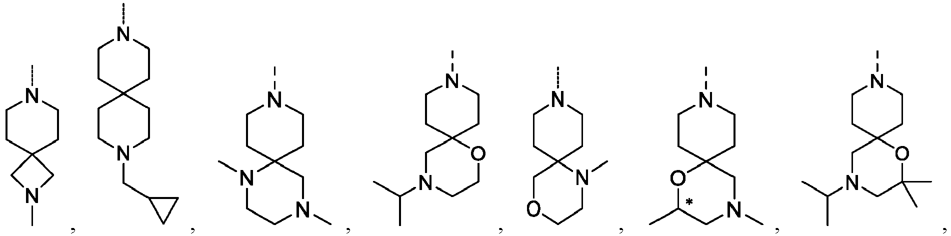

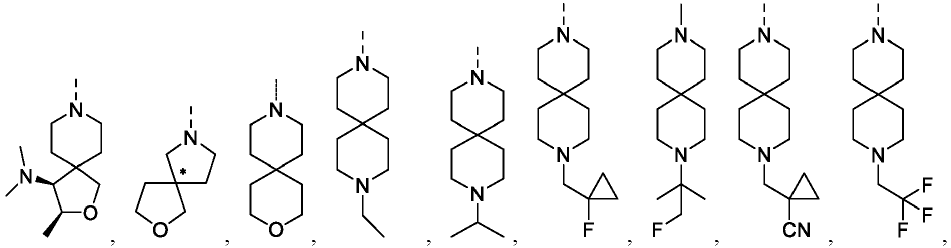

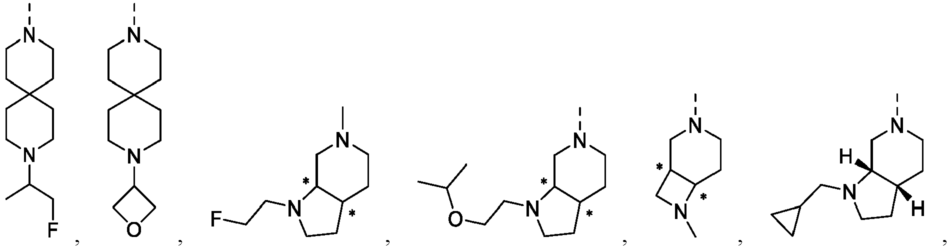

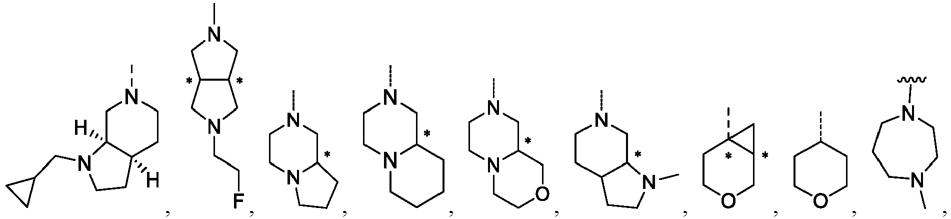

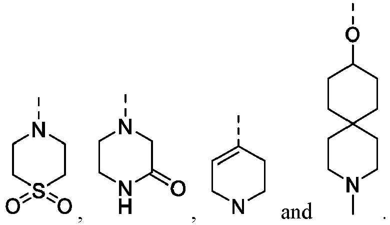

In some embodiments of the present disclosure, the R₂ is selected from 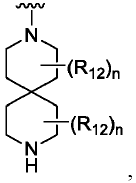 wherein the R₁₂ and n are defined as above.

In some embodiments of the present disclosure, the R₂ is selected from

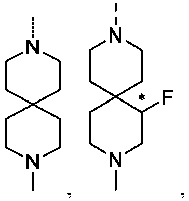

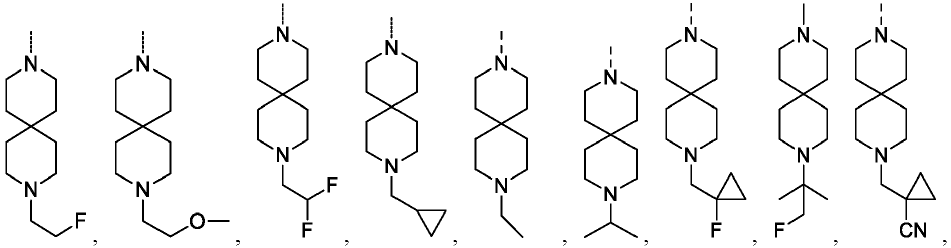

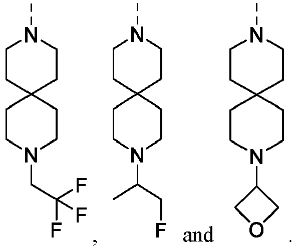

In some embodiments of the present disclosure, the R₂ is selected from

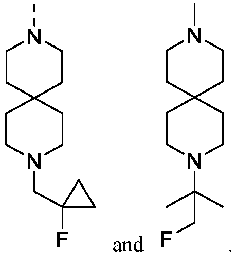

In some embodiments of the present disclosure, the R₃ is selected from -CN, phenyl, 5-6-membered heteroaryl and 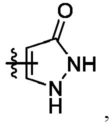 and the phenyl, 5-6-membered heteroaryl and 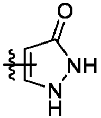 are optionally substituted by one or more R₁₃ groups.

In some embodiments of the present disclosure, the R₃ is selected from -CN, phenyl, pyranyl, pyridyl, pyrimidinyl, pyridazinyl, pyrazinyl, triazinyl, furanyl, thienyl, pyrrolyl, pyrazolyl, thiazolyl, imidazolyl, benzofuranyl, benzimidazolyl, benzothienyl, benzoxazolyl, benzothiazolyl, indolyl, pyrazolo[1,5-*α*]pyridyl, quinolinyl, isoquinolinyl, tetrahydropyrrolyl, piperidinyl, piperazinyl, morpholinyl, tetrahydrofuranyl, tetrahydropyranyl, cyclobutyl, cyclopentyl, cyclohexyl and 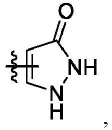 and the phenyl, pyranyl, pyridyl, pyrimidinyl, pyridazinyl, pyrazinyl, triazinyl, furanyl, thienyl, pyrrolyl, pyrazolyl, thiazolyl, imidazolyl, benzofuranyl, benzimidazolyl, benzothienyl, benzoxazolyl, benzothiazolyl, indolyl, pyrazolo[1,5-a]pyridyl, quinolinyl, isoquinolinyl, tetrahydropyrrolyl, piperidinyl, piperazinyl, morpholinyl, tetrahydrofuranyl, tetrahydropyranyl, cyclobutyl, cyclopentyl, cyclohexyl and 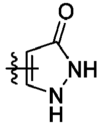 are optionally substituted by one or more R₁₃ groups, wherein the R₁₃ group is defined as above.

In some embodiments of the present disclosure, the R₃ is selected from -CN, 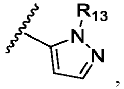 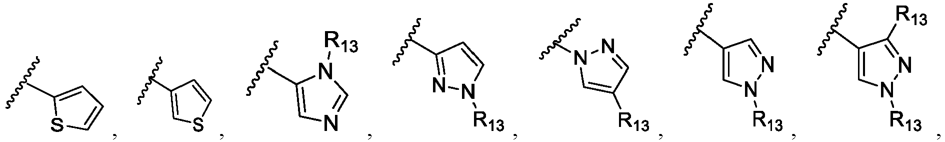 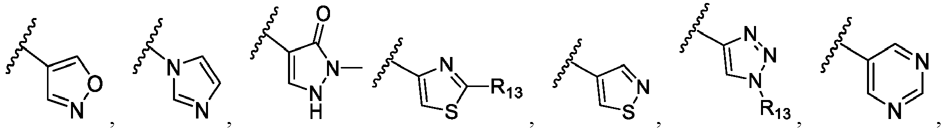 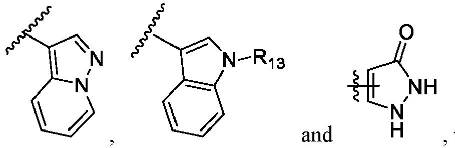 wherein the R₁₃ is defined as above.

In some embodiments of the present disclosure, the R₃ is selected from 5-6-membered heteroaryl optionally substituted by one or more R₁₃ groups.

In some embodiments of the present disclosure, the R₃ is selected from -CN,

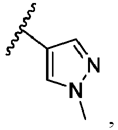

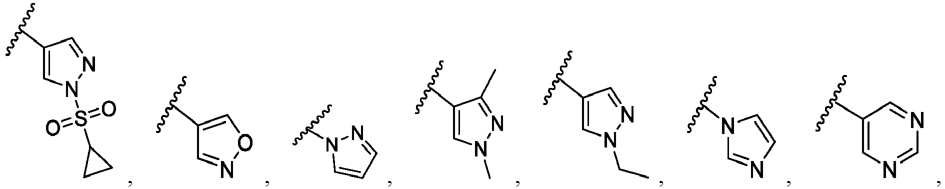

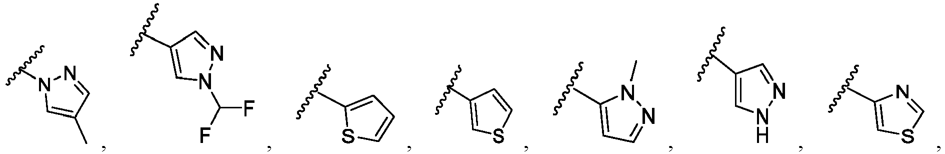

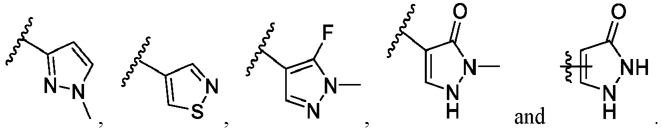

In some embodiments of the present disclosure, the R₃ is selected from 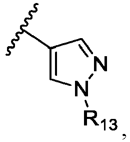 wherein the R₁₃ is defined as above.

In some embodiments of the present disclosure, the R₃ is selected from

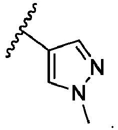

In some embodiments of the present disclosure, the R₄ and R₅ are each independently selected from H, F, Cl, Br, CN, methyl, ethyl, isopropyl, methoxy, ethoxy, isopropoxy, trifluoromethyl, 2,2,2-trifluoroethyl and cyclopropyl.

In some embodiments of the present disclosure, the R₄ and R₅ are each independently selected from H, F, Cl, Br, CN, methyl, ethyl, trifluoromethyl and cyclopropyl.

In some embodiments of the present disclosure, the R₄ is selected from H, and R₅ is selected from H, F, Cl, Br, CN, methyl, ethyl, trifluoromethyl and cyclopropyl.

In some embodiments of the present disclosure, the R₄ is selected from H, and R₅ is selected from Br.

In some embodiments of the present disclosure, the R₄ and R₅ form C₅₋₆ alkyl or 5-6-membered heteroaryl.

In some embodiments of the present disclosure, the R₆ is selected from

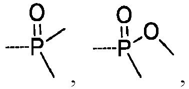

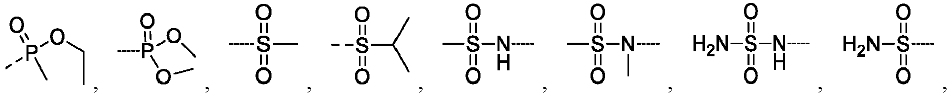

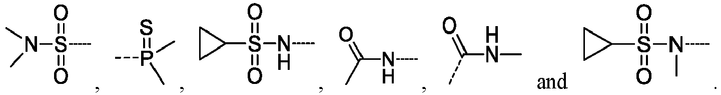

In some embodiments of the present disclosure, the R₆ is selected from

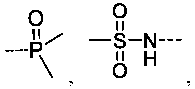

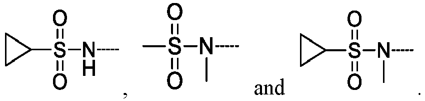

In some embodiments of the present disclosure, the R₆ is selected from

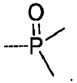

In some embodiments of the present disclosure, R₇ and R₈ are cyclized to C₄₋₆ cycloalkyl, 4-6-membered heterocycloalkyl, C₅₋₆ aryl or 5-7-membered heteroaryl, or R₈ and R₉ are cyclized to C₄₋₆ cycloalkyl, 4-6-membered heterocycloalkyl, C₅₋₆ aryl or 5-7-membered heteroaryl.

In some embodiments of the present disclosure, R₇ and R₈ or R₈ and R₉ are independently cyclized to cyclobutane, cyclopentane, tetrahydropyrrole ring, tetrahydrofuran ring, tetrahydropyran ring, thiophene ring, imidazole ring, pyrazole ring, pyrrole ring, oxazole ring, thiazole ring, isoxazole ring, piperazine ring, isothiazole ring, benzene ring, pyridine ring, piperidine ring, pyrimidine ring, pyridazine ring or pyrazine ring.

In some embodiments of the present disclosure, R₇ and R₈ or R₈ and R₉ are independently cyclized to cyclobutane, pyridine ring or pyrazine ring.

In some embodiments of the present disclosure, R₇ and R₈ are independently cyclized to cyclobutane, pyridine ring or pyrazine ring.

In some embodiments of the present disclosure, R₇ and R₈ are independently cyclized to pyrazine ring.

In some embodiments of the present disclosure, R₉ and R₈ are independently cyclized to cyclobutane.

In some embodiments of the present disclosure, R₇, R₈ and R₉ are all H.

In some embodiments of the present disclosure, the compound represented by formula (I), the stereoisomer thereof, the tautomer thereof, the pharmaceutically acceptable salt thereof, the prodrug thereof, the hydrate thereof, the solvate thereof or the isotope-labeled derivative thereof is selected from, 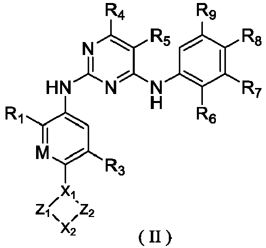 wherein, X₁ is independently selected from CR_{c} and N;
X₂ is independently selected from -CR_{c}R_{d}-, -NR_{c}- and -O-;
Z₁ and Z₂ are each independently selected from -(CRₑR_{f})ₘ(CRₑR_{f})ₙ-;
the R_{c}, R_{d}, Rₑ, and R_{f} are each independently selected from H, halogen, -CN, -OH, -NRₐR_{b}, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, phenyl, -(CH₂)ₘNRₐR_{b}, hydroxy-C₁₋₆ alkyl-, - O(CH₂)ₘNRₐR_{b}, C₃₋₈ cycloalkylalkyl-, C₃₋₈ heterocycloalkylalkyl-, C₃₋₈ cycloalkyl-C₁₋₆ alkoxy-, C₃₋₈ heterocycloalkyl-C₁₋₆ alkoxy-, C₁₋₆ alkylcarbonyl-, C₃₋₈ cycloalkylcarbonyl-, NRₐR_{b}CO-, C₁₋₆ alkylcarbonylsulfonyl-, C₃₋₈ cycloalkylsulfonyl-, C₁₋₆ alkyl-O-C₁₋₆ alkyl- and 6-14-membered spiro heterocyclyl, wherein the Rₐ, R_{b}, m and n are defined as above;
or, R_{c} and R_{d} together form C₃₋₈ cycloalkyl or 3-8-membered heterocycloalkyl;
or, Rₑ and R_{f} together form C₃₋₈ cycloalkyl or 3-8-membered heterocycloalkyl;
or, R_{c} and Rₑ, or R_{c} and R_{f}, or R_{d} and Rₑ, or R_{d} and R_{f} together form C₃₋₈ cycloalkyl or 3-8-membered heterocycloalkyl;
wherein, a ring formed by X₁, X₂, Z₁ and Z₂ and a ring further formed by substituent groups R_{c}, R_{d}, Rₑ and R_{f} thereof are optionally substituted by one or more R₁₂ groups;
R₁, R₃, R₄, R₅, R₆, R₇, R₈, R₉, R₁₂ and M are defined as above.

In some embodiments of the present disclosure, the compound represented by formula (I), the stereoisomer thereof, the tautomer thereof, the pharmaceutically acceptable salt thereof, the prodrug thereof, the hydrate thereof, the solvate thereof or the isotope-labeled derivative thereof is selected from, 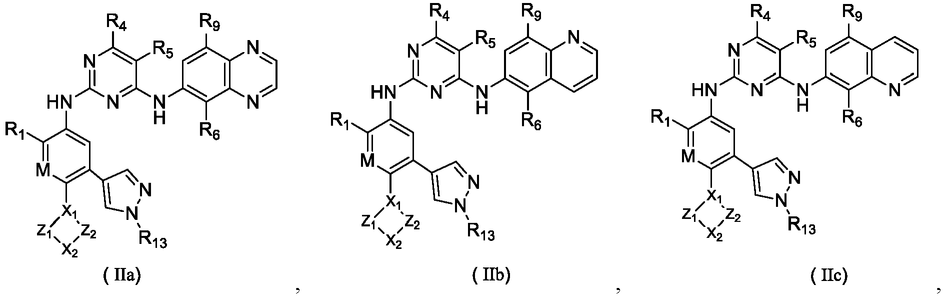 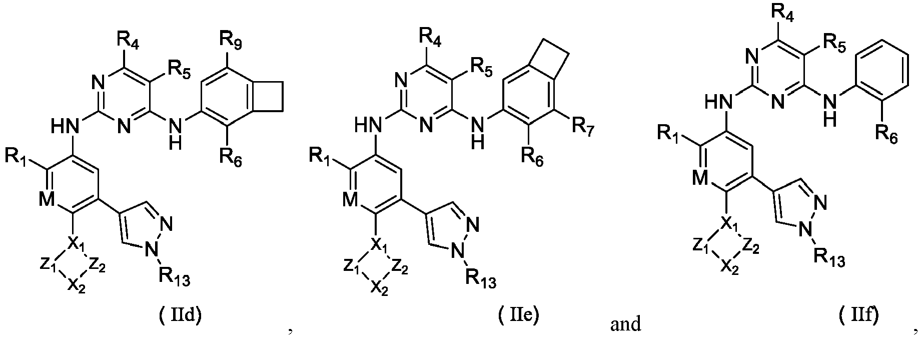 wherein, the ring formed by X₁, X₂, Z₁ and Z₂ and the ring further formed by substituent groups thereof are optionally substituted by one or more R₁₂ groups;
X₁, X₂, Z₁, Z₂, R₁, R₄, R₅, R₆, R₇, R₉, R₁₂, R₁₃ and M are defined as above.

In some embodiments of the present disclosure, the compound represented by formula (I), the stereoisomer thereof, the tautomer thereof, the pharmaceutically acceptable salt thereof, the prodrug thereof, the hydrate thereof, the solvate thereof or the isotope-labeled derivative thereof is selected from, 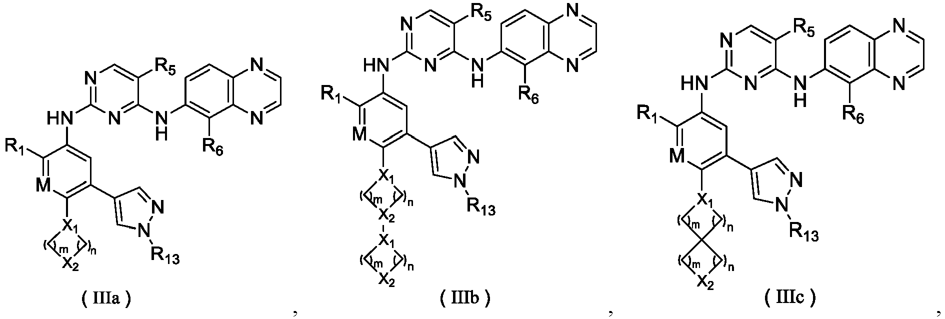 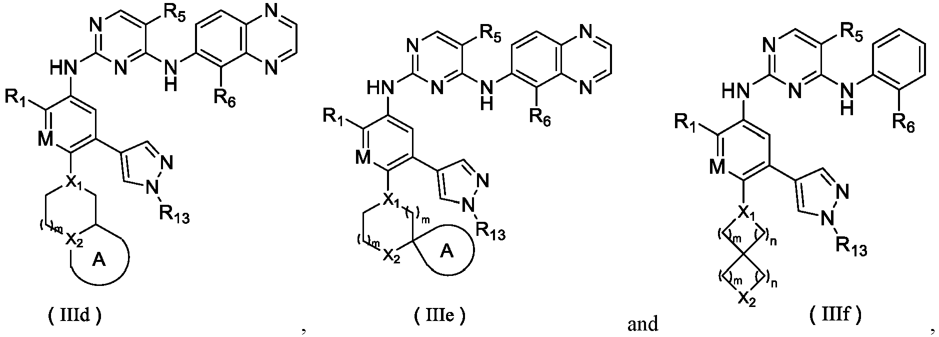 wherein, ring A is selected from C₅₋₇ cycloalkyl and 5-7-membered heterocycloalkyl;
a monocyclic ring, a bicyclic ring, a spiro ring, a fused ring formed by X₁ and X₂ and ring A are optionally substituted by one or more R₁₂ groups;
X₁, X₂, R₁, R₅, R₆, m, n, M, R₁₂ and R₁₃ are defined as above.

In some embodiments of the present disclosure, the compound represented by formula (I), the stereoisomer thereof, the tautomer thereof, the pharmaceutically acceptable salt thereof, the prodrug thereof, the hydrate thereof, the solvate thereof or the isotope-labeled derivative thereof is selected from, 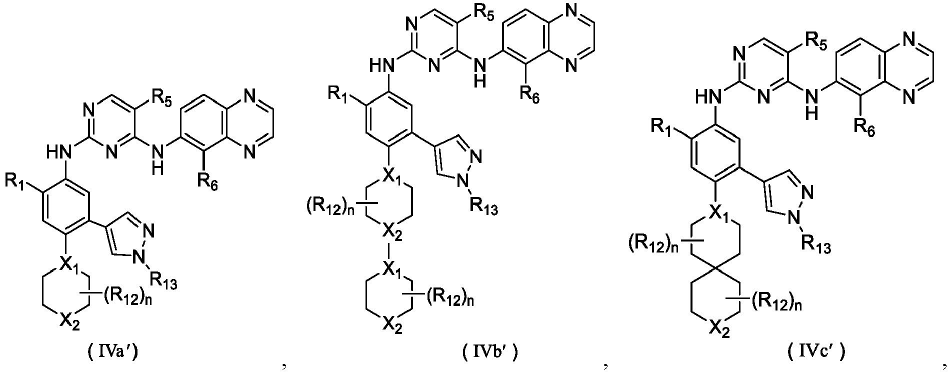 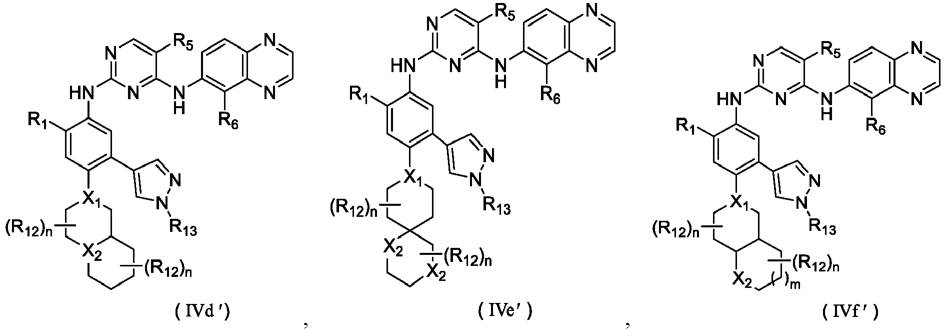 and 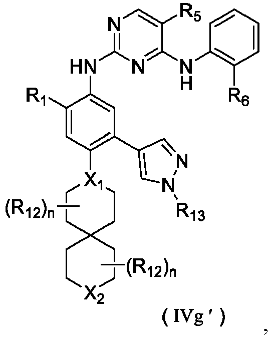 X₁, X₂, R₁, R₅, R₆, m, n, M, R₁₂ and R₁₃ are defined as above.

In some embodiments of the present disclosure, the compound represented by formula (I), the stereoisomer thereof, the tautomer thereof, the pharmaceutically acceptable salt thereof, the prodrug thereof, the hydrate thereof, the solvate thereof or the isotope-labeled derivative thereof is selected from, 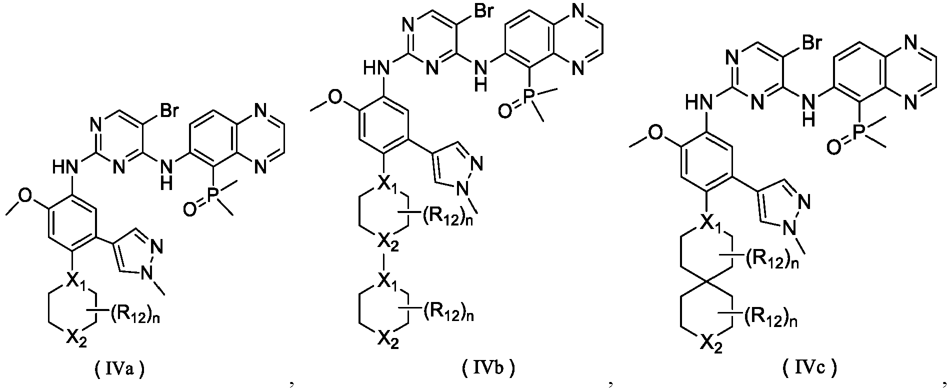 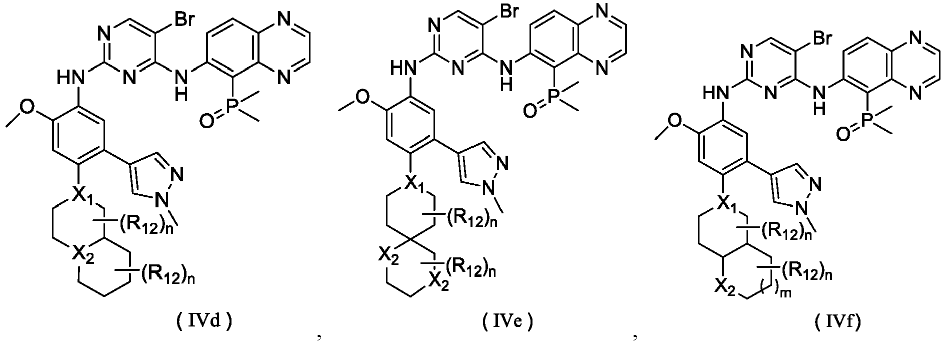 and 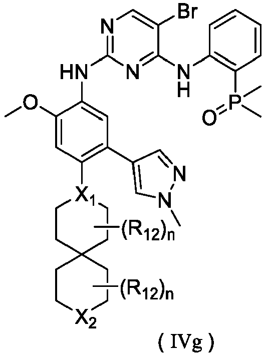 wherein X₁, X₂, R₁₂, m and n are defined as above.

In some embodiments of the present disclosure, the compound represented by formula (I), the stereoisomer thereof, the tautomer thereof, the pharmaceutically acceptable salt thereof, the prodrug thereof, the hydrate thereof, the solvate thereof or the isotope-labeled derivative thereof is selected from,

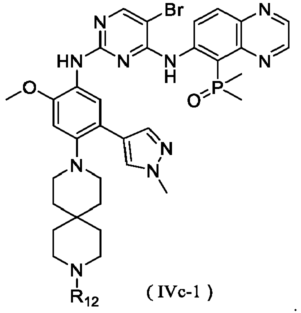

In some embodiments of the present disclosure, the compound represented by formula (I), the stereoisomer thereof, the tautomer thereof, the pharmaceutically acceptable salt thereof, the prodrug thereof, the hydrate thereof, the solvate thereof or the isotope-labeled derivative thereof is selected from, 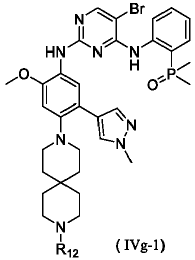 wherein R₁₂ is defined as above.

The present disclosure further provides a compound represented by the following formula, a stereoisomer thereof, a tautomer thereof, a pharmaceutically acceptable salt thereof, a prodrug thereof, a hydrate thereof, a solvate thereof or a isotope-labeled derivative thereof, which is selected from,

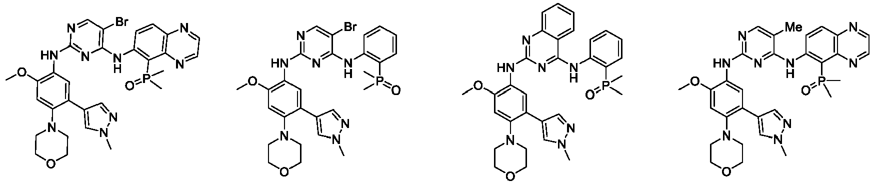

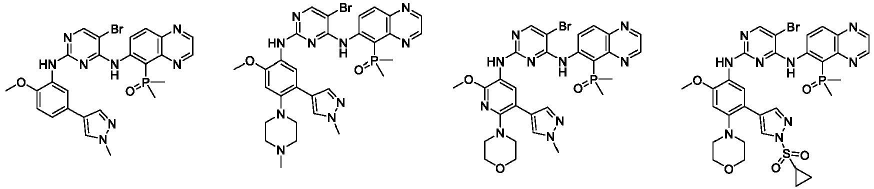

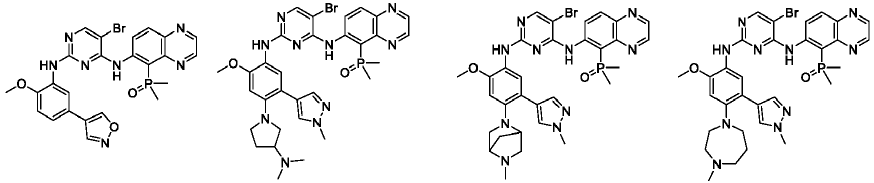

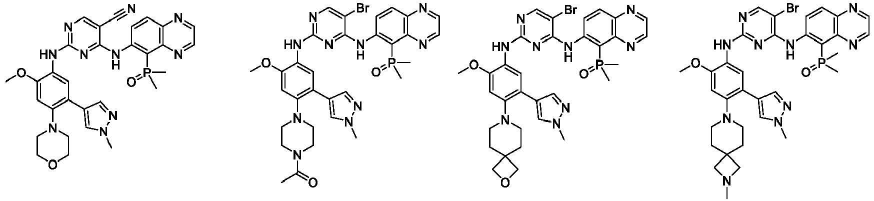

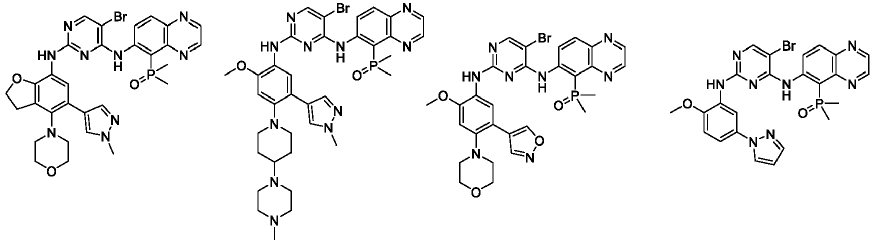

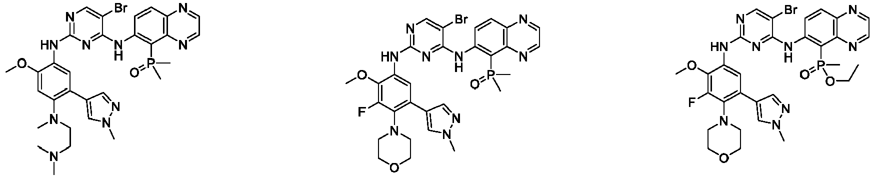

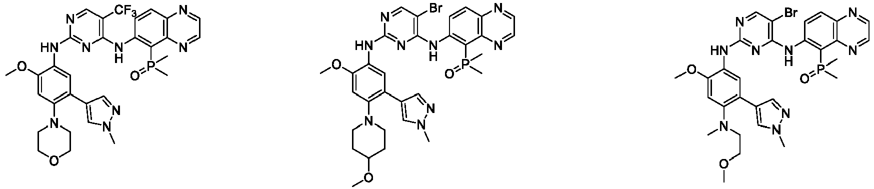

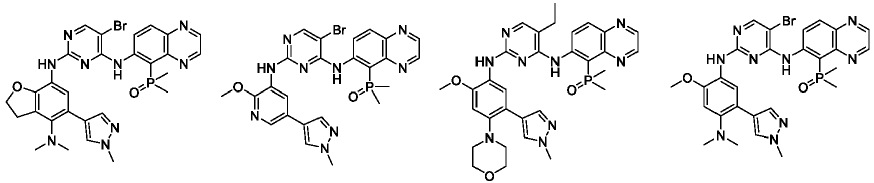

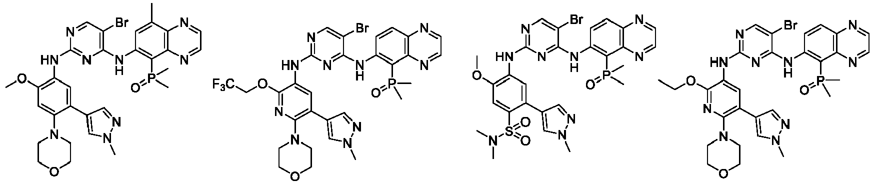

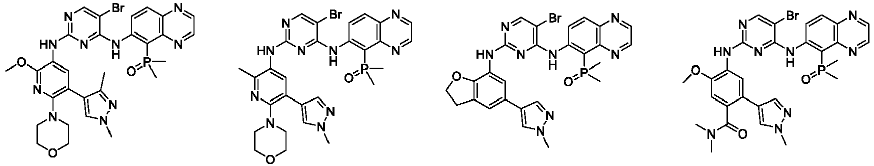

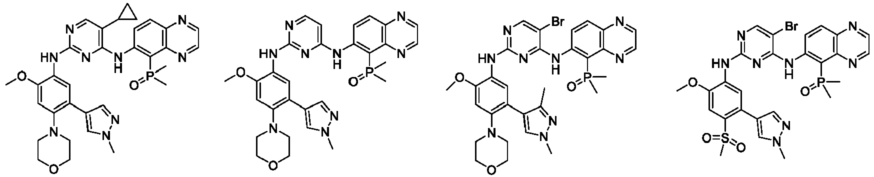

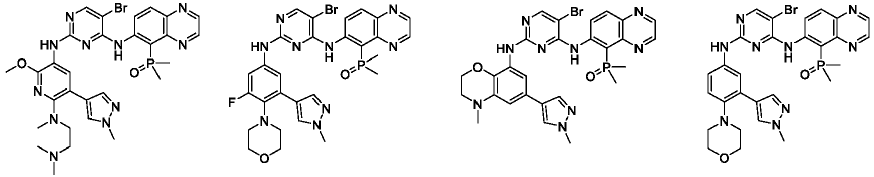

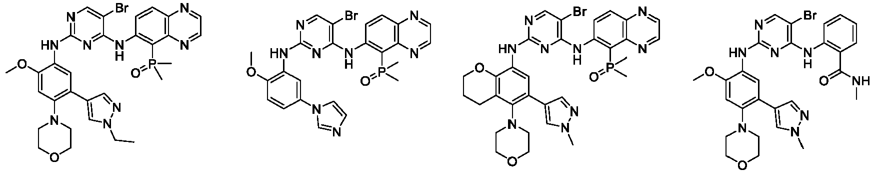

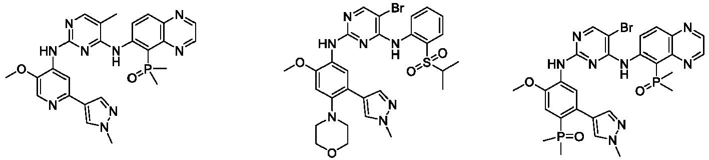

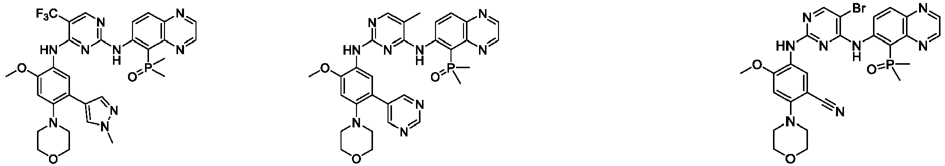

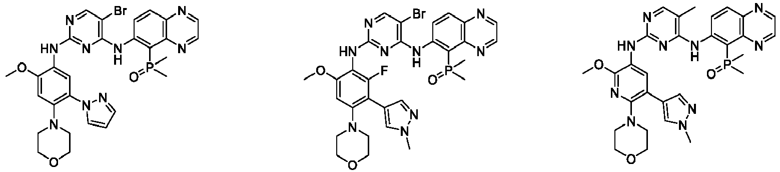

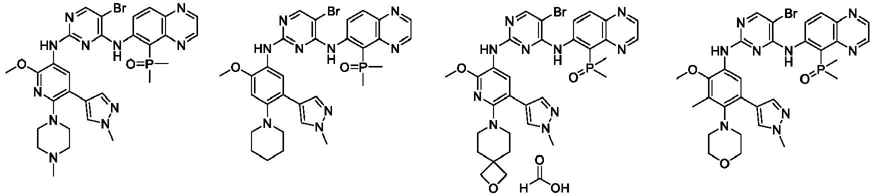

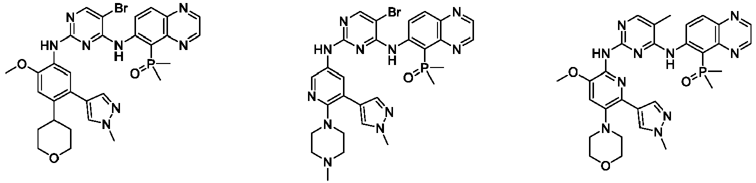

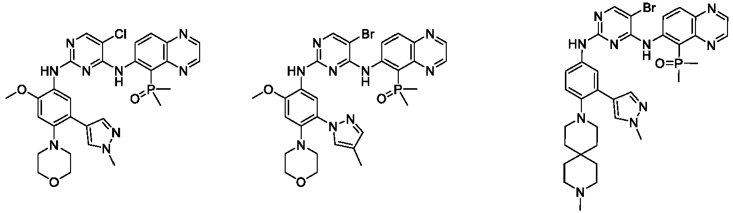

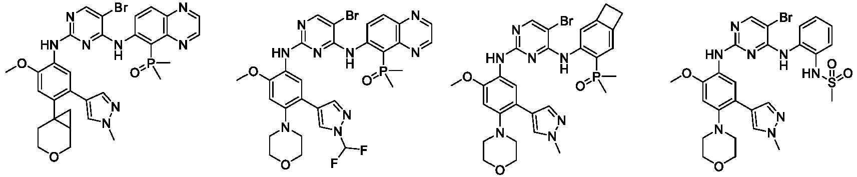

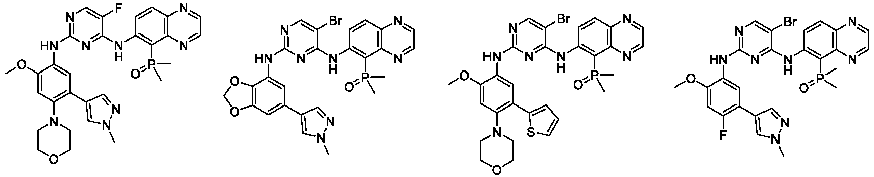

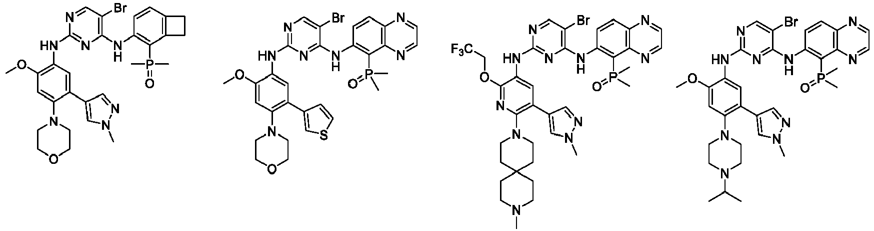

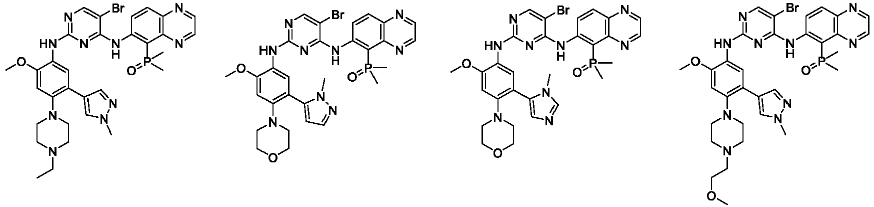

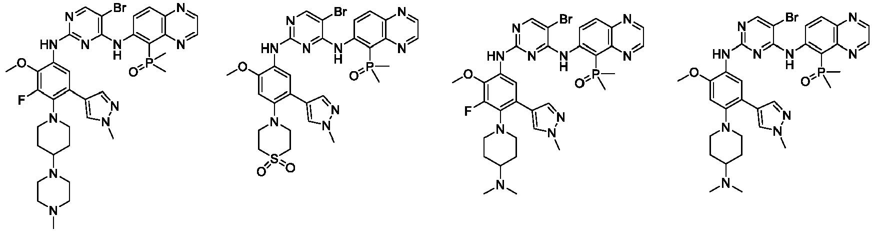

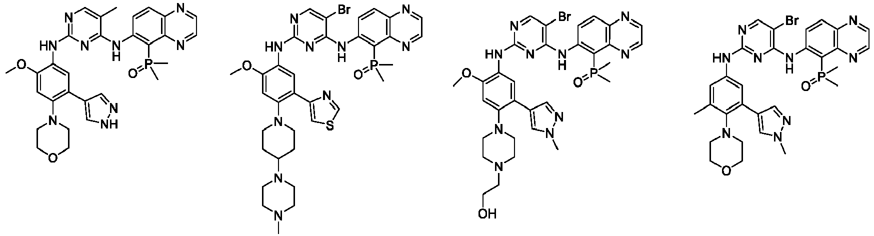

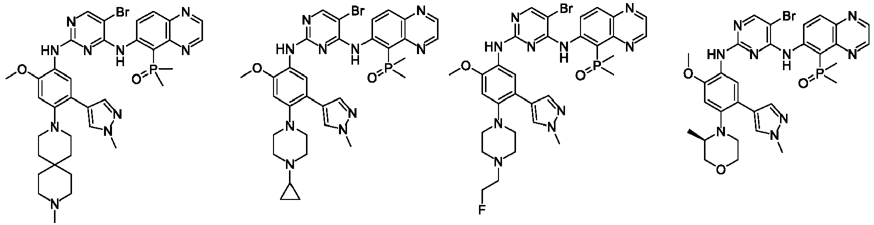

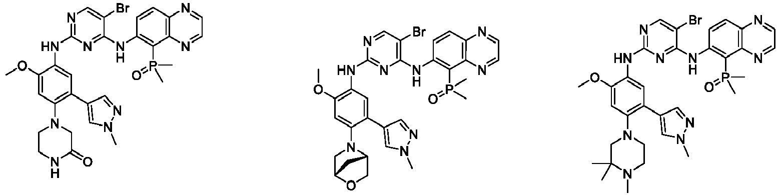

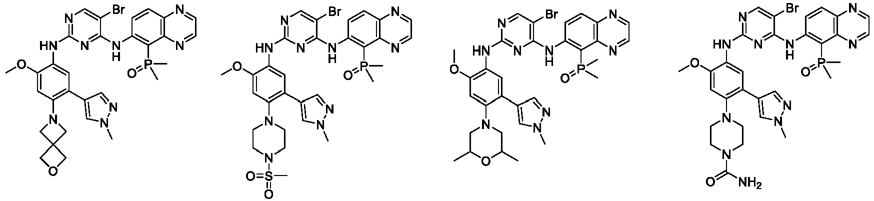

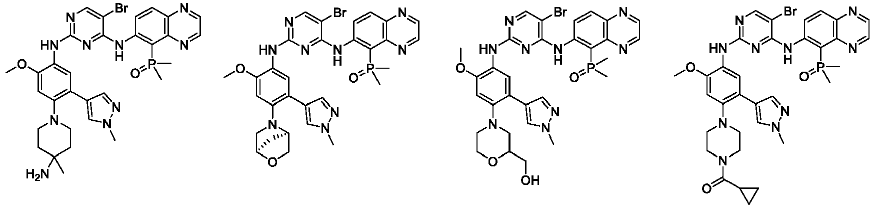

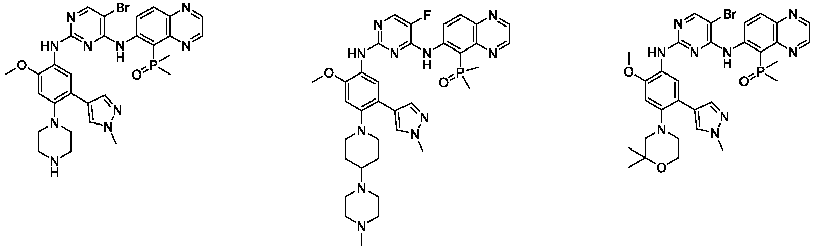

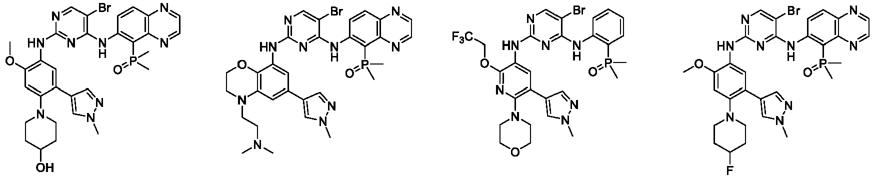

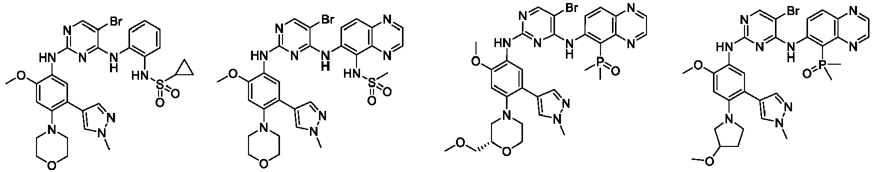

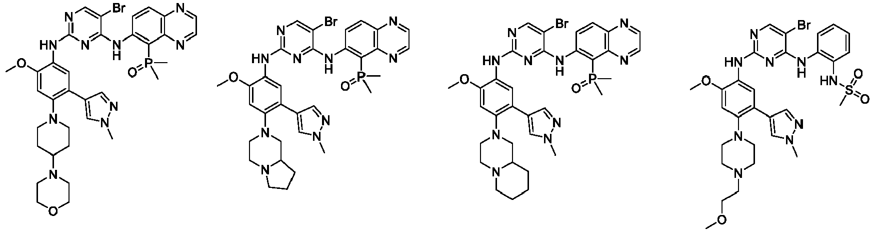

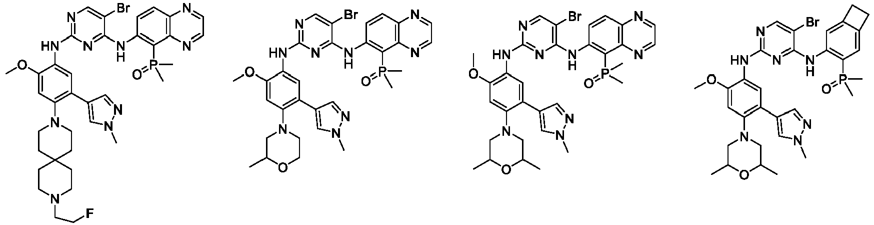

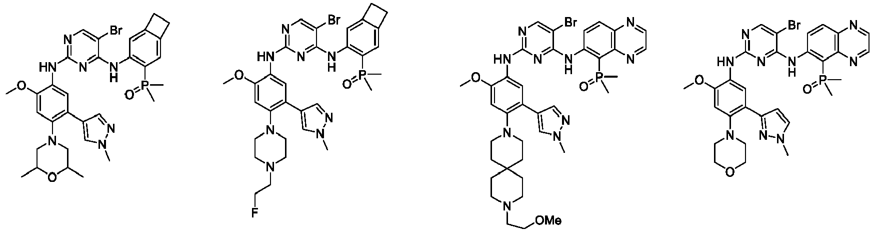

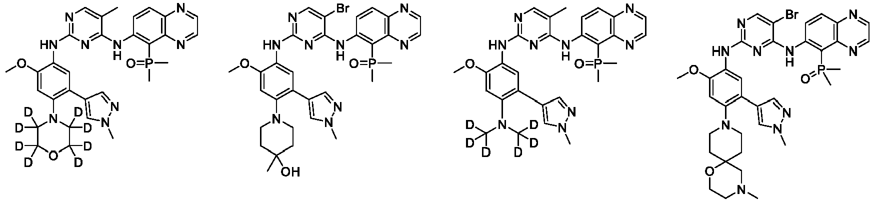

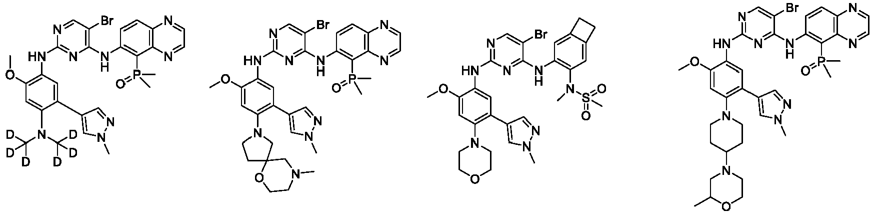

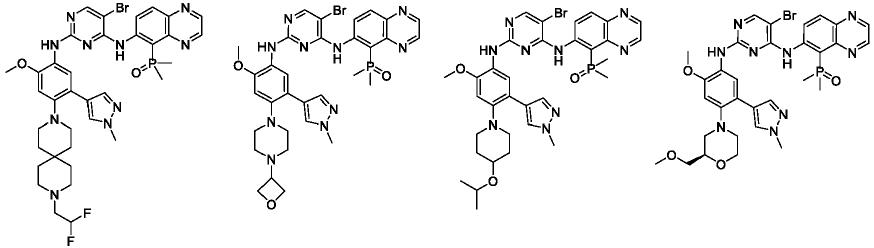

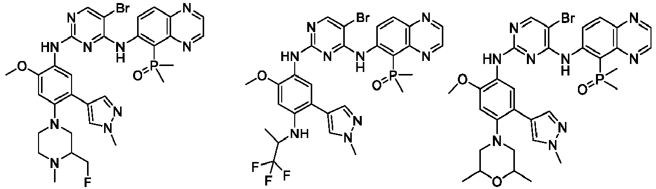

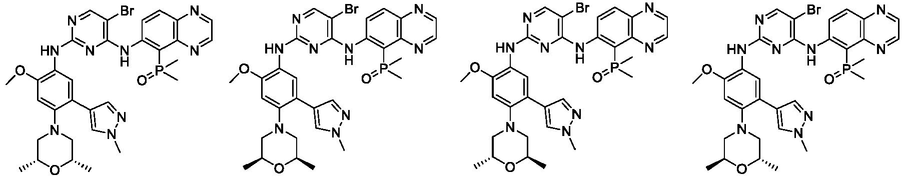

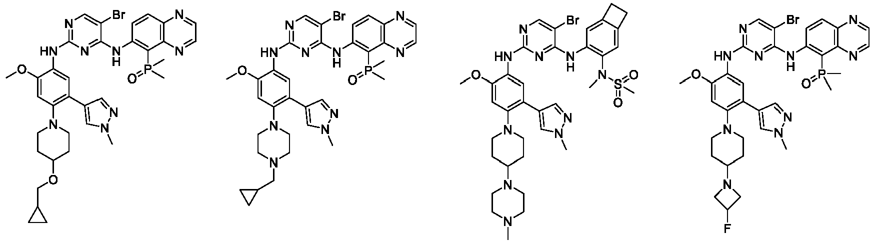

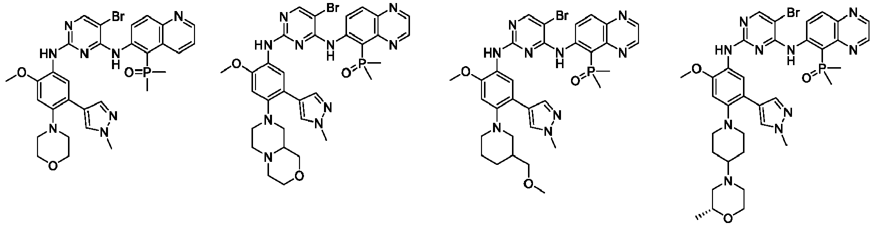

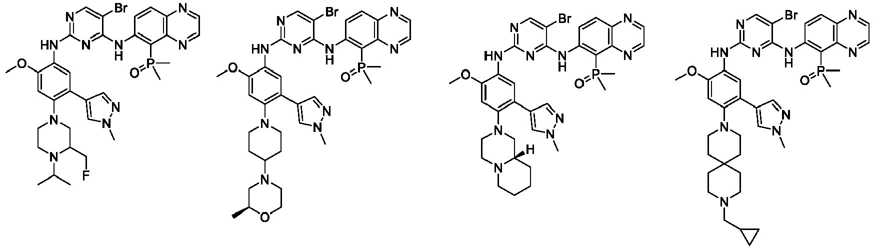

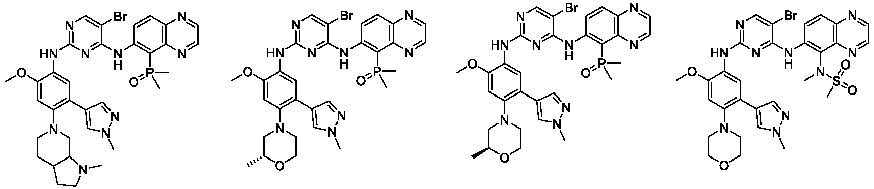

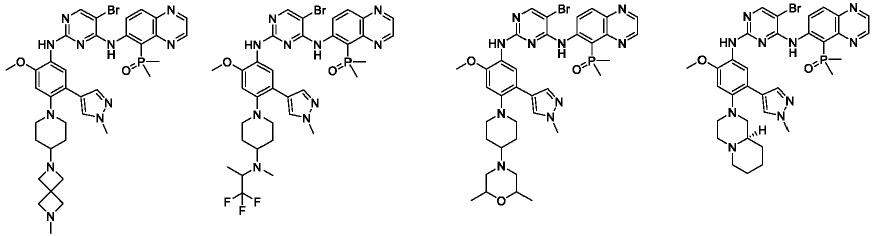

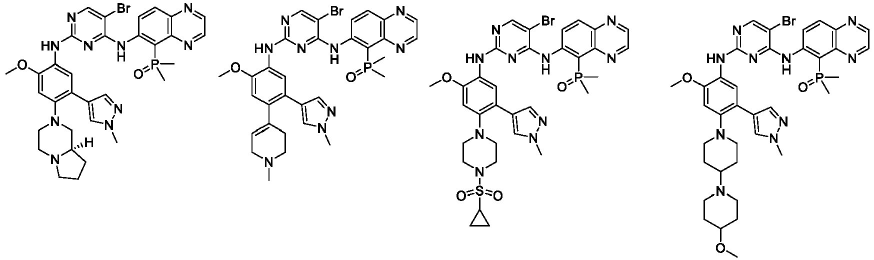

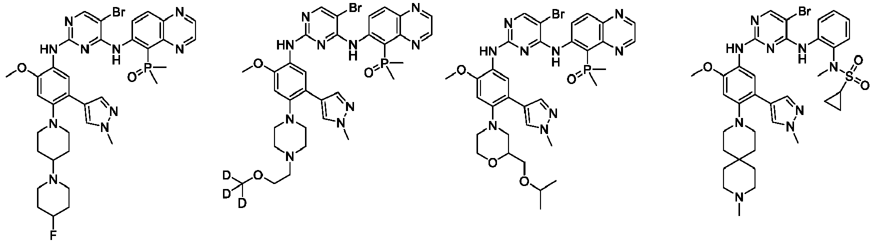

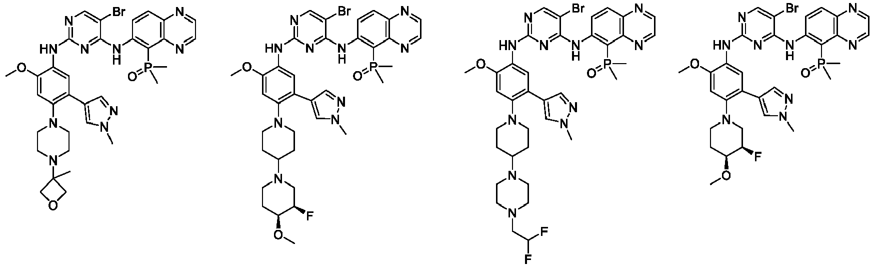

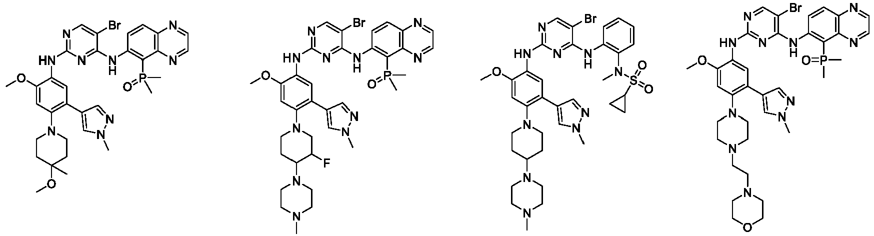

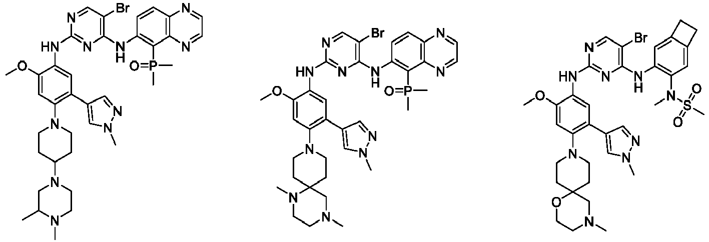

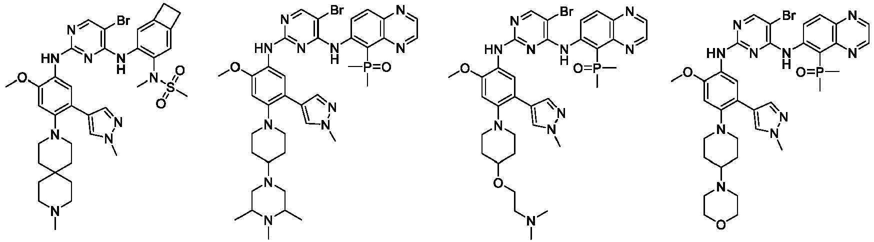

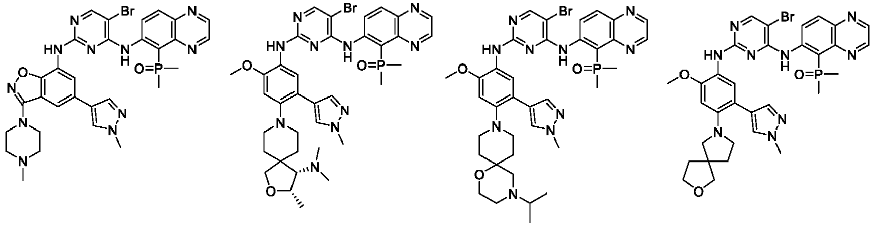

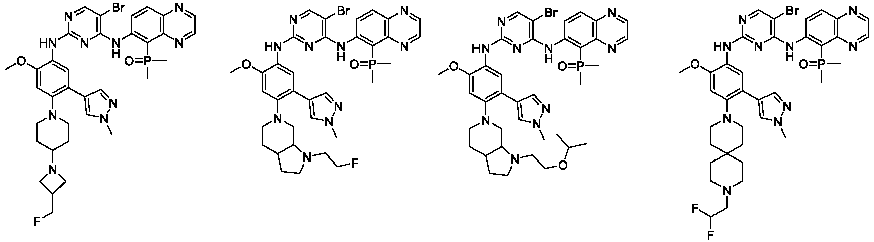

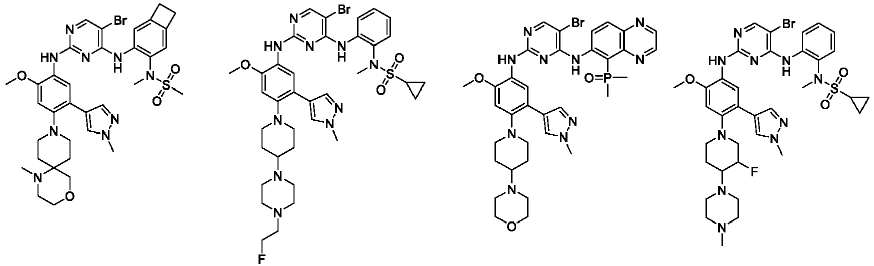

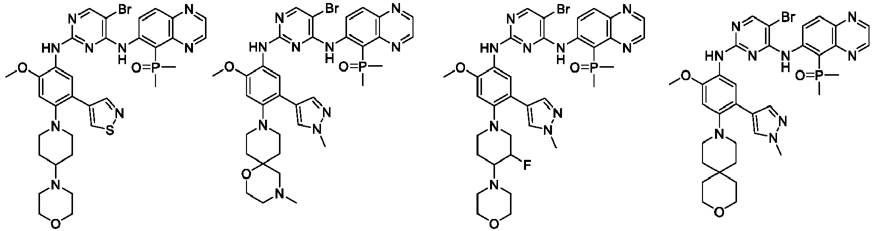

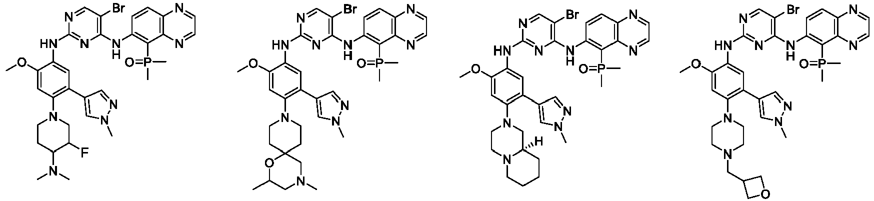

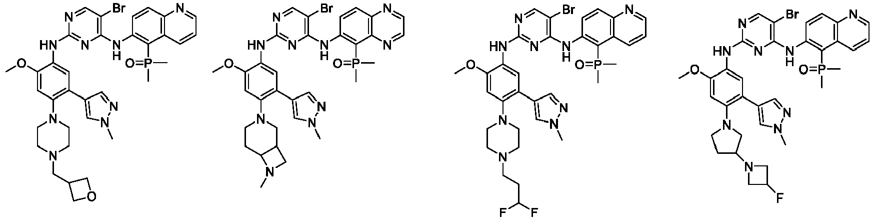

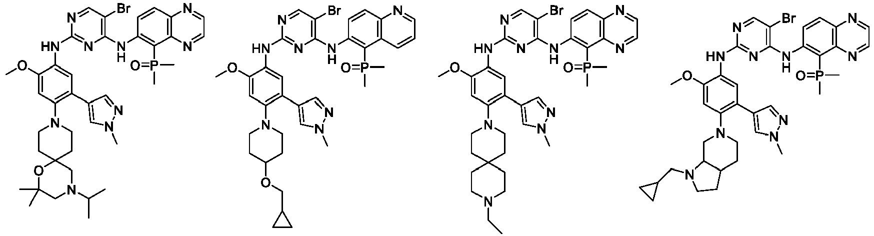

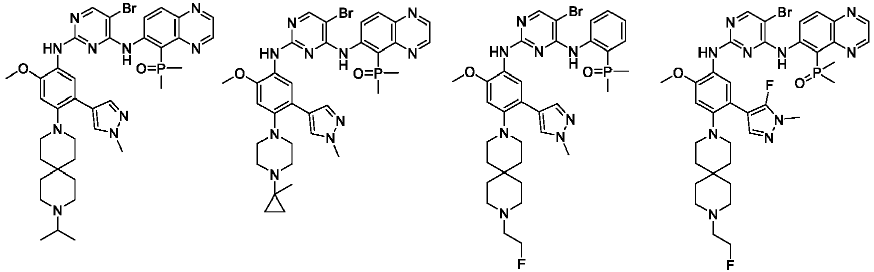

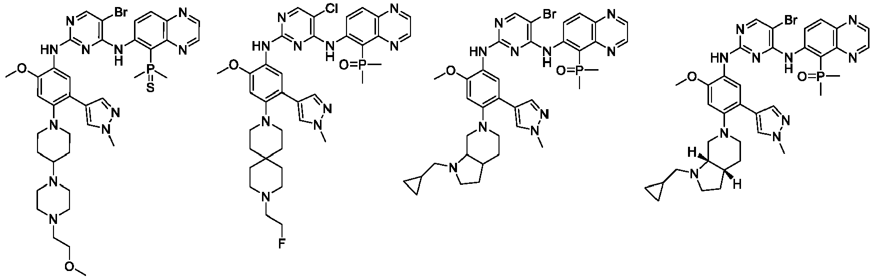

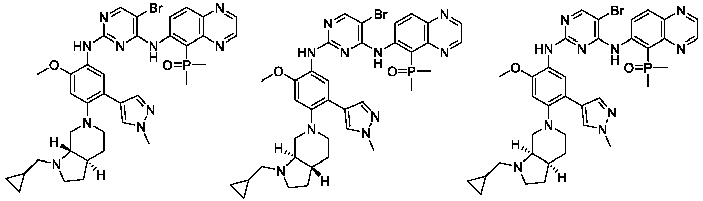

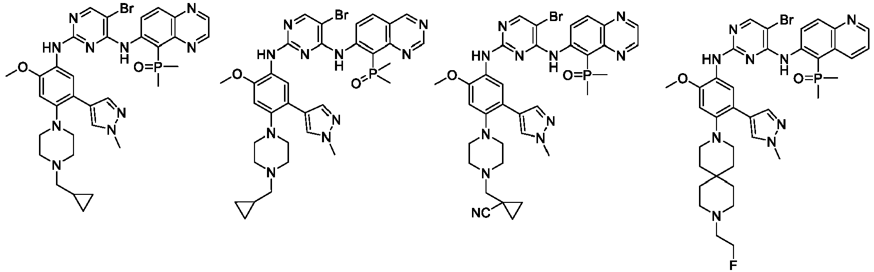

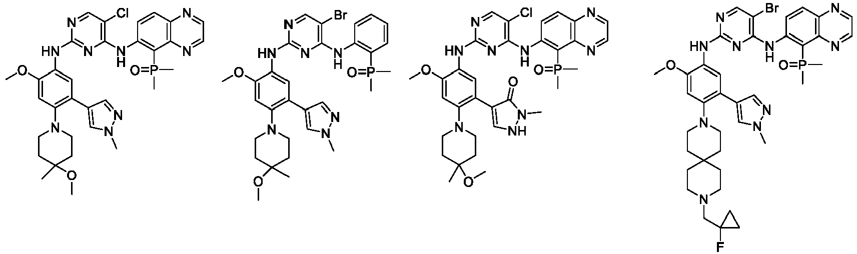

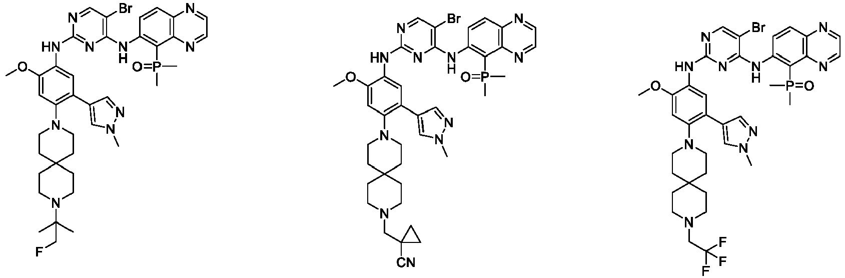

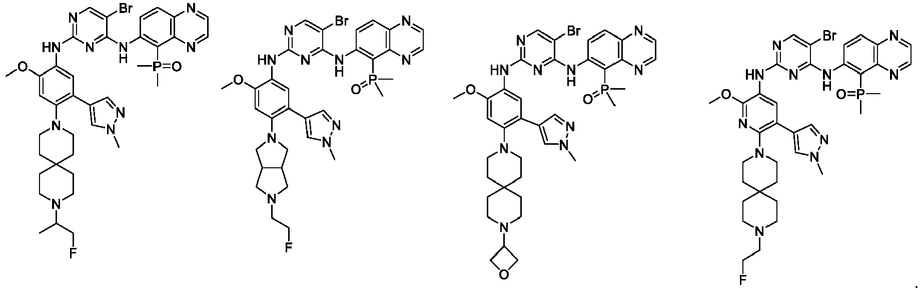

The present disclosure also provides a pharmaceutical composition comprising a therapeutically effective amount of the above compound or the pharmaceutically acceptable salt thereof and a pharmaceutically acceptable carrier, diluent and excipient. The pharmaceutical composition may be formulated for specific routes of administration, such as oral administration, parenteral administration, rectal administration, and the like. Oral administration, for example, a tablet, a capsule (including a sustained release or timed release prescription), a pill, a powder, a granule, an elixir, a tincture, a suspension (including a nano-suspension, a micro-suspension, a spray-dried dispersant), a syrup and an emulsion; sublingual administration; buccal administration; parenteral administration, for example, by subcutaneous, intravenous, intramuscular, or intrasternal injection, or infusion techniques (for example, as a sterile injectable aqueous solution or non-aqueous solution or a mixed suspension); nasal administration, including administration to the nasal mucosa, for example, by inhalation spray; topical administration, for example, in the form of a cream or an ointment; or rectal administration, for example, in the form of a suppository. They can be administered alone, but they are usually administered together with a pharmaceutical carrier selected according to the selected route of administration and standard pharmaceutical practice.

"Pharmaceutically acceptable carrier" refers to a medium generally acceptable in the art for delivering a biologically active agent to animals, particularly mammals, including, for example, an adjuvant, an excipient or a vehicle, such as a diluent, a preservative, a filler, a flow regulator, a disintegrant, a wetting agent, an emulsifier, a suspending agent, a sweetener, a flavor, a fragrance, an antibacterial agent, an antifungal agent, a lubricants agent and a dispersant, according to the mode of administration and the nature of dosage forms. The pharmaceutically acceptable carrier is formulated according to a large number of factors within the purview of those of ordinary skill in the art, including, but not limited to, the type and nature of the active agent formulated, the subjects to which the composition containing the agent is to be administered, the expected route of administration of the composition, and the target therapeutic indication. The pharmaceutically acceptable carriers include both aqueous and non-aqueous media and various solid and semisolid dosage forms. In addition to the active agent, such carriers include many different ingredients and additives, and such additional ingredients included in prescriptions for a variety of reasons (e.g., stabilizing the active agent and an adhesive, etc.) are well known to those of ordinary skill in the art.

As a general guideline, when used for the indicated effects, the daily oral dosage of various active ingredients is in the range of about 0.001- about 5000 mg, or about 1-500 mg, or about 1-250 mg, or about 1-150 mg, or about 0.5-100 mg, or about 1-50 mg of active ingredients per day; the most preferred dose intravenously during a constant rate infusion is in the range of about 0.01 to about 10 mg/kg/min. The compounds of the present disclosure may be administered in a single daily dose, or the total daily dose may be administered in divided doses of 2, 3 or 4 times per day.

It is certain that administration regimens for the compounds of the present disclosure may vary depending on known factors, such as the pharmacodynamic characteristics of a specific drug and its mode and route of administration, the species, age, sex, health, medical condition and weight of the recipient, nature and extent of symptoms, types of coexisting treatments, frequency of treatments, routes of administration, renal and hepatic function of the patient, and desired effects. A therapeutically effective dose of the compound, a pharmaceutical composition or a combination thereof depends on the species, weight, age and individual condition of the subject, the disorder or disease being treated or its severity. A physician, clinician or veterinarian of ordinary skill may readily determine the effective amount of each active ingredient required to prevent, treat or inhibit the progression of a disorder or disease.

The present disclosure also provides use of the above compound or the pharmaceutically acceptable salt thereof or the above pharmaceutical composition in the manufacture of a medicament for the treatment of cancer.

Epidermal growth factor receptor (EGFR) is widely distributed on the surface of mammalian epithelial cells, fibroblasts, glial cells and other cells. The EGFR signaling pathway plays an important role in physiological processes such as cell growth, proliferation and differentiation. EGFR mutation is also the most common type of mutation in NSCLC patients, especially in Asian populations, which can account for 40 % to 50 %. Therefore, the compounds of the present disclosure can be used to treat cancers caused by high expression of EGFR. The above cancers comprise lymphoma, non-Hodgkin lymphoma, ovarian cancer, cervical cancer, prostate cancer, colorectal cancer, breast cancer, pancreatic cancer, glioma, glioblastoma, melanoma, leukemia, gastric cancer, endometrial cancer, lung cancer, hepatocellular carcinoma, gastric cancer, gastrointestinal stromal tumor (GIST), acute myeloid leukemia (AML), cholangiocarcinoma, renal carcinoma, thyroid carcinoma, anaplastic large cell lymphoma, mesothelioma, multiple myeloma and melanoma.

In some embodiments of the present disclosure, the above cancer is lung cancer.

The present disclosure also provides a method for treating cancer, comprising administering a therapeutically effective amount of the above compound or the pharmaceutically acceptable salt thereof or the above pharmaceutical composition to a patient. The above cancers comprise lymphoma, non-Hodgkin lymphoma, ovarian cancer, cervical cancer, prostate cancer, colorectal cancer, breast cancer, pancreatic cancer, glioma, glioblastoma, melanoma, leukemia, gastric cancer, endometrial cancer, lung cancer, hepatocellular carcinoma, gastric cancer, gastrointestinal stromal tumor (GIST), acute myeloid leukemia (AML), cholangiocarcinoma, renal carcinoma, thyroid carcinoma, anaplastic large cell lymphoma, mesothelioma, multiple myeloma and melanoma.

In some embodiments of the present disclosure, the above cancer is lung cancer.

The present disclosure also provides an intermediate compound represented by formula (V), and the intermediate compound, a stereoisomer thereof or a pharmaceutically acceptable salt thereof is used in the manufacture of the above compound, 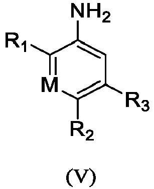 wherein R₁, R₂, R₃ and M are defined as above in formula (I).

In some embodiments of the present disclosure, the compound represented by formula (V) is selected from, 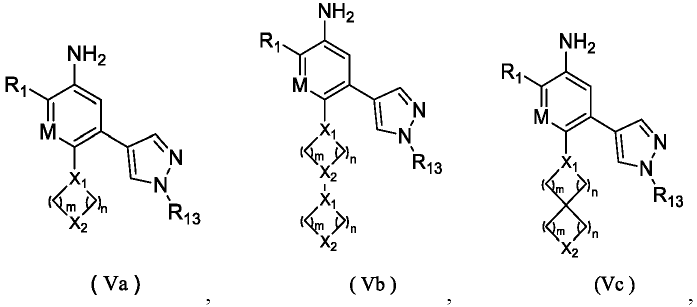 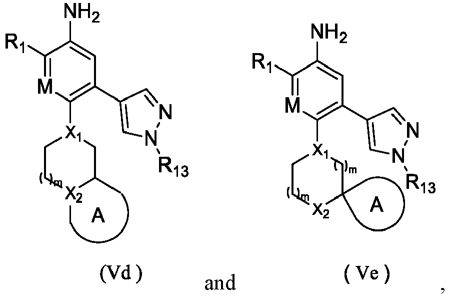 wherein, X₁, X₂, R₁, R₁₃, M, ring A, m and n are defined in the above formulas (IIIa)-(IIIe).

In some embodiments of the present disclosure, the compound represented by formula (V) is selected from, 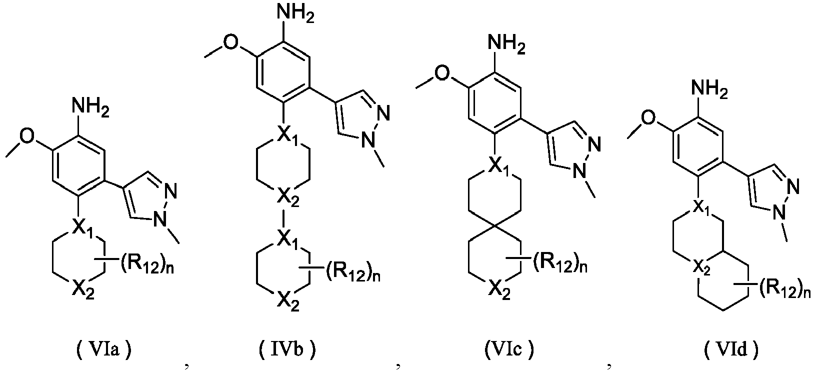 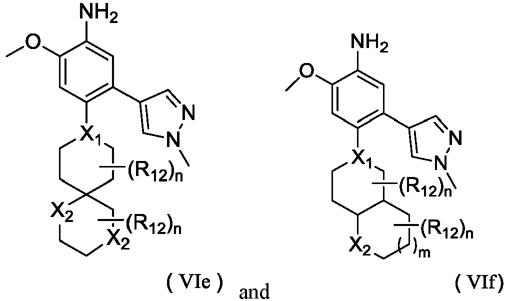 wherein X₁, X₂, R₁₂ and n are defined in formula (II).

In some embodiments of the present disclosure, the compound represented by formula (V) is selected from,

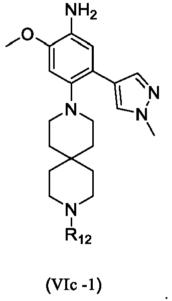

The present disclosure provides a compound represented by formula (I-A), a stereoisomer thereof, a tautomer thereof, a pharmaceutically acceptable salt thereof, a prodrug thereof, a hydrate thereof, a solvate thereof or an isotope-labeled derivative thereof, 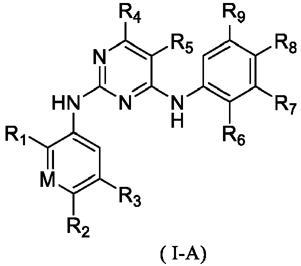 wherein,
R₁ is selected from H, halogen, -CN, C₁₋₆ alkyl, C₁₋₆ alkoxy, C₁₋₆ haloalkyl, C₁₋₆ haloalkoxy, C₃₋₆ cycloalkyl, 3-6-membered heterocycloalkyl, C₃₋₆ cycloalkyloxy, 3-6-membered heterocycloalkyloxy and C₂₋₆ alkenyloxy;
M is selected from N or CR₁₀;
R₁₀ and R₁ may form a 5-8-membered heterocycloalkyl, and the 5-8-membered heterocycloalkyl is optionally substituted by one or more R₁₁ groups;
R₂ is selected from H, halogen, -CN, -OH, -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, C₃₋₆ heterocycloalkenyl and phenyl; wherein, the -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, C₃₋₆ heterocycloalkenyl and phenyl are optionally substituted by one or more R₁₂ groups;
R₃ is selected from C₆₋₁₀ aryl, 5-12-membered heteroaryl, 3-8-membered heterocycloalkyl, C₃₋₈ cycloalkyl and C₃₋₆ cycloalkenyl, wherein the C₆₋₁₀ aryl, 5-12-membered heteroaryl, 3-8-membered heterocycloalkyl, C₃₋₈ cycloalkyl and C₃₋₆ cycloalkenyl are optionally substituted by one or more R₁₃ groups;
R₄ and R₅ are each independently selected from H, halogen, -CN, C₁₋₆ alkyl, C₁₋₆ alkoxy, C₁₋₆ haloalkyl, C₁₋₆ haloalkoxy, C₃₋₈ cycloalkyl and 3-8-membered heterocycloalkyl;
R₆ is selected from amino, amido, sulfonyl, phosphonyl, sulfonylamino and aminosulfonyl, wherein the amino, amido, sulfonyl, phosphonyl, sulfonylamino and aminosulfonyl are optionally substituted by one or more R₁₄ groups;
R₇, R₈ and R₉ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6-membered heterocycloalkyl and 5-7-membered heteroaryl;
or, R₇ and R₈ are cyclized to C₄₋₆ cycloalkyl, 4-6-membered heterocycloalkyl, C₅₋₆ aryl, or 5-7-membered heteroaryl;
or, R₈ and R₉ are cyclized to C₄₋₆ cycloalkyl, 4-6-membered heterocycloalkyl, C₅₋₆ aryl, or 5-7-membered heteroaryl;
R₁₀, R₁₁, R₁₂, R₁₃ and R₁₄ are each independently selected from H, halogen, -CN, -OH, -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₈ cycloalkyl, 3-8-membered heterocycloalkyl, hydroxy-C₁₋₆ alkyl, C₃₋₈ cycloalkylalkyl, C₃₋₈ cycloalkyl-C₁₋₆ alkoxy, C₁₋₆ alkylsulfonyl, C₃₋₆ cycloalkylsulfonyl, aminocarbonyl, C₃₋₈ cycloalkylcarbonyl, C₁₋₆ alkoxy-C₁₋₆ alkyl, C₃₋₆ cycloalkenyl, phenyl, -(CH₂)ₘNRₐR_{b} and -(CH₂)ₘO(CH₂)ₙCH₃; wherein Rₐ and R_{b} are H, C₁₋₆ alkyl or C₁₋₆ alkoxy, or Rₐ and R_{b} together form C₃₋₈ cycloalkyl or 3-8-membered heterocycloalkyl; wherein m and n are independently optionally 0, 1, 2 or 3, C₃₋₈ cycloalkyl and 3-8-membered heterocycloalkyl are optionally substituted by halogen, -OH, -NH₂, C₁₋₆ alkyl, C₁₋₆ alkoxy and C₁₋₆ haloalkyl.

In some embodiments of the present disclosure, the R₃ is selected from C₆₋₁₀ aryl, 5-12-membered heteroaryl and C₃₋₆ cycloalkenyl.

In some embodiments of the present disclosure, the R₁ is selected from H, halogen, -CN, C₁₋₆ alkyl, C₁₋₆ alkoxy, C₁₋₆ haloalkyl, C₁₋₆ haloalkoxy, C₃₋₆ cycloalkyl, 3-6-membered heterocycloalkyl, C₃₋₆ cycloalkyloxy, 3-6-membered heterocycloalkyloxy and C₂₋₆ alkenyloxy;
M is selected from N or CR₁₀;
R₁₀ and R₁ may form a 5-8-membered heterocycloalkyl, and the 5-8-membered heterocycloalkyl is optionally substituted by one or more R₁₁ groups;
R₂ is selected from H, halogen, -CN, -OH, -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, C₃₋₆ heterocycloalkenyl and phenyl; wherein, the -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, C₃₋₆ heterocycloalkenyl and phenyl are optionally substituted by one or more R₁₂ groups;
R₃ is selected from C₆₋₁₀ aryl, 5-12-membered heteroaryl and C₃₋₆ cycloalkenyl; wherein the C₆₋₁₀ aryl, 5-12-membered heteroaryl and C₃₋₆ cycloalkenyl are optionally substituted by one or more R₁₃ groups;
R₄ and R₅ are each independently selected from H, halogen, -CN, C₁₋₆ alkyl, C₁₋₆ alkoxy, C₁₋₆ haloalkyl, C₁₋₆ haloalkoxy, C₃₋₈ cycloalkyl and 3-8-membered heterocycloalkyl;
R₆ is selected from amino, amido, sulfonyl, phosphonyl, sulfonylamino and aminosulfonyl, wherein the amino, amido, sulfonyl, phosphonyl, sulfonylamino and aminosulfonyl are optionally substituted by one or more R₁₄ groups;
R₇, R₈ and R₉ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6-membered heterocycloalkyl and 5-7-membered heteroaryl;
or, R₇ and R₈ are cyclized to C₄₋₆ cycloalkyl, 4-6-membered heterocycloalkyl, C₅₋₆ aryl, or 5-7-membered heteroaryl;
or, R₈ and R₉ are cyclized to C₄₋₆ cycloalkyl, 4-6-membered heterocycloalkyl, C₅₋₆ aryl, or 5-7-membered heteroaryl;
R₁₀ and R₁₁ are each independently selected from H, halogen, C₁₋₆ alkyl, C₁₋₆ alkoxy, C₁₋₆ haloalkyl and C₁₋₆ haloalkoxy;
R₁₂ is selected from H, halogen, -CN, -OH, -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₈ cycloalkyl, 3-8-membered heterocycloalkyl, hydroxy-C₁₋₆ alkyl, C₃₋₈ cycloalkylalkyl, C₃₋₈ cycloalkyl-C₁₋₆ alkoxy, C₁₋₆ alkylsulfonyl, C₃₋₆ cycloalkylsulfonyl, aminocarbonyl, C₃₋₈ cycloalkylcarbonyl, C₁₋₆ alkoxy-C₁₋₆ alkyl, C₃₋₆ cycloalkenyl, phenyl, -(CH₂)ₘNRₐR_{b} and - (CH₂)ₘO(CH₂)ₙCH₃; wherein Rₐ and R_{b} are H, C₁₋₆ alkyl or C₁₋₆ alkoxy, or Rₐ and R_{b} together form C₃₋₈ cycloalkyl or 3-8-membered heterocycloalkyl; wherein m and n are independently optionally 0, 1, 2 or 3, C₃₋₈ cycloalkyl and 3-8-membered heterocycloalkyl are optionally substituted by halogen, -OH, -NH₂, C₁₋₆ alkyl, C₁₋₆ alkoxy and C₁₋₆ haloalkyl;
R₁₃ is selected from H, halogen, -CN, C₁₋₆ alkyl, C₁₋₆ alkoxy, C₁₋₆ haloalkyl, hydroxy-C₁₋₆ alkyl, C₁₋₆ haloalkoxy, C₃₋₈ cycloalkyl and 3-8-membered heterocycloalkyl;
R₁₄ is selected from H, C₁₋₆ alkyl, C₁₋₆ alkoxy, C₁₋₆ haloalkyl, C₁₋₆ haloalkoxy, C₃₋₈ cycloalkyl and 3-8-membered heterocycloalkyl.

In some embodiments of the present disclosure, the R₁ is selected from H, halogen, -CN, C₁₋₃ alkyl, C₁₋₃ alkoxy, C₁₋₃ haloalkyl and C₁₋₃ haloalkoxy.

In some embodiments of the present disclosure, the R₁ is selected from H, methyl, ethyl, n-propyl, isopropyl, n-butyl, methoxy, ethoxy, n-propoxy, isopropoxy, n-butoxy, trifluoromethyl, trifluoromethoxy, trichloromethyl, trichloromethoxy and 2,2,2-trifluoroethoxy.

In some embodiments of the present disclosure, the R₂ is selected from H, halogen, -NH₂, C₃₋₇ cycloalkyl, 3-7-membered heterocycloalkyl, C₆₋₁₄ spiro cyclyl, C₆₋₁₄ fused cyclyl, C₆₋₁₄ bridged cyclyl, 6-14-membered spiro heterocyclyl, 6-14-membered bridged heterocyclyl and 6-14-membered fused heterocyclyl; wherein, the -NH₂, C₃₋₇ cycloalkyl, 3-7-membered heterocycloalkyl, C₆₋₁₄ spiro cyclyl, C₆₋₁₄ fused cyclyl, C₆₋₁₄ bridged cyclyl, 6-14-membered spiro heterocyclyl, 6-14-membered bridged heterocyclyl and 6-14-membered fused heterocyclyl are optionally substituted by one or more R₁₂ groups, and the R₁₂ group is defined as above.

In some embodiments of the present disclosure, the R₂ is selected from H, halogen, -CN, -OH, - NH₂, -NHR₁₂, -NR₁₂R₁₂, 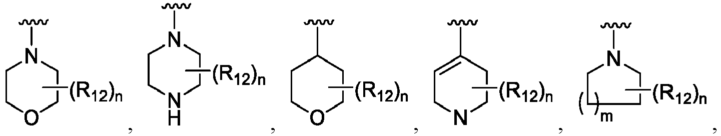 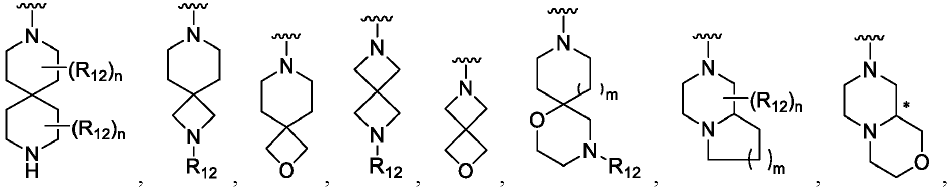 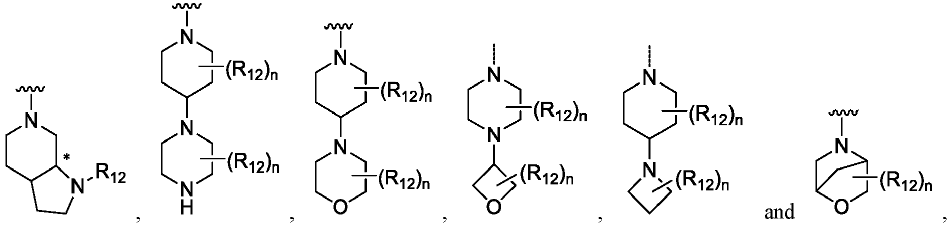 wherein the R₁₂, m and n are defined as above.

In some embodiments of the present disclosure, the R₂ is selected from H, fluorine, chlorine, bromine, iodine, -NH₂,

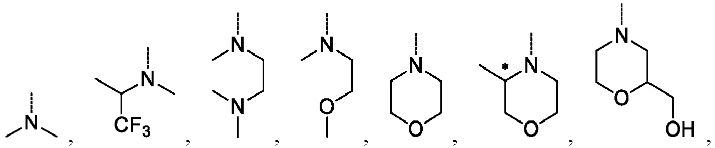

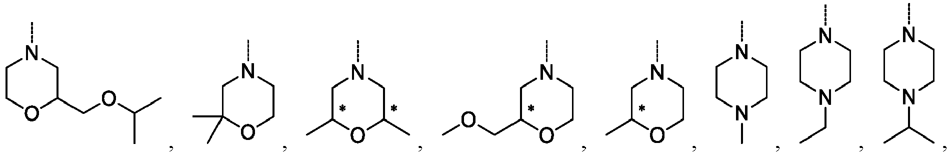

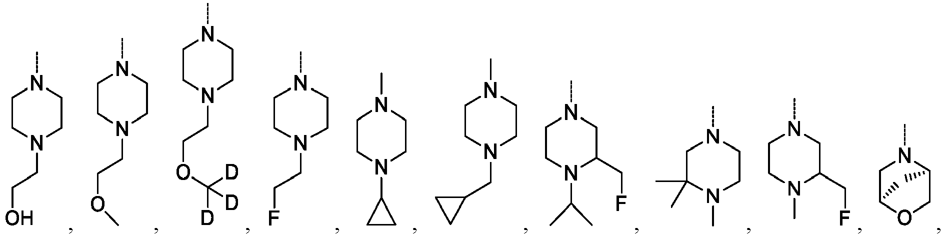

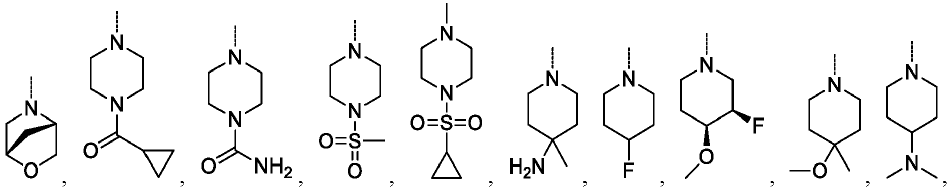

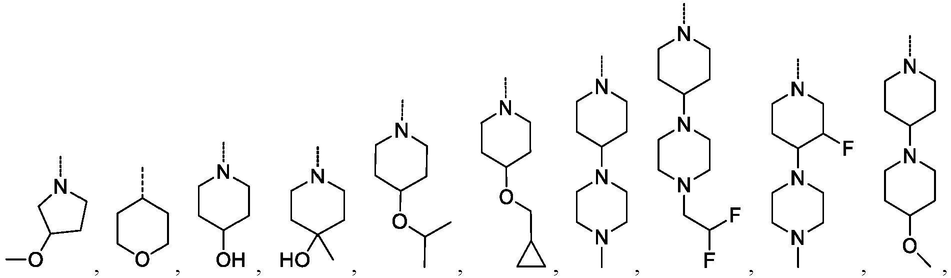

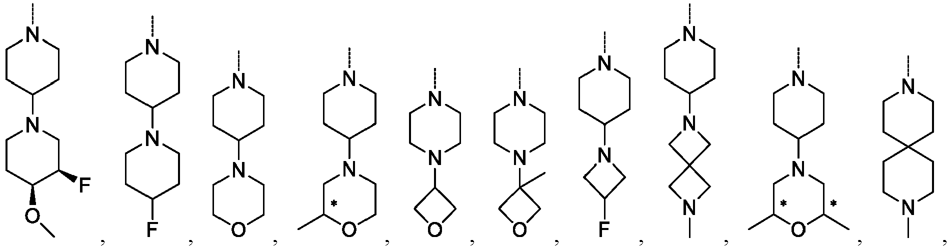

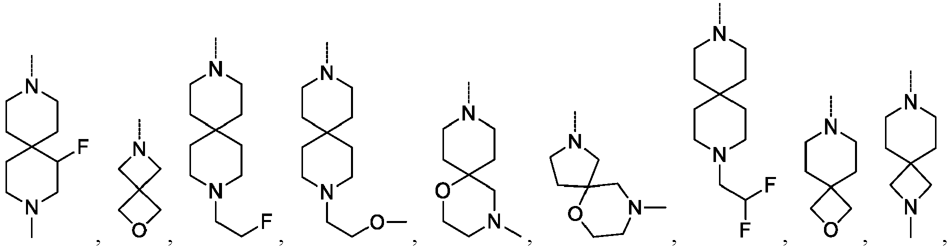

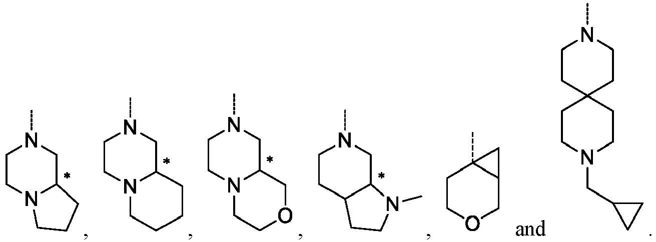

In some embodiments of the present disclosure, the R₂ is selected from

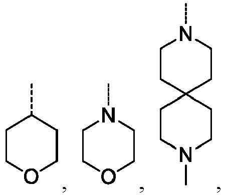

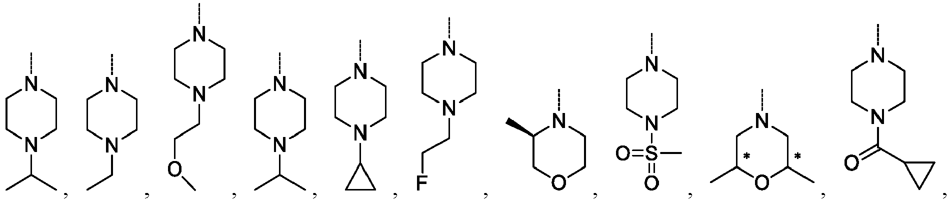

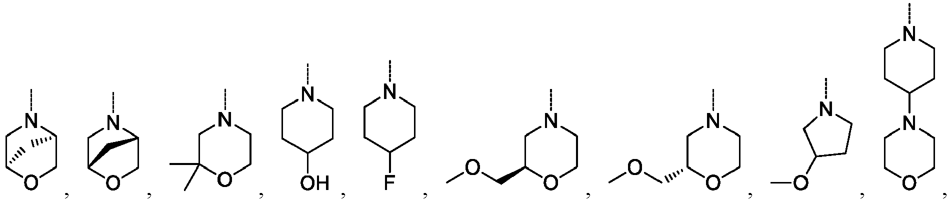

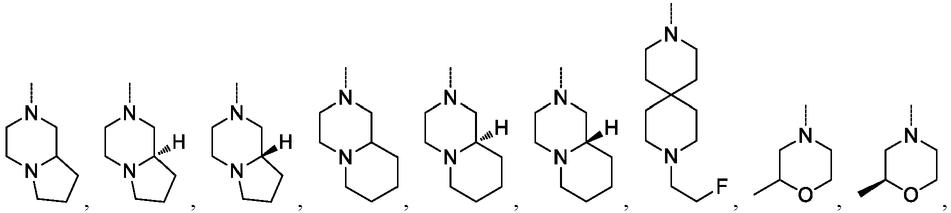

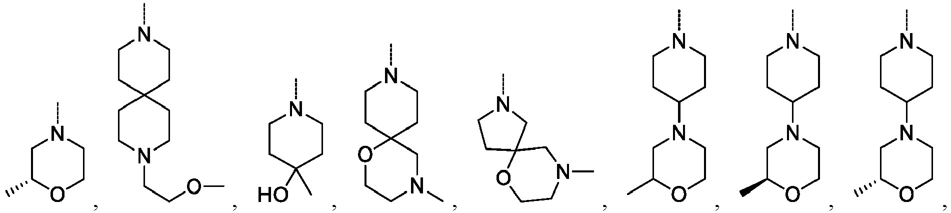

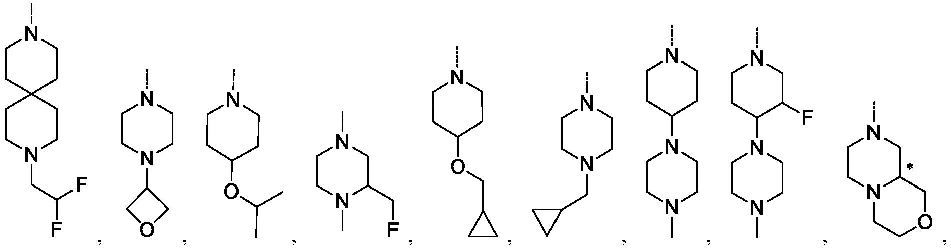

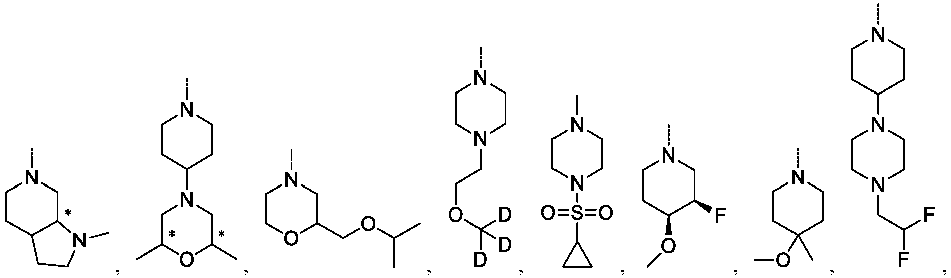

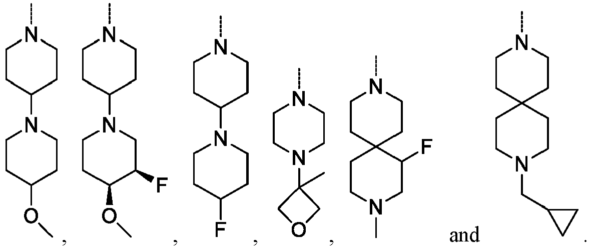

In some embodiments of the present disclosure, the R₃ is selected from phenyl, pyranyl, pyridyl, pyrimidinyl, pyridazinyl, pyrazinyl, furanyl, thienyl, pyrrolyl, pyrazolyl, thiazolyl, imidazolyl, benzofuranyl, benzimidazolyl, benzothienyl, benzoxazolyl, benzothiazolyl, indolyl, pyrazolo[1,5-α]pyridyl, quinolinyl, isoquinolinyl, tetrahydropyrrolyl, piperidinyl, piperazinyl, morpholinyl, tetrahydrofuranyl, tetrahydropyranyl, cyclobutyl, cyclopentyl and cyclohexyl, and the phenyl, pyranyl, pyridyl, pyrimidinyl, pyridazinyl, pyrazinyl, furanyl, thienyl, pyrrolyl, pyrazolyl, thiazolyl, imidazolyl, benzofuranyl, benzimidazolyl, benzothienyl, benzoxazolyl, benzothiazolyl, indolyl, pyrazolo[1,5-α]pyridyl, quinolinyl, isoquinolinyl, tetrahydropyrrolyl, piperidinyl, piperazinyl, morpholinyl, tetrahydrofuranyl, tetrahydropyranyl, cyclobutyl, cyclopentyl and cyclohexyl are optionally substituted by one or more R₁₃ groups, wherein the R₁₃ group is defined as above.

In some embodiments of the present disclosure, the R₃ is selected from 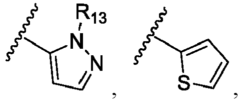 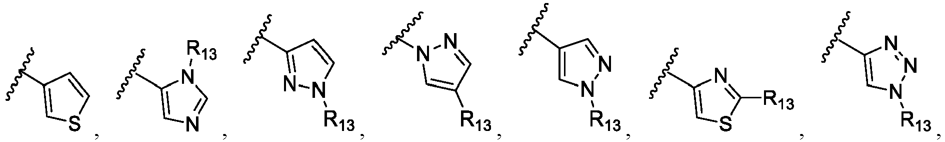 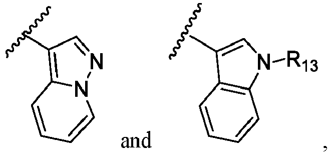 wherein the R₁₃ is defined as above.

In some embodiments of the present disclosure, the R₃ is selected from 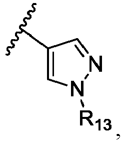 wherein the R₁₃ is defined as above.

In some embodiments of the present disclosure, the R₄ and R₅ are each independently selected from H, F, Cl, Br, CN, methyl, ethyl, isopropyl, methoxy, ethoxy, isopropoxy, trifluoromethyl, 2,2,2-trifluoroethyl and cyclopropyl.

In some embodiments of the present disclosure, the R₆ is selected from

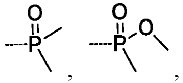

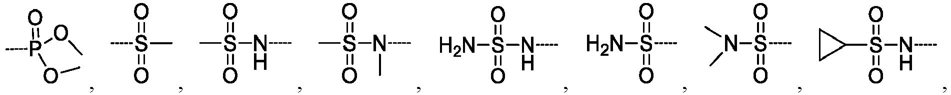

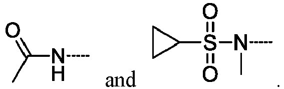

In some embodiments of the present disclosure, the R₆ is selected from

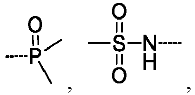

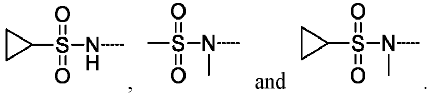

In some embodiments of the present disclosure, R₇ and R₈ are cyclized to C₄₋₆ cycloalkyl, 4-6-membered heterocycloalkyl, C₅₋₆ aryl or 5-7-membered heteroaryl, or R₈ and R₉ are cyclized to C₄₋₆ cycloalkyl, 4-6-membered heterocycloalkyl, C₅₋₆ aryl or 5-7-membered heteroaryl.

In some embodiments of the present disclosure, R₇ and R₈ or R₈ and R₉ are independently cyclized to cyclobutane, cyclopentane, tetrahydropyrrole ring, tetrahydrofuran ring, tetrahydropyran ring, thiophene ring, imidazole ring, pyrazole ring, pyrrole ring, oxazole ring, thiazole ring, isoxazole ring, piperazine ring, isothiazole ring, benzene ring, pyridine ring, piperidine ring, pyrimidine ring, pyridazine ring or pyrazine ring.

In some embodiments of the present disclosure, R₇ and R₈ or R₈ and R₉ are independently cyclized to cyclobutane, pyridine ring or pyrazine ring.

In some embodiments of the present disclosure, the above compound, the stereoisomer thereof, the tautomer thereof, the pharmaceutically acceptable salt thereof, the prodrug thereof, the hydrate thereof, the solvate thereof or the isotope-labeled derivative thereof is selected from, 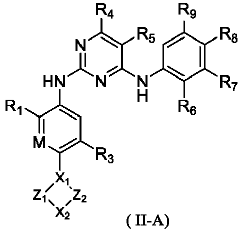 wherein, X₁ is independently selected from CR_{c} and N;
X₂ is independently selected from -CR_{c}R_{d}-, -NR_{c}- and -O-;
Z₁ and Z₂ are each independently selected from -(CRₑR_{f})ₘ(CRₑR_{f})ₙ-;
the R_{c}, R_{d}, Rₑ, and R_{f} are each independently selected from H, halogen, -CN, -OH, -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, phenyl, -(CH₂)ₘNRₐR_{b} and -(CH₂)ₘO(CH₂)ₙCH₃; wherein the Rₐ, R_{b}, m and n are defined as above;
or, R_{c} and R_{d} together form C₃₋₈ cycloalkyl or 3-8-membered heterocycloalkyl;
or, Rₑ and R_{f} together form C₃₋₈ cycloalkyl or 3-8-membered heterocycloalkyl;
or, R_{c} and Rₑ, or R_{c} and R_{f}, or R_{d} and Rₑ, or R_{d} and R_{f} together form C₃₋₈ cycloalkyl or 3-8-membered heterocycloalkyl;
wherein, a ring formed by X₁, X₂, Z₁ and Z₂ and a ring further formed by substituent groups R_{c}, R_{d}, Rₑ and R_{f} thereof are optionally substituted by one or more R₁₂ groups;
R₁, R₃, R₄, R₅, R₆, R₇, R₈, R₉, R₁₂ and M are defined as above.

In some embodiments of the present disclosure, the compound, the stereoisomer thereof, the tautomer thereof, the pharmaceutically acceptable salt thereof, the prodrug thereof, the hydrate thereof, the solvate thereof or the isotope-labeled derivative thereof is selected from, 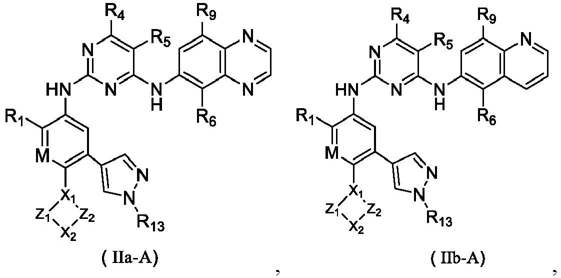 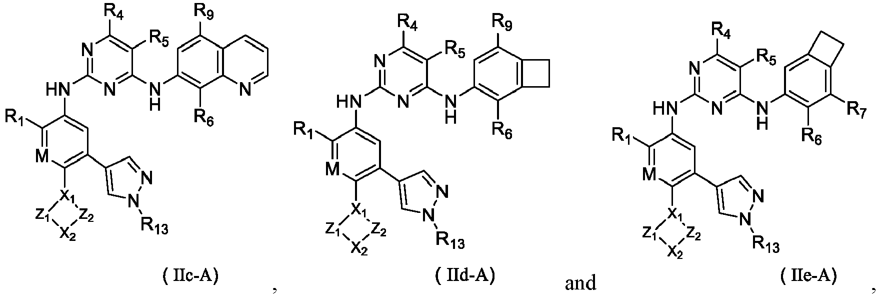
wherein, the ring formed by X₁, X₂, Z₁ and Z₂ and the ring further formed by substituent groups thereof are optionally substituted by one or more R₁₂ groups;
X₁, X₂, Z₁, Z₂, R₁, R₄, R₅, R₆, R₇, R₉, R₁₂, R₁₃ and M are defined as above.

In some embodiments of the present disclosure, the compound, the stereoisomer thereof, the tautomer thereof, the pharmaceutically acceptable salt thereof, the prodrug thereof, the hydrate thereof, the solvate thereof or the isotope-labeled derivative thereof is selected from, 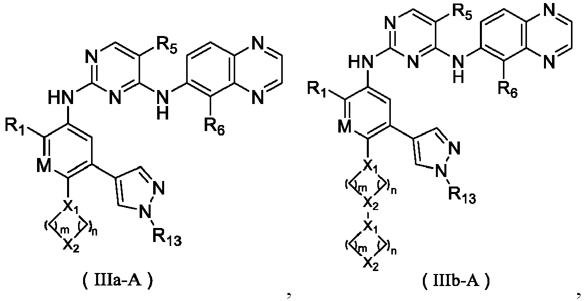 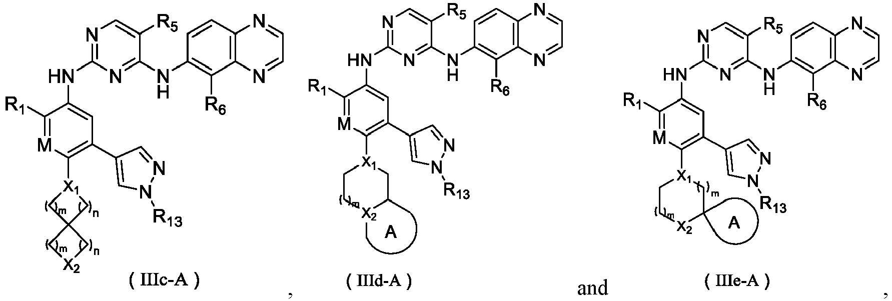 wherein, ring A is selected from C₅₋₇ cycloalkyl and 5-7-membered heterocycloalkyl;
a monocyclic ring, a bicyclic ring, a spiro ring, a fused ring formed by X₁ and X₂ and ring A are optionally substituted by one or more R₁₂ groups;
X₁, X₂, R₁, R₅, R₆, m, n, M, R₁₂ and R₁₃ are defined as above.

In some embodiments of the present disclosure, the compound, the stereoisomer thereof, the tautomer thereof, the pharmaceutically acceptable salt thereof, the prodrug thereof, the hydrate thereof, the solvate thereof or the isotope-labeled derivative thereof is selected from, 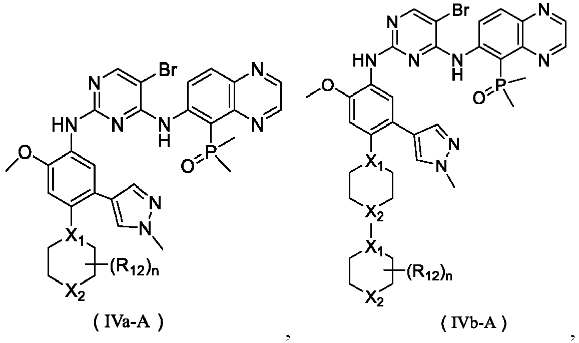 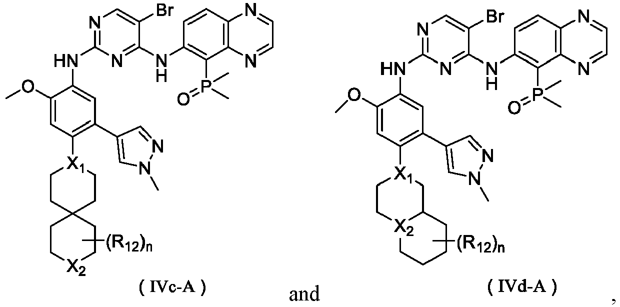 wherein X₁, X₂, R₁₂ and n are defined as above.

The present disclosure further provides a compound represented by the following formula, a stereoisomer thereof, a tautomer thereof, a pharmaceutically acceptable salt thereof, a prodrug thereof, a hydrate thereof, a solvate thereof or an isotope-labeled derivative thereof, which is selected from,

| Compound | Structural formula |
|---|---|
| **68** | 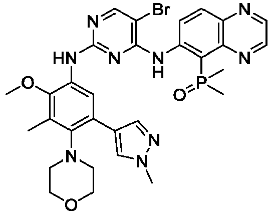 |
| **69** | 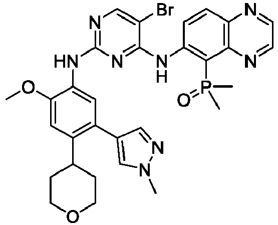 |
| **70** | 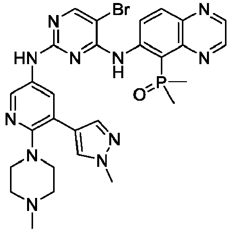 |
| **71** | 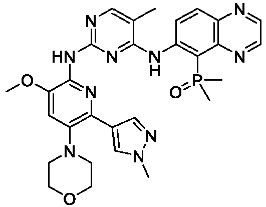 |
| **72** | 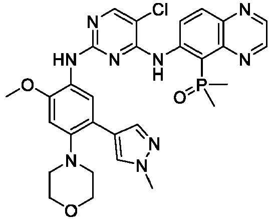 |
| **73** | 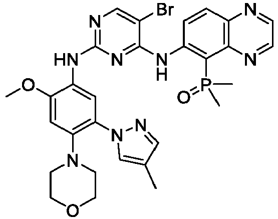 |
| **74** | 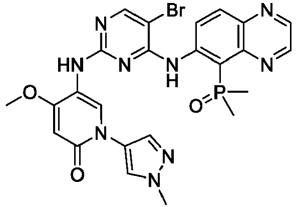 |
| **75** | 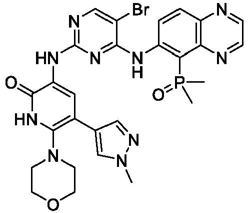 |
| **76** | 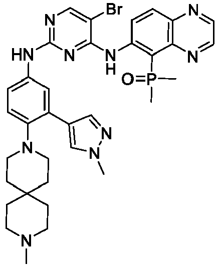 |
| **77** | 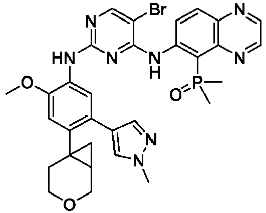 |
| **78** | 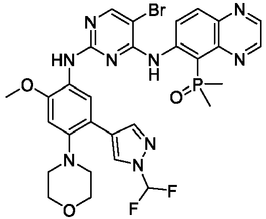 |
| **79** | 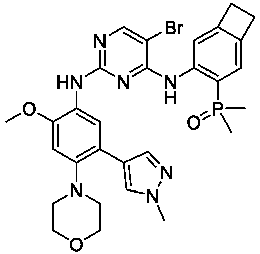 |
| **80** | 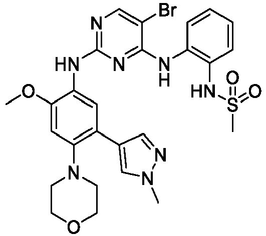 |
| **81** | 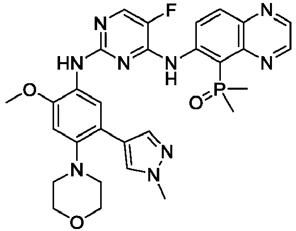 |
| **82** | 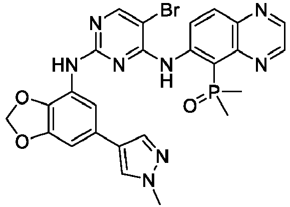 |
| **83** | 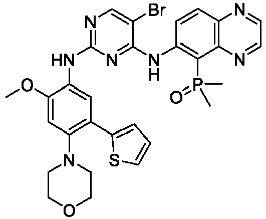 |
| **84** | 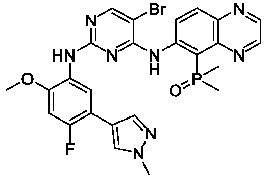 |
| **85** | 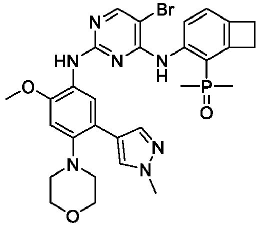 |
| **86** | 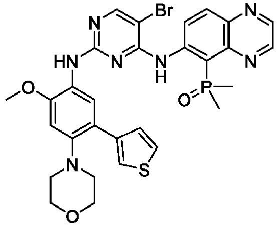 |
| **87** | 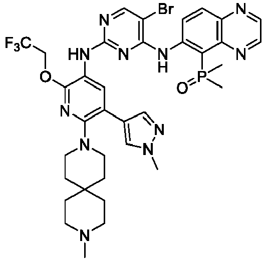 |
| **88** | 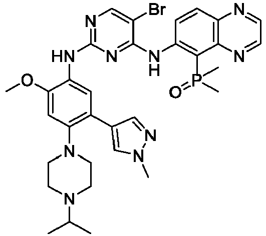 |
| **89** | 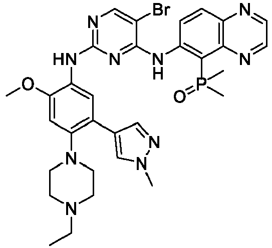 |
| **90** | 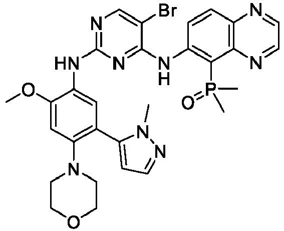 |
| **91** | 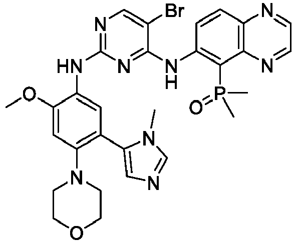 |
| **92** | 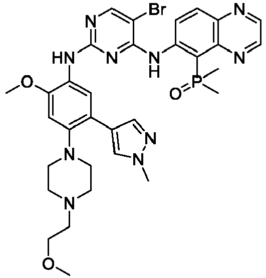 |
| **93** | 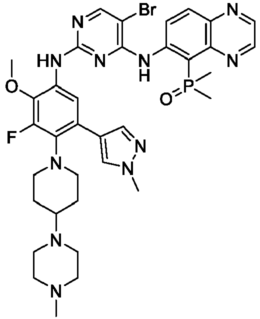 |
| **94** | 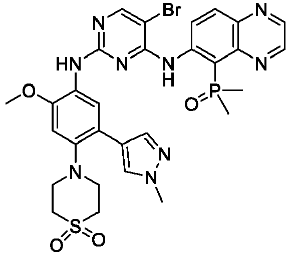 |
| **95** | 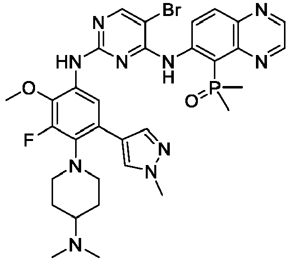 |
| **96** | 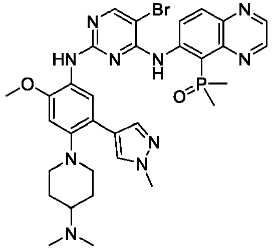 |
| **97** | 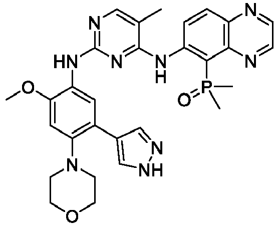 |
| **98** | 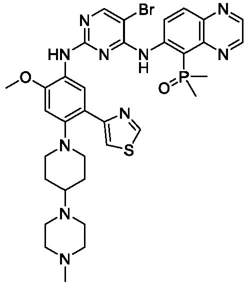 |
| **99** | 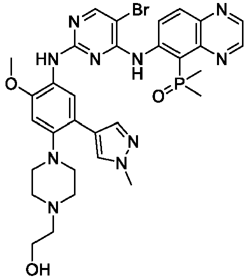 |
| **100** | 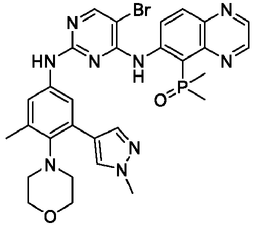 |
| **101** | 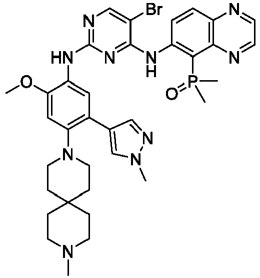 |
| **102** | 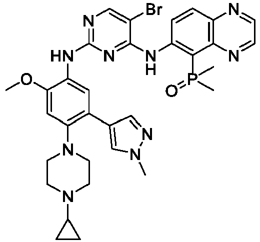 |
| **103** | 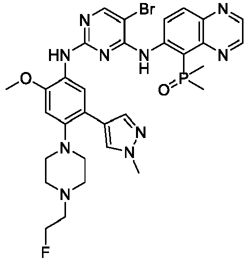 |
| **104** | 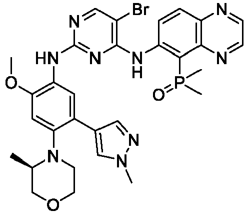 |
| **105** | 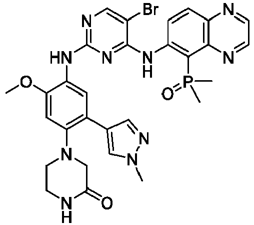 |
| **106** | 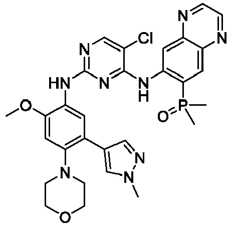 |
| **107** | 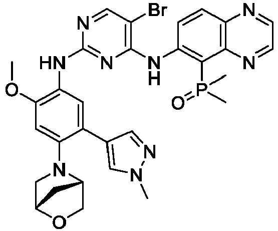 |
| **108** | 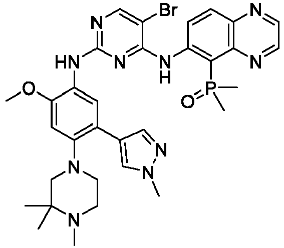 |
| **109** | 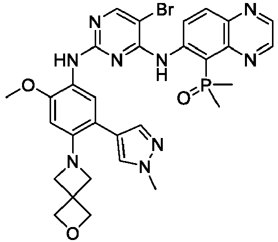 |
| **110** | 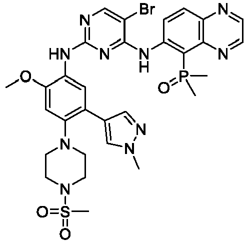 |
| **111** | 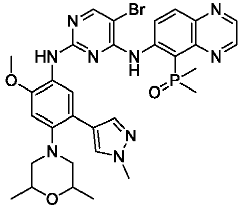 |
| | ***(2S,6R and 2R,6S)*** |
| **112** | 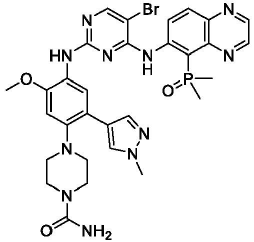 |
| **113** | 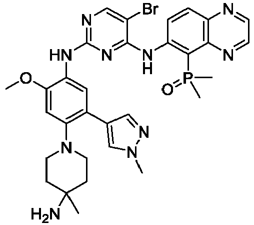 |
| **114** | 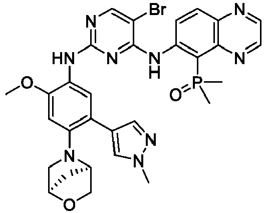 |
| **115** | 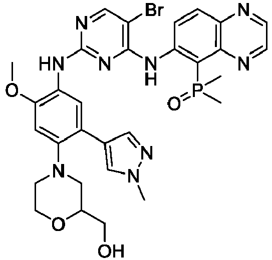 |
| **116** | 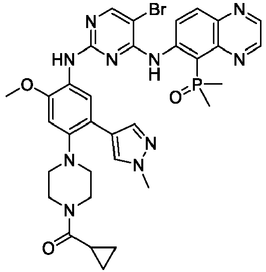 |
| **117** | 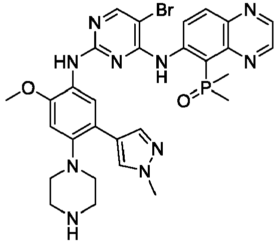 |
| **118** | 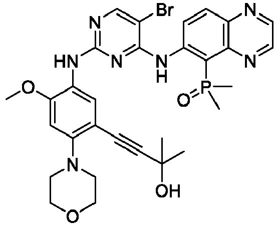 |
| **119** | 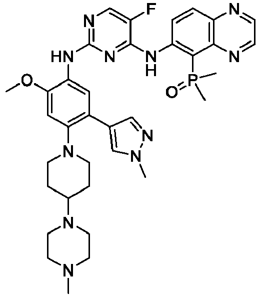 |
| **120** | 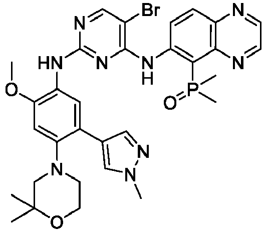 |
| **121** | 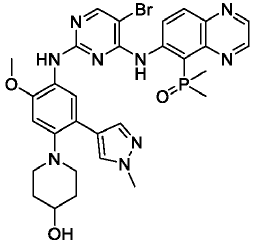 |
| **122** | 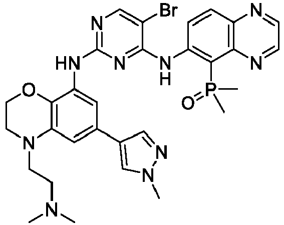 |
| **123** | 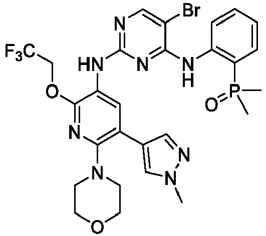 |
| **124** | 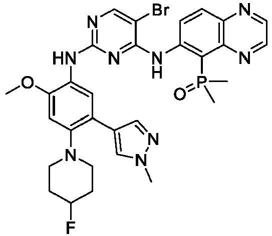 |
| **125** | 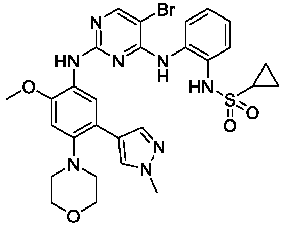 |
| **126** | 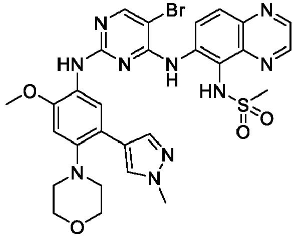 |
| **127** | 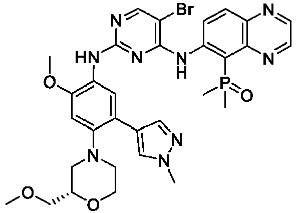 |
| **128** | 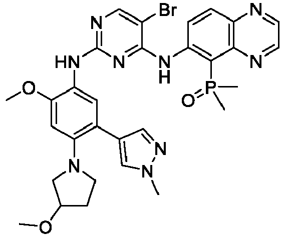 |
| **129** | 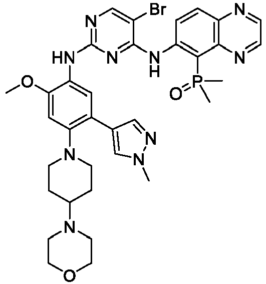 |
| **130** | 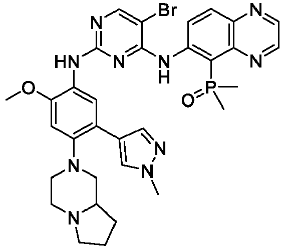 |
| **131** | 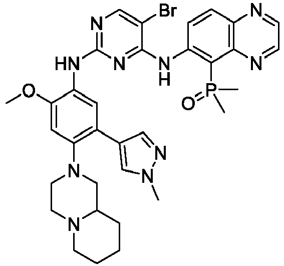 |
| **132** | 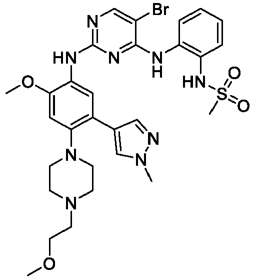 |
| **133** | 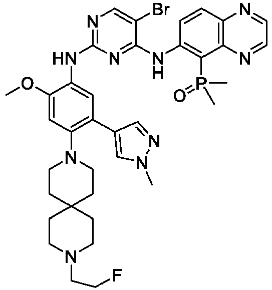 |
| **134** | 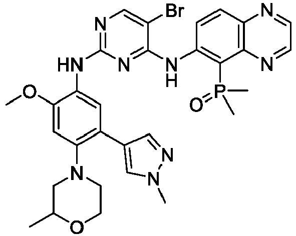 |
| **135** | 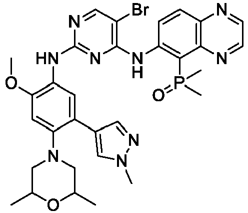 |
| | **(2S,8S *and 2R,6R)*** |
| **136** | 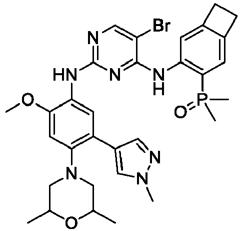 |
| | **(2S,6Rand *2R,6S)*** |
| **137** | 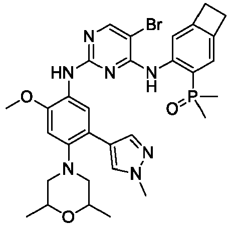 |
| | **(2S,6S *and 2R,6R)*** |
| **138** | 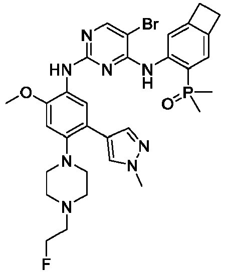 |
| **139** | 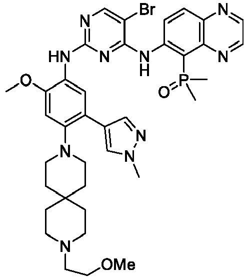 |
| **140** | 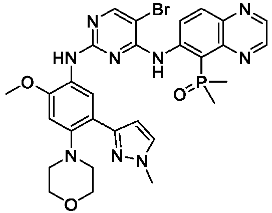 |
| **141** | 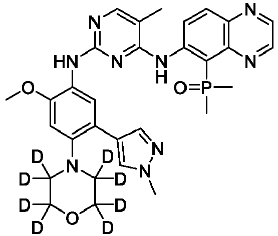 |
| **142** | 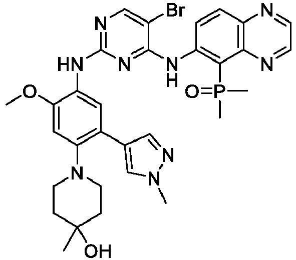 |
| **143** | 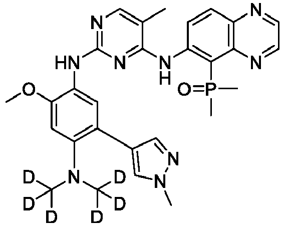 |
| **144** | 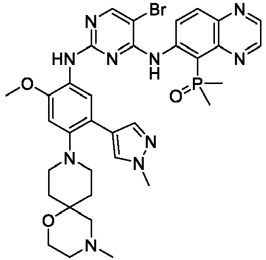 |
| **145** | 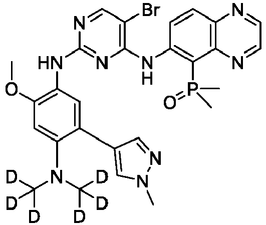 |
| **146** | 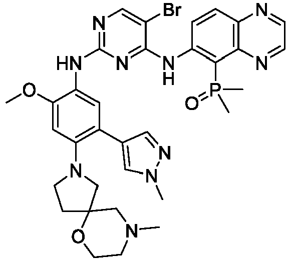 |
| **147** | 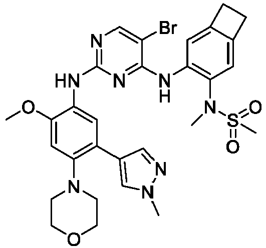 |
| **148** | 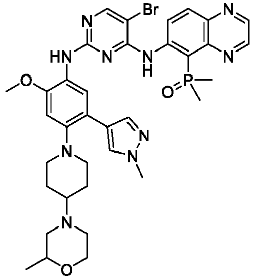 |
| **149** | 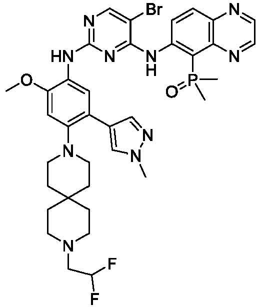 |
| **150** | 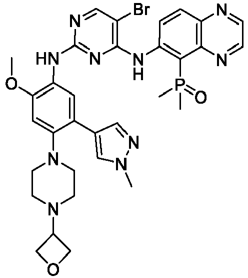 |
| **151** | 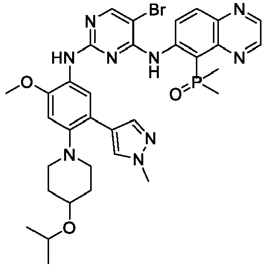 |
| **152** | 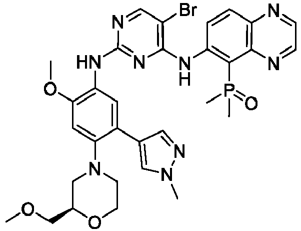 |
| **153** | 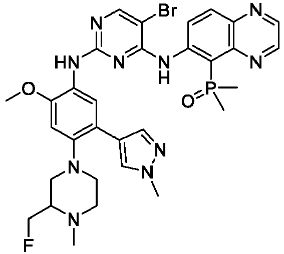 |
| **154** | 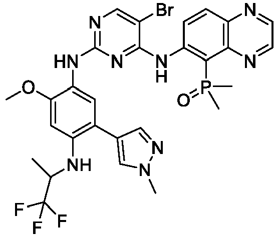 |
| **155a** | 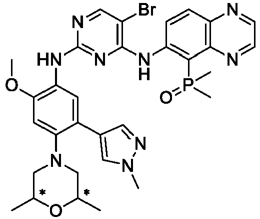 |
| | *(2S,6S or 2R,6R)* |
| **155b** | 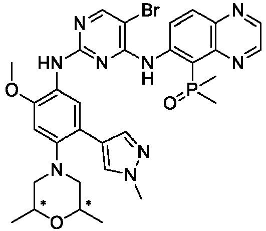 |
| | Enantiomer of 155a *(* 2*R,6R or 2S,6S)* |
| **156** | 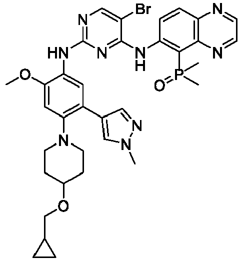 |
| **157** | 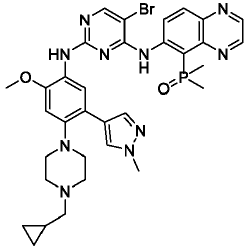 |
| **158** | 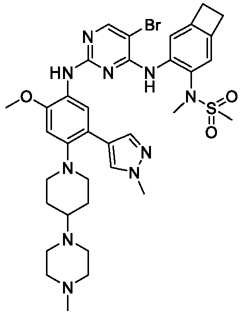 |
| **159** | 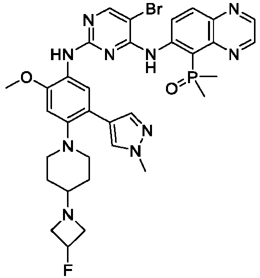 |
| **160** | 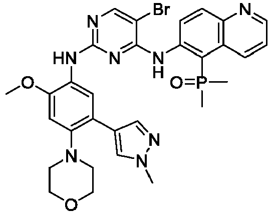 |
| **161** | 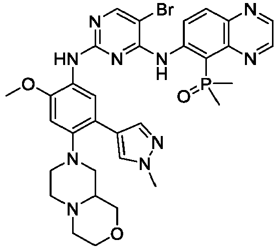 |
| **162** | 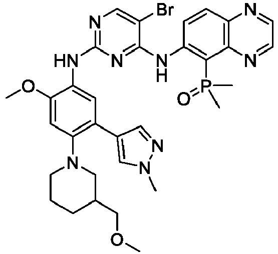 |
| **163** | 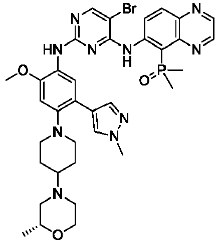 |
| **164** | 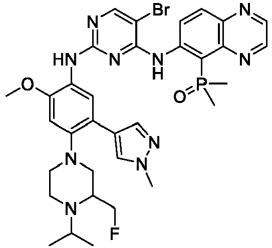 |
| **165** | 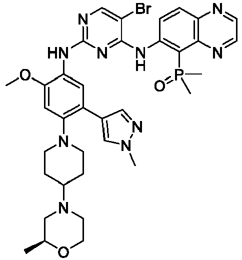 |
| **166** | 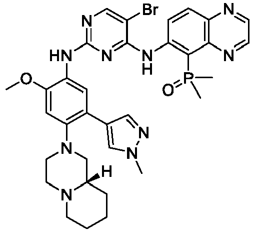 |
| **167** | 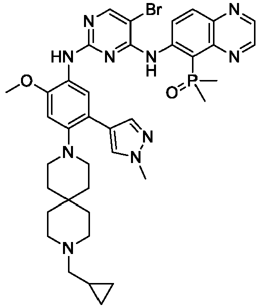 |
| **168** | 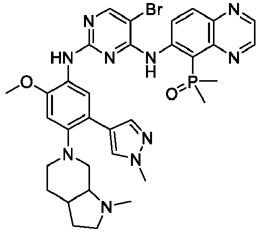 |
| **169** | 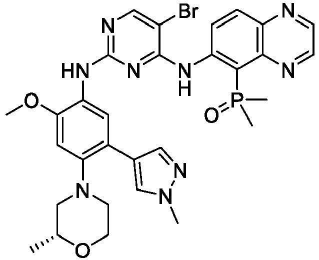 |
| **170** | 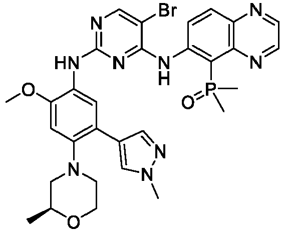 |
| **171** | 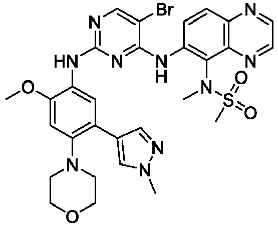 |
| **172** | 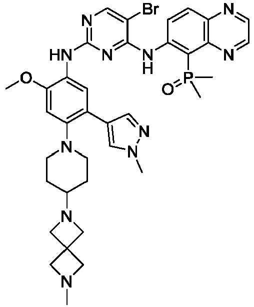 |
| **173** | 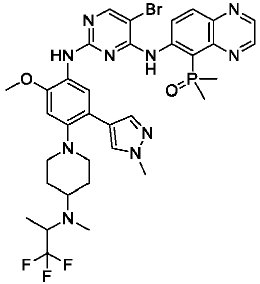 |
| **174** | 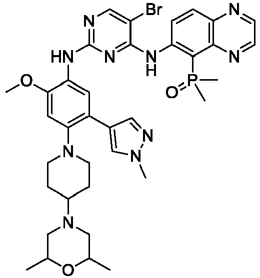 |
| | **(2S,6S *and 2R,6R)*** |
| **175** | 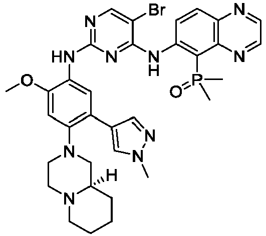 |
| **176** | 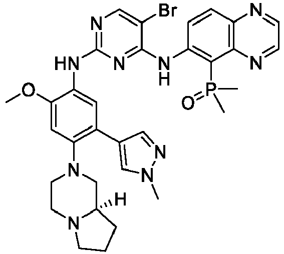 |
| **177** | 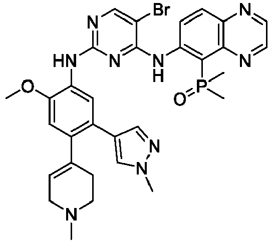 |
| **178** | 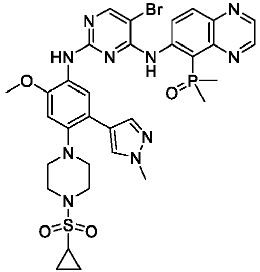 |
| **179** | 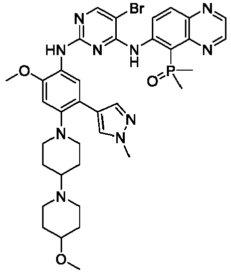 |
| **180** | 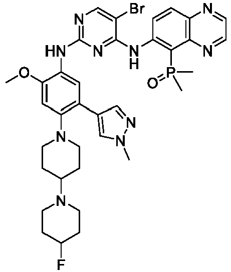 |
| **181** | 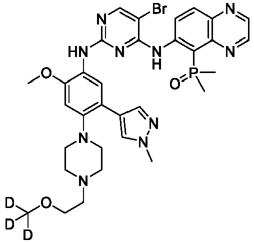 |
| **182** | 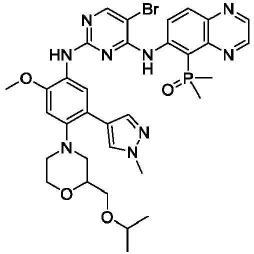 |
| **183** | 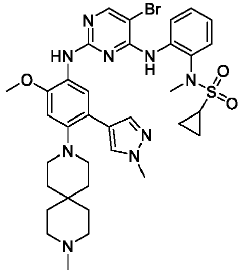 |
| **184** | 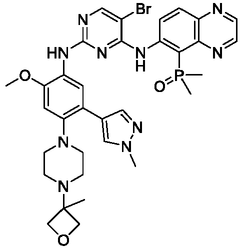 |
| **185** | 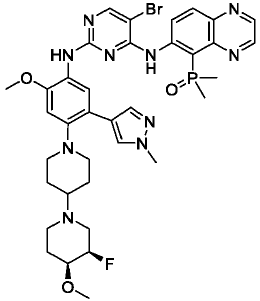 |
| **186** | 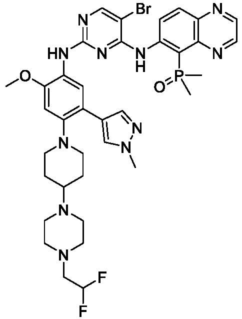 |
| **187** | 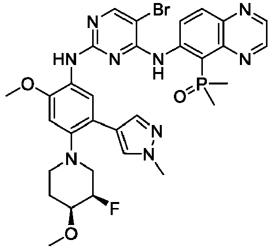 |
| **188** | 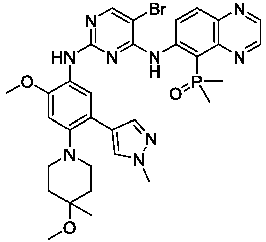 |
| **189** | 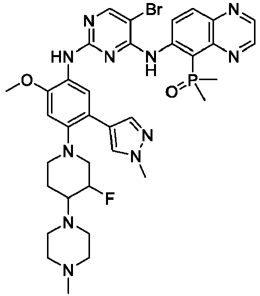 |
| **190** | 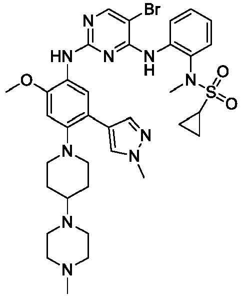 |

The present disclosure also provides a pharmaceutical composition comprising a therapeutically effective amount of the above compound or the pharmaceutically acceptable salt thereof and a pharmaceutically acceptable carrier, diluent and excipient. The pharmaceutical composition may be formulated for specific routes of administration, such as oral administration, parenteral administration, rectal administration, and the like. Oral administration, for example, a tablet, a capsule (including a sustained release or timed release prescription), a pill, a powder, a granule, an elixir, a tincture, a suspension (including a nano-suspension, a micro-suspension, a spray-dried dispersant), a syrup and an emulsion; sublingual administration; buccal administration; parenteral administration, for example, by subcutaneous, intravenous, intramuscular, or intrasternal injection, or infusion techniques (for example, as a sterile injectable aqueous solution or non-aqueous solution or a mixed suspension); nasal administration, including administration to the nasal mucosa, for example, by inhalation spray; topical administration, for example, in the form of a cream or an ointment; or rectal administration, for example, in the form of a suppository. They can be administered alone, but they are usually administered together with a pharmaceutical carrier selected according to the selected route of administration and standard pharmaceutical practice.

"Pharmaceutically acceptable carrier" refers to a medium generally acceptable in the art for delivering a biologically active agent to animals, particularly mammals, including, for example, an adjuvant, an excipient or a vehicle, such as a diluent, a preservative, a filler, a flow regulator, a disintegrant, a wetting agent, an emulsifier, a suspending agent, a sweetener, a flavor, a fragrance, an antibacterial agent, an antifungal agent, a lubricants agent and a dispersant, according to the mode of administration and the nature of dosage forms. The pharmaceutically acceptable carrier is formulated according to a large number of factors within the purview of those of ordinary skill in the art, including, but not limited to, the type and nature of the active agent formulated, the subjects to which the composition containing the agent is to be administered, the expected route of administration of the composition, and the target therapeutic indication. The pharmaceutically acceptable carriers include both aqueous and non-aqueous media and various solid and semisolid dosage forms. In addition to the active agent, such carriers include many different ingredients and additives, and such additional ingredients included in prescriptions for a variety of reasons (e.g., stabilizing the active agent and an adhesive, etc.) are well known to those of ordinary skill in the art.

As a general guideline, when used for the indicated effects, the daily oral dose of various active ingredients is in the range of about 0.001- about 5000 mg, or about 1-500 mg, or about 1-250 mg, or about 1-150 mg, or about 0.5-100 mg, or about 1-50 mg of active ingredients per day; the most preferred dose intravenously during a constant rate infusion is in the range of about 0.01 to about 10 mg/kg/min. The compounds of the present disclosure may be administered in a single daily dose, or the total daily dose may be administered in divided doses of 2, 3 or 4 times per day.

It is certain that administration regimens for the compounds of the present disclosure may vary depending on known factors, such as the pharmacodynamic characteristics of a specific drug and its mode and route of administration, the species, age, sex, health, medical condition and weight of the recipient, nature and extent of symptoms, types of coexisting treatments, frequency of treatments, routes of administration, renal and hepatic function of the patient, and desired effects. A therapeutically effective dose of the compound, a pharmaceutical composition or a combination thereof depends on the species, weight, age and individual condition of the subject, the disorder or disease being treated or its severity. A physician, clinician or veterinarian of ordinary skill may readily determine the effective amount of each active ingredient required to prevent, treat or inhibit the progression of a disorder or disease.

The present disclosure also provides use of the above compound or the pharmaceutically acceptable salt thereof or the above pharmaceutical composition in the manufacture of a medicament for the treatment of cancer.

Epidermal growth factor receptor (EGFR) is widely distributed on the surface of mammalian epithelial cells, fibroblasts, glial cells and other cells. The EGFR signaling pathway plays an important role in physiological processes such as cell growth, proliferation and differentiation. EGFR mutation is also the most common type of mutation in NSCLC patients, especially in Asian populations, which can account for 40 % to 50 %. Therefore, the compounds of the present disclosure can be used to treat cancers caused by high expression of EGFR. The above cancers comprise lymphoma, non-Hodgkin lymphoma, ovarian cancer, cervical cancer, prostate cancer, colorectal cancer, breast cancer, pancreatic cancer, glioma, glioblastoma, melanoma, leukemia, gastric cancer, endometrial cancer, lung cancer, hepatocellular carcinoma, gastric cancer, gastrointestinal stromal tumor (GIST), acute myeloid leukemia (AML), cholangiocarcinoma, renal carcinoma, thyroid carcinoma, anaplastic large cell lymphoma, mesothelioma, multiple myeloma and melanoma.

In some embodiments of the present disclosure, the above cancer is lung cancer.

The present disclosure also provides an intermediate compound represented by formula (V), and the intermediate compound, a stereoisomer thereof or a pharmaceutically acceptable salt thereof is used in the manufacture of the above compound. 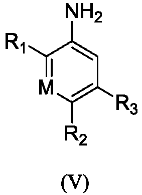 wherein, R₁, R₂, R₃ and M are defined in formula (I).

In some embodiments of the present disclosure, the compound represented by formula (V) is selected from, 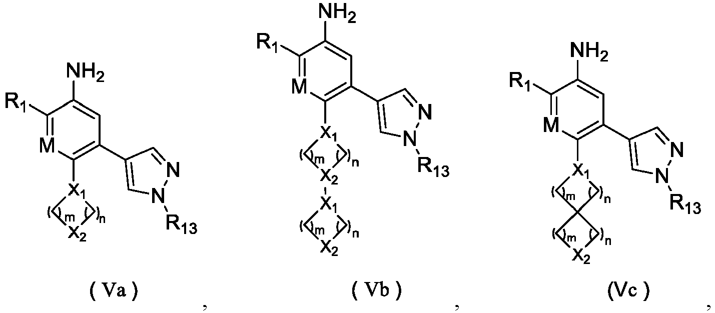 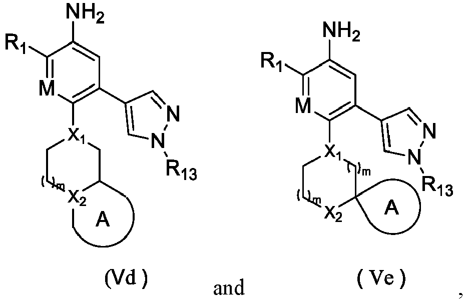 wherein, X₁, X₂, R₁, R₁₃, M, ring A, m and n are defined in the above formulas (IIIa)-(IIIe).

In some embodiments of the present disclosure, the compound represented by formula (V) is selected from, 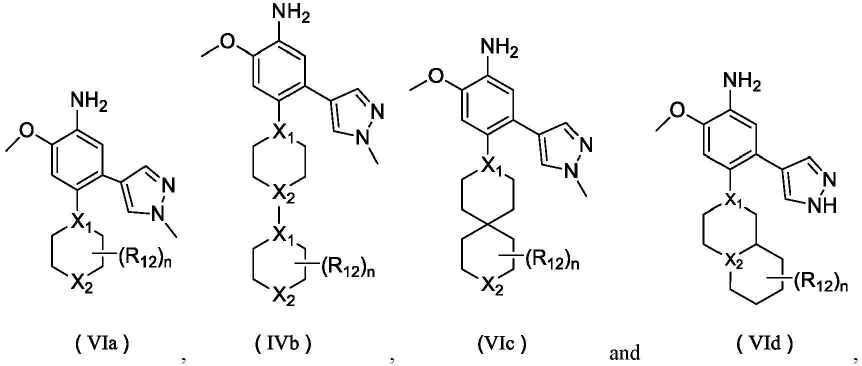 wherein X₁, X₂, R₁₂ and n are defined in formula (II).

The present disclosure provides a compound represented by formula (I), a stereoisomer thereof, a tautomer thereof, a pharmaceutically acceptable salt thereof, a prodrug thereof, a hydrate thereof, a solvate thereof or an isotope-labeled derivative thereof, 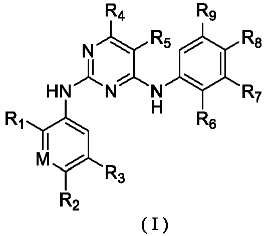 wherein,
R₁ is selected from H, halogen, -CN, C₁₋₆ alkyl, C₁₋₆ alkoxy, C₁₋₆ haloalkyl, C₁₋₆ haloalkoxy, C₃₋₆ cycloalkyl, 3-6-membered heterocycloalkyl, C₃₋₆ cycloalkyloxy, 3-6-membered heterocycloalkyloxy and C₂₋₆ alkenyloxy;
M is selected from N or CR₁₀;
R₁₀ and R₁ may form a 5-8-membered heterocycloalkyl, and the 5-8-membered heterocycloalkyl is optionally substituted by one or more R₁₁ groups;
R₂ is selected from H, halogen, -CN, -OH, -NH₂, phosphonyl, sulfonyl, aminosulfonyl, aminocarbonyl, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, C₃₋₆ heterocycloalkenyl and phenyl; wherein, the -OH, -NH₂, phosphonyl, sulfonyl, aminosulfonyl, aminocarbonyl, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, C₃₋₆ heterocycloalkenyl and phenyl are optionally substituted by one or more R₁₂ groups;
R₃ is selected from C₆₋₁₀ aryl, 5-12-membered heteroaryl, 3-8-membered heterocycloalkyl, C₄₋₈ cycloalkyl and C₃₋₆ cycloalkenyl, wherein the C₆₋₁₀ aryl, 5-12-membered heteroaryl, 3-8-membered heterocycloalkyl, C₃₋₈ cycloalkyl and C₃₋₆ cycloalkenyl are optionally substituted by one or more R₁₃ groups;
R₄ and R₅ are each independently selected from H, halogen, -CN, C₁₋₆ alkyl, C₁₋₆ alkoxy, C₁₋₆ haloalkyl, C₁₋₆ haloalkoxy, C₃₋₈ cycloalkyl and 3-8-membered heterocycloalkyl;
or, R₄ and R₅ are cyclized to 4-6-membered cycloalkyl, 4-6-membered heterocycloalkyl, 4-6-membered aryl or 4-6-membered heteroaryl;
R₆ is selected from amino, amido, sulfonyl, thiophosphonyl, phosphonyl, sulfonylamino and aminosulfonyl, wherein the amino, amido, sulfonyl, phosphonyl, sulfonylamino and aminosulfonyl are optionally substituted by one or more R₁₄ groups;
R₇, F₈ and R₉ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6-membered heterocycloalkyl and 5-7-membered heteroaryl;
or, R₇ and F₈ are cyclized to C₄₋₆ cycloalkyl, 4-6-membered heterocycloalkyl, C₅₋₆ aryl, or 5-7-membered heteroaryl;
or, F₈ and R₉ are cyclized to C₄₋₆ cycloalkyl, 4-6-membered heterocycloalkyl, C₅₋₆ aryl, or 5-7-membered heteroaryl;
R₁₀ and R₁₁ are each independently selected from H, halogen, C₁₋₆ alkyl, C₁₋₆ alkoxy, C₁₋₆ haloalkyl and C₁₋₆ haloalkoxy;
R₁₂ is selected from H, halogen, -CN, -OH, -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₈ cycloalkyl, 3-8-membered heterocycloalkyl, hydroxy-C₁₋₆ alkyl, C₃₋₈ cycloalkylalkyl-, C₃₋₈ heterocycloalkylalkyl-, C₃₋₈ cycloalkyl-C₁₋₆ alkoxy-, C₁₋₆ alkylsulfonyl, C₃₋₆ cycloalkylsulfonyl, NRₐR_{b}CO-, C₁₋₆ alkylcarbonyl, C₃₋₈ cycloalkylcarbonyl, C₁₋₆ alkoxy-C₁₋₆ alkyl-, C₃₋₆ cycloalkenyl, phenyl, NRₐR_{b}S(O)₂-, -(CH₂)ₘNRₐR_{b}, -(CH₂)ₘO(CH₂)ₙCH₃ and -O(CH₂)ₘNRₐR_{b}; wherein the Rₐ and R_{b} are H, C₁₋₆ alkyl or C₁₋₆ alkoxy, or Rₐ and R_{b} together form C₃₋₈ cycloalkyl or 3-8-membered heterocycloalkyl; wherein m and n are independently optionally 0, 1, 2 or 3, C₃₋₈ cycloalkyl and 3-8-membered heterocycloalkyl are optionally substituted by halogen, -OH, -NH₂, -CN, C₁₋₆ alkyl, C₁₋₆ alkoxy and C₁₋₆ haloalkyl;
R₁₃ is selected from H, halogen, -CN, C₁₋₆ alkyl, C₁₋₆ alkoxy, C₁₋₆ haloalkyl, C₁₋₆ haloalkoxy, C₃₋₈ cycloalkyl and 3-8-membered heterocycloalkyl;
R₁₄ is selected from H, C₁₋₆ alkyl, C₁₋₆ alkoxy, C₁₋₆ haloalkyl, C₁₋₆ haloalkoxy, C₃₋₈ cycloalkyl and 3-8-membered heterocycloalkyl.

In some embodiments of the present disclosure, the R₃ is selected from C₆₋₁₀ aryl, 5-12-membered heteroaryl and C₃₋₆ cycloalkenyl.

In some embodiments of the present disclosure, the R₁₀ and R₁₁ are each independently selected from H, fluorine, chlorine, bromine, methyl, ethyl, n-propyl, isopropyl, n-butyl, methoxy, ethoxy, n-propoxy, isopropoxy, n-butoxy, trifluoromethyl, trifluoromethoxy, trichloromethyl, trichloromethoxy and 2,2,2-trifluoroethoxy.

In some embodiments of the present disclosure, the R₁₃ is selected from H, fluorine, chlorine, bromine, methyl, ethyl, n-propyl, isopropyl, difluoromethyl, trifluoromethyl, trichloromethyl and cyclopropyl.

In some embodiments of the present disclosure, the R₁₃ is selected from H, fluorine, methyl, ethyl and difluoromethyl.

In some embodiments of the present disclosure, the R₁₄ is selected from methyl, ethyl, n-propyl, isopropyl, n-butyl, methoxy, ethoxy, n-propoxy, isopropoxy and cyclopropyl.

In some embodiments of the present disclosure, the R₁ is selected from H, halogen, -CN, C₁₋₃ alkyl, C₁₋₃ alkoxy, C₁₋₃ haloalkyl and C₁₋₃ haloalkoxy.

In some embodiments of the present disclosure, the R₁ is selected from H, methyl, ethyl, n-propyl, isopropyl, n-butyl, methoxy, ethoxy, n-propoxy, isopropoxy, n-butoxy, trifluoromethyl, trifluoromethoxy, trichloromethyl, trichloromethoxy and 2,2,2-trifluoroethoxy.

In some embodiments of the present disclosure, the R₂ is selected from H, halogen, -CN, -OH, - NH₂, C₃₋₇ cycloalkyl, 3-7-membered heterocycloalkyl, C₆₋₁₄ spiro cyclyl, C₆₋₁₄ fused cyclyl, C₆₋₁₄ bridged cyclyl, 6-14-membered spiro heterocyclyl, 6-14-membered bridged heterocyclyl and 6-14-membered fused heterocyclyl; wherein, the -NH₂, C₃₋₇ cycloalkyl, 3-7-membered heterocycloalkyl, C₆₋₁₄ spiro cyclyl, C₆₋₁₄ fused cyclyl, C₆₋₁₄ bridged cyclyl, 6-14-membered spiro heterocyclyl, 6-14-membered bridged heterocyclyl and 6-14-membered fused heterocyclyl are optionally substituted by one or more R₁₂ groups, and the R₁₂ group is defined as above.

In some embodiments of the present disclosure, the R₂ is selected from H, halogen, -CN, -OH, - NH₂, -NHR₁₂, -NR₁₂R₁₂, 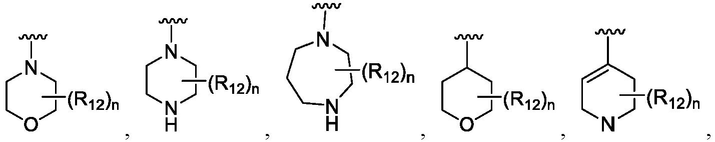 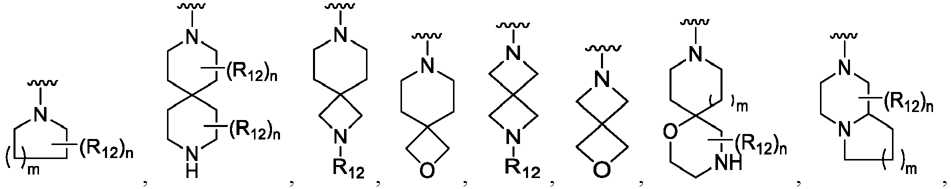 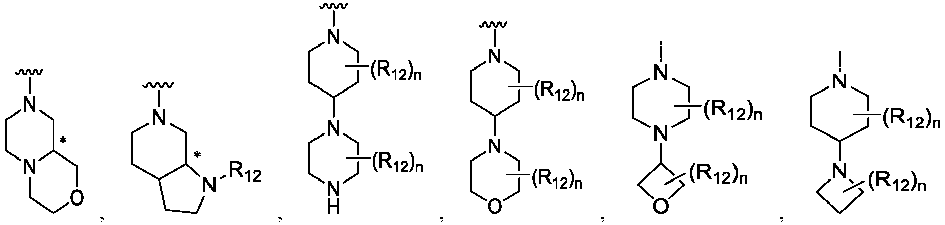 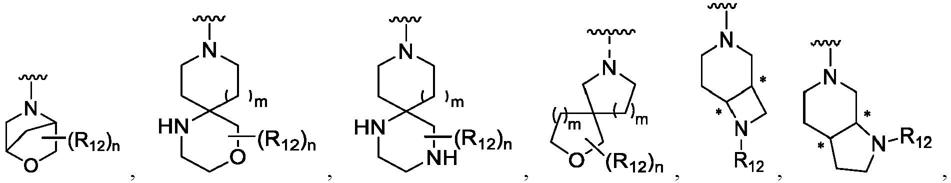 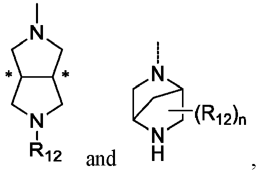 wherein the R₁₂, m and n are defined as above.

In some embodiments of the present disclosure, the R₂ is selected from H, fluorine, chlorine, bromine, iodine, -NH₂,

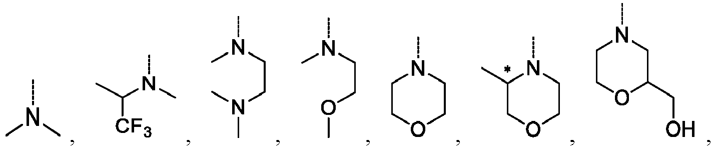

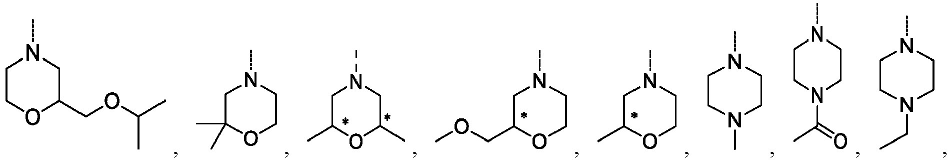

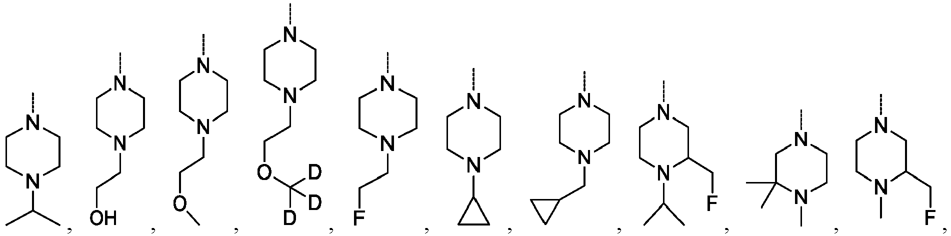

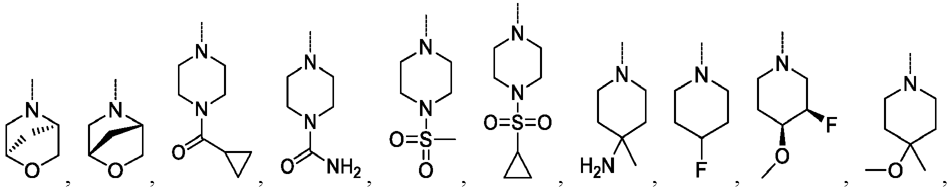

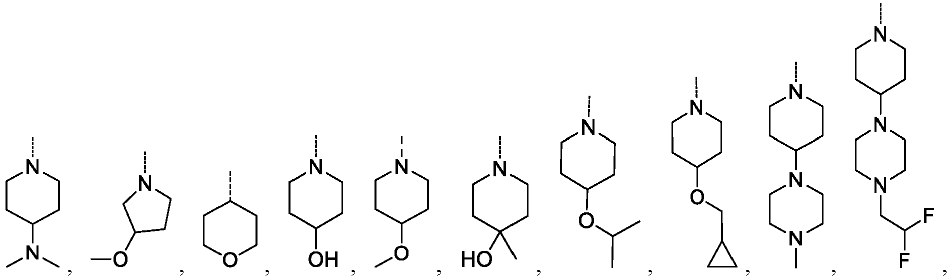

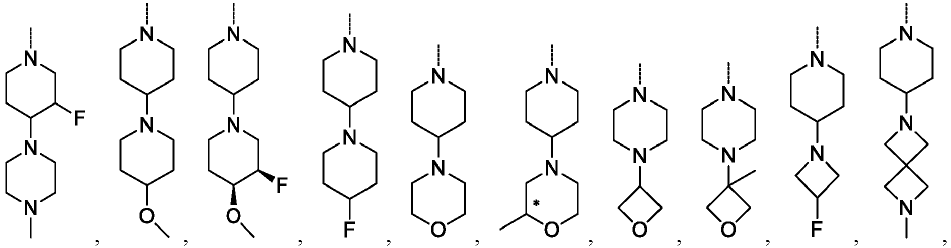

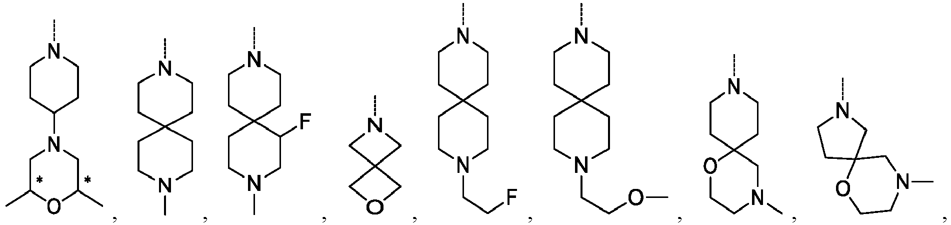

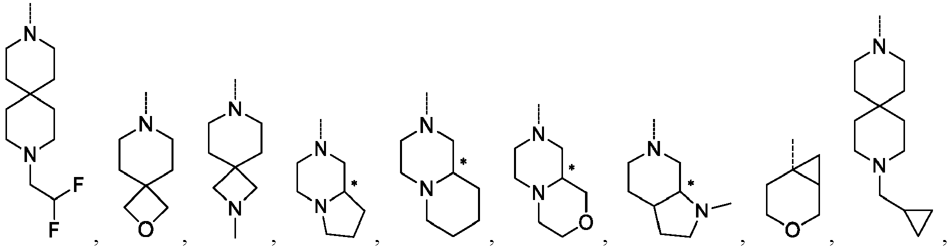

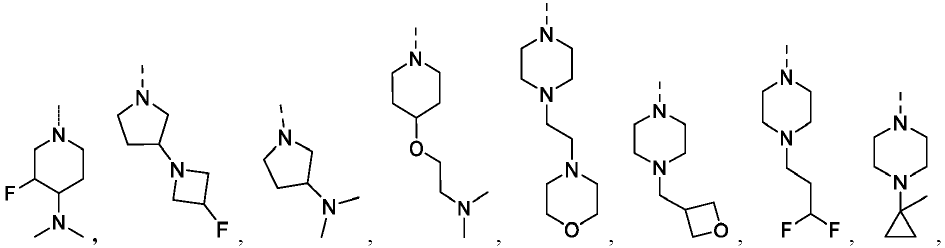

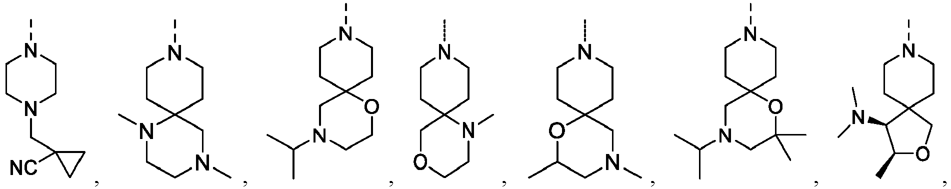

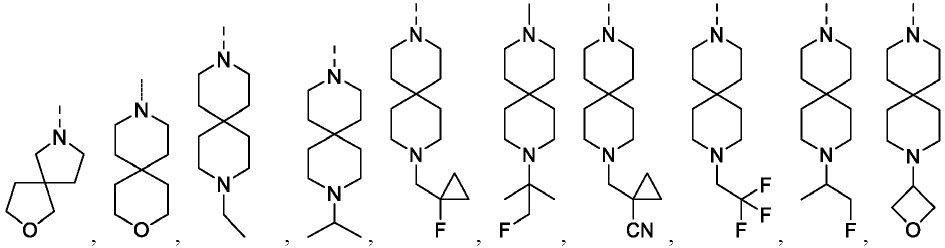

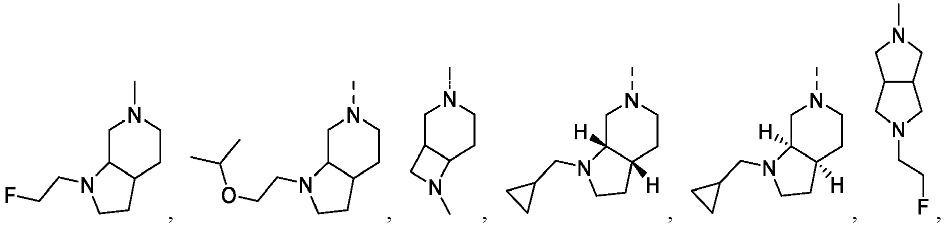

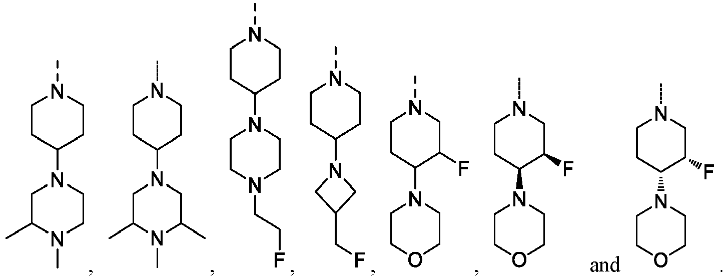

In some embodiments of the present disclosure, the R₂ is selected from

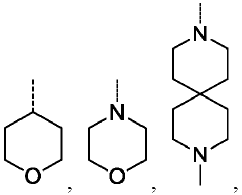

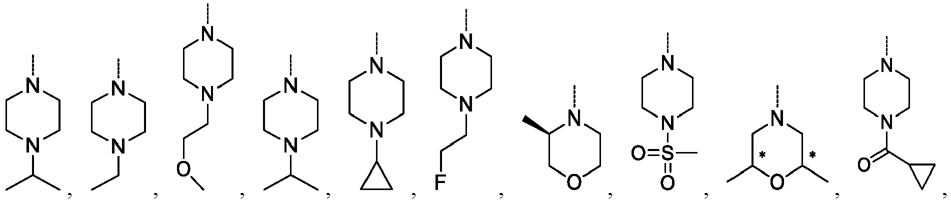

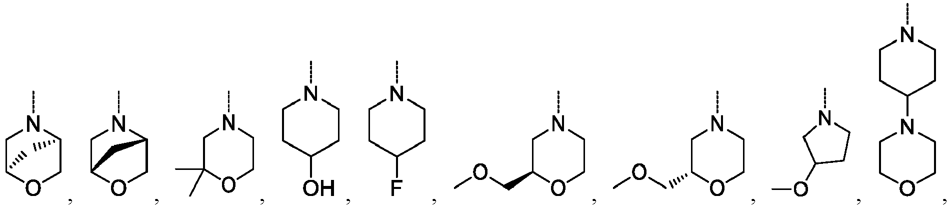

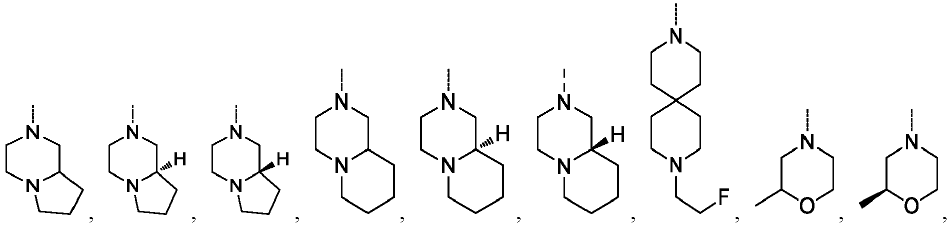

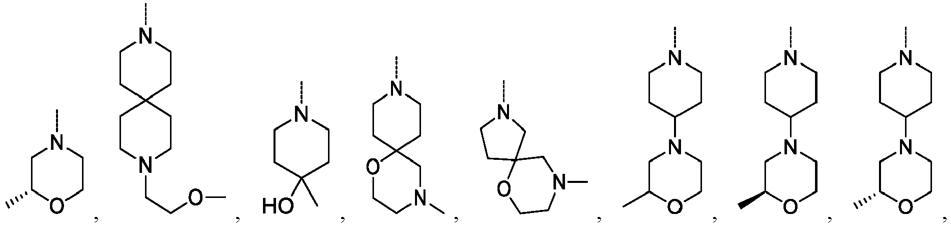

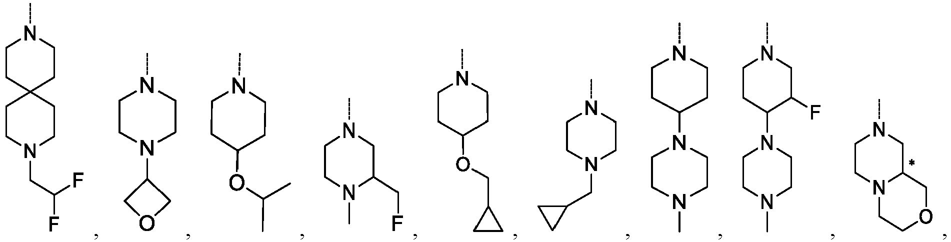

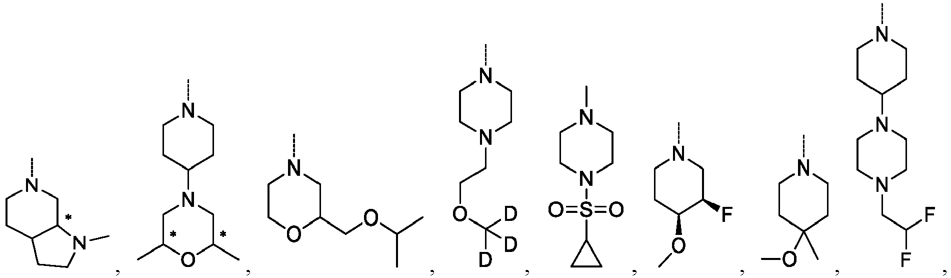

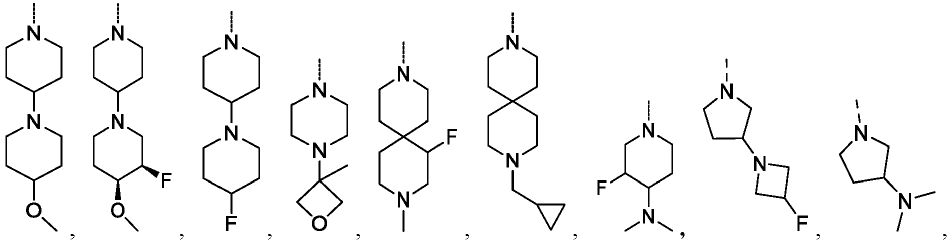

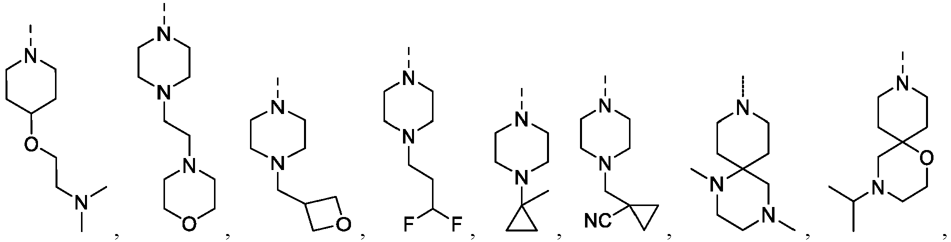

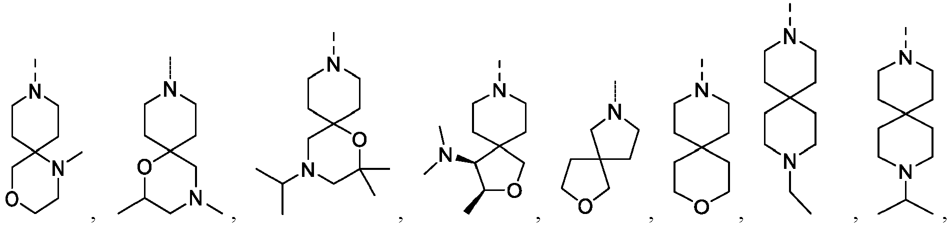

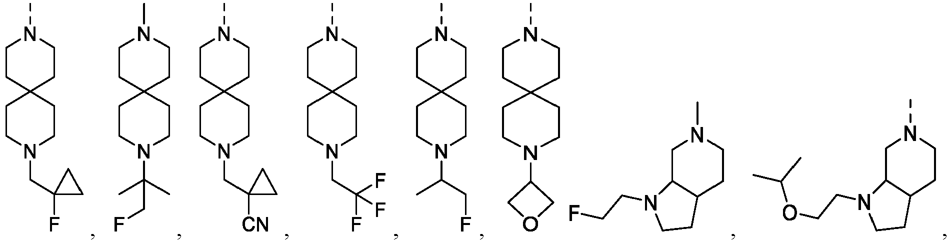

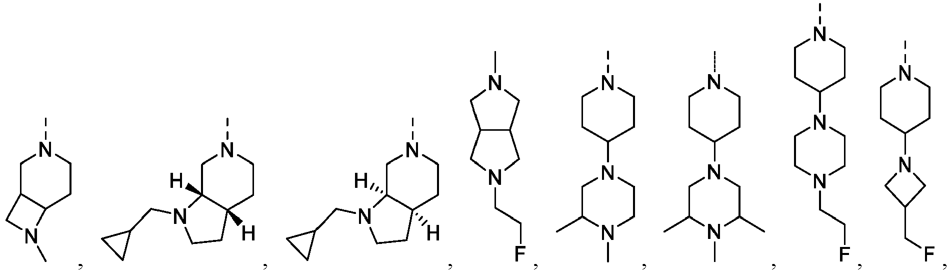

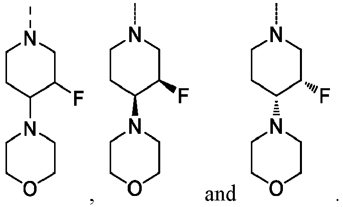

In some embodiments of the present disclosure, the R₃ is selected from phenyl, pyranyl, pyridyl, pyrimidinyl, pyridazinyl, pyrazinyl, furanyl, thienyl, pyrrolyl, pyrazolyl, thiazolyl, imidazolyl, benzofuranyl, benzimidazolyl, benzothienyl, benzoxazolyl, benzothiazolyl, indolyl, pyrazolo[1,5-*a*]pyridyl, quinolinyl, isoquinolinyl, tetrahydropyrrolyl, piperidinyl, piperazinyl, morpholinyl, tetrahydrofuranyl, tetrahydropyranyl, cyclobutyl, cyclopentyl and cyclohexyl, and the phenyl, pyranyl, pyridyl, pyrimidinyl, pyridazinyl, pyrazinyl, furanyl, thienyl, pyrrolyl, pyrazolyl, thiazolyl, imidazolyl, benzofuranyl, benzimidazolyl, benzothienyl, benzoxazolyl, benzothiazolyl, indolyl, pyrazolo[1,5-*a*]pyridyl, quinolinyl, isoquinolinyl, tetrahydropyrrolyl, piperidinyl, piperazinyl, morpholinyl, tetrahydrofuranyl, tetrahydropyranyl, cyclobutyl, cyclopentyl and cyclohexyl are optionally substituted by one or more R₁₃ groups, wherein the R₁₃ group is defined as above.

In some embodiments of the present disclosure, the R₃ is selected from 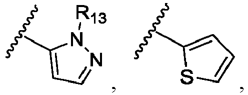 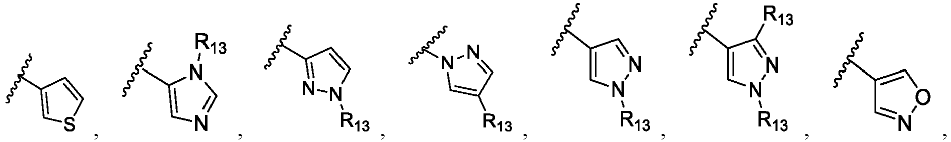 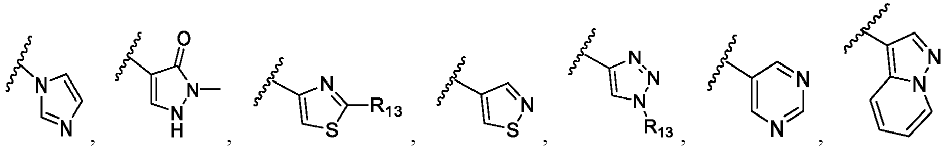 and 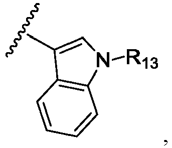 wherein the R₁₃ is defined as above.

In some embodiments of the present disclosure, the R₃ is selected from 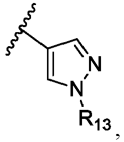 wherein the R₁₃ is defined as above.

In some embodiments of the present disclosure, the R₄ and R₅ are each independently selected from H, F, Cl, Br, CN, methyl, ethyl, isopropyl, methoxy, ethoxy, isopropoxy, trifluoromethyl, 2,2,2-trifluoroethyl and cyclopropyl.

In some embodiments of the present disclosure, the R₆ is selected from

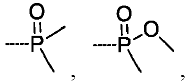

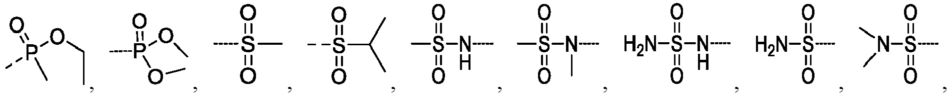

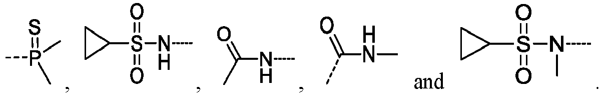

In some embodiments of the present disclosure, the R₆ is selected from

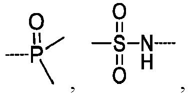

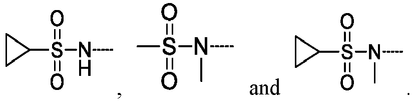

In some embodiments of the present disclosure, R₇ and F₈ are cyclized to C₄₋₆ cycloalkyl, 4-6-membered heterocycloalkyl, C₅₋₆ aryl or 5-7-membered heteroaryl, or F₈ and R₉ are cyclized to C₄₋₆ cycloalkyl, 4-6-membered heterocycloalkyl, C₅₋₆ aryl or 5-7-membered heteroaryl.

In some embodiments of the present disclosure, R₇ and R₈ or R₈ and R₉ are independently cyclized to cyclobutane, cyclopentane, tetrahydropyrrole ring, tetrahydrofuran ring, tetrahydropyran ring, thiophene ring, imidazole ring, pyrazole ring, pyrrole ring, oxazole ring, thiazole ring, isoxazole ring, piperazine ring, isothiazole ring, benzene ring, pyridine ring, piperidine ring, pyrimidine ring, pyridazine ring or pyrazine ring.

In some embodiments of the present disclosure, R₇ and R₈ or R₈ and R₉ are independently cyclized to cyclobutane, pyridine ring or pyrazine ring.

In some embodiments of the present disclosure, the compound, the stereoisomer thereof, the tautomer thereof, the pharmaceutically acceptable salt thereof, the prodrug thereof, the hydrate thereof, the solvate thereof or the isotope-labeled derivative thereof is selected from, 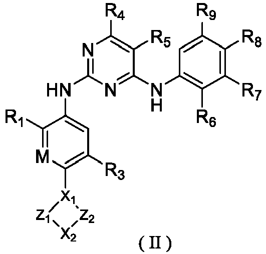 wherein, X₁ is independently selected from CR_{c} and N;
X₂ is independently selected from -CR_{c}R_{d}-, -NR_{c}- and -O-;
Z₁ and Z₂ are each independently selected from -(CRₑR_{f})ₘ(CRₑR_{f})ₙ-;
the R_{c}, R_{d}, Rₑ, and R_{f} are each independently selected from H, halogen, -CN, -OH, -NRₐR_{b}, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, phenyl, -(CH₂)ₘNRₐR_{b}, hydroxy-C₁₋₆ alkyl-O(CH₂)ₘNRₐR_{b}, C₃₋₈ cycloalkylalkyl-, C₃₋₈ heterocycloalkylalkyl-, C₃₋₈ cycloalkyl-C₁₋₆ alkoxy -, C₃₋₈ heterocycloalkyl-C₁₋₆ alkoxy-, C₁₋₆ alkylcarbonyl-, C₃₋₈ cycloalkylcarbonyl-, NRₐR_{b}CO-, C₁₋₆ alkylcarbonylsulfonyl- and C₃₋₈ cycloalkylsulfonyl-, wherein the Rₐ, R_{b}, m and n are defined as above;
or, R_{c} and R_{d} together form C₃₋₈ cycloalkyl or 3-8-membered heterocycloalkyl;
or, Rₑ and R_{f} together form C₃₋₈ cycloalkyl or 3-8-membered heterocycloalkyl;
or, R_{c} and Rₑ, or R_{c} and R_{f}, or R_{d} and Rₑ, or R_{d} and R_{f} together form C₃₋₈-membered cycloalkyl or 3-8-membered heterocycloalkyl;
wherein, a ring formed by X₁, X₂, Z₁ and Z₂ and a ring further formed by substituent groups R_{c}, R_{d}, Rₑ and R_{f} thereof are optionally substituted by one or more R₁₂ groups;
R₁, R₃, R₄, R₅, R₆, R₇, R₈, R₉, R₁₂ and M are defined as above.

In some embodiments of the present disclosure, the compound, the stereoisomer thereof, the tautomer thereof, the pharmaceutically acceptable salt thereof, the prodrug thereof, the hydrate thereof, the solvate thereof or the isotope-labeled derivative thereof is selected from, 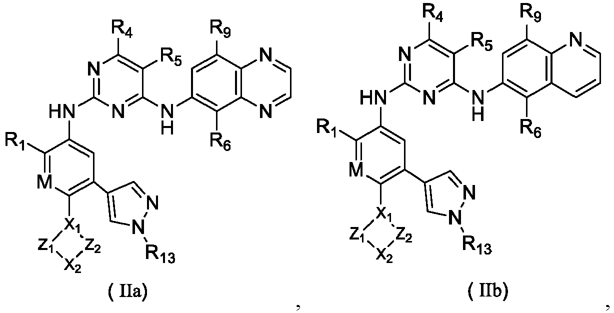 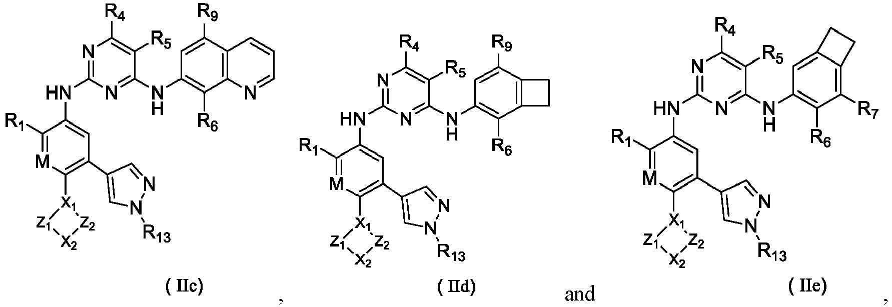
wherein, the ring formed by X₁, X₂, Z₁ and Z₂ and the ring further formed by substituent groups thereof are optionally substituted by one or more R₁₂ groups;
X₁, X₂, Z₁, Z₂, R₁, R₄, R₅, R₆, R₇, R₉, R₁₂, R₁₃ and M are defined as above.

In some embodiments of the present disclosure, the compound, the stereoisomer thereof, the tautomer thereof, the pharmaceutically acceptable salt thereof, the prodrug thereof, the hydrate thereof, the solvate thereof or the isotope-labeled derivative thereof is selected from, 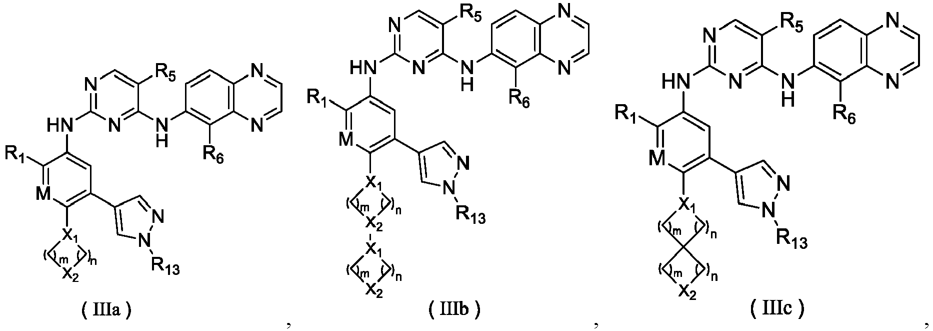 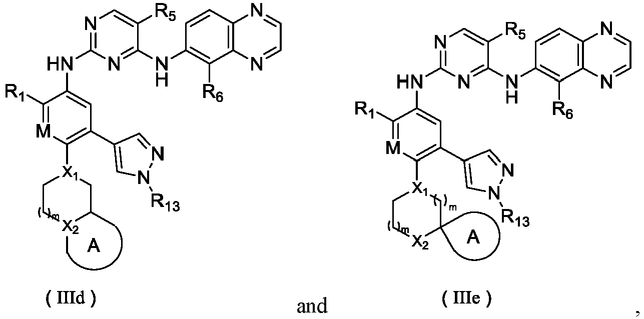 wherein, ring A is selected from C₅₋₇ cycloalkyl and 5-7-membered heterocycloalkyl;
a monocyclic ring, a bicyclic ring, a spiro ring, a fused ring formed by X₁ and X₂ and ring A are optionally substituted by one or more R₁₂ groups;
X₁, X₂, R₁, R₅, R₆, m, n, M, R₁₂ and R₁₃ are defined as above.

In some embodiments of the present disclosure, the compound, the stereoisomer thereof, the tautomer thereof, the pharmaceutically acceptable salt thereof, the prodrug thereof, the hydrate thereof, the solvate thereof or the isotope-labeled derivative thereof is selected from, 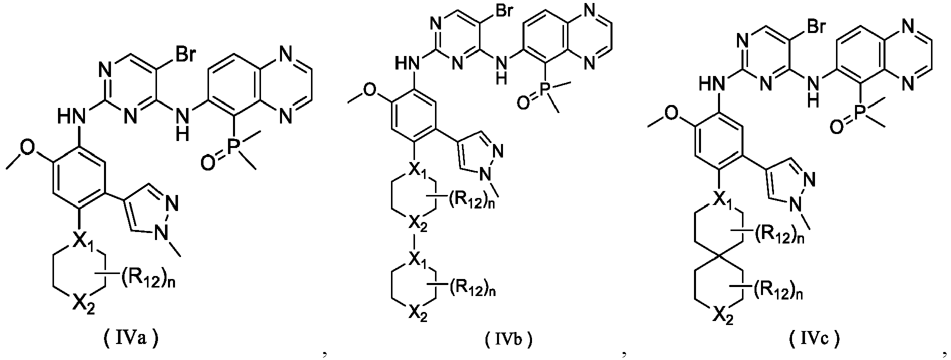 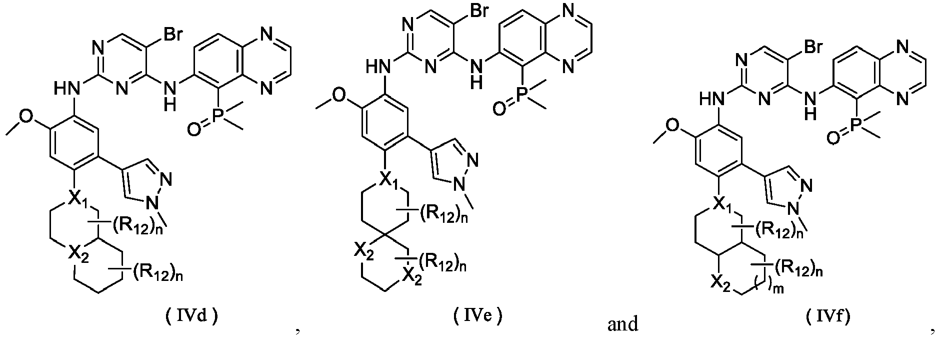 wherein X₁, X₂, R₁₂, m and n are defined as above.

In some embodiments of the present disclosure, the compound, the stereoisomer thereof, the tautomer thereof, the pharmaceutically acceptable salt thereof, the prodrug thereof, the hydrate thereof, the solvate thereof or the isotope-labeled derivative thereof is selected from, 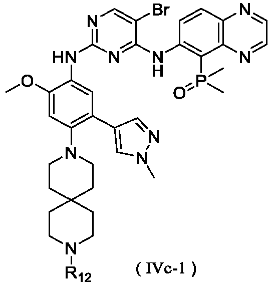 wherein R₁₂ is defined as above.

The present disclosure further provides a compound represented by the following formula, a stereoisomer thereof, a tautomer thereof, a pharmaceutically acceptable salt thereof, a prodrug thereof, a hydrate thereof, a solvate thereof or an isotope-labeled derivative thereof, which is selected from,

| Compound | Structural formula |
|---|---|
| **1** | 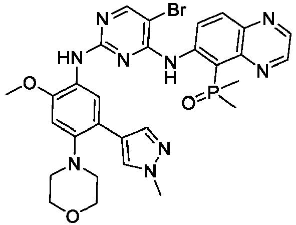 |
| **2** | 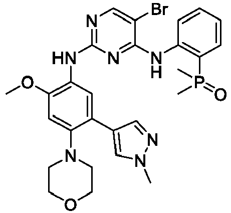 |
| **3** | 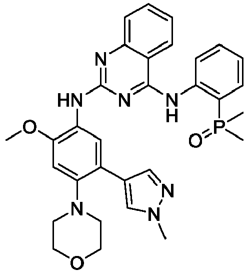 |
| **4** | 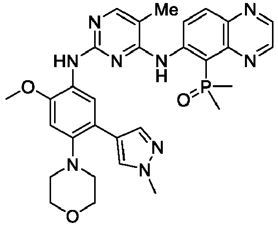 |
| **5** | 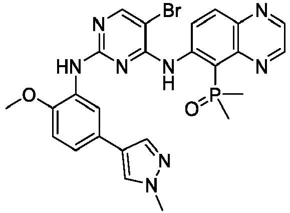 |
| **6** | 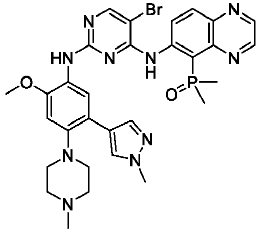 |
| **7** | 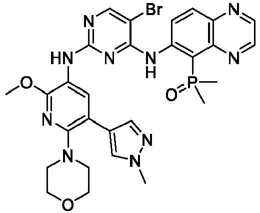 |
| **8** | 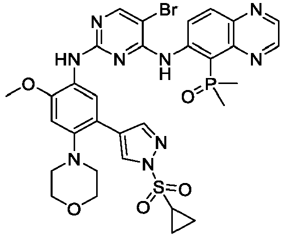 |
| **9** | 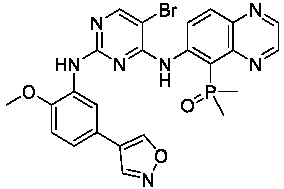 |
| **10** | 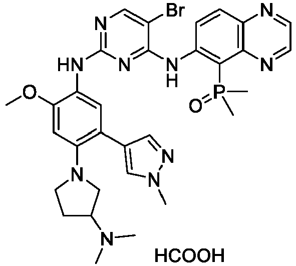 |
| **11** | 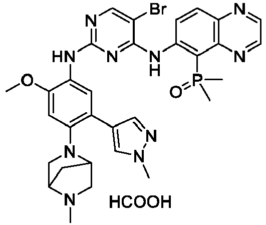 |
| **12** | 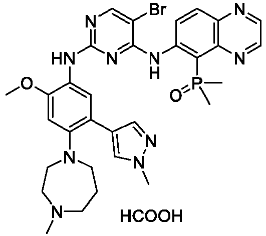 |
| **13** | 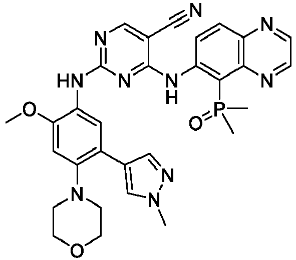 |
| **14** | 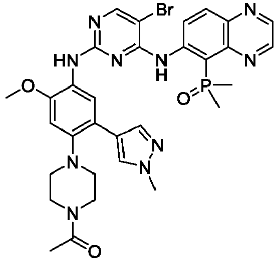 |
| **15** | 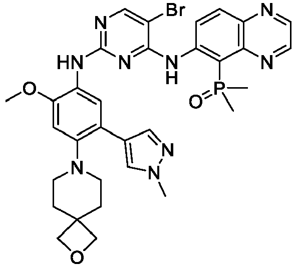 |
| **16** | 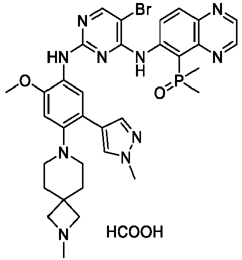 |
| **17** | 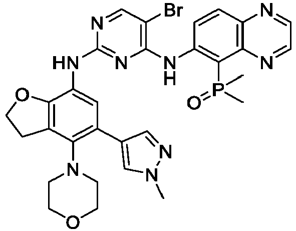 |
| **18** | 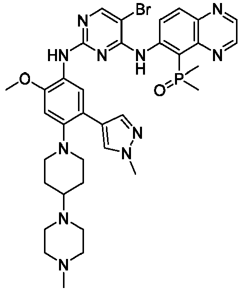 |
| **19** | 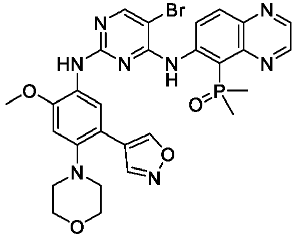 |
| **20** | 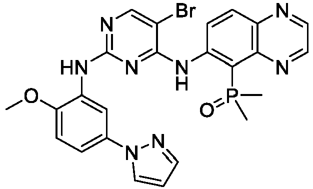 |
| **21** | 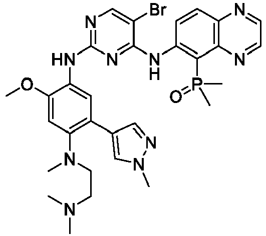 |
| **22** | 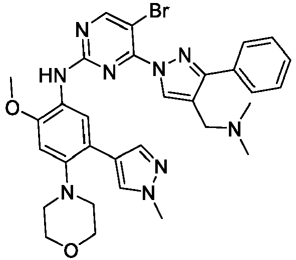 |
| **23** | 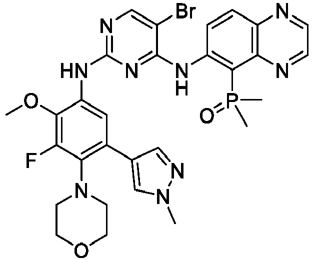 |
| **24** | 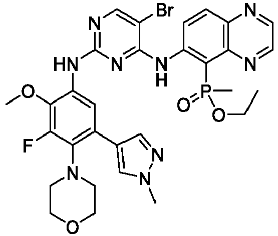 |
| **25** | 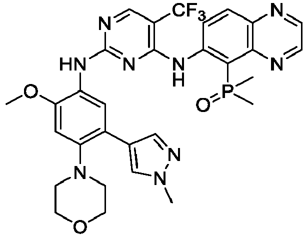 |
| **26** | 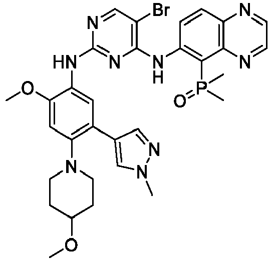 |
| **27** | 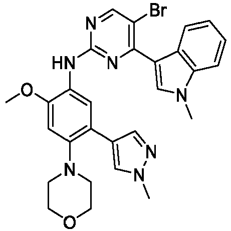 |
| **28** | 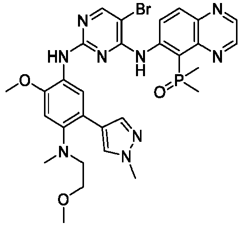 |
| **29** | 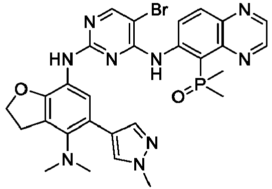 |
| **30** | 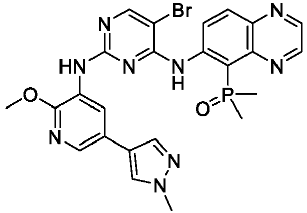 |
| **31** | 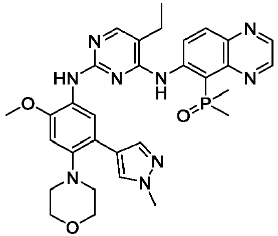 |
| **32** | 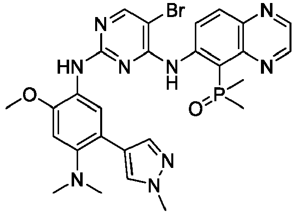 |
| **33** | 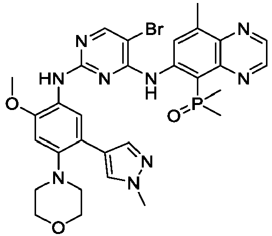 |
| **34** | 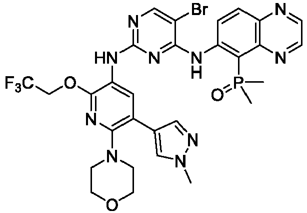 |
| **35** | 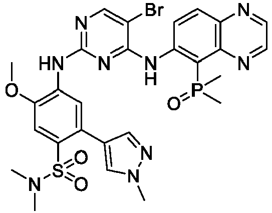 |
| **36** | 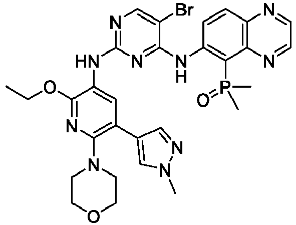 |
| **37** | 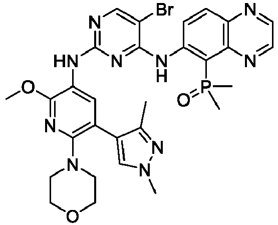 |
| **38** | 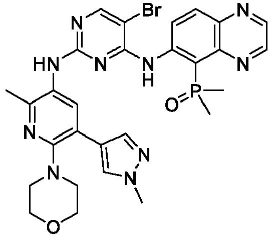 |
| **39** | 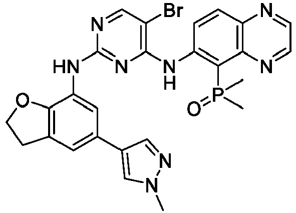 |
| **40** | 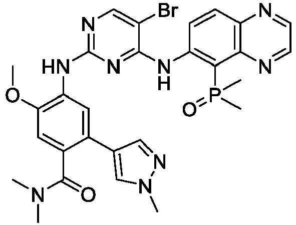 |
| **41** | 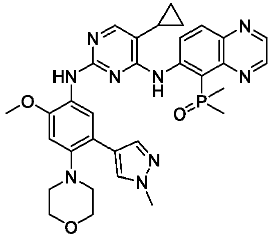 |
| **42** | 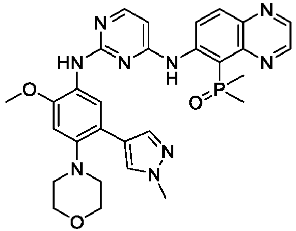 |
| **43** | 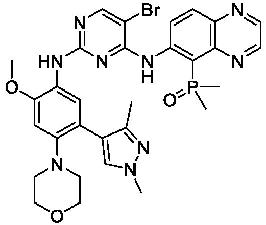 |
| **44** | 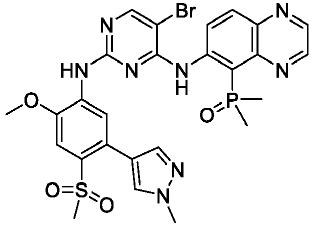 |
| **45** | 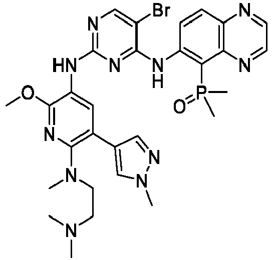 |
| **46** | 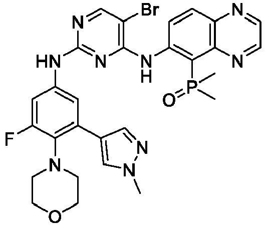 |
| **47** | 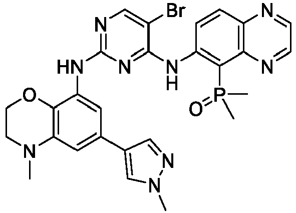 |
| **48** | 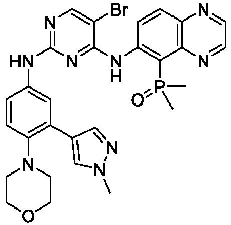 |
| **49** | 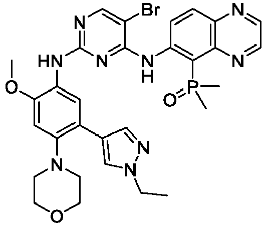 |
| **50** | 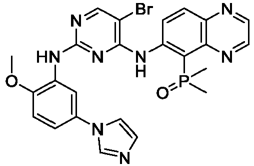 |
| **51** | 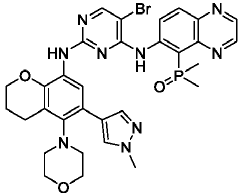 |
| **52** | 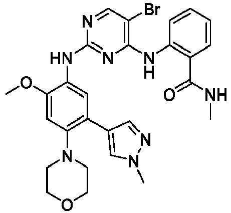 |
| **53** | 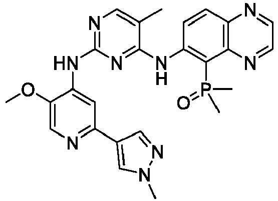 |
| **54** | 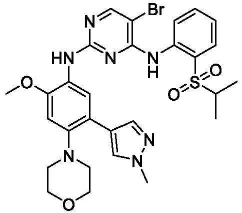 |
| **55** | 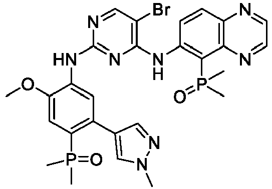 |
| **56** | 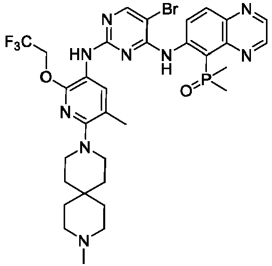 |
| **57** | 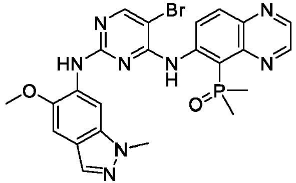 |
| **58** | 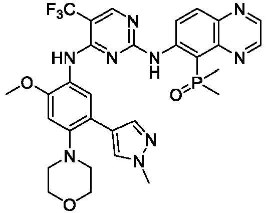 |
| **59** | 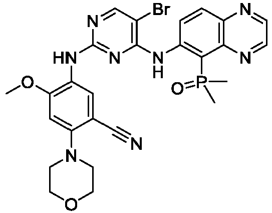 |
| **60** | 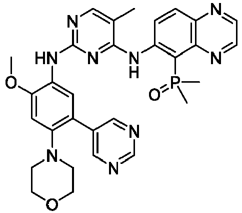 |
| **61** | 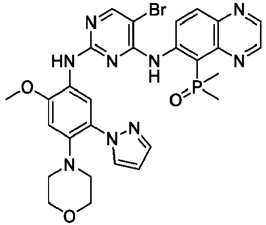 |
| **62** | 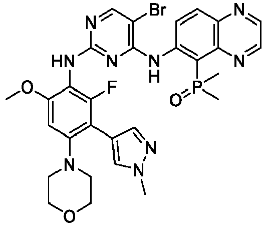 |
| **63** | 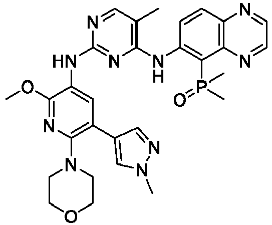 |
| **64** | 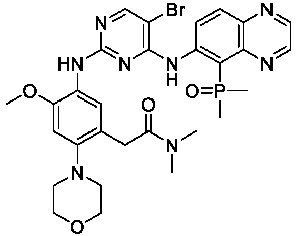 |
| **65** | 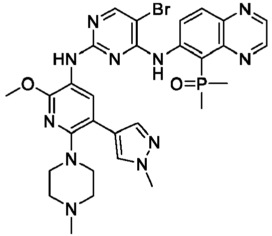 |
| **66** | 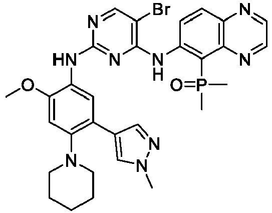 |
| **67** | 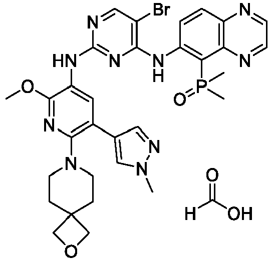 |
| **68** | 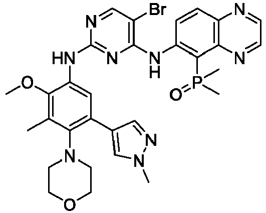 |
| **69** | 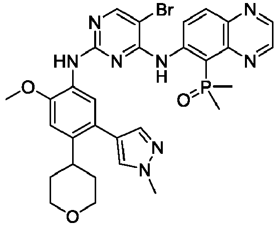 |
| **70** | 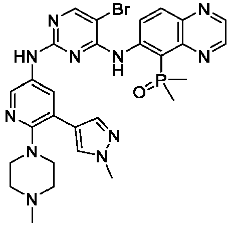 |
| **71** | 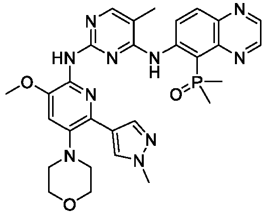 |
| **72** | 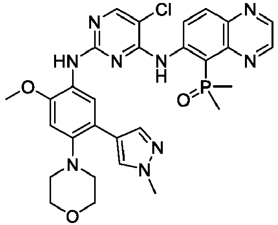 |
| **73** | 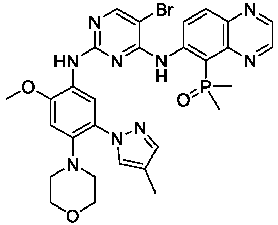 |
| **74** | 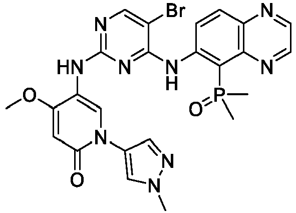 |
| **75** | 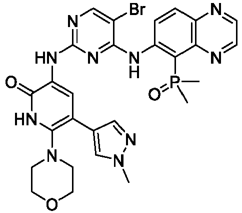 |
| **76** | 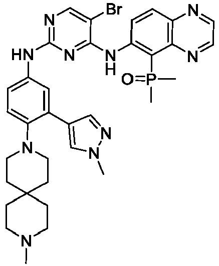 |
| **77** | 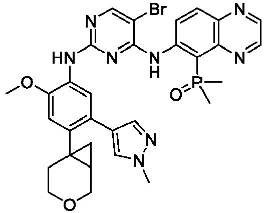 |
| **78** | 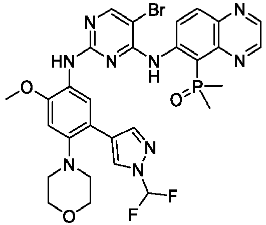 |
| **79** | 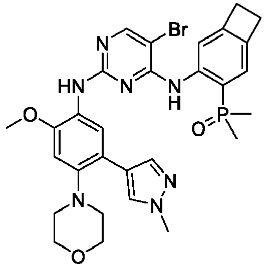 |
| **80** | 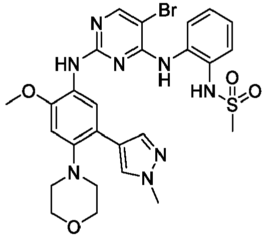 |
| **81** | 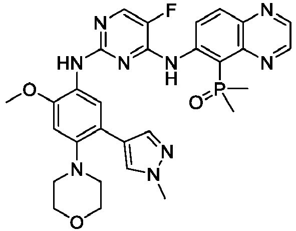 |
| **82** | 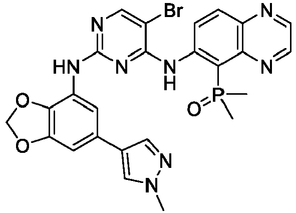 |
| **83** | 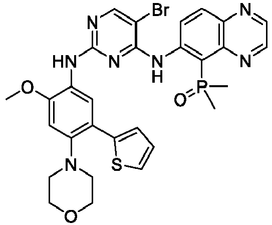 |
| **84** | 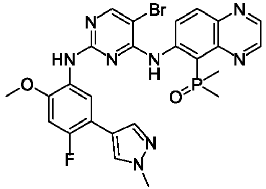 |
| **85** | 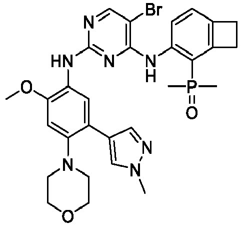 |
| **86** | 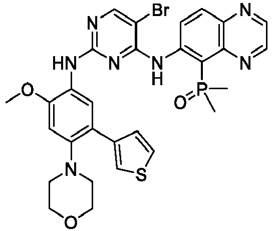 |
| **87** | 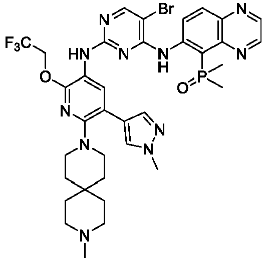 |
| **88** | 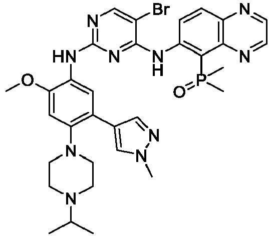 |
| **89** | 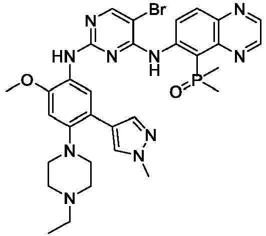 |
| **90** | 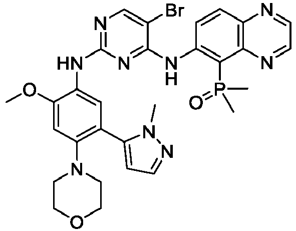 |
| **91** | 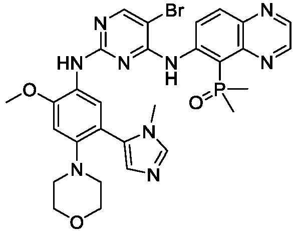 |
| **92** | 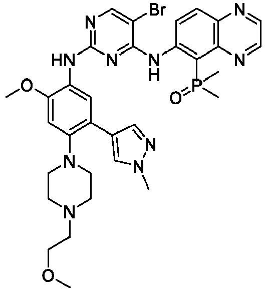 |
| **93** | 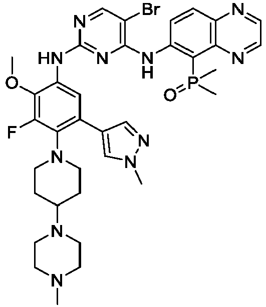 |
| **94** | 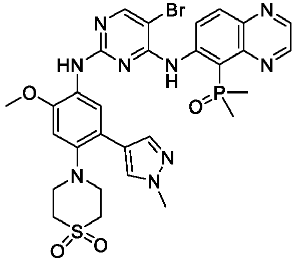 |
| **95** | 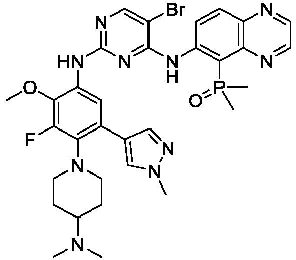 |
| **96** | 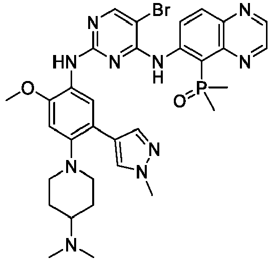 |
| **97** | 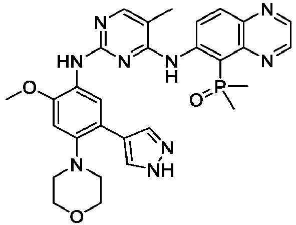 |
| **98** | 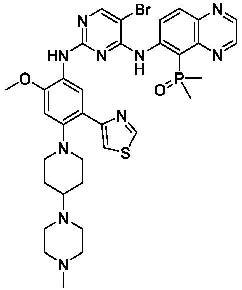 |
| **99** | 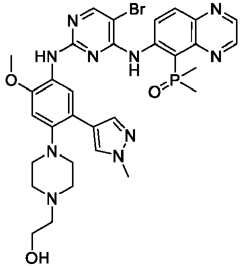 |
| **100** | 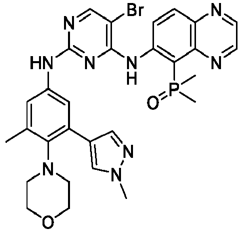 |
| **101** | 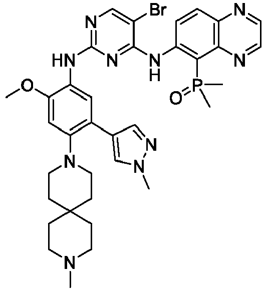 |
| **102** | 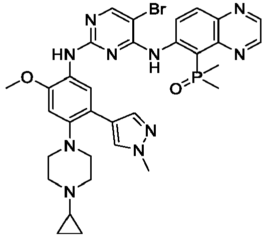 |
| **103** | 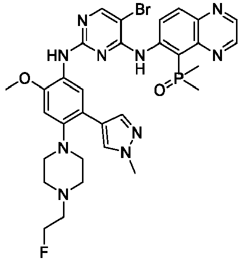 |
| **104** | 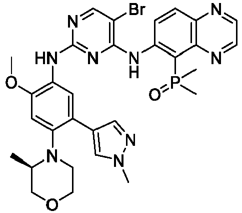 |
| **105** | 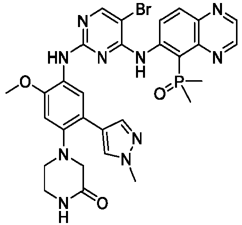 |
| **106** | 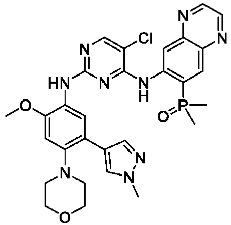 |
| **107** | 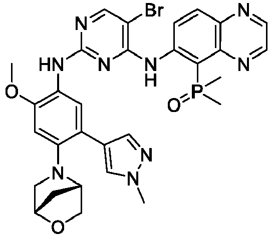 |
| **108** | 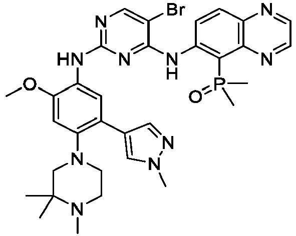 |
| **109** | 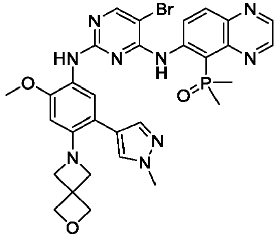 |
| **110** | 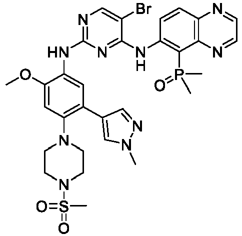 |
| **111** | 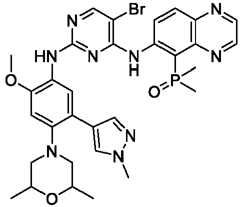 |
| **112** | 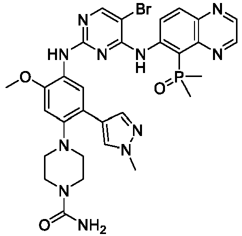 |
| **113** | 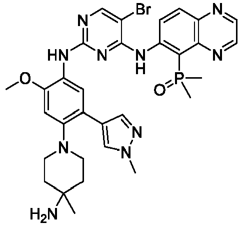 |
| **114** | 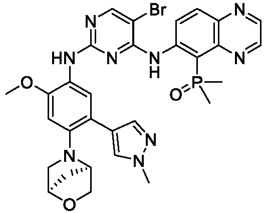 |
| **115** | 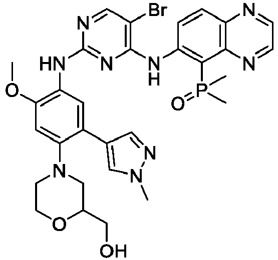 |
| **116** | 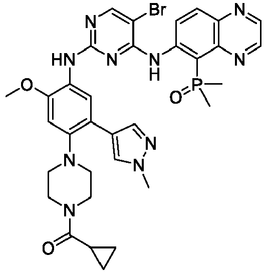 |
| **117** | 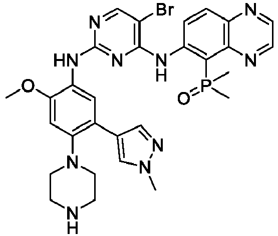 |
| **118** | 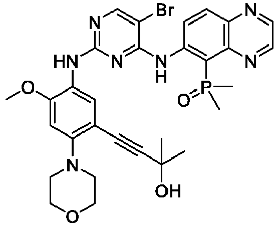 |
| **119** | 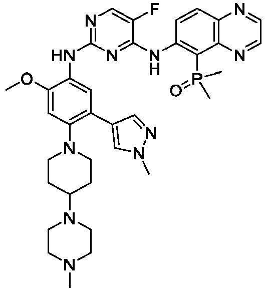 |
| **120** | 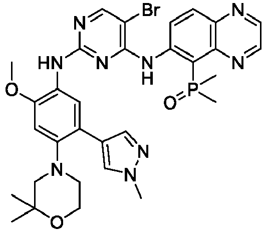 |
| **121** | 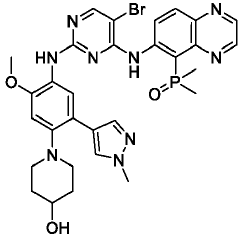 |
| **122** | 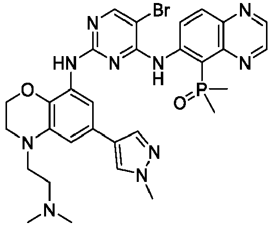 |
| **123** | 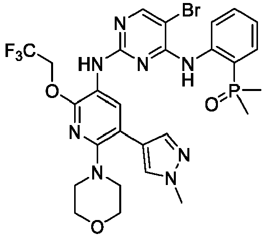 |
| **124** | 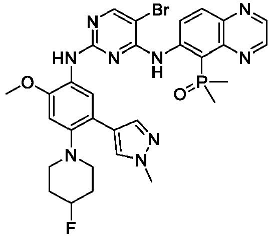 |
| **125** | 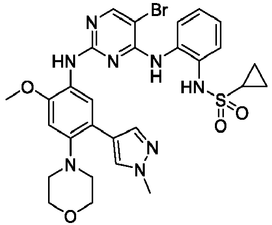 |
| **126** | 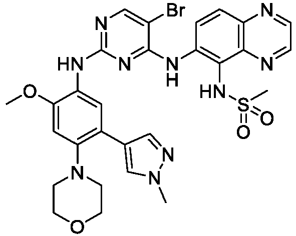 |
| **127** | 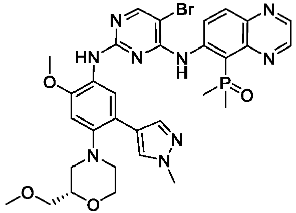 |
| **128** | 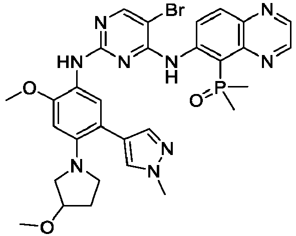 |
| **129** | 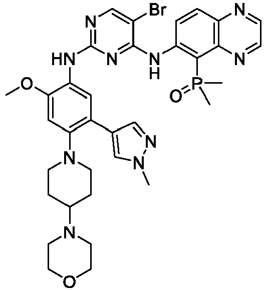 |
| **130** | 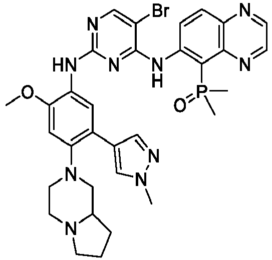 |
| **131** | 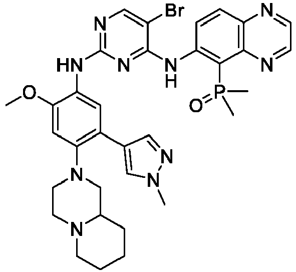 |
| **132** | 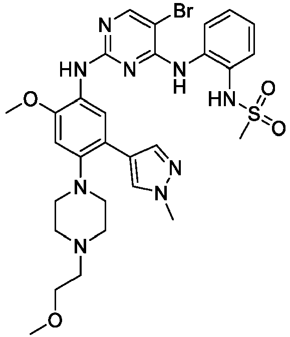 |
| **133** | 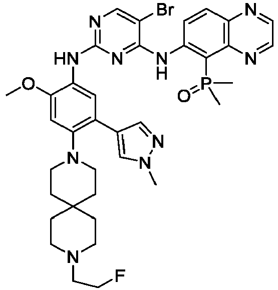 |
| **134** | 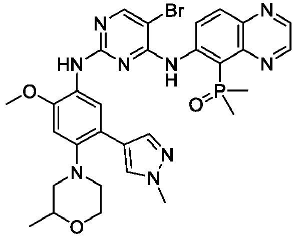 |
| **135** | 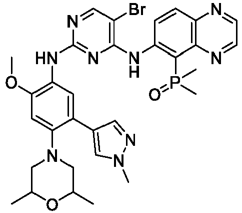 |
| **136** | 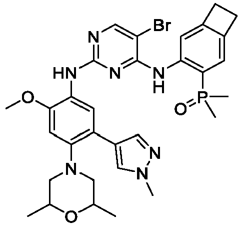 |
| **137** | 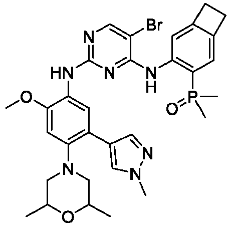 |
| **138** | 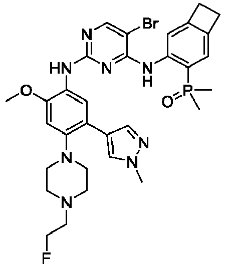 |
| **139** | 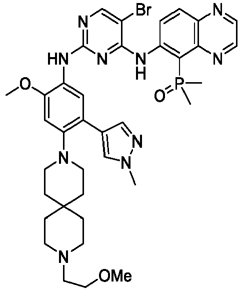 |
| **140** | 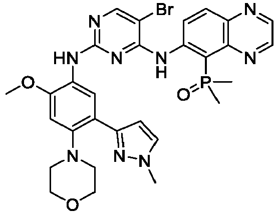 |
| **141** | 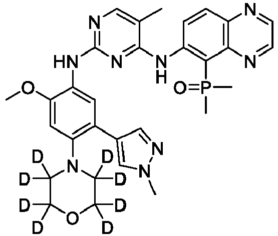 |
| **142** | 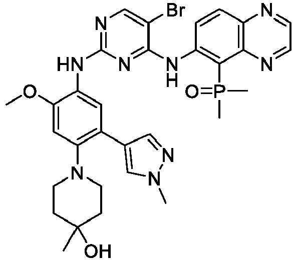 |
| **143** | 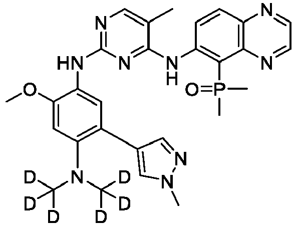 |
| **144** | 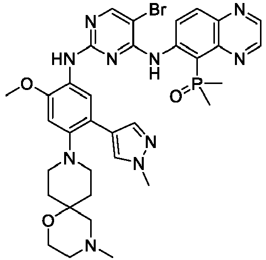 |
| **145** | 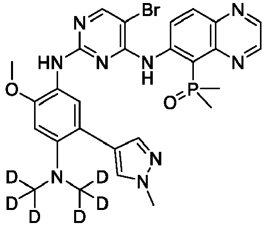 |
| **146** | 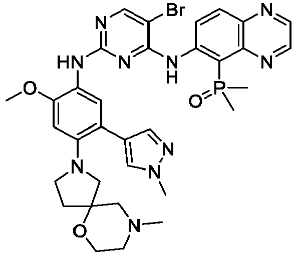 |
| **147** | 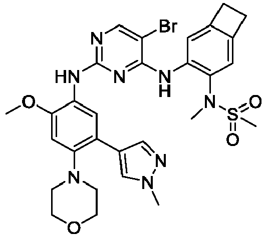 |
| **148** | 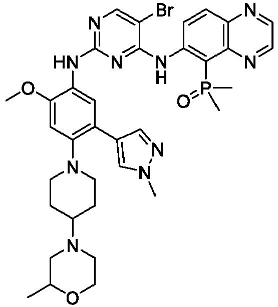 |
| **149** | 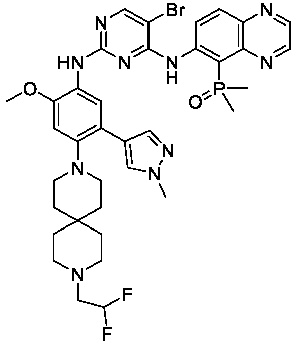 |
| **150** | 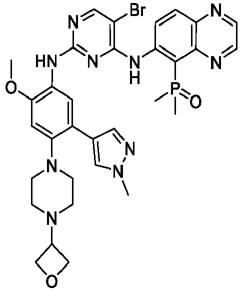 |
| **151** | 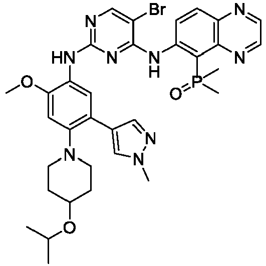 |
| **152** | 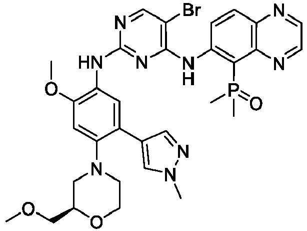 |
| **153** | 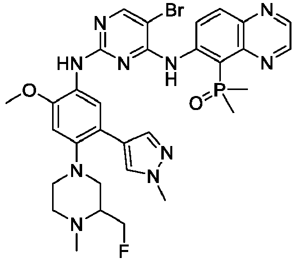 |
| **154** | 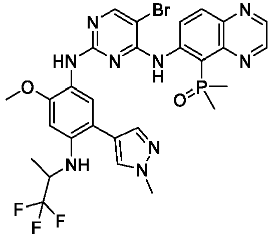 |
| **155a** | 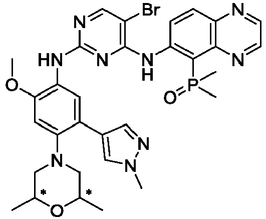 |
| | *(2S,6S or 2R,6R)* |
| **155b** | 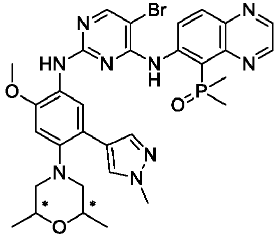 |
| | Enantiomer of 155a *( 2R,6R or 2S,6S)* |
| **156** | 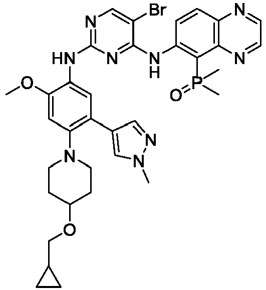 |
| **157** | 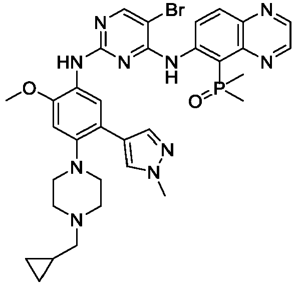 |
| **158** | 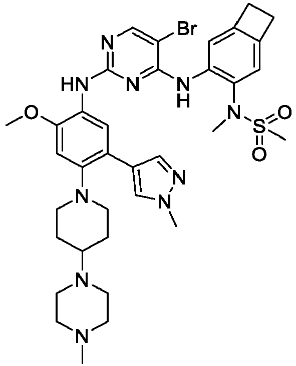 |
| **159** | 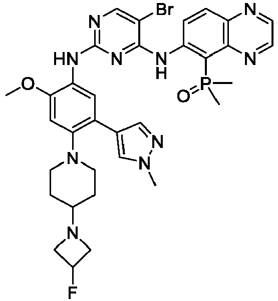 |
| **160** | 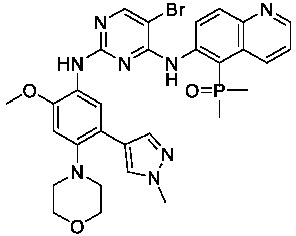 |
| **161** | 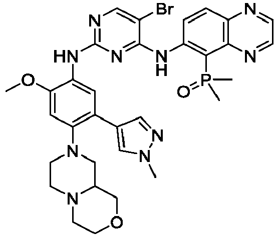 |
| **162** | 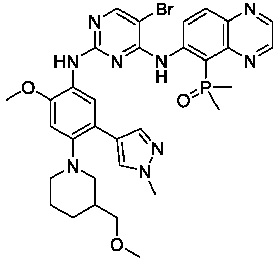 |
| **163** | 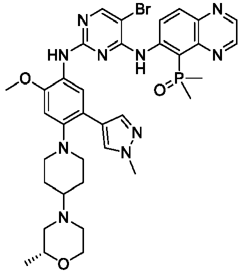 |
| **164** | 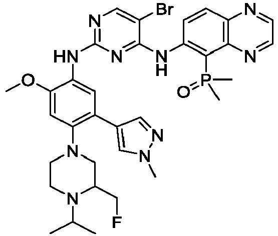 |
| **165** | 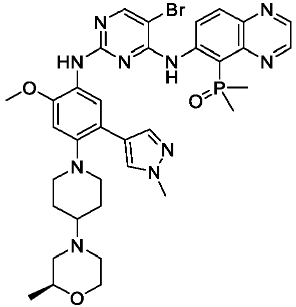 |
| **166** | 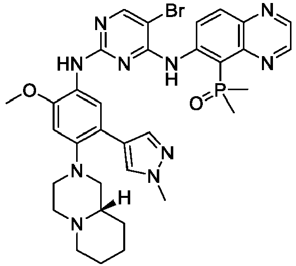 |
| **167** | 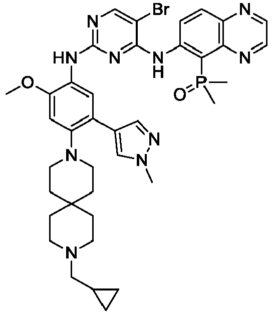 |
| **168** | 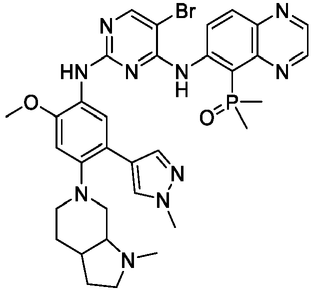 |
| **169** | 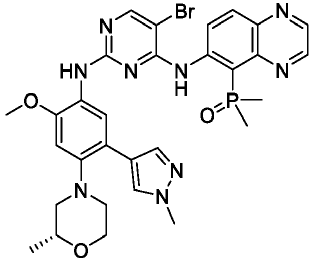 |
| **170** | 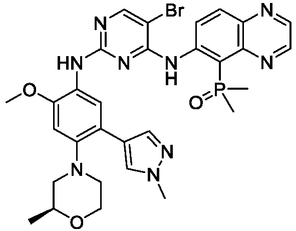 |
| **171** | 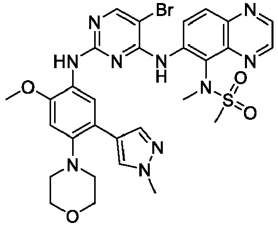 |
| **172** | 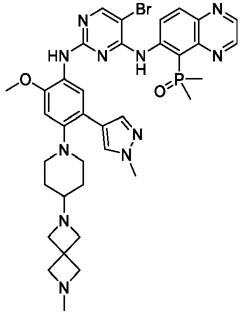 |
| **173** | 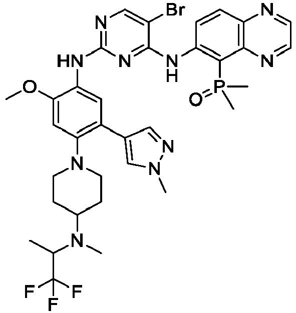 |
| **174** | 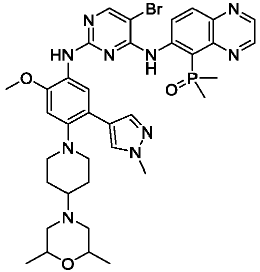 |
| **175** | 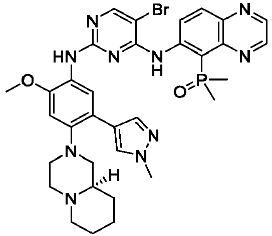 |
| **176** | 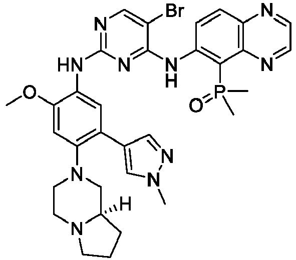 |
| **177** | 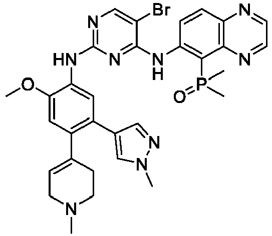 |
| **178** | 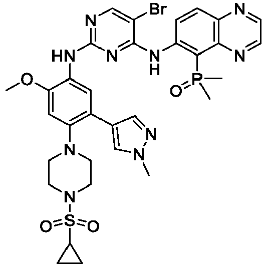 |
| **179** | 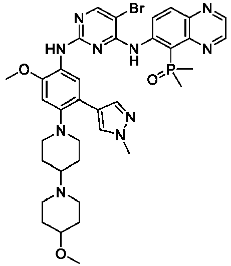 |
| **180** | 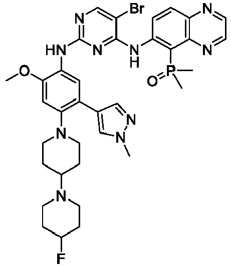 |
| **181** | 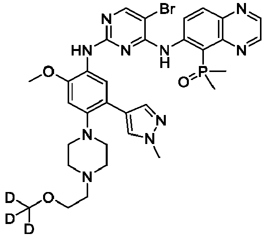 |
| **182** | 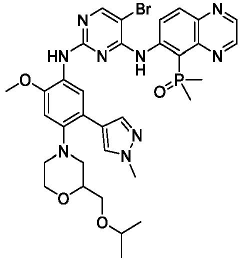 |
| **183** | 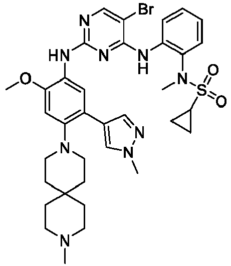 |
| **184** | 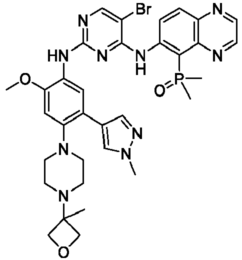 |
| **185** | 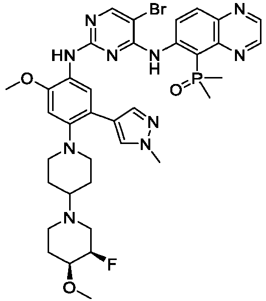 |
| **186** | 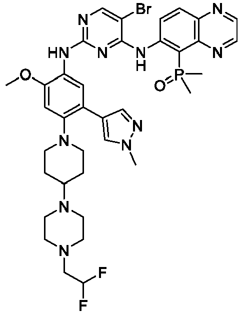 |
| **187** | 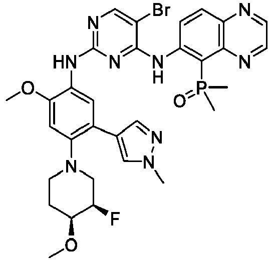 |
| **188** | 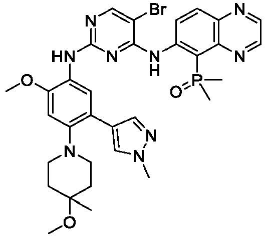 |
| **189** | 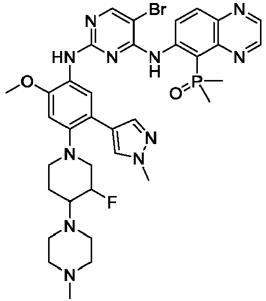 |
| **190** | 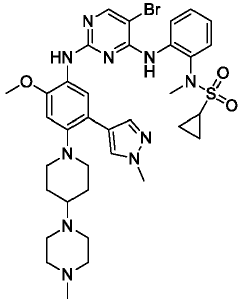 |
| **191** | 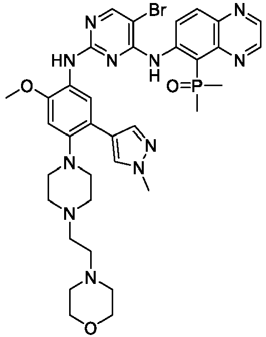 |
| **192** | 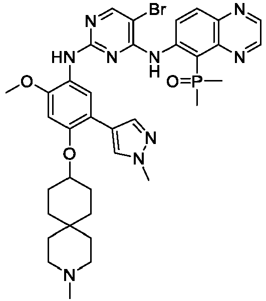 |
| **193** | 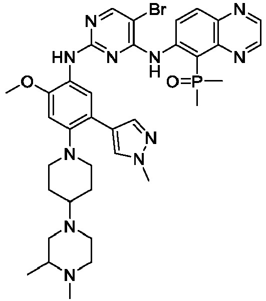 |
| **194** | 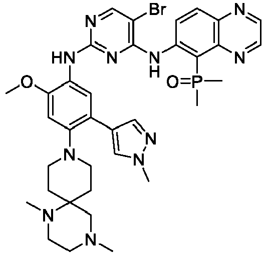 |
| **195** | 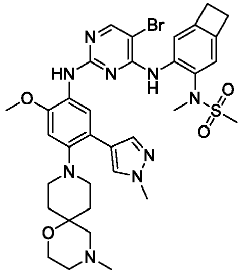 |
| **196** | 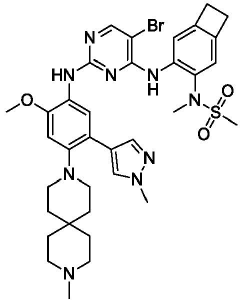 |
| **197** | 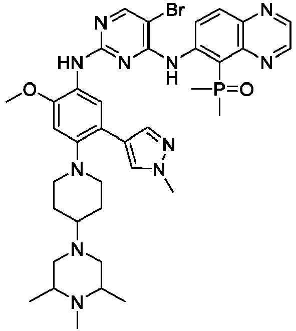 |
| **198** | 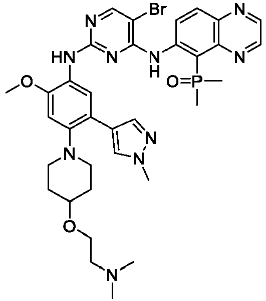 |
| **199** | 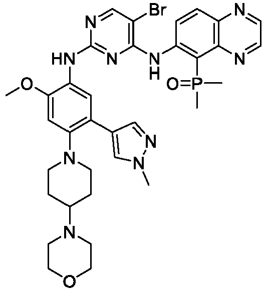 |
| **200** | 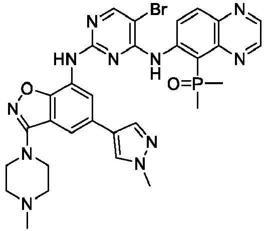 |
| **201** | 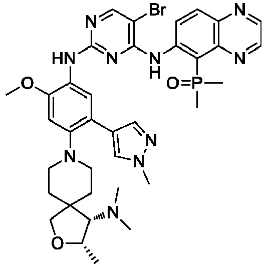 |
| **202** | 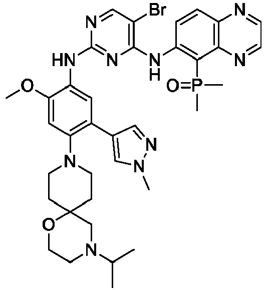 |
| **203** | 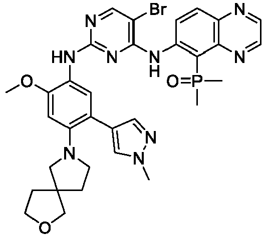 |
| **204** | 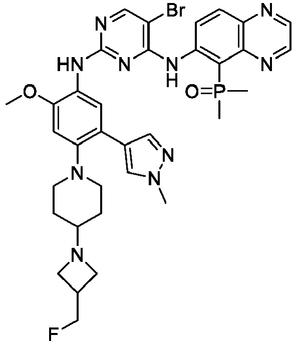 |
| **205** | 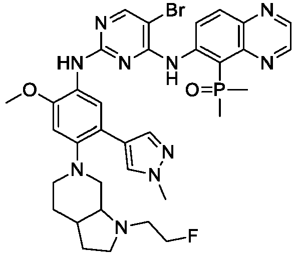 |
| **206** | 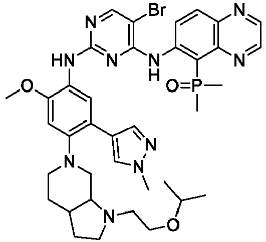 |
| **207** | 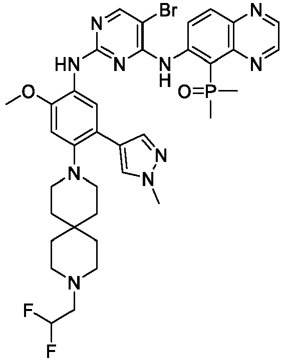 |
| **208** | 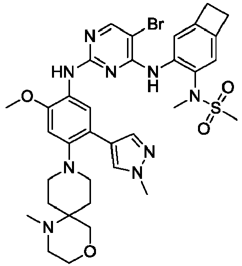 |
| **209** | 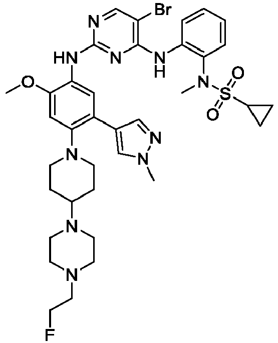 |
| **210** | 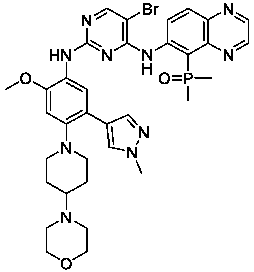 |
| **211** | 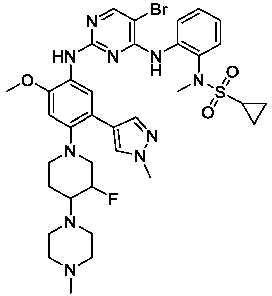 |
| **212** | 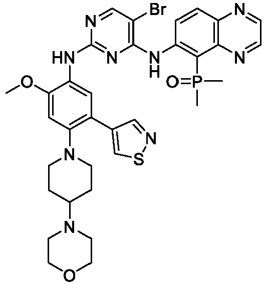 |
| **213** | 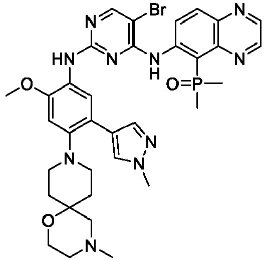 |
| **214** | 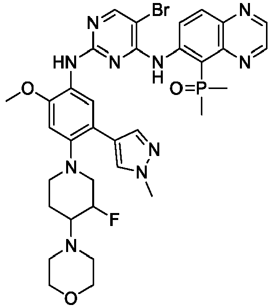 |
| **215** | 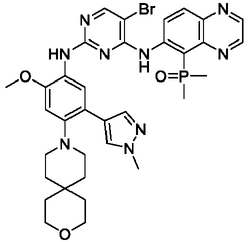 |
| **216** | 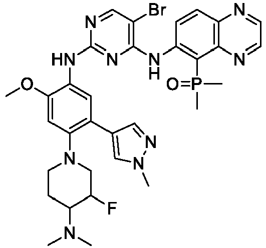 |
| **217** | 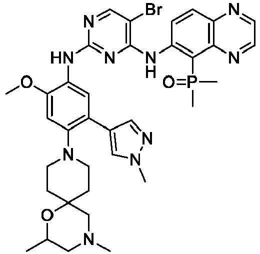 |
| **218** | 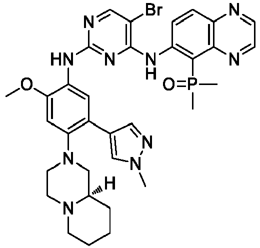 |
| **219** | 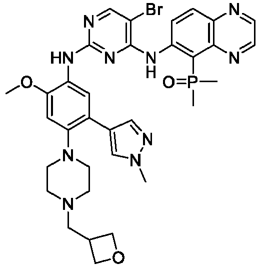 |
| **220** | 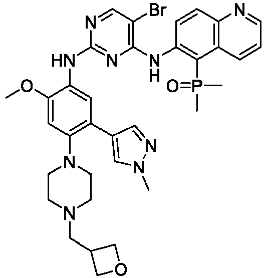 |
| **221** | 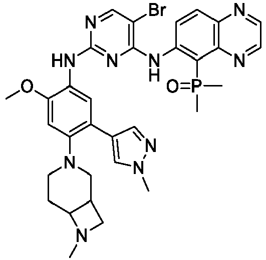 |
| **222** | 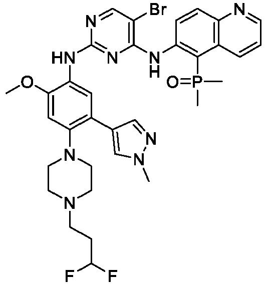 |
| **223** | 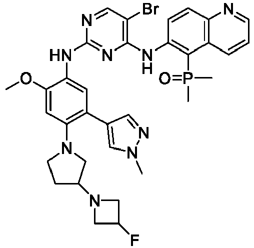 |
| **224** | 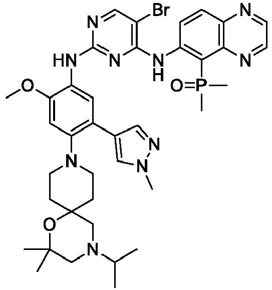 |
| **225** | 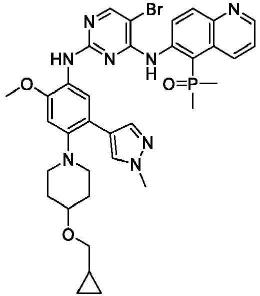 |
| **226** | 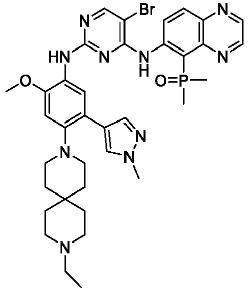 |
| **227** | 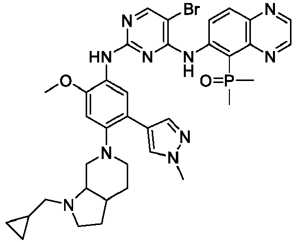 |
| **228** | 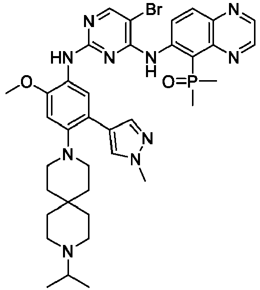 |
| **229** | 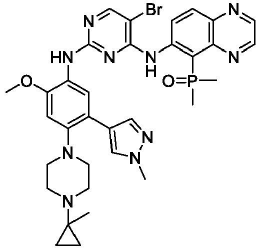 |
| **230** | 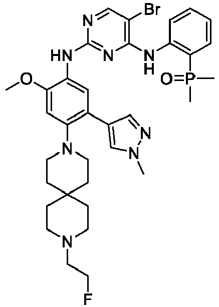 |
| **231** | 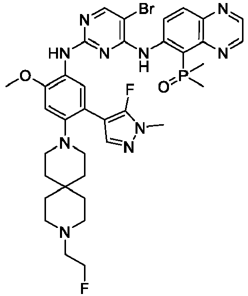 |
| **232** | 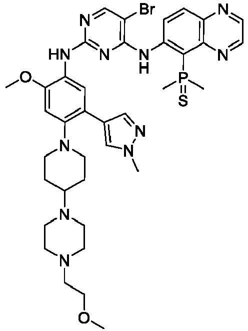 |
| **233** | 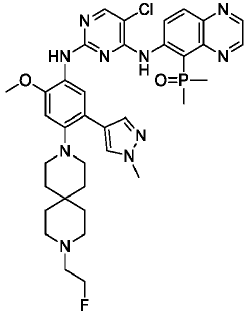 |
| **234a** | 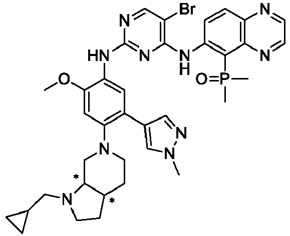 |
| | 3aS,7aS or 3aR,7aR |
| **234b** | 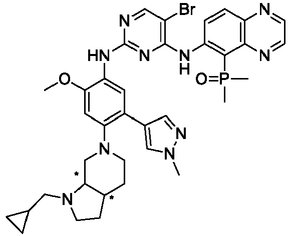 |
| | Enantiomer of 234a |
| | 3aR,7aR *or* 3aS,7aS |
| **235** | 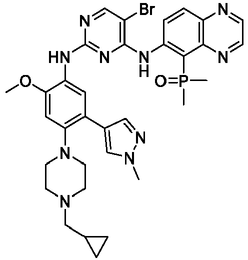 |
| **236** | 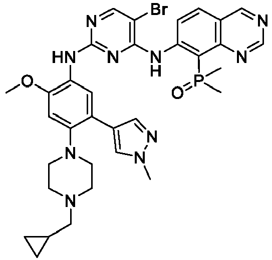 |
| **237** | 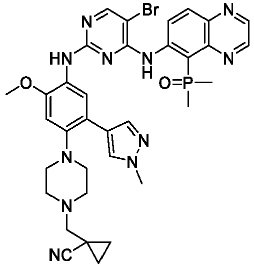 |
| **238** | 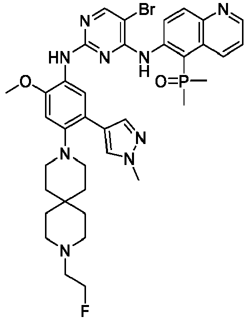 |
| **239** | 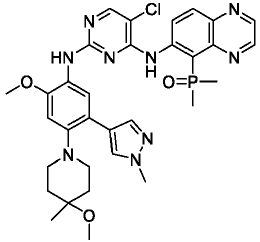 |
| **240** | 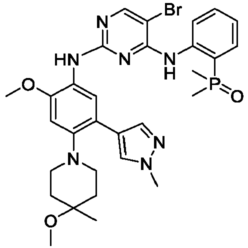 |
| **241** | 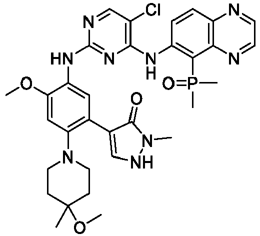 |
| **242** | 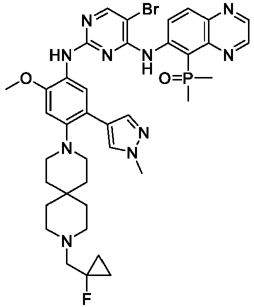 |
| **243** | 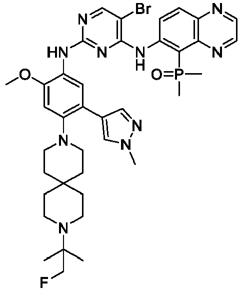 |
| **244** | 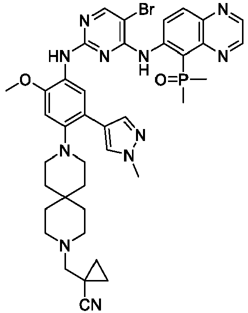 |
| **245** | 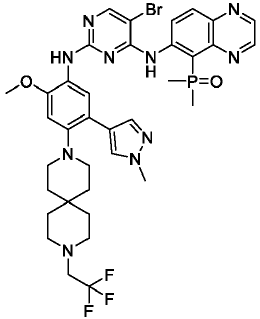 |
| **246** | 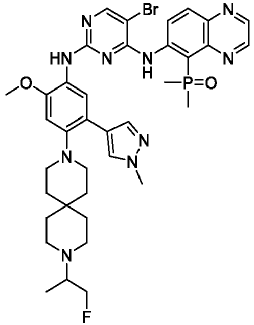 |
| **247** | 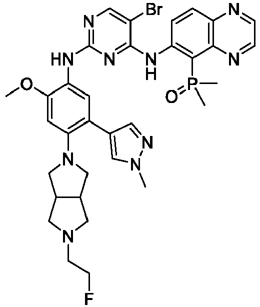 |
| **248** | 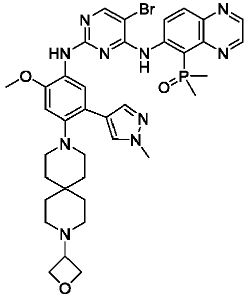 |
| **249** | 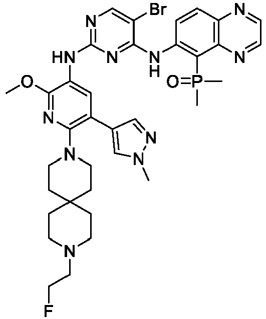 |

The present disclosure also provides a pharmaceutical composition comprising a therapeutically effective amount of the above compound or the pharmaceutically acceptable salt thereof and a pharmaceutically acceptable carrier, diluent and excipient. The pharmaceutical composition may be formulated for specific routes of administration, such as oral administration, parenteral administration, rectal administration, and the like. Oral administration, for example, a tablet, a capsule (including a sustained release or timed release prescription), a pill, a powder, a granule, an elixir, a tincture, a suspension (including a nano-suspension, a micro-suspension, a spray-dried dispersant), a syrup and an emulsion; sublingual administration; buccal administration; parenteral administration, for example, by subcutaneous, intravenous, intramuscular, or intrasternal injection, or infusion techniques (for example, as a sterile injectable aqueous solution or non-aqueous solution or a mixed suspension); nasal administration, including administration to the nasal mucosa, for example, by inhalation spray; topical administration, for example, in the form of a cream or an ointment; or rectal administration, for example, in the form of a suppository. They can be administered alone, but they are usually administered together with a pharmaceutical carrier selected according to the selected route of administration and standard pharmaceutical practice.

"Pharmaceutically acceptable carrier" refers to a medium generally acceptable in the art for delivering a biologically active agent to animals, particularly mammals, including, for example, an adjuvant, an excipient or a vehicle, such as a diluent, a preservative, a filler, a flow regulator, a disintegrant, a wetting agent, an emulsifier, a suspending agent, a sweetener, a flavor, a fragrance, an antibacterial agent, an antifungal agent, a lubricants agent and a dispersant, according to the mode of administration and the nature of dosage forms. The pharmaceutically acceptable carrier is formulated according to a large number of factors within the purview of those of ordinary skill in the art, including, but not limited to, the type and nature of the active agent formulated, the subjects to which the composition containing the agent is to be administered, the expected route of administration of the composition, and the target therapeutic indication. The pharmaceutically acceptable carriers include both aqueous and non-aqueous media and various solid and semisolid dosage forms. In addition to the active agent, such carriers include many different ingredients and additives, and such additional ingredients included in prescriptions for a variety of reasons (e.g., stabilizing the active agent and an adhesive, etc.) are well known to those of ordinary skill in the art.

As a general guideline, when used for the indicated effects, the daily oral dose of various active ingredients is in the range of about 0.001- about 5000 mg, or about 1-500 mg, or about 1-250 mg, or about 1-150 mg, or about 0.5-100 mg, or about 1-50 mg of active ingredients per day; the most preferred dose intravenously during a constant rate infusion is in the range of about 0.01 to about 10 mg/kg/min. The compounds of the present disclosure may be administered in a single daily dose, or the total daily dose may be administered in divided doses of 2, 3 or 4 times per day.

It is certain that administration regimens for the compounds of the present disclosure may vary depending on known factors, such as the pharmacodynamic characteristics of a specific drug and its mode and route of administration, the species, age, sex, health, medical condition and weight of the recipient, nature and extent of symptoms, types of coexisting treatments, frequency of treatments, routes of administration, renal and hepatic function of the patient, and desired effects. A therapeutically effective dose of the compound, a pharmaceutical composition or a combination thereof depends on the species, weight, age and individual condition of the subject, the disorder or disease being treated or its severity. A physician, clinician or veterinarian of ordinary skill may readily determine the effective amount of each active ingredient required to prevent, treat or inhibit the progression of a disorder or disease.

The present disclosure also provides use of the above compound or the pharmaceutically acceptable salt thereof or the above pharmaceutical composition in the manufacture of a medicament for the treatment of cancer.

Epidermal growth factor receptor (EGFR) is widely distributed on the surface of mammalian epithelial cells, fibroblasts, glial cells and other cells. The EGFR signaling pathway plays an important role in physiological processes such as cell growth, proliferation and differentiation. EGFR mutation is also the most common type of mutation in NSCLC patients, especially in Asian populations, which can account for 40 % to 50 %. Therefore, the compounds of the present disclosure can be used to treat cancers caused by high expression of EGFR. The above cancers comprise lymphoma, non-Hodgkin lymphoma, ovarian cancer, cervical cancer, prostate cancer, colorectal cancer, breast cancer, pancreatic cancer, glioma, glioblastoma, melanoma, leukemia, gastric cancer, endometrial cancer, lung cancer, hepatocellular carcinoma, gastric cancer, gastrointestinal stromal tumor (GIST), acute myeloid leukemia (AML), cholangiocarcinoma, renal carcinoma, thyroid carcinoma, anaplastic large cell lymphoma, mesothelioma, multiple myeloma and melanoma.

In some embodiments of the present disclosure, the above cancer is lung cancer.

The present disclosure also provides an intermediate compound represented by formula (V), and the intermediate compound, a stereoisomer thereof or a pharmaceutically acceptable salt thereof is used in the manufacture of the above compound. 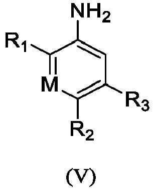 wherein, R₁, R₂, R₃ and M are defined in formula (I).

In some embodiments of the present disclosure, the compound represented by formula (V) is selected from, 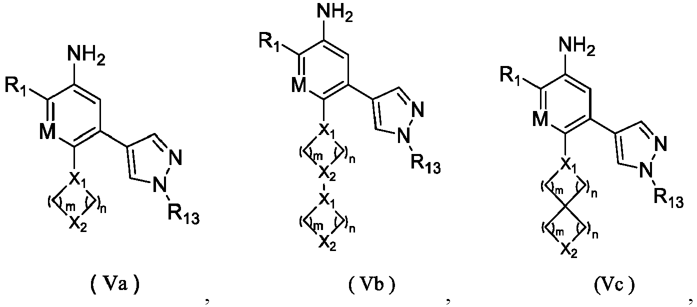 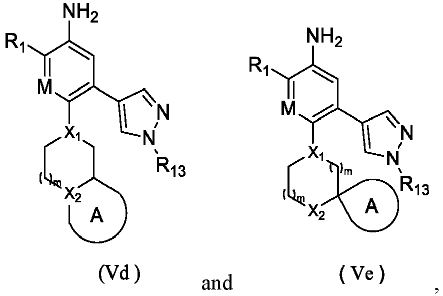 wherein, X₁, X₂, R₁, R₁₃, M, ring A, m and n are defined in the above formulas (IIIa)-(IIIe).

In some embodiments of the present disclosure, the compound represented by formula (V) is selected from, 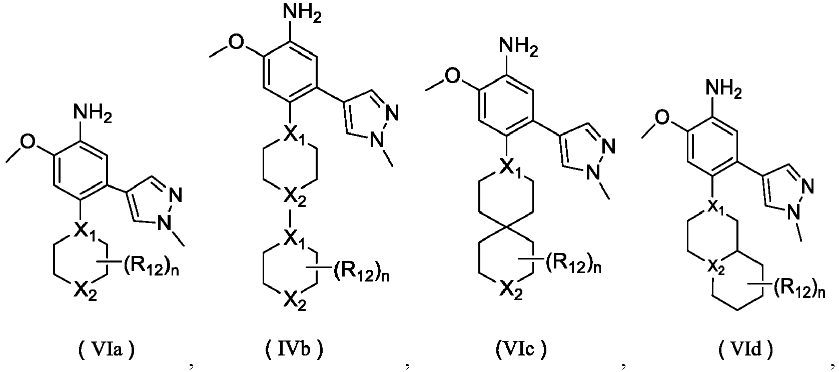 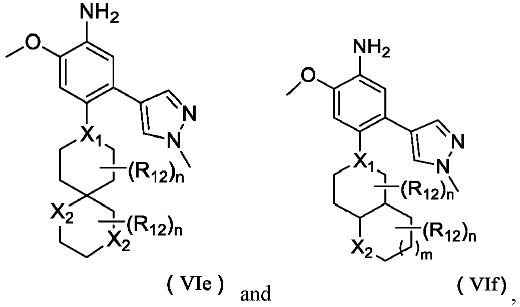 wherein X₁, X₂, R₁₂ and n are defined in formula (II).

In some embodiments of the present disclosure, the compound represented by formula (V) is selected from,

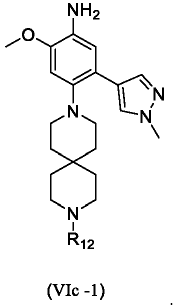

### Technical effect:

The compounds of the present disclosure have a good inhibitory effect on EGFR (L858R/T790M/C797S) kinase, a weak inhibitory effect on wild-type EGFR kinase, and good selectivity.

The compounds of the present disclosure have a good inhibitory effect on the cell proliferation of Ba/F3 Dell9/T790M/C797S EGFR triple mutant cell line and Ba/F3 L858R/T790M/C797S EGFR triple mutant cell line; showing a weak inhibitory effect on EGFR wild-type cell line A431, and good selectivity.

### Description and definitions:

Unless otherwise specified, the following terms and phrases when used herein have the following meanings. A specific term or phrase should not be considered indefinite or unclear in the absence of a particular definition, but should be understood in the ordinary sense.

The term "pharmaceutically acceptable" refers to those compounds, materials, compositions and/or dosage forms, which are suitable for use in contact with human and animal tissues within the scope of reasonable medical judgment, without excessive toxicity, irritation, allergic reaction or other problems or complications, and is commensurate with a reasonable benefit/risk ratio.

The term "pharmaceutically acceptable salt" refers to a derivative prepared by the compound of the present disclosure with a relatively nontoxic acid or base. These salts may be prepared during the synthesis, separation and purification of the compound, or the free form of the purified compound may be used alone to react with a suitable acid or base. When the compound contains a relatively acidic functional group, the compound reacts with alkali metal, alkaline earth metal hydroxide or organic amine to obtain an alkali addition salt, including cations based on alkali metals and alkaline earth metals, such as sodium, lithium, potassium, calcium, magnesium, etc., and non-toxic ammonium, quaternary ammonium and amine cations, including but not limited to ammonium, tetramethylammonium, tetraethylammonium, methylamine, dimethylamine, trimethylamine, triethylamine, ethylamine, etc. Salts of amino acids, such as arginine, gluconate, galacturonate, etc., are also covered. When the compound contains a relatively basic functional group, the compound reacts with an organic acid or an inorganic acid to obtain an acid addition salt, including an inorganic acid salt, such as hydrochloride, hydrobromide, nitrate, carbonate, bicarbonate, phosphate, monohydrogen phosphate, dihydrogen phosphate, sulfate, bisulfate, hydroiodide, hydrobromide, metaphosphate, and pyrophosphate; the organic acid salts include, for example, acetate, propionate, caprylate, isobutyrate, oxalate, malonate, succinate, suberate, sebacate, fumarate, maleate, mandelate, benzoate, chlorobenzoate, methylbenzoate, dinitrobenzoate, naphthalate, benzenesulfonate, toluenesulfonate, phenylacetate, citrate, lactate, maleate, tartrate, mesylate, etc.

The compounds provided by the present disclosure also include the form of prodrugs, which means that the compounds which are rapidly converted *in vivo* to the parent compound of the above formula are converted into the compounds of the present disclosure in the *in vivo* or *in vitro* environment by chemical or biochemical methods, such as hydrolysis in blood.

The compounds of the present disclosure may exist in unsolvated as well as solvated forms, and the solvated form includes a hydrate form. In general, the solvated form is equivalent to the unsolvated form, which is also encompassed within the scope of the present disclosure.

The compounds of the present disclosure have geometric isomers as well as stereoisomers, such as *cis-trans* isomers, enantiomers, diastereoisomers, and racemic mixtures thereof, and other mixtures, all of which are within the scope of the present disclosure.

The term "enantiomer" refers to stereoisomers that are mirror images of each other.

The term "diastereomer" refers to stereoisomers in which the molecules have two or more chiral centers and the relationship between the molecules is not mirror images.

The term *"cis-trans* isomer" refers to a configuration in which a double bond or a single bond of a ring-forming carbon atom in a molecule cannot rotate freely.

Unless otherwise specified, the absolute configuration of a stereogenic center is represented by a wedged solid bond 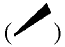 and a wedged dashed bond 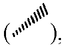 and the relative configuration of a stereogenic center is represented by a straight solid bond 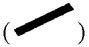 and a straight dashed bond 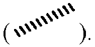 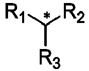
indicates that the carbon atom is a chiral carbon atom, and the structure represents an optically pure compound in which the stereo configuration of the carbon atom is (*R*) configuration or (S) configuration and a mixture thereof, and the ratio of the mixture may be 1:1 or other ratios. For example, 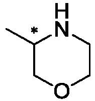 represents that the structure may be 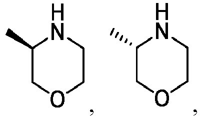 or a mixture of the two, and when the ratio of the mixture is 1:1, the structure is a racemic compound;
when a molecular structure involves a chiral atom, but it is represented by an ordinary carbon-carbon bond 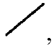 and the chiral atom is not marked, usually, the molecule is considered to be a racemate, for example, 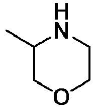 represents that the molecule is a racemate. 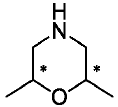
represents that the compound may be *cis*-2,6-dimethylmorpholine or *trans-2,6-*dimethylmorpholine or a mixture of the two. *trans*-2,6-Dimethylmorpholine is an equivalent mixture of 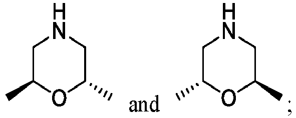 and cis-2,6-dimethylmorpholine is a meso compound;
stereoisomers of the compounds of the present disclosure may be prepared by chiral synthesis or chiral reagents or other conventional techniques. For example, an enantiomer of a certain compound of the present disclosure may be prepared by an asymmetric catalysis technique or a chiral auxiliary derivatization technique. Alternatively, compounds with a single stereo configuration may be obtained from a mixture by a chiral resolution technique. Alternatively, it can be prepared directly from chiral starting materials. Separation of optically pure compounds in the present disclosure is usually accomplished by preparative chromatography, and a chiral chromatographic column is used to achieve the purpose of separating chiral compounds.

In general, when an optically pure compound participates in the reaction as a starting material, the stereo configuration of the chiral atoms in its structure will also be transferred. Without special circumstances, its configuration is considered to remain unchanged in the reaction, that is, the product has the same configuration as the starting material. When an optically pure mixture participates in the reaction, the resulting compound is also an optically pure corresponding mixture. For example, (R)-3-methylmorpholine participates in the reaction as a starting material, and the obtained compound is also in R configuration; *trans*-2,6-dimethylmorpholine participates in the reaction as a starting material, and the obtained compound is a racemate of 2S, 6S and 2R, 6R configuration.

The term "optically pure" or "enriched in enantiomers" refer to the content of the isomer or enantiomer is greater than or equal to 60 %, or greater than or equal to 70 %, or greater than or equal to 80 %, or greater than or equal to 90 %, or greater than or equal to 95 %, or greater than or equal to 96 %, or greater than or equal to 97 %, or greater than or equal to 98 %, or greater than or equal to 99 %, or greater than or equal to 99.5 %, or greater than or equal to 99.6 %, or greater than or equal to 99.7 %, or greater than or equal to 99.8 %, or greater than or equal to 99.9 %.

The absolute stereo configuration of a compound may be confirmed by conventional technical means in the art. For example, a single crystal X-ray diffraction method may also confirm the absolute configuration of the compound by the chiral structure of the raw material and the reaction mechanism of asymmetric synthesis. Compounds marked herein as "absolute configuration not determined" are typically split from racemic compounds into single isomers by chiral preparative HPLC or SFC, which are then characterized and tested.

For example, the following formula represents that compounds 155a and 155b are split from compound 135 and are enantiomers of each other, but the absolute stereo configurations of 155a and 155b are not determined.

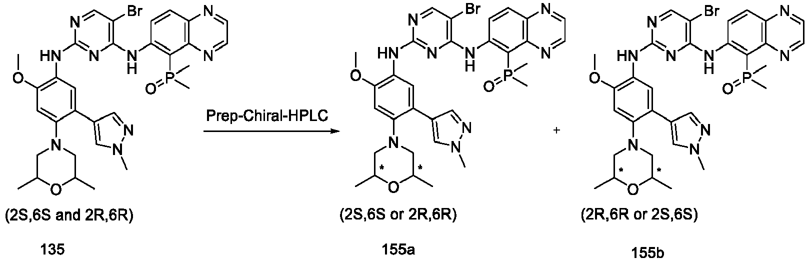

The present disclosure also includes isotope-labeled compounds. Included are isotopes of hydrogen, carbon, nitrogen, oxygen, phosphorus, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F and ³⁶Cl, respectively. Compounds of the present disclosure containing the above isotopes and/or other isotopes of other atoms are within the scope of the present disclosure.

The term "pharmaceutically acceptable carrier" refers to a medium generally acceptable in the art for delivering a biologically active agent to animals, particularly mammals, including, for example, an adjuvant, an excipient or a vehicle, such as a diluent, a preservative, a filler, a flow regulator, a disintegrant, a wetting agent, an emulsifier, a suspending agent, a sweetener, a flavor, a fragrance, an antibacterial agent, an antifungal agent, a lubricants agent and a dispersant, according to the mode of administration and the nature of dosage forms. The pharmaceutically acceptable carrier is formulated according to a large number of factors within the purview of those of ordinary skill in the art, including, but not limited to, the type and nature of the active agent formulated, the subjects to which the composition containing the agent is to be administered, the expected route of administration of the composition, and the target therapeutic indication. The pharmaceutically acceptable carriers include both aqueous and non-aqueous media and various solid and semisolid dosage forms. In addition to the active agent, such carriers include many different ingredients and additives, and such additional ingredients included in prescriptions for a variety of reasons (e.g., stabilizing the active agent and an adhesive, etc.) are well known to those of ordinary skill in the art.

The term "excipient" generally refers to a carrier, diluent and/or medium required to formulate an effective pharmaceutical composition.

The term "prophylactically or therapeutically effective amount" refers to a compound or a pharmaceutically acceptable salt thereof of the present disclosure, which refers to a compound in an amount sufficient to treat a disorder at a reasonable effect/risk ratio suitable for any medical treatment and/or prophylaxis. It should be recognized that the total daily dose of the compound represented by formula I, or the pharmaceutically acceptable salt thereof and the composition thereof in the present disclosure, is required to be determined by the attending physician within the scope of reliable medical judgment. For any specific patient, the specific therapeutically effective dose level depends on a variety of factors, including a disorder being treated and the severity of the disorder; activity of a specific compound used; a specific composition used; age, weight, general health status, sex and diet of the patient; administration time, administration route and excretion rate of the specific compound used; duration of treatment; drugs used in combination or simultaneously with the specific compounds used; and similar factors known in the medical field. For example, it is the practice in the art to start with a dose of the compound lower than the level required to obtain the desired therapeutic effect and gradually increase the dose until the desired effect is obtained. Generally speaking, the dose of the compound represented by formula I or the pharmaceutically acceptable salt thereof in the present disclosure for mammals, especially humans, may range from 0.001 to 1000 mg/kg weight/day, for example, from 0.01 to 100 mg/kg weight/day, for example, from 0.01 to 10 mg/kg weight/day.

The term "optionally substituted" means an atom can be substituted by a substituent or not, unless otherwise specified, the type and number of the substituent may be arbitrary as long as being chemically achievable. For example, the term "optionally substituted by one or more R₀" means that an atom may or may not be substituted by one or more R₀.

When any variable (such as R₁₂) occurs in the constitution or structure of the compound more than once, the definition of the variable at each occurrence is independent. For example, if a group is substituted by 0-2 R₁₂, the group can be optionally substituted by up to two R₁₂, wherein the definition of R₁₂ at each occurrence is independent.

When the number of a linking group is 0, such as -O(CH₂)ₙCH₃, n=0, it means that the linking group is a single bond, i.e., -OCH₃-.

When the bond of a substituent may be cross-connected to two atoms on a ring, the substituent may be bonded to any atom on the ring. For example, the structural moiety 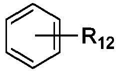 means that the substituent R₁₂ may be substituted at any position a benzene ring.

When a listed substituent does not indicate through which atom it is attached to a compound included in the general formula but not specifically mentioned, such substituent may be bonded through any of its atoms. For example, pyrazole as a substituent means that any carbon atom on a pyrazole ring is connected to a substituted group; when 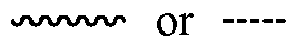 appears in the structure, it means that the atom is a bonded atom, for example, 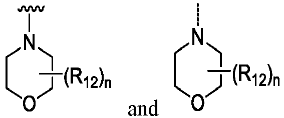 both mean that the N atom on a morpholine ring is a bonded atom.

Unless otherwise specified, "ring" refers to saturated, partially saturated or unsaturated monocyclic and polycyclic rings, and the "polycyclic rings" include a linked ring, a spiro ring, a fused ring and a bridged ring. Representative "rings" include substituted or unsubstituted cycloalkyl, heterocycloalkyl, cycloalkenyl, heterocycloalkenyl, cycloalkynyl, heterocycloalkynyl, aryl, or heteroaryl. The term "hetero" refers to substituted or unsubstituted heteroatoms and oxidized forms of heteroatoms, the heteroatoms being generally selected from N, O, S, and the oxidized forms generally including NO, SO and S(O)₂. Nitrogen atoms may be substituted, namely NR (R is H or other substituents defined herein). The number of atoms on the ring is usually defined as the number of members of the ring. For example, "3-14-membered heterocycloalkyl" refers to a mono-heterocyclic ring, a linked heterocyclic ring, a spiro heterocyclic ring, a fused heterocyclic ring or a bridged heterocyclic ring formed by 3-14 atoms arranged around, and each ring optionally contains 1-3 heteroatoms, namely N, O, S, NO, SO, S(O)₂ or NR.

Unless otherwise specified, "cycloalkyl" refers to a saturated monocyclic or polycyclic hydrocarbon group, and the polycyclic hydrocarbon group includes a spiro cyclyl, a fused cyclyl or a bridged cyclyl. When the bridge atom in the bridged cyclyl is zero, the bridged cyclyl is equivalent to the fused cyclyl. 3-8-membered cycloalkyl is preferably 3-8-membered monocycloalkyl, more preferably 3-6-membered monocycloalkyl, and examples of these monocycloalkyl include, but are not limited to, cyclopropyl, cyclobutyl, cyclopentyl, cyclohexyl, cycloheptyl, and cyclooctyl; "spiro cyclyl" refers to a polycyclic hydrocarbon group in which a single carbon atom is shared between monocyclic rings. The spiro cyclyl is preferably a 5-13-membered spiro cyclyl, a 6-12-membered spiro cyclyl, or a 7-11-membered spiro cyclyl, and the 6-12-membered spiro cyclyl refers to a hydrocarbon group with a spirocyclic skeletal structure consisting of 6-12 atoms. Examples of the spiro cyclyl include, but are not limited to, spiro[2.2]pentyl, spiro[2.3]hexyl, spiro[2.4]heptyl, spiro[2.5]octyl, spiro[2.6]nonyl, spiro[3.3]heptyl, spiro[3.4]octyl, spiro[3.5]nonyl, spiro[3.6]decyl, spiro[4.4]nonyl, spiro[4.5]decyl, spiro[4.6]undecyl, spiro[5.5]undecyl, spiro[5.6]dodecyl, spiro[6.6]tridecyl, spiro[6.7]tetradecyl; "bridged cyclyl" refers to a polycyclic hydrocarbon group sharing two or more carbon atoms. The bridged cyclyl is preferably a 4-13-membered bridged cyclyl, a 5-12-membered bridged cyclyl, a 6-12-membered bridged cyclyl, a 6-11-membered bridged cyclyl, and a 7-11-membered bridged cyclyl. Examples of the bridged cyclyl include, but are not limited to, bicyclo[3.1.0]hexyl, bicyclo[3.2.0]heptyl, bicyclo[3.3.0]octyl, bicyclo[4.1.0]heptyl, bicyclo[4.1.0]heptyl, bicyclo[4.2.0]octyl, bicyclo[4.3.0]nonyl, bicyclo[4.4.0]decyl and bicyclo[3.2.1]octyl.

Unless otherwise specified, "heterocycloalkyl" refers to monoheterocycloalkyl and polyheterocycloalkyl containing a certain number of heteroatoms in the ring, and the heteroatoms are generally selected from N, O, S, NO, SO, S(O)₂, and NR. Polyheterocycloalkyl includes spiro heterocyclyl, fused heterocyclyl or bridged heterocyclyl. When the bridge atom in the bridged heterocyclyl is zero, the bridged heterocyclyl is equivalent to fused heterocyclyl. 3-8-membered heterocycloalkyl is preferably 3-8-membered monoheterocycloalkyl, more preferably 3-6-membered monoheterocycloalkyl. Examples of these monoheterocycloalkyl include, but are not limited to, oxiranyl, tetrahydropyrrolyl, piperidinyl, piperazinyl, morpholinyl, tetrahydrofuranyl, tetrahydrothienyl, tetrahydropyranyl, 1,3-dioxolanyl, 1,4-dioxanyl and the like. "Spiroheterocyclyl" refers to a spiro cyclyl in which one or more carbon atoms in the spirocyclic skeleton structure are substituted by heteroatoms selected from N, O, and S. The spiroheterocyclyl is preferably a 5-13-membered spiroheterocyclyl, a 6-12-membered spiroheterocyclyl, or a 7-11-membered spiroheterocyclyl. Examples of spiroheterocyclyl include, but are not limited to, 2-oxa-7-azaspiro[5.3]nonan-7-yl, 2-oxa-7-azaspiro[4.4]nonan-7-yl, 2-oxa-6-azaspiro[3.3]heptan-6-yl, 2-oxa-8-azaspiro[4.5]decan-8-yl, 1,4,9-triazaspiro[5.5]undecan-9-yl, 3-oxa-9-azaspiro[5.5]undecan-9-yl, 2,6-diazaspiro[3.3]heptan-2-yl, 2,7-diazaspiro[5.3]nonan-7-yl, 2,7-dioxaspiro[5.3]nonyl, 3,9-diazaspiro[5.5]undecan-3-yl, 1-oxa-4,9-diazaspiro[5.5]undecan-9-yl, 1-oxa-4,8-diazaspiro[5.4]decan-8-yl, 3-azaspiro[5.5]undecan-3-yl, 7-azaspiro[3.5]decan-7-yl, 1-oxa-4,9-diazaspiro[5.5]undecan-4-yl, 6-oxa-2,9-diazaspiro[4.5]decan-9-yl, 9-oxa-2,6-diazaspiro[4.5]decan-6-yl, 3-azaspiro[5.5]undecan-3-yl, 4-oxa-1,9-diazaspiro[5.5]undecan-9-yl; "bridged heterocyclyl" refers to a bridged cyclyl in which one or more carbon atoms forming the skeleton of the bridged ring are substituted by heteroatoms, and the heteroatoms are selected from N, O and S. Preferably, the bridged heterocyclyl is selected from the following bridged cyclyl in which the carbon atoms of the bridged ring skeleton are substituted by 1-3 heteroatoms selected from N, O and S: bicyclo[3.1.0]hexyl, bicyclo[3.2.0]heptyl, bicyclo[3.3.0]octyl, bicyclo[4.1.0]heptyl, bicyclo[4.2.0]octyl, bicyclo[4.3.0]nonyl, bicyclo[4.4.0]decyl and bicyclo[3.2.1]octyl. Examples of bridged heterocyclyl include, but are limited to, 1,4-diazabicyclo[4.4.0]decan-4-yl, 1,4-diazabicyclo[4.3.0]-nonan-4-yl, 8-oxa-1,4-diazabicyclo[4.4.0]decan-4-yl, 1,4-diazabicyclo[4.4.0]decan-4-yl, 4,7-diazabicyclo[4.3.0]nonan- 4-yl, 2-oxa-5-azabicyclo[2.2.1]heptan-5-yl, 3,7-diazabicyclo[4.3.0]nonan-3-yl, 3,7-diazabicyclo[3.3.0]octan-3-yl, 3,7-diazabicyclo[4.4.0]decan-3-yl, 3,6-diazabicyclo[4.3.0]nonan-3-yl, 3,6-diazabicyclo[4.4.0]decan-3-yl, 3,6,9-triazabicyclo[4.4.0]decan-3-yl, 3,7-diazabicyclo[4.2.0]octan-3-yl and 3,7-diazabicyclo[3.3.0]octan-3-yl.

Cycloalkyl, heterocycloalkyl, aryl, and heteroaryl may all be fused with a benzene ring to form a corresponding polycyclic structure. For example, in the structure 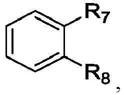 "R₇ and R₈ may be cyclized to C₄₋₆ cycloalkyl" means that the structure may be 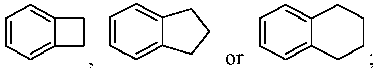 examples of "R₇ and R₈ may be cyclized to 4-6-membered heterocycloalkyl" include, but are not limited to 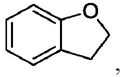 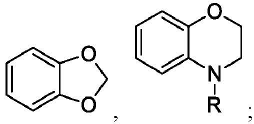 "R₇ and R₈ may be cyclized to C₅₋₆ aryl" means that the structure may be 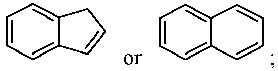 examples of "R₇ and R₈ may be cyclized to 5-7-membered heteroaryl" include, but are not limited to,

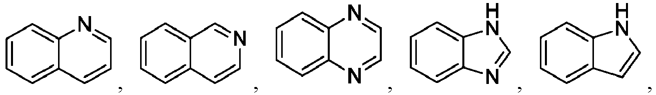

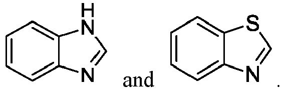

Unless otherwise specified, the term "aryl" refers to an unsaturated, usually aromatic, hydrocarbon group, which may be a single ring or multiple rings fused together. Examples of aryl include, but are not limited to, phenyl and naphthyl.

Unless otherwise specified, the term "heteroaryl" means a stable monocyclic or polycyclic aromatic hydrocarbon containing at least one heteroatom (N, O, S, NO, SO, S(O)₂ or NR.). 5-12-membered heteroaryl is preferred, and 5-, 6-, 7-membered monocyclic or bicyclic or 7-, 8-, 9- or 10-membered bicyclic heteroaryl is more preferred; carbon atoms and 1, 2, 3 or 4 ring heteroatoms independently selected from N, O and S are preferably included. Examples of heteroaryl include, but are not limited to, pyrrolyl, pyrazolyl, imidazolyl, pyrazinyl, oxazolyl, benzoxazolyl, thiazolyl, furyl, thienyl, pyridyl, pyrimidinyl, benzothiazolyl, purinyl, benzimidazolyl, indolyl, isoquinolinyl, quinoxalinyl and quinolinyl.

Unless otherwise specified, the term "alkyl" is used to refer to a linear or branched saturated hydrocarbyl. C₁₋₆ alkyl is preferred, and C₁₋₃ alkyl is more preferred. Examples of alkyl include, but are not limited to, methyl, ethyl, n-propyl, isopropyl, butyl, isobutyl, pentyl, isopentyl, neopentyl, n-hexyl, etc.

Unless otherwise specified, the term "heteroalkyl" refers to alkyl in which one or more carbon atoms are substituted by heteroatoms selected from B, O, N and S, wherein nitrogen and sulfur atoms are optionally oxidized and nitrogen heteroatoms are optionally quaternized, including but not limited to "alkoxy", "alkylamino" and "alkylthio", etc. Examples of "heteroalkyl" include, but are not limited to - OCH₃, -OCH₂CH₃, -OCH₂CH₂CH₃, -OCH(CH₃)₂, -N(CH₃)₂, -CH₂-CH₂-O-CH₃, -CH₂-CH₂-NH-CH₃, -CH₂-CH₂-N(CH₃)-CH₃, -CH₂-S-CH₂-CH₃, -CH₂-CH₂, -S(O)-CH₃, -S(O)₂-CH₃, -CH₂-CH₂-S(O)₂-CH₃, -CH=CHO-CH₃, -CH₂-CH=N-OCH₃, and -O=CH-N(CH₃)-CH₃, etc.

Unless otherwise specified, "alkenyl" refers to alkyl having one or more carbon-carbon double bonds. C₂₋₈ alkenyl is preferred, and examples of alkenyl include, but are not limited to, vinyl, propenyl, butenyl, pentenyl, hexenyl, etc.

Unless otherwise specified, "alkynyl" refers to alkyl having one or more carbon-carbon triple bonds. C₂₋₈ alkynyl is preferred, and examples of alkynyl include, but are not limited to, ethynyl, propynyl, butynyl, pentynyl, etc.

Unless otherwise specified, the term "halogen" refers to fluorine, chlorine, bromine or iodine atom.

Unless otherwise specified, the term "haloalkyl" refers to alkyl in which one or more hydrogen atoms are substituted by halogen atoms. C₁₋₆ haloalkyl is preferred, and examples of haloalkyl include but are not limited to fluoromethyl, difluoromethyl, trifluoromethyl, trichloromethyl, tribromomethyl, 2,2,2-trifluoroethyl, 2,2,2 trichloroethyl, etc.

Unless otherwise specified, the term "alkoxy" refers to alkyl connected by an oxygen bridge, that is, a group obtained by substituting hydrogen atoms in hydroxyl with alkyl. C₁₋₆ alkoxy is preferred, and C₁₋₃ alkoxy is more preferred. Examples of alkoxy include, but are not limited to, methoxy, ethoxy, n-propoxy, isopropoxy, n-butoxy, sec-butoxy, tert-butoxy, n-pentoxy, neopentoxy and n-hexyloxy.

Unless otherwise specified, the term "cycloalkyloxy" refers to cycloalkyl connected by an oxygen bridge, that is, a group obtained by substituting hydrogen atoms in hydroxyl with cycloalkyl. The cycloalkyloxy is preferably 3-7-membered, 4-7-membered, or 5-7-membered cycloalkoxy. Examples of cycloalkyloxy include, but are not limited to, cyclopropyloxy, cyclobutyloxy, cyclopentyloxy and cyclohexyloxy.

Unless otherwise specified, the term "haloalkoxy" refers to alkoxy in which one or more hydrogen atoms are substituted by halogen atoms. Examples of haloalkoxy include, but are not limited to, trifluoromethoxy, trichloromethoxy, 2,2,2-trifluoroethoxy, and 2,2,2-trichloroethoxy.

Unless otherwise specified, the term "amido" refers to a group with a structure of 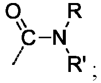 "phosphonyl" or "phosphine oxide" refers to a group with a structure of 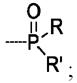 "sulfonyl" refers to a group with a structure of 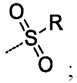 sulfonylamino refers to a group with a structure of 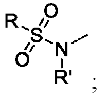 aminosulfonyl refers to a group with a structure of

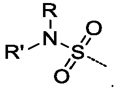

Unless otherwise specified, the term "cycloalkenyl" refers to a stable monocyclic or polycyclic hydrocarbon group containing one or more unsaturated carbon-carbon double bonds in the ring. Examples of such cycloalkenyl include, but are not limited to, cyclopropenyl, cyclobutenyl, cyclopentenyl, cyclohexenyl, 1,3-cyclohexadienyl, 1,4-cyclohexadienyl, etc.

Unless otherwise specified, the term "heterocycloalkenyl" refers to cycloalkenyl containing 1-3 heteroatoms in the ring.

Unless otherwise specified, the term "cycloalkylalkyl" refers to a group with a structure of 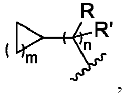 m and n are integers according to the definitions herein, and R and R' are the groups defined herein.

Unless otherwise specified, the term "cycloalkylalkoxy" refers to a group with a structure of 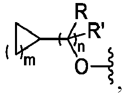 m and n are integers according to the definitions herein, and R and R' are the groups defined herein.

Unless otherwise specified, the term "hydroxyalkyl" refers to a group with a structure of 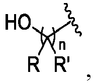 n is an integer according to the definitions herein, and R and R' are the groups defined herein.

Unless otherwise specified, the term "aminocarbonyl" refers to a group with a structure of 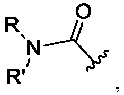 and R and R' are the groups defined herein.

Unless otherwise specified, the term "cycloalkylcarbonyl" refers to a group with a structure of 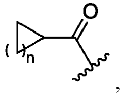 and n is an integer according to the definition herein.

Unless otherwise specified, the term "alkoxy-C₁₋₆ alkyl" refers to a group with a structure of 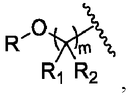 where n is an integer according to the definition herein, and R, R₁ and R₂ are the groups defined herein.

Unless otherwise specified, the group "C₀₋₄ alkyl", C₀ alkyl means that there is no alkyl there and is a bond. For example "-C₀₋₄ alkyl-O-C₁₋₄ alkyl" may be -O-C₀₋₄ alkyl.

Unless otherwise specified, the group "sulfoximino" refers to a -N=S(=O)R_{b}R_{c} or R_{b}N=S(=O)(R_{c})-group, wherein R_{b} and R_{c} are each independently selected from H, -CN, C₁₋₃ alkyl, C₁₋₃ haloalkyl, C₃₋₆ cycloalkyl, C₄₋₆ heterocycloalkyl, C₅₋₁₀ aryl or 5-10-membered heteroaryl.

Specifically, all combinations of the substituent and/or the variant thereof herein are allowed only when such combinations result in a stable compound.

In the embodiments of the present disclosure, the nomenclature of the title compound is converted from the compound structure by means of Chemdraw. If there is any inconsistency between the compound name and the compound structure, it may be determined by synthesizing relevant information and reaction routes; if it cannot be confirmed by other methods, the given structural formula of the compound shall prevail.

The preparation methods of some compounds in the present disclosure refer to the preparation methods of the aforementioned similar compounds. Those skilled in the art should know that when using or referring to the preparation methods cited therein, the feed ratio of reactants, reaction solvent, reaction temperature, etc. may be appropriately adjusted according to the different reactants.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred implementations include but are not limited to the embodiments of the present disclosure.

Abbreviations used in the embodiments of the present disclosure and their corresponding chemical names are as follows:

| **Abbreviation** | **Chemical Name** |
|---|---|
| Me | Methyl |
| Bpin | bis(pinacolato)diboron |
| XPhos Pd G2 | Chloro(2-dicyclohexylphosphino-2,4,6-triisopropyl-1,1-biphenyl)[2-(2-amino-1,1-biphenyl)]palladium(II) |
| NBS | *N*-Bromosuccinimide |
| Davephos | 2-Dicyclohexylphosphino-2'-(*N*,*N-*dimethylamine)-biphenyl |
| Boc | tert-Butoxycarbonyl |
| HATU | 2-(7-Azabenzotriazole)-*N*,*N*,*N*',*N*'-tetramethyluronium hexafluorophosphate |
| DPPA | Diphenylphosphoryl azide |
| t-BuOH | tert-Butanol |
| NIS | *N*-Iodosuccinimide |
| TFA | Trifluoroacetic acid |
| Ac | Acetyl |
| THP | Tetrahydropyran |

### BRIEF DESCRIPTION OF THE DRAWINGS

Fig. 1 is a graph of tumor growth curves of each group of animals in an *in vivo* efficacy research experiment A.
Fig. 2 is a graph of weight curves of each group of animals in the *in vivo* efficacy research experiment A.
Fig. 3 is a graph of tumor growth curves of each group of animals in an *in vivo* efficacy research experiment B.
Fig. 4 is a graph of weight curves of each group of animals in the *in vivo* efficacy research experiment B.

### DETAILED DESCRIPTION OF THE PREFERRED EMBODIMENT

The present disclosure will be specifically described below by way of embodiments, but the scope of the present disclosure is not limited thereto. The present disclosure is described in detail herein, and specific embodiments thereof have also been disclosed. For those skilled in the art, it is obvious that various changes and improvements can be made to the specific embodiments of the present disclosure without departing from the spirit and scope of the present disclosure.

The structures of the compounds of the present disclosure were determined by nuclear magnetic resonance (NMR) or/and liquid chromatography-mass spectrometry (LC-MS). NMR chemical shift (δ) was given in units of parts per million (ppm). NMR was determined using a Bruker AVANCE III HD 400 or a Bruker AVANCE III HD 300 NMR instrument with deuterated dimethyl sulfoxide (DMSO-*d*₆), deuterated methanol (CD₃OD) and deuterated chloroform (CDCl₃) as solvents and tetramethylsilane (TMS) as an internal standard.

Liquid chromatography-mass spectrometry LC-MS was determined using a SHIMADZU LCMS-2020 mass spectrometer (an ion source was electrospray ionization). HPLC was determined using SHIMADZU LC-20 AP _{XR} and SPD-M20A high pressure liquid chromatography.

Thin layer chromatography silica gel plate used Yantai Xinnuo Chemical GF254 silica gel plate. The specification used for TLC was 0.15 mm - 0.20 mm. Column chromatography generally used 200-300 mesh silica gel from Yucheng Chemical as a carrier.

The optical rotation value was detected using the an AutoPol-III instrument.

The starting materials in the embodiments of the present disclosure are known and commercially available, or can be synthesized by using or following methods known in the art.

### Embodiment 1:

### Preparation of (6-((5-bromo-2-((2-methoxy-5-(1-methyl-1H pyrazol-4-yl)-4-morpholinophenyl)amino)pyrimidin-4-yl)amino)quinoxalin-5-yl)-dimethylphosphine oxide (compound 1)

### Step 1:

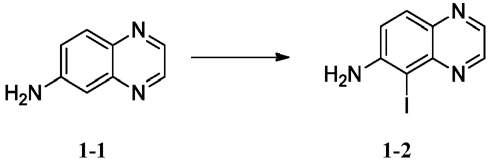

Compound 1-1 (59 g, 406.4 mmol) was dissolved in 600 mL of *N*,*N-*dimethylformamide, and *N-*iodosuccinimide (NIS for short, 100.6 g, 447.1 mmol) was added in batches at room temperature. The reaction system was stirred at room temperature for 1 hour. After LCMS monitoring showed that the raw materials disappeared, water (3000 mL) was added to the reaction solution to quench. The mixture was extracted with ethyl acetate (1000 mL × 3 times), and the organic phases were combined. The organic phases were washed with saturated brine (500 mL × 3 times), then dried over anhydrous sodium sulfate, filtered, and finally concentrated under reduced pressure; the resulting residue was purified by silica gel column chromatography to obtain 50 g of compound 1-2.

MS (ESI) m/z: 272.0 [M + H]⁺.

### Step 2:

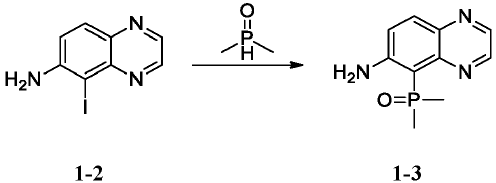

Compound **1-2** (40 g, 147.6 mmol) was dissolved in *N,N-*dimethylformamide (400 mL) at room temperature under nitrogen protection. Then, dimethylphosphine oxide (17.3 g, 221.4 mmol), palladium acetate (3.3 g, 14.7 mmol), 4,5-bis(diphenylphosphino)-9,9-dimethylxanthene (12.8 g, 22.1 mmol) *and N,N-*diisopropylethylamine (38.1 g, 295.1 mmol) were sequentially added to the reaction. The reaction solution was heated to 120 °C and continued to stir for 16 hours.

After LCMS monitoring showed that the raw materials disappeared, the reaction solution was cooled to room temperature, filtered, and the filter cake was washed with ethanol (100 mL × 3 times); the filtrate was concentrated under reduced pressure, and the resulting residue was purified by silica gel column chromatography to obtain 30 g of compound **1-3.**

MS (ESI) m/z: 222.0 [M + H]⁺.

### Step 3:

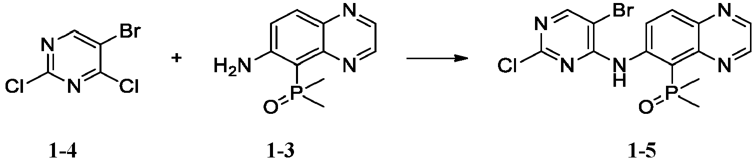

Compound **1-3** (5 g, 22.6 mmol) was dissolved in N,N dimethylformamide (100 mL) at room temperature under nitrogen protection. Then, sodium hydride (60 %, 1.99 g, 49.8 mmol) was added to the reaction solution in batches at 0 °C, and continued to stir at this temperature for 30 minutes; then, compound **1-4** (6.18 g, 27.1 mmol) was added to the above reaction solution at 0 °C, and the reaction solution was heated to room temperature and continued to stir for 2 hours.

After LCMS monitoring showed that the raw material disappeared, a saturated ammonium chloride aqueous solution (600 mL) was added to the reaction solution to quench. The mixture was extracted with ethyl acetate (200 mL × 3 times), and the organic phases were combined. The organic phases were washed with saturated brine (500 mL × 1 time), then dried over anhydrous sodium sulfate, filtered, and finally concentrated under reduced pressure; the resulting residue was purified by silica gel column chromatography to obtain 3 g of compound **1-5.**

MS (ESI) m/z: 411.9, 413.9 [M + H]⁺.

### Step 4:

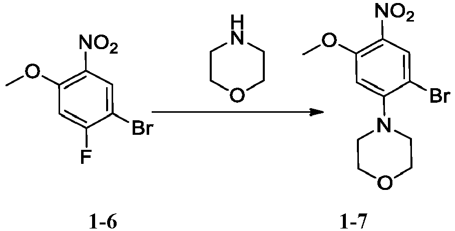

Compound **1-6** (5 g, 20.0 mmol) was dissolved in *N,N*-dimethylformamide (30 mL). The anhydrous potassium carbonate (5.5 g, 40.0 mmol) and morpholine (2.1 g, 24.0 mmol) were then added sequentially. The reaction solution was heated to 60 °C and continued to stir for 16 hours.

After LCMS monitoring showed that the raw materials disappeared, the reaction solution was cooled to room temperature, and water (600 mL) was added to the reaction solution to quench. The mixture was extracted with ethyl acetate (250 mL × 3 times), and the organic phases were combined. The organic phases were washed with saturated brine (300 mL × 3 times), then dried over anhydrous sodium sulfate, filtered, and concentrated under reduced pressure; the resulting residue was purified by silica gel column chromatography to obtain 5.4 g of compound **1-7.**

MS (ESI) m/z: 317.3, 319.2 [M + H]⁺.

### Step 5:

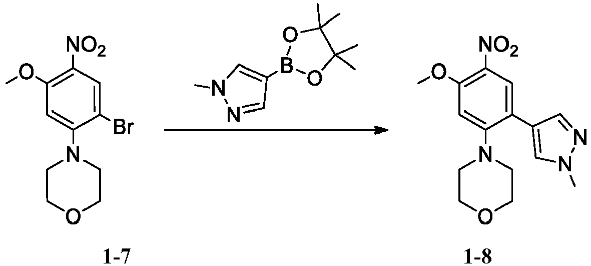

Compound **1-7** (8.5 g, 26.8 mmol) was dissolved in dioxane (88 mL) and water (18 mL) at room temperature under nitrogen protection. Then, 1-methyl-1*H*-4-pyrazoleboronic acid pinacol ester (7.25 g, 34.8 mmol), [1,1'-bis(diphenylphosphino)ferrocene]dichloropalladium(II) (2.19g, 2.7 mmol) and sodium carbonate (5.68 g, 53.6mmol) were added thereto; the reaction solution was heated to 80 °C and continued to stir for 16 hours.

After LCMS monitoring showed that the raw materials disappeared, the reaction solution was cooled to room temperature and added with water (500 mL) to quench. The mixture was extracted with ethyl acetate (300 mL × 3 times); the organic phases were combined, and the organic phases were washed with saturated brine (200 mL × 3 times), then dried over anhydrous sodium sulfate, filtered, and concentrated under reduced pressure. The resulting residue was purified by silica gel column chromatography to obtain 8 g of compound **1-8.**

MS (ESI) m/z: 319.1 [M + H]⁺.

### Step 6:

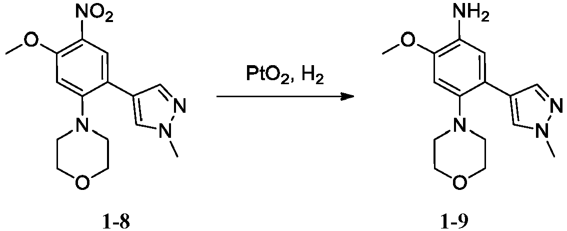

Compound **1-8** (8.5 g, 26.7 mmol) was dissolved in ethanol (80 mL); then, platinum dioxide (0.85 g) was added to the above solution; the reaction system was replaced with hydrogen for 3 times, and then stirred at room temperature for 16 hours.

After LCMS monitoring showed that the raw materials disappeared, the reaction solution was filtered through diatomite, and the filter cake was washed with ethanol (50 mL × 3 times). The filtrate was concentrated under reduced pressure, and the resulting residue was purified by silica gel column chromatography to obtain 6.5 g of compound **1-9.**

MS (ESI) m/z: 289.1 [M + H]⁺.

### Step 7:

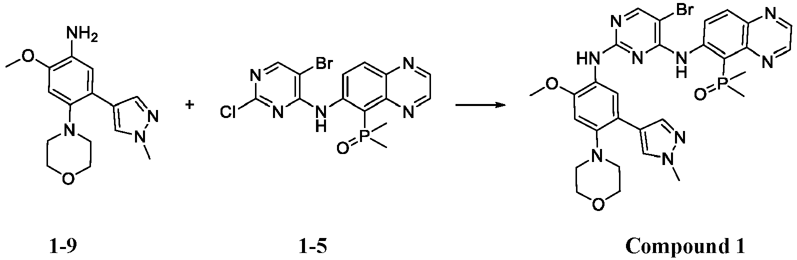

Compound **1-9** (2.0 g, 6.9 mmol) was dissolved in *N*-methylpyrrolidone (15 mL), and then compound **1-5** (2.86 g, 6.9 mmol) and methanesulfonic acid (2.0 g, 20.8 mmol) were added to the above solution sequentially. The reaction solution was heated to 95 °C and further stirred for 16 hours.

After LCMS monitoring showed that the raw material disappeared, the reaction solution was cooled to room temperature and directly purified by reversed-phase C18 column. Purification conditions were as follows: chromatographic column 120 g C18 reversed-phase column; mobile phase water (containing 0.1 % formic acid) and acetonitrile; flow rate of 50 mL/min; gradient: acetonitrile increased from 10 % to 80 % within 30 minutes; detection wavelength of 254 nm. The product was collected and lyophilized under reduced pressure to obtain 2.5 g of compound **1.**

MS (ESI) m/z: 664.2, 666.2 [M + H]⁺.

¹H NMR (400 MHz, DMSO-*d₆*) δ 12.68 (s, 1H), 8.85-8.82 (m, 3H), 8.44 (s, 1H), 8.28 (s, 1H), 8.07 (s, 1H), 7.79 (s, 1H), 7.60-7.54 (m, 2H), 6.85 (s, 1H), 3.83 (s, 3H), 3.78 (s, 3H), 3.77-3.75 (m, 4H), 2.87-2.85 (m, 4H), 2.04 (s, 3H), 2.00 (s, 3H).

### Embodiment 2:

### Preparation of (2-((5-bromo-2-((2-methoxy-5-(1-methyl-1H pyrazol-4-yl)-4-morpholinylphenyl)amino)pyrimidin-4-yl)amino)phenyl)dimethylphosphine oxide (compound 2)

### Step 1:

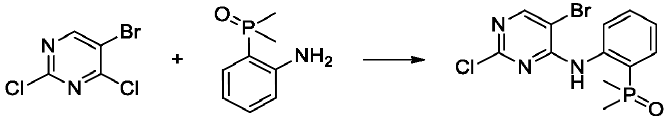

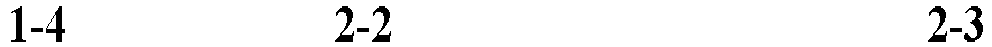

5-Bromo-2,4-dichloropyrimidine (2 g, 8.8 mmol) was dissolved in *N*,*N*-dimethylformamide (30 mL), and then anhydrous potassium carbonate (3.64 g, 26.3 mmol) and (2-aminophenyl)dimethylphosphine oxide (1.48 g, 8.8 mmol) were added sequentially. The reaction solution was heated to 60 °C and further stirred for 12 hours.

After LCMS monitoring showed that the raw materials disappeared, the reaction solution was cooled to room temperature, and water (150 mL) was added to the reaction solution to quench. The mixture was extracted with ethyl acetate (100 mL × 3 times), and the organic phases were combined. The organic phases were washed with saturated brine (80 mL × 3 times), then dried over anhydrous sodium sulfate, filtered, and concentrated under reduced pressure; the resulting residue was purified by silica gel column chromatography (eluent: dichloromethane/methanol=10/1) to obtain 2.96 g of compound **2-3.**

MS (ESI) m/z: 360.0, 362.0 [M + H]⁺.

### Step 2:

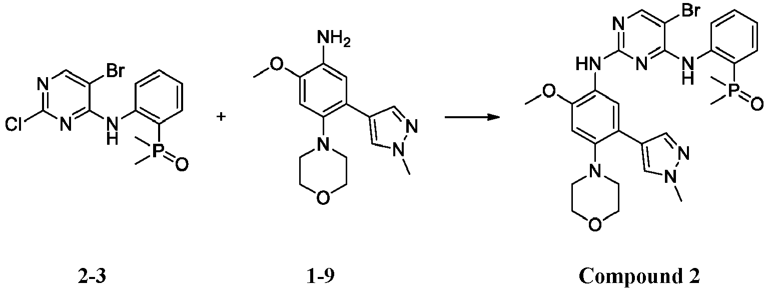

**1-9** (100 mg, 0.35 mmol) was dissolved in N-methylpyrrolidone (1 mL). Then, **2-3** (125 mg, 0.347 mmol) and methanesulfonic acid (100 mg, 1.04 mmol) were sequentially added to the above solution. The reaction solution was heated to 120 °C and further stirred for 12 hours. After LCMS monitoring showed that the raw material disappeared, the reaction solution was cooled to room temperature and directly purified by reversed-phase C18 column. Purification conditions were as follows: chromatographic column 40 g C18 reversed-phase column; mobile phase water (containing 0.1 % formic acid) and acetonitrile; flow rate of 30 mL/min; gradient: acetonitrile increased from 35 % to 85 % within 25 minutes; detection wavelength of 254 nm. The product was collected and lyophilized under reduced pressure to obtain 28 mg of compound **2.**

MS (ESI) m/z: 612.1,614.1 [M + H]⁺.

¹H NMR (400 MHz, DMSO-*d₆*) δ 10.96 (s, 1H), 8.35-8.29 (m, 1H), 8.26 (s, 1H), 8.19-8.18 (m, 1H), 8.03 (s, 1H), 7.85 (s, 1H), 7.59 (s, 1H), 7.53-7.47 (m, 1H), 7.01-6.97 (m, 2H), 6.81 (s, 1H), 3.84 (s, 3H), 3.81 (s, 3H), 3.76-3.73 (m, 4H), 2.86-2.84 (m, 4H), 1.78 (s, 3H), 1.74 (s, 3H).

### Embodiment 6:

### Preparation of (6-((5-bromo-2-((2-methoxy-5-(1-methyl-1H pyrazol-4-yl)-4-(4-methylpiperazin-1-yl))phenyl)amino)pyrimidin-4-yl)amino)quinoxalin-5-yl)dimethylphosphine oxide (compound 6)

### Step 1:

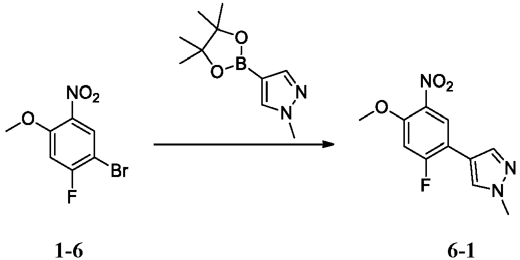

Compound **1-6** (10 g, 40.1 mmol) was dissolved in dioxane (100 mL) and water (20 mL) at room temperature under nitrogen protection. Then, 1-methyl-4-pyrazoleboronic acid pinacol ester (14.4 g, 52.1 mmol), [1,1'-bis(diphenylphosphino)ferrocene]dichloropalladium(II) (3.3 g, 4.0 mmol) and sodium carbonate (8.5 g, 80.0 mmol) were added thereto. The reaction solution was heated to 80 °C and further stirred for 16 hours.

After LCMS monitoring showed that the raw materials disappeared, the reaction solution was cooled to room temperature and added with water (100 mL) to quench. The mixture was extracted with ethyl acetate (100 mL × 3 times); the organic phases were combined, and the organic phases were washed with saturated brine (80 mL × 3 times) first, then dried over anhydrous sodium sulfate, filtered, and finally concentrated under reduced pressure. The resulting residue was purified by silica gel column chromatography (eluent: dichloromethane/methanol = 15/1) to obtain 9.8 g of compound **6-1.**

MS (ESI) m/z: 252.1 [M + H]⁺.

### Step 2:

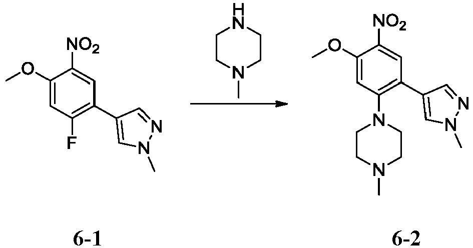

Compound **6-1** (500 mg, 2.0 mmol) was dissolved in *N,N*-dimethylformamide (5 mL). Then, anhydrous potassium carbonate (550 mg, 4.0 mmol) and N-methylpiperazine (239 mg, 2.4 mmol) were sequentially added to the above solution. The reaction solution was heated to 90 °C and further stirred for 16 hours.

After LCMS monitoring showed that the raw materials disappeared, the reaction solution was cooled to room temperature, and water (50 mL) was added to the reaction solution to quench. The mixture was extracted with ethyl acetate (30 mL × 3 times), and the organic phases were combined. The organic phases were washed with saturated brine (30 mL × 3 times) first, then dried over anhydrous sodium sulfate, filtered, and finally concentrated under reduced pressure. The resulting residue was purified by silica gel column chromatography (eluent: dichloromethane/methanol = 20/1) to obtain 309 mg of compound **6-2.**

MS (ESI) m/z: 332.2 [M + H]⁺.

### Step 3:

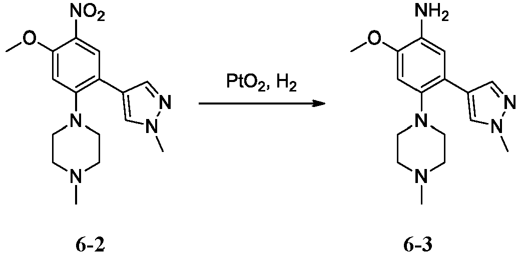

Prepared according to the method of step 6 in Embodiment 1, the raw materials were replaced with compound **6-2** (309 mg, 0.9 mmol) to obtain 259 mg of compound **6-3.**

MS (ESI) m/z: 302.1 [M + H]⁺.

### Step 4:

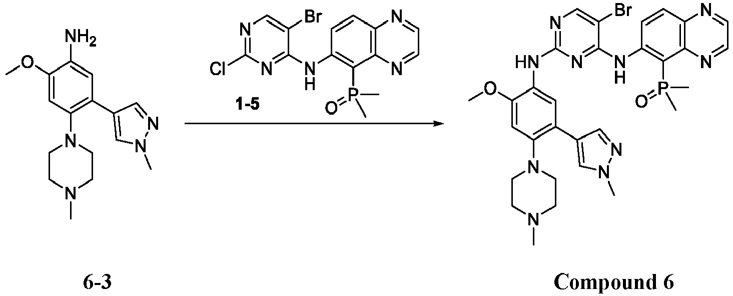

Prepared according to the method of step 7 in Embodiment 1, the raw materials were replaced with compound **6-3** (64 mg, 0.2 mmol) and N-(5-bromo-2-chloropyrimidin-4-yl)-5-(dimethylphosphoryl)quinoxalin-6-amine (87 mg, 0.2 mmol) to obtain 22 mg of compound **6.**

MS (ESI) m/z: 676.9, 678.9 [M + H]⁺.

¹H NMR (300 MHz, CDCl₃) δ 12.59 (s, 1H), 9.00 (dd, J= 9.6, 4.2 Hz, 1H), 8.75 (dd, *J* = 11.4, 2.1 Hz, 2H), 8.32 (s, 1H), 8.24 (s, 1H), 7.77 (s, 1H), 7.71 (d, *J* = 9.6 Hz, 1H), 7.44 (d, *J* = 7.5 Hz, 2H), 6.74 (s, 1H), 3.94 (s, 3H), 3.72 (s, 3H), 3.21 (s, 4H), 3.08 (s, 4H), 2.72 (s, 3H), 2.18 (s, 3H), 2.13 (s, 3H).

### Embodiment 7:

### Preparation of (6-((5-bromo-2-((2-methoxy-5-(1-methyl-1H pyrazol-4-yl)-6-morpholinylpyridin-3-yl)amino)pyrimidin-4-yl)amino)quinoxalin-5-yl)dimethylphosphine oxide (compound 7)

### Step 1:

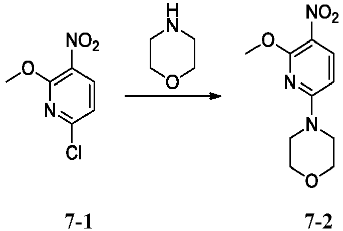

6-Chloro-2-methoxy-3-nitropyridine (10 g, 53.0 mmol) was dissolved in a mixed solvent of acetonitrile/N,N-dimethylformamide (2/1 by volume, 200 mL), and then, morpholine (4.6 g, 53.0 mmol) and triethylamine (5.4 g, 53.0 mmol) were sequentially added to the above reaction. The reaction system was stirred at room temperature for 16 hours.

After LCMS monitoring showed that the raw materials disappeared, water (500 mL) was added to the reaction solution to quench. The mixture was extracted with ethyl acetate (300 mL × 3 times), and the organic phases were combined. The organic phases were washed with saturated brine (500 mL × 3 times) first, then dried over anhydrous sodium sulfate, filtered, and finally concentrated under reduced pressure; the resulting residue was purified by silica gel column chromatography (eluent: petroleum ether/ethyl acetate = 1/1) to obtain 6.5 g of compound **7-2.**

MS (ESI) m/z: 240.0 [M + H]⁺.

### Step 2:

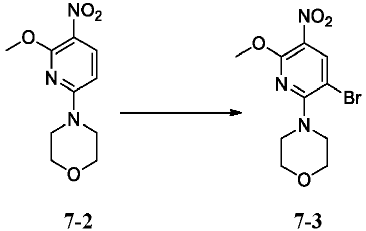

Compound **7-2** (6.5 g, 27.2 mmol) was dissolved in acetonitrile (40 mL). Then, N-bromosuccinimide (7.3 g, 27.2 mmol) was added to the above reaction solution in batches at 0 °C. The reaction temperature was raised to room temperature, and the mixture was further stirred for 2 hours. After LCMS monitoring showed that the raw materials disappeared, water (100 mL) was added to the reaction solution to quench. The mixture was extracted with ethyl acetate (100 mL × 3 times), and the organic phases were combined. The organic phases were washed with saturated brine (80 mL × 3 times) first, then dried over anhydrous sodium sulfate, filtered, and finally concentrated under reduced pressure; the resulting residue was purified by silica gel column chromatography (eluent: petroleum ether/ethyl acetate = 1/1) to obtain 7 g of compound **7-3.**

MS (ESI) m/z: 317.9, 319.9 [M + H]⁺.

### Step 3:

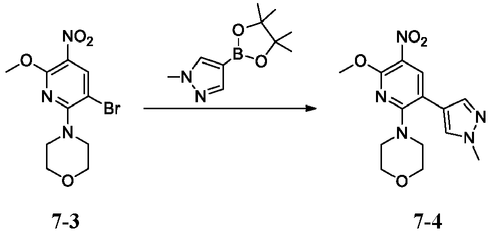

Prepared according to the method of step 5 in Embodiment 1, the raw materials were replaced with compound **7-3** (2 g, 6.3 mmol) to obtain 1.53 g of compound **7-4.**

MS (ESI) m/z: 320.3 [M + H]⁺.

### Step 4:

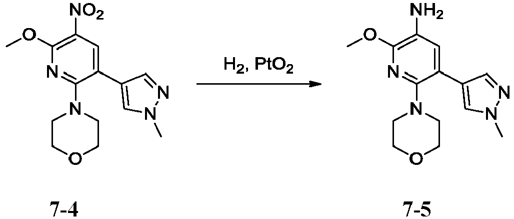

Prepared according to the method of step 6 in Embodiment 1, the raw materials were replaced with compound **7-4** (1.53 g, 4.8 mmol) to obtain 0.65 g of compound **7-5.**

MS (ESI) m/z: 290.3 [M + H]⁺.

### Step 5:

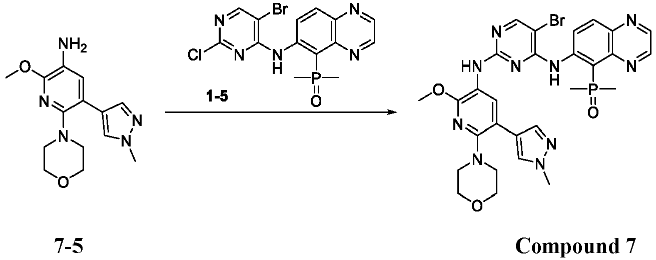

Prepared according to the method of step 7 in Embodiment 1, the raw materials were replaced with compound **7-5** (42 mg, 0.1 mmol) to obtain 31 mg of compound **7.**

MS (ESI) m/z: 664.8, 666.8 [M + H]⁺.

¹H NMR (300 MHz, CDCl₃) δ 13.06 (s, 1H), 8.86 (dd, *J* = 9.6, 4.2 Hz, 1H), 8.78 (dd, *J* = 14.1, 1.8 Hz, 2H), 8.20 (s, 1H), 8.14 (s, 1H), 7.67 (s, 1H), 7.54 (s, 1H), 4.03 (s, 3H), 3.84 (s, 3H), 3.82 (t, *J* = 4.8 Hz, 4H), 3.18 (t, *J* = 4.8 Hz, 4H), 2.18 (s, 3H), 2.13 (s, 3H).

### Embodiment 8:

### Preparation of (6-((5-bromo-2-((5-(1-(cyclopropylsulfonyl)-1H-pyrazol-4-yl)-2-methoxy-4-morpholinylphenyl)amino)pyrimidin-4-yl)amino)quinoxalin-5-yl)dimethylphosphine oxide (compound 8)

### Step 1:

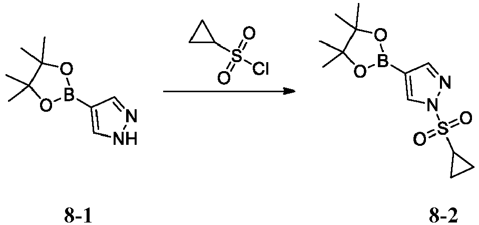

Pyrazole-4-boronic acid pinacol ester **8-1** (50 g, 257.7 mmol) was dissolved in tetrahydrofuran (800 mL) at room temperature under nitrogen protection, and then sodium hydride (60 %, 15.5 g, 387.5 mmol) was added to the reaction solution in batches at 0 °C, and the mixture was further stirred at this temperature for 30 minutes; then cyclopropanesulfonyl chloride (43.3 g, 308 mmol) was slowly added dropwise to the above reaction solution at 0 °C; and the reaction solution was heated to room temperature and further stirred for 16 hours. After LCMS monitoring showed that the raw materials disappeared, water (7 mL) was added to the reaction solution to quench. The resulting mixture was filtered, and the filter cake was washed with ethyl acetate (50 mL × 3 times). The filtrate was concentrated under reduced pressure, and the resulting residue was purified by silica gel column chromatography (eluent: dichloromethane/methanol = 10/1) to obtain 50 g of compound **8-2.**

MS (ESI) m/z: 299.1 [M + H]⁺.

### Step 2:

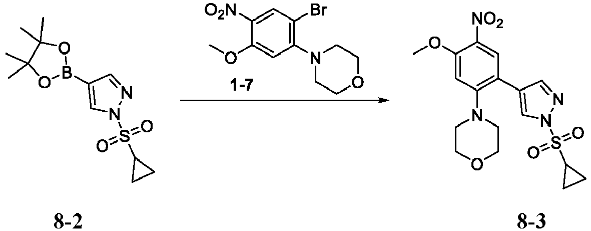

Prepared according to the method of step 5 in Embodiment 1, the raw materials were replaced with compound **8-2** (940 mg, 3.2 mmol) to obtain 819 mg of compound **8-3.**

MS (ESI) m/z: 409.3 [M + H]⁺.

### Step 3:

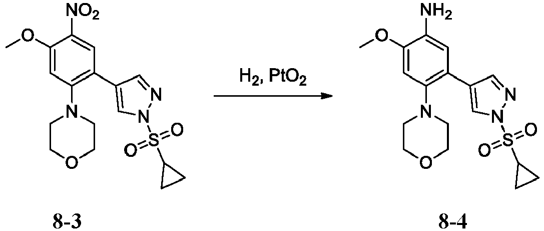

Prepared according to the method of step 6 in Embodiment 1, the raw materials were replaced with compound **8-3** (820 mg, 2.0 mmol) to obtain 650 mg of compound **8-4.**

MS (ESI) m/z: 379.0 [M + H]⁺.

### Step 4:

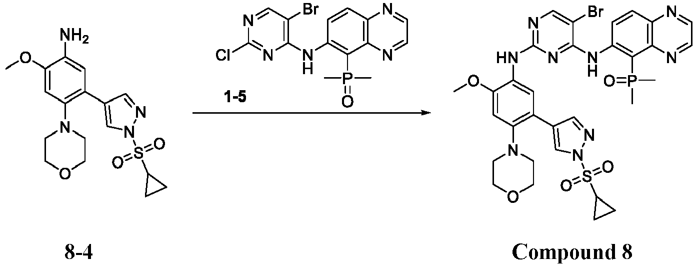

Prepared according to the method of step **7** in Embodiment 1, the raw materials were replaced with compound **8-4** (55 mg, 0.1 mmol) to obtain 6 mg of title compound **8**.

MS (ESI) m/z: 753.8, 755.8 [M + H]⁺.

¹H NMR (300 MHz, CDCl₃) δ 13.10 (s, 1H), 8.85 (d, *J*= 8.4 Hz, 1H), 8.77 (d, *J*= 5.1 Hz, 2H), 8.20 (s, 1H), 8.08 (s, 1H), 8.01 (s, 1H), 7.71 (d, *J* = 9.3 Hz, 1H), 6.79 (s, 1H), 3.97 (s, 3H), 3.85 (t, *J* = 9.3 Hz, 4H), 2.95 (t, *J* = 9.3 Hz, 4H), 2.69 (m, 1H), 2.20 (s, 3H), 2.15 (s, 3H), 1.42 - 1.36 (m, 2H), 1.17 - 1.07 (m, 2H).

### Embodiment 13:

### Preparation of 4-((5-(dimethylphosphoryl)quinoxalin-6-yl)amino)-2-((2-methoxy-5-(1-methyl-1H pyrazol-4-yl)-4-morpholinylphenyl)amino)pyrimidine-5-carbonitrile (compound 13)

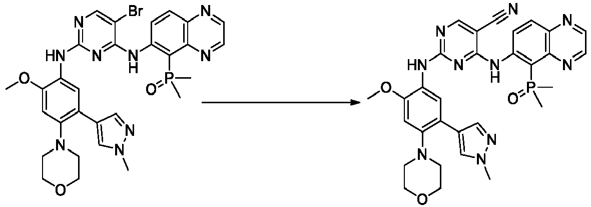

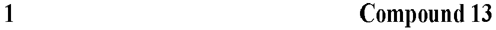

Compound 1 (220 mg, 0.3 mmol) was dissolved in *N,N*-dimethylformamide (3 mL) at room temperature under nitrogen protection. XPhos Pd G2 (chloro(2-dicyclohexylphosphino-2',4',6'-triisopropyl-1,1'-biphenyl)[2-(2'-amino-1,1'-biphenyl)]palladium(II) (28 mg, 0.03 mmol), zinc cyanide (51 mg, 0.4 mmol) and anhydrous potassium phosphate (105 mg, 0.5 mmol) were sequentially added to the above reaction solution, and the reaction system was heated to 160 °C with microwave and stirred for 2 hours.

After LCMS monitoring showed that the raw material disappeared, the reaction solution was cooled to room temperature and directly purified by reversed-phase C18 column. Purification conditions were as follows: chromatographic column 40 g C18 reversed-phase column; mobile phase water (containing 0.1 % formic acid) and methanol; flow rate of 30 mL/min; gradient: acetonitrile increased from 10 % to 50 % within 10 minutes; detection wavelength of 254 nm. The product was collected and lyophilized under reduced pressure. 50 mg of compound **13** was obtained.

MS (ESI) m/z: 611.3 [M + H]⁺.

¹H NMR (400 MHz, DMSO-*d*₆) δ 13.21 (s, 1H), 9.37 (s, 1H), 8.85 (d, *J =* 7.2 Hz, 2H), 8.66 (s, 1H), 8.55 (s, 1H), 8.14 (s, 1H), 7.87 *(d, J=* 1.8 Hz, 1H), 7.49 (s, 1H), 7.38 (s, 1H), 6.87 (s, 1H), 3.82 (s, 6H), 3.77 (m, 4H), 2.89 (m, 4H), 2.04 (s, 3H), 2.01 (s, 3H).

### Embodiment 16:

### Preparation of (6-((5-bromo-2-((2-methoxy-5-(1-methyl-1H pyrazol-4-yl)-4-(2-methyl-2,7-diazaspiro [3.5]non an-7-yl)phenyl)amino)pyrimidin-4-yl)amino)quinoxalin-5-yl)dimethylph osphine oxide formate (compound 16)

### Step 1:

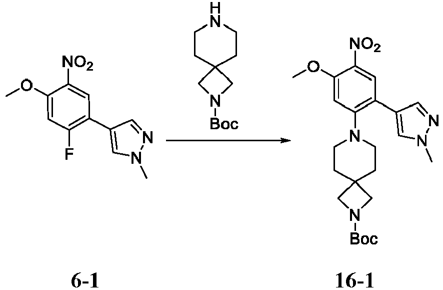

Prepared according to the method of step 2 in Embodiment 6, the raw materials were replaced with 2-*tert*-butoxycarbonyl-2,7-diazaspiro[3.5]nonane (1.1 g, 4.8 mmol) to obtain 0.75 g of compound **16-1.**

MS (ESI) m/z: 458.2 [M + H]⁺.

### Step 2:

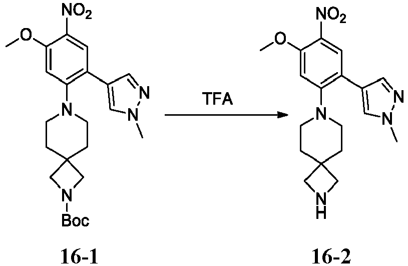

Compound **16-1** (0.65 g, 1.4 mmol) was dissolved in dichloromethane (20 mL), and then trifluoroacetic acid (7 mL) was added to the above reaction solution. The reaction system was stirred at room temperature for 2 hours. After LCMS monitoring showed that the raw materials disappeared, the reaction solution was concentrated under reduced pressure to obtain a crude product of compound **16-2** (500 mg) for direct use in the next step.

MS (ESI) m/z: 358.4 [M + H]⁺.

### Step 3:

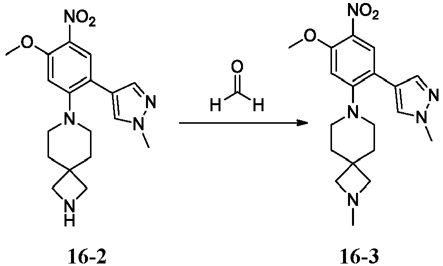

Compound **16-2** (650 mg, crude product) was dissolved in 1,2-dichloroethane (13 mL). Then, an aqueous formaldehyde solution (129 mg, 4.3 mmol) was added to the above reaction solution and the mixture was further stirred for 30 minutes; then, sodium triacetoxyborohydride (907 mg, 4.3 mmol) was added to the above reaction. The reaction system was further stirred at room temperature for 2 hours.

After LCMS monitoring showed that the raw materials disappeared, water (50 mL) was added to the reaction solution. The mixture was extracted with chloroform (100 mL × 3 times), and the organic phases were combined. The organic phases were washed with saturated brine (100 mL × 3 times) first, then dried over anhydrous sodium sulfate, filtered, and finally concentrated under reduced pressure. The resulting residue was purified by silica gel column chromatography (eluent: dichloromethane/methanol = 10/1) to obtain 520 mg of compound **16-3.**

MS (ESI) m/z: 372.2 [M + H]⁺.

### Step 4:

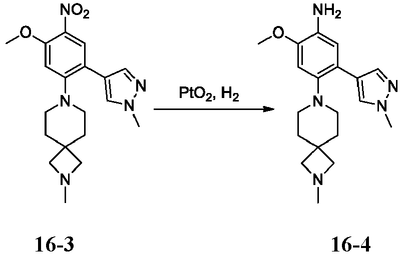

Prepared according to the method of step 6 in Embodiment 1, the raw materials were replaced with compound **16-3** (500 mg, 1.3 mmol) to obtain 420 mg of compound **16-4.**

MS (ESI) m/z: 342.2 [M + H]⁺.

### Step 5:

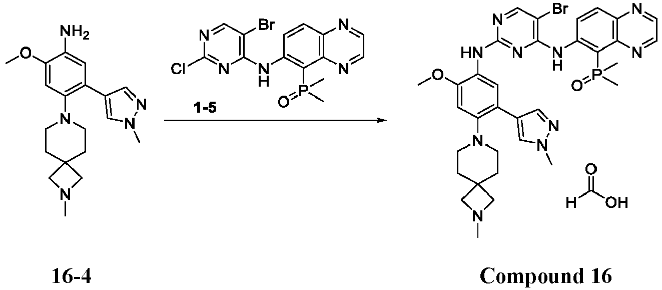

Prepared according to the method of step 7 in Embodiment 1, the raw materials were replaced with compound **16-4** (50 mg, 0.1 mmol) to obtain 40 mg of compound **16.**

MS (ESI) m/z: 716.9, 718.9 [M + H]⁺.

¹H NMR (300 MHz, DMSO-*d₆*) δ 12.67 (s, 1H), 8.85 - 8.76 (m, 3H), 8.41 (s, 1H), 8.28 (s, 1H), 8.24 (s, 1H), 8.01 (s, 1H), 7.78 (s, 1H, 7.59 - 7.52 (m, 2H), 6.80 (s, 1H), 3.80 (s, 3H), 3.77 (s, 3H), 3.19 (s, 4H), 2.79 - 2.72 (m, 4H), 2.38 (s, 3H), 2.04 (s, 3H), 1.99 (s, 3H), 1.85 - 1.82 (m, 4H).

### Embodiment 17:

### Preparation of (6-((5-bromo-2-((5-(1-methyl-1H-pyrazol-4-yl)-4-morpholino-2,3-dihydrobenzofuran-7-yl)amino)pyrimidin-4-yl)amino)quinoxalin-5-yl)dimethylphosphine oxide (compound 17)

### Step 1:

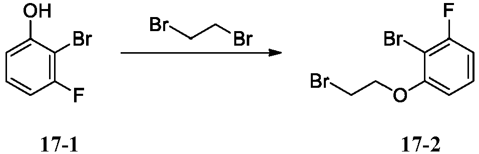

2-Bromo-3-fluorophenol (50 g, 261.8 mmol) was dissolved in acetonitrile (500 mL), and then anhydrous potassium carbonate (76 g, 549.8 mmol) and 1,2-dibromoethane (98.4 g, 523.6 mmol) were sequentially added to the reaction solution. The reaction solution was heated to 50 °C and further stirred for 38 hours.

After TLC monitoring showed that the raw materials disappeared, the reaction solution was cooled to room temperature and filtered, and the filter cake was washed with ethyl acetate (20 mL × 3 times). The filtrate was concentrated under reduced pressure, and the resulting residue was purified by silica gel column chromatography (eluent: petroleum ether/ethyl acetate = 50/1) to obtain 35 g of compound **17-2** (colorless transparent liquid, yield of 45 %).

¹H NMR: (400 MHz, CDCl₃) δ 7.27 - 7.22 (m, 1H), 6.85 -6.81 (m, 1H), 6.73 - 6.70 (m, 1H), 4.38 (t, *J* = 6.4 Hz, 2H), 3.71 (t, *J* = 6.4 Hz, 2H).

### Step 2:

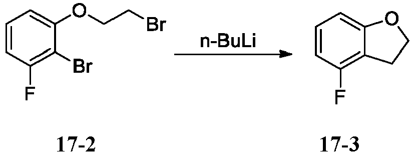

Compound **17-2** (35 g, 117 mmol) was dissolved in tetrahydrofuran (175 mL). Then, *n-*butyllithium (2.5 M, 52 mL, 129 mmol) was slowly added dropwise to the reaction solution at -78 °C under nitrogen atmosphere and the mixture was continued to stir at this temperature for 2 hours. After TLC monitoring showed that the raw materials disappeared, water (90 mL) was added to the reaction solution to quench. The mixture was extracted with ethyl acetate (200 mL × 3 times), and the organic phases were combined. The organic phases were washed with saturated brine (300 mL × 3 times) first, then dried over anhydrous sodium sulfate, filtered, and finally concentrated under reduced pressure. The resulting residue was purified by silica gel column chromatography (eluent: petroleum ether/methyl *tert*-butyl ether = 13/1) to obtain 11.3 g of compound **17-3.**

¹H NMR: (300 MHz, CDCl₃) δ 7.13 - 7.06 (m, 1H), 6.62 - 6.55 (m, 2H), 4.64 (t, *J* = 8.7 Hz, 2H), 3.27 (t, *J* = 8.7 Hz, 2H).

### Step 3:

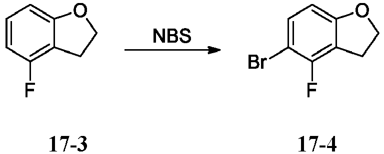

Compound **17-3** (11.3 g, 82 mmol) was dissolved in acetonitrile (100 mL). Then, N-bromosuccinimide (16 g, 90 mmol) was added to the reaction solution in batches at 0 °C. The reaction solution was heated to room temperature and continued to stir for 2 hours. After TLC monitoring showed that the raw materials disappeared, the reaction solution was directly concentrated under reduced pressure, and the resulting residue was purified by silica gel column chromatography (eluent: petroleum ether/methyl tert-butyl ether = 15/1) to obtain 17 g of compound **17-4.**

¹H NMR: (300 MHz, CDCl₃) δ 7.30-7.25 (m, 1H), 6.52 (d, *J =* 8.4 Hz, 1H), 4.64 (t, *J =* 8.7 Hz, 2H), 3.28 (t, *J* = 8.7 Hz, 2H).

### Step 4:

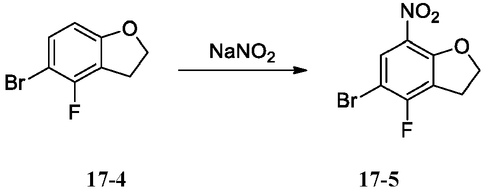

Compound **17-4** (17 g, 78 mmol) was dissolved in trifluoroacetic acid (240 mL). Then, sodium nitrite (10.8 g, 157 mmol) was added to the above reaction solution in batches at 0 °C under nitrogen atmosphere. The reaction system was heated to room temperature and continued to stir for 16 hours. After TLC monitoring showed that the raw materials disappeared, the reaction solution was poured into water (1000 mL). The mixture was extracted with ethyl acetate (200 mL × 3 times), and the organic phases were combined. The organic phases were washed with saturated brine (200 mL × 3 times) first, then dried over anhydrous sodium sulfate, filtered, and finally concentrated under reduced pressure. The resulting residue was purified by silica gel column chromatography (eluent: petroleum ether/ethyl acetate = 7/1) to obtain 13.8 g of compound **17-5.**

¹H NMR: (300 MHz, CDCl₃) δ 8.24 - 8.22 (m, 1H), 4.97 (t, *J =* 8.7 Hz, 2H), 3.42 (t, *J* = 8.7 Hz, 2H).

### Step 5:

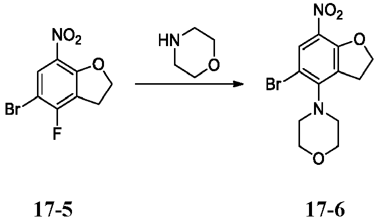

Prepared according to the method of step 4 in Embodiment 1, the raw materials were replaced with compound **17-5** (500 mg, 1.9 mmol) to obtain 345 mg of compound **17-6.**

MS (ESI) m/z: 329.2, 331.2 [M + H]⁺.

### Step 6:

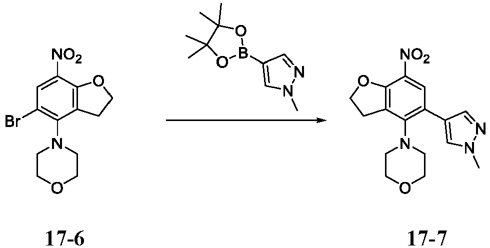

Prepared according to the method of step 5 in Embodiment 1, the raw materials were replaced with compound **17-6** (380 mg, 1.2 mmol) to obtain 380 mg of compound **17-7.**

MS (ESI) m/z: 331.4 [M + H]⁺.

### Step 7:

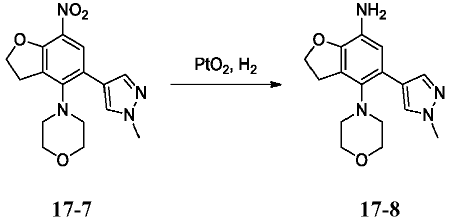

Prepared according to the method of step 6 in Embodiment 1, the raw materials were replaced with compound **17-7** (370 mg, 1.1 mmol) to obtain 220 mg of compound **17-8.**

MS (ESI) m/z: 301.4 [M + H]⁺.

### Step 8:

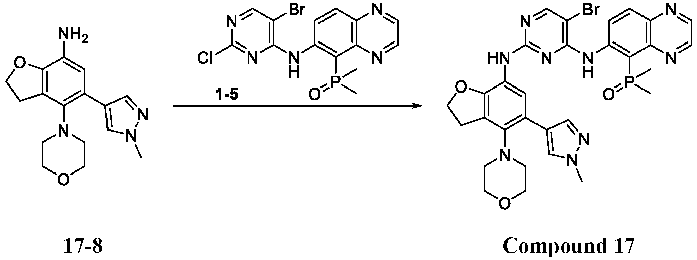

Prepared according to the method of step **7** in Embodiment 1, the raw materials were replaced with compound **17-8** (44 mg, 0.2 mmol) to obtain 21 mg of compound **17.**

MS (ESI) m/z: 675.9, 677.9 [M + H]⁺.

¹H NMR: (300 MHz, CDCl₃) δ 12.61 (s, 1H), 9.03 - 8.98 (m, 1H), 8.77 (s, 1H), 8.73 (s, 1H), 8.30 (s, 1H), 7.97 (s, 1H), 7.69 - 7.63 (m, 2H), 7.58 (s, 1H), 7.00 (s, 1H), 4.65 (t, *J =* 8.4 Hz, 2H), 3.82 (s, 3H), 3.78-3.75 (m, 4H), 3.48 (t, *J* = 8.4 Hz, 2H), 3.03 (s, 4H), 2.17 (s, 3H), 2.12 (s, 3H).

### Embodiment 31:

### Preparation of (6-((5-ethyl-2-((2-methoxy-5-(1-methyl-1H pyrazol-4-yl)-4-morpholinylphenyl)amino)pyrimidin-4-yl)amino)quinoxalin-5-yl)dimethylphosphine oxide (compound 31)

### Step 1:

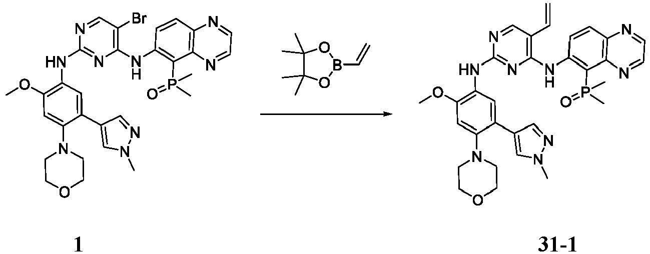

Compound **1** (100 mg, 0.2 mmol) was dissolved in dioxane (2 mL) and water (0.4 mL) at room temperature under nitrogen protection; then, pinacol vinylboronate (30 mg, 0.2 mmol), tetrakis(triphenylphosphine)palladium (17 mg, 0.02 mmol) and potassium carbonate (42 g, 0.3 mmol) were added thereto; the reaction solution was heated to 90 °C and continued to stir for 16 hours.

After LCMS monitoring showed that the raw materials disappeared, the reaction solution was cooled to room temperature and added with water (10 mL) to quench. The mixture was extracted with ethyl acetate (10 mL × 3 times); the organic phases were combined, and the organic phases were washed with saturated brine (10 mL × 3 times) first, then dried over anhydrous sodium sulfate, filtered, and finally concentrated under reduced pressure. The resulting residue was purified by silica gel column chromatography (eluent: dichloromethane/methanol = 10/1) to obtain 80 g of compound **31-1.**

MS (ESI) m/z: 612.1 [M + H]⁺.

### Step 2:

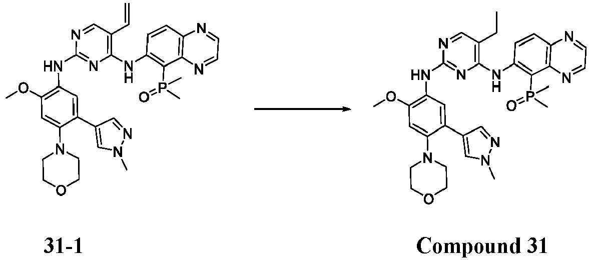

Compound **31-1** (80 mg, 0.1 mmol) was dissolved in ethanol (5 mL), and then platinum dioxide (8 mg) was added to the above solution; the reaction system was replaced with hydrogen for 3 times, and then stirred at room temperature for 16 hours.

After LCMS monitoring showed that the raw materials disappeared, the reaction solution was filtered through diatomite, and the filter cake was washed with ethanol (50 mL × 3 times). The filtrate was concentrated under reduced pressure, and the resulting residue was purified by reverse preparation (chromatographic column: Gemini-NX C18 AXAI Packed, 21.2 × 150 mm, 5 I m; mobile phase water (containing 0.1 % formic acid) and acetonitrile; flow rate of 30 mL/min; gradient: acetonitrile increased from 23 % to 29 % within 8 minutes; detection wavelength of 254 nm. The product was collected and lyophilized under reduced pressure to obtain 20 mg of compound **31.**

MS (ESI) m/z: 614.3 [M + H]⁺.

¹H NMR (300 MHz, DMSO-*d*₆) δ 13.06 (s, 1H), 9.05 (s, 1H), 8.89 (dd, *J=* 6.9, 2.1 Hz, 2H), 8.11 (s, 1H), 7.95 (s, 1H), 7.80 (s, 1H), 7.68 (s, 2H), 6.89 (s, 1H), 3.86 (s, 3H), 3.80 (s, 3H), 3.77 (t, *J=* 4.8 Hz, 4H), 2.89 (t, *J*= 4.8 Hz, 4H), 2.63 (q, *J* =14.7, 7.5 Hz, 2H), 2.08 (s, 3H), 2.03 (s, 3H), 1.21 (t, *J* = 7.5 Hz, 3H).

### Embodiment 33:

### Preparation of (6-((5-bromo-2-((2-methoxy-5-(1-methyl-1H-pyrazol-4-yl)-4-morpholinylphenyl)amino)pyrimidin-4-yl)amino)-8-methylquinoxalin-5-yl)dimethylphosphine oxide (compound 33)

### Step 1:

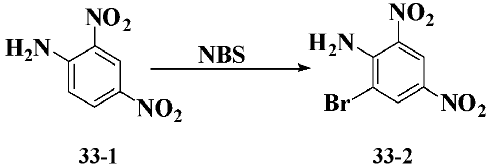

2,4-Dinitroaniline (55 g, 0.3 mol) was dissolved in acetic acid (42 mL) and water (420 mL), and then bromine (72 g, 0.45 mol) was slowly added to the reaction solution at 0 °C. The reaction solution was heated to 100 °C and continued to stir for 2 hours.

After TLC monitoring showed that the raw materials disappeared, the reaction solution was cooled to room temperature and poured into ice water (500 g). The pH of the resulting solution was adjusted to 9, and the precipitated solid was filtered. The filter cake was washed with water (100 mL × 3 times) and dried. 65 g of compound **33-2** was obtained.

¹H NMR: (300 MHz, DMSO-*d₆*) δ 8.83 (d, *J* = 2.7 Hz, 1H), 8.60 (d, *J* = 2.7 Hz, 1H), 8.14 (s, 2H).

### Step 2:

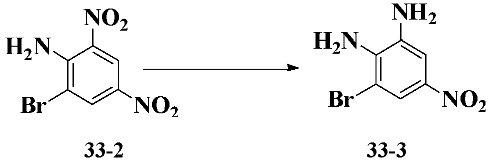

Sodium sulfide nonahydrate (27.5 g, 114.5 mmol) was dissolved in ethanol (120 mL) and water (30 mL). Then, sulphur (3.7 g, 114.7 mmol) was added to the above reaction solution; the resulting reaction solution was heated to 100 °C and continued to stir for 1 hour. The reaction solution was cooled to room temperature and added to ethanol (120 mL) and water (210 mL) containing 2-bromo-4,6-dinitroaniline (30 g, 114.5 mmol) and ammonium chloride (6.1 g, 114.4 mmol). The resulting reaction system was heated to 65 °C and continued to stir for 30 minutes. At 65 °C, 2 mol/L sodium hydroxide aqueous solution (135 mL) was slowly added dropwise to the reaction system and continued to stir for 15 minutes.

The reaction system was cooled to room temperature, poured into a mixed solvent of 2 mol/L hydrochloric acid (135 mL) and ice water (100 g). The resulting solution was extracted with ethyl acetate (1.5 L × 2 times); the organic phases were combined, and the organic phases were washed with saturated brine (1.5 L×2 times) first, then dried over anhydrous sodium sulfate, filtered, and finally concentrated under reduced pressure to obtain 22 g of compound **33-3.**

¹H NMR: (300 MHz, DMSO-*d₆*) δ 7.64 (d, *J* = 2.7 Hz, 1H), 7.39 (d, *J* = 2.7 Hz, 1H), 6.08 (s, 2H), 5.50 (s, 2H).

### Step 3:

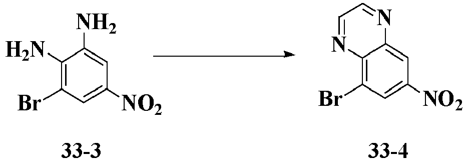

Compound **33-3** (20 g, 86.2 mmol) was dissolved in water (800 mL). Then, an aqueous solution of glyoxal (40 %, 23.6 mL) was added to the above reaction solution. The resulting reaction system was heated to 100 °C and continued to stir for 4 hours.

After TLC monitoring showed that the raw materials disappeared, the reaction solution was cooled to room temperature. The precipitated solid was filtered, and the filter cake was washed with water (100 mL×2 times). The filter cake was dried to obtain 20 g of compound **33-4.**

¹H NMR: (300 MHz, DMSO-*d₆*) δ 9.28 - 9.22 (m, 2H), 8.95 (d, *J* = 2.7 Hz, 1H), 8.89 (d, *J* = 2.7 Hz, 1H).

### Step 4:

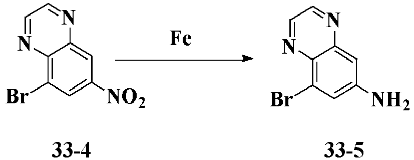

Compound **33-4** (59 g, 232 mmol) was dissolved in ethanol (250 mL). Then, ammonium chloride (50 g, 929 mmol), iron powder (65 g, 1.1 mol) and water (170 mL) were added to the above reaction. The resulting reaction system was heated to 90 °C and continued to stir for 2 hours.

After the LCMS monitoring showed that the raw materials disappeared, the reaction solution was cooled to room temperature and filtered. The filter cake was washed with ethyl acetate (100 mL×2 times) and the filtrate was concentrated under reduced pressure. Water (1 L) was added to the resulting residue, and the mixture was extracted with ethyl acetate (1.5 L×2 times). The organic phases were combined, and the organic phases were washed with saturated brine (1 L×2 times) first, and then dried over anhydrous sodium sulfate, filtered, and finally concentrated under reduced pressure. The resulting residue was purified by silica gel column chromatography (eluent: petroleum ether/ethyl acetate = 1/1) to obtain 20 g of compound **33-5.**

MS (ESI) m/z: 224.2, 226.2 [M + H]⁺.

### Step 5:

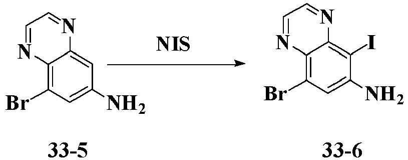

Compound **33-5** (11.3 g, 50.4 mmol) was dissolved in *N,N-*dimethylformamide (110 mL), and then, *N*-iodosuccinimide (12.5 g, 55.6 mmol) was added to the above reaction solution. The reaction system was continued to stir at room temperature for 1 hour. After LCMS monitoring showed that the raw materials disappeared, water (200 mL) was added to the reaction solution to quench. The mixture was extracted with ethyl acetate (200 mL × 3 times), and the organic phases were combined. The organic phases were washed with saturated brine (20 mL × 3 times) first, then dried over anhydrous sodium sulfate, filtered, and finally concentrated under reduced pressure. The resulting residue was purified by silica gel column chromatography (eluent: petroleum ether/ethyl acetate = 1/1) to obtain 15 g of compound **33-6.**

MS (ESI) m/z: 350.1, 352.1 [M + H]⁺.

### Step 6:

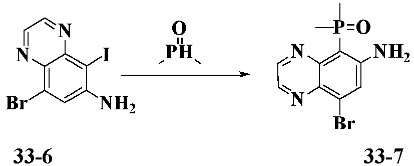

Compound **33-6** (5 g, 14.3 mmol) was dissolved in *N,N-*dimethylformamide (30 mL) at room temperature under nitrogen protection. Then, anhydrous potassium phosphate (4.55 g, 21.4 mmol), palladium acetate (321 mg, 1.4 mmol), 4,5-bis(diphenylphosphino)-9,9-dimethylxanthene (827 mg, 1.4 mmol) and dimethylphosphine oxide (1.1 g, 14.2 mmol) were sequentially added to the above reaction solution, and the reaction system was heated to 50 °C and continued to stir for 5 hours. After LCMS monitoring showed that the raw materials disappeared, water (400 mL) was added to the reaction solution to quench. The mixture was extracted with ethyl acetate (500 mL × 3 times), and the organic phases were combined. The organic phases were washed with saturated brine (500 mL × 3 times) first, then dried over anhydrous sodium sulfate, filtered, and finally concentrated under reduced pressure. The resulting residue was purified by silica gel column chromatography (eluent: dichloromethane/methanol = 10/1) to obtain 1.1 g of compound **33-7.**

MS (ESI) m/z: 300.2, 302.2 [M + H]⁺.

### Step 7:

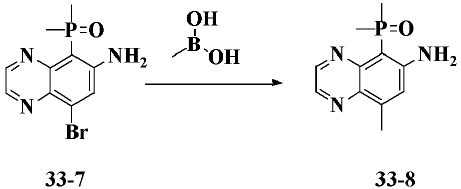

Compound **33-7** (500 mg, 1.7 mmol) was dissolved in a mixed solvent of dioxane (2 mL) and water (0.5 mL) at room temperature under nitrogen protection; then, methylboronic acid (150 mg, 2.5 mmol), [1,1'-bis(diphenylphosphino)ferrocene]dichloropalladium(II) (122 mg, 0.2 mmol) and potassium phosphate (707 mg, 3.3 mmol) were sequentially added to the above reaction solution. The reaction system was heated to 100 °C and continued to stir for 2 hours.

After LCMS monitoring showed that the raw materials disappeared, water (10 mL) was added to the reaction solution to quench. The mixture was extracted with dichloromethane (20 mL × 3 times), and the organic phases were combined. The organic phases were washed with saturated brine (20 mL × 3 times) first, then dried over anhydrous sodium sulfate, filtered, and finally concentrated under reduced pressure. The resulting residue was purified by silica gel column chromatography (eluent: dichloromethane/methanol = 12/1) to obtain 300 mg of compound **33-8.**

MS (ESI) m/z: 236.0 [M + H]⁺.

### Step 8:

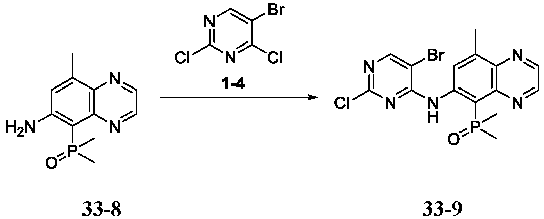

Prepared according to the method of step 3 in Embodiment 1, the raw materials were replaced with compound **33-8** (120 mg, 0.5 mmol) to obtain 70 mg of compound **33-9.**

MS (ESI) m/z: 426.0, 428.0 [M + H]⁺.

### Step 9:

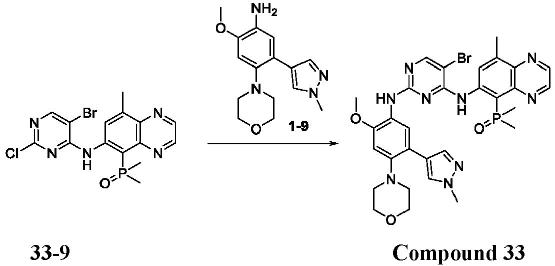

Prepared according to the method of step 7 in Embodiment 1, the raw materials were replaced with compound **33-9** (60 mg, 0.1 mmol) to obtain 10 mg of title compound **33.**

MS (ESI) m/z: 678.0, 680.0 [M + H]⁺.

¹H NMR (300 MHz, CDCl₃) δ 12.53 (s, 1H), 8.84 (d, *J* = 4.5 Hz, 1H), 8.74 (dd, *J* = 6.6, 1.8 Hz, 2H), 8.31 (s, 1H), 8.28 (s, 1H), 7.51 (s, 2H), 7.45 (s, 1H), 6.73 (s, 1H), 3.95 (s, 3H), 3.82 (t, *J* = 4.2 Hz, 4H), 3.55 (s, 3H), 2.91 (t, *J* = 4.2 Hz, 4H), 2.38 (s, 3H), 2.18 (s, 3H), 2.13 (s, 3H).

### Embodiment 35:

### Preparation of 4-((5-bromo-4-((5-(dimethylphosphoryl)quinoxalin-6-yl)amino)pyrimidin-2-yl)amino)-5-methoxy-N,N-dimethyl-2-(1-methyl-1H-pyrazol-4-yl)benzenesulfonamide (compound 35)

### Step 1:

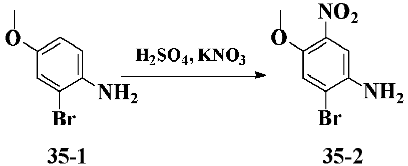

2-Bromo-4-methoxyaniline (5 g, 24.7 mmol) was dissolved in concentrated sulfuric acid (20 mL) at 0 °C. Then, potassium nitrate (2.63 g, 26 mmol) was added to the above reaction solution in batches; the reaction system was continued to stir at 0 °C for 1 hour.

After TLC monitoring showed that the raw materials disappeared, the reaction solution was poured into ice water (100 g), and the pH was adjusted to 8 with 2 mol/L potassium hydroxide; the precipitated solid was filtered, and the filter cake was washed with water (100 mL × 3 times). The filter cake was dried to obtain 5.1 g of compound **35-2.**

¹H NMR (400 MHz, DMSO-*d*₆) δ 7.43 (s, 1H), 7.34 (s, 1H), 5.41 (brs, 2H), 3.82 (s, 3H).

### Step 2:

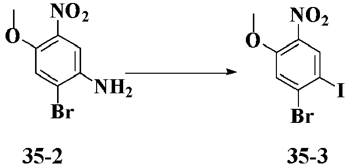

Compound **35-2** (10 g, 40.5 mmol) was dissolved in a mixed solvent of water (40 mL) and concentrated hydrochloric acid (40 mL). Then, sodium nitrite (2.8 g, 40.5 mmol) was added to the above reaction solution at 0 °C. The reaction system was continued to stir at 0 °C for 10 minutes. Then, a solution of potassium iodide (20.2 g, 121.4 mmol) in water (60 mL) was slowly added dropwise to the above reaction solution at 0 °C. The reaction system was heated to room temperature and continued to stir for 30 minutes.

After TLC monitoring showed that the raw materials disappeared, the reaction system was extracted with dichloromethane (200 mL × 3 times), and the organic phases were combined. The organic phases were washed with saturated brine (200 mL × 3 times) first, then dried over anhydrous sodium sulfate, filtered, and finally concentrated under reduced pressure. The resulting residue was purified by silica gel column chromatography (eluent: petroleum ether/ethyl acetate = 4/1) to obtain 9.3 g of compound **35-3.**

¹H NMR (400 MHz, DMSO-*d*₆) δ 8.34 (s, 1H), 7.74 (s, 1H), 3.94 (s, 3H).

### Step 3:

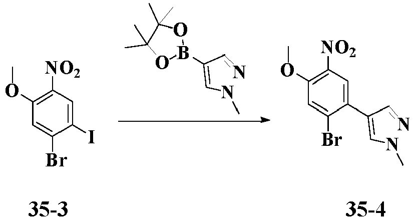

Prepared according to the method of step 5 in Embodiment 1, the raw materials were replaced with compound **35-3** (6 g, 16.8 mmol) to obtain 2.8 g of compound **35-4.**

MS (ESI) m/z: 312.0, 314.0 [M + H]⁺.

### Step 4:

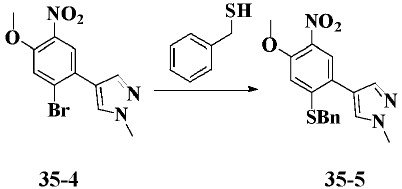

Compound **35-4** and benzyl mercaptan (438 mg, 3.5 mmol) were dissolved in dioxane (10 mL) at room temperature under nitrogen protection. Then, tris(dibenzylideneacetone)dipalladium (147 mg, 0.16 mmol), 4,5-bisdiphenylphosphino-9,9-dimethylxanthene (185 mg, 0.32 mmol) and *N,N-*diisopropylethylamine (828 mg, 6.4 mmol) were added to the above reaction solution. The reaction system was heated to 80 °C and continued to stir for 16 hours.

After LCMS monitoring showed that the raw materials disappeared, the reaction solution was cooled to room temperature and concentrated under reduced pressure. The resulting residue was purified by silica gel column chromatography (eluent: petroleum ether/ethyl acetate = 3/1) to obtain 1.2 g of compound **35-5.**

MS (ESI) m/z: 356.1 [M + H]⁺.

### Step 5:

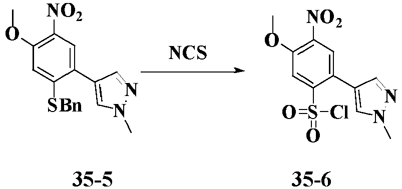

Compound **35-5** (1.2 g, 3.6 mmol) was dissolved in acetic acid (12 mL). Then, N-chlorosuccinimide (NCS, 1.35 g, 10.8 mmol) and water (1.2 mL) were sequentially added to the above reaction solution. The reaction system was continued to stir at room temperature for 2 hours.

After LCMS monitoring showed that the raw materials disappeared, water (20 mL) was added to the reaction solution to quench, and the mixture was extracted with dichloromethane (30 mL × 3 times). The organic phases were combined, and the organic phases were washed with saturated brine (30 mL × 3 times) first, then dried over anhydrous sodium sulfate, filtered, and finally concentrated under reduced pressure to obtain 0.6 g of compound **35-6.**

MS (ESI) m/z: 332.0, 334.0 [M + H]⁺.

### Step 6:

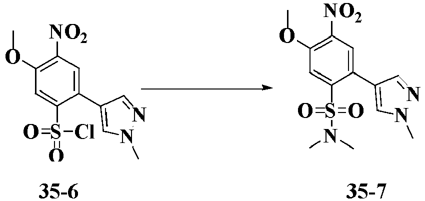

Compound **35-6** (600 mg, crude product) was dissolved in dichloromethane (6 mL). Then, dimethylamine (122 mg, 2.7 mmol) was added to the above reaction solution and the mixture was continued to stir for 3 hours at room temperature. After LCMS monitoring showed that the raw materials disappeared, water (10 mL) was added to the reaction solution to quench. The mixture was extracted with dichloromethane (20 mL × 3 times), and the organic phases were combined. The organic phases were washed with saturated brine (20 mL × 3 times) first, then dried over anhydrous sodium sulfate, filtered, and finally concentrated under reduced pressure. The resulting residue was purified by silica gel column chromatography (eluent: petroleum ether/ethyl acetate = 1/1). 250 mg of compound **35-7** was obtained.

MS (ESI) m/z: 341.0 [M + H]⁺.

### Step 7:

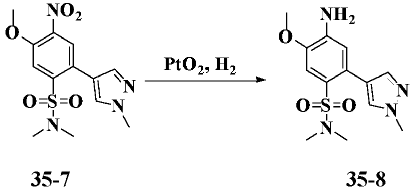

Prepared according to the method of step 6 in Embodiment 1, the raw materials were replaced with compound **35-7** (100 mg, 0.3 mmol) to obtain 90 mg of compound **35-8.**

MS (ESI) m/z: 311.1 [M + H]⁺.

### Step 8:

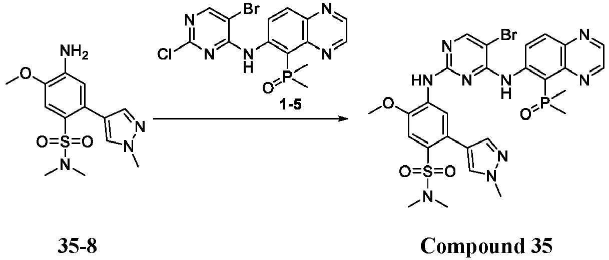

Prepared according to the method of step 7 in Embodiment 1, the raw materials were replaced with compound **35-8** (45 mg, 0.2 mmol) to obtain 24 mg of compound **35.**

MS (ESI) m/z: 686.1, 688.1 [M + H]⁺.

¹H NMR (300 MHz, DMSO-*d*₆) δ 12.79 (s, 1H), 8.90 (s, 2H), 8.78 (dd, *J* = 9.6, 3.9 Hz, 1H), 8.49 (s, 1H), 8.44 (s, 1H), 8.09 (s, 1H), 7.71 (s, 2H), 7.47 (s, 1H), 7.33 (s, 1H), 3.96 (s, 3H), 3.76 (s, 3H), 2.43 (s, 6H), 2.07 (s, 3H), 2.02 (s, 3H).

### Embodiment 41 and embodiment 42:

### Preparation of (6-((5-cyclopropyl-2-((2-methoxy-5-(1-methyl-1H-pyrazol-4-yl)-4-morpholinylphenyl)amino)pyrimidin-4-yl)amino)quinoxalin-5-yl)dimethylphosphine oxide (compound 41)

### and (6-((2-((2-methoxy-5-(1-methyl-1H-pyrazol-4-yl)-4-morpholinophenyl)amino)pyrimidin-4-yl)amino)quinoxalin-5-yl)dimethylphosphine oxide (compound 42)

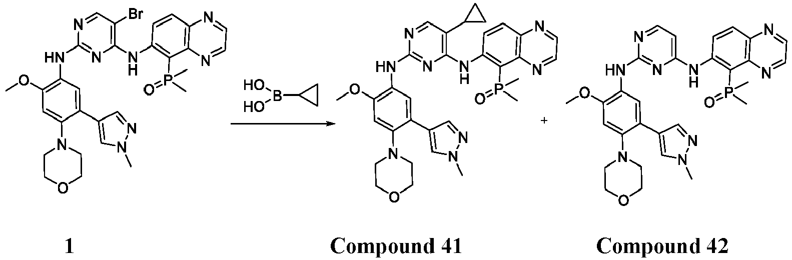

**Step 1:** Compound 1 (100 mg, 0.15 mmol) was dissolved in dioxane (3 mL) and water (1 mL) under nitrogen protection. Then, cyclopropylboronic acid (39 mg, 0.49 mmol), potassium carbonate (67 mg, 0.48 mmol) and tetrakis(triphenylphosphine)palladium (17 mg, 0.05 mmol) were sequentially added to the reaction solution. The reaction system was heated to 80 °C and continued to stir for 4 hours. After LCMS monitoring showed that the raw materials disappeared, the reaction solution was cooled to room temperature and concentrated under reduced pressure. The resulting residue was purified by silica gel column chromatography (eluent: dichloromethane/methanol = 10/1) to obtain 40 mg of compound 41 and 45 mg of compound **42.**

**Compound 41:** MS (ESI) M/Z: 626.3 [M + H]⁺.

**Compound 41:** ¹H NMR (300 MHz, DMSO-*d*₆) δ 12.72 (s, 1H), 9.19 (dd, *J* = 10.5, 3.9 Hz, 1H), 8.84-8.80 (m, 2H), 8.05 (s, 1H), 7.93 (s, 1H), 7.92, (s, 1H), 7.80 (s, 1H), 7.77 (s, 1H), 7.56 (d, *J* = 9.6 Hz, 1H), 6.84 (s, 1H), 3.84 (s, 3H), 3.77-3.74 (m, 7H), 2.88-2.84 (m, 4H), 2.06 (s, 3H), 2.01 (s, 3H), 1.82-1.73 (m, 1H), 0.96-0.92 (m, 2H), 0.65-0.60 (m, 2H).

**Compound 42:** MS (ESI) M/Z: 586.3 [M + H]⁺.

**Compound 42:** ¹H NMR (400 MHz, CDCl₃) δ 12.66 (s, 1H), 9.37 (dd, *J* = 9.6, 4.4 Hz, 1H), 8.69 (dd, *J* = 11.6, 1.6 Hz, 2H), 8.34 (s, 1H), 8.16 (d, *J* = 5.6 Hz, 1H), 7.89 (s, 1H), 7.84 (s, 1H), 7.52 (d, *J* = 9.2 Hz, 1H), 7.42 (s, 1H), 6.75 (s, 1H), 6.28 (d, *J* = 5.6 Hz, 1H), 3.96 (s, 3H), 3.87 (s, 3H), 3.84 (t, *J* = 4.4 Hz, 4H), 2.99 - 2.92 (t, *J* = 4.4 Hz, 4H), 2.14 (s, 3H), 2.11 (s, 3H).

### Embodiment 68:

### Preparation of (6-((5-bromo-2-((2-methoxy-3-methyl-5-(1-methyl-1H-pyrazol-4-yl)-4-morpholinylphenyl)amino)pyrimidin-4-yl)amino)quinoxalin-5-yl)dimethylphosphine oxide (compound 68)

### Step 1:

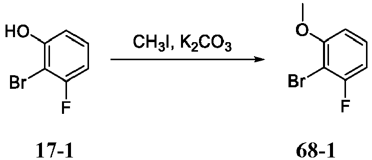

Compound **17-1** (5 g, 26.2 mmol) was dissolved in 35 mL of *N,N*-dimethylformamide, and anhydrous potassium carbonate (5.5 g, 39.3 mmol) and iodomethane (5.6 g, 39.3 mmol) were sequentially added thereto at 0 °C. The reaction system was heated to room temperature and continued to stir for 16 hours. After TLC monitoring showed that the raw materials disappeared, water (100 mL) was added to the reaction solution to quench. The mixture was extracted with ethyl acetate (50 mL × 3 times), and the organic phases were combined. The organic phases were washed with saturated brine (50 mL × 3 times) first, then dried over anhydrous sodium sulfate, filtered, and finally concentrated under reduced pressure to obtain 5.1 g of compound **68-1.**

¹H NMR (300 MHz, DMSO-*d*₆) δ 7.44-7.36 (m, 1H), 7.00-6.94 (m, 2H), 3.89 (s, 3H).

### Step 2:

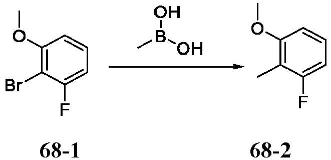

Compound **68-1** (4.57 g, 22.3 mmol) was dissolved in ethylene glycol dimethyl ether (80 mL) at room temperature under nitrogen protection. Then, methylboronic acid (4 g, 66.9 mmol), [1,1'-bis(diphenylphosphino)ferrocene]dichloropalladium(II) (0.91 g, 1.1 mmol) and potassium phosphate (14.2 g, 66.9 mmol) were sequentially added to the above reaction solution. The reaction system was heated to 80 °C and continued to stir for 16 hours. After TLC monitoring showed that the raw materials disappeared, water (300 mL) was added to the reaction solution to quench. The mixture was extracted with ethyl acetate (100 mL × 3 times), and the organic phases were combined. The organic phases were washed with saturated brine (100 mL × 3 times) first, then dried over anhydrous sodium sulfate, filtered, and finally concentrated under reduced pressure. The resulting residue was purified by silica gel column chromatography (eluent: petroleum ether/methyl *tert*-butyl ether = 5/1) to obtain 1.5 g of compound **68-2.**

¹H NMR (300 MHz, DMSO-*d₆*) δ 7.23-7.15 (m, 1H), 6.82-6.73 (m, 2H), 3.81 (s, 3H), 2.06-2.05 (m, 3H).

### Step 3:

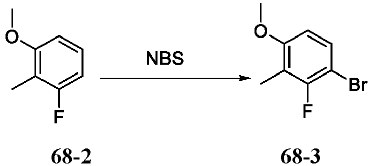

Compound **68-2** (1.4 g, 10 mmol) was dissolved in acetonitrile (12 mL). Then, N-bromosuccinimide (NBS, 2.1 g, 12 mmol) was added to the reaction solution in batches at 0 °C. The reaction solution was heated to room temperature and continued to stir for 2 hours. After TLC monitoring showed that the raw materials disappeared, the reaction solution was directly concentrated under reduced pressure, and the resulting residue was purified by silica gel column chromatography (eluent: petroleum ether/methyl tert-butyl ether = 15/1) to obtain 1.8 g of compound **68-3.**

¹H NMR (300 MHz, DMSO-*d₆*) δ 7.50-7.44 (m, 1H), 6.81 (dd, *J* = 9.0, 1.5 Hz, 1H), 3.82 (s, 3H), 2.10 (d, *J* = 2.4 Hz, 3H).

### Step 4:

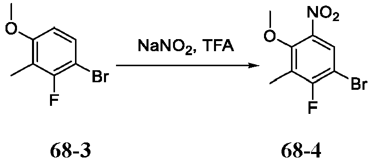

Compound **68-3** (1.67 g, 7.6 mmol) was dissolved in trifluoroacetic acid (24 mL). Then, sodium nitrite (1.1 g, 15.7 mmol) was added to the above reaction solution in batches at 0 °C under nitrogen atmosphere. The reaction system was heated to room temperature and continued to stir for 16 hours. After TLC monitoring showed that the raw materials disappeared, the reaction solution was poured into water (100 mL). The mixture was extracted with ethyl acetate (20 mL × 3 times), and the organic phases were combined. The organic phases were washed with saturated brine (20 mL × 3 times) first, then dried over anhydrous sodium sulfate, filtered, and finally concentrated under reduced pressure. The resulting residue was purified by silica gel column chromatography (eluent: petroleum ether/ethyl acetate = 7/1) to obtain 1.3 g of compound **68-4.**

¹H NMR (300 MHz, DMSO-*d₆*) δ 8.26 (d, *J* = 7.2 Hz, 1H), 3.86 (s, 3H), 2.27 (d, *J* = 2.4 Hz, 3H).

### Step 5:

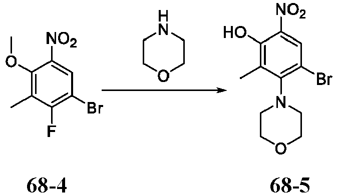

Compound **68-4** (300 mg, 1.1 mmol) was dissolved in *N*-methylpyrrolidone (2 mL). Then, morpholine (119 mg, 1.4 mmol) and *N,N*-diisopropylethylamine (734 mg, 5.7 mmol) were sequentially added to the above reaction solution. The reaction system was heated to 150 °C by microwave and continued to stir for 1 hour. After LCMS monitoring showed that the raw materials disappeared, water (30 mL) was added to the reaction solution to quench. The mixture was extracted with ethyl acetate (30 mL × 3 times), and the organic phases were combined. The organic phases were washed with saturated brine (30 mL × 3 times) first, then dried over anhydrous sodium sulfate, filtered, and finally concentrated under reduced pressure. The resulting residue was purified by silica gel column chromatography (eluent: petroleum ether/methyl tert-butyl ether = 5/1) to obtain 88 mg of compound **68-5.**

MS (ESI, m/z): 317.0, 319.0 [M + H]⁺[M + H]⁺.

### Step 6:

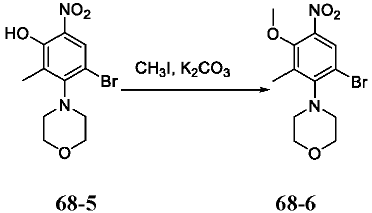

Prepared according to the method of step 1 in Embodiment 68, the raw materials were replaced with compound **68-5** (81 mg, 0.3 mmol) to obtain 75 mg of compound **68-6.**

¹H NMR (300 MHz, DMSO-*d₆*) δ 8.05 (s, 1H), 3.80 (s, 3H), 3.74 (t, *J* = 4.6 Hz, 4H), 3.20 (s, 4H), 2.33 (s, 3H).

### Step 7:

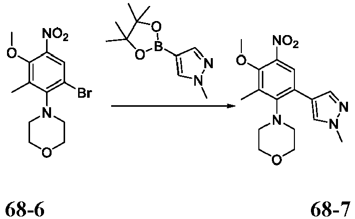

Prepared according to the method of step 5 in Embodiment 1, the raw materials were replaced with compound **68-6** (74 mg, 0.2 mmol) to obtain 70 mg of compound 68-7.

MS (ESI) m/z: 333.1 [M + H]⁺.

### Step 8:

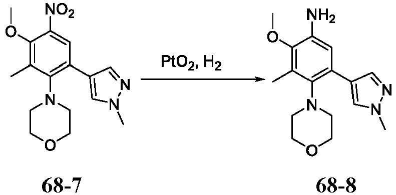

Prepared according to the method of step 6 in Embodiment 1, the raw materials were replaced with compound **68-7** (66 mg, 0.2 mmol) to obtain 39 mg of compound **68-8.**

MS (ESI) m/z: 303.2 [M + H]⁺.

### Step 9:

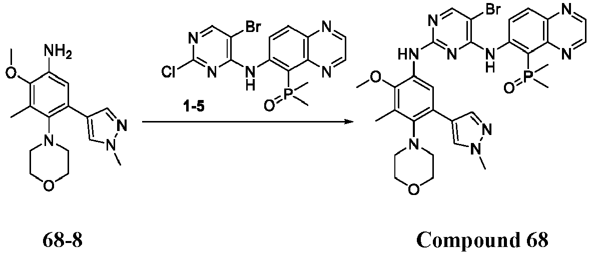

Prepared according to the method of step 7 in Embodiment 1, the raw materials were replaced with compound **68-8** (39 mg, 0.1 mmol) to obtain 34 mg of compound **68.**

MS (ESI) m/z: 678.0, 680.0 [M + H]⁺[M + H]⁺.

¹H NMR (300 MHz, DMSO-*d₆*) δ 12.72 (s, 1H), 8.90-8.78 (m, 3H), 8.51 (s, 1H), 8.32 (s, 1H), 7.75 (s, 1H), 7.55 (d, *J* = 9.6 Hz, 1H), 7.44 (s, 2H), 3.83 (s, 3H), 3.65 (s, 3H), 3.64-3.61 (m, 4H), 2.94-2.78 (m, 4H), 2.32 (s, 3H), 2.03 (d, *J* = 14.4 Hz, 6H).

### Embodiment 69:

### Preparation of (6-((5-bromo-2-((2-methoxy-5-(1-methyl-1H-pyrazol-4-yl)-4-(tetrahydro-2H-pyran-4-yl)phenyl)amino))pyrimidin-4-yl)amino)quinoxalin-5-yl)dimethylphosphine oxide (compound 69)

### Step 1:

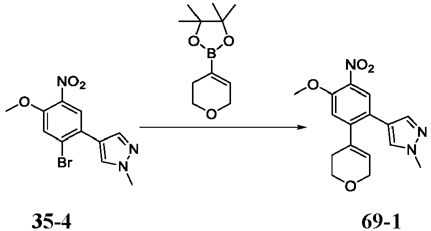

Prepared according to the method of step 5 in Embodiment 1, the raw materials were replaced with compound **35-4** (200 mg, 0.6 mmol) and 3,6-dihydro-2*H*-pyran-4-boronic acid pinacol ester (175 mg, 0.8 mmol) to obtain 150 mg of compound **69-1.**

MS (ESI) m/z: 316.0 [M + H]⁺.

### Step 2:

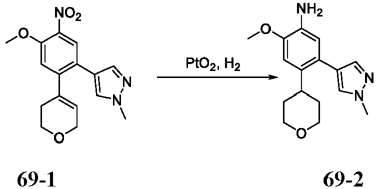

Prepared according to the method of step 6 in Embodiment 1, the raw materials were replaced with compound **69-1** (60 mg, 0.2 mmol) to obtain 40 mg of compound **69-2.**

MS (ESI) m/z: 288.1 [M + H]⁺.

### Step 3:

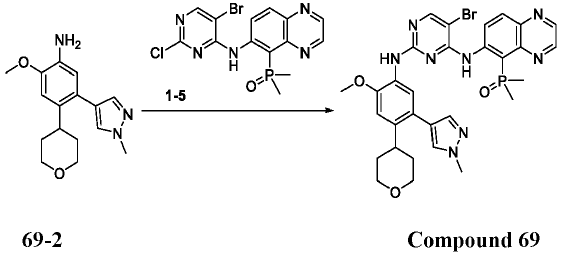

Prepared according to the method of step 7 in Embodiment 1, the raw materials were replaced with compound **69-2** (40 mg, 0.1 mmol) to obtain 16 mg of compound **69.**

MS (ESI) m/z: 663.2, 665.2 [M + H]⁺[M + H]⁺.

¹H NMR (300 MHz, CD₃OD) δ 8.90-8.82 (m, 3H), 8.29 (d, *J* = 1.2 Hz, 1H), 7.96 (s, 1H), 7.57 (d, *J* = 9.0 Hz, 1H), 7.51 (s, 1H), 7.22 (s, 1H), 7.00 (s, 1H), 4.06 - 4.01 (m, 2H), 3.97 (s, 3H), 3.81 (s, 3H), 3.51-3.44 (m, 2H), 3.16-3.10 (m, 1H), 2.18 (s, 3H), 2.13 (s, 3H), 1.97-1.83 (m, 2H), 1.69-1.65 (m, 2H).

### Embodiment 70:

### Preparation of (6-((5-bromo-2-((5-(1-methyl-1H-pyrazol-4-yl)-6-(4-methylpiperazin-1-yl)pyridin-3-yl)amino)pyrimidin-4-yl)amino)quinoxalin-5-yl)dimethylphosphine oxide (compound 70)

### Step 1:

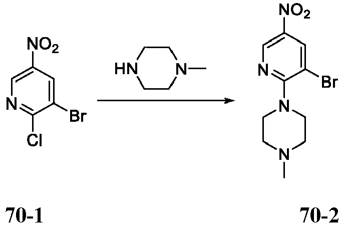

**70-1** (500 mg, 2.1 mmol) was dissolved in the mixed solvent of acetonitrile/*N,N-*dimethylformamide (2/1 by volume, 10 mL). Then, *N*-methylpiperazine (253 mg, 2.5 mmol) and triethylamine (0.27 g, 2.65 mmol) were sequentially added to the above reaction. The reaction system was stirred at room temperature for 16 hours.

After LCMS monitoring showed that the raw materials disappeared, water (25 mL) was added to the reaction solution to quench. The mixture was extracted with ethyl acetate (15 mL × 3 times), and the organic phases were combined. The organic phases were washed with saturated brine (25 mL × 3 times) first, then dried over anhydrous sodium sulfate, filtered, and finally concentrated under reduced pressure; the resulting residue was purified by silica gel column chromatography (eluent: petroleum ether/ethyl acetate = 1/1) to obtain 689 mg of compound **70-2.**

MS (ESI) m/z: 300.9, 302.9 [M + H]⁺[M + H]⁺.

### Step 2:

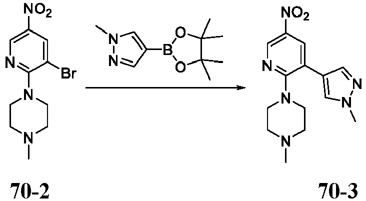

Prepared according to the method of step 5 in Embodiment 1, the raw materials were replaced with compound **70-2** (689 mg, 2.3 mmol) to obtain 368 mg of compound **70-3.**

MS (ESI) m/z: 303.1 [M + H]⁺.

### Step 3:

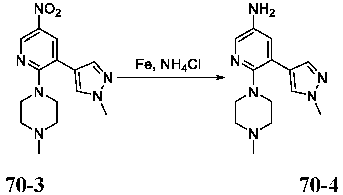

Compound **70-3** (368 mg, 1.2 mmol) was dissolved in ethanol (1.5 mL). Then, ammonium chloride (260 mg, 4.8 mmol), iron powder (337 mg, 6 mmol) and water (0.9 mL) were added to the above reaction. The resulting reaction system was heated to 90 °C and continued to stir for 2 hours.

After the LCMS monitoring showed that the raw materials disappeared, the reaction solution was cooled to room temperature and filtered. The filter cake was washed with ethyl acetate (0.6 mL × 2 times) and the filtrate was concentrated under reduced pressure. Water (5m L) was added to the resulting residue, and the mixture was extracted with ethyl acetate (7.5 mL×2 times). The organic phases were combined, and the organic phases were washed with saturated brine (5 mL×2 times) first, and then dried over anhydrous sodium sulfate, filtered, and finally concentrated under reduced pressure. The resulting residue was purified by silica gel column chromatography (eluent: petroleum ether/ethyl acetate = 1/1) to obtain 323 mg of compound **70-4.**

MS (ESI) m/z: 273.2 [M + H]⁺.

### Step 4:

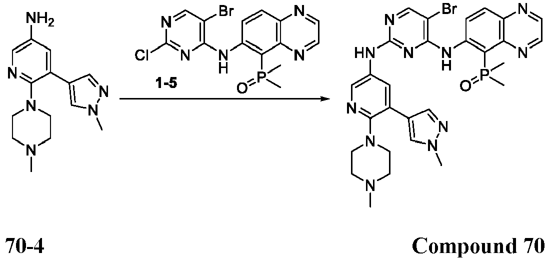

Prepared according to the method of step 7 in Embodiment 1, the raw materials were replaced with compound **70-4** (40 mg, 0.1 mmol) to obtain 26 mg of compound **70.**

MS (ESI) m/z: 648.2, 650.2 [M + H]⁺.

¹H NMR (300 MHz, CDCl₃) δ 12.65 (s, 1H), 8.97-8.92 (m, 1H), 8.77-8.73 (m, 2H), 8.29 (s, 1H), 8.24 (d, *J* = 2.7 Hz, 1H), 7.92-7.91 (m, 1H), 7.84-7.82 (m, 2H), 7.73 (s, 1H), 6.91 (s, 1H), 3.91 (s, 3H), 3.22 (s, 4H), 2.60 (s, 4H), 2.41 (s, 3H), 2.14 (d, *J* = 14.1 Hz, 6H).

### Embodiment 79:

### Preparation of (4-((5-bromo-2-((2-methoxy-5-(1-methyl-1H-pyrazol-4-yl)-4-morpholinylphenyl)amino)pyrimidin-4-yl)amino)bicycl [4.2.0]octa-1,3,5-trien-3-yl)dimethylphosphine oxide (compound 79)

### Step 1:

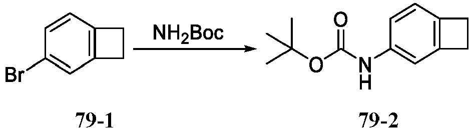

Compound **79-1** (5 g, 27.3 mmol) was dissolved in dioxane (60 mL) under nitrogen atmosphere. Then, *tert*-butyl carbamate (4.8 g, 40.9 mmol), palladium acetate (613 mg, 2.7 mmol), 2-dicyclohexylphosphino-2',4',6'-triisopropylbiphenyl (2.0 g, 4.1 mmol) and cesium carbonate (17.8 g, 54.6 mmol) were added to the reaction solution. The reaction system was heated to 100 °C and continued to stir for 2 hours. After LCMS monitoring showed that the raw materials disappeared, the reaction solution was cooled to room temperature, and water (100 mL) was added to the reaction solution to quench. The mixture was extracted with dichloromethane (250 mL × 3 times), and the organic phases were combined. The organic phases were washed with saturated brine (200 mL × 3 times), then dried over anhydrous sodium sulfate, filtered, and concentrated under reduced pressure. The resulting residue was purified by silica gel column chromatography (eluent: petroleum ether/ethyl acetate = 10/1) to obtain 5.9 g of compound **79-2.**

¹H NMR (300 MHz, CDCl₃) δ 7.23-7.19 (m, 1H), 7.03-6.98 (m, 1H), 6.96-6.92 (m, 1H), 3.13-3.09 (m, 4H), 1.51 (s, 9H).

### Step 2:

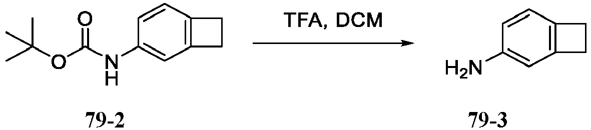

Compound **79-2** (5 g, 22.8 mmol) was dissolved in dichloromethane (20 mL), and then trifluoroacetic acid (7 mL) was added to the above reaction solution. The reaction system was stirred at room temperature for 2 hours. After LCMS monitoring showed that the raw materials disappeared, the reaction solution was concentrated under reduced pressure to obtain 2.4 g of compound **79-3.**

¹H NMR (400 MHz, CDCl₃) δ 6.86 (d, *J* = 7.6 Hz, 1H), 6.60 (dd, *J* = 7.6, 2.0 Hz, 1H), 6.49 (d, *J* = 2.0 Hz, 1H), 3.11-3.08 (m, 4H).

### Step 3:

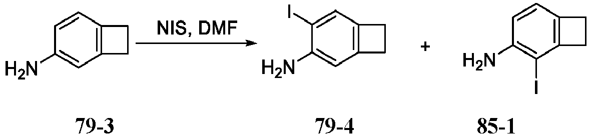

Prepared according to the method of step 1 in Embodiment 1, the raw materials were replaced with compound **79-3** (12 g, 101 mmol) to obtain 2.8 g of compound **79-4** and 0.75 g of compound **85-1.**

Compound **79-4:** ¹H NMR (300 MHz, CDCl₃) δ 7.31 (s, 1H), 6.56 (s, 1H), 3.10-3.02 (m, 4H).

Compound **85-2:** ¹H NMR (300 MHz, CDCl₃) δ 6.79 (d, *J* = 7.6 Hz, 1H), 6.59-6.55 (m, 1H), 3.95 (br s, 2H), 2.97-2.95 (m, 4H).

### Step 4:

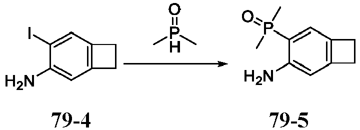

Prepared according to the method of step 2 in Embodiment 1, the raw materials were replaced with compound **79-4** (1 g, 4.1 mmol) to obtain 0.77 g of compound **79-5.**

MS (ESI) m/z: 196.2 [M + H]⁺.

### Step 5:

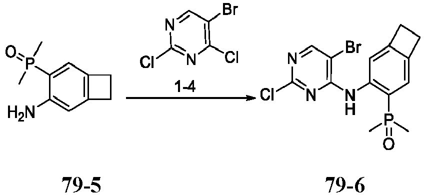

Prepared according to the method of step 3 in Embodiment 1, the raw materials were replaced with compound **79-5** (400 mg, 2.1 mmol) to obtain 220 mg of compound **79-6.**

MS (ESI) m/z: 385.9, 387.9 [M + H]⁺.

### Step 6:

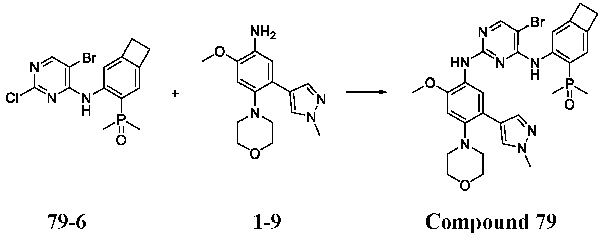

Prepared according to the method of step 7 in Embodiment 1, the raw materials were replaced with compound **79-6** (100 mg, 0.3 mmol) to obtain 53 mg of compound **79.**

MS (ESI) m/z: 638.3, 640.3 [M + H]⁺.

¹H NMR (400 MHz, CD₃OD) δ 8.19 (s, 1H), 8.09 (s, 1H), 7.85 (d, *J* = 8.8 Hz, 2H), 7.77 (d, *J* = 3.2 Hz, 1H), 7.57 (s, 1H), 7.33 (d, *J* = 13.2 Hz, 1H), 6.87 (s, 1H), 3.94 (s, 3H), 3.90 (s, 3H), 3.85-3.80 (m, 4H), 3.11-3.06 (m, 2H), 2.95-2.87 (m, 6H), 1.89 (d, *J* = 13.2 Hz, 6H).

### Embodiment 80:

### Preparation of N-(2-((5-bromo-2-((2-methoxy-5-(1-methyl-1H-pyrazol-4-yl)-4-morpholinylphenyl)amino)pyrimidin-4-yl)amino)phenylmethanesulfonamide (compound 80)

### Step 1:

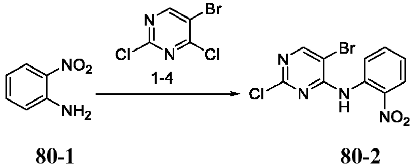

Prepared according to the method of step 3 in Embodiment 1, the raw materials were replaced with compound **80-1** (3.1 g, 22.2 mmol) to obtain 3.2 g of compound **80-2.**

MS (ESI) m/z: 328.8, 330.8 [M + H]⁺[M + H]+.

### Step 2:

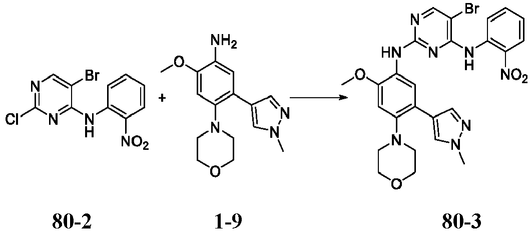

Prepared according to the method of step 7 in Embodiment 1, the raw materials were replaced with compound **80-2** (200 mg, 0.6 mmol) to obtain 300 mg of compound **80-3.**

MS (ESI) m/z: 581.2, 583.2 [M + H]⁺.

### Step 3:

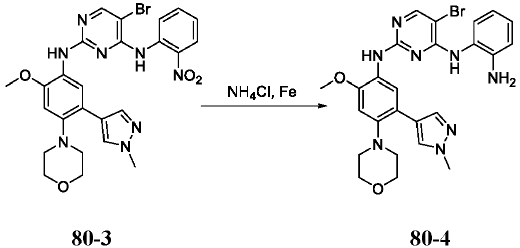

Prepared according to the method of step 3 in Embodiment 70, the raw materials were replaced with compound **80-3** (250 mg, 0.43 mmol) to obtain 100 mg of compound **80-4.**

MS (ESI) m/z: 551.2, 553.2 [M + H]⁺.

### Step 4:

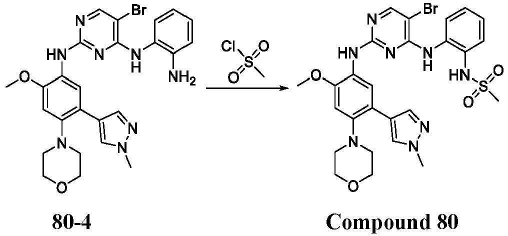

Compound **80-4** (40 mg, 0.07 mmol) was dissolved in pyridine (4 mL) at room temperature. Then, methanesulfonyl chloride (42 mg, 0.4 mmol) was added to the above reaction solution. The reaction system was heated to 50 °C and continued to stir for 3 hours. After LCMS monitoring showed that the raw material disappeared, the reaction solution was cooled to room temperature and directly purified by a reversed-phase C18 column (purification conditions were as follows: chromatographic column: 40 g C18 reversed-phase column; mobile phase: water (containing 0.1 % formic acid) and acetonitrile; flow rate: 35 mL/min; gradient: acetonitrile increased from 20 % to 57 % within 25 minutes; detection wavelength: 254 nm. The product was collected and lyophilized under reduced pressure.) to obtain 17 mg of compound **80.**

MS (ESI, m/z): 629.1, 631.1 [M + H]⁺.

¹H NMR (400 MHz, DMSO-*d*₆) δ 9.63 (s, 1H), 8.46 (s, 1H), 8.18 (s, 1H), 8.16 (s, 1H), 8.04 (d, *J* = 8.0 Hz, 1H), 7.97 (s, 1H), 7.83 (s, 1H), 7.60 (s, 1H), 7.28 (d, *J* = 7.6 Hz, 1H), 7.01-6.97 (m, 1H), 6.79 (s, 2H), 3.85 (s, 3H), 3.80 (s, 3H), 3.75-3.72 (m, 4H), 2.95 (s, 3H), 2.85-2.83 (m, 4H).

### Embodiment 82:

### Preparation of (6-((5-bromo-2-((6-(1-methyl-1H pyrazol-4-yl)benzo[d][1,3]dioxol-4-yl)amino)pyrimidin-4-yl)amino)quinoxalin-5-yl)dimethylphosphine oxide (compound 82)

### Step 1:

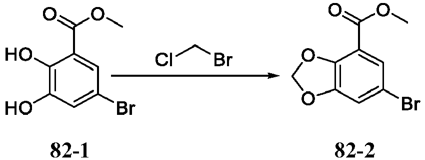

Compound **82-1** (750 mg, 3 mmol) was dissolved in *N,N-*dimethylformamide (10 mL). Then, cesium carbonate (1.5 g, 4.6 mmol) and chlorobromomethane (786 mg, 6.1 mmol) were sequentially added to the above reaction solution. The reaction system was heated to 110 °C and continued to stir for 3 hours. After LCMS monitoring showed that the raw materials disappeared, the reaction solution was cooled to room temperature, and water (100 mL) was added thereto to quench. The mixture was extracted with ethyl acetate (50 mL × 3 times), and the organic phases were combined. The organic phases were washed with saturated brine (100 mL × 3 times), then dried over anhydrous sodium sulfate, filtered, and concentrated under reduced pressure. The resulting residue was purified by silica gel column chromatography (eluent: petroleum ether/ethyl acetate = 5/1) to obtain 750 mg of compound **82-2.**

¹H NMR (300 MHz, DMSO-*d₆*) δ 7.43 (d, *J* = 2.1 Hz, 1H), 7.38 (d, *J* = 2.1 Hz, 1H), 6.22 (s, 2H), 3.83 (s, 3H).

### Step 2:

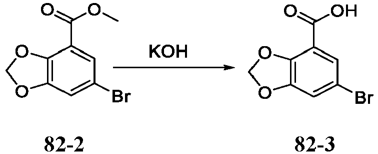

Compound **82-2** (1.00 g, 3.86 mmol) was dissolved in methanol (10 mL). Then, 2 M potassium hydroxide aqueous solution (3.8 mL, 7.7 mmol) was added to the above reaction solution. The reaction system was continued to stir at room temperature for 2 hours. After LCMS monitoring showed that the raw materials disappeared, the reaction solution was concentrated under reduced pressure. Then, water (20 mL) was added thereto and the pH of the mixture was adjusted to 5 with concentrated hydrochloric acid, and a solid was precipitated, filtered and dried to obtain 970 mg of compound **82-3.**

¹H NMR (300 MHz, DMSO-*d₆*) δ 13.32 (s, 1H), 7.56-7.23 (m, 2H), 6.19 (s, 2H).

### Step 3:

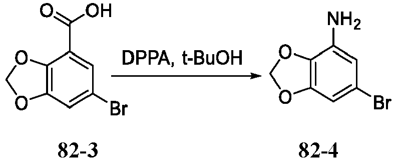

Compound **82-3** (500 mg, 2 mmol) was dissolved in *tert*-butanol (10 mL) under nitrogen atmosphere. Then, triethylamine (826 mg, 8.2 mmol) and diphenylphosphoryl azide (842 mg, 3.1 mmol) were added to the above reaction solution. The reaction system was stirred at room temperature for 2 hours, then heated to 90 °C and continued to stir for 16 hours. After LCMS monitoring showed that the raw materials disappeared, the reaction solution was concentrated under reduced pressure. The resulting residue was purified by silica gel column chromatography (eluent: petroleum ether/ethyl acetate = 5/1) to obtain 100 mg of compound **82-4.**

¹H NMR (400 MHz, DMSO-*d*₆) δ 6.44 (d, *J* = 2.0 Hz, 1H), 6.38 (d, *J* = 2.0 Hz, 1H), 5.94 (s, 2H).

### Step 4:

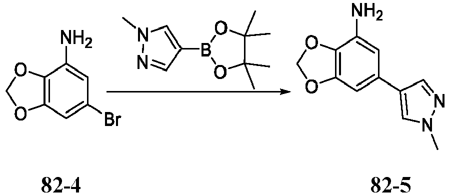

Prepared according to the method of step 5 in Embodiment 1, the raw materials were replaced with compound **82-4** (100 mg, 0.5 mmol) to obtain 60 mg of compound **82-5.**

MS (ESI) m/z: 218.2 [M + H]⁺.

### Step 5:

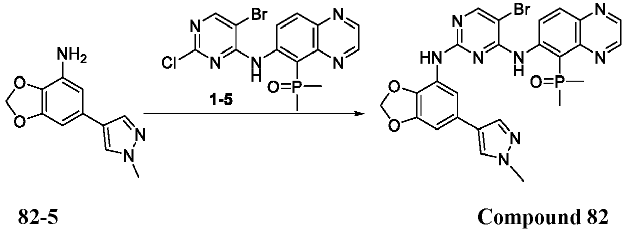

Prepared according to the method of step 7 in Embodiment 1, the raw materials were replaced with compound **82-5** (60 mg, 0.3 mmol) to obtain 20 mg of compound **82.**

MS (ESI) m/z: 593.1, 595.1 [M + H]⁺.

¹H NMR (300 MHz, DMSO-*d*₆) δ 12.76 (s, 1H), 9.19 (s, 1H), 8.97 (dd, *J* = 9.6, 3.9 Hz, 1H), 8.86 (d, *J* = 1.8 Hz, 1H), 8.81 (d, *J* = 1.8 Hz, 1H), 8.32 (s, 1H), 8.00 (s, 1H), 7.74 (s, 1H), 7.65 (d, *J* = 9.3 Hz, 1H), 7.14 (d, *J* = 1.5 Hz, 1H), 7.08 (d, *J* = 1.5 Hz, 1H), 5.98 (s, 2H), 3.77 (s, 3H), 2.02 (d, *J* = 14.4 Hz, 6H).

### Embodiment 88:

### Preparation of (6-((5-bromo-2-((4-(4-isopropylpiperazin-1-yl)-2-methoxy-5-(1-methyl-1H-pyrazol-4-yl)phenyl)amino)pyrimidin-4-yl)amino)quinoxalin-5-yl)dimethylphosphine oxide (compound 88)

### Step 1:

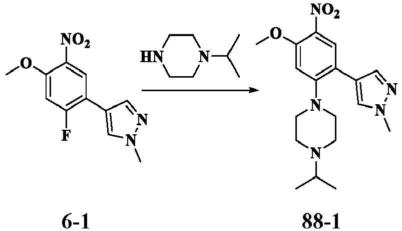

Compound **6-1** (500 mg, 2.0 mmol) was dissolved in *N,N*-dimethylformamide (5 mL). Then, anhydrous potassium carbonate (550 mg, 4.0 mmol) and *N-*isopropylpiperazine (316 mg, 2.4 mmol) were sequentially added to the above solution. The reaction solution was heated to 90 °C and continued to stir for 16 hours.

After LCMS monitoring showed that the raw materials disappeared, the reaction solution was cooled to room temperature, and water (50 mL) was added to the reaction solution to quench. The mixture was extracted with ethyl acetate (30 mL × 3 times), and the organic phases were combined. The organic phases were washed with saturated brine (30 mL × 3 times) first, then dried over anhydrous sodium sulfate, filtered, and finally concentrated under reduced pressure. The resulting residue was purified by silica gel column chromatography (eluent: dichloromethane/methanol = 20/1) to obtain 439 mg of compound **88-1.**

MS (ESI) m/z: 360.2 [M + H]⁺.

### Step 2:

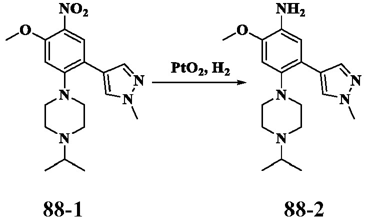

Prepared according to the method of step 6 in Embodiment 1, the raw materials were replaced with compound **88-1** (260 mg, 0.7 mmol) to obtain 280 mg of compound **88-2.**

MS (ESI) m/z: 330.2 [M + H]⁺.

### Step 3:

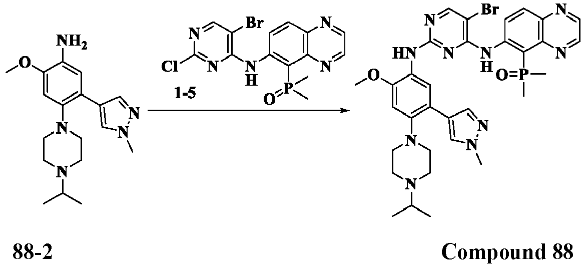

Prepared according to the method of step 7 in Embodiment 1, the raw materials were replaced with compound **88-2** (48 mg, crude product) to obtain 20 mg of compound **88.**

MS (ESI) m/z: 705.2, 707.2 [M + H]⁺.

¹H NMR (400 MHz, CDCl₃) δ 12.59 (s, 1H), 9.02-8.99 (m, 1H), 8.76 (d, *J* = 2.0 Hz, 1H), 8.73 (d, *J* = 2.0 Hz, 1H), 8.54 (s, 1H), 8.31 (s, 1H), 8.23 (s, 1H), 7.75 (s, 1H), 7.69-7.67 (m, 1H), 7.53 (s, 1H), 7.38 (s, 1H), 6.77 (s, 1H), 3.92 (s, 3H), 3.71 (s, 3H), 3.17 (s, 5H), 2.96 (s, 4H), 2.15 (d, *J* = 14.4 Hz, 6H), 1.29 (d, *J* = 6.8 Hz, 6H).

### Embodiment 93:

### Preparation of (6-((5-bromo-2-((3-fluoro-2-methoxy-5-(1-methyl-1H-pyrazol-4-yl)-4-(4-(4-methylpiperazin-1-yl)piperidin-1-yl)phenyl)aminopyrimidin-4-yl)amino)quinoxalin-5-yl)dimethylphosphine oxide (compound 93)

### Step 1:

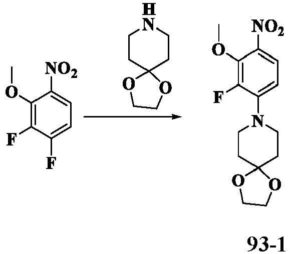

Prepared according to the method of step 4 in Embodiment 1, the raw materials were replaced with 2,3-difluoro-6-nitroanisole (5 g, 26.4 mmol) and 4-piperidone ethylene acetal (4.5 g, 31.7 mmol) to obtain 6.8 g of compound **93-1.**

MS (ESI) m/z: 313.1 [M + H]⁺.

### Step 2:

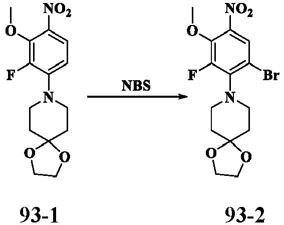

Prepared according to the method of step 3 in Embodiment 68, the raw materials were replaced with compound **93-1** (6.7 g, 21.5 mmol) to obtain 4.7 g of compound **93-2.**

MS (ESI) m/z: 391.2, 393.2 [M + H]⁺.

### Step 3:

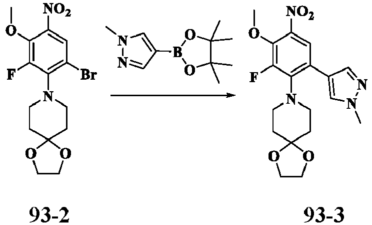

Prepared according to the method of step 5 in Embodiment 1, the raw materials were replaced with compound **93-2** (4.5 g, 11.4 mmol) to obtain 4.38 g of compound **93-3.**

MS (ESI) m/z: 393.1 [M + H]⁺.

### Step 4:

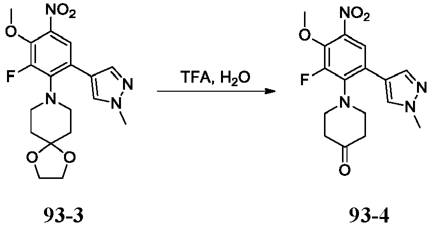

Compound **93-3** (2.00 g, 5.1 mmol) was dissolved in trifluoroacetic acid (15 mL) and water (3 mL) at room temperature. Then, the reaction system was heated to 60 °C and continued to stir for 1.5 hours. After LCMS monitoring showed that the raw materials disappeared, the reaction solution was cooled to room temperature, and water (80 mL) was added to the reaction solution to quench. The mixture was extracted with ethyl acetate (100 mL × 3 times), and the organic phases were combined. The organic phases were washed with saturated brine (80 mL × 3 times) first, then dried over anhydrous sodium sulfate, filtered, and concentrated under reduced pressure; the resulting residue was purified by a reversed-phase C18 column. The purification methods were as follows: mobile phase: water (containing 0.1 % ammonium bicarbonate) and acetonitrile; gradient: acetonitrile increased from 30 % to 55 % within 25 minutes; detection wavelength: 254 nm. The product was collected and lyophilized under reduced pressure to obtain 1.1 g of compound **93-4.**

MS (ESI) m/z: 349.3 [M + H]⁺.

### Step 5:

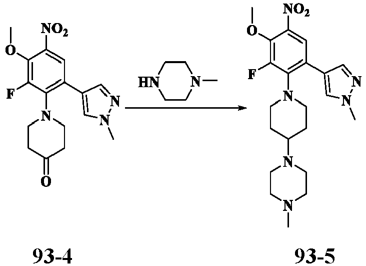

Compound **93-4** (400 mg, 1.1 mmol) was dissolved in 1,2-dichloroethane (13 mL). Then, N-methylpiperazine (230 mg, 2.3 mmol) was added to the above reaction solution and the mixture was continued to stir for 30 minutes; then, sodium triacetoxyborohydride (907 mg, 4.3 mmol) was added to the above reaction. The reaction system was continued to stir at room temperature for 2 hours.

After LCMS monitoring showed that the raw materials disappeared, water (50 mL) was added to the reaction solution. The mixture was extracted with chloroform (100 mL × 3 times), and the organic phases were combined. The organic phases were washed with saturated brine (100 mL × 3 times) first, then dried over anhydrous sodium sulfate, filtered, and finally concentrated under reduced pressure. The resulting residue was purified by silica gel column chromatography (eluent: dichloromethane/methanol = 10/1) to obtain 403 mg of compound **93-5.**

MS (ESI) m/z: 433.2 [M + H]⁺.

### Step 6:

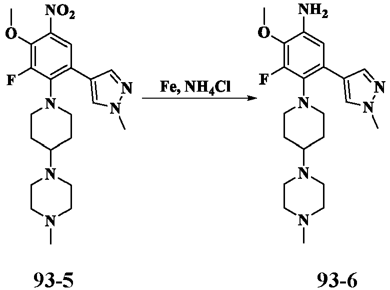

Prepared according to the method of step 3 in Embodiment 70, the raw materials were replaced with compound **93-5** (220 mg, 0.5 mmol) to obtain 202 mg of compound **93-6.**

MS (ESI) m/z: 403.3 [M + H]⁺.

### Step 7:

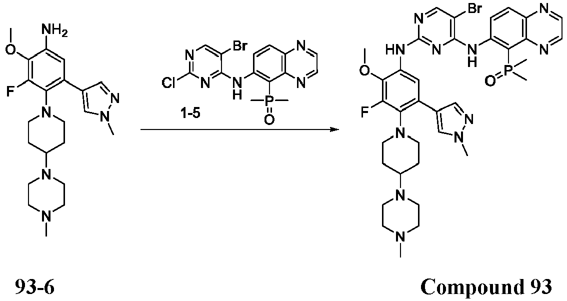

Prepared according to the method of step 7 in Embodiment 1, the raw materials were replaced with compound 93-6 (202 mg, 0.5 mmol) to obtain 77 mg of compound **93.**

MS (ESI) m/z: 778.4, 780.4 [M + H]⁺.

¹H NMR (300 MHz, DMSO-*d₆*) δ 12.70 (s, 1H), 8.89 - 8.74 (m, 4H), 8.34 (s, 1H), 7.98 (s, 1H), 7.74 (s, 1H), 7.58 (d, *J* = 9.6 Hz, 1H), 7.49 (d, *J* = 2.1 Hz, 1H), 3.79 (s, 3H), 3.75 (s, 3H), 3.09-2.97 (m, 4H), 2.61-2.53 (m, 4H) 2.37-2.29 (m, 5H), 2.16 (s, 3H), 2.03 (d, *J* = 14.4 Hz, 6H), 1.82-1.78 (m, 2H), 1.60-1.48 (m, 2H).

¹⁹F NMR (282 MHz, DMSO-*d₆*) δ -137.78.

### Embodiment 100:

### Preparation of (6-((5-bromo-2-((3-methyl-5-(1-methyl-1H-pyrazol-4-yl)-4-morpholinylphenyl)amino)pyrimidin-4-yl)amino)quinoxalin-5-yl)dimethylphosphine oxide (compound 100)

### Step 1:

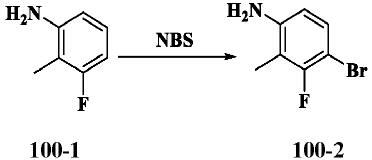

Prepared according to the method of step 3 in Embodiment 68, the raw materials were replaced with compound **100-1** (10 g, 80 mmol) to obtain 11.4 g of compound **100-2.**

¹H-NMR (400 MHz, DMSO-*d*₆) δ 7.10 (dd, *J* = 8.4, 8.4 Hz, 1H), 6.41 (dd, *J* = 8.8, 1.2 Hz, 1H), 5.37 (s, 2H), 1.99 (d, *J* = 2.0 Hz, 3H).

### Step 2:

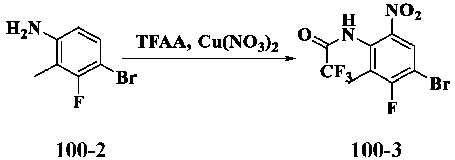

Compound **100-2** (8.5 g, 41.7 mmol) was dissolved in trifluoroacetic anhydride (TFAA, 87.5 g, 416 mmol). Then, copper nitrate (23.36 g, 125 mmol) was added to the above reaction solution at 0 °C. The reaction system was heated to room temperature and continued to stir for 1 hour. After TLC monitoring showed that the raw materials disappeared, the reaction solution was added dropwise into saturated sodium bicarbonate (500 mL) to quench. The mixture was extracted with ethyl acetate (300 mL × 3 times), and the organic phases were combined. The organic phases were washed with saturated brine (200 mL × 3 times) first, then dried over anhydrous sodium sulfate, filtered, and finally concentrated under reduced pressure. The resulting residue was purified by silica gel column chromatography (eluent: petroleum ether/ethyl acetate = 3/1) to obtain 8 g of compound **100-3.**

¹H-NMR (400 MHz, DMSO-*d*₆) δ 11.76 (s, 1H), 8.41 (d, *J* = 6.8 Hz, 1H), 2.24 (d, *J* = 2.4 Hz, 3H).

### Step 3:

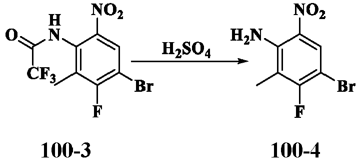

Compound **100-3** (890 mg, 2.6 mmol) was dissolved in 1,4-dioxane (8 mL). Then, a dilute sulfuric acid aqueous solution (2 mol/L, 8 mL) was added to the above reaction solution. The reaction system was heated to 100 °C and continued to stir for 2 hours. After TLC monitoring showed that the raw materials disappeared, the reaction solution was cooled to room temperature and diluted with ethyl acetate (50 mL). The pH of the mixture was adjusted to 8 with saturated sodium bicarbonate aqueous solution. The mixture was extracted with ethyl acetate (50 mL × 3 times), and the organic phases were combined. The organic phases were washed with saturated brine (80 mL × 3 times) first, then dried over anhydrous sodium sulfate, filtered, and finally concentrated under reduced pressure. The resulting residue was purified by silica gel column chromatography (eluent: petroleum ether/ethyl acetate = 3/1) to obtain 600 mg of compound **100-4.**

¹H-NMR (300 MHz, DMSO-*d*₆) δ 8.20 (d, *J* = 7.5 Hz, 1H), 7.52 (s, 2H), 2.15 (d, *J* = 2.7 Hz, 3H).

### Step 4:

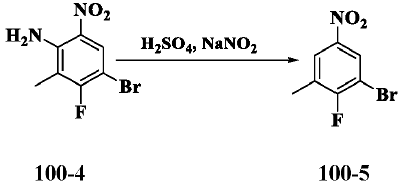

Compound **100-4** (600 mg, 2.4 mmol) was dissolved in concentrated sulfuric acid (6 mL). Then, sodium nitrite (382 mg, 5.5 mmol) was added to the above reaction solution at 0 °C and continued to stir at this temperature for 1 hour. Then, ice water (3 g) and a solution of copper sulfate (500 mg, 3.1 mmol) in ethanol (6 mL) were sequentially added to the reaction solution. The reaction system was heated to 80 °C and continued to stir for 1 hour. After TLC monitoring showed that the raw materials disappeared, the reaction solution was cooled to room temperature, and water (100 mL) was added to the reaction solution to quench. The mixture was extracted with ethyl acetate (50 mL × 3 times), and the organic phases were combined. The organic phases were washed with saturated brine (80 mL × 3 times) first, then dried over anhydrous sodium sulfate, filtered, and finally concentrated under reduced pressure. The resulting residue was purified by silica gel column chromatography (eluent: petroleum ether/ethyl acetate = 4/1) to obtain 370 mg of compound **100-5.**

¹H-NMR (300 MHz, CDCl₃) δ 8.33 (m, 1H), 8.11-8.08 (m, 1H), 2.48-2.41 (m, 3H).

### Step 5:

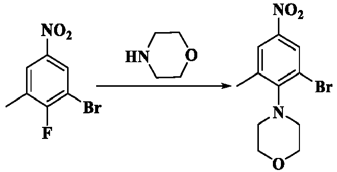

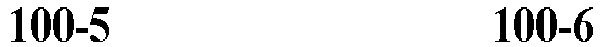

Prepared according to the method of step 4 in Embodiment 1, the raw materials were replaced with **100-5** (270 mg, 1.2 mmol) to obtain 130 mg of compound **100-6.**

MS (ESI) m/z: 301.1, 303.0 [M + H]⁺.

### Step 6:

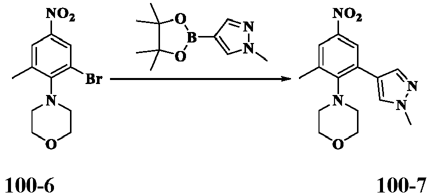

Prepared according to the method of step 5 in Embodiment 1, the raw materials were replaced with **100-6** (160 mg, 0.5 mmol) to obtain 130 mg of compound **100-7.**

MS (ESI) m/z: 303.1 [M + H]⁺.

### Step 7:

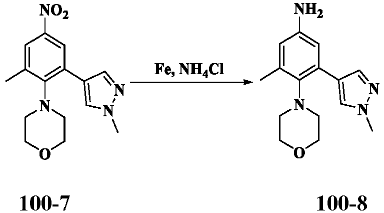

Prepared according to the method of step 3 in Embodiment 70, the raw materials were replaced with compound **100-7** (130 mg, 0.4 mmol) to obtain 90 mg of compound **100-8.**

MS (ESI) m/z: 273.1 [M + H]⁺.

### Step 8:

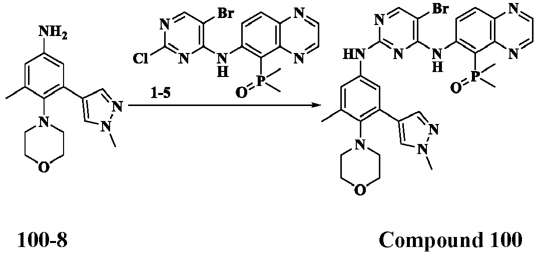

Prepared according to the method of step 7 in Embodiment 1, the raw materials were replaced with **100-8** (60 mg, 0.2 mmol) to obtain 62 mg of compound **100.**

MS (ESI) m/z: 648.2, 650.2 [M + H]⁺.

¹H-NMR (400 MHz, DMSO-*d*₆) δ 12.71 (s, 1H), 9.41 (s, 1H), 9.01-8.85 (m, 3H), 8.36 (s, 1H), 7.84-7.80 (m, 2H), 7.46 (s, 1H), 7.41-7.33 (m, 2H), 3.83 (s, 3H), 3.61-3.59 (m, 4H), 2.97 (s, 2H), 2.71 (s, 2H), 2.25 (s, 3H), 2.05 (d, *J* = 14.4 Hz, 6H).

### Embodiment 111:

### Preparation of (6-((5-bromo-2-((4-(cis-2,6-dimethylmorpholinyl)-2-methoxy-5-(1-methyl-1H-pyrazol-4-yl)phenyl)amino))pyrimidin-4-yl)amino)quinoxalin-5-yl)dimethylphosphine oxide (compound 111)

### Step 1:

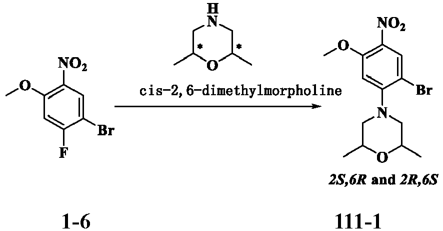

Prepared according to the method of step 4 in Embodiment 1, the raw materials were replaced with *cis*-2,6-dimethylmorpholine (345 mg, 3 mmol) to obtain 580 mg of compound **111-1.**

MS (ESI) m/z: 345.1, 347.1 [M + H]⁺.

### Step 2:

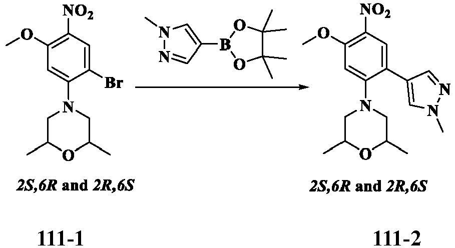

Prepared according to the method of step 5 in Embodiment 1, the raw materials were replaced with compound **111-1** (530 mg, 1.5 mmol) to obtain 500 mg of compound **111-2.**

MS (ESI) m/z: 347.2 [M + H]⁺.

### Step 3:

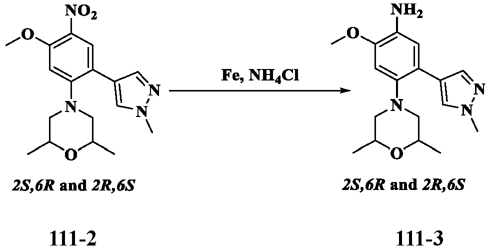

Prepared according to the method of step 3 in Embodiment 70, the raw materials were replaced with compound **111-2** (160 mg, 0.5 mmol) to obtain 130 mg of compound **111-3.**

MS (ESI) m/z: 317.2 [M + H]⁺.

### Step 4:

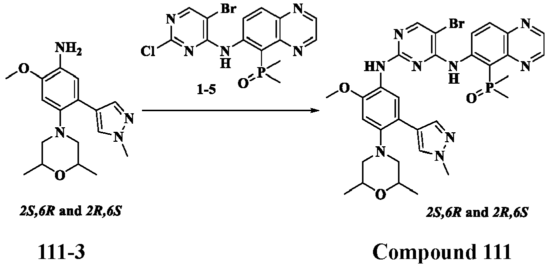

Prepared according to the method of step 7 in Embodiment 1, the raw materials were replaced with compound **111-3** (120 mg, 0.4 mmol) to obtain 98 mg of compound **111.**

MS: (ESI, *m*/*z*): 691.8, 693.8 [M + H]⁺.

¹H NMR (400 MHz, CDCl₃) δ 12.60 (s, 1H), 8.99 (dd, *J* = 9.2, 4.0 Hz, 1H), 8.75 (d, *J* = 2.0 Hz, 1H), 8.72 (d, *J* = 2.0 Hz, 1H), 8.31 (s, 1H), 8.25 (s, 1H), 7.74 (s, 1H), 7.63 (d, *J* = 8.8 Hz, 1H), 7.58 (s, 1H), 7.42 (s, 1H), 6.71 (s, 1H), 3.74 (s, 3H), 3.85-3.79 (m, 2H), 3.76 (s, 3H), 2.96 (d, *J* = 11.2 Hz, 2H), 2.41 (t, *J* = 10.8 Hz, 2H), 2.15 (d, *J* = 14.4 Hz, 6H), 1.20 (d, *J* = 6.0 Hz, 6H).

### Embodiment 121:

### Preparation of (6-((5-bromo-2-((4-(4-hydroxypiperidin-1-yl)-2-methoxy-5-(1-methyl-1H-pyrazol-4-yl)phenyl)amino]pyrimidin-4-yl)amino)quinoxalin-5-yl)dimethylphosphine oxide (compound 121)

### Step 1:

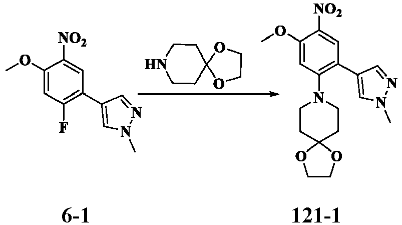

Prepared according to the method of step 1 in Embodiment 88, the raw materials were replaced with 4-piperidone glycol acetal (0.68 g, 4.7 mmol) to obtain 1 g of compound **121-1.**

MS: (ESI, *m*/*z*): 375.1 [M + H]⁺.

### Step 2:

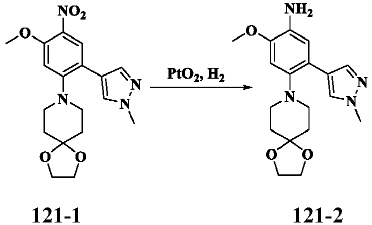

Prepared according to the method of step 6 in Embodiment 1, the raw materials were replaced with compound **121-1** (1 g, 2.7 mmol) to obtain 0.7 g of compound **121-2.**

MS (ESI) m/z: 345.3 [M + H]⁺.

### Step 3:

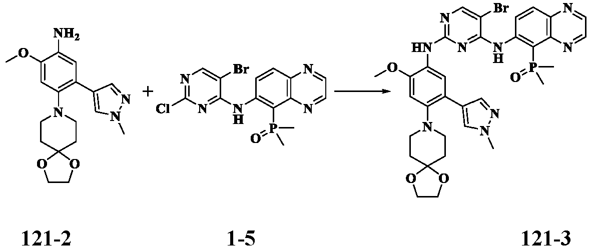

Compound **121-2** (600 mg, 1.7 mmol) and compound **1-5** (719 mg, 1.7 mmol) were dissolved in 1,4-dioxane (6 mL) at room temperature under nitrogen protection. Then, cesium carbonate (1.1 g, 3.5 mmol) and PEPPSI^{™}-IPr (CAS#: 905459-27-0, 118 mg, 0.2 mmol) were added to the reaction solution. The reaction system was heated to 90 °C and continued to stir for 16 hours. After LCMS monitoring showed that the raw materials disappeared, the reaction solution was cooled to room temperature, and water (20 mL) was added to the reaction solution to quench. The mixture was extracted with ethyl acetate (50 mL × 3 times), and the organic phases were combined. The organic phases were washed with saturated brine (50 mL × 3 times), then dried over anhydrous sodium sulfate, filtered, and concentrated under reduced pressure. The resulting residue was purified by silica gel column chromatography (eluent: dichloromethane/methanol = 10/1) to obtain 400 mg of compound **121-3.**

MS (ESI) m/z: 720.1, 722.1 [M + H]⁺.

### Step 4:

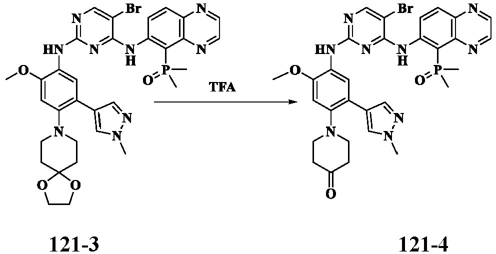

Prepared according to the method of step 4 in Embodiment 93, the raw materials were replaced with compound **121-3** (0.4 g, 0.6 mmol) to obtain 0.11 g of compound **121-4.**

MS (ESI) m/z: 676.0, 678.0 [M + H]⁺.

### Step 5:

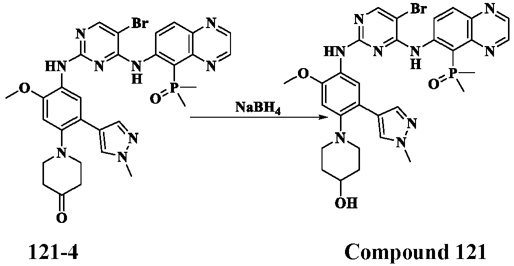

Compound **121-4** (50 mg, 0.07 mmol) was dissolved in methanol (1 mL). Then, sodium borohydride (4 mg, 0.1 mmol) was added to the reaction solution at 0 °C. The reaction solution was heated to room temperature and continued to stir for 1 hour. After LCMS monitoring showed that the raw materials disappeared, the reaction solution was subjected to reversed-phase preparation: chromatographic column: 80 g C18 reversed-phase column; mobile phase: water (with 10 mM ammonium bicarbonate) and acetonitrile; flow rate: 50 mL/min; gradient: acetonitrile increased from 10 % to 60 % within 30 minutes; detection wavelength: 254 nm. 25 mg of compound **121** was obtained.

MS (ESI) m/z: 678.4, 680.4 [M + H]⁺.

¹H NMR (300 MHz, CDCl₃) δ 12.64 (s, 1H), 9.00 (dd, *J* = 9.6, 3.2 Hz, 1H), 8.74 (dd, *J* = 9.0, 1.8 Hz, 2H), 8.30 (s, 1H), 8.22 (s, 1H), 7.81 (s, 1H), 7.63-7.43 (m, 3H), 6.75 (s, 1H), 3.92-3.80 (m, 7H), 3.16-3.12 (m, 2H), 2.75-2.68 (m, 2H), 2.15 *(d, J=* 14.4 Hz, 6H), 2.02-1.98 (m, 2H), 1.76-1.68 (m, 3H).

### Embodiment 133:

### Preparation of (6-((5-bromo-2-((4-(9-(2-fluoroethyl)-3,9-diazaspiro[5.5]undecan-3-yl)-2-methoxy-5-(1-methyl-1H-pyrazol-4-yl)phenyl)amino)pyrimidin-4-yl)amino)quinoxalin-5-yl)dimethylphosphine oxide (compound 133)

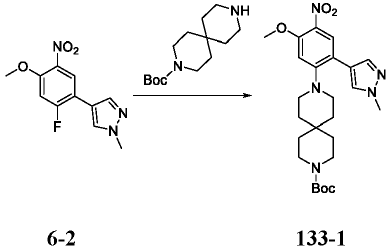

Prepared according to the method of step 1 in Embodiment 88, the raw materials were replaced with *tert*-butyl 3,9-diazaspiro[5.5]undecane-3-carboxylate (1.22 g, 4.8 mmol) to obtain 1.6 g of compound 133-1.

MS (ESI) m/z: 486.2 [M + H]⁺.

### Step 2:

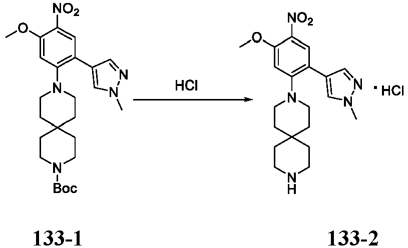

Compound **133-1** (1.6 g, 3.3 mmol) was dissolved in dichloromethane (10 mL); and then at 0 °C, 1,4-dioxane solution of hydrogen chloride (4 mol/L, 10 mL) was added to the above reaction solution. The reaction system was heated to room temperature and continued to stir for 1 hour; after LCMS monitoring showed that the raw materials disappeared, the reaction solution was concentrated under reduced pressure to obtain 1.38 g of compound **133-2** hydrochloride.

MS (ESI) m/z: 386.1 [M + H]⁺.

### Step 3:

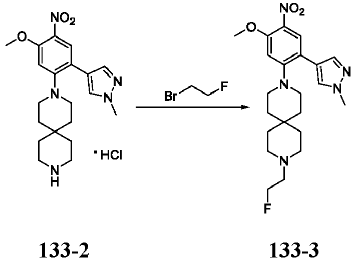

Compound **133-2** hydrochloride (200 mg, 0.47 mmol) and 1-bromo-2-fluoroethane (132 mg, 1.04 mmol) were dissolved in acetonitrile (5 mL). Then, anhydrous potassium carbonate (359 mg, 2.6 mmol) was added to the above reaction solution. The reaction system was heated to 80 °C and continued to stir for 16 hours. After LCMS monitoring showed that the raw materials disappeared, the reaction solution was cooled to room temperature and concentrated under reduced pressure. The resulting residue was purified by silica gel column chromatography (eluent: dichloromethane/methanol = 8/1) to obtain 130 mg of compound **133-**3.

MS (ESI) m/z: 432.2 [M + H]⁺.

### Step 4:

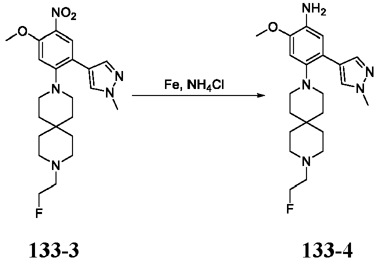

Prepared according to the method of step 3 in Embodiment 70, the raw materials were replaced with compound **133-3** (130 mg, 0.3 mmol) to obtain 80 mg of compound **133-4.**

MS (ESI) m/z: 401.5 [M + H]⁺.

### Step 5:

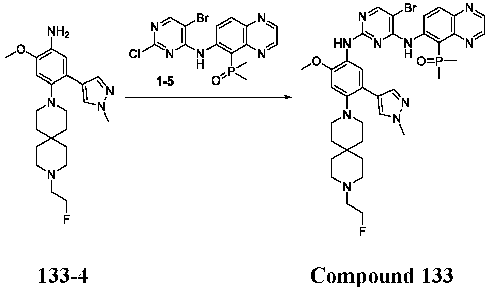

Prepared according to the method of step 7 in Embodiment 1, the raw materials were replaced with compound **133-4** (70 mg, 0.17 mmol) to obtain 51 mg of compound **133.**

MS (ESI) m/z: 777.2, 779.2 [M + H]⁺.

¹H-NMR (300 MHz, DMSO-*d*₆) δ 12.67 (s, 1H), 8.85-8.75 (m, 3H), 8.43 (s, 1H), 8.27 (s, 1H), 8.01 (s, 1H), 7.81 (s, 1H), 7.54 (s, 2H), 6.89 (s, 1H), 4.64-4.60 (m, 1H), 4.48-4.44 (m, 1H), 3.81 (s, 3H), 3.76 (s, 3H), 2.84-2.80 (m, 4H), 2.73-2.67 (m, 1H), 2.60-2.57 (m, 1H), 2.46 (s, 4H), 2.01 (d, *J* = 14.4 Hz, 6H), 1.56 (s, 8H).

¹⁹F NMR (377 MHz, DMSO-*d₆*) δ -216.89.

### Embodiment 135:

### Preparation of (6-((5-bromo-2-((4-(trans-2,6-dimethylmorpholinyl)-2-methoxy-5-(1-methyl-1H-pyrazol-4-yl)phenyl)amino))pyrimidin-4-yl)amino)quinoxalin-5-yl)dimethylphosphine oxide (compound 135)

### Step 1:

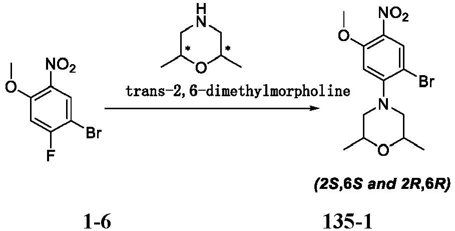

Prepared according to the method of step 4 in Embodiment 1, the raw materials were replaced with *trans*-2,6-dimethylmorpholine (racemic mixture of 2*S*,6*S* configuration and 2*R*,6*R*, 553 mg, 4.8 mmol) to obtain 1.02 g of compound **135-1.**

MS (ESI, m/z): 345.0, 347.0 [M + H]⁺.

### Step 2:

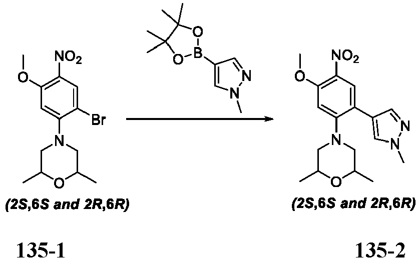

Prepared according to the method of step 5 in Embodiment 1, the raw materials were replaced with compound **135-1** (1 g, 2.9 mmol) to obtain 828 mg of compound **135-2.**

MS (ESI, m/z): 347.1 [M + H]⁺.

### Step 3:

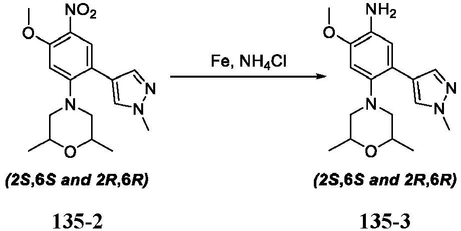

Prepared according to the method of step 3 in Embodiment 70, the raw materials were replaced with compound **135-2** (150 mg, 0.43 mmol) to obtain 134 mg of compound **135-3.**

MS (ESI) m/z: 317.1 [M + H]⁺.

### Step 4:

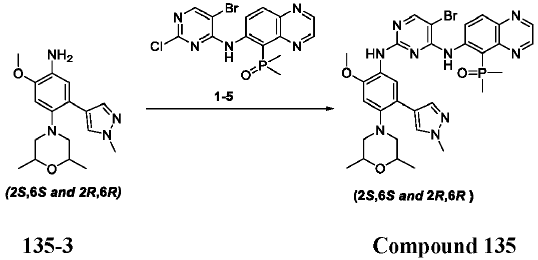

Prepared according to the method of step 7 in Embodiment 1, the raw materials were replaced with compound **135-3** (70 mg, 0.2 mmol) to obtain 80 mg of racemic compound **135.**

MS (ESI) m/z: 692.1, 694.1 [M + H]⁺.

¹H NMR (300 MHz, DMSO-*d₆*) δ 12.67 (s, 1H), 8.87-8.80 (m, 2H), 8.76 (s, 1H), 8.45 (s, 1H), 8.28 (s, 1H), 7.89 (s, 1H), 7.69 (s, 1H), 7.59-7.53 (m, 2H), 6.84 (s, 1H), 4.07-4.02 (m, 2H), 3.83 (s, 3H), 3.77 (s, 3H), 3.32 (s, 2H), 2.98-2.93 (m, 2H), 2.02 (d, *J* = 14.4 Hz, 6H), 1.18 (d, *J* = 6.3 Hz, 6H).

### Embodiment 144:

### Preparation of (6-((5-bromo-2-((2-methoxy-5-(1-methyl-1H-pyrazol-4-yl)-4-(4-methyl-1-oxa-4,9-diazaspiro[5.5]undecan-9-yl)phenyl)amino)pyrimidin-4-yl)amino)quinoxalin-5-yl)dimethylphosphine oxide (compound 144)

### Step 1:

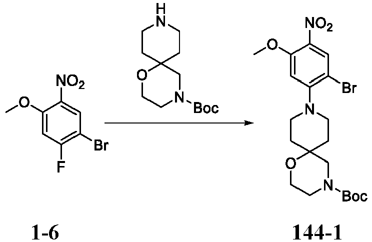

Prepared according to the method of step 4 in Embodiment 1, the raw materials were replaced with tert-butyl 1-oxa-4,9-diazaspiro[5.5]undecane-4-carboxylate (615 mg, 2.4 mmol) to obtain 900 mg of compound **144-1.**

MS (ESI, m/z): 486.1, 488.1 [M + H]⁺.

### Step 2:

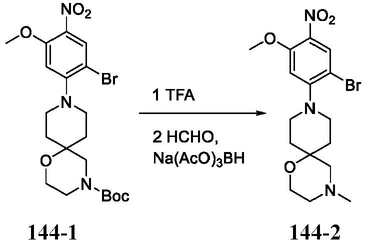

Compound **144-1** (900 mg, 1.85 mmol) was dissolved in dichloromethane (10 mL). Trifluoroacetic acid (3 mL) was then added to the above reaction solution and the mixture was continued to stir at room temperature for 2 hours. After LCMS monitoring showed that the raw materials disappeared, the reaction solution was concentrated under reduced pressure. The resulting residue was dissolved in methanol (10 mL). Then, glacial acetic acid (223 mg, 3.71 mmol) and formaldehyde aqueous solution (37 %, 2 mL) were added to the above reaction solution and the mixture was continued to stir at room temperature for 30 minutes. Sodium triacetoxyborohydride (787 mg, 3.71 mmol) was added to the above reaction solution in batches, and the mixture was continued to stir at room temperature for 1.5 hours. After LCMS monitoring showed that the raw materials disappeared, saturated sodium bicarbonate aqueous solution (50 mL) was added to the reaction solution to quench. The mixture was extracted with dichloromethane (80 mL × 3 times), and the organic phases were combined. The organic phases were washed with saturated brine (50 mL × 3 times), then dried over anhydrous sodium sulfate, filtered, and concentrated under reduced pressure. 600 mg of compound **144-2** was obtained.

MS (ESI) m/z: 400.0, 402.0 [M + H]⁺.

### Step 3:

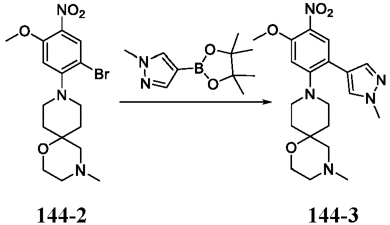

Prepared according to the method of step 5 in Embodiment 1, the raw materials were replaced with compound **144-2** (420 mg, 1.05 mmol) to obtain 310 mg of compound **144-3.**

MS (ESI, m/z): 402.2 [M + H]⁺.

### Step 4:

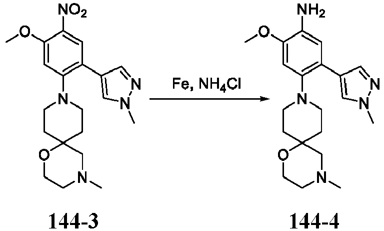

Prepared according to the method of step 3 in Embodiment 70, the raw materials were replaced with compound **144-3** (300 mg, 0.75 mmol) to obtain 142 mg of compound **144-4.**

MS (ESI) m/z: 372.2 [M + H]⁺.

### Step 5:

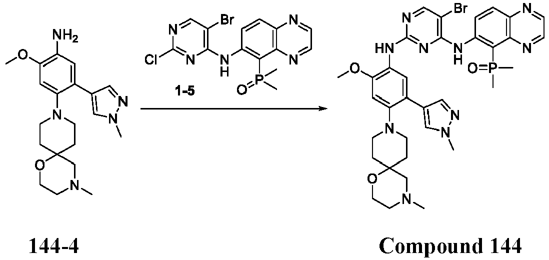

Prepared according to the method of step 7 in Embodiment 1, the raw materials were replaced with compound **144-4** (72 mg, 0.2 mmol) to obtain 70 mg of compound **144.**

MS: (ESI, *m*/*z*): 747.2, 749.2 [M + H]⁺.

¹H-NMR (400 MHz, CDCl₃) δ 12.59 (s, 1H), 9.01 (dd, *J* = 9.6, 4.0 Hz, 1H), 8.75 (d, *J* = 2.0 Hz, 1H), 8.71 (d, *J* = 2.0 Hz, 1H), 8.31 (s, 1H), 8.22 (s, 1H), 7.69 (s, 1H), 7.64-7.60 (m, 2H), 7.35 (s, 1H), 6.77 (s, 1H), 3.93 (s, 3H), 3.88 (s, 2H), 3.77 (s, 3H), 2.97-2.84 (m, 4H), 2.65-2.47 (m, 7H), 2.15 (d, *J* = 14.0 Hz, 8H), 1.74 (s, 2H).

### Embodiment 147:

### Preparation of N (4-((5-bromo-2-((2-methoxy-5-(1-methyl-1H-pyrazol-4-yl)-4-morpholinylphenyl)amino)pyrimidin-4-yl)amino)bicyclo [4.2.0] octa-1,3,5-trien-3-yl)-N-methylmethanesulfonamide (compound 147)

### Step 1:

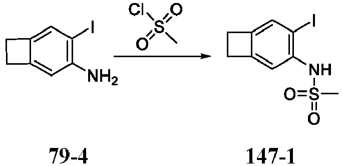

Prepared according to the method of step 4 in Embodiment 80, the raw materials were replaced with compound **79-4** (1 g, 4.1 mmol) to obtain 1.1 g of compound **147-1.**

MS (ESI) m/z: 321.8 [M - H]⁺.

### Step 2:

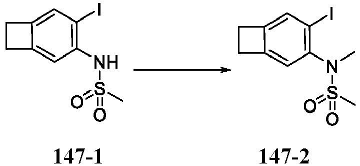

Compound **147-1** (1.4 g, 4.3 mmol) and anhydrous potassium carbonate (1.2 g, 8.5 mmol) were dissolved in *N,N-*dimethylformamide (15 mL). Then, iodomethane (909 mg, 6.4 mmol) was added to the above reaction solution and the mixture was continued to stir for 2 hours at room temperature. After LCMS monitoring showed that the raw materials disappeared, water (100 mL) was added to the reaction solution to quench. The mixture was extracted with ethyl acetate (100 mL × 3 times), and the organic phases were combined. The organic phases were washed with saturated brine (100 mL × 3 times), then dried over anhydrous sodium sulfate, filtered, and concentrated under reduced pressure. The resulting residue was purified by silica gel column chromatography (eluent: petroleum ether/ethyl acetate = 3/1) to obtain 1.3 g of compound **147-2.**

MS (ESI) m/z: 338.1 [M + H]⁺.

### Step 3:

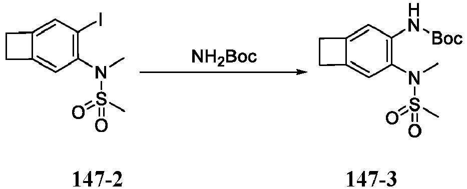

Prepared according to the method of step 1 in Embodiment 79, the raw materials were replaced with compound **147-2** (1.3 g, 3.7 mmol) to obtain 0.52 g of compound **147-3.**

MS (ESI) m/z: 325.0 [M - H]⁺.

### Step 4:

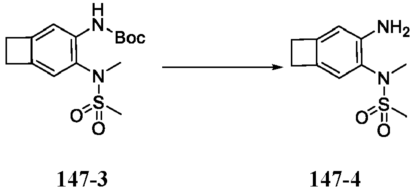

Prepared according to the method of step 2 in Embodiment 79, the raw materials were replaced with compound **147-3** (520 mg, 1.6 mmol) to obtain 250 mg of compound **147-4.**

MS (ESI) m/z: 227.2 [M - H]⁺.

### Step 5:

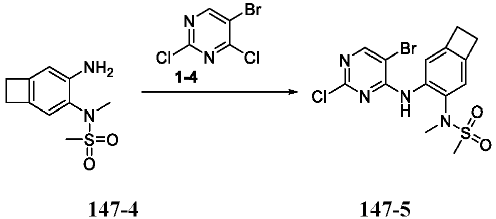

5-Bromo-2,4-dichloropyrimidine (2 g, 8.8 mmol) was dissolved in *N*,*N*-dimethylformamide (30 mL), and then anhydrous potassium carbonate (3.64 g, 26.3 mmol) and **147-4** (240 mg, 1.1 mmol) were added sequentially. The reaction solution was heated to 60 °C and continued to stir for 12 hours.

After LCMS monitoring showed that the raw materials disappeared, the reaction solution was cooled to room temperature, and water (150 mL) was added to the reaction solution to quench. The mixture was extracted with ethyl acetate (100 mL × 3 times), and the organic phases were combined. The organic phases were washed with saturated brine (80 mL × 3 times), then dried over anhydrous sodium sulfate, filtered, and concentrated under reduced pressure; the resulting residue was purified by silica gel column chromatography (eluent: dichloromethane/methanol=10/1) to obtain 200 mg of compound **147-5.**

MS (ESI) m/z: 416.9, 418.9 [M + H]⁺.

### Step 6:

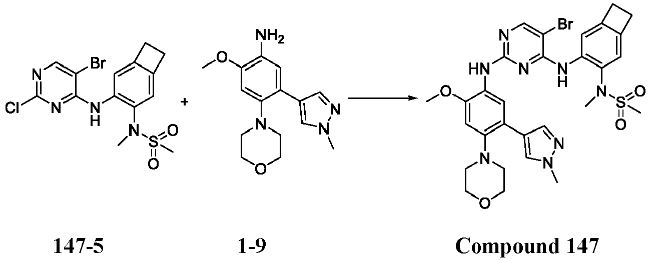

Prepared according to the method of step 7 in Embodiment 1, the raw materials were replaced with compound **147-5** (100 mg, 0.24 mmol) to obtain 62 mg of compound **147.**

MS (ESI) m/z: 669.2, 671.2 [M + H]⁺.

¹H NMR (300 MHz, CD₃OD) δ 8.12 (s, 1H), 8.04 (s, 1H), 7.76 (s, 1H), 7.72 (s, 1H), 7.68 (s, 1H), 7.26 (s, 1H), 6.83 (s, 1H), 3.92 (s, 3H), 3.85 (s, 3H), 3.83-3.79 (m, 4H), 3.29 (s, 3H), 3.06 (s, 3H), 3.03-2.97 (m, 2H), 2.91-2.86 (m, 4H), 2.83-2.68 (m, 2H).

### Embodiment 148:

### Preparation of (6-((5-bromo-2-((2-methoxy-5-(1-methyl-1H-pyrazol-4-yl)-4-(4-(2-methylmorpholinyl)piperidin-1-yl)phenyl)amino)pyrimidin-4-yl)amino)quinoxalin-5-yl)dimethylphosphine oxide (compound 148)

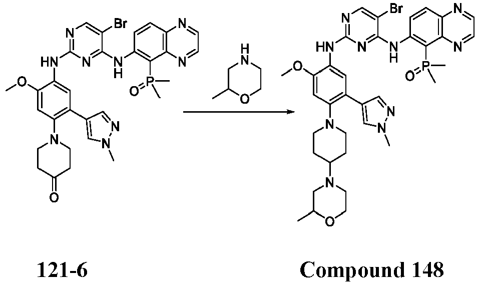

Compound **121-6** (50 mg, 0.07 mmol) was dissolved in dichloromethane. Then, glacial acetic acid (8.8 mg, 0.15 mmol) and 2-methylmorpholine (15 mg, 0.15 mmol) were added to the above reaction solution and the mixture was continued to stir at room temperature for 30 minutes. Sodium triacetoxyborohydride (47 mg, 0.2 mmol) was added to the reaction solution, and the mixture was continued to stir at room temperature for 2 hours. After LCMS monitoring showed that the raw materials disappeared, the reaction solution was concentrated under reduced pressure. The resulting residue was purified by C18 column. Purification conditions were as follows: chromatographic column: 20 g C18 reversed-phase column; mobile phase water (containing 0.1 % formic acid) and acetonitrile; flow rate of 15 mL/min; gradient: acetonitrile increased from 10 % to 70 % within 30 minutes; detection wavelength of 254 nm. The product was collected and lyophilized under reduced pressure to obtain 23 mg of compound **148** (racemic).

MS (ESI) m/z: 761.2, 763.2 [M + H]⁺.

¹H NMR (300 MHz, CDCl₃) δ 12.60 (s, 1H), 9.01 (dd, *J* = 9.6, 4.2 Hz, 1H), 8.78-8.64 (m, 2H), 8.31 (s, 1H), 8.24 (s, 1H), 7.76 (s, 1H), 7.61 *(d, J=* 9.6 Hz, 1H), 7.54 (s, 1H), 7.34 (s, 1H), 6.72 (s, 1H), 3.92 (s, 4H), 3.79 (s, 5H), 3.26-3.22 (m, 2H), 2.92-2.86 (m, 2H), 2.65-2.57 (m, 2H), 2.32 (s, 2H), 2.17 (s, 3H), 2.12 (s, 3H), 1.98 (s, 3H), 1.28 (s, 2H), 1.21 (d, *J* = 6.2 Hz, 3H).

### Embodiment 150:

### Preparation of (6-((5-bromo-2-((2-methoxy-5-(1-methyl-1H-pyrazol-4-yl)-4-(4-(oxetan-3-yl)piperazin-1-yl)phenyl)amino)pyrimidin-4-yl)amino)quinoxalin-5-yl)dimethylphosphine oxide (compound 150)

### Step 1:

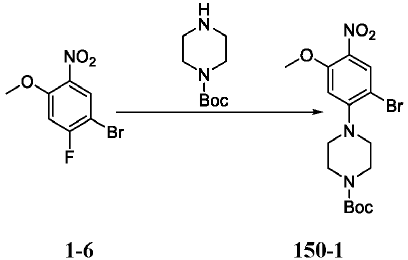

Prepared according to the method of step 4 in Embodiment 1, the raw materials were replaced with 1-*tert*-butoxycarbonylpiperazine (4.5 g, 24 mmol) to obtain 8 g of compound **150-1.**

MS (ESI, m/z): 416.0, 418.0 [M + H]⁺.

### Step 2:

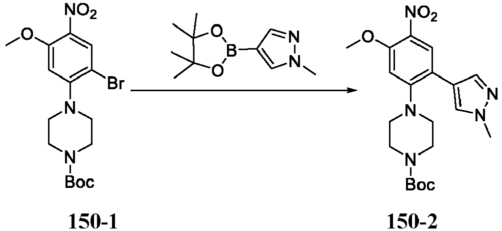

Prepared according to the method of step 5 in Embodiment 1, the raw materials were replaced with compound **150-1** (7.9 g, 19 mmol) to obtain 7 g of compound **150-2.**

MS (ESI, m/z): 418.1 [M + H]⁺.

### Step 3:

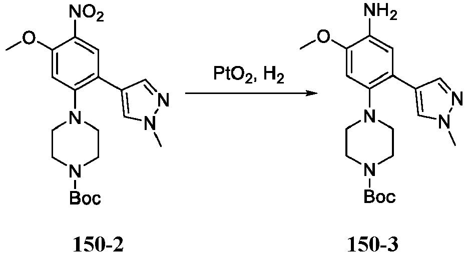

Prepared according to the method of step 6 in Embodiment 1, the raw materials were replaced with compound **150-2** (5.5 g, 13.2 mmol) to obtain 3.6 g of compound **150-3.**

MS (ESI, m/z): 388.3 [M + H]⁺.

### Step 4:

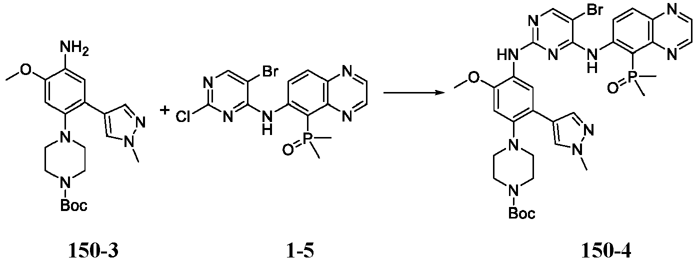

Compounds **1-5** (3.7 g, 9 mmol) and **150-3** (3.5 g, 9.0 mmol) were dissolved in dioxane (5 mL) under nitrogen atmosphere. Then, dichloro-[1,3-bis(diisopropylphenyl)imidazolylidene]-(3-chloropyridyl)palladium (35 mg, 0.05 mmol) and cesium carbonate (249 mg, 0.8 mmol) were added to the above reaction solution. The reaction system was heated to 95 °C and continued to stir for 16 hours. After LCMS monitoring showed that the raw materials disappeared, the reaction solution was cooled to room temperature and concentrated under reduced pressure. The resulting residue was purified by silica gel column chromatography (eluent: dichloromethane/methanol = 10/1) to obtain 3.2 g of compound **150-4.**

MS (ESI, m/z): 763.3, 765.3 [M + H]⁺.

### Step 5:

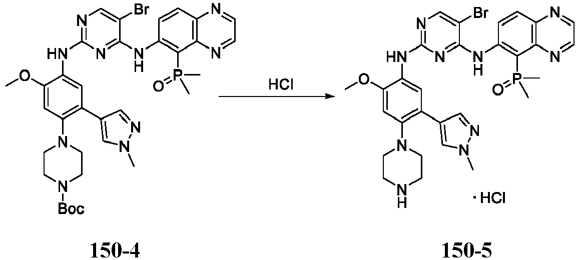

Prepared according to the method of step 2 in Embodiment 133, the raw materials were replaced with compound **150-4** (1 g, 1.3 mmol) to obtain 0.9 g of compound **150-5.**

MS (ESI, m/z): 663.3, 665.3 [M + H]⁺.

### Step 6:

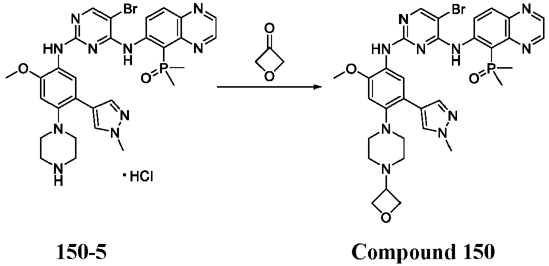

Prepared according to the method of step 1 in Embodiment 148, the raw materials were replaced with compound **150-5** (150 mg, 0.23 mmol) and 3-oxetanone (49 mg, 0.68 mmol) to obtain 40 mg of compound **150.**

MS (ESI, m/z): 719.4, 721.4 [M + H]⁺.

¹H NMR (400 MHz, CDCl₃) δ 12.58 (s, 1H), 8.98 (dd, *J* = 9.6, 4.4 Hz, 1H), 8.72 (dd, *J* = 13.2, 2..0 Hz, 2H), 8.29 (s, 1H), 8.22 (s, 1H), 7.62 (s, 3H), 7.38 (s, 1H), 6.75 (s, 1H), 4.70 (d, *J* = 6.8 Hz, 4H), 3.90 (s, 3H), 3.72 (s, 3H), 3.63 (s, 1H), 3.01 (s, 4H), 2.49 (s, 4H),2.13 (d, *J=* 14.4 Hz, 6H).

### Embodiment 153:

### Preparation of (6-((5-bromo-2-((4-(3-(fluoromethyl)-4-methylpiperazin-1-yl)-2-methoxy-5-(1-methyl-1H-pyrazol-4-yl))phenyl)amino)pyrimidin-4-yl)amino)quinoxalin-5-yl)dimethylphosphine oxide (compound 153)

### Step 1:

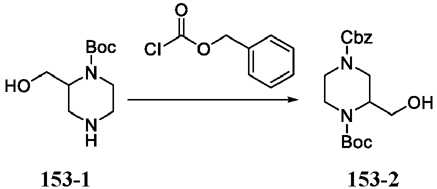

*tert*-Butyl 2-(hydroxymethyl)piperazine-1-carboxylate (2 g, 9.3 mmol) and triethylamine (2.8 g, 27.8 mmol) were dissolved in dichloromethane (25 mL). Benzyl chloroformate (3.2 g, 18.5 mmol) was added to the above reaction solution at 0 °C. The reaction system was heated to room temperature and continued to stir for 3 hours. After TLC monitoring showed that the raw materials disappeared, ice water (25 g) was added to the reaction solution to quench. The mixture was extracted with ethyl acetate (50 mL × 3 times), and the organic phases were combined. The organic phases were washed with saturated brine (50 mL × 3 times), then dried over anhydrous sodium sulfate, filtered, and concentrated under reduced pressure. The resulting residue was purified by silica gel column chromatography (eluent: petroleum ether/ethyl acetate = 5/1) to obtain 3 g of compound **153-2.**

¹H NMR (300 MHz, DMSO-*d*₆) δ 7.43-7.27 (m, 5H), 5.09 (s, 2H), 4.83 (s, 1H), 4.13-3.66 (m, 4H), 3.41 (dd, *J* = 16.2, 9.6 Hz, 1H), 3.30 (s, 1H), 2.92 (d, *J* = 10.8 Hz, 3H), 1.40 (s, 9H).

### Step 2:

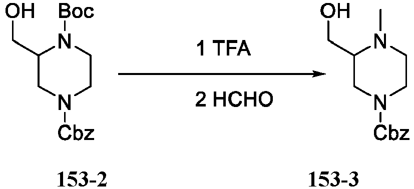

Prepared according to the method of step 2 in Embodiment 144, the raw materials were replaced with compound **153-2** (3 g, 8.6 mmol) to obtain 1.3 g of compound **153-3.**

MS (ESI, m/z): 265.2 [M + H]⁺.

### Step 3:

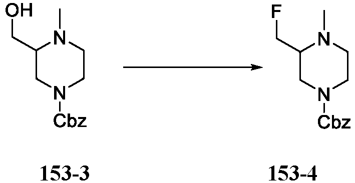

Compound **153-3** (1.3 g, 4.9 mmol) was dissolved in dichloromethane (20 mL). Then, diethylamino sulfur trifluoride (4.76 g, 29.5 mmol) was added dropwise to the above reaction solution under nitrogen atmosphere at -60 °C, and the mixture was continued to stir at this temperature for 30 minutes. The reaction system was heated to room temperature and continued to stir for 16 hours. After LCMS monitoring showed that the raw materials disappeared, sodium hydroxide aqueous solution (1 mol/L, 30 mL) was slowly added dropwise to the reaction solution at 0 °C to quench. The mixture was extracted with dichloromethane (50 mL × 3 times), and the organic phases were combined. The organic phases were washed with saturated brine (50 mL × 3 times), then dried over anhydrous sodium sulfate, filtered, and concentrated under reduced pressure. The resulting residue was purified by silica gel column chromatography (eluent: dichloromethane/methanol = 10/1) to obtain 0.78 g of compound **153-4.**

MS (ESI, m/z): 267.2 [M + H]⁺.

### Step 4:

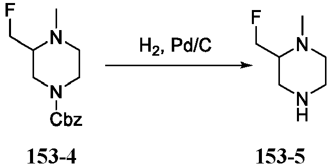

Compound **153-4** (780 mg, 2.9 mmol) was dissolved in ethanol (10 mL). Then, wet palladium on carbon (10 %, 250 mg) was added to the above solution; the reaction system was replaced with hydrogen for 3 times, and then stirred at room temperature for 16 hours. After LCMS monitoring showed that the raw materials disappeared, the reaction solution was filtered through diatomite, and the filter cake was washed with ethanol (50 mL × 3 times). The filtrate was concentrated under reduced pressure to obtain 250 mg of compound **153-5.**

MS (ESI) m/z: 133.3 [M + H]⁺.

### Step 5:

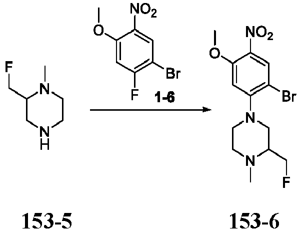

Prepared according to the method of step 4 in Embodiment 1, the raw materials were replaced with compound **153-5** (250 mg, 1.9 mmol) to obtain 400 mg of compound **153-6.**

MS (ESI, m/z): 362.0, 364.0 [M + H]⁺.

### Step 6:

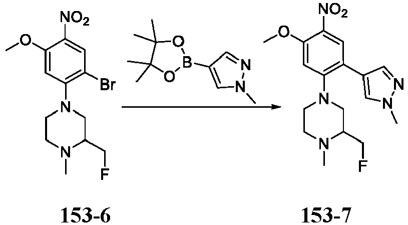

Prepared according to the method of step 5 in Embodiment 1, the raw materials were replaced with compound **153-6** (309 mg, 0.9 mmol) to obtain 220 mg of compound **153-7.**

MS (ESI, m/z): 364.1 [M + H]⁺.

### Step 7:

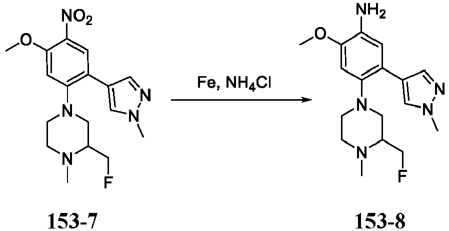

Prepared according to the method of step 3 in Embodiment 70, the raw materials were replaced with compound **153-7** (120 mg, 0.3 mmol) to obtain 100 mg of compound **153-8.**

MS (ESI, m/z): 334.3 [M + H]⁺.

### Step 8:

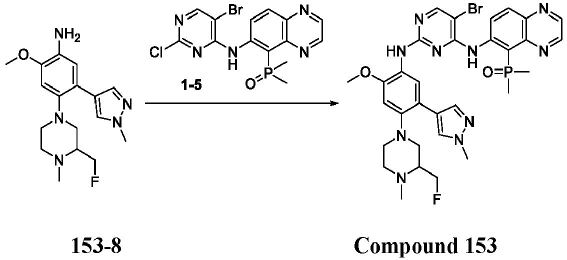

Prepared according to the method of step 7 in Embodiment 1, the raw materials were replaced with compound **153-8** (70 mg, 0.2 mmol) to obtain 30 mg of compound **153.**

MS (ESI, m/z): 709.3, 711.3 [M + H]⁺.

¹H NMR (300 MHz,CDCl₃) δ 12.56 (s, 1H), 8.97 (dd, *J =* 9.6, 4.2 Hz, 1H), 8.77-8.66 (m, 2H), 8.29 (s, 1H), 8.22 (s, 1H), 8.14 (s, 1H), 7.66-7.58 (m, 3H), 7.41 (s, 1H), 6.72 (s, 1H), 4.82 - 4.33 (m, 2H), 3.91 (s, 3H), 3.73 (s, 3H), 3.13-3.01 (m, 4H), 2.83 (s, 1H), 2.54 (s, 5H), 2.16 (s, 3H), 2.11 (s, 3H).

### Embodiment 155:

### (6-((5-Bromo-2-((4-((2S,6S)-2,6-dimethylmorpholinyl)-2-methoxy-5-(1-methyl-1H-pyrazol-4-yl)phenyl)amino)pyrimidin-4-yl)amino)quinoxalin-5-yl)dimethylphosphine oxide and

### (6-((5-bromo-2-((4-((2R,6R)-2,6-dimethylmorpholinyl)-2-methoxy-5-(1-methyl-1H-pyrazol-4-yl)phenyl)amino)pyrimidin-4-yl)amino)quinoxalin-5-yl)dimethylphosphine oxide

### Step 1:

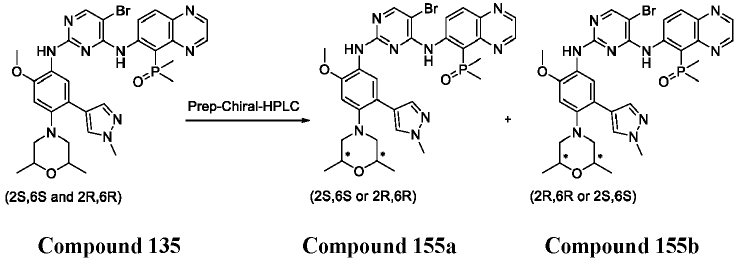

The racemic compound 135 (*trans,* 70 mg, 0.1 mmol) was subjected to a chiral resolution (resolution conditions: chiral column (*R*, *R*)-Whelk-01, 2.12 × 25 cm, 5 Jm; mobile phase A *n*-hexane (10 mM ammonia in methanol), mobile phase B ethanol; flow rate of 20 mL/min; gradient of 50 % B; detection wavelength of 210/270 nm) to obtain 23 mg of optically pure compound **155a** and 11 mg of optically pure compound **155b.**

**155a:** MS (ESI, m/z) 692.2, 694.2 [M + H]⁺.

[*α*]_{D}²⁵ = -31.5° (*c* = 0.35, MeOH)

¹H NMR (300 MHz, CDCl₃) δ 12.71 (s, 1H), 8.97 (dd, *J* = 9.6, 4.2 Hz, 1H), 8.75 (dd, *J=* 12.9, 1.8 Hz, 2H), 8.28 (s, 1H), 8.17 (s, 1H), 7.64 (s, 2H), 7.60 (s, 1H), 7.54 (s, 1H), 6.72 (s, 1H), 4.13-4.08 (m, 2H), 3.94 (s, 3H), 3.80 (s, 3H), 3.00-2.96 (m, 2H), 2.58-2.52 (m, 2H), 2.15 (d, *J* = 14.4 Hz, 6H), 1.26 (d, *J* = 6.6 Hz, 6H).

**155b:** MS (ESI, m/z) 692.2, 694.2 [M + H]⁺.

[*α*]_{D}²⁵ = +35.8° (c = 0.22, MeOH)

¹H NMR (300 MHz, CDCl₃) δ 12.73 (s, 1H), 8.97 (dd, *J* = 9.6, 4.2 Hz, 1H), 8.75 (dd, *J=* 13.2, 1.8 Hz, 2H), 8.27 (s, 1H), 8.16 (s, 1H), 7.64-7.54 (m, 4H), 6.72 (s, 1H), 4.13-4.08 (m, 2H), 3.94 (s, 3H), 3.81 (s, 3H), 3.00-2.96 (m, 2H), 2.58-2.52 (m, 2H), 2.15 *(d, J=* 14.1 Hz, 6H), 1.26 *(d, J=* 6.3 Hz, 6H).

### Embodiment 164:

### Preparation of (6-((5-bromo-2-((4-(3-(fluoromethyl)-4-isopropylpiperazin-1-yl)-2-methoxy-5-(1-methyl)-1H-pyrazol-4-yl)phenyl)amino)pyrimidin-4-yl)amino)quinoxalin-5-yl)dimethylphosphine oxide (compound 164)

### Step 1:

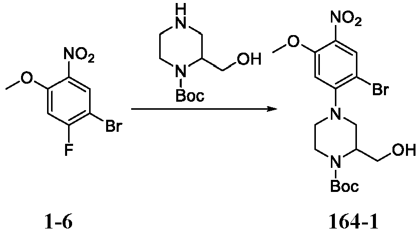

Prepared according to the method of step 4 in Embodiment 1, the raw materials were replaced with 1-*tert*-butoxycarbonyl-2-(hydroxymethyl)piperazine (1.1 g, 8 mmol) to obtain 1.7 g of compound **164-1.**

MS (ESI, m/z): 446.1, 448.1 [M + H]⁺.

### Step 2:

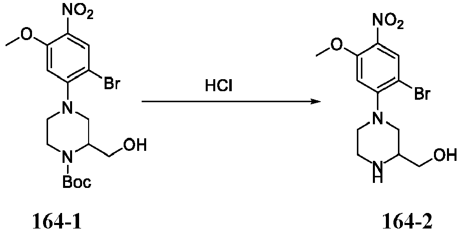

Prepared according to the method of step 2 in Embodiment 133, the raw materials were replaced with compound **164-1** (528 mg, 1.2 mmol) to obtain 453 mg of compound **164-2.**

MS (ESI, m/z): 346.1, 348.1 [M + H]⁺.

### Step 3:

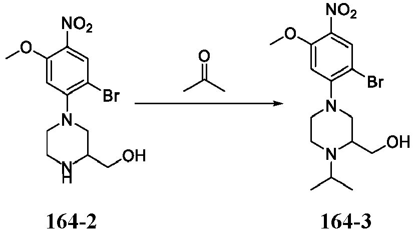

Compound **164-2** (409 mg, 1.2 mmol) was dissolved in 1,2-dichloroethane (13 mL). Then, acetone (823 mg, 14.2 mmol) was added to the above reaction solution and the mixture was continued to stir for 30 minutes; then, sodium triacetoxyborohydride (907 mg, 4.3 mmol) was added to the above reaction. The reaction system was continued to stir at room temperature for 2 hours. After LCMS monitoring showed that the raw materials disappeared, water (50 mL) was added to the reaction solution. The mixture was extracted with chloroform (100 mL × 3 times), and the organic phases were combined. The organic phases were washed with saturated brine (100 mL × 3 times) first, then dried over anhydrous sodium sulfate, filtered, and finally concentrated under reduced pressure. The resulting residue was purified by silica gel column chromatography (eluent: dichloromethane/methanol = 10/1) to obtain 310 mg of compound **164-3.**

MS (ESI, m/z): 388.2, 390.2 [M + H]⁺.

### Step 4:

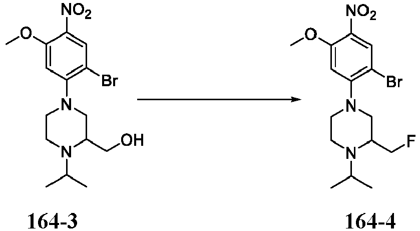

Prepared according to the method of step 3 in Embodiment 153, the raw materials were replaced with compound **164-3** (284 mg, 0.7 mmol) to obtain 108 mg of compound **164-4.**

MS (ESI, m/z): 390.0, 392.0 [M + H]⁺.

### Step 5:

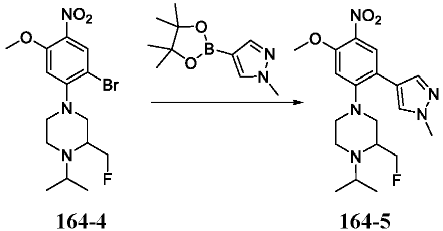

Prepared according to the method of step 5 in Embodiment 1, the raw materials were replaced with compound 164-4 (108 mg, 0.3 mmol) to obtain 59 mg of compound **164-5.**

MS (ESI, m/z): 392.1 [M + H]⁺.

### Step 6:

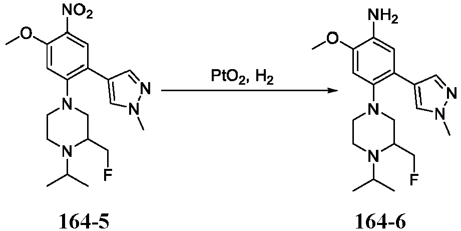

Prepared according to the method of step 6 in Embodiment 1, the corresponding raw materials were replaced with compound **164-5** (59 mg, 0.15 mmol) to obtain 59 mg of compound **164-6.**

MS (ESI, m/z): 362.3 [M + H]⁺.

### Step 7:

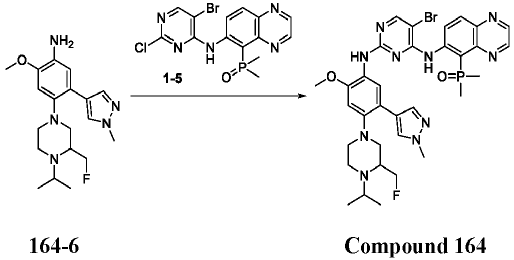

Prepared according to the method of step 7 in Embodiment 1, the raw materials were replaced with compound **164-6** (59 mg, 0.16 mmol) to obtain 25 mg of compound **164.**

MS (ESI, m/z): 737.2, 739.2 [M + H]⁺.

¹H NMR (300 MHz, CD₃OD) δ 8.86-8.74 (m, 3H), 8.43 (s, 1H), 8.26 (s, 1H), 7.94 (s, 1H), 7.73 (s, 1H), 7.55-7.50 (m, 2H), 6.84 (s, 1H), 4.70 (d, *J* = 3.9 Hz, 1H), 4.54 (d, *J* = 3.9 Hz, 1H), 3.92 (s, 3H), 3.67 (s, 3H), 3.53-3.48 (m, 1H), 3.23-2.96 (m, 4H), 2.91-2.71 (m, 3H), 2.15 (d, *J =* 14.4 Hz, 6H), 1.25 (d, *J* = 6.6 Hz, 3H), 1.15 (d, *J* = 6.6 Hz, 3H).

### Embodiment 168:

### Preparation of (6-((5-bromo-2-((2-methoxy-5-(1-methyl-1H-pyrazol-4-yl)-4-(1-methyloctahydro-6H-pyrrolo[2,3-c]pyridin-6-yl)phenyl)amino)pyrimidin-4-yl)amino)quinoxalin-5-yl)dimethylphosphine oxide (compound 168)

### Step 1:

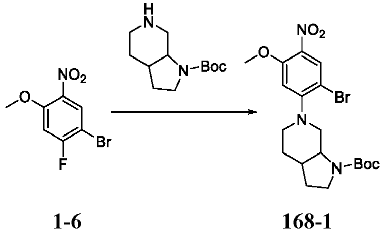

Prepared according to the method of step 4 in Embodiment 1, the raw materials were replaced with 7-*tert*-butoxycarbonyl-4,7-diazabicyclo[4.3.0]nonane (550 mg, 2.43 mmol) to obtain 700 mg of compound **168-1.**

MS (ESI, m/z): 456.0, 458.0 [M + H]⁺.

### Step 2:

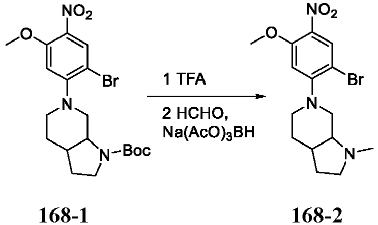

Prepared according to the method of step 2 in Embodiment 144, the raw materials were replaced with compound **168-1** (650 mg, 1.42 mmol) to obtain 550 mg of compound **168-2.**

MS (ESI, m/z): 370.1, 372.1 [M + H]⁺.

### Step 3:

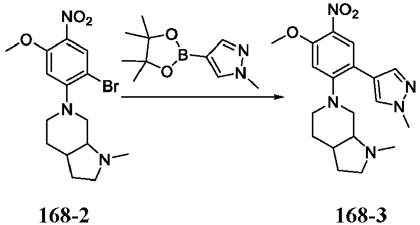

Prepared according to the method of step 5 in Embodiment 1, the raw materials were replaced with compound **168-2** (600 mg, 1.62 mmol) to obtain 360 mg of compound **168-3.**

MS (ESI, m/z): 372.3 [M + H]⁺.

### Step 4:

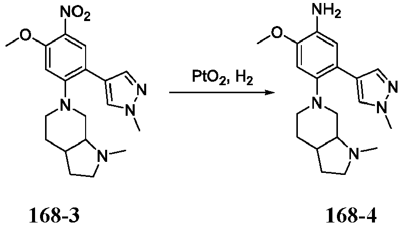

Prepared according to the method of step 6 in Embodiment 1, the raw materials were replaced with compound **168-3** (160 mg, 0.43 mmol) to obtain 130 mg of compound **168-4.**

MS (ESI, m/z): 342.3 [M + H]⁺.

### Step 5:

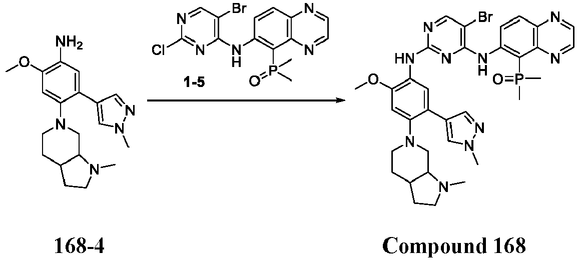

Prepared according to the method of step 7 in Embodiment 1, the raw materials were replaced with compound **168-4** (87 mg, 0.26 mmol) to obtain 47 mg of compound **168.**

MS (ESI, m/z): 717.2, 719.2 [M + H]⁺.

¹H NMR (400 MHz, CDCl₃) δ 12.56 (s, 1H), 8.96 (dd, *J* = 9.6, 4.4 Hz, 1H), 8.72 (s, 1H), 8.69 (s, 1H),, 8.28 (s, 1H), 8.23 (s, 1H), 8.16 (s, 1H), 7.59 (d, *J* = 10.0 Hz, 1H), 7.38 (s, 1H), 7.34 (s, 1H), 6.74 (s, 1H), 3.91 (s, 3H), 3.83 (s, 3H), 3.46 (s, 1H), 3.11-2.58 (m, 7H), 2.35 (s, 5H), 2.12 (d, *J=* 14.4 Hz, 8H).

### Embodiment 171:

### Preparation of N-(6-((5-bromo-2-((2-methoxy-5-(1-methyl-1-H-pyrazol-4-yl)-4-morpholinophenyl)amino)pyrimidin-4-yl)amino)quinoxalin-5-yl)-N methylmethanesulfonamide (compound 171)

### Step 1:

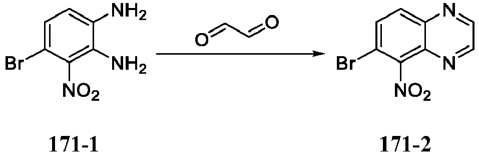

Compound **171-1** (15 g, 65 mmol) was dissolved in water (800 mL); and then an aqueous solution of glyoxal (40 %) (23.6 mL) was added to the above reaction solution. The resulting reaction system was heated to 100 °C and continued to stir for 4 hours.

After TLC monitoring showed that the raw materials disappeared, the reaction solution was cooled to room temperature. The precipitated solid was filtered, and the filter cake was washed with water (100 mL×2 times). The filter cake was dried to obtain 18.2 g of compound **171-2.**

¹H NMR (300 MHz, DMSO-*d*₆) δ 9.18 (d, *J* = 1.8 Hz, 1H), 9.10 (d, *J=* 1.8 Hz, 1H), 8.31 (d, *J* = 9.0 Hz, 1H), 8.27 (d, *J=* 9.0 Hz, 1H).

### Step 2:

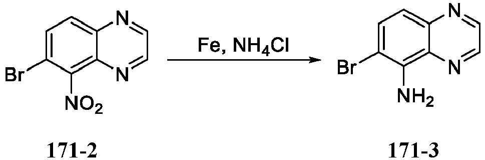

Compound **171-2** (16 g, 63 mmol) was dissolved in ethanol (250 mL). Then, ammonium chloride (50 g, 929 mmol), iron powder (65 g, 1.1 mol) and water (170 mL) were added to the above reaction; The resulting reaction system was heated to 90 °C and continued to stir for 2 hours.

After the LCMS monitoring showed that the raw materials disappeared, the reaction solution was cooled to room temperature and filtered. The filter cake was washed with ethyl acetate (100 mL × 2 times) and the filtrate was concentrated under reduced pressure. Water (1 L) was added to the resulting residue, and the mixture was extracted with ethyl acetate (1.5 L×2 times). The organic phases were combined, and the organic phases were washed with saturated brine (1 L×2 times) first, and then dried over anhydrous sodium sulfate, filtered, and finally concentrated under reduced pressure. The resulting residue was purified by silica gel column chromatography (eluent: petroleum ether/ethyl acetate = 1/1) to obtain 6.3 g of compound **171-3.**

¹H NMR (300 MHz, DMSO-*d*₆) δ 8.94 (d, *J* = 1.8 Hz, 1H), 8.82 (d, *J=* 1.8 Hz, 1H), 7.80 (d, *J* = 9.0 Hz, 1H), 7.20 (d, *J* = 9.0 Hz, 1H), 6.21 (br, 2H).

### Step 3:

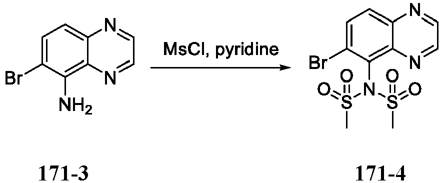

Prepared according to the method of step 4 in Embodiment 80, the raw materials were replaced with compound **171-3** (3 g, 13.4 mmol) to obtain 3.5 g of compound **171-4.**

¹H NMR (300 MHz, DMSO-*d*₆) δ 9.13-9.09 (m, 2H), 8.28 *(d, J=* 9.0 Hz, 1H), 8.22 (d, *J* = 9.0 Hz, 1H), 3.71-3.67 (m, 6H).

### Step 4:

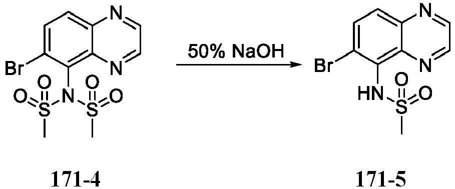

Compound **171-4** (8 g, 21 mmol) was dissolved in a mixed solvent of methanol (80 mL) and tetrahydrofuran (80 mL). Then, 50 % sodium hydroxide aqueous solution (33.6 g, 420 mmol) was added to the reaction solution at 0 °C. The reaction system was heated to room temperature and continued to stir for 1 hour. After LCMS monitoring showed that the raw materials disappeared, the reaction solution was diluted with water (200 mL), and then the pH was adjusted to 6 with concentrated hydrochloric acid. The mixture was extracted with a mixed solvent of chloroform and isopropanol (3:1, 80 mL × 3 times). The organic phases were combined, and the organic phases were washed with saturated brine (300 mL × 2 times) first, then dried over anhydrous sodium sulfate, filtered, and finally concentrated under reduced pressure; the resulting residue was purified by silica gel column chromatography (eluent: dichloromethane/methanol=5/1) to obtain 6 g of compound **171-5.**

¹H NMR (300 MHz, DMSO-*d*₆) δ 9.83 (br, 1H), 9.09-9.01 (m, 2H), 8.15 *(d, J=* 9.0 Hz, 1H), 8.01 (d, *J* = 9.0 Hz, 1H), 3.31 (s, 3H).

### Step 5:

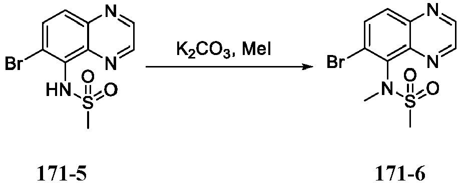

Prepared according to the method of step 2 in Embodiment 147, the raw materials were replaced with compound **171-5** (3 g, 9.9 mmol) to obtain 3 g of compound **171-6.**

¹H NMR (300 MHz, CDCl₃) δ 8.92-8.90 (m, 2H), 8.06-8.02 (m, 2H), 3.41 (s, 3H), 3.30 (s, 3H).

### Step 6:

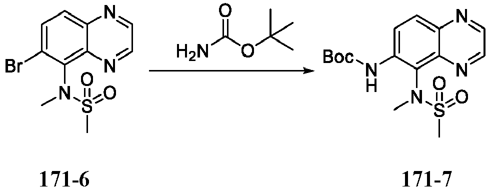

Prepared according to the method of step 1 in Embodiment 79, the raw materials were replaced with compound **171-6** (2.8 g, 8.8 mmol) to obtain 4 g of compound **171-7.**

¹H NMR (300 MHz, CDCl₃) δ 8.82 (d, *J* = 9.6 Hz, 1H), 8.79-8.77 (m, 2H), 8.10 (d, *J* = 9.6 Hz, 1H), 7.87 (br, 1H), 3.45 (s, 3H), 3.14 (s, 3H), 1.45 (s, 9H).

### Step 7:

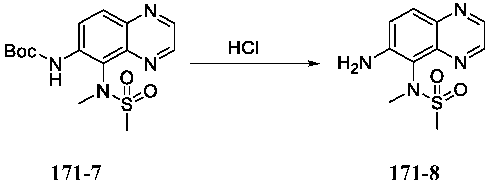

Prepared according to the method of step 2 in Embodiment 133, the raw materials were replaced with compound **171-7** (1 g, 2.8 mmol) to obtain 0.68 g of compound **171-8.**

¹H NMR (300 MHz, CDCl₃) δ 8.67 (d, *J* = 2.1 Hz, 1H), 8.58 (d, *J* = 2.1 Hz, 1H), 7.89 (d, *J* = 9.0 Hz, 1H), 7.30 (d, *J=* 9.0 Hz, 1H), 4.83 (br, 2H), 3.41 (s, 3H), 3.16 (s, 3H).

### Step 8:

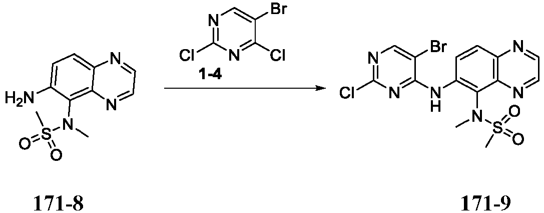

Prepared according to the method of step 3 in Embodiment 1, the raw materials were replaced with compound **171-8** (650 mg, 2.6 mmol) to obtain 1 g of compound 171-9.

¹H NMR (300 MHz, DMSO-*d*₆) δ 9.11 (s, 1H), 9.05-8.97 (m, 2H), 8.66 (s, 1H), 8.56 (d, *J* = 9.6 Hz, 1H), 8.25 (d, *J=* 9.3 Hz, 1H), 3.41 (s, 3H), 3.19 (s, 3H).

### Step 9:

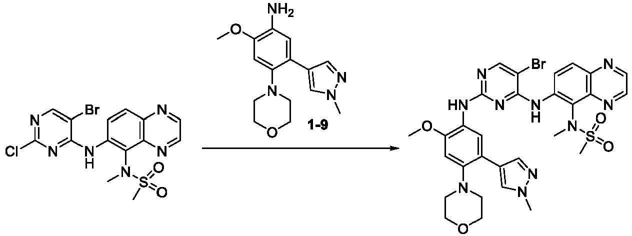

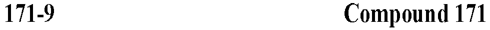

Prepared according to the method of step 7 in Embodiment 1, the raw materials were replaced with compound **171-9** (80 mg, 0.18 mmol) to obtain 18 mg of compound **171.**

MS (ESI) m/z: 695.1, 697.1 [M + H]⁺.

¹H NMR (300 MHz, CD₃OD) δ 8.91 (d, *J* = 1.8 Hz, 1H), 8.81 (d, *J* = 1.8 Hz, 1H), 8.73 (d, *J* = 9.3 Hz, 1H), 8.24 (s, 1H), 7.92 (s, 1H), 7.88 (s, 1H), 7.51 (s, 1H), 7.45 (d, *J=* 9.3 Hz, 1H), 6.83 (s, 1H), 3.91 (s, 3H), 3.82-3.78 (m, 4H), 3.72 (s, 3H), 3.49 (s, 3H), 3.20 (s, 3H), 2.92-2.86 (m, 4H).

### Embodiment 183:

### N-(2-((5-Bromo-2-((2-methoxy-5-(1-methyl-1H-pyrazol-4-yl)-4-)(9-methyl-3,9-diazaspiro[5.5]undecan-3-yl)phenyl)amino)pyrimidin-4-yl)amino)phenyl-7V-methylcyclopropanesulfonamide (Compound 183)

### Step 1:

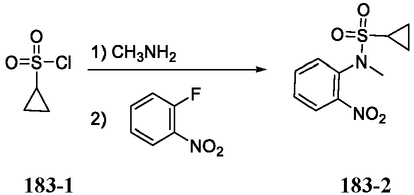

A tetrahydrofuran solution of triethylamine (2.16 g, 21.3 mmol) and methylamine (22 mL, 43 mmol) was dissolved in dichloromethane (16 mL) at 0 °C. Then, cyclopropylsulfonyl chloride (2 g, 14.2 mmol) was added to the above reaction solution. The reaction system was heated to room temperature and continued to stir for 24 hours. After LCMS monitoring showed that the raw materials disappeared, the reaction system was distilled under reduced pressure, and the resulting residue was dissolved in acetonitrile (20 mL). After cesium carbonate (5.8 g, 18 mmol) and 2-nitrofluorobenzene (2.5 g, 18 mmol) were added to the reaction solution, the mixture was continued to stir at room temperature for 24 hours. After TLC monitoring showed that the raw materials disappeared, the reaction system was concentrated under reduced pressure. The resulting residue was purified by silica gel column chromatography (eluent: petroleum ether/ethyl acetate = 1/1) to obtain 2.7 g of compound **183-2.**

MS (ESI) m/z: 257.0 [M + H]⁺.

### Step 2:

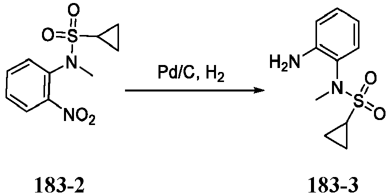

Prepared according to the method of step 6 in Embodiment 1, the corresponding raw materials were replaced with compound **183-2** (2.4 g, 9.37 mmol) to obtain 2.1 g of compound **183-3.**

MS (ESI, m/z): 227.1 [M + H]⁺.

### Step 3:

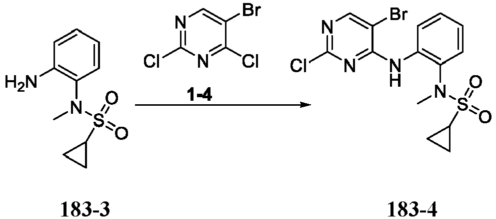

Prepared according to the method of step 1 in Embodiment 2, the corresponding raw materials were replaced with compound **183-3** (1 g, 4.6 mmol) to obtain 0.4 g of compound **183-4.**

MS (ESI, m/z): 416.9, 418.9 [M + H]⁺.

### Step 4:

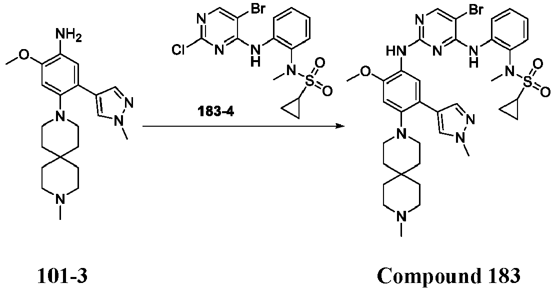

Prepared according to the method of step 7 in Embodiment 1, the corresponding raw materials were replaced with compound **183-4** (100 mg, 0.24 mmol) and compound **101-3** (133 mg, 0.36 mmol) to obtain 36 mg of compound **183.**

MS (ESI, m/z): 750.4, 752.4 [M + H]⁺.

¹H NMR (300 MHz, CD₃OD) δ 8.56 (s, 1H), 8.20-8.15 (m,2H), 7.92 (s, 1H), 7.83 (s, 1H), 7.70 (s, 1H), 7.61 (dd, *J* = 7.8, 1.5 Hz, 1H), 7.06-7.01 (m, 1H), 6.86-6.78 (m, 2H), 3.92 (s, 3H), 3.87 (s, 3H), 3.30 (s, 3H), 3.12 (s, 4H), 2.89 (s, 4H), 2.78 (s, 3H), 2.75-2.69 (m, 1H), 1.82-1.70 (m, 8H), 1.11-1.01 (m, 4H).

### Embodiment 200:

### (6-((5-Bromo-2-((5-(1-methyl-1H-pyrazol-4-yl)-3-(4-methylpiperazin-1-yl)benzo[d]isoxazol-7-yl)amino)pyrimidin-4-yl)aminoquinoxalin-5-yl)dimethylphosphine oxide

### Step 1:

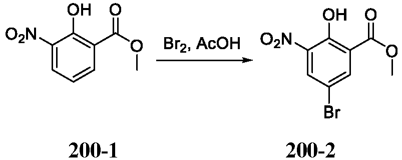

Methyl 2-hydroxy-3-nitrobenzoate (10.0 g, 50.7 mmol) was dissolved in glacial acetic acid (150 g) at room temperature. Then, bromine (3.9 mL) was added dropwise to the above reaction solution. The reaction system was continued to stir at room temperature for 16 hours. After LCMS monitoring showed that the raw materials disappeared, the reaction solution was poured into water (500 mL). The precipitated solid was filtered and dried to obtain 13 g of compound **200-2.**

MS (ESI) m/z: 274.0, 276.0 [M - H]⁺.

### Step 2:

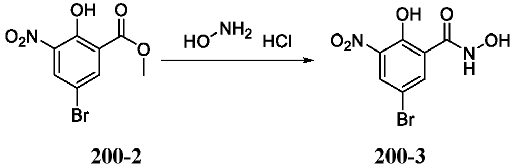

Compound **200-2** (1 g, 3.6 mmol) and hydroxylamine hydrochloride (1.01 g, 14.49 mmol) were dissolved in methanol (80 mL) at room temperature. Then, potassium hydroxide (1.63 g, 28.98 mmol) was added to the reaction solution. The reaction system was continued to stir at room temperature for 16 hours. After LCMS monitoring showed that the raw materials disappeared, the reaction solution was concentrated under reduced pressure, diluted with water (20 mL), and the pH of the mixture was adjusted to 2 with 2N hydrochloric acid. The precipitated solid was filtered and dried to obtain 0.8 g of compound **200-3.**

MS (ESI) m/z: 275.0, 277.0 [M - H]⁺.

### Step 3:

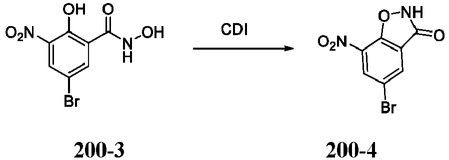

Compound **200-3** (6 g, 21.66 mmol) was dissolved in tetrahydrofuran (80 mL). Then, carbonyldiimidazole (7.02 g, 43.32 mmol) was added to the reaction solution, and the reaction system was heated to 65 °C and continued to stir for 2 hours. After LCMS monitoring showed that the raw materials disappeared, the reaction solution was cooled to room temperature and concentrated under reduced pressure. Water (100 mL) was added to the residue, and the pH of the mixture was adjusted to 2 with 2N aqueous hydrochloric acid. The precipitated solid was filtered and dried to obtain 5.5 g of compound **200-4.**

MS (ESI) m/z: 257.0, 259.0 [M - H]⁺.

### Step 4:

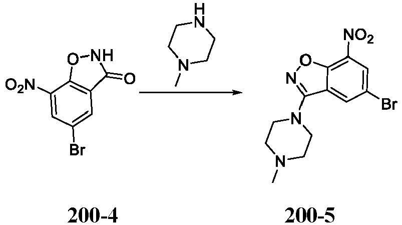

Compound **200-4** (1 g, 3.8 mmol) and 1,8-diazabicycloundec-7-ene (1.76 g, 11.7 mmol) were dissolved in *N*,*N*-dimethylformamide (20 mL). Then, a BOP reagent (benzotriazol-1-yloxytris(dimethylamino)phosphonium hexafluorophosphate, 3.42 g, 7.8 mmol) was added to the reaction solution at 0 °C. The reaction system was heated to 50 °C and continued to stir for 2 hours. After LCMS monitoring showed that the raw material disappeared, the reaction solution was cooled to room temperature and directly purified by a C18 reverse-phase column. Purification conditions were as follows: chromatographic column: 80 g C18 reversed-phase column; mobile phase, water (with 10 mM ammonium bicarbonate) and acetonitrile; flow rate, 50 mL/min; gradient, acetonitrile increased from 25 % to 50 % within 15 minutes; detection wavelength, 254 nm. The product was collected and lyophilized under reduced pressure to obtain 0.7 g of compound **200-5.**

MS (ESI) m/z: 341.0, 343.0 [M + H]⁺.

### Step 5:

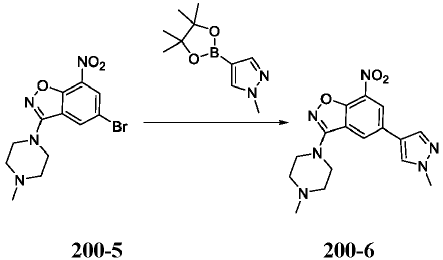

Prepared according to the method of step 5 in Embodiment 1, the raw materials were replaced with **200-5** (400 mg, 1.18 mmol) to obtain 350 mg of compound **200-6.**

MS (ESI) m/z: 343.1 [M + H]⁺.

### Step 6:

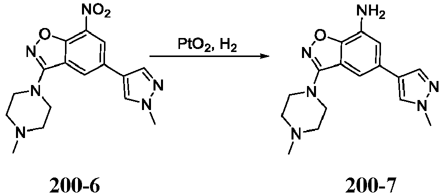

Prepared according to the method of step 6 in Embodiment 1, the raw materials were replaced with 200-6 (400 mg, 1.17 mmol) to obtain 200 mg of compound **200-7.**

MS (ESI) m/z: 313.2 [M + H]⁺.

### Step 7:

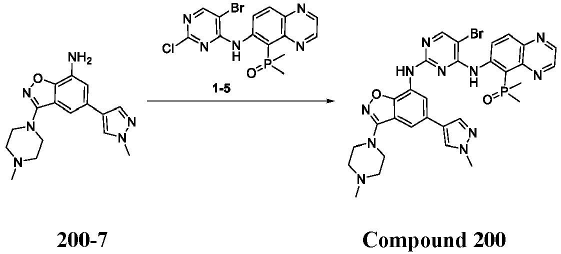

Prepared according to the method of step 7 in Embodiment 1, the raw materials were replaced with 200-7 (100 mg, 0.32 mmol) to obtain 45 mg of compound **200.**

MS (ESI) m/z: 688.3, 690.3 [M + H]⁺.

¹H NMR (300 MHz, CD₃OD) δ 8.91 (d, *J* = 1.8 Hz, 1H), 8.85 (d, *J=* 1.8 Hz, 1H), 8.75 (dd, *J* = 9.6, 4.2 Hz, 1H), 8.43 (d, *J* = 6.3 Hz, 1H), 8.15 (s, 1H), 7.89 (s, 1H), 7.78 (s, 1H), 7.65-7.62 (m, 2H), 3.83 (s, 3H), 3.80-3.71 (m, 4H), 3.04-3.01 (m, 4H), 2.66 (s, 3H), 2.26 (s, 3H), 2.21 (s, 3H).

### Embodiment 205:

### (6-((5-Bromo-2-((4-(1-(2-fluoroethyl)octahydro-6H-pyrrolo[2,3-c]pyridin-6-yl)-2-methoxy-5-(1-methyl-1H-pyrazol-4-yl)phenyl)amino)pyrimidin-4-yl)amino)quinoxalin-5-yl)dimethylphosphine oxide

### Step 1:

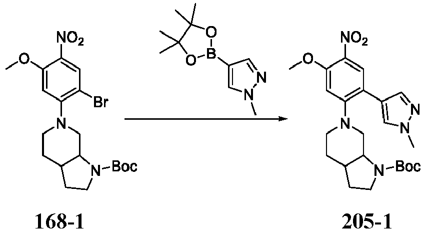

Prepared according to the method of step 5 in Embodiment 1, the raw materials were replaced with compound **168-1** (600 mg, 1.3 mmol) to obtain 456 mg of compound **205-1.**

MS (ESI) m/z: 457.3 [M + H]⁺.

### Step 2:

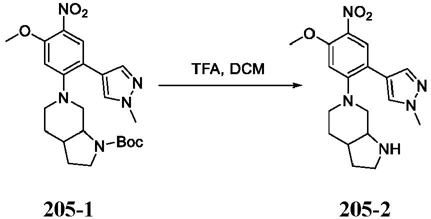

Prepared according to the method of step 2 in Embodiment 16, the raw materials were replaced with compound **205-1** (600 mg, 1.3 mmol) to obtain 456 mg of compound **205-2.**

MS (ESI) m/z: 357.1 [M + H]⁺.

### Step 3:

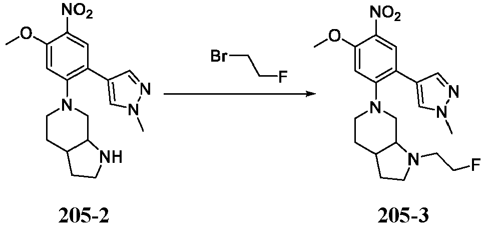

Prepared according to the method of step 3 in Embodiment 133, the raw materials were replaced with compound **205-2** (200 mg, 0.56 mmol) to obtain 70 mg of compound **205-3.**

MS (ESI) m/z: 403.4 [M + H]⁺.

### Step 4:

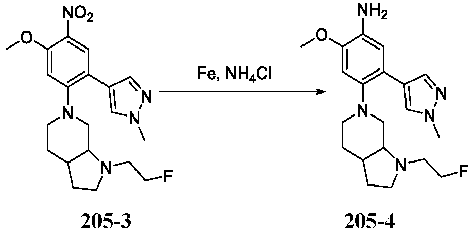

Prepared according to the method of step 3 in Embodiment 70, the raw materials were replaced with compound **205-3** (70 mg, 0.17 mmol) to obtain 60 mg of compound **205-4.**

MS (ESI) m/z: 373.4 [M + H]⁺.

### Step 5:

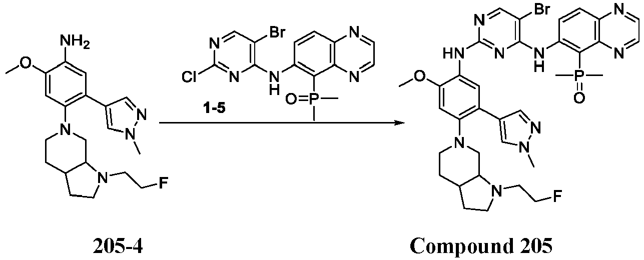

Prepared according to the method of step 7 in Embodiment 1, the raw materials were replaced with compound **205-4** (60 mg, 0.1 mmol) to obtain 5 mg of compound **205.**

MS (ESI) m/z: 749.3, 751.3 [M + H]⁺.

¹H NMR (400 MHz, CDCl₃) δ 12.57 (s, 1H), 8.98 (dd, *J* = 9.6, 4.0 Hz, 1H), 8.74-8.67 (m, 2H), 8.29 (s, 1H), 8.20 (s, 1H), 8.09 (s, 1H), 7.61 (s, 1H), 7.50 (s, 1H), 7.31 (s, 1H), 6.72 (s, 1H), 4.57-4.45 (m, 2H), 3.90 (s, 3H), 3.78 (s, 3H), 3.28-3.62 (m, 9H), 2.27-2.20 (m, 1H), 2.12 (d, *J* = 14.4 Hz, 6H), 1.99-1.91 (m, 1H), 1.72 (s, 3H).

### Embodiment 217:

### (6-((5-Bromo-2-((4-(2,4-dimethyl-1-oxa-4,9-diazaspiro[5.5]undecan-9-yl)-2-methoxy-5-(1-methyl-1H-pyrazol-4-yl)phenyl)amino)pyrimidin-4-yl)amino)quinoxalin-5-yl)dimethylphosphine oxide

### Step 1:

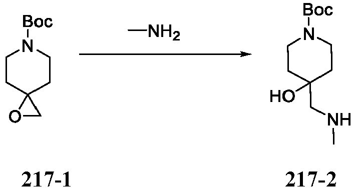

*tert*-Butyl 1-oxa-6-aza-spiro[2.5]octane-6-carboxylate **217-1** (5 g, 23.44 mmol) was dissolved in ethanol (80 mL) and water (9 mL) at room temperature. Then, methylamine aqueous solution (30 %, 73 g, 705.2 mmol) was added to the reaction solution. The reaction system was continued to stir at room temperature for 16 hours. After LCMS monitoring showed that the raw materials disappeared, the reaction solution was concentrated under reduced pressure to obtain 5.7 g of compound **217-2.**

MS (ESI) m/z: 245.2 [M + H]⁺.

### Step 2:

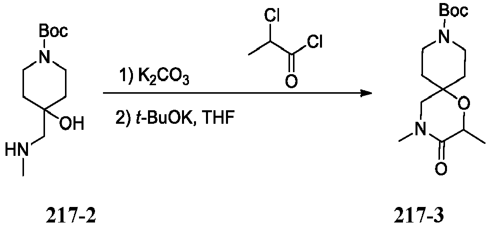

Compound **217-2** (1 g, 4.1 mmol) and potassium carbonate (1.69 g, 12.2 mmol) were dissolved in dichloromethane (10 mL). Then, a solution of 2-chloropropionyl chloride (769 mg, 6.1 mmol) in dichloromethane (5 mL) was added dropwise to the reaction solution at 0 °C. The reaction system was heated to room temperature and continued to stir for 1 hour. After LCMS monitoring showed that the raw materials disappeared, water (100 mL) was added to the reaction solution to quench. The mixture was extracted with ethyl acetate (150 mL × 3 times), and the organic phases were combined. The organic phases were washed with saturated brine (50 mL × 3 times) first, then dried over anhydrous sodium sulfate, filtered, and finally concentrated under reduced pressure. The resulting residue was dissolved in tetrahydrofuran (15 mL). Potassium *tert*-butoxide (479 mg, 4.27 mmol) was added to the reaction solution at -78 °C under nitrogen atmosphere. The reaction solution was heated to room temperature and continued to stir for 1 hour. After LCMS monitoring showed that the raw materials disappeared, water (100 mL) was added to the reaction solution to quench. The mixture was extracted with ethyl acetate (150 mL × 3 times), and the organic phases were combined. The organic phases were washed with saturated brine (50 mL × 3 times) first, then dried over anhydrous sodium sulfate, filtered, and finally concentrated under reduced pressure. The resulting residue was purified by silica gel column chromatography (eluent: petroleum ether/ethyl acetate = 1/1) to obtain 1.2 g of racemic compound **217-3.**

MS (ESI) m/z: 299.3 [M + H]⁺.

### Step 3:

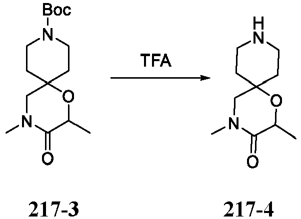

Prepared according to the method of step 2 in Embodiment 16, the raw materials were replaced with compound **217-3** (1.1 g, 3.69 mmol) to obtain 0.7 g of compound **217-4.**

MS (ESI) m/z: 199.3 [M + H]⁺.

### Step 4:

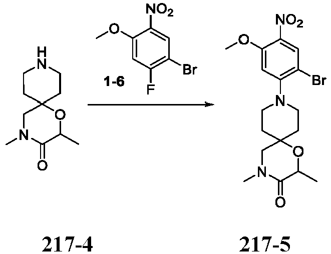

Prepared according to the method of step 4 in Embodiment 1, the raw materials were replaced with compound **217-4** (750 mg, 3.78 mmol) to obtain 1.1 g of compound **217-5.**

MS (ESI) m/z: 427.9, 429.9 [M + H]⁺.

### Step 5:

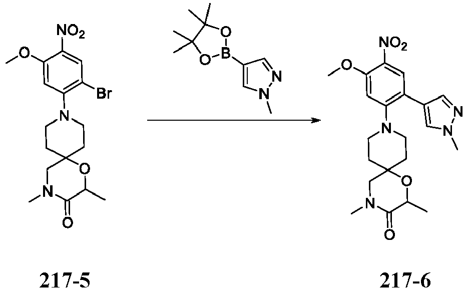

Prepared according to the method of step 5 in Embodiment 1, the raw materials were replaced with compound **217-5** (1 g, 2.335 mmol) to obtain 1 g of compound **217-6.**

MS (ESI) m/z: 430.2 [M + H]⁺.

### Step 6:

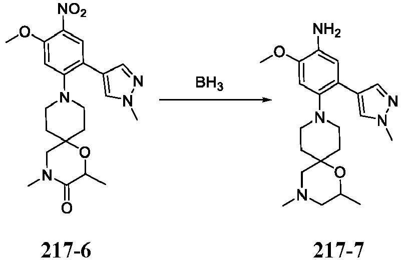

Compound **217-6** (200 mg, 0.47 mmol) was dissolved in tetrahydrofuran (3 mL) under nitrogen atmosphere. Then, borane dimethyl sulfide (0.44 mL) was added to the reaction solution at 0 °C. The reaction system was heated to room temperature and continued to stir for 16 hours. After LCMS monitoring showed that the raw materials disappeared, methanol (5 mL) was added to the reaction solution at 0 °C and concentrated under reduced pressure. The residue was purified by reversed-phase column, and the purification conditions were as follows: chromatographic column: 40 g C18 reversed-phase column; mobile phase: water (with 10 mM ammonium bicarbonate) and acetonitrile; flow rate: 30 mL/min; gradient: acetonitrile increased from 30 % to 50 % within 30 minutes; detection wavelength: 254 nm. The product was collected and lyophilized under reduced pressure to obtain 80 mg of compound **217-7.**

MS (ESI) m/z: 386.2 [M + H]⁺.

### Step 7:

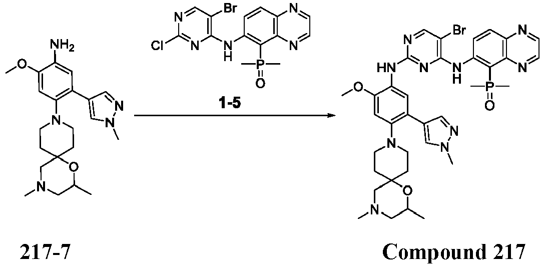

Prepared according to the method of step 7 in Embodiment 1, the raw materials were replaced with compound **217-7** (60 mg, 0.156 mmol) to obtain 40 mg of racemic compound **217.**

MS (ESI) m/z: 761.4, 763.4 [M + H]⁺.

¹H NMR (300 MHz, CD₃OD) δ 8.87-8.78 (m, 3H), 8.26 (s, 1H), 7.92 (s, 1H), 7.87 (s, 1H), 7.54-7.49 (m, 2H), 6.86 (s, 1H), 3.93 (s, 4H), 3.71 (s, 3H), 3.07-3.00 (m, 1H), 2.91-2.80 (m, 5H), 2.31 (s, 4H), 2.16 (d, *J* = 14.4 Hz, 6H), 1.90-1.63 (m, 5H), 1.17 (d, *J* = 6.3 Hz, 3H).

### Embodiment 219:

### (6-((5-Bromo-2-((2-methoxy-5-(1-methyl-1H-pyrazol-4-yl)-4-(4-(oxetan-3-ylmethyl)piperazin-1-yl)phenyl)amino)pyrimidin-4-yl)amino)quinoxalin-5-yl)dimethylphosphine oxide

### Step 1:

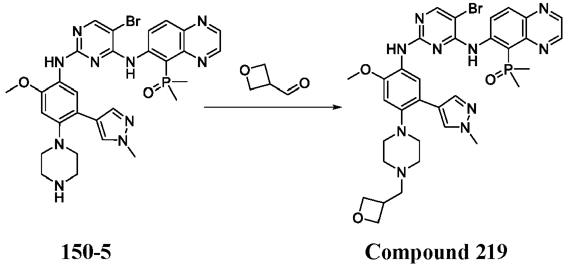

Prepared according to the method in Embodiment 148, the raw materials were replaced with compound **150-5** (100 mg, 0.15 mmol) and oxetane-3-carbaldehyde (39 mg, 0.45 mmol) to obtain 50 mg of compound **219.**

MS (ESI, m/z): 733.3, 735.3 [M + H]⁺.

¹H NMR (300 MHz, CD₃OD) δ 8.86-8.79 (m, 3H), 8.26 (s, 1H), 7.94 (s, 1H), 7.82 (s, 1H), 7.53-7.49 (m, 2H), 6.84 (s, 1H), 4.86-4.78 (m, 2H), 4.48 (t, *J* = 6.3 Hz, 2H), 3.92 (s, 3H), 3.69 (s, 3H), 3.40-3.38 (m, 1H), 2.94-2.82 (m, 6H), 2.59 (s, 4H), 2.16 *(d, J=* 14.4 Hz, 6H).

### Embodiment 221:

### (6-((5-Bromo-2-((2-methoxy-5-(1-methyl-1H-pyrazol-4-yl)-4-(7-methyl-3,7-diazabicyclo[4.2.0]octan-3-yl)phenyl)aminopyrimidin-4-yl)amino)quinoxalin-5-yl)dimethylphosphine oxide

### Step 1:

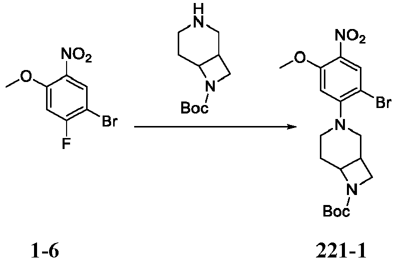

Prepared according to the method of step 4 in Embodiment 1, the raw materials were replaced with compound *tert*-butyl 3,7-diazabicyclo[4.2.0]octane-7-carboxylate (cas #: 885271-73-8, 509 mg, 2.4 mmol) to obtain 700 mg of compound **221-1.**

MS (ESI, m/z): 442.1, 444.1 [M + H]⁺.

### Step 2:

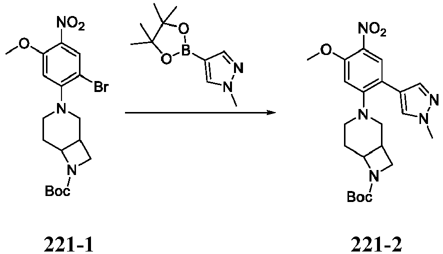

Prepared according to the method of step 5 in Embodiment 1, the raw materials were replaced with compound **221-1** (700 mg, 1.58 mmol) to obtain 400 mg of compound **221-2.**

MS (ESI, m/z): 444.2 [M + H]⁺.

### Step 3:

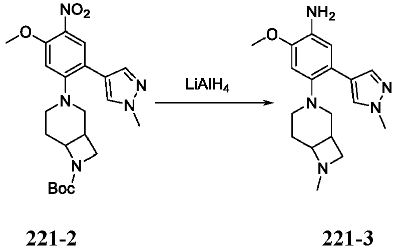

Compound **221-2** (350 mg, 0.8 mmol) was dissolved in tetrahydrofuran (4 mL) under nitrogen atmosphere. Then, lithium aluminum hydride (60 mg, 1.5 mmol) was added to the reaction solution at 0 °C. The reaction system was heated to 65 °C and continued to stir for 2 hours. After LCMS monitoring showed that the raw materials disappeared, the reaction solution was cooled to 0 °C and sodium hydroxide aqueous solution (1 M, 0.5 mL) was added thereto and filtered. The filtrate was concentrated under reduced pressure to obtain 200 mg of compound **221-3.**

MS (ESI, m/z): 328.2 [M + H]⁺.

### Step 4:

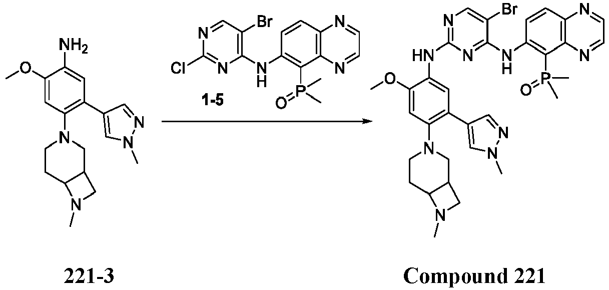

Prepared according to the method of step 7 in Embodiment 1, the raw materials were replaced with compound **221-3** (150 mg, 0.4 mmol) to obtain 24 mg of racemic compound **221.**

MS (ESI, m/z): 703.3, 705.3 [M + H]⁺.

¹H NMR (300 MHz, CD₃OD) δ 8.88-8.78 (m, 3H), 8.28 (s, 1H), 7.95 (s, 1H), 7.79 (s, 1H), 7.55 (d, *J* = 9.6 Hz, 1H), 7.48 (s, 1H), 6.90 (s, 1H), 3.95 (s, 3H), 3.70 (s, 5H), 3.39-3.26 (m, 2H), 3.09-2.89 (m, 3H), 2.72-2.65 (m, 1H), 2.54 (s, 3H), 2.17 (d, *J=* 14.4 Hz, 6H), 1.97-1.85 (m, 2H).

### Embodiment 222:

### (6-((5-Bromo-2-((4-(4-(3,3-difluoropropyl)piperazin-1-yl)-2-methoxy-5-(1-methyl-1H-pyrazol-4-yl)phenyl)amino)pyrimidin-4-yl)amino)quinoxalin-5-yl)dimethylphosphine oxide

### Step 1:

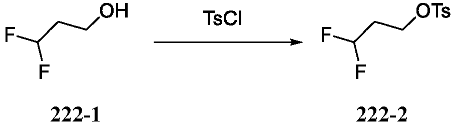

3,3-Difluoro-1-propanol (200 mg, 2.1 mmol), triethylamine (421 mg, 4.16 mmol) and 4-dimethylaminopyridine (51 mg, 0.42 mmol) were dissolved in dichloromethane (10 mL). Then, *p-*toluenesulfonyl chloride (600 mg, 3.15 mmol) was added to the reaction solution in batches at 0 °C. The reaction system was heated to room temperature and continued to stir for 16 hours. After TLC monitoring showed that the raw materials disappeared, water (30 mL) was added to the reaction solution to quench. The mixture was extracted with ethyl acetate (30 mL × 3 times), and the organic phases were combined. The organic phases were washed with saturated brine (30 mL × 3 times) first, then dried over anhydrous sodium sulfate, filtered, and finally concentrated under reduced pressure. The resulting residue was purified by silica gel column chromatography (eluent: petroleum ether/ethyl acetate = 10/1) to obtain 380 mg of compound **222-2.**

¹H NMR (400 MHz, CDCl₃) δ 7.80 (d, *J* = 8.4 Hz, 2H), 7.37 (d, *J* = 8.0 Hz, 2H), 6.05-5.74 (m, 1H), 4.18 (t, *J* = 6.0 Hz, 2H), 2.46 (s, 3H), 2.27-2.15 (m, 2H).

¹⁹F NMR (377 MHz, CDCl₃) δ -118.40.

### Step 2:

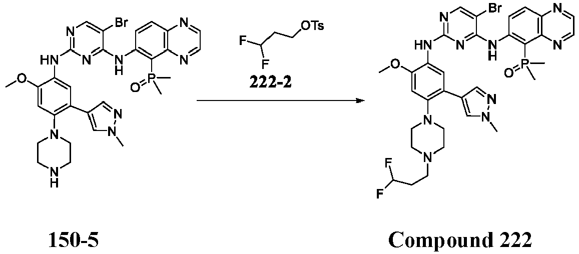

Compound **150-5** (300 mg, 0.43 mmol) and potassium carbonate (250 mg, 1.81 mmol) were dissolved in *N*,*N*-dimethylformamide (5 mL). Then, compound **222-2** (136 mg, 0.5 mmol) was added to the reaction solution. The reaction system was heated to 100 °C and continued to stir for 16 hours. After TLC monitoring showed that the raw materials disappeared, the reaction solution was cooled to room temperature. Water (30 mL) was added to the reaction solution to quench. The mixture was extracted with ethyl acetate (50 mL × 3 times), and the organic phases were combined. The organic phases were washed with saturated brine (30 mL × 3 times) first, then dried over anhydrous sodium sulfate, filtered, and finally concentrated under reduced pressure. The resulting residue was purified by silica gel column chromatography (eluent: dichloromethane/methanol = 10/1) to obtain 38 mg of compound **222.**

MS (ESI, m/z): 741.2, 743.2 [M + H]⁺.

¹H NMR (300 MHz, CDCl₃) δ 12.61 (s, 1H), 8.01 (dd, *J* = 9.6, 4.2 Hz, 1H), 8.76 *(d, J=* 2.1 Hz, 1H), 8.72 *(d, J=* 2.1 Hz, 1H), 8.31 (s, 1H), 8.24 (s, 1H), 7.66-7.64 (m, 3H), 7.38 (s, 1H), 6.75 (s, 1H), 6.19-5.79 (m, 1H), 3.93 (s, 3H), 3.76 (s, 3H), 3.02 (s, 4H), 2.70 (s, 6H), 2.17-2.13 (m, 8H).

¹⁹F NMR (282 MHz, CDCl₃) δ -116.99.

### Embodiment 223:

### (6-((5-Bromo-2-((4-(3-(3-fluoroazetidin-1-yl)pyrrolidin-1-yl)-2-methoxy-5-(1-methyl-1H-pyrazol-4-yl)phenyl)amino)pyrimidin-4-yl)amino)quinoxalin-5-yl)dimethylphosphine oxide

### Step 1:

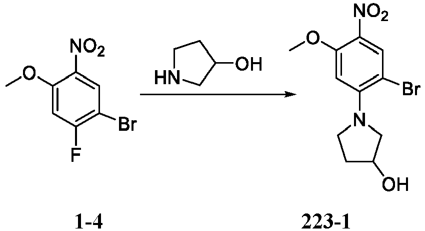

Prepared according to the method of step 4 in Embodiment 1, the raw materials were replaced with 3-pyrrolidinol (2.51 g, 28.8 mmol) to obtain 7.5 g of compound **223-1.**

MS (ESI, m/z): 317.0, 319.0 [M + H]⁺.

### Step 2:

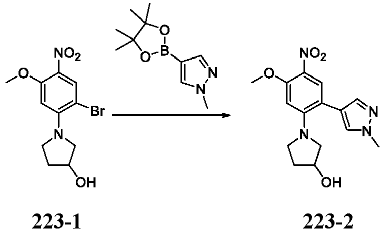

Prepared according to the method of step 5 in Embodiment 1, the raw materials were replaced with compound **223-1** (7.5 g, 23.7 mmol) to obtain 4.5 g of compound **223-2.**

MS (ESI, m/z): 319.1 [M + H]⁺.

### Step 3:

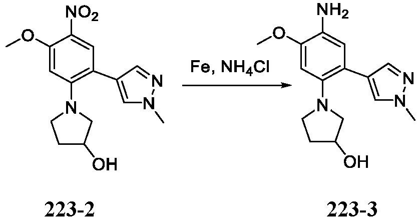

Prepared according to the method of step 3 in Embodiment 70, the raw materials were replaced with compound **223-2** (500 mg, 1.57 mmol) to obtain 420 mg of compound **223-3.**

MS (ESI, m/z): 289.1 [M + H]⁺.

### Step 4:

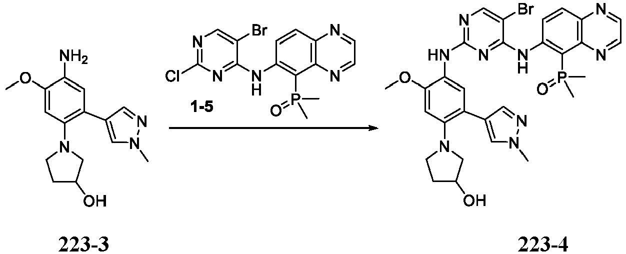

Prepared according to the method of step 7 in Embodiment 1, the raw materials were replaced with compound **223-3** (377 mg, 1.31 mmol) to obtain 470 mg of compound **223-4.**

MS (ESI, m/z): 664.1, 666.1 [M + H]⁺.

### Step 5:

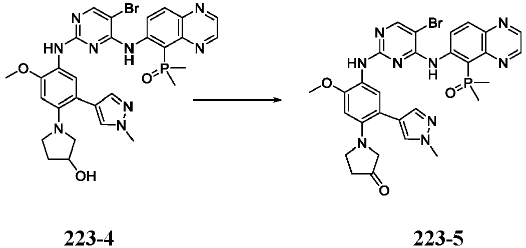

Compound **223-4** (500 mg, 0.75 mmol) was dissolved in dimethyl sulfoxide (5 mL). Then, triethylamine (761 mg, 7.52 mmol) was added to the reaction solution and the mixture was continued to stir for 30 minutes. Sulfur trioxide pyridine complex (1.2 g, 7.52 mmol) was added to the reaction solution in batches at 40 °C. The reaction system was continued to stir at 40 °C for 3 hours. After LCMS monitoring showed that the raw materials disappeared, the reaction solution was cooled to room temperature and added with ice water (30 g) to quench. The mixture was extracted with ethyl acetate (50 mL × 3 times), and the organic phases were combined. The organic phases were washed with saturated brine (30 mL × 3 times) first, then dried over anhydrous sodium sulfate, filtered, and finally concentrated under reduced pressure. The resulting residue was purified by silica gel column chromatography (eluent: dichloromethane/methanol = 10/1) to obtain 260 mg of compound **223-5.**

MS (ESI, m/z): 662.3, 664.3 [M + H]⁺.

### Step 6:

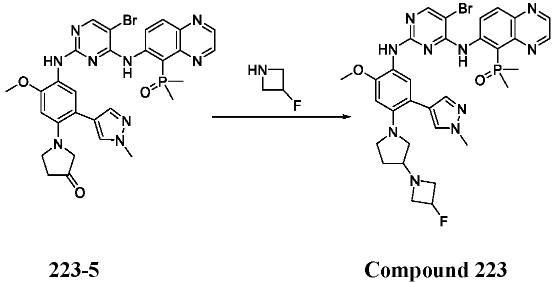

Prepared according to the method of step 1 in Embodiment 148, the raw materials were replaced with compound **223-5** (240 mg, 0.36 mmol) and 3-fluoroazetidine hydrochloride (201 mg, 1.81 mmol) to obtain 170 mg of racemic compound **223.**

MS (ESI, m/z): 721.0, 723.0 [M + H]⁺.

¹H NMR (300 MHz, CD₃OD) δ 8.90-8.79 (m, 3H), 8.25 (s, 1H), 7.79 (s, 1H), 7.74 (s, 1H), 7.56 (d, *J =* 9.6 Hz, 1H), 7.40 (s, 1H), 6.78 (s, 1H), 5.33-5.11 (m, 1H), 3.92 (s, 3H), 3.87-3.82 (m, 2H), 3.76 (s, 3H), 3.59-3.48 (m, 2H), 3.42-3.38 (m, 1H), 3.26-3.19 (m, 1H), 3.10-2.90 (m, 3H), 2.19-2.09 (m, 7H), 1.79-1.70 (m, 1H).

¹⁹F NMR (282 MHz, CD₃OD) δ -181.14.

### Embodiment 224:

### (6-((5-Bromo-2-((4-(4-isopropyl-2,2-dimethyl-1-oxa-4,9-diazaspiro[5.5]undecane- 9-yl)-2-methoxy-5-(1-methyl-1H-pyrazol-4-yl)phenyl)amino)pyrimidin-4-yl)amino)quinoxalin-5-yl)dimethylphosphine oxide

### Step 1:

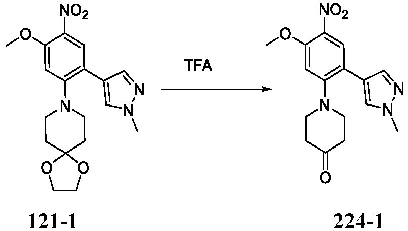

Prepared according to the method of step 4 in Embodiment 93, the raw materials were replaced with compound **121-1** (7 g, 18.7 mmol) to obtain 4.64 g of compound **224-1.**

MS (ESI) m/z: 331.1 [M + H]⁺.

### Step 2:

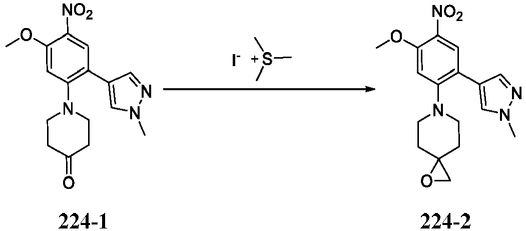

Trimethylsulfonium iodide (1.05 mg, 5.1 mmol) was dissolved in tetrahydrofuran (20 mL) under nitrogen atmosphere at 0 °C. Then, sodium hydride (60 %, 206 mg, 5.1 mmol) was added to the reaction solution and the mixture was continued to stir for 30 minutes. A solution of compound **224-1** (850 mg, 2.6 mmol) in dimethyl sulfoxide (10 mL) was added to the reaction solution. The reaction system was continued to stir at 0 °C for 2 hours. After LCMS monitoring showed that the raw materials disappeared, ice water (80 g) was added to the reaction solution. The mixture was extracted with ethyl acetate (80 mL × 3 times), and the organic phases were combined. The organic phases were washed with saturated brine (50 mL × 3 times) first, then dried over anhydrous sodium sulfate, filtered, and finally concentrated under reduced pressure. The resulting residue was purified by silica gel column chromatography (eluent: ethyl acetate) to obtain 200 mg of compound **224-2.**

MS (ESI, m/z): 345.2 [M + H]⁺.

### Step 3:

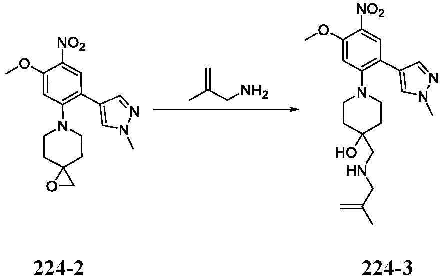

Compound **224-2** (320 mg, 0.9 mmol) and 2-methylallylamine (132 mg, 1.9 mmol) were dissolved in ethanol (6 mL). Then, diisopropylethylamine (240 mg, 1.9 mmol) was added to the reaction solution. The reaction system was heated to 70 °C and continued to stir for 16 hours. After LCMS monitoring showed that the raw materials disappeared, the reaction solution was cooled to room temperature and concentrated under reduced pressure. The resulting residue was purified by silica gel column chromatography (eluent: dichloromethane/methanol = 10/1) to obtain 330 mg of compound **224-3.**

MS (ESI, m/z): 416.2 [M + H]⁺.

### Step 4:

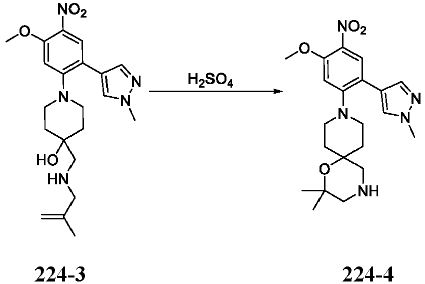

Compound **224-3** (245 mg, 0.6 mmol) was dissolved in concentrated sulfuric acid (1.5 mL). The reaction system was continued to stir at room temperature for 2 hours. After LCMS monitoring showed that the raw materials disappeared, the reaction solution was added to ice water (30 g) to quench, and the mixture of the pH was adjusted to 10 with sodium carbonate. The mixture was extracted with dichloromethane (80 mL × 3 times), and the organic phases were combined. The organic phases were washed with saturated brine (50 mL × 3 times) first, then dried over anhydrous sodium sulfate, filtered, and finally concentrated under reduced pressure. The resulting residue was purified by silica gel column chromatography (eluent: dichloromethane/methanol = 10/1) to obtain 95 mg of compound **224-4.**

MS (ESI, m/z): 416.2 [M + H]⁺.

### Step 5:

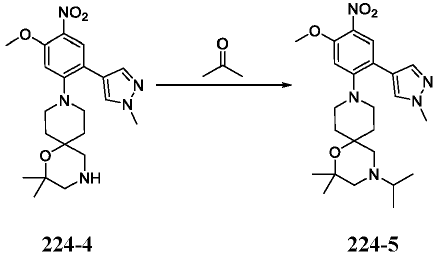

Prepared according to the method of step 3 in Embodiment 164, the raw materials were replaced with compound **224-4** (95 mg, 0.2 mmol) to obtain 100 mg of compound **224-5.**

MS (ESI) m/z: 458.3 [M + H]⁺.

### Step 6:

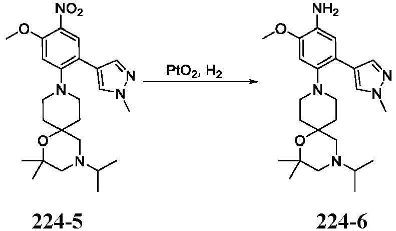

Prepared according to the method of step 6 in Embodiment 1, the raw materials were replaced with compound **224-5** (100 mg, 0.2 mmol) to obtain 80 mg of compound **224-6.**

MS (ESI) m/z: 428.3 [M + H]⁺.

### Step 7:

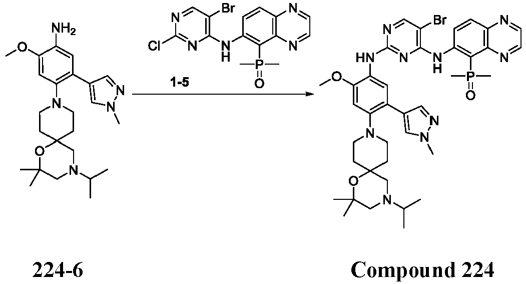

Prepared according to the method of step 7 in Embodiment 1, the raw materials were replaced with compound **224-6** (60 mg, 0.1 mmol) to obtain 20 mg of compound **224.**

MS (ESI) m/z: 803.2, 805.2 [M + H]⁺.

¹H NMR (300 MHz, CD₃OD) δ 8.88-8.78 (m, 3H), 8.27 (s, 1H), 7.91 (s, 1H), 7.88 (s, 1H), 7.56-7.50 (m, 2H), 6.87 (s, 1H), 3.93 (s, 3H), 3.72 (s, 3H), 3.05-2.99 (m, 2H), 2.81-2.75 (m, 3H), 2.53 (s, 2H), 2.41 (s, 2H), 2.17 (d, *J* = 14.6 Hz, 6H), 1.90-1.81 (m, 4H), 1.29 (s, 6H), 1.11 *(d, J=* 6.6 Hz, 6H).

### Embodiment 229:

### (6-((5-Bromo-2-((2-methoxy-5-(1-methyl-1H pyrazol-4-yl)-4-(4-(1-methylcyclopropyl))piperazin-1-yl)phenyl)amino)pyrimidin-4-yl)amino)quinoxalin-5-yl)dimethylphosphine oxide

### Step 1:

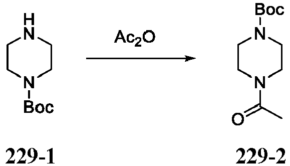

Compound 1-tert-butoxycarbonylpiperazine (5 g, 26.9 mmol) was dissolved in dichloromethane (50 mL). Then, acetic anhydride (2.77 mL, 29.5 mmol) was added to the reaction solution at 0 °C and the mixture was continued to stir at this temperature for 20 minutes. After LCMS monitoring showed that the raw materials disappeared, saturated sodium bicarbonate aqueous solution (30 mL) was added to the reaction solution to quench. The mixture was extracted with dichloromethane (80 mL × 3 times), and the organic phases were combined. The organic phases were washed with saturated brine (50 mL × 3 times) first, then dried over anhydrous sodium sulfate, filtered, and finally concentrated under reduced pressure. 6 g of compound **229-2** was obtained.

MS (ESI, m/z): 229.2 [M + H]⁺.

### Step 2:

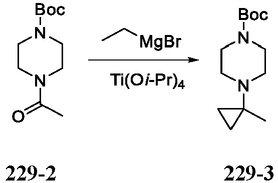

Compound **229-2** (2 g, 8.76 mmol) was dissolved in tetrahydrofuran (20 mL) under nitrogen atmosphere. Then, a solution of tetraisopropyl titanate (10.5 mL, 10.5 mmol) and ethylmagnesium bromide (10.3 mL, 35.0 mmol) in tetrahydrofuran was added dropwise to the reaction solution at -78 °C. The reaction system was slowly heated to room temperature and continued to stir for 1 hour. After LCMS monitoring showed that the raw materials disappeared, water (10 mL) and sodium potassium tartrate aqueous solution (30 mL) were added to the reaction solution, and the mixture was continued to stir for 30 minutes. The mixture was extracted with ethyl acetate (80 mL × 3 times), and the organic phases were combined. The organic phases were washed with saturated brine (150 mL × 3 times) first, then dried over anhydrous sodium sulfate, filtered, and finally concentrated under reduced pressure. The resulting residue was purified by silica gel column chromatography (eluent: petroleum ether/methyl tert-butyl ether = 2/1) to obtain 0.85 g of compound **229-3.**

MS (ESI, m/z): 241.4 [M + H]⁺.

### Step 3:

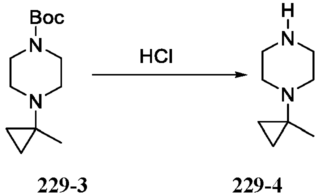

Prepared according to the method of step 2 in Embodiment 133, the raw materials were replaced with compound **229-3** (850 mg, 3.54 mmol) to obtain 430 mg of compound **229-4.**

MS (ESI) m/z: 141.1 [M + H]⁺.

### Step 4:

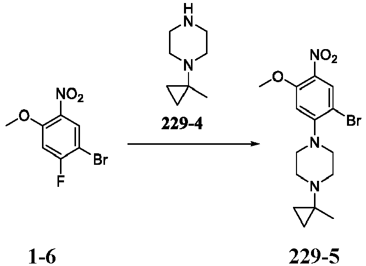

Prepared according to the method of step 4 in Embodiment 1, the raw materials were replaced with compound **229-4** (430 mg, 3.1 mmol) to obtain 200 mg of compound **229-5.**

MS (ESI) m/z: 370.2, 372.2 [M + H]⁺.

### Step 5:

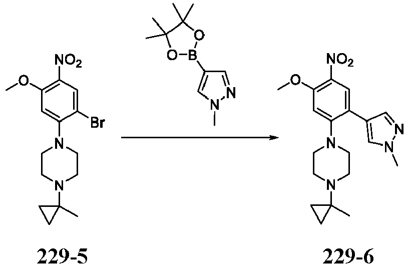

Prepared according to the method of step 5 in Embodiment 1, the raw materials were replaced with compound **229-5** (200 mg, 0.54 mmol) to obtain 100 mg of compound **229-6.**

MS (ESI) m/z: 372.2 [M + H]⁺.

### Step 6:

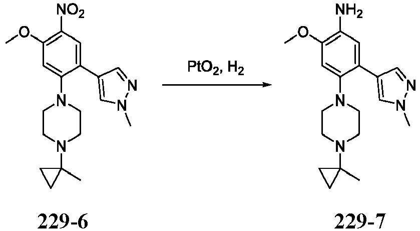

Prepared according to the method of step 6 in Embodiment 1, the raw materials were replaced with compound **229-6** (100 mg, 0.3 mmol) to obtain 80 mg of compound **229-7.**

MS (ESI) m/z: 342.1 [M + H]⁺.

### Step 7:

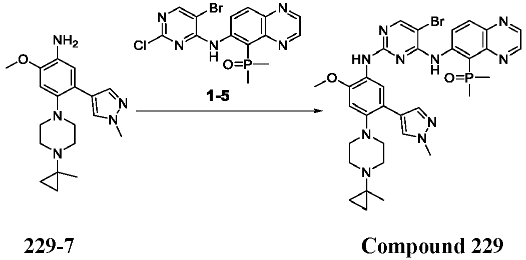

Prepared according to the method of step 7 in Embodiment 1, the raw materials were replaced with compound **229-7** (80 mg, 0.23 mmol) to obtain 15 mg of compound **229.**

MS (ESI) m/z: 717.2, 719.2 [M + H]⁺.

¹H NMR (300 MHz, CD₃OD) δ 8.87-8.78 (m, 3H), 8.26 (s, 1H), 7.93 (s, 1H), 7.83 (s, 1H), 7.56-7.51 (m, 2H), 6.83 (s, 1H), 3.91 (s, 3H), 3.68 (s, 3H), 2.89-2.82 (m, 8H), 2.18 (s, 3H), 2.13 (s, 3H), 1.19 (s, 3H), 0.65-0.62 (m, 2H), 0.45-0.42 (m, 2H).

### Embodiment 232:

### (6-((5-Bromo-2-((2-methoxy-4-(4-(4-(2-methoxyethyl)piperazin-1-yl)piperidin-1-yl))-5-(1-methyl-1H-pyrazol-4-yl)phenyl)amino)pyrimidin-4-yl)amino)quinoxolin-5-yl)dimethylphosphine sulfide

### Step 1:

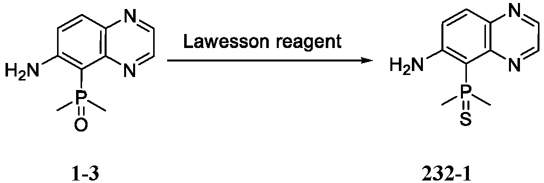

Compound **1-3** (1 g, 4.52 mmol) was dissolved in toluene (10 mL). Lawesson reagent (2,4-bis(*p-*methoxyphenyl)-1,3-dithio-diphosphetane-2,4 sulfide, 3.66 g, 9.0 mmol) was then added to the reaction solution. The reaction system was heated to 110 °C and continued to stir for 3 hours. After LCMS monitoring showed that the raw materials disappeared, the reaction solution was cooled to room temperature, and water (50 mL) was added thereto. The mixture was extracted with ethyl acetate (80 mL × 3 times), and the organic phases were combined. The organic phases were washed with saturated brine (50 mL × 3 times), then dried over anhydrous sodium sulfate, filtered, and finally concentrated under reduced pressure; the resulting residue was purified by silica gel column chromatography (eluent: dichloromethane/methanol=10/1) to obtain 500 mg of compound **232-1.**

MS (ESI) m/z: 238.2 [M + H]⁺.

### Step 2:

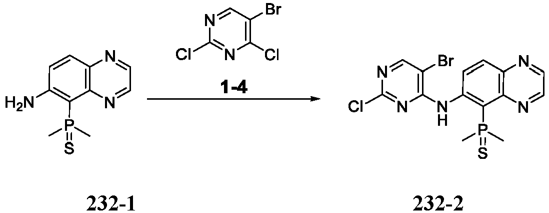

Prepared according to the method of step 3 in Embodiment 1, the raw materials were replaced with compound **232-1** (417 mg, 1.76 mmol) to obtain 470 mg of compound **232-2.**

MS (ESI, m/z): 427.9, 429.9 [M + H]⁺.

### Step 3:

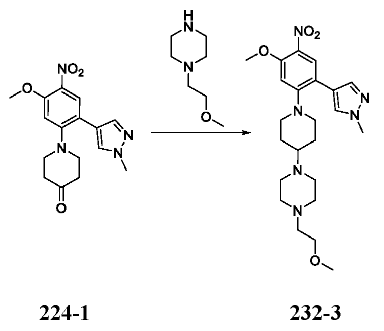

Prepared according to the method of step 5 in Embodiment 93, the raw materials were replaced with compound **224-1** (160 mg, 1.45 mmol) and 1-(2-methoxyethyl)piperazine (210 mg, 1.45 mmol) to obtain 112 mg of compound **232-3**.

MS (ESI, m/z): 459.4 [M + H]⁺.

### Step 4:

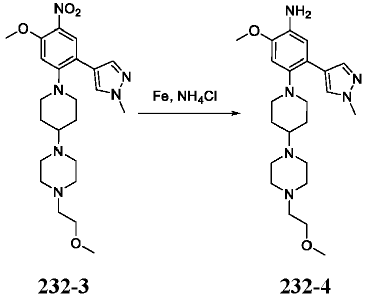

Prepared according to the method of step 3 in Embodiment 70, the raw materials were replaced with compound **232-3** (110 mg, 0.24 mmol) to obtain 80 mg of compound **232-4.**

MS (ESI, m/z): 429.3 [M + H]⁺.

### Step 5:

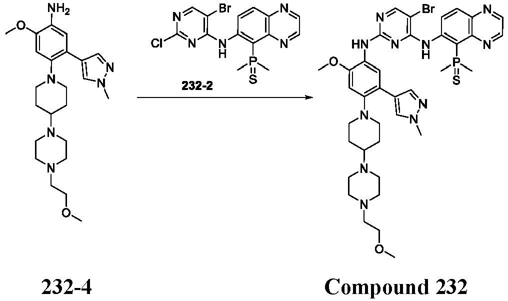

Prepared according to the method of step 7 in Embodiment 1, the raw materials were replaced with compound **232-4** (80 mg, 0.19 mmol) and **232-2** (80 mg, 0.19 mmol) to obtain 14 mg of compound **232.**

MS (ESI, m/z): 820.3, 822.3 [M + H]⁺.

¹H NMR (300 MHz, CDCl₃) δ 11.71 (s, 1H), 8.79-8.77 (m, 2H), 8.36-8.31 (m, 2H), 8.20 (s, 1H), 7.72-7.68 (m, 2H), 7.40 (s, 1H), 7.12 (s, 1H), 6.67 (s, 1H), 3.89 (s, 3H), 3.74 (s, 3H), 3.58-3.55 (m, 2H), 3.38 (s, 3H), 3.17-3.14 (m, 2H), 2.81-2.66 (m, 10H), 2.61-2.54 (m, 2H), 2.49 (s 3H), 2.44 (s, 3H), 2.41-2.37 (m, 1H), 2.03-1.99 (m, 2H), 1.72-1.65 (m, 2H).

### Embodiment 234:

### (6-((5-Bromo-2-((4-((3aS,7aS)-1-(cyclopropylmcthyl)octahydro-6H-pyrrolo[2,3-c]pyridin-6-yl)-2-methoxy-5-(1-methyl-1H-pyrazol-4-yl)phenyl)amino)pyrimidin-4-yl)amino)quinoxalin-5-yl)dimethylphosphine oxide

### (6-((5-Bromo-2-((4-(((3aR,7aR)-1-(cyclopropylmethyl)octahydro-6H-pyrrolo[2,3-c]pyridine-6-6yl)-2-methoxy-5-(1-methyl-1H-pyrazol-4-yl)phenyl)amino)pyrimidin-4-yl)amino)quinoxalin-5-yl)dimethylphosphine oxide

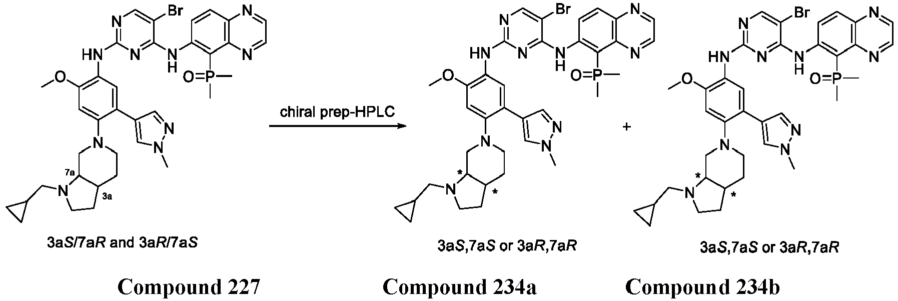

Compound **227** (100 mg) was subjected to a chiral resolution, and resolution conditions were as follows: chiral column, CHIRALPAK ID 2 x 25 cm (5 Cm); mobile phase A methyl tert-butyl ether (10 mM ammonia methanol), mobile phase B ethanol; flow rate of 20 mL/min; gradient of 50 % B; detection wavelength of 210/270 nm; 20 mg of compound **234a** was obtained at 9.45 minutes and 20 mg of compound 234b was obtained at 11.8 minutes.

**234a:** MS (ESI) M/Z: 757.3, 759.3 [M + H]⁺; ¹H NMR (300 MHz, CD₃OD) δ 8.87-8.77 (m, 3H), 8.25 (s, 1H), 8.10 (s, 1H), 7.92 (s, 1H), 7.53-7.48 (m, 2H), 6.85 (s, 1H), 3.92 (s, 3H), 3.72 (s, 3H), 3.21-3.16 (m, 1H), 3.08-2.98 (m, 2H), 2.86-2.70 (m, 4H), 2.50-2.24 (m, 3H), 2.16 (dd, *J* = 14.7, 1.8 Hz, 6H), 1.96-1.77 (m, 4H), 0.83-0.78 (m, 1H), 0.47-0.43 (m, 2H), 0.10-0.07 (m, 2H).

**234b:** MS (ESI) M/Z: 757.4, 759.4 [M + H]⁺; ¹H NMR (400 MHz, CD₃OD) δ 8.84-8.75 (m, 3H), 8.23 (s, 1H), 8.08 (s, 1H), 7.90 (s, 1H), 7.50-7.46 (m, 2H), 6.83 (s, 1H), 3.90 (s, 3H), 3.70 (s, 3H), 3.19-3.13 (m, 1H), 3.05-2.94 (m, 2H), 2.84-2.66 (m, 4H), 2.47-2.23 (m, 3H), 2.14 (dd, *J* = 14.4, 2.8 Hz, 6H), 1.94-1.76 (m, 4H), 0.80-0.77 (m, 1H), 0.44-0.40 (m, 2H), 0.07-0.04 (m, 2H).

### Embodiment 237:

### 1-((4-(4-((5-Bromo-4-((5-(dimethylphosphoryl)quinoxalin-6-yl)amino)pyrimidin-2-yl)amino)-5-methoxy-2-(1-methyl-1H-pyrazol-4-yl)phenyl)piperazin-1-yl)methyl)cyclopropane-1-carbonitrile

### Step 1:

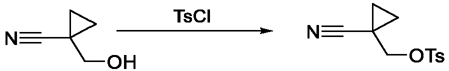

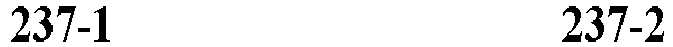

Prepared according to the method of step 1 in Embodiment 222, the raw materials were replaced with compound **237-1** (1 g, 10.3 mmol) to obtain 330 mg of compound **237-2.**

¹H NMR: (400 MHz, CDCl₃) δ 7.84-7.81 (m, 2H), 7.40-7.36 (m, 2H), 4.00 (s, 2H), 2.46 (s, 3H), 1.39-1.35 (m, 2H), 1.09-1.05 (m, 2H).

### Step 2:

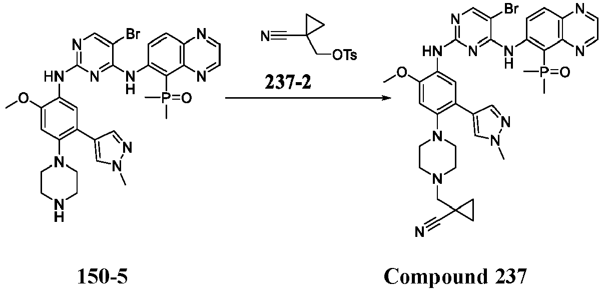

Prepared according to the method of step 2 in Embodiment 222, the raw materials were replaced with compound **237-2** (57 mg, 0.23 mmol) to obtain 9 mg of compound **237.**

MS (ESI) m/z: 742.4, 744.4 [M + H]⁺.

¹H NMR: (300 MHz, CDCl₃) δ 12.58 (s, 1H), 8.99 (dd, *J* = 9.6, 4.2 Hz, 1H), 8.73 (d, *J* = 2.1 Hz, 1H), 8.69 (d, *J=* 1.8 Hz, 1H), 8.29 (s, 1H), 8.22 (s, 1H), 7.68-7.62 (m, 3H), 7.34 (s, 1H), 6.75 (s, 1H), 3.91 (s, 3H), 3.72 (s, 3H), 2.98-2.95 (m, 4H), 2.72-2.63 (m, 4H), 2.51 (s, 2H), 2.15 (s, 3H), 2.10 (s, 3H), 1.35-1.28 (m, 2H), 0.93-0.86 (m, 2H).

### Embodiment 242:

### (6-((5-Bromo-2-((4-(9-((1-fluorocyclopropyl)methyl)-3,9-diazaspiro[5.5]undecan-3-yl)-2-methoxy-5-(1-methyl-1H-pyrazol-4-yl)phenyl)amino)pyrimidin-4-yl)amino)quinoxalin-5-yl)dimethylphosphine oxide

### Step 1:

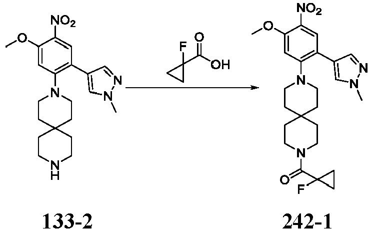

1-Fluorocyclopropanecarboxylic acid (162 mg, 1.56 mmol) was dissolved in *N,N-*dimethylformamide (5 mL), and 2-(7-azabenzotriazole)-*N,N,N',N*'-tetramethyluronium hexafluorophosphate (740 mg, 1.95 mmol) was added to the reaction solution, and the mixture was continued to stir at room temperature for 30 minutes. Then, **133-2** (500 mg, 1.3 mmol) and *N,N*-diisopropylethylamine (838 mg, 6.49 mmol) were added to the reaction solution. The reaction system was continued to stir at room temperature for 2 hours. After LCMS monitoring showed that the raw materials disappeared, water (50 mL) was added to the reaction solution to quench. The mixture was extracted with ethyl acetate (80 mL × 3 times), and the organic phases were combined. The organic phases were washed with saturated brine (80 mL × 3 times), then dried over anhydrous sodium sulfate, filtered, and concentrated under reduced pressure. The resulting residue was purified by silica gel column chromatography (eluent: dichloromethane/ethyl acetate = 1/1) to obtain 560 mg of compound **242-1.**

MS (ESI) m/z: 472.0 [M + H]⁺.

### Step 2:

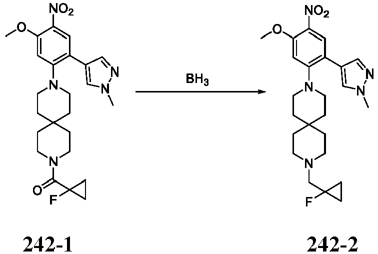

Compound **242-1** (250 mg, 0.53 mmol) was dissolved in anhydrous tetrahydrofuran (5 mL), and borane dimethyl sulfide (5 mL) was added to the reaction solution under nitrogen protection at 0 °C. The reaction system was heated to room temperature and continued to stir for 2 hours. After LCMS monitoring showed that the raw materials disappeared, the reaction solution was slowly added dropwise to methanol (5 mL) at 0 °C. The reaction solution was heated to 65 °C and continued to stir for 30 minutes. The reaction solution was cooled to room temperature and concentrated under reduced pressure, and the resulting residue was purified by silica gel column chromatography (eluent: dichloromethane/methanol = 10/1) to obtain 140 mg of compound **242-2.**

MS (ESI) m/z: 458.1 [M + H]⁺.

### Step 3:

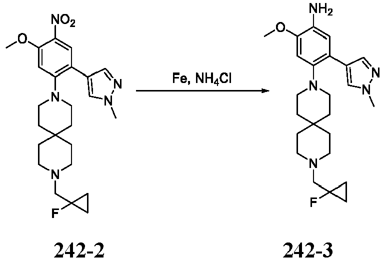

Compound **242-2** (140 mg, 0.31 mmol) was dissolved in ethanol (2.5 mL). Then, ammonium chloride (65 mg, 1.22 mmol), iron powder (85 mg, 1.53 mmol) and water (0.5 mL) were added to the reaction. The resulting reaction system was heated to 90 °C and continued to stir for 2 hours. After the LCMS monitoring showed that the raw materials disappeared, the reaction solution was cooled to room temperature and filtered. The filter cake was washed with ethanol (2 mL × 2 times) and the filtrate was concentrated under reduced pressure. Water (5 mL) was added to the resulting residue, and the mixture was extracted with ethyl acetate (7.5 mL×2 times). The organic phases were combined, and the organic phases were washed with saturated brine (5 mL×2 times) first, and then dried over anhydrous sodium sulfate, filtered, and finally concentrated under reduced pressure. The resulting residue was purified by silica gel column chromatography (eluent: dichloromethane/methanol = 10/1) to obtain 100 mg of compound **242-3.**

MS (ESI) m/z: 428.2 [M + H]⁺.

### Step 4:

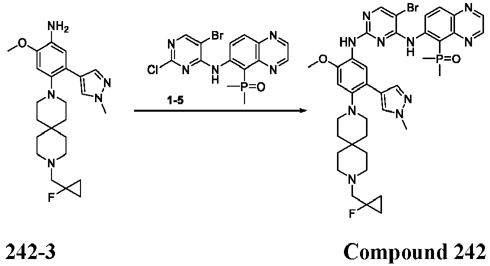

Compound **242-3** (93 mg, 0.22 mmol) was dissolved in N-methylpyrrolidone (1 mL), then compound **1-5** (60 mg, 0.15 mmol) and methanesulfonic acid (42 mg, 0.44 mmol) were added to the above solution sequentially. The reaction solution was heated to 95 °C and continued to stir for 16 hours. After LCMS monitoring showed that the raw material disappeared, the reaction solution was cooled to room temperature and directly purified by reversed-phase C18 column. Purification conditions were as follows: chromatographic column 80 g C18 reversed-phase column; mobile phase water (containing 0.1 % formic acid) and acetonitrile; flow rate of 50 mL/min; gradient: acetonitrile increased from 10 % to 50 % within 10 minutes; detection wavelength of 254 nm. The product was collected and lyophilized under reduced pressure to obtain 18 mg of compound **242.**

MS (ESI) m/z: 802.9, 804.9 [M + H]⁺.

¹H NMR (300 MHz, CDCl₃) δ 12.60 (s, 1H), 9.02 (dd, *J* = 9.6, 4.2 Hz, 1H), 8.75 (d, *J =* 2.1 Hz, 1H), 8.71 *(d, J=* 1.8 Hz, 1H), 8.31 (s, 1H), 8.21 (s, 1H), 7.70 (s, 1H), 7.65 (s, 2H), 7.33 (s, 1H), 6.75 (s, 1H), 3.93 (s, 3H), 3.76 (s, 3H), 2.97-2.78 (m, 10H), 2.17 (s, 3H), 2.12 (s, 3H), 1.76-1.63 (m, 8H), 1.20-1.10 (m, 2H), 0.88-0.80 (m, 2H).

### Embodiment 243:

### (6-((5-Bromo-2-((4-(9-(1-fluoro-2-methylpropan-2-yl)-3,9-diazaspiro[5.5]undecan-3-yl)-2-methoxy-5-(1-methyl-1H-pyrazol-4-yl)phenyl)amino)pyrimidin-4-yl)amino)quinoxalin-5-yl)dimethylphosphine oxide

### Step 1:

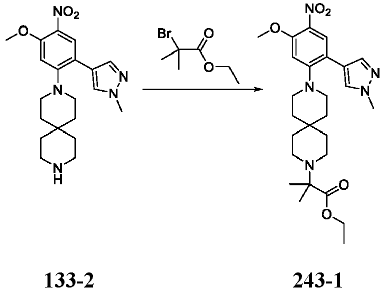

Compound **133-2** (9.7 g, 25.16 mmol) was dissolved in *N*,*N*-dimethylformamide (100 mL), then anhydrous potassium carbonate (14 g, 100.66 mmol) and ethyl 2-bromoisobutyrate (19.63 g, 100.66 mmol) were added to the reaction solution . The reaction system was heated to 80 °C and continued to stir for 16 hours. After LCMS monitoring showed that the raw materials disappeared, the reaction solution was cooled to room temperature and poured into water (800 mL) to quench. The mixture was extracted with ethyl acetate (300 mL × 3 times), and the organic phases were combined. The organic phases were washed with saturated brine (300 mL × 3 times), then dried over anhydrous sodium sulfate, filtered, and concentrated under reduced pressure. The resulting residue was purified by silica gel column chromatography (eluent: dichloromethane/ethyl acetate = 1/1) to obtain 8.6 g of compound **243-1.**

MS (ESI) m/z: 500.2 [M + H]⁺.

### Step 2:

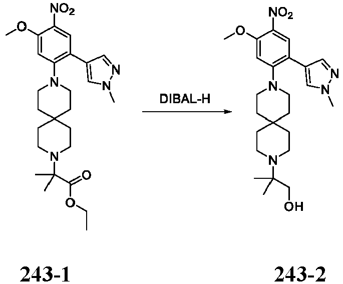

Compound **243-1** (500 mg, 1 mmol) was dissolved in dichloromethane (10 mL) under nitrogen atmosphere. Then, the reaction solution was cooled to -78 °C, and diisobutylaluminum hydride (1.5 *M,* 1.67 mL, 2.5 mmol) was added to the reaction solution, and the mixture was continued to stir for 15 minutes. The reaction system was heated to 0 °C and continued to stir for 1 hour. After LCMS monitoring showed that the raw material disappeared, saturated ammonium chloride aqueous solution (10 mL) was added to the reaction solution. The mixture was extracted with dichloromethane (50 mL × 3 times), and the organic phases were combined. The organic phases were washed with saturated brine (50 mL × 3 times) first, then dried over anhydrous sodium sulfate, filtered, and finally concentrated under reduced pressure; the resulting residue was purified by silica gel column chromatography (eluent: dichloromethane/methanol=10/1) to obtain 370 mg of compound **243-2.**

MS (ESI) m/z: 458.3 [M + H]⁺.

### Step 3:

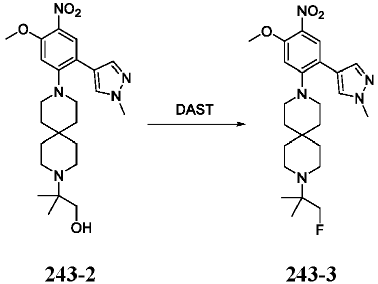

Compound **243-2** (500 mg, 1.09 mmol) was dissolved in dichloromethane (20 mL) under nitrogen atmosphere. Then, diethylamino sulfur trifluoride (1057 mg, 6.56 mmol) was slowly added dropwise to the reaction solution at -10 °C, and the mixture was continued to stir at this temperature for 30 minutes. The reaction system was heated to room temperature and continued to stir for 1 hour. After LCMS monitoring showed that the raw materials disappeared, water (10 mL) was added to the reaction solution to quench. The mixture was extracted with dichloromethane (50 mL × 3 times), and the organic phases were combined. The organic phases were washed with saturated brine (50 mL × 3 times) first, then dried over anhydrous sodium sulfate, filtered, and finally concentrated under reduced pressure; the resulting residue was purified by silica gel column chromatography (eluent: dichloromethane/methanol=10/1) to obtain 400 mg of compound **243-3.**

MS (ESI) m/z: 460.4 [M + H]⁺.

### Step 4:

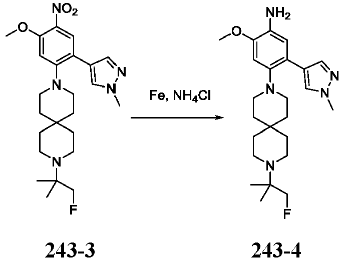

Compound **243-3** (100 mg, 0.22 mmol) was dissolved in ethanol (5 mL) and water (1 mL). Iron powder (61 mg, 1.1 mmol) and ammonium chloride (58 mg, 1.1 mmol) were then added to the reaction solution. The reaction system was heated to 80 °C and continued to stir for 2 hours. After LCMS monitoring showed that the raw materials disappeared, the reaction solution was cooled to room temperature and concentrated under reduced pressure. The resulting residue was purified by silica gel column chromatography (eluent: dichloromethane/methanol = 10/1) to obtain 90 mg of compound **243-4.**

MS (ESI) m/z: 430.2 [M + H]⁺.

### Step 5:

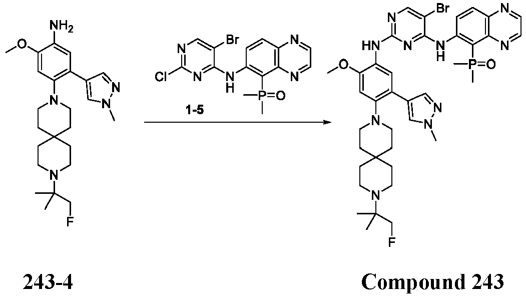

Compound **243-4** (50 mg, 0.12 mmol) was dissolved in *N*-methylpyrrolidone (2 mL), then compound **1-5** (48 mg, 0.12 mmol) and methanesulfonic acid (34 mg, 0.35 mmol) were added to the above solution sequentially. The reaction solution was heated to 95 °C and continued to stir for 16 hours. After LCMS monitoring showed that the raw material disappeared, the reaction solution was cooled to room temperature and directly purified by reversed-phase C18 column. Purification conditions were as follows: chromatographic column 40 g C18 reversed-phase column; mobile phase water (containing 0.1 % formic acid) and acetonitrile; flow rate of 35 mL/min; gradient: acetonitrile increased from 10 % to 45 % within 25 minutes; detection wavelength of 254 nm. The product was collected and lyophilized under reduced pressure to obtain 15 mg of compound **243.**

MS (ESI) m/z: 805.4, 807.4 [M + H]⁺.

¹H NMR (300 MHz, CDCl₃) δ 12.58 (s, 1H), 9.04-8.99 (m, 1H), 8.74 (d, *J =* 10.2 Hz, 2H), 8.30 (s, 1H), 8.19 (s, 1H), 7.69-7.65 (m, 3H), 7.40 (s, 1H), 6.74 (s, 1H), 3.93 (s, 3H), 3.76 (s, 3H), 2.86-2.78 (m, 10H), 2.17 (s, 3H), 2.13 (s, 3H), 1.78 (s, 4H), 1.63 (s, 4H), 1.52 (s, 3H), 1.45 (s, 3H).

¹⁹F NMR (282 MHz, CDCl₃) δ -137.04.

### Embodiment 244:

### 1-((9-(4-((5-Bromo-4-((5-(dimethylphosphoryl)quinoxalin-6-yl)amino)pyrimidin-2-yl)amino)-5-methoxy-2-(1-methyl-1H-pyrazol-4-yl)phenyl)-3,9-diazaspiro[5.5]undecan-3-yl)methyl)cyclopropane-1-carbonitrile

### Step 1:

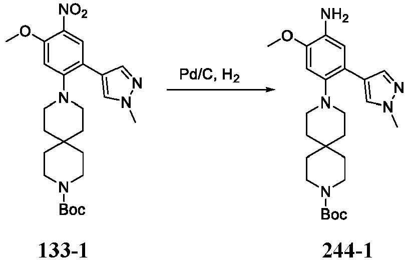

Compound **133-1** (15 g, 30.9 mmol) was dissolved in ethanol (80 mL); and then wet palladium on carbon (2 g) was added to the above solution; the reaction system was replaced with hydrogen for 3 times, and then stirred at room temperature for 16 hours. After LCMS monitoring showed that the raw materials disappeared, the reaction solution was filtered through diatomite, and the filter cake was washed with ethanol (50 mL × 3 times). The filtrate was concentrated under reduced pressure, and the resulting residue was purified by silica gel column chromatography (eluent: dichloromethane/methanol = 10/1) to obtain 13.5 g of compound **244-1.**

MS (ESI) m/z: 456.3 [M + H]⁺.

### Step 2:

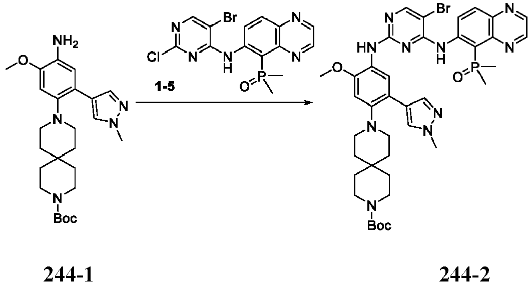

Compounds **224-1** (2.2 g, 4.83 mmol) and 1-5 (1.79 g, 4.35 mmol) were dissolved in dioxane (30 mL) under nitrogen atmosphere. Then, dichloro-[1,3-bis(diisopropylphenyl)imidazolylidene]-(3-chloropyridyl)palladium (0.33 g, 0.48 mmol) and cesium carbonate (3.15 g, 9.7 mmol) were added to the above reaction solution. The reaction system was heated to 100 °C and continued to stir for 16 hours. After LCMS monitoring showed that the raw materials disappeared, the reaction solution was cooled to room temperature and concentrated under reduced pressure. The resulting residue was purified by silica gel column chromatography (eluent: dichloromethane/methanol = 10/1) to obtain 1.2 g of compound **244-2.**

MS (ESI) m/z: 831.0, 833.0 [M + H]⁺.

### Step 3:

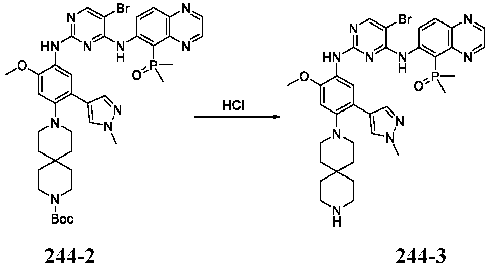

Prepared according to the method of step 2 in Embodiment 133, the raw materials were replaced with compound **244-2** (1.2 g, 1.44 mmol) to obtain 1 g of compound **244-3.**

MS (ESI) m/z: 731.1, 733.1 [M + H]⁺.

¹H NMR (400 MHz, CD₃OD) δ 8.82-8.75 (m, 3H), 8.22 (s, 1H), 7.88 (s, 1H), 7.80 (s, 1H), 7.48-7.73 (m, 2H), 6.83 (s, 1H), 3.89 (s, 3H), 3.66 (s, 3H), 2.83 (t, *J=* 5.6 Hz, 8H), 2.15 (s, 3H), 2.11 (s, 3H), 1.63 (t, *J* = 5.6 Hz, 4H), 1.57 (t, *J* = 5.6 Hz, 4H).

### Step 4:

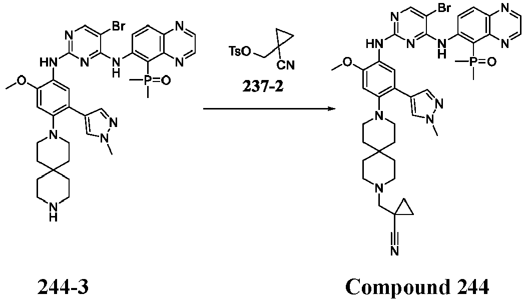

Prepared according to the method of step 3 in Embodiment 133, the raw materials were replaced with compound 237-2 (74 mg, 0.29 mmol) to obtain 15 mg of compound 244.

MS (ESI) m/z: 810.2, 812.2 [M + H]⁺.

¹H NMR: (300 MHz, CDCl₃) δ 12.61 (s, 1H), 9.02 (dd, *J* = 9.6, 4.2 Hz, 1H), 8.75 (d, *J =* 1.8 Hz, 1H), 8.71 (d, *J* = 1.8 Hz, 1H), 8.31 (s, 1H), 8.21 (s, 1H), 7.72 (s, 1H), 7.64 (s, 2H), 7.33 (s, 1H), 6.75 (s, 1H), 3.93 (s, 3H), 3.76 (s, 3H), 2.87-2.83 (m, 4H), 2.57-2.49 (m, 6H), 2.17 (s, 3H), 2.12 (s, 3H), 1.62-1.60 (m, 8H), 1.32 (s, 2H), 0.90 (s, 2H).

### Embodiment 247:

### (6-((5-Bromo-2-((4-(5-(2-fluoroethyl)hexahydropyrrolo[3,4-c]pyrrol-2(1H)-yl)-2-methoxy-5-(1-methyl-1H-pyrazol-4-yl)phenyl)amino)pyrimidin-4-yl)amino)quinoxalin-5-yl)dimethylphosphine oxide

### Step 1:

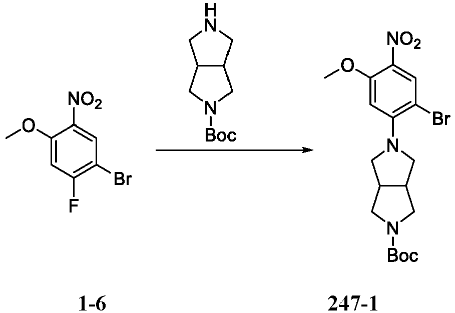

Prepared according to the method of step 4 in Embodiment 1, the raw materials were replaced with tert-butyl hexahydropyrrolo[3,4-*c*]pyrrole-2(1*H*)-carboxylate (5 g, 23.56 mmol) to obtain 6.6 g of compound 247-1.

MS (ESI) m/z: 442.2, 444.2 [M + H]⁺.

### Step 2:

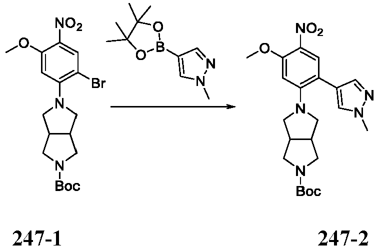

Prepared according to the method of step 5 in Embodiment 1, the raw materials were replaced with 247-1 (500 mg, 1.1 mmol) to obtain 380 mg of compound **247-2.**

MS (ESI) m/z: 444.1 [M + H]⁺.

### Step 3:

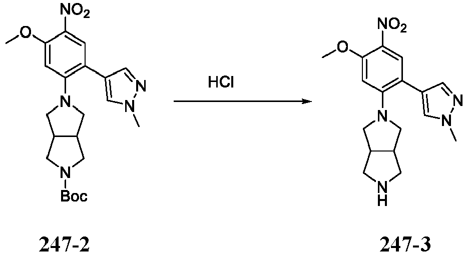

Prepared according to the method of step 2 in Embodiment 133, the raw materials were replaced with **247-2** (380 mg, 0.86 mmol) to obtain 290 mg of compound **247-3.**

MS (ESI) m/z: 344.1 [M + H]⁺.

### Step 4:

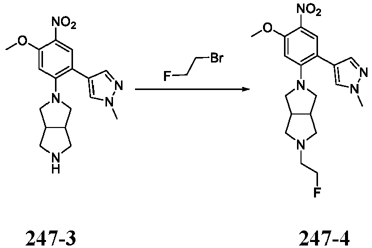

Prepared according to the method of step 3 in Embodiment 133, the raw materials were replaced with **247-3** (290 mg, 0.85 mmol) to obtain 280 mg of compound **247-4.**

MS (ESI) m/z: 390.3 [M + H]⁺.

### Step 5:

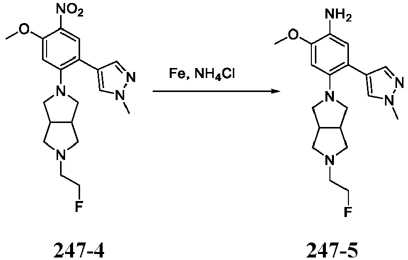

Prepared according to the method of step 3 in Embodiment 70, the raw materials were replaced with **247-4** (280 mg, 0.72 mmol) to obtain 230 mg of compound **247-5.**

MS (ESI) m/z: 360.3 [M + H]⁺.

### Step 6:

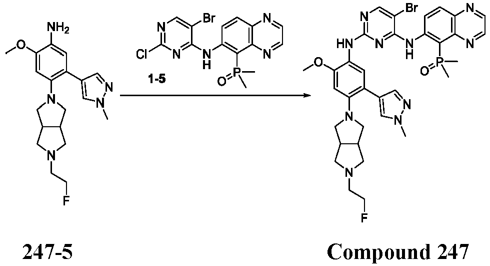

Prepared according to the method of step 7 in Embodiment 1, the raw materials were replaced with 247-5 (60 mg, 0.17 mmol) to obtain 25 mg of compound **247.**

MS (ESI) m/z: 735.3, 737.3 [M + H]⁺.

¹H NMR: (300 MHz, CDCl₃) δ 12.58 (s, 1H), 8.01 (dd, J= 9.6, 4.2 Hz, 1H), 8.74-8.69 (m, 2H), 8.27 (s, 1H), 8.12 (s, 1H), 7.60 (s, 2H), 7.50 (s, 1H), 7.30 (s, 1H), 6.68 (s, 1H), 4.74c4.55 (m, 2H), 3.91 (s, 3H), 3.78 (s, 3H), 3.01-2.84 (m, 10H), 2.44-2.41 (m, 2H), 2.14 (s, 3H), 2.10 (s, 3H).

The compounds given in Table 1 below were prepared by substantially the same procedure as described in the above embodiments.

**Table 1 Compounds**

| **Compound** | **Structural formula** | **¹HNMR** | **MS (ESI) M/Z [M+H]⁺** |
|---|---|---|---|
| 2 | 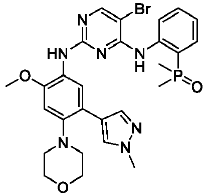 | ¹H NMR (400 MHz, DMSO-*d₆*) δ 10.96 (s, 1H), 8.35-8.29 (m, 1H), 8.26 (s, 1H), 8.19-8.18 (m, 1H), 8.03 (s, 1H), 7.85 (s, 1H), 7.59 (s, 1H), 7.53-7.47 (m, 1H), 7.01-6.97 (m, 2H), 6.81 (s, 1H), 3.84 (s, 3H), 3.81 (s, 3H), 3.76-3.73 (m, 4H), 2.86-2.84 (m, 4H), 1.78 (s, 3H), 1.74 (s, 3H). | 612.1, 614.1 |
| 3 | 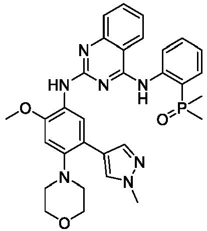 | ¹H NMR (400 MHz, CDCl₃) δ 11.80 (s, 1H), 9.06-9.02 (m, 1H), 8.74 (s, 1H), 8.24 (d, *J* = 8.0 Hz, 1H), 8.03 (s, 1H), 7.85 (s, 1H), 7.68-7.61 (m, 2H), 7.42 (brs, 1H), 7.33-7.29 (m, 2H), 7.28-7.26 (m, 1H), 7.09-7.05 (m, 1H), 6.74 (s, 1H), 3.96 (s, 3H), 3.95 (s, 3H), 3.86-3.83 (m, 4H), 2.96-2.94 (m, 4H), 1.92 (s, 3H), 1.88 (s, 3H). | 584.3 |
| 4 | 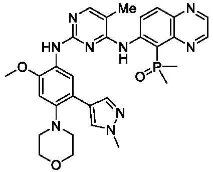 | ¹H NMR (400 MHz, DMSO-*d₆*) δ 12.51 (s, 1H), 9.18 (dd, *J* = 9.6, 3.6 Hz, 1H), 8.84 (d, *J* = 2.0 Hz, 1H), 8.80 (d, *J* = 2.0 Hz, 1H), 8.06 (s, 1H), 8.00 (s, 1H), 7.90 (s, 1H), 7.84 - 7.75 (m, 2H), 7.55 (d, *J* = 9.6 Hz, 1H), 6.85 (s, 1H), 3.85 (s, 3H), 3.77 (s, 3H), 3.75 (t, *J* = 4.4 Hz, 4H), 2.86 (t, *J* = 4.4 Hz, 4H), 2.15 (s, 3H), 2.05 (s, 3H), 2.02 (s, 3H) | 600.2 |
| 5 | 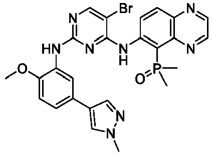 | ¹H NMR (300 MHz, CDCl₃) δ 12.70 (s, 1H), 8.99 (dd, J= 9.6, 4.2 Hz, 1H), 8.75 (dd, J= 7.2, 1.8 Hz, 2H), 8.35 (s, 1H), 8.33 (s, 1H), 7.75 (d, *J* = 9.6 Hz, 2H), 7.35 (s, 2H), 7.12 (dd, *J* = 8.1, 2.1 Hz, 1H), 6.92 (d, *J* = 8.4 Hz, 1H), 3.94 (s, 3H), 3.75 (s, 3H), 2.18 (s,3H), 2.13 (s, 3H). | 579.1, 581.1 |
| 9 | 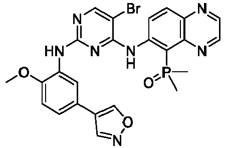 | ¹H NMR (300 MHz, CDCl₃) δ 12.58 (s, 1H), 8.86 (dd, J= 9.6, 4.2 Hz, 1H), 8.78 (dd, *J* = 7.5, 1.8 Hz, 2H), 8.47 (s, 1H), 8.36 (s, 1H), 8.23 (s, 1H), 8.10 (s, 1H), 7.84 (d, *J* = 9.6 Hz, 1H), 7.68 (s, 1H), 7.09 (dd, *J* = 8.1, 2.1 Hz, 1H), 6.94 (d, *J* = 8.1 Hz, 1H), 3.96 (s, 3H), 2.20 (s, 3H), 2.15 (s, 3H). | 566.0, 568.0 |
| 10 | 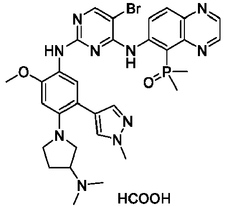 | ¹H NMR (300 MHz, CDCl₃) δ 12.58 (s, 1H), 9.00 (dd, J= 9.6, 4.2 Hz, 1H), 8.74 (dd, *J* = 10.8, 1.8 Hz, 2H), 8.35 (s, 1H), 8.30 (s, 1H), 8.14 (s, 1H), 7.66 (d, *J* = 9.6 Hz, 1H), 7.50 (s, 1H), 7.48 (s, 1H),7.37 (s, 1H), 6.67 (s, 1H), 3.94 (s, 3H), 3.80 (s, 3H), 3.61 - 3.52 (m, 1H), 3.46 - 3.41 (m, 1H), 3.30 - 3.23 (m, 2H), 3.08 - 3.00 (m, 1H), 2.69 (s, 6H), 2.30 - 2.25 (m, 2H), 2.17 (s, 3H), 2.12 (s, 3H) | 691.1, 693.1 |
| 11 | 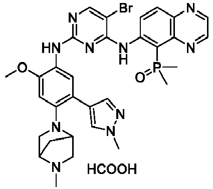 | ¹H NMR (400 MHz, DMSO-*d₆*) δ 12.68 (s, 1H), 8.91-8.75 (m, 3H), 8.42 (s, 1H), 8.25 (s, 2H), 7.72 (s, 2H), 7.50 (s, 1H), 7.27 (s, 1H), 6.64 (s, 1H), 4.10 (s, 1H), 3.96 (s, 3H), 3.89 (s, 3H), 3.51-3.45 (m, 1H), 2.96-2.94 (m, 2H), 2.84-2.78 (m, 2H), 2.37 - 2.29 (m, 3H), 2.04 (s, 3H), 2.00 (s, 3H), 1.88-1.79 (m, 2H). | 689.3, 691.3 |
| 12 | 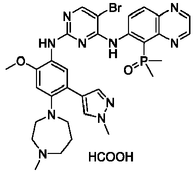 | ¹H NMR (300 MHz, CDCl₃) δ 12.59 (s, 1H), 8.98 (dd, *J* = 9.6, 4.2 Hz, 1H), 8.78 (d, *J* = 2.1 Hz, 1H), 8.74 (d, *J* = 2.1 Hz, 1H), 8.49 (s, 1H), 8.31 (s, 1H), 8.22 (s, 1H), 7.69 (d, *J* = 9.6 Hz, 1H), 7.63 (s, 1H), 7.45 (d, *J* = 3.3 Hz, 2H), 6.74 (s, 1H), 3.93 (s, 3H), 3.76 (s, 3H), 3.41 (m, 2H), 3.30 (m, 2H), 3.17 (m, 2H), 3.15 (d, *J* = 12.3 Hz, 2H), 2.77 (s, 3H), 2.17 (s, 5H), 2.13 (s, 3H). | 691.2, 693.2 |
| 14 | 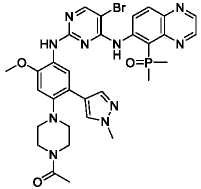 | ¹H NMR (400 MHz, CDCl₃) δ 13.02 (s, 1H), 8.91-8.88 (m, 1H), 8.80 (d, *J* = 1.6 Hz, 1H), 7.54 (d, *J* = 2.0 Hz, 1H), 8.22 (s, 1H), 8.02 (s, 1H), 7.74 (s, 1H), 7.64 - 7.57 (m, 2H), 6.69 (s, 1H), 3.92 (s, 3H), 3.81 (s, 3H), 2.76 (s, 2H), 3.56 (s, 2H), 2.95 - 2.90 (m, 4H), 2.17 (s, 3H), 2.15 (s, 3H), 2.13 (s, 3H). | 705.1, 707.1 |
| 15 | 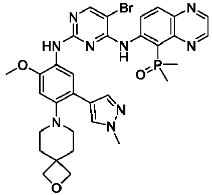 | ¹H NMR (400 MHz, CDsOD) δ 8.88 (dd, J= 9.6, 4.0 Hz, 1H), 8.83 (d, *J* = 2.0 Hz, 1H), 8.79 (d, *J* = 2.0 Hz, 1H), 8.23 (s, 1H), 7.72 (s, 1H), 7.60 -7.56 (m, 2H), 7.39 (s, 1H), 6.75 (s, 1H), 3.95 - 3.84 (m, 5H), 3.75 - 3.73 (m, 4H), 3.67 - 3.65 (m, 1H), 3.10 - 3.02 (m, 4H), 2.17 (s, 3H), 2.14 (s, 3H), 2.06 - 1.92 (m, 4H). | 704.2, 706.2 |
| 18 | 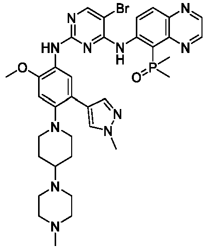 | ¹H NMR: (300 MHz, CDsOD) δ 8.88-8.83 (m, 2H), 8.79 (d, *J* = 2.1 Hz, 1H), 8.46 (s, 1H), 8.27 (s, 1H), 7.94 (s, 1H), 7.80 (s, 1H), 7.53 - 7.51 (m, 2H), 6.84 (s, 1H), 3.92 (s, 3H), 3.69 (s, 3H), 3.24 - 3.20 (m, 2H), 3.11 - 2.85 (m, 8H), 2.72 - 2.60 (m, 6H), 2.19 (s, 3H), 2.14 (s, 3H), 2.04 - 2.00 (m, 2H), 1.77 - 1.65 (m, 2H). | 760.2, 762.2 |
| 19 | 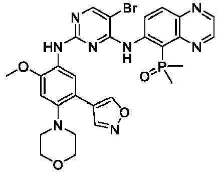 | ¹H NMR: (400 MHz, CDCl₃) δ 8.83 (s, 1H), 8.79 (d, J = 2.0 Hz, 1H), 8.76 - 8.71 (m, 1H), 8.63 (s, 1H), 8.19 (s, 1H), 8.07 -7.95 (m, 1H), 7.72 (d, J = 7.6 Hz, 1H), 6.80 (s, 1H), 3.97 (s, 3H), 3.85-3.83 (m, 4H), 2.94 - 2.91 (m, 4H), 2.19 (s, 3H), 2.16 (s, 3H). | 651.1, 653.1 |
| 20 | 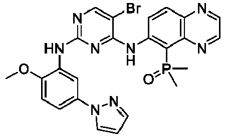 | ¹H NMR (300 MHz, CDCl₃) δ 12.78 (s, 1H), 8.88 (dd, J= 9.6, 3.9 Hz, 1H), 8.77 (dd, *J* = 13.5, 1.8 Hz, 2H), 8.62 (s, 1H), 8.32 (s, 1H), 8.11 (s, 1H), 7.78 (d, *J* = 9.0 Hz, 1H), 7.62 (s, 1H), 7.47 (s, 1H), 7.33 (d, *J* = 7.8 Hz, 1H), 6.97 (d, *J* = 8.7 Hz, 1H), 6.19 (s, 1H), 3.97 (s, 3H), 2.18 (s, 3H), 2.13 (s, 3H). | 565.1, 567.1 |
| 21 | 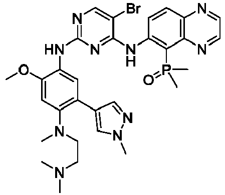 | ¹H NMR: (300 MHz, CDsOD) δ 8.88-8.83 (m, 2H), 8.79 (d, *J* = 2.1 Hz, 1H), 8.46 (s, 1H), 8.27 (s, 1H), 7.94 (s, 1H), 7.80 (s, 1H), 7.53 - 7.51 (m, 2H), 6.84 (s, 1H), 3.92 (s, 3H), 3.69 (s, 3H), 3.24 - 3.20 (m, 2H), 3.11 - 2.85 (m, 8H), 2.72 - 2.60 (m, 6H), 2.19 (s, 3H), 2.14 (s, 3H), 2.04 - 2.00 (m, 2H), 1.77 - 1.65 (m, 2H). | 679.0, 681.0 |
| 23 | 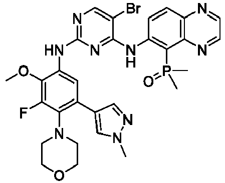 | ¹H NMR (400 MHz, CDCl₃-*d*) δ 12.64 (s, 1H), 8.93 (dd, *J* = 9.6, 4.0 Hz, 1H), 8.80 - 8.72 (dd, *J* = 7.2, 2.0 Hz, 2H), 8.34 (s, 1H), 8.17 (d, *J* = 2.0 Hz, 1H), 7.78 (s, 1H), 7.67 (d, *J* = 9.6 Hz, 1H), 7.47 (d, *J* = 7.6 Hz, 2H), 7.28 (s, 1H), 4.01 (d, *J* = 1.2 Hz, 3H), 3.80 (t, *J* = 4.4 Hz, 4H), 3.77 (s, 3H), 3.13 (s, 4H), 2.17 (s, 3H), 2.14 (s, 3H). | 681.9, 683.9 |
| | | ¹⁹F NMR: (377 MHz, CDCl₃) δ -137.83. | |
| 24 | 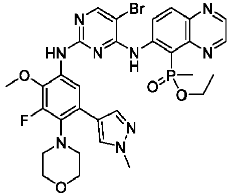 | ¹H NMR (300 MHz, DMSO-*d*₆) δ 12.40 (s, 1H), 8.90 (d, *J* = 2.1 Hz, 1H), 8.89 - 8.78 (m, 3H), 8.38 (s, 1H), 8.10 (s, 1H), 7.67 (s, 1H), 7.58 (d, *J* = 1.5 Hz, 2H), 4.03 - 3.91 (m, 1H), 3.81 (s, 3H), 3.78 (s, 3H), 3.72 (t, *J* = 4.5 Hz, 4H), 3.03 (s, 4H), 2.07 (d, *J* = 16.2 Hz, 3H), 1.07 (t, *J* = 7.2 Hz, 3H). | 712.1, 714.1 |
| | | ¹⁹F NMR: (282 MHz, CDCl₃) δ -138.08. | |
| 25 | 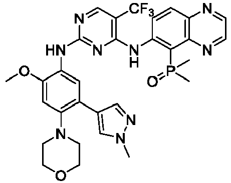 | ¹H NMR (300 MHz, DMSO-*d*₆) δ 12.64 (s, 1H), 8.99 (s, 1H), 8.83 (d, *J* = 6.9 Hz, 2H), 8.45 (s, 1H), 8.06 (s, 1H), 7.78 (s, 1H), 7.53 (s, 1H), 6.83 (s, 1H), 3.82 (s, 3H), 3.79 (s, 3H), 3.75 (t, *J* = 4.5 Hz, 4H), 2.85 (t, *J* = 4.5 Hz, 4H), 2.03 (s, 3H), 1.98 (s, 3H). | 654.2 |
| 26 | 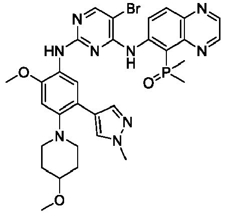 | ¹H NMR (300 MHz, DMSO-*d*₆) δ 12.68 (s, 1H), 8.84 (d, *J* = 6.9 Hz, 3H), 8.41 (s, 1H), 8.28 (s, 1H), 8.03 (s, 1H), 7.77 (s, 1H), 7.59 (s, 1H), 6.83 (s, 1H), 3.81 (s, 3H), 3.78 (s, 3H), 3.29 (s, 3H), 3.08 - 2.96 (m, 2H), 2.69 (t, *J* = 10.8 Hz, 2H), 2.04 (s, 3H), 2.00 (s, 3H), 1.97-1.95 (m, 2H), 1.69 - 1.55 (m, 2H). | 692.1, 694.1 |
| 28 | 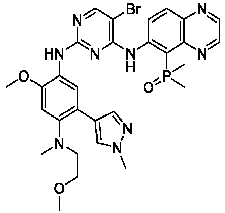 | ¹H NMR (300 MHz, CDCl₃) δ 12.58 (s, 1H), 9.01 (dd, J= 9.6, 4.2 Hz, 1H), 8.73 (d, *J* = 7.2 Hz, 2H), 8.31 (s, 1H), 8.26 (s, 1H), 7.87 (s, 1H), 7.63 (d, *J* = 9.0 Hz, 1H), 7.42 (d, *J* = 8.1 Hz, 2H), 6.78 (s, 1H), 3.92 (s, 3H), 3.79 (s, 3H), 3.48 (t, *J* = 6.0 Hz, 2H), 3.33 (s, 3H), 3.12 (t, *J* = 6.0 Hz, 2H), 2.68 (s, 3H), 2.18 (s, 3H), 2.13 (s, 3H). | 666.3, 668.3 |
| 29 | 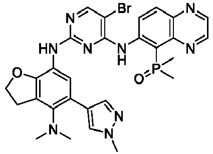 | ¹H NMR (300 MHz, CD₃OD) δ 8.93 (dd, *J* = 9.6, 4.8 Hz, 1H), 8.80 (d, *J* = 2.1 Hz, 2H), 8.24 (s, 1H), 7.80 (s, 1H), 7.64 (s, 1H), 7.60 (d, *J* = 10.5 Hz, 1H), 7.41 (s, 1H), 4.58 (t, *J* = 8.7 Hz, 2H), 3.76 (s, 3H), 3.45 (t, *J* = 8.7 Hz, 2H), 2.75 (s, 6H), 2.19 (s, 3H), 2.14 (s, 3H). | 633.9, 635.9 |
| 30 | 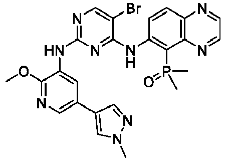 | ¹H NMR (300 MHz, CDCl₃) δ 12.65 (s, 1H), 8.92 (dd, *J* = 9.6, 4.2 Hz, 1H), 8.77 (dd, *J* = 7.8, 1.8 Hz, 2H), 8.65 (d, *J* = 2.1 Hz, 1H), 8.37 (s, 1H), 7.91 (d, *J* = 2.1 Hz, 1H), 7.87 (d, *J* = 9.6 Hz, 1H), 7.52 (s, 1H), 7.42 (s, 1H), 7.36 (s, 1H), 4.07 (s, 3H), 3.82 (s, 3H), 2.19 (s, 3H), 2.14 (s, 3H). | 580.1, 582.1 |
| 32 | 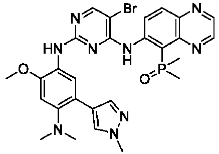 | ¹H NMR (300 MHz, CDCl₃) δ 12.83 (s, 1H), 8.95 (dd, *J* = 9.6, 4.2 Hz, 1H), 8.77-8.73 (m, 2H), 8.25 (s, 1H), 8.11 (s, 1H), 7.94-7.71 (m, 2H), 7.62 (d, *J* = 4.8 Hz, 1H), 7.46 (s, 1H), 6.83 (s, 1H), 3.95 (s, 3H), 3.84 (s, 3H), 2.74 (s, 6H), 2.17 (s, 3H), 2.12 (s, 3H). | 622.1, 624.1 |
| 34 | 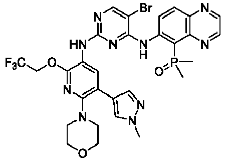 | ¹H NMR (400 MHz, CDCl₃) δ 12.68 (s, 1H), 8.91 (dd, *J* = 9.6, 4.2 Hz, 1H), 8.76 (dd, *J* = 7.5, 1.5 Hz, 2H), 8.46 (s, 1H), 8.32 (s, 1H), 7.72 (d, *J* = 9.6 Hz, 1H), 7.66 (s, 1H), 7.53 (s, 1H), 7.19 (s, 1H), 4.87 (q, *J* = 12.6, 6.3 Hz, 2H), 3.81 (m, 7H), 3.10 (t, *J* = 3.3 Hz, 4H), 2.18 (s, 3H), 2.14 (s, 3H). | 733.0, 735.0 |
| 35 | 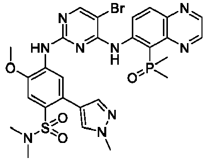 | ¹H NMR (300 MHz, DMSO-*d*₆) δ 12.79 (s, 1H), 8.90 (s, 2H), 8.78 (dd, *J* = 9.6, 3.9 Hz, 1H), 8.49 (s, 1H), 8.44 (s, 1H), 8.09 (s, 1H), 7.71 (s, 2H), 7.47 (s, 1H), 7.33 (s, 1H), 3.96 (s, 3H), 3.76 (s, 3H), 2.43 (s, 6H), 2.07 (s, 3H), 2.02 (s, 3H). | 686.1, 688.1 |
| 36 | 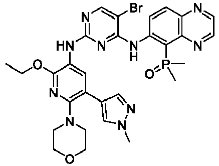 | ¹H NMR (300 MHz, DMSO-*d*₆) δ 12.68 (s, 1H), 8.83 (dd, *J* = 9.0, 1.8 Hz, 2H), 8.74 (s, 1H), 8.56 (s, 1H), 8.30 (s, 1H), 8.00 (s, 1H), 7.88 (s, 1H), 7.77 (s, 1H), 7.52 (s, 1H), 4.35 (q, *J* = 14.1, 9.6 Hz, 2H), 3.79 (s, 3H), 3.75 (t, *J* = 4.5 Hz, 4H), 3.04 (t, *J* = 4.5 Hz, 4H), 2.05 (s, 3H), 2.00 (s, 3H), 1.26 (t, *J* = 6.9 Hz, 4H). | 679.2, 681.2 |
| 37 | 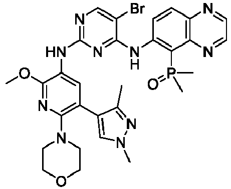 | ¹H NMR (400 MHz, CDCl₃) δ 12.54 (s, 1H), 8.88 (dd, J = 9.2, 4.0 Hz, 1H), 8.76 (dd, J = 18.4, 2.4 Hz, 2H), 8.30 (d, J = 12.0 Hz, 2H), 7.72 (s, 1H), 7.48 (s, 1H), 7.23 (s, 1H), 4.03 (s, 3H), 3.83 (s, 3H), 3.70 (t, J = 4.4 Hz, 4H), 3.08 (t, J = 4.4 Hz, 4H), 2.16 (s, 3H), 2.12 (s, 3H), 2.04 (s, 3H). | 678.9, 680.9 |
| 38 | 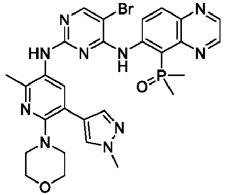 | ¹H NMR (300 MHz, CDCl₃) δ 13.20 (s, 1H), 8.79 (d, *J* = 2.1 Hz, 1H), 8.78 - 8.70 (m, 2H), 8.14 (s, 1H), 7.80 (s, 1H), 7.77 (s, 1H), 7.67 (s, 1H), 7.59 (d, *J* = 9.3 Hz, 1H), 3.90 (s, 3H), 3.83 (t, *J* = 4.5 Hz, 4H), 3.20 (t, *J* = 4.5 Hz,, 4H), 2.49 (s, 3H), 2.16 (s, 3H), 2.12 (s, 3H). | 649.2, 651.2 |
| 39 | 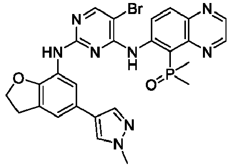 | ¹H NMR (300 MHz, CDCl₃) δ 12.94 (s, 1H), 8.97 (dd, J= 9.6, 4.2 Hz, 1H), 8.76 (dd, *J* = 9.3, 2.1 Hz, 2H), 8.17 (s, 1H), 8.12 (s, 1H), 7.90 (s, 1H), 7.80 (d, *J* = 9.3 Hz, 1H), 7.64 (s, 1H), 7.42 (d, *J* = 2.1 Hz, 1H), 7.24 (s, 1H), 4.72 (t, *J* = 8.7 Hz, 2H), 3.91 (s, 3H), 3.25 (t, *J* = 8.7 Hz, 2H), 2.18 (s, 3H), 2.13 (s, 3H). | 591.1, 593.1 |
| 40 | 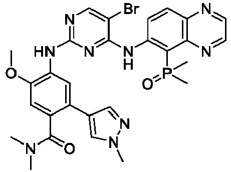 | ¹H NMR (300 MHz, DMSO-*d*₆) δ 12.73 (s, 1H), 8.85 (dd, *J* = 9.6, 1.8 Hz, 2H), 8.78 (dd, *J* = 9.6, 4.2 Hz, 1H), 8.51 (s, 1H), 8.36 (d, *J* = 1.2 Hz, 1H), 7.86 (d, *J* = 6.0 Hz, 1H), 7.63 (d, *J* = 9.6 Hz, 1H), 7.55 (s, 1H), 7.33 (s, 1H), 6.95 (s, 1H), 3.84 (s, 3H), 3.70 (s, 3H), 2.97 (s, 3H), 2.60 (s, 3H), 2.06 (s, 3H), 2.01 (s, 3H). | 650.0, 652.0 |
| 43 | 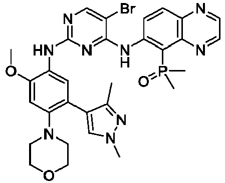 | ¹H NMR (300 MHz, DMSO-*d*₆) δ 12.68 (s, 1H), 8.87 (d, *J* = 1.2 Hz, 2H), 8.80 (d, *J* = 10.2 Hz, 1H), 8.33 (s, 1H), 8.28 (s, 1H), 7.68 (s, 2H), 7.42 (s, 1H), 6.78 (s, 1H), 3.84 (s, 3H), 3.74 (s, 3H), 3.60 (t, *J* = 4.8 Hz, 4H), 2.80 (t, *J* = 4.8 Hz, 4H), 2.05 (s, 3H), 2.02 (s, 3H), 2.00 (s, 3H). | 678.1, 680.1 |
| 44 | 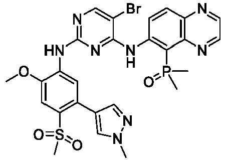 | ¹H NMR (300 MHz, DMSO-*d*₆) δ 12.80 (s, 1H), 8.90 (d, *J* = 1.2 Hz, 2H), 8.78 (dd, *J* = 9.6, 3.9 Hz, 1H), 8.49 (s, 1H), 8.46 (s, 1H), 8.22 (d, *J* = 5.4 Hz, 1H), 7.81 (s, 1H), 7.68 (d, *J* = 9.6 Hz, 1H), 7.61 (s, 1H), 7.43 (s, 1H), 3.97 (s, 3H), 3.77 (s, 3H), 2.85 (s, 3H), 2.08 (s, 3H), 2.03 (s, 3H). | 657.1, 659.1 |
| 45 | 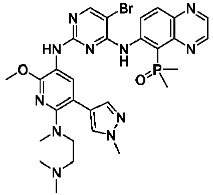 | ¹H NMR (300 MHz, CDCl₃) δ 12.61 (s, 1H), 8.92 (dd, J= 9.6, 4.2 Hz, 1H), 8.75 (dd, *J* = 6.6, 1.8 Hz, 2H), 8.51 (d, *J* = 10.5 Hz, 2H), 8.32 (s, 1H), 7.76 (s, 2H), 7.35 (s, 1H), 7.20 (s, 1H), 4.02 (s, 3H), 3.85 (s, 3H), 3.57 (t, *J* = 6.9 Hz, 2H), 2.98 (t, *J* = 6.9 Hz, 2H), 2.71 (s, 3H), 2.62 (s, 6H), 2.18 (s, 3H), 2.13 (s, 3H). | 680.2, 682.2 |
| 46 | 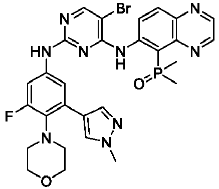 | ¹H NMR (300 MHz, DMSO-*d*₆) δ 12.72 (s, 1H), 9.65 (s, 1H), 8.89 (dd, *J* = 7.2, 1.8 Hz,, 3H), 8.41 (s, 1H), 8.11 (s, 1H), 7.92 (d, *J* = 9.3 Hz, 1H), 7.68 (s, 1H), 7.58 - 7.43 (m, 2H), 3.81 (s, 3H), 3.70 (d, *J* = 4.5 Hz, 4H), 2.96 (s, 4H), 2.09 (s, 3H), 2.04 (s, 3H). | 652.0, 654.0 |
| 47 | 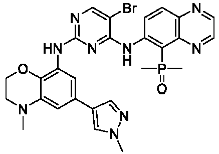 | ¹H NMR (300 MHz, DMSO-*d*₆) δ 12.72 (s, 1H), 8.94 (dd, *J* = 9.6, 4.2 Hz, 1H), 8.84 (dd, *J* = 13.8, 1.8 Hz, 2H), 8.29 (d, *J* = 6.0 Hz, 2H), 7.88 (s, 1H), 7.64 (s, 1H), 7.58 (d, *J* = 9.3 Hz, 1H), 7.20 (d, *J* = 1.8 Hz, 1H), 6.74 (d, *J* = 1.8 Hz, 1H), 4.24 (t, *J* = 4.2 Hz, 2H), 3.72 (s, 3H), 3.24 (t, *J* = 4.2 Hz, 2H), 2.93 (s, 3H), 2.06 (s, 3H), 2.01 (s, 3H). | 620.2, 622.2 |
| 48 | 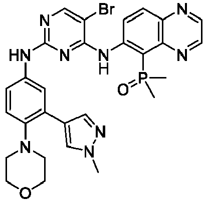 | ¹H NMR (300 MHz, CDCl₃) δ 12.66 (s, 1H), 9.40 (s, 1H), 8.93-8.85 (m, 3H), 8.35 (s, 1H), 8.09 (s, 1H), 7.75 (s, 2H), 7.68 (d, *J* = 2.4 Hz, 1H), 7.38 (d, *J* = 8.4, 1H), 7.05 (d, *J* = 8.7 Hz, 1H), 3.78 (s, 3H), 3.74-3.71 (m, 4H), 2.78-2.75 (m, 4H), 2.08 (s, 3H), 2.03 (s, 3H). | 634.3, 636.3 |
| 49 | 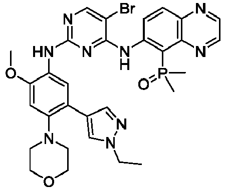 | ¹H NMR (300 MHz, CDCl₃) δ 12.62 (s, 1H), 9.05 (dd, J= 9.6, 4.8 Hz, 1H), 8.72 (dd, *J* = 7.5, 1.8 Hz, 2H), 8.31 (s, 1H), 8.25 (s, 1H), 7.83 (s, 1H), 7.63 (m, 2H), 7.33 (s, 1H), 6.72 (s, 1H), 4.06 (q, *J* = 14.7, 7.5 Hz, 2H), 3.94 (s, 3H), 3.82 (t, *J* = 4.5 Hz, 4H), 2.93 (t, *J* = 4.5 Hz, 4H), 2.17 (s, 3H), 2.12 (s, 3H), 1.41 (t, *J* = 7.5 Hz, 3H). | 678.2, 680.2 |
| 50 | 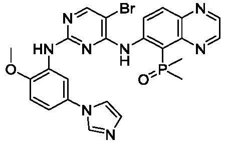 | 1H NMR (400 MHz, DMSO-d6) δ 12.69 (s, 1H), 8.85 (dd, J = 16.0, 2.0 Hz, 2H), 8.80 - 8.75 (m, 1H), 8.51 (s, 1H), 8.38 (s, 1H), 8.09 (d, J = 2.8 Hz, 1H), 8.04 (s, 1H), 7.59 (d, J = 9.2 Hz, 1H), 7.34 (dd, J = 8.8, 2.8 Hz, 1H), 7.19 (d, J = 8.8 Hz, 1H), 6.90 (s, 1H), 3.88 (s, 3H), 2.05 (s, 3H), 2.01 (s, 3H). | 565.0, 567.0 |
| 51 | 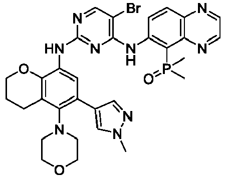 | 1H NMR (400 MHz, DMSO-d6) δ 12.72 (s, 1H), 8.88 (m, 3H), 8.30 (s, 1H), 8.11 (s, 1H), 7.66 (s, 1H), 7.61 (s, 1H), 7.48 (s, 1H), 7.35 (s, 1H), 4.5 (t, J = 4.8 Hz, 2H), 3.82 (s, 3H), 3.60 (m, 4H), 2.87 - 2.75 (m, 6H), 2.05 (s, 3H), 2.02 (s, 3H), 1.89 (t, J = 5.6 Hz, 2H). | 690.3, 692.3 |
| 52 | 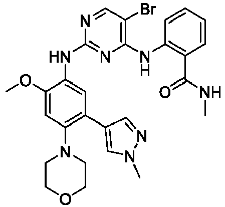 | ¹H NMR (400 MHz, DMSO-*d*₆) δ 11.45 (s, 1H), 8.71 (m, 1H), 8.50 (s, 1H), 8.31 (s, 1H), 8.20 (s, 1H), 8.05 (s, 1H), 7.86 (s, 1H), 7.68 - 7.66 (m, 1H), 7.62 (d, *J* = 7.2 Hz, 1H), 6.97 (q, *J* = 17.6, 10.4 Hz, 2H), 6.82 (s, 1H), 3.84 (s, 3H), 3.81 (s, 3H), 3.75 (t, *J* = 4.8 Hz, 4H), 2.86 (t, *J* = 4.8 Hz, 4H), 2.79 (d, *J* = 4.4 Hz, 3H). | 593.1, 595.1 |
| 53 | 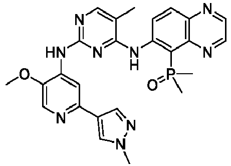 | ¹H NMR (400 MHz, DMSO-*d*₆) δ 12.67 (s, 1H), 9.23 (dd, *J* = 9.6, 4.0 Hz, 1H), 8.89 (d, *J* = 2.0 Hz, 1H), 8.84 (d, *J* = 2.0 Hz, 1H), 8.44 (d, *J* = 3.2 Hz, 1H), 8.20 (d, *J* = 1.2 Hz, 2H), 8.18 (s, 1H), 8.10 (s, 1H), 7.88 (d, *J* = 9.6 Hz, 1H), 7.82 (s, 1H), 7.58 (d, *J* = 1.2 Hz, 1H), 3.97 (s, 3H), 3.66 (s, 3H), 2.23 (s, 3H), 2.11 (s, 3H), 2.07 (s, 3H). | 516.2 |
| 54 | 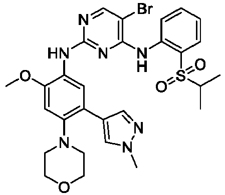 | ¹H NMR (300 MHz, DMSO-*d*₆) δ 9.40 (s, 1H), 8.48 (s, 2H), 8.8 (s, 1H), 8.04 (s, 1H), 7.82 - 7.73 (m, 2H), 7.60 (d, *J* = 4.5 Hz, 1H), 7.19 (m, 2H), 6.81 (s, 1H), 3.84 (s, 3H), 3.80 (s, 3H), 3.75 (t, *J* = 4.5 Hz, 5H), 3.44 (m, 1H), 2.85 (t, *J* = 4.5 Hz, 4H), 1.18 (d, *J* = 6.9 Hz, 6H). | 642.2, 644.2 |
| 55 | 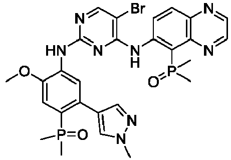 | ¹H NMR (300 MHz, DMSO-*d₆*) δ 12.77 (s, 1H), 8.89 (s, 2H), 8.82-8.78 (m, 1H), 8.41 (s, 1H), 8.40 (s, 1H), 8.00 (d, *J* = 4.5Hz, 1H), 7.92 (s, 1H), 7.65 (d, *J* = 9.6Hz, 1H), 7.48 (s, 1H), 7.39 (d, *J* = 13.5 Hz, 1H), 3.94 (s, 3H), 3.80 (s, 3H), 2.04 (d, *J* = 14.4 Hz, 6H), 1.48 (d, *J* = 13.2 Hz, 6H). | 655.1, 657.1 |
| 59 | 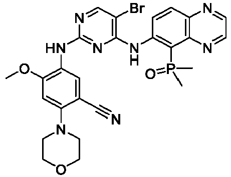 | ¹H NMR (300 MHz, CDCl₃) δ 12.69 (s, 1H), 8.95 - 8.81 (m, 2H), 8.75 (d, *J* = 1.8 Hz, 1H), 8.55 (s, 1H), 8.41 - 8.23 (m, 2H), 7.65 (s, 1H), 6.55 (s, 1H), 4.03 - 3.88 (m, 7H), 3.24 - 3.14 (m, 4H), 2.16 (d, J= 14.4 Hz, 6H). | 608.8, 610.8 |
| 60 | 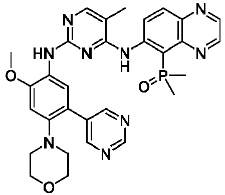 | ¹H NMR (300 MHz, DMSO-*d*₆) δ 12.51 (s, 1H), 9.16 - 9.07 (m, 1H), 8.96 (s, 1H), 8.92 (s, 2H), 8.83 (dd, *J* = 10.5, 1.8 Hz, 2H), 8.03 (s, 1H), 7.96 (d, *J* = 2.7 Hz, 2H), 7.59 (d, *J* = 9.6 Hz, 1H), 6.98 (s, 1H), 3.94 (s, 3H), 3.53 (t, *J* = 4.5 Hz, 4H), 2.79 (t, *J* = 4.5 Hz, 4H), 2.15 (s, 3H), 2.07 (s, 3H), 2.02 (s, 3H). | 597.2 |
| 61 | 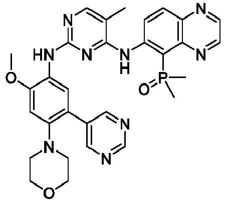 | ¹H NMR (400 MHz, DMSO-d6) δ 12.71 (s, 1H), 8.87-8.85 (m, 3H), 8.45 (s, 1H), 8.31 (s, 1H), 8.16 (d, J = 2.4 Hz, 1H), 7.84-7.82 (m, 1H), 7.70 (s, 1H), 7.55 (s, 1H), 6.85 (s, 1H), 6.43 (dd, J = 2.4, 2.4 Hz, 1H), 3.89 (s, 3H), 3.59-3.57 (m, 4H), 2.67-2.64 (m, 4H), 2.02 (d, J = 14.4 Hz, 6H). | 650.2, 652.2 |
| 62 | 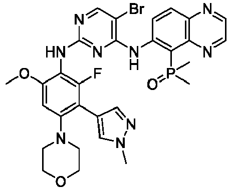 | ¹H NMR (300 MHz, CDCl₃) δ 12.60 (s, 1H), 8.94 (dd, *J* = 9.7, 4.2 Hz, 1H), 8.70 (dd, *J* = 11.4, 1.8 Hz, 2H), 8.25 (s, 1H), 8.05 (d, *J* = 1.5 Hz, 1H), 7.78 (d, *J* = 2.7 Hz, 1H), 7.61 (d, *J* = 9.9 Hz, 1H), 6.50 (d, *J* = 1.5 Hz, 1H), 6.39 (s, 1H), 3.96 (s, 3H), 3.87 (s, 3H), 3.81 (t, *J* = 4.5 Hz, 4H), 2.97 (t, *J* = 4.5 Hz, 4H), 2.12 (s, 3H), 2.08 (s, 3H). ¹⁹F NMR: (282 MHz, CDCl₃) δ -117.00. | 681.8, 683.8 |
| 63 | 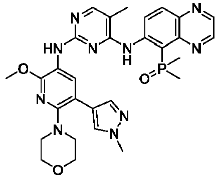 | ¹H NMR (300 MHz, CDCl₃) δ 12.47 (s, 1H), 9.26-9.22 (m, 1H), 8.74-8.71 (m, 2H), 8.45 (s, 1H), 8.00 (s, 1H), 7.71-7.66 (m, 3H), 4.04 (s, 3H), 3.83-3.81 (m, 7H), 3.16-3.13 (m, 4H), 2.31 (s, 3H), 2.14 (d, *J* = 14.4 Hz, 6H). | 601.3 |
| 65 | 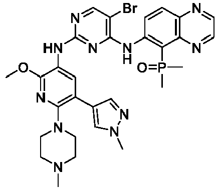 | ¹H NMR (300 MHz, DMSO-*d₆*) δ 12.69 (s, 1H), 8.85-8.75 (m, 3H), 8.63 (s, 1H), 8.28 (s, 1H), 7.96 (s, 1H), 7.82 (s, 1H), 7.77 (s, 1H), 7.56 (s, 1H), 3.88 (s, 3H), 3.80 (s, 3H), 3.14-3.04 (m, 4H), 2.47-2.41 (m, 4H), 2.25 (s, 3H), 2.02 (d, *J* = 14.4 Hz, 6H). | 678.3, 680.3 |
| 66 | 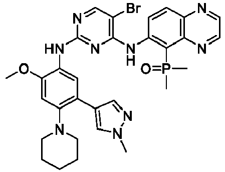 | ¹H NMR (300 MHz, DMSO-*d₆*) δ 12.68 (s, 1H), 8.85 - 8.82 (m, 3H), 8.42 (s, 1H), 8.28 (s, 1H), 8.04 (s, 1H), 7.79 (s, 1H), 7.58 (s, 2H), 6.83 (s, 1H), 3.81 (s, 3H), 3.78 (s, 3H), 2.83-2.80 (m, 4H), 2.02 (d, *J* = 14.4 Hz, 6H), 1.66-1.50 (m, 6H). | 662.1, 664.1 |
| 67 | 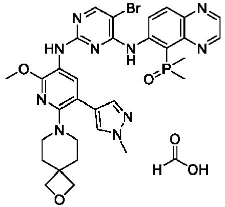 | ¹H NMR (300 MHz, DMSO-d6) δ 12.69 (s, 1H), 8.86 - 8.82 (m, 3H), 8.54 (s, 1H), 8.24 (s, 1H), 8.14 (s, 1H), 7.68 (s, 1H), 7.60 (s, 1H), 7.47 -7.45 (m, 2H), 3.85 (s, 3H), 3.82 (s, 3H), 3.81-3.71 (m, 2H), 3.59-3.54 (m, 2H), 3.32-3.20 (m, 4H), 2.02 (d, J = 14.4 Hz, 6H), 1.91-1.84 (m, 4H). | 705.2, 707.2 |
| 71 | 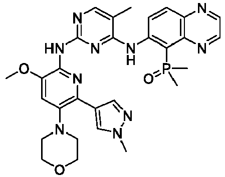 | ¹H NMR (300 MHz, DMSO-*d₆*) δ 12.50 (s, 1H), 9.26 (dd, *J* = 9.6, 4.2 Hz, 1H), 8.76 (dd, *J* = 11.7, 2.1 Hz, 2H), 8.22 (s, 1H), 8.13 (s, 1H), 8.08-7.91 (m, 2H), 7.44 (d, *J* = 9.6 Hz, 1H), 7.35 (s, 1H), 4.00-3.70 (m, 10H), 2.98-2.95 (m, 4H), 2.28-2.12 (m, 3H), 2.02 (d, *J* = 14.4 Hz, 6H). | 601.3 |
| 72 | 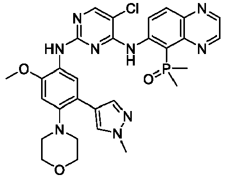 | ¹H NMR (300 MHz, DMSO-d6) δ 12.90 (s, 1H), 9.01-8.94 (m, 1H), 8.86-8.82 (m, 2H), 8.47 (s, 1H), 8.21 (s, 1H), 8.10 (s, 1H), 7.82 (s, 1H), 7.62-7.51 (m, 2H), 6.86 (s, 1H), 3.97-3.75 (m, 10H), 2.89-2.86 (m, 4H), 2.02 (d, J = 14.4 Hz, 6H). | 620.3, 622.3 |
| 73 | 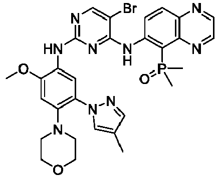 | ¹H NMR (400 MHz, DMSO-*d₆*) δ 12.71 (s, 1H), 8.87-8.81 (m, 3H), 8.41 (s, 1H), 8.30 (s, 1H), 7.95 (s, 1H), 7.77-7.70 (m, 2H), 7.32 (s, 1H), 6.81 (s, 1H), 3.88 (s, 3H), 3.6-3.59 (m, 4H), 2.67-2.64 (m, 4H), 2.10 (s, 3H), 2.02 (d, *J* = 14.4 Hz, 6H). | 664.2, 666.2 |
| 76 | 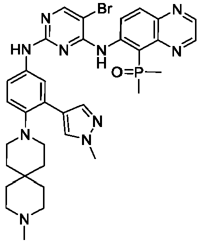 | ¹H NMR (400 MHz, DMSO-*d₆*) δ 12.69 (s, 1H), 9.35 (s, 1H), 8.97-8.85 (m, 3H), 8.34 (s, 1H), 8.23 (s, 1H), 8.02 (s, 1H), 7.78 (s, 2H), 7.65 (d, *J* = 2.8 Hz, 1H), 7.34 (s, 1H), 7.09 (d, *J* = 8.8 Hz, 1H), 3.76 (s, 3H), 2.74-2.72 (m, 4H), 2.39 (s, 4H), 2.23 (s, 3H), 2.05 (d, *J* = 14.4 Hz, 6H), 1.54 (s, 8H). | 715.3, 717.3 |
| 77 | 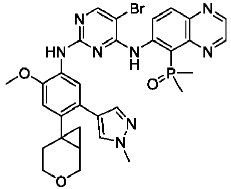 | ¹H-NMR (400 MHz, CDCl₃) δ 12.62 (s, 1H), 8.95 (s, 1H), 8.74 (s, 2H), 8.31 (s,, 1H), 8.21 (s, 1H), 7.63 (s, 2H), 7.54 (s, 1H), 7.36 (s, 1H), 6.92 (s, 1H), 3.96-3.89 (m, 8H), 3.51-3.48 (m, 1H), 3.21 (s, 1H), 2.16-2.05 (m, 7H), 1.89-1.86 (m, 1H), 1.17 (s, 1H), 0.98-0.96 (m, 1H), 0.86-0.83 (m, 1H). | 675.1, 677.1 |
| 78 | 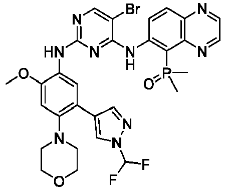 | ¹H-NMR (400 MHz, DMSO-*d₆*) δ 12.68 (s, 1H), 8.85-8.77 (m, 3H), 8.56 (s, 1H), 8.50 (s, 1H), 8.29 (s, 1H), 8.16 (s, 1H), 7.77 (dd, *J* = 59.2, 59.2 Hz, 1H), 7.71 (s, 1H), 7.59 (s, 1H), 6.92 (s, 1H), 3.85 (s, 3H), 3.77-3.74 (m, 4H), 2.88-2.86 (m, 4H), 2.02 (d, *J* = 14.4 Hz, 6H). ¹⁹F NMR (377 MHz, DMSO-*d₆*) δ -94.01. | 700.1, 702.1 |
| 81 | 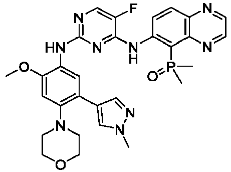 | ¹H NMR (400 MHz, CDCl₃) δ 13.15 (s, 1H), 9.40 (dd, J= 9.6, 4.0 Hz, 1H), 8.74 (dd, *J* = 15.2, 2.0 Hz, 2H), 8.26 (s, 1H), 8.08 (s, 1H), 7.90 (s, 1H), 7.82 (s, 1H), 7.56 (d, *J* = 9.6 Hz, 1H), 7.47-7.38 (m, 1H), 6.75 (s, 1H), 3.96 (s, 3H), 3.90 (s, 3H), 3.84 (s, 4H), 2.96 (s, 4H), 2.14 (d, *J* = 14.4 Hz, 6H). ¹⁹F NMR (377 MHz, CDCl₃) δ -162.16. | 604.3 |
| 83 | 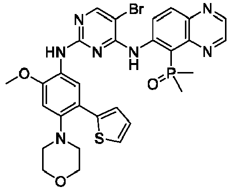 | ¹H NMR (400 MHz, CDCl₃) δ 12.57 (s, 1H), 8.86 (dd, *J* = 9.6, 4.4 Hz, 1H), 8.74 (dd, *J* = 11.6, 2.0 Hz, 2H), 8.48-8.38 (m, 1H), 8.31 (s, 1H), 7.69 (d, *J* = 9.2 Hz, 1H), 7.54 (s, 1H), 7.05 (d, *J* = 7.2 Hz, 1H), 6.99 (s, 1H), 6.75 (s, 1H), 6.50 (d, *J* = 8.8 Hz, 1H), 3.95 (s, 3H), 3.92-3.85 (m, 4H), 2.97-2.90 (m, 4H), 2.17 (s, 3H), 2.14 (s, 3H). | 666.1, 668.1 |
| 84 | 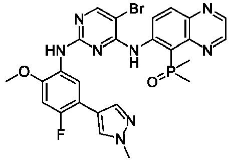 | ¹H NMR (300 MHz, CDCl₃) δ 13.00 (s, 1H), 8.89-8.79 (m, 1H), 8.79-8.71 (m, 2H), 8.23 (s, 1H), 8.18 (s, 1H), 7.72-7.61 (m, 2H), 7.26-7.24 (m, 1H), 6.76 (d, *J* = 11.9 Hz, 1H), 3.92 (s, 3H), 3.83 (s, 3H), 2.19 (s, 3H), 2.14 (s, 3H). ¹⁹F NMR (282 MHz, DMSO-*d₆*) δ -116.7. | 597.1, 599.1 |
| 85 | 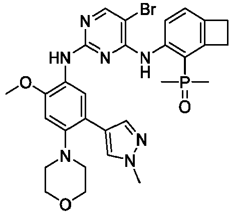 | ¹H NMR (400 MHz, CDsOD) δ 8.17 (s, 1H), 7.96-7.93 (m, 2H), 7.92 (s, 1H), 7.52 (s, 1H), 6.82 (s, 1H), 6.72-6.67 (m, 1H), 3.93 (s, 3H), 3.92 (s, 3H), 3.83-3.79 (m, 4H), 3.36-3.33 (m, 2H), 3.12-3.08 (m, 2H), 2.91-2.87 (m, 4H), 1.83 (d, *J* = 13.2 Hz, 6H). | 638.1, 640.1 |
| 86 | 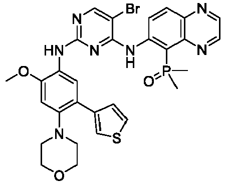 | ¹H NMR (400 MHz, DMSO-*d₆*) δ 12.67 (s, 1H), 8.84 (dd, J= 11.2, 2.0 Hz, 3H), 8.45 (s, 1H), 8.28 (s, 1H), 7.68-7.52 (m, 4H), 7.47-7.45 (m, 1H), 6.81 (s, 1H), 3.85 (s, 3H), 3.66 (t, *J* = 4.4 Hz, 4H), 2.83 (t, *J* = 4.4 Hz, 4H), 2.02 (d, *J* = 14.4 Hz, 6H). | 666.1, 668.1 |
| 87 | 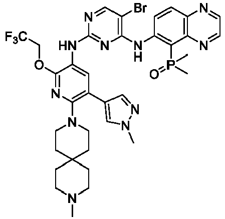 | ¹H NMR (400 MHz, DMSO-*d₆*) δ 12.66 (s, 1H), 8.84 (d, *J* = 1.6 Hz, 1H), 8.79 (d, *J* = 1.6 Hz, 1H), 8.77 (s, 1H), 8.65 (s, 1H)8.28 (s, 1H), 8.02 (s, 1H), 7.82-7.08 (m, 2H), 7.56 (s, 1H), 4.92 (q, *J* = 9.2 Hz, 2H), 3.81 (s, 3H), 3.07-3.04 (m, 4H), 2.35-2.32 (m, 4H), 2.19 (s, 3H), 2.01 (d, *J* = 14.0 Hz, 6H), 1.59-1.51 (m, 8H). | 814.3, 816.3 |
| 89 | 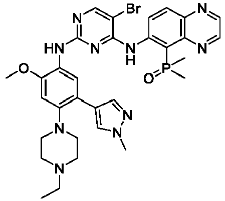 | ¹H NMR (300 MHz, D₂O) δ 8.61 (s, 1H), 8.50 (s, 1H), 8.34 (s, 1H), 7.78 (s, 1H), 7.61 (s, 1H), 7.24 (s, 1H), 6.87 (s, 1H), 6.32-6.07 (m, 2H), 5.96 (s, 1H), 3.52 (s, 3H), 3.23-2.97 (m, 4H), 2.88 (s, 3H), 2.76-2.57 (m, 2H), 2.52-2.04 (m, 4H), 1.66-1.46 (m, 6H), 1.15 (t, *J* = 7.2 Hz, 3H). | 691.2, 693.2 |
| 90 | 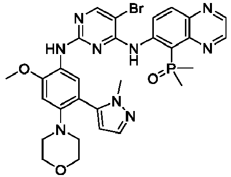 | ¹H NMR (400 MHz, CDCl₃) δ 12.48 (s, 1H), 8.82 (dd, *J* = 9.2, 3.2 Hz, 2H), 8.73 (d, *J* = 2.0 Hz, 1H), 8.29 (s, 1H), 8.18 (s, 1H), 7.77 (d, *J* = 9.6 Hz, 1H), 7.42 (s, 1H), 6.66 (s, 1H), 6.09 (s, 1H), 3.97 (s, 3H), 3.74 (s, 3H), 3.63 (t, *J* = 4.8 Hz, 4H), 2.81 (t, *J* = 4.8 Hz, 4H), 2.15 (s, 3H), 2.11 (s, 3H). | 664.2, 666.2 |
| 91 | 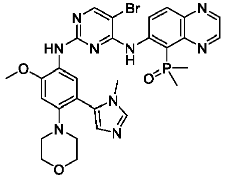 | ¹H NMR (300 MHz, CDCl₃) δ 12.50 (s, 1H), 8.87 - 8.72 (m, 3H), 8.29 (s, 1H), 8.19 (s, 1H), 7.66 (s, 2H), 7.45 (s, 1H), 6.80 (s, 1H), 6.69 (s, 1H), 3.98 (s, 3H), 3.64 (s, 7H), 2.83 (t, *J* = 4.8 Hz, 4H), 2.14 (d, *J* = 14.4 Hz, 6H). | 664.2, 666.2 |
| 92 | 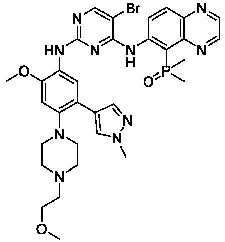 | ¹H NMR (400 MHz, CDCl₃) δ 12.59 (s, 1H), 9.01 (dd, J= 9.6, 4.4 Hz, 1H), 8.75 (d, *J* = 2.0 Hz, 1H), 8.72 (d, *J* = 2.0 Hz, 1H), 8.39 (s, 1H), 8.31 (s, 1H), 8.23 (s, 1H), 7.68-7.62 (m, 3H), 7.38 (s, 1H), 6.76 (s, 1H), 3.91 (s, 3H), 3.75 (s, 3H), 3.65 (t, *J* = 5.2 Hz, 2H), 3.39 (s, 3H), 3.07-3.03 (m, 4H), 2.85-2.76 (m, 6H), 2.17 (s, 3H), 2.13 (s, 3H). | 721.2, 723.2 |
| 94 | 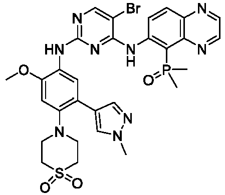 | ¹H NMR (300 MHz, CDCl₃) δ 12.72 (s, 1H), 8.93 (s, 1H), 8.81-8.73 (m, 2H), 8.29-8.22 (m, 2H), 7.63 (s, 1H), 7.57 (s, 2H), 6.74 (s, 1H), 3.94 (s, 3H), 3.79 (s, 3H), 3.44 (s, 4H), 3.16 (s, 4H), 2.18 (s, 3H), 2.13 (s, 3H). | 712.3, 714.3 |
| 95 | 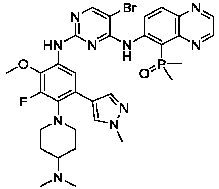 | ¹H NMR (300 MHz, DMSO-*d₆*) δ 12.70 (s, 1H), 8.89 - 8.73 (m, 4H), 8.34 (s, 1H), 8.00 (s, 1H), 7.73 (s, 1H), 7.57 (d, *J* = 9.6 Hz, 1H), 7.52-7.50 (m, 1H), 3.79(s, 3H), 3.75 (s, 3H), 3.12-3.98 (m, 4H), 2.25 (s, 7H), 2.03 (d, *J* = 14.4 Hz, 6H), 1.82-1.78 (m, 2H), 1.59-1.48 (m, 2H). | 723.2, 725.2 |
| | | ¹⁹F NMR (282 MHz, DMS O*-d₆*) δ -137.81. | |
| 96 | 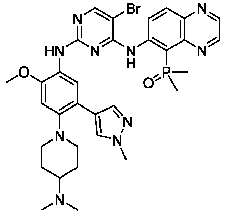 | ¹H NMR (400 MHz, DMSO-*d₆*) δ 12.68 (s, 1H), 8.90-8.79 (m, 3H), 8.41 (s, 1H), 8.28 (s, 1H), 8.20 (d, *J* = 2.8 Hz, 1H), 8.00 (s, 1H), 7.79 (s, 1H), 7.59 (s, 1H), 7.54 (s, 1H), 6.82 (s, 1H), 3.81 (s, 3H), 3.77 (s, 3H), 3.15-3.12 (m, 2H), 2.66-2.57 (m, 2H), 2.34 (d, 7H), 2.02 (d, *J* = 14.4 Hz, 6H), 1.89-1.86 (m, 2H), 1.66-1.58 (m, 2H). | 705.2, 707.2 |
| 97 | 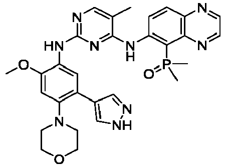 | ¹H NMR (300 MHz, CDCl₃) δ 12.55 (s, 1H), 9.21 (dd, J = 9.6, 4.2 Hz, 1H), 8.76 - 8.67 (m, 2H), 8.21 (s, 1H), 7.96-7.82 (m, 3H), 7.53 (d, J = 9.6 Hz, 1H), 6.75 (s, 1H), 3.95 (s, 3H), 3.82 (t, J = 4.5 Hz, 4H), 2.94 (t, J = 4.5 Hz, 4H), 2.31 (s, 3H), 2.15 (s, 3H), 2.10 (s, 3H). | 586.3 |
| 98 | 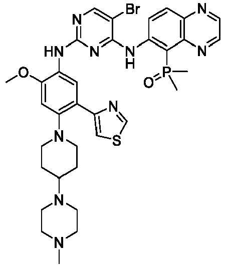 | ¹H NMR (400 MHz, CDCl₃) δ 12.57 (s, 1H), 8.86 (dd, *J* = 9.5, 4.4 Hz, 1H), 8.74-8.69 (m, 3H), 8.42 (s, 1H), 8.38 (s, 1H), 8.29 (s, 1H), 8.10 (d, *J* = 2.0 Hz, 1H), 7.52 (s, 1H), 7.43 (s, 1H), 6.73 (s, 1H), 3.94 (s, 3H), 3.23 - 3.20 (m, 2H), 3.05-2.98 (m, 8H), 2.70-2.56 (m, 6H), 2.13 (d, *J* = 14.0 Hz, 6H), 1.99-1.95 (m, 2H), 1.73-1.65 (m, 2H). | 763.3, 765.3 |
| 99 | 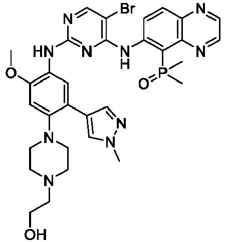 | ¹H NMR (400 MHz, DMSO-*d*₆) δ 12.66 (s, 1H), 8.84-8.80 (m, 3H), 8.47 (s, 1H), 8.34 (s, 2H), 8.26 (s, 1H), 8.13 (s, 1H), 7.82 (s, 1H), 7.65 (s, 1H), 7.54 (s, 1H), 6.86 (s, 1H), 4.25 (t, *J* = 5.6 Hz, 2H), 3.83 (s, 3H), 3.79(s, 3H), 2.90-2.88 (m, 4H), 2.84 (t, *J* = 5.6 Hz, 2H), 2.58 (s, 4H), 2.01 (d, *J* = 14.4 Hz, 6H). | 707.2, 709.2 |
| 101 | 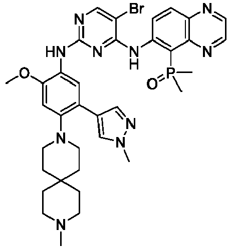 | ¹H NMR (400 MHz, CDCl₃) δ 12.59 (s, 1H), 9.01 (dd, *J* = 5.2, 4.0 Hz, 1H), 8.74 (dd, *J* = 9.6, 2.0 Hz, 2H), 8.42 (s, 1H), 8.31 (s, 1H), 8.21 (s, 1H), 7.69-7.61 (m, 3H), 7.38 (s, 1H), 6.72 (s, 1H), 3.93 (s, 3H), 3.74 (s, 3H), 3.05 (s, 3H), 2.87 (s, 5H), 2.72 (s, 3H), 2.15 (d, *J* = 14.4 Hz, 6H), 1.94 (s, 4H), 1.67 (s, 4H). | 745.3, 747.3 |
| 102 | 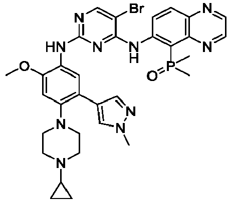 | ¹H NMR (400 MHz, CDsOD) δ 8.86-8.83 (m, 2H), 8.80 (d, *J* = 2.0 Hz, 1H), 8.27 (s, 1H), 7.95 (s, 1H), 7.87 (s, 1H), 7.54 (s, 1H), 7.51 (s, 1H), 6.84 (s, 1H), 3.93 (s, 3H), 3.71 (s, 3H), 2.92 (s, 4H), 2.80 (s, 4H), 2.17 (d, *J* = 14.4 Hz, 6H), 1.83-1.78 (m, 1H), 0.58-0.46 (m, 4H). | 703.2, 705.2 |
| 103 | 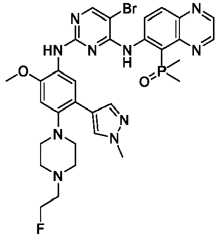 | ¹H NMR (300 MHz, CDCl₃) δ 12.60 (s, 1H), 9.01 (dd, *J* = 9.6, 4.2 Hz, 1H), 8.74 (dd, *J* = 9.9, 1.8 Hz, 2H), 8.31 (s, 1H), 8.25 (s, 1H), 7.67-7.63 (m, 3H), 7.34 (s, 1H), 6.76 (s, 1H), 4.79-4.64 (m, 2H), 3.92 (s, 3H), 3.74 (s, 3H), 3.04-2.80 (m, 10H), 2.15 (d, *J* = 14.1 Hz, 6H). | 709.2, 711.2 |
| 104 | 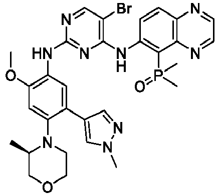 | ¹H NMR (300 MHz, CDCl₃) δ 12.63 (s, 1H), 9.00 (dd, *J* = 9.6, 4.2 Hz, 1H), 8.74 (dd, *J* = 7.8, 2.1 Hz, 2H), 8.35 (s, 1H), 8.33 (s, 1H), 7.82 (s, 1H), 7.65 (d, *J* = 9.6 Hz, 1H), 7.58 (s, 1H), 7.47 (s, 1H), 6.81 (s, 1H), 3.93 (s, 3H), 3.91-3.85 (m, 2H), 3.77-3.69 (m, 4H), 3.45-3.38 (m, 1H), 3.25-3.20 (m, 1H), 2.91-2.78 (m, 2H), 2.16 (dd, *J* = 14.1, 2.7 Hz, 6H), 0.88 (d, *J* = 6.3 Hz, 3H). | 678.2, 680.2 |
| | | [*α*]_{D}²⁵ = -20.9° (c = 0.3, MeOH) | |
| 105 | 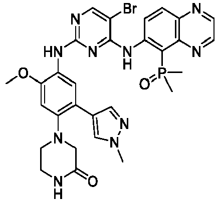 | ¹H NMR (300 MHz, CDCl₃) δ 12.59 (s, 1H), 8.97 (dd, *J* = 9.6, 4.2 Hz, 1H), 8.78-8.70 (m, 2H), 8.30 (d, *J* = 12.0 Hz, 2H), 8.10 (s, 1H), 7.64 (d, *J* = 7.8 Hz, 2H), 7.51 (s, 1H), 7.43 (s, 1H), 6.68 (s, 1H), 6.18 (s, 1H), 3.94 (s, 3H), 3.78 (s, 3H), 3.68 (s, 2H), 3.38 (s, 2H), 3.13 (t, *J* = 5.4 Hz, 2H), 2.17 (s, 3H), 2.13 (s, 3H). | 677.0, 679.0 |
| 107 | 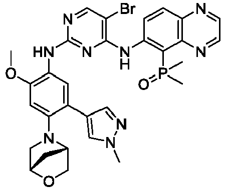 | ¹H NMR (400 MHz, CDCl₃) δ 12.66 (s, 1H), 9.03 (dd, *J* = 9.6, 4.0 Hz, 1H), 8.74 (dd, J= 17.6, 2.0 Hz, 2H), 8.27 (s, 1H), 8.04 (s, 1H), 7.65 (s, 1H), 7.47 (s, 3H), 6.59 (s, 1H), 4.54 (s, 1H), 4.14-4.01 (m, 2H), 3.92 (s, 3H), 3.81 (s, 4H), 3.13 (s, 2H), 2.14 (dd, *J* = 14.0, 2.0 | 676.2, 678.2 |
| | | Hz, 6H), 1.99-1.82 (m, 2H). [*α*]_{D}²⁵ = -1.7° (c = 0.26, MeOH) | |
| 108 | 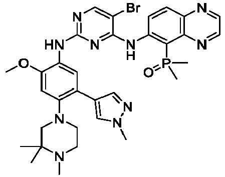 | ¹H NMR (400 MHz, CDCl₃) δ 12.60 (s, 1H), 8.99 (dd, *J* = 9.6, 4.4 Hz, 1H), 8.75 (dd, *J* = 17.6, 2.0 Hz, 2H), 8.57 (s, 1H), 8.31 (s, 1H), 8.22 (s, 1H), 7.67 (d, *J* = 9.6 Hz, 1H) 7.60 (s, 1H), 7.50 (s, 1H), 7.41 (s, 1H), 6.74 (s, 1H), 3.94 (s, 3H), 3.77 (s, 3H), 2.99-2.83 (m, 6H), 2.49 (s, 3H), 2.15 (d, *J* = 14.4 Hz, 6H), 1.28 (s, 6H). | 705.3, 707.3 |
| 109 | 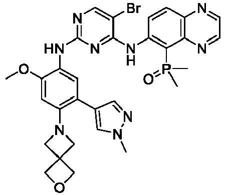 | ¹H NMR (400 MHz, CDCl₃) δ 12.57 (s, 1H), 9.00 (dd, *J* = 9.6, 4.4 Hz, 1H), 8.74 (dd, *J* = 19.6, 2.0 Hz, 2H), 8.26 (s, 1H), 7.90 (s, 1H), 7.65 (s, 1H), 7.42 (s, 1H) 7.34 (s, 1H), 7.21 (s, 1H), 6.18 (s, 1H), 4.77 (s, 4H), 3.92 (s, 3H), 3.87 (s, 3H), 3.74 (s, 4H), 2.13 (d, *J* = 14.4 Hz, 6H). | 676.3, 678.3 |
| 110 | 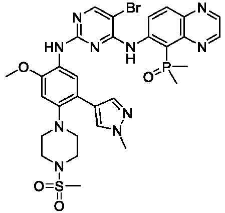 | ¹H NMR (300 MHz, DMSO-*d₆*) δ 12.68 (s, 1H), 8.94-8.77 (m, 3H), 8.46 (s, 1H), 8.29 (s, 1H), 8.04 (s, 1H), 7.82 (s, 1H), 7.62 (s, 1H) 7.55 (s, 1H), 6.90 (s, 1H), 3.83 (s, 3H), 3.78 (s, 3H), 3.30-3.27 (m, 4H), 3.00 (s, 3H), 2.98-2.95 (m, 4H), 2.02 (d, *J* = 14.7 Hz, 6H). | 740.9, 742.9 |
| 112 | 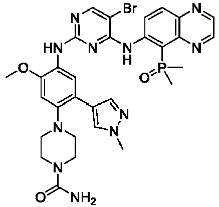 | ¹H NMR (300 MHz, DMSO-*d₆*) δ 12.68 (s, 1H), 8.88 - 8.77 (m, 3H), 8.43 (s, 1H), 8.28 (s, 1H), 8.10 (s, 1H), 7.76 (s, 1H), 7.61-7.54 (m, 2H), 6.84 (s, 1H), 6.05 (s, 2H), 3.82 (s, 3H), 3.79 (s, 3H), 3.45 (s, 4H), 2.80 (s, 4H), 2.02 (d, *J* = 14.4 Hz, 6H). | 706.1, 708.1 |
| 113 | 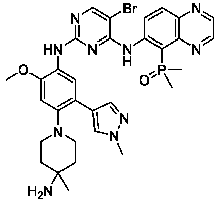 | ¹H NMR (300 MHz, CDCl₃) δ 12.58 (s, 1H), 8.99 (dd, *J* = 9.6, 4.2 Hz, 1H), 8.77 - 8.67 (m, 2H), 8.55 (s, 1H), 8.30 (s, 1H), 8.23 (s, 1H), 7.75 (s, 1H), 7.58 (s, 2H), 7.41 (s, 1H), 6.79 (s, 1H), 3.92 (s, 3H), 3.77 (s, 3H), 3.12 (d, *J* = 12.3 Hz, 2H), 2.84 (t, *J* = 9.9 Hz, 2H), 2.14 (d, *J* = 14.4 Hz, 8H), 1.90 (s, 2H), 1.52 (s, 3H). | 691.4, 693.4 |
| 114 | 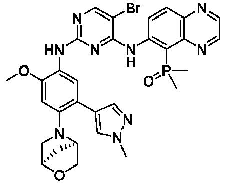 | ¹H NMR (300 MHz, CDCl₃) δ 12.68 (s, 1H), 8.91-8.83 (m, 3H), 8.41 (s, 1H), 8.25 (s, 1H), 7.72 (s, 1H), 7.62 (s, 1H), 7.50 (s, 1H), 7.32 (s, 1H), 6.65 (s, 1H), 4.48 (s, 1H), 4.29 (s, 1H), 3.91-3.88 (m, 1H), 3.83 (s, 3H), 3.79 (s, 3H), 3.71-3.68 (m, 1H), 3.10-3.07 (m, 1H), 2.79-2.76 (m, 1H), 2.02 (d, *J* = 14.4 Hz, 6H), 1.93-1.90 (m, 1H), 1.78-1.75 (m, 1H). | 676.3, 678.3 |
| | | [*α*]_{D}²⁵ = +3.9° (c = 0.45, MeOH) | |
| 115 | 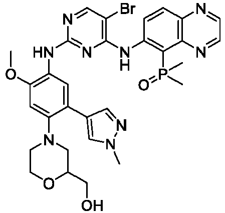 | ¹H NMR (400 MHz, DMSO-*d₆*) δ 12.67 (s, 1H), 8.85-8.76 (m, 3H), 8.43 (s, 1H), 8.29 (s, 1H), 8.07 (s, 1H), 7.77 (s, 1H), 7.62 (s, 1H), 7.56 (s, 1H), 6.85 (s, 1H), 4.72 (s, 1H), 3.87-3.67 (m, 9H), 3.49-3.45 (m, 1H), 3.38-3.34 (m, 1H), 3.04-3.01 (m, 1H), 2.90-2.87 (m, 1H), 2.76-2.69 (m, 1H), 2.57-2.53 (m, 1H), 2.02 (d, *J* = 14.4 Hz, 6H). | 694.2, 696.2 |
| 116 | 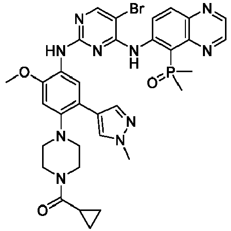 | ¹H NMR (400 MHz, DMSO-*d₆*) δ 12.68 (s, 1H), 8.85-8.77 (m, 3H), 8.43 (s, 1H), 8.29 (s, 1H), 8.14 (s, 1H), 7.77 (s, 1H), 7.64 (s, 1H), 7.55 (s, 1H), 6.87 (s, 1H), 3.86 (s, 2H), 3.82 (s, 3H), 3.80 (s, 3H), 3.66 (s, 2H), 3.89-3.83 (m, 4H), 2.02 (d, *J* = 14.4 Hz, 7H), 0.81-0.71 (m, 4H). | 731.4, 733.4 |
| 117 | 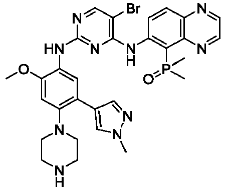 | ¹H NMR (300 MHz, DMSO-*d₆*) δ 12.69 (s, 1H), 8.87 - 8.79 (m, 3H), 8.43 (s, 1H), 8.30 (s, 1H), 8.28 (s, 1H), 8.06 (s, 1H), 7.79 (s, 1H), 7.60 (s, 1H), 7.54 (s, 1H), 6.80 (s, 1H), 3.82 (s, 3H), 3.78 (s, 3H), 2.97 (s, 4H), 2.86 (s, 4H), 2.02 (d, *J* = 14.4 Hz, 6H). | 663.4, 665.4 |
| 119 | 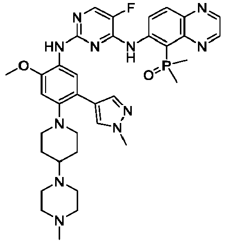 | ¹H NMR (400 MHz, CDsOD) δ 9.25 (dd, *J* = 9.6, 4.0 Hz, 1H), 8.80 (dd, *J* = 17.6, 2.0 Hz, 2H), 8.08 (d, *J* = 3.6 Hz, 1H), 8.00 (s, 2H), 7.68 (s, 1H), 7.47 (d, *J* = 9.6 Hz, 1H), 6.85 (s, 1H), 3.93 (s, 3H), 3.82 (s, 3H), 3.23-3.20 (m, 2H), 2.74-2.47 (m, 10H), 2.33 (s, 4H), 2.15 (d, *J* = 14.4 Hz, 6H), 2.02-1.99 (m, 2H), 1.72-1.60 (m, 2H). | 700.3 |
| | | ¹⁹F NMR (377 MHz, CDCl₃) δ -165.77. | |
| 120 | 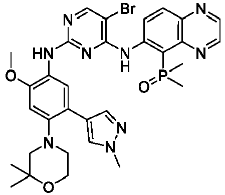 | ¹H NMR (300 MHz, DMSO-*d₆*) δ 12.68 (s, 1H), 8.86-8.76 (m, 3H), 8.44 (s, 1H), 8.28 (s, 1H), 7.89 (s, 1H), 7.70 (s, 1H), 7.59 (d, *J* = 9.0 Hz, 1H), 7.52 (s, 1H), 6.83 (s, 1H), 3.83 (s, 3H), 3.78 (s, 3H), 3.71-3.67 (m, 2H), 2.74 (s, 2H), 2.72-2.69 (m, 2H), 2.02 (d, *J* = 14.4 Hz, 6H), 1.26 (s, 6H). | 692.1, 694.1 |
| 121 | 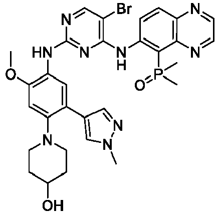 | ¹H NMR (300 MHz, CDCl₃) δ 12.64 (s, 1H), 9.00 (dd, *J* = 9.6, 3.2 Hz, 1H), 8.74 (dd, *J* = 9.0, 1.8 Hz, 2H), 8.30 (s, 1H), 8.22 (s, 1H), 7.81 (s, 1H), 7.63-7.43 (m, 3H), 6.75 (s, 1H), 3.92-3.80 (m, 7H), 3.16-3.12 (m, 2H), 2.75-2.68 (m, 2H), 2.15 (d, *J* = 14.4 Hz, 6H), 2.02-1.98 (m, 2H), 1.76-1.68 (m, 3H). | 678.4, 680.4 |
| 122 | 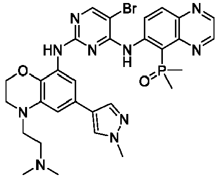 | ¹H NMR (400 MHz, CDCl₃) δ 12.60 (s, 1H), 9.05 (dd, *J* = 9.6, 4.0 Hz, 1H), 8.79 - 8.68 (m, 2H), 8.45 (s, 1H), 8.32 (s, 1H), 7.84 - 7.73 (m, 2H), 7.46 (d, *J* = 10.0 Hz, 2H), 7.34 (s, 1H), 6.58 (d, *J* = 2.0 Hz, 1H), 4.36 - 4.30 (m, 2H), 3.76 (s, 3H), 3.64 (t, *J* = 7.2 Hz, 2H), 3.46 (t*, J* = 4.4 Hz, 2H), 2.87 (t, *J* = 7.2 Hz, 2H), 2.57 (s, 6H), 2.18 (s, 3H), 2.14 (s, 3H). | 677.4, 679.4 |
| 123 | 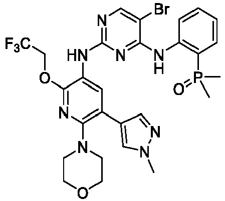 | ¹H NMR (300 MHz, DMSO-*d₆*) δ 10.97 (s, 1H), 8.60 (s, 1H), 8.19 (s, 2H), 8.05 (s, 1H), 7.86 (s, 2H), 7.55-7.43 (m, 1H), 7.04-6.92 (m, 2H), 4.92 (q, *J* = 9.0 Hz, 2H), 3.86 (s, 3H), 3.80-3.69 (m, 4H), 3.06 (t, *J* = 4.5 Hz, 4H), 1.78 (s, 3H), 1.73 (s, 3H). | 681.0, 683.0 |
| 124 | 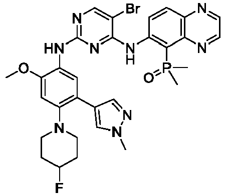 | ¹H NMR (400 MHz, CDCl₃) δ 12.62 (s, 1H), 9.01 (dd, *J* = 9.3, 1.2 Hz, 1H), 8.75 (d, *J* = 1.8 Hz, 1H), 8.72 (d, *J* = 1.8 Hz, 1H), 8.31 (s, 1H), 8.24 (s, 1H), 7.73 (s, 1H), 7.64-7.57 (m, 2H), 7.40 (s, 1H), 6.75 (s, 1H), 4.90-4.73 (m, 1H), 3.93 (s, 3H), 3.78 (s, 3H), 3.09-3.05 (m, 2H), 2.86-2.80 (s, 2H), 2.15 (d, *J* = 14.1 Hz, 6H), 2.05-1.95 (m, 4H). | 680.1, 682.1 |
| 125 | 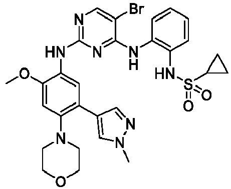 | ¹H NMR (300 MHz, DMSO-*d₆*) δ 9.41 (s, 1H), 8.40 (s, 1H), 8.20 (s, 2H), 8.06 (d, *J* = 8.1 Hz, 1H), 7.97 (s, 1H), 7.84 (s, 1H), 7.59 (s, 1H), 7.32 (dd, *J* = 7.8, 1.5 Hz, 1H), 7.02-7.00 (m, 1H), 6.79 (s, 2H), 3.85 (s, 3H), 3.80 (s, 3H), 3.75-3.72 (m, 4H), 2.85-2.82 (m, 4H), 2.62-2.56 (m, 1H), 0.99-0.90 (m, 2H), 0.87-0.84 (m, 2H). | 655.2, 657.2 |
| 126 | 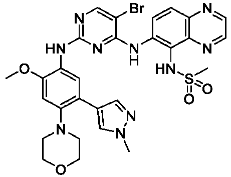 | ¹H NMR (400 MHz, DMSO-*d₆*) δ 10.01 (s, 1H), 8.96 (d, *J* = 2.0 Hz, 1H), 8.89-8.85 (m, 2H), 8.68 (s, 1H), 8.41 (s, 1H), 8.29 (s, 1H), 8.06 (s, 1H), 7.77 (s, 1H), 7.62 (d, *J* = 6.4 Hz, 1H), 7.46 (s, 1H), 6.83 (s, 1H), 3.81 (s, 3H), 3.80 (s, 3H), 3.78-3.73 (m, 4H), 3.01 (s, 3H), 2.88-2.83 (m, 4H). | 681.0, 683.0 |
| 127 | 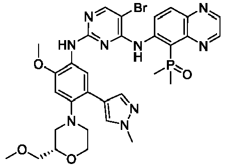 | ¹H-NMR (400 MHz, CDCl₃) δ 12.68 (s, 1H), 8.98 (dd, *J* = 9.6, 4.4 Hz, 1H), 8.74 (dd, *J* = 12.8, 2.0 Hz, 2H), 8.29 (s, 1H), 8.22 (s, 1H), 7.76 (s, 1H), 7.65-7.58 (m, 1H), 7.55 (s, 2H), 6.72 (s, 1H), 4.02-3.95 (m, 1H), 3.94 (s, 3H), 3.91-3.84 (m, 1H), 3.83-3.73 (m, 4H), 3.53-3.40 (m, 2H), 3.41 (s, 3H), 3.05-2.91 (m, 2H), 2.86-2.75 (m, 1H), 2.67 (t, *J* = 10.8 Hz, 1H), 2.15 (d, *J* = 14.0 Hz, 6H). | 708.1, 710 |
| | | [*α*]_{D}²⁵ = +32.5° (c = 0.33, MeOH) | |
| 128 | 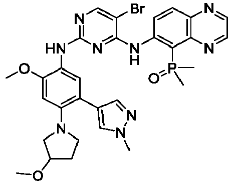 | ¹H NMR (300 MHz, DMSO-*d₆*) δ 12.68 (s, 1H), 8.87-8.77 (m, 3H), 8.41 (s, 1H), 8.26 (s, 1H), 7.82 (s, 1H), 7.60 (s, 2H), 7.43 (s, 1H), 6.72 (s, 1H), 4.02-3.98 (m, 1H), 3.80 (s, 3H), 3.77 (s, 3H), 3.23 (m, 3H), 3.16-3.08 (m, 2H), 3.02-2.94 (m, 2H), 2.17-2.08 (m, 1H), 2.04 (s, 3H), 1.99 (s, 3H), 1.88-1.83 (m, 1H). | 678.2, 680.2 |
| 129 | 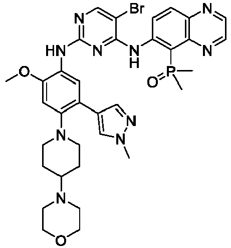 | ¹H NMR (300 MHz, CDCl₃) δ 12.59 (s, 1H), 8.99 (dd, *J* = 9.6, 4.2 Hz, 1H), 8.75 (d, *J* = 1.8 Hz, 1H), 8.72 (d, *J* = 2.1 Hz, 1H), 8.30 (s, 1H), 8.24 (s, 1H), 7.82 (s, 1H), 7.61 (d, *J* = 9.3 Hz, 1H), 7.49 (s, 1H), 7.37 (s, 1H), 6.70 (s, 1H), 3.95-3.92 (m, 7H), 3.83 (s, 3H), 3.29-3.25 (m, 2H), 2.86 (s, 4H), 2.66-2.59 (m, 4H), 2.15 (d, *J* = 14.1 Hz, 6H), 2.09-2.05 (m, 3H). | 747.4, 749.4 |
| 130 | 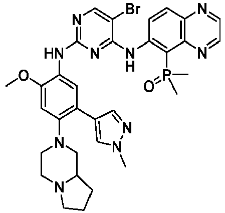 | ¹H NMR (400 MHz, DMSO-*d₆*) δ 12.68 (s, 1H), 8.85-8.77 (m, 3H), 8.43 (s, 1H), 8.28 (s, 1H), 8.00 (s, 1H), 7.80 (s, 1H), 7.58 (s, 2H), 6.87 (s, 1H), 3.81 (s, 3H), 3.76 (s, 3H), 3.16-3.13 (m, 1H), 3.05-2.96 (m, 3H), 2.78-2.72 (m, 1H), 2.59-2.54 (m, 1H), 2.35-2.29 (m, 1H), 2.23-2.13 (m, 2H), 2.02 (d, *J* = 14.4 Hz, 6H), 1.79-1.68 (m, 3H), 1.41-1.32 (m, 1H). | 703.2, 705.2 |
| 131 | 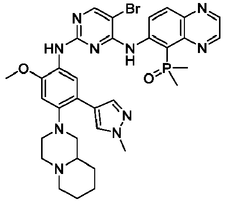 | ¹H NMR (400 MHz, CDsOD) δ 8.89-8.74 (m, 3H), 8.49 (s, 1H), 8.28 (s, 1H), 7.99 (s, 1H), 7.81 (s, 1H), 7.53-7.50 (m, 2H), 6.85 (s, 1H), 3.95 (s, 3H), 3.71 (s, 3H), 3.26- 3.12 (m, 4H), 3.00 - 2.88 (m, 3H), 2.76-2.71 (m, 2H), 2.17 (d, *J* = 14.4 Hz, 6H), 1.92-1.89 (m, 2H), 1.77-1.74 (m, 2H), 1.59-1.45 (m, 2H). | 717.3, 719.3 |
| 132 | 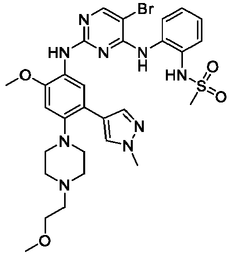 | ¹H NMR (300 MHz, CDCl₃) δ 8.21 (s, 1H), 7.91 (s, 1H), 7.75 (s, 1H), 7.68-7.65 (m, 1H), 7.53 (s, 1H), 7.41 (s, 1H), 7.33 (s, 1H), 7.13-7.09 (m, 2H), 6.69 (s, 1H), 3.91 (s, 3H), 3.87 (s, 3H), 3.68 (s, 2H), 3.39 (s, 3H), 3.03 (s, 5H), 2.90 (s, 3H), 2.84 (s, 5H). | 686.1, 688.1 |
| 134 | 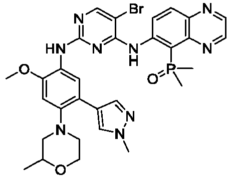 | ¹H NMR (300 MHz, CDCl₃) δ 12.60 (s, 1H), 9.00 (dd, *J* = 9.6, 4.2 Hz, 1H), 8.75 (d, *J* = 1.8 Hz, 1H), 8.72 (d, *J* = 1.8 Hz, 1H), 8.30 (s, 1H), 8.24 (d, *J* = 2.7 Hz, 1H), 7.75 (s, 1H), 7.64-7.58 (m, 2H), 7.44 (s, 1H), 6.72 (s, 1H), 3.94 (s, 4H), 3.83-3.72 (m, 5H), 3.01-2.92 (m, 2H), 2.77 (m, 1H), 2.56-2.44 (m, 1H), 2.17 (s, 3H), 2.13 (s, 3H), 1.20 (d, *J* = 6.3 Hz, 3H). | 677.9, 679.9 |
| 135 | 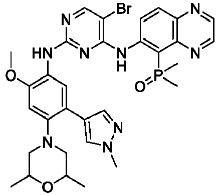 | ¹H NMR (300 MHz, DMSO-*d₆*) δ 12.67 (s, 1H), 8.87-8.80 (m, 2H), 8.76 (s, 1H), 8.45 (s, 1H), 8.28 (s, 1H), 7.89 (s, 1H), 7.69 (s, 1H), 7.59-7.53 (m, 2H), 6.84 (s, 1H), 4.07-4.02 (m, 2H), 3.83 (s, 3H), 3.77 (s, 3H), 3.32 (s, 2H), 2.98-2.93 (m, 2H), 2.02 (d*, J* = 14.4 Hz, 6H), 1.18 (d, *J* = 6.3 Hz, 6H). | 692.1, 694.1 |
| 136 | 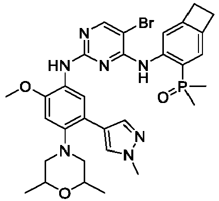 | ¹H NMR (300 MHz, CDCl₃) δ 10.70 (s, 1H), 8.29 (s, 1H), 8.18 (s, 1H), 8.02 (s, 1H), 7.76 - 7.50 (m, 3H), 7.04 (d, *J* = 12.9 Hz, 1H), 6.71 (s, 1H), 3.93-3.70 (m, 8H), 3.06 (s, 2H), 2.94 (s, 2H), 2.80 (s, 2H), 2.41 (s, 2H), 1.88 (s, 3H), 1.84 (s, 3H), 1.22 (s, 3H), 1.20 (s, 3H). | 665.9, 667.9 |
| 137 | 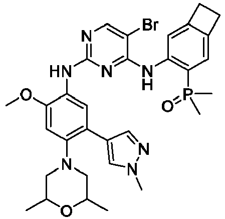 | ¹H NMR (400 MHz, CDCl₃) δ 10.56 (s, 1H), 8.27 (s, 1H), 8.20 (s, 1H), 8.09 (s, 1H), 7.59-7.57 (m, 3H), 7.01 (d, *J* = 13.2 Hz, 1H), 6.69 (s, 1H), 4.15-4.11 (m, 2H), 3.93 (s, 3H), 3.89 (s, 3H), 3.06 (t, *J* = 4.4 Hz, 2H), 2.96 (dd, *J* = 11.2, 3.2 Hz, 2H), 2.86 (s, 2H), 2.50 (dd, *J* = 11.2, 5.6 Hz, 2H), 1.83 (d, *J* = 13.2 Hz, 6H), 1.25 (d, *J* = 6.4 Hz, 6H). | 666.2, 668.2 |
| 138 | 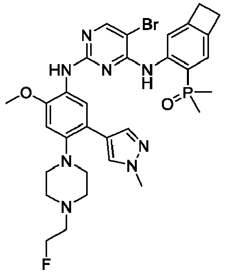 | ¹H NMR (400 MHz, CDCl₃) δ 10.43 (s, 1H), 8.34 (s, 1H), 8.22 (s, 1H), 8.02 (d, *J* = 3.6 Hz, 1H), 7.72 (s, 1H), 7.53-7.51 (m, 2H), 7.04 (d, *J* = 13.6 Hz, 1H), 6.75 (s, 1H), 4.88 (s, 1H), 4.76 (s, 1H), 3.92 (s, 3H), 3.88 (s, 3H), 3.14 - 3.05 (m, 8H), 3.00 (s, 4H), 2.86 (s, 2H), 1.86 (d, *J=* 13.2 Hz, 6H). | 683.2, 685.2 |
| 139 | 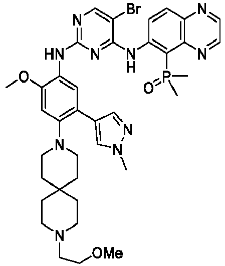 | ¹H NMR (300 MHz, CDCl₃) δ 12.60 (s, 1H), 9.02 (dd, *J* = 9.6, 4.2 Hz, 1H), 8.79 - 8.69 (m, 2H), 8.31 (s, 1H), 8.21 (s, 1H), 7.66 (s, 3H), 7.33 (s, 1H), 6.73 (s, 1H), 3.93 (s, 3H), 3.75 (s, 5H), 3.38 (s, 3H), 2.95-2.84 (m, 10H), 2.15 (d, *J* = 14.4 Hz, 6H), 1.87-1.83 (m, 4H), 1.65-1.62 (m, 4H). | 789.4, 791.4 |
| 140 | 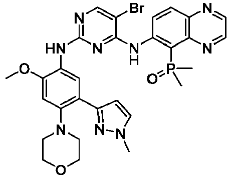 | ¹H NMR (400 MHz, CDCl₃): δ 12.67 (s, 1H), 9.05 (dd, *J* = 9.2, 4.0 Hz, 1H), 8.75 (d, *J* = 1.6 Hz, 1H), 8.70 (d, *J* = 2.0 Hz, 1H), 8.47 (s, 1H), 8.27 (s, 1H), 7.49 (s, 2H), 7.30 (d, *J* = 2.0 Hz, 1H), 6.96 (s, 1H), 6.70 (s, 1H), 3.94 (s, 3H), 3.83-3.81 (m, 4H), 3.54 (s, 3H), 2.95 (s, 4H), 2.12 (d, *J* = 14.4 Hz, 6H). | 664.2, 666.2 |
| 141 | 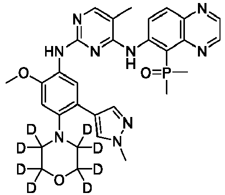 | ¹H NMR (300 MHz, CDCl₃): δ 12.35 (s, 1H), 9.32 (dd, *J* = 9.6, 4.5 Hz, 1H), 8.72 (d, *J* = 1.8 Hz, 1H), 8.69 (d, *J* = 1.8 Hz, 1H), 8.36 (s, 1H), 8.05 (s, 1H), 7.80 (s, 1H), 7.71 (s, 1H), 7.59 (d, *J* = 9.6 Hz, 1H), 6.73 (s, 1H), 3.95 (s, 3H), 3.79 (s, 3H), 2.30 (s, 3H), 2.13 (d, *J* = 14.1 Hz, 6H). | 608.3 |
| 142 | 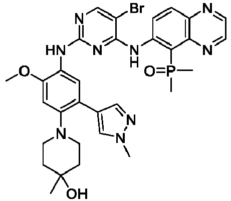 | ¹H NMR (300 MHz, CDCl₃) δ 12.61 (s, 1H), 9.02 (dd, *J* = 9.6, 4.2 Hz, 1H), 8.79-8.66 (m, 2H), 8.30 (s, 1H), 8.21 (s, 1H), 7.71 (s, 1H), 7.63 (s, 2H), 7.38 (s, 1H), 6.80 (s, 1H), 3.93 (s, 3H), 3.76 (s, 3H), 3.06-2.82 (m, 4H), 2.17 (s, 3H), 2.12 (s, 3H), 1.84-1.67 (m, 5H), 1.35 (s, 3H). | 692.2, 694.2 |
| 143 | 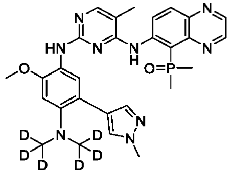 | ¹H-NMR (300 MHz, DMSO-*d₆*) δ 12.50 (s, 1H), 9.23-9.12 (m, 1H), 8.81 (dd, *J* = 13.8, 1.8 Hz, 2H), 8.17 (s, 1H), 7.98 (d, *J* = 10.2 Hz, 2H), 7.89 (s, 1H), 7.77 (s, 1H), 7.69 (s, 1H), 7.55 (d, *J* = 9.6 Hz, 1H), 6.84 (s, 1H), 3.83 (s, 3H), 3.76 (s, 3H), 2.14 (s, 3H), 2.03 (d, *J* = 14.4 Hz, 6H). | 564.3 |
| 145 | 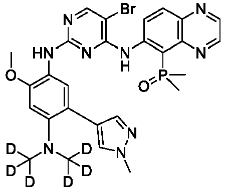 | ¹H-NMR (400 MHz, DMSO-*d₆*) δ 12.70 (s, 1H), 8.85-8.76 (m, 3H), 8.46 (s, 1H), 8.28 (s, 1H), 7.97 (s, 1H), 7.70 (s, 1H), 7.58 (s, 2H), 6.85 (s, 1H), 3.82 (s, 3H), 3.77 (s, 3H), 2.02 (d, *J* = 14.4 Hz, 6H). | 628.2, 630.2 |
| 146 | 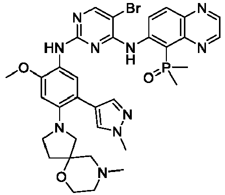 | ¹H NMR (300 MHz, CD₃OD) δ 8.88 (dd, *J* = 9.6, 1.2 Hz, 1H), 8.83 (d, *J* = 2.1 Hz, 1H), 8.79 (d, *J* = 1.8 Hz, 1H), 8.30 (s, 1H), 8.25 (s, 1H), 7.84 (s, 1H), 7.75 (s, 1H), 7.55 (d, *J* = 9.6 Hz, 1H), 7.42 (s, 1H), 6.79 (s, 1H), 3.92 (s, 3H), 3.88-3.82 (m, 1H), 3.80-3.74 (m, 1H), 3.72 (s, 3H), 3.24-3.19 (m, 2H), 3.12-3.00 (m, 2H), 2.80-2.76 (m, 1H), 2.70-2.66 (m, 3H), 2.49 (s, 3H), 2.18 (s, 3H), 2.14 (s, 3H), 2.11-1.98 (m, 2H). | 733.2, 735.2 |
| 149 | 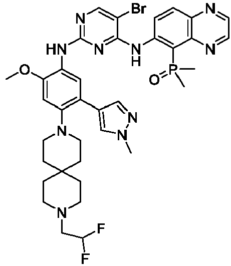 | ¹H NMR (300 MHz, CDsOD) δ 8.87-8.82 (m, 2H), 8.79 (d, *J* = 1.8 Hz, 1H), 8.26 (s, 1H), 7.90 (s, 1H), 7.85 (s, 1H), 7.56-7.50 (m, 2H), 6.88 (s, 1H), 6.22-5.79 (m, 1H), 3.92 (s, 3H), 3.69 (s, 3H), 2.91-2.85 (m, 4H), 2.83-2.72 (m, 2H), 2.66-2.58 (m, 4H), 2.19 (s, 3H), 2.14 (s, 3H), 1.69-1.61 (m, 8H). | 795.0, 797.0 |
| | | ¹⁹F NMR (300 MHz, CDsOD) δ -120.23. | |
| 151 | 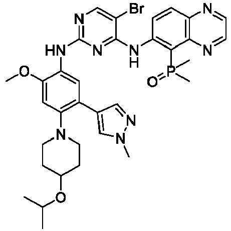 | ¹H-NMR (400 MHz, DMSO-*d₆*) δ 12.67 (s, 1H), 8.84-8.81 (m, 3H), 8.38 (s, 1H), 8.27 (s, 1H), 8.01 (s, 1H), 7.75 (s, 1H), 7.66-7.45 (m, 2H), 6.82 (s, 1H), 3.80 (s, 3H), 3.77 (s, 3H), 3.76-3.70 (m, 1H), 3.55-3.45 (m, 1H), 3.07-2.97 (m, 2H), 2.76-2.64 (m, 2H), 2.01 (d, *J* = 14.4 Hz, 6H), 1.94-1.84 (m, 2H), 1.63-1.50 (m, 2H), 1.11 (d, *J* = 6.0 Hz, 6H). | 720.2, 722.2 |
| 152 | 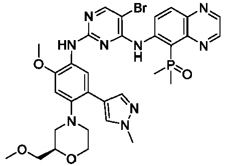 | ¹H NMR (300 MHz, CDsOD) δ 8.91-8.88 (m, 2H), 8.75 (s, 1H), 8.30 (s, 1H), 7.98 (s, 1H), 7.75 (s, 1H), 7.65-7.61 (m, 2H), 6.89 (s, 1H), 3.97-3.80 (m, 8H), 3.52-3.42 (m, 2H), 3.37 (s, 3H), 3.12-2.99 (m, 2H), 2.87-2.76 (m, 2H), 2.67-2.59 (m, 1H), 2.20 (s, 3H), 2.16 (s, 3H). [*α*]_{D}²⁵ =-19.9° (c= 0.34, MeOH) | 708.2, 710.2 |
| 154 | 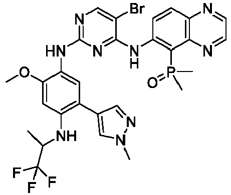 | ¹H NMR (400 MHz, CDCl₃) δ 12.67 (s, 1H), 8.93 (dd, *J* = 9.6, 4.0 Hz, 1H), 8.75 (d, *J* = 1.6 Hz, 1H), 8.70 (d, *J* = 2.0 Hz, 1H), 8.21 (s, 1H), 7.85 (s, 1H), 7.59 (s, 1H), 7.40 (d, *J* = 4.4 Hz, 2H), 6.37 (s, 1H), 4.00 (s, 2H), 3.90 (s, 3H), 3.89 (s, 3H), 2.11 (dd, *J* = 14.4, 2.0 Hz, 6H), 1.36 (d, *J* = 6.4 Hz, 3H). | 690.2, 692.2 |
| 156 | 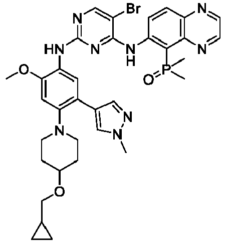 | ¹H NMR (300 MHz, CDCl₃) δ 12.64 (s, 1H), 9.00 (dd, *J* = 9.6, 4.2 Hz, 1H), 8.78-8.68 (m, 2H), 8.29 (s, 1H), 8.22 (s, 1H), 7.89 (s, 1H), 7.59 (d, *J* = 9.6 Hz, 1H), 7.43 (s, 2H), 6.74 (s, 1H), 3.92 (s, 3H), 3.82 (s, 3H), 3.50-3.38 (m, 1H), 3.35 (d, *J* = 6.9 Hz, 2H), 3.16-3.12 (m, 2H), 2.67 (t, *J* = 10.8 Hz, 2H), 2.14 (d, *J* = 14.4 Hz, 6H), 2.03-1.99 (m, 2H), 1.75-1.66 (m, 2H), 1.14-1.07 (m, 1H), 0.62 - 0.56 (m, 2H), 0.27-0.22 (m, 2H). | 732.3, 734.3 |
| 157 | 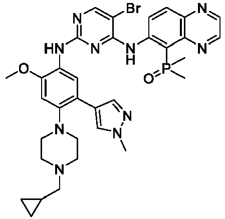 | ¹H NMR (400 MHz, CDsOD) δ 8.84-8.80 (m, 2H), 8.77 (d, *J* = 2.0 Hz, 1H), 8.48 (s, 1H), 8.26 (s, 1H), 7.98 (s, 1H), 7.79 (s, 1H), 7.51-7.48 (m, 2H), 6.85 (s, 1H), 3.93 (s, 3H), 3.68 (s, 3H), 3.20 (s, 3H), 3.11 (s, 5H), 2.88 (d, *J* = 6.8 Hz, 2H), 2.14 (d, *J* = 14.4 Hz, 6H), 1.11-1.06 (m, 1H), 0.76-0.71 (m, 2H), 0.40-0.36 (m, 2H). | 716.9, 718.9 |
| 158 | 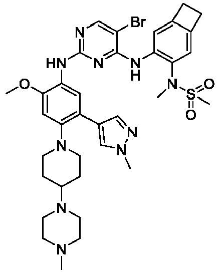 | ¹H NMR (300 MHz, CDCl₃) δ 8.44 (s, 1H), 8.28 (d, *J* = 8.1 Hz, 2H), 8.19 (s, 1H), 7.99 (s, 1H), 7.67 (d, *J* = 12.9 Hz, 2H), 7.37 (s, 1H), 7.07 (s, 1H), 6.69 (s, 1H), 3.91 (s, 6H), 3.31 (s, 3H), 3.21 (s, 2H), 3.05 (d, *J* = 7.9 Hz, 6H), 2.96 (s, 6H), 2.87 (s, 1H), 2.76 (s, 1H), 2.62 (s, 3H), 2.55 (s, 4H), 2.00 (d, *J* = 11.8 Hz, 2H), 1.78 (d, *J* = 12.4 Hz, 2H). | 765.2, 767.2 |
| 159 | 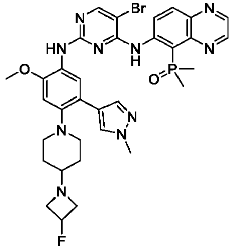 | ¹H NMR (300 MHz, CDsOD) δ 8.87-8.81 (m, 2H), 8.79 (d*, J* = 2.1 Hz, 1H), 8.27 (s, 1H), 7.95 (s, 1H), 7.81 (s, 1H), 7.50 (d, *J* = 9.6 Hz, 1H), 7.46 (s, 1H), 6.85 (s, 1H), 5.42 (s, 0.5H), 5.24 (s, 0.5H), 4.26-4.16 (m, 2H), 3.98-3.87 (m, 5H), 3.70 (s, 3H), 3.21-3.18 (m, 2H), 2.93-2.84 (m, 1H), 2.74-2.66 (m, 2H), 2.17 (d, *J* = 14.4 Hz, 6H), 2.01-1.97 (m, 2H), 1.61-1.49 (m, 2H). | 735.1, 737.1 |
| | | ¹⁹F NMR (282 MHz, CDsOD) δ -181.60. | |
| 160 | 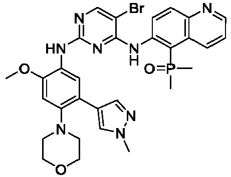 | ¹H NMR (400 MHz, CDCl₃) δ 12.40 (s, 1H), 8.84 (d, *J* = 4.0 Hz, 1H), 8.73 (dd, *J* = 9.6, 4.4 Hz, 1H), 8.23 (s, 1H), 8.15 (s, 1H), 8.08 (d, *J* = 8.8 Hz, 1H), 7.77 (s, 1H), 7.68 (s, 1H), 7.56 (s, 1H), 7.44 (dd, *J* = 8.8, 4.4 Hz, 1H), 6.69 (s, 1H), 3.91 (s, 3H), 3.83-3.75 (m, 4H), 3.71 (s, 3H), 2.89 (t, *J* = 4.6 Hz, 4H), 2.14 (d, *J* = 12.8 Hz, 6H). | 663.4, 665.4 |
| 161 | 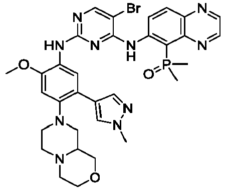 | ¹H NMR (300 MHz, CDCl₃) δ 12.59 (s, 1H), 8.99 (dd, *J* = 9.6, 4.2 Hz, 1H), 8.78-8.67 (m, 2H), 8.29 (s, 1H), 8.23 (s, 1H), 7.75-7.54 (m, 3H), 7.38 (s, 1H), 6.74 (s, 1H), 3.96-3.70(m, 9H), 3.43 (s, 1H), 3.16-2.77 (m, 5H), 2.57 (s, 4H), 2.17 (s, 3H), 2.13 (s, 3H). | 719.2, 721.2 |
| 162 | 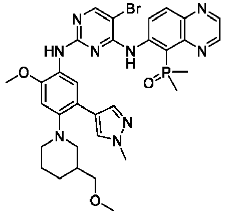 | ¹H NMR (300 MHz, CDsOD) δ 8.84-8.77 (m, 3H), 8.24 (s, 1H), 7.93 (s, 1H), 7.83 (s, 1H), 7.50 (d, *J* = 9.6 Hz, 1H), 7.44 (s, 1H), 6.81 (s, 1H), 3.91 (s, 3H), 3.70 (s, 3H), 3.31-3.24 (m, 5H), 3.19-3.11 (m, 1H), 3.06-2.97 (m, 1H), 2.61-2.49 (m, 1H), 2.41-2.29 (m, 1H), 2.18 (s, 3H), 2.13 (s, 3H), 2.09-1.99 (m, 1H), 1.87-1.66 (m, 3H), 1.20-1.04 (m, 1H). | 705.9, 707.9 |
| 163 | 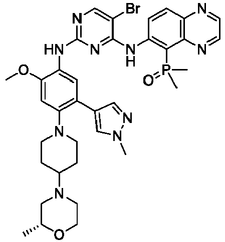 | ¹H NMR (300 MHz, CDsOD) δ 8.89-8.82 (m, 2H), 8.79 (d, *J* = 1.8 Hz, 1H), 8.40 (s, 1H), 8.27 (s, 1H), 7.95 (s, 1H), 7.82 (s, 1H), 7.52 (d, *J* = 10.8 Hz, 2H), 6.85 (s, 1H), 4.05-4.00 (m, 1H), 3.93 (s, 3H), 3.83-3.72 (m, 2H), 3.70 (s, 3H), 3.26-3.22 (m, 4H), 2.73-2.66 (m, 4H), 2.41-2.36 (m, 1H), 2.19 (s, 3H), 2.17-2.14 (m, 5H), 1.80-1.69 (m, 2H), 1.23 (d, *J* = 6.3 Hz, 3H). | 761.3, 763.3 |
| | | [*α*]_{D}²⁵ = +6.4°(c = 0.31, MeOH) | |
| 165 | 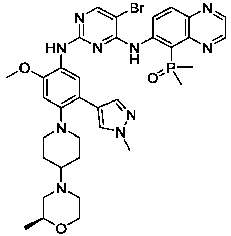 | ¹H NMR (300 MHz, CDsOD) δ 8.89-8.82 (m, 2H), 8.79 (d, *J* = 1.8 Hz, 1H), 8.40 (s, 1H), 8.27 (s, 1H), 7.95 (s, 1H), 7.82 (s, 1H), 7.55-7.51 (m, 2H), 6.85 (s, 1H), 4.05-4.00 (m, 1H), 3.93 (s, 3H), 3.83-3.72 (m, 2H), 3.70 (s, 3H), 3.29-3.20 (m, 4H), 2.74-2.67 (m, 4H), 2.40-2.33 (m, 1H), 2.19 (s, 3H), 2.17-2.14 (m, 5H), 1.80-1.69 (m, 2H), 1.23 (d, .7= 6.3 Hz, 3H). | 761.3, 763.3 |
| | | [*α*]_{D}²⁵ = -5.6° (c = 0.36, MeOH) | |
| 166 | 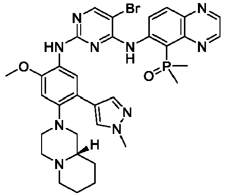 | ¹H NMR (300 MHz, CDsOD) δ 8.86-8.78 (m, 3H), 8.50 (s, 1H), 8.28 (s, 1H), 7.99 (s, 1H), 7.80 (s, 1H), 7.54-7.50 (m, 2H), 6.85 (s, 1H), 3.95 (s, 3H), 3.70 (s, 3H), 3.27-3.10 (m, 4H), 3.04-2.83 (m, 3H), 2.78-2.68 (m, 2H), 2.17 (d, *J* = 14.4 Hz, 6H), 1.92-1.88 (m, 2H), 1.78-1.73 (m, 2H), 1.59-1.44 (m, 2H). | 717.2, 719.2 |
| | | [*α*]_{D}²⁵ = -19.4° (c = 0.27, MeOH) | |
| 167 | 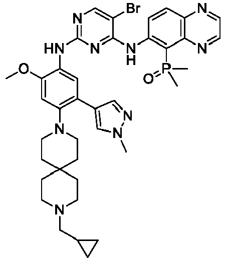 | ¹H NMR (400 MHz, CDCl₃) δ 12.58 (s, 1H), 9.00 (dd, *J* = 9.6, 4.0 Hz, 1H), 8.73 (d, *J* = 2.0 Hz, 1H), 8.70 (d, *J* = 1.6 Hz, 1H), 8.29 (s, 1H), 8.19 (s, 1H), 7.65-7.63 (m, 3H), 7.31 (s, 1H), 6.71 (s, 1H), 3.91 (s, 3H), 3.72 (s, 3H), 2.93 (brs, 3H), 2.84 (t, *J* = 5.6 Hz, 5H), 2.65 (d, *J* = 6.8 Hz, 2H), 2.12 (d, *J* = 14.4 Hz, 6H), 1.86 (brs, 4H), 1.63 (t, *J* = 5.2 Hz, 4H), 1.10 (brs, 1H), 0.75-0.65 (m, 2H), 0.30-0.26 (m, 2H). | 785.4, 787.4 |
| 169 | 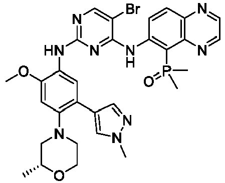 | ¹H NMR (300 MHz, CDCl₃) δ 12.64 (s, 1H), 9.00 (dd, *J* = 9.6, 4.2 Hz, 1H), 8.79-8.67 (m, 2H), 8.31 (s, 1H), 8.24 (s, 1H), 7.74 (s, 1H), 7.65-7.59 (m, 2H), 7.44 (s, 1H), 6.72 (s, 1H), 3.94 (s, 3H), 3.91 (s, 1H), 3.82-3.75 (m, 5H), 3.01-2.93 (s, 2H), 2.82-2.74 (m, 1H), 2.57-2.45 (m, 1H), 2.15 (d, *J* = 14.4 Hz, 6H), 1.20 (d, *J* = 6.3 Hz, 3H). | 678.3, 680.3 |
| | | [*α*]_{D}²⁵ = +18.2° (c= 0.26, MeOH) | |
| 170 | 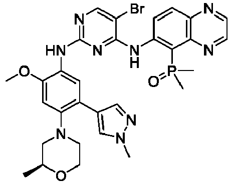 | [*α*]_{D}²⁵ = -16.6° (c = 0.29, MeOH) | 678.3, 680.3 |
| | | ¹H NMR (400 MHz, CDCl₃) δ 12.68 (s, 1H), 8.96 (dd, *J* = 9.6, 4.4 Hz, 1H), 8.73 (d, *J* = 2.0 Hz, 1H), 8.70 (d, *J* = 2.0 Hz, 1H), 8.26 (s, 1H), 8.18 (s, 1H), 7.73 (s, 1H), 7.61-7.58 (m, 3H), 6.70 (s, 1H), 3.92 (s, 3H), 3.89 (s, 1H), 3.79-3.73 (m, 5H), 2.99-2.91 (m, 2H), 2.79-2.73 (m, 1H), 2.56-2.44 (m, 1H), 2.17 (s, 3H), 2.13 (s, 3H), 1.20 (d, *J* = 6.4 Hz, 3H). | |
| 172 | 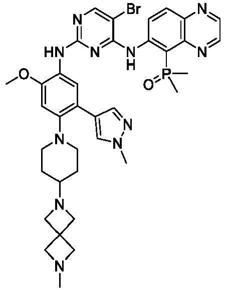 | ¹H NMR (300 MHz, CDsOD) δ 8.89-8.81 (m, 2H), 8.78 (d, *J* = 1.8 Hz, 1H), 8.49 (s, 1H), 8.27 (s, 1H), 7.94 (s, 1H), 7.82 (s, 1H), 7.52 (d, *J* = 9.6 Hz, 1H), 7.45 (s, 1H), 6.84 (s, 1H), 4.09 (s, 4H), 3.92 (s, 3H), 3.69 (s, 3H), 3.63 (s, 4H), 3.16-3.12 (m, 2H), 2.78 (s, 3H), 2.69-2.62 (m, 2H), 2.44-2.37 (m, 1H), 2.16 (d, *J* = 14.4 Hz, 6H), 1.88-1.84 (m, 2H), 1.51-1.35 (m, 2H). | 772.4, 774.4 |
| 173 | 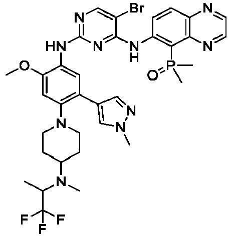 | ¹H NMR (300 MHz, CDsOD) δ 8.88-8.79 (m, 3H), 8.27 (s, 1H), 7.91 (s, 1H), 7.85 (s, 1H), 7.54 (s, 2H), 6.85 (s, 1H), 3.92 (s, 3H), 3.70 (s, 3H), 3.61-3.58 (m, 1H), 3.21-3.17 (m, 2H), 2.69-2.62 (m, 3H), 2.46 (s, 3H), 2.17 (d, *J* = 14.4 Hz, 6H), 1.97-1.91 (m, 1H), 1.82-1.65 (m, 3H), 1.30 (d, *J* = 6.9 Hz, 3H). ¹⁹F NMR (282 MHz, MeOD) δ -76.05. | 787.4, 789.4 |
| 174 | 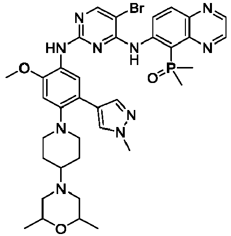 | ¹H NMR (300 MHz, DMSO-*d₆*) δ 12.68 (s, 1H), 8.85-8.81 (m, 3H), 8.39 (s, 1H), 8.27 (s, 1H), 7.95 (s, 1H), 7.56 (s, 1H), 7.56 (s, 2H), 6.82 (s, 1H), 3.93-3.87 (m, 2H), 3.80 (s, 3H), 3.75 (s, 3H), 3.13-3.11 (m, 2H), 2.64-2.54 (m, 4H), 2.27-2.17 (m, 3H), 2.01 (d, *J* = 14.4 Hz, 6H), 1.84-1.80 (m, 2H), 1.59-1.52 (m, 2H), 1.15 (d, *J* = 6.4 Hz, 6H). | 775.1, 777.1 |
| 175 | 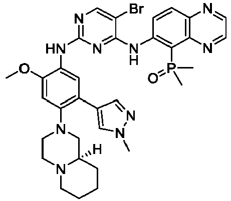 | ¹H NMR (300 MHz, CDsOD) δ 8.85-8.79 (m, 3H), 8.49 (s, 1H), 8.29 (s, 1H), 7.98 (s, 1H), 7.81 (s, 1H), 7.55-7.51 (m, 2H), 6.86 (s, 1H), 3.95 (s, 3H), 3.70 (s, 3H), 3.18-3.06 (m, 4H), 2.99-2.91 (m, 1H), 2.85-2.60 (s, 4H), 2.20 (s, 3H), 2.15 (s, 3H), 1.90-1.68 (m, 4H), 1.55-1.39 (m, 2H). | 717.3, 719.3 |
| | | [α]_{D}²⁵ = +24.5(c = 0.35, MeOH) | |
| 176 | 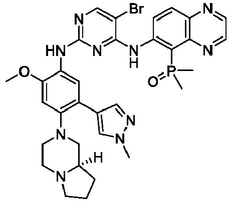 | ¹H NMR (300 MHz, CDsOD) δ 8.85-8.79 (m, 3H), 8.45 (s, 1H), 8.29 (s, 1H), 8.00 (s, 1H), 7.83 (s, 1H), 7.54 (d, *J* = 9.3 Hz, 1H), 7.48 (s, 1H), 6.90 (s, 1H), 3.96 (s, 3H), 3.71 (s, 3H), 3.52-3.40 (m, 3H), 3.24-3.17 (m, 2H), 3.06-3.00 (m, 3H), 2.91-2.84 (s, 1H), 2.19-2.12 (m, 9H) 1.83-1.73 (m, 1H). | 703.2, 705.2 |
| | | [*α*]_{D}²⁵ = +5.5° (c = 0.4 , MeOH) | |
| 177 | 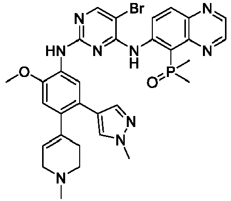 | ¹H NMR (400 MHz, CDCl₃) δ 12.61 (s, 1H), 8.94 (dd, *J* = 9.6, 4.0 Hz, 1H), 8.77 (d, *J* = 2.0 Hz, 1H), 8.73 (d, *J* = 2.0 Hz, 1H), 8.38 (s, 1H), 8.33 (s, 2H), 7.64 (d, *J* = 9.6 Hz,1H), 7.58 (s. 1H), 7.46 (s, 1H), 6.74 (s, 1H), 5.73 (s, 1H), 3.94 (s, 3H), 3.84 (s, 3H), 3.62 (s, 2H), 3.05 (s, 2H), 2.75 (s, 3H), 2.48 (s, 2H), 2.15 (d, *J* = 14.4 Hz, 6H). | 674.4, 676.4 |
| 178 | 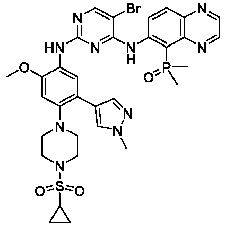 | ¹H NMR (400 MHz, CDCl₃) δ 13.42 (s, 1H), 8.81-8.75 (m, 3H), 8.07 (s, 1H), 7.71 (s, 2H), 7.59-7.54 (m, 2H), 6.71 (s, 1H), 3.90 (s, 3H), 3.78 (s, 3H), 3.43 (s, 4H), 3.04 (s, 4H), 2.34 (s, 1H), 2.13 (d, *J* = 14.0 Hz, 6H), 1.26-1.21 (m, 2H), 1.08-1.03 (m, 2H). | 767.1, 769.1 |
| 179 | 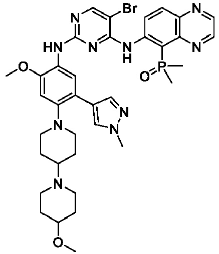 | ¹H NMR (400 MHz, CDsOD) δ 8.82 (p, *J* = 4.0 Hz, 2H), 8.77 (d, *J* = 2.0 Hz, 1H), 8.49 (s, 1H), 8.25 (s, 1H), 7.92 (s, 1H), 7.79 (s, 1H), 7.52 (s, 1H), 7.48 (s, 1H), 6.82 (s, 1H), 3.90 (s, 3H), 3.68 (s, 3H), 3.43 (s, 1H), 3.37 (s, 3H), 3.21 (d, *J* = 11.6 Hz, 2H), 3.13 (s, 2H), 2.79 (s, 3H), 2.71-2.62 (m, 2H), 2.16 (s, 3H), 2.12 (s, 3H), 2.06-2.00 (m, 4H), 1.85-1.68 (m, 4H). | 775.5, 777.5 |
| 180 | 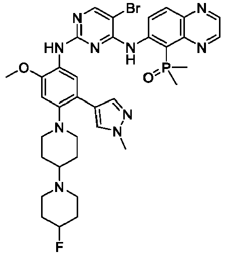 | ¹H NMR (300 MHz, CDsOD) δ 8.89-8.83 (m, 2H), 8.79 (d, *J* = 2.1 Hz, 1H), 8.55 (s, 1H), 8.28 (s, 1H), 7.94 (s, 1H), 7.84 (s, 1H), 7.55 (s, 1H), 7.50 (s, 1H), 6.86 (s, 1H), 4.71-4.61 (m, 1H), 3.93 (s, 3H), 3.71 (s, 3H), 3.24 (d, *J* = 12.3 Hz, 2H), 3.03-2.92 (m, 4H), 2.74-2.66 (m, 3H), 2.17 (d, *J* = 14.4 Hz, 6H), 2.12-1.92 (m, 6H), 1.79-1.75 (m, 2H). | 763.4, 765.4 |
| 181 | 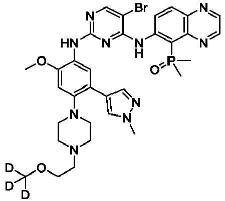 | ¹H NMR (300 MHz, CDCl₃) δ 12.60 (s, 1H), 9.01 (dd, *J* = 9.6, 4.2 Hz, 1H), 8.78-8.66 (m, 2H), 8.31 (s, 1H), 8.24 (s, 1H), 7.65 (d, *J* = 6.6 Hz, 3H), 7.38 (s, 1H), 6.76 (s, 1H), 3.92 (s,3H), 3.75 (s, 3H), 3.67 (t, *J* = 5.4 Hz, 2H), 3.06 (d, *J* = 5.1 Hz, 4H), 2.84 (d, *J* = 6.6 Hz, 6H), 2.17 (s, 3H), 2.12 (s, 3H). | 723.9, 725.9 |
| 182 | 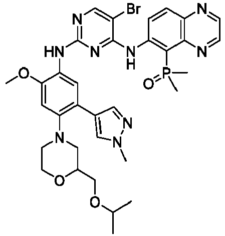 | ¹H NMR (400 MHz, CDCl₃) δ 12.59 (s, 1H), 8.97 (dd, *J* = 9.6, 4.4 Hz, 1H), 8.73 (d, *J* = 1.6 Hz, 1H), 8.70 (d, *J* = 1.6 Hz, 1H), 8.29 (s, 1H), 8.23 (s, 1H), 7.77 (s, 1H), 7.60 (d, *J* = 9.2 Hz, 1H), 7.51 (s, 1H), 7.40 (s, 1H), 6.70 (s, 1H), 3.96-3.82 (m, 4H), 3.85-3.74 (m, 5H), 3.62-3.56 (m, 1H), 3.53-3.49 (m, 1H), 3.43-3.93 (m, 1H), 3.06-3.02 (m, 1H), 2.93-2.90 (m, 1H), 2.81-2.75 (m, 1H), 2.62-2.57 (m, 1H), 2.13 (d, *J* = 14.4 Hz, 6H), 1.17 (s, 3H), 1.16 (s, 3H). | 736.1, 737.9 |
| 184 | 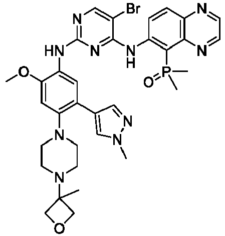 | ¹H NMR (400 MHz, CDsOD) δ 8.82 (dd, *J* = 8.8, 3.2 Hz, 2H), 8.76 (d, *J* = 2.0 Hz, 1H), 8.25 (s, 1H), 7.91 (s, 1H), 7.77 (s, 1H), 7.51 (s, 2H), 6.85 (s, 1H), 4.63 (d, *J* = 6.0 Hz, 2H), 4.28 (d, *J* = 6.0 Hz, 2H), 3.92 (s, 3H), 3.65 (s, 3H), 2.99-2.92 (m, 4H), 2.57-2.51 (m, 4H), 2.16 (s, 3H), 2.12 (s, 3H), 1.47 (s, 3H). | 733.4, 735.4 |
| 185 | 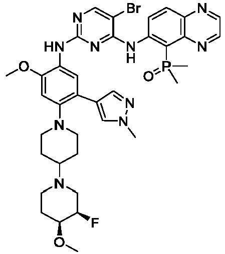 | ¹H NMR (300 MHz, CDCl₃) δ 12.59 (s, 1H), 9.00 (dd, *J* = 9.6, 4.2 Hz, 1H), 8.77-8.69 (m, 2H), 8.31 (s, 1H), 8.24 (d, *J* = 7.8 Hz, 1H), 7.72 (s, 1H), 7.62 (d, *J* = 9.0 Hz, 1H), 7.56 (s, 1H), 7.36 (s, 1H), 6.71 (s, 1H), 4.92-4.74 (m, 1H), 3.92 (s, 3H), 3.78 (s, 3H), 3.48 (s, 4H), 3.24 (d, *J* = 11.1 Hz, 2H), 3.07 (s, 1H), 2.83 (s, 2H), 2.61 (t, *J=* 11.4 Hz, 4H), 2.17 (s, 3H), 2.13 (s, 3H), 1.96 (s, 4H), 1.73 (s, 2H). | 793.0, 795.0 |
| | | [α]_{D}²⁵ = +1.6° ( *c*= 0.29 , MeOH) | |
| 186 | 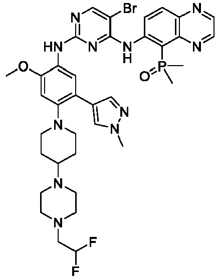 | ¹H NMR (300 MHz, CDsOD) δ 8.89-8.78 (m, 3H), 8.27 (s, 1H), 7.94 (s, 1H), 7.89 (s, 1H), 7.53 (d, *J* = 9.9 Hz, 1H), 7.45 (s, 1H), 6.85 (s, 1H), 6.00 (t, *J* = 55.8 Hz, 1H), 3.92 (s, 3H), 3.72 (s, 3H), 3.19 (d, *J* = 11.7 Hz, 2H), 2.87-2.59 (m, 12H), 2.33 (s, 1H), 2.17 (d, *J* = 14.4 Hz, 6H), 2.01 (d, *J* = 10.5 Hz, 2H), 1.70-1.58 (m, 2H). | 810.4, 812.4 |
| 187 | 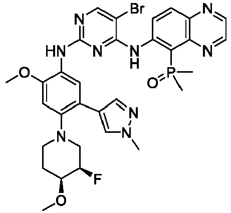 | ¹H NMR (300 MHz, CDCl₃) δ 12.83 (s, 1H), 8.94 (dd, *J* = 9.6, 4.2 Hz, 1H), 8.76 (d, *J* = 2.1 Hz, 1H), 8.73 (d, *J* = 2.1 Hz, 1H), 8.25 (s, 1H), 8.18 (s, 1H), 8.04 (s, 1H), 7.56 (d, *J* = 9.3 Hz, 1H), 7.32 (s, 1H), 6.73 (s, 1H), 4.98-4.82 (m, 1H), 3.93 (s, 3H), 3.84 (s, 3H), 3.51 (s, 3H), 3.48-3.42 (m, 2H), 3.11-3.07 (m, 1H), 2.99-2.86 (m, 1H), 2.76-2.69 (m, 1H), 2.15 (d, *J* = 14.1 Hz, 6H), 2.06-1.99 (m, 1H), 1.91-1.83 (m, 1H). | 709.9, 711.9 |
| | | [*α*]_{D}²⁵ = -7.0° (c = 0.38 , MeOH) | |
| 188 | 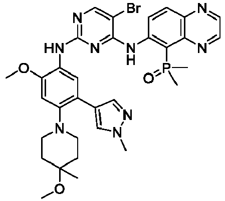 | ¹H NMR (400 MHz, CDCl₃) δ 12.63 (s, 1H), 8.98 (dd, *J* = 9.6, 4.4 Hz, 1H), 8.71 (dd, *J* = 14.4, 2.0 Hz, 2H), 8.27 (s, 1H), 8.16 (s, 1H), 7.70 (s, 1H), 7.60 (s, 2H), 7.46 (s, 1H), 6.76 (s, 1H), 3.91 (s, 3H), 3.75 (s, 3H), 3.23 (s, 3H), 2.97-2.77 (m, 4H), 2.12 (d, *J* = 14.4 Hz, 6H), 1.85-1.64 (m, 4H), 1.22 (s, 3H). | 706.4, 708.4 |
| 189 | 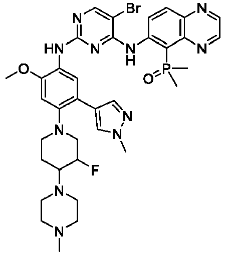 | ¹H NMR: (300 MHz, CDCl₃) δ 12.59 (s, 1H), 8.98 (dd, *J* = 9.6, 4.2 Hz, 1H), 8.75-8.70 (m, 2H), 8.32-8.27 (m, 2H), 8.06 (s, 1H), 7.59 (d, *J* = 9.6 Hz, 1H), 7.39-7.32 (m, 2H), 6.70 (s, 1H), 5.13-4.91 (m, 1H), 3.92 (s, 3H), 3.82 (s, 3H), 3.57-3.43 (m, 1H), 3.29-3.18 (m, 1H), 2.88-2.69 (m, 9H), 2.47 (s, 3H), 2.43-2.39 (m, 1H), 2.38-2.35 (m, 1H), 2.17 (s, 3H), 2.12 (s, 3H), 2.10-2.03 (m, 1H), 1.87-1.79 (m, 1H). | 778.4, 780.4 |
| | | ¹⁹F NMR: (282 MHz, CDCl₃) δ -198.58. | |
| 190 | 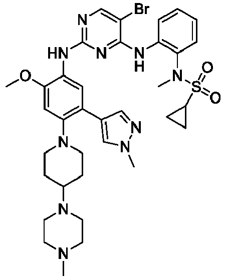 | ¹H NMR (300 MHz, CDCl₃) δ 8.45-8.33 (m, 2H), 8.20 (d, *J* = 2.1 Hz, 2H), 7.80 (s, 1H), 7.61 (s, 1H), 7.53-7.37 (m, 1H), 7.31 (s, 1H), 7.04-6.86 (m, 1H), 6.87 (s, 1H), 6.70 (s, 1H), 3.91 (s, 3H), 3.89 (s, 3H), 3.34 (s, 3H), 3.23 (d, *J* = 11.4 Hz, 2H), 2.94-2.59 (m, 9H), 2.60-2.44 (m, 2H), 2.42 (s, 3H), 2.38 (s, 1H), 1.97 (d, *J* = 12.0 Hz, 2H), 1.76-1.60 (m, 2H), 1.19 (s, 2H), 1.05 (s, 2H). | 765.4, 767.4 |
| 191 | 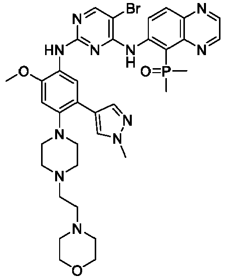 | ¹H NMR (400 MHz, CDsOD) δ 8.81-8.76 (m, 3H), 8.25 (s, 1H), 7.94 (s, 1H), 7.81 (s, 1H), 7.52 (s, 1H), 7.48 (s, 1H), 6.84 (s, 1H), 3.92 (s, 3H), 3.73 (t, *J* = 4.8 Hz, 4H), 3.67 (s, 3H), 2.98 (t, *J* = 4.8 Hz, 4H), 2.81-2.77 (m, 6H), 2.68-2.65 (m, 2H), 2.60-2.58 (m, 4H), 2.15 (d, *J* = 14.4 Hz, 6H). | 776.4, 778.4 |
| 193 | 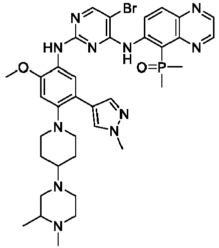 | ¹H NMR (300 MHz, CDCl₃) δ 12.59 (s, 1H), 8.99 (dd, *J* = 9.6, 3.9 Hz, 1H), 8.76-8.82 (m, 2H), 8.31 (s, 1H), 8.22 (s, 1H), 7.69-7.59 (m, 3H), 7.36 (s, 1H), 6.69 (s, 1H), 3.92 (s, 3H), 3.79 (s, 3H), 3.27-3.15 (m, 5H), 2.99 (s, 3H), 2.66-2.57 (m, 7H), 2.15 (d, *J* = 14.1 Hz, 6H), 2.01 (s, 4H), 1.37 (s, 3H). | 774.4, 776.4 |
| 194 | 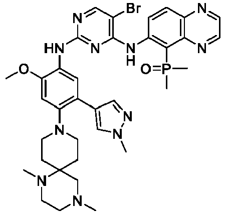 | ¹H NMR (300 MHz, CDsOD) δ 8.87-8.79 (m, 3H), 8.27 (s, 1H), 7.92 (s, 1H), 7.81 (s, 1H), 7.57 (s, 1H), 7.52 (s, 1H), 6.90 (s, 1H), 3.94 (s, 3H), 3.70 (s, 3H), 3.08-3.04 (m, 4H), 2.90-2.83 (m, 3H), 2.66 (s, 6H), 2.40 (s, 3H), 2.16 (d, *J* = 14.4 Hz, 6H), 2.07-2.03 (m, 2H), 1.92-1.85 (s, 2H). | 760.2, 762.2 |
| 195 | 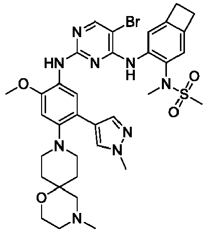 | ¹H NMR (300 MHz, CDCl₃) δ 8.29-8.25 (m, 2H), 8.18 (s, 1H), 8.01 (s, 1H), 7.74 (s, 1H), 7.63 (s, 1H), 7.37 (s, 1H), 7.07 (s, 1H), 6.77 (s, 1H), 3.91-3.83 (m, 8H), 3.31 (s, 3H), 3.08-2.77 (m, 11H), 2.55-2.38 (m, 7H), 2.10-2.05 (m, 2H), 1.77-1.70 (m, 2H). | 751.9, 753.9 |
| 196 | 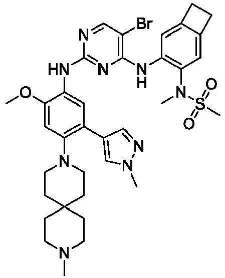 | ¹H NMR (300 MHz, CDCl₃) δ 8.27-8.26 (m, 2H), 8.19 (s, 1H), 8.01 (s, 1H), 7.85 (s, 1H), 7.50 (s, 1H), 7.35 (s, 1H), 7.07 (s, 1H), 6.71 (s, 1H), 3.92 (s, 3H), 3.86 (s, 3H), 3.31 (s, 3H), 3.07-3.04 (m, 8H), 2.87-2.84 (m, 7H), 2.75 (s, 3H), 1.95 (s, 4H). 1.71-1.67 (m, 4H). | 750.1, 752.1 |
| 197 | 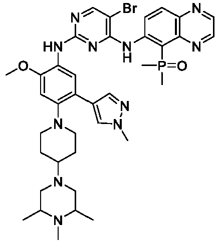 | ¹H NMR (400 MHz, CDsOD) δ 8.86-8.76 (m, 3H), 8.25 (s, 1H), 7.91 (s, 1H), 7.75 (s, 1H), 7.55-7.51 (m, 2H), 6.83 (s, 1H), 3.90 (s, 3H), 3.66 (s, 3H), 3.27-3.18 (m, 4H), 3.11-3.08 (m, 2H), 2.75 (s, 3H), 2.69-2.63 (m, 2H), 2.58-2.52 (m, 1H), 2.44-2.38 (m, 2H), 2.16 (s, 3H), 2.13 (s, 3H), 1.98-1.95 (m, 2H), 1.72-1.64 (m, 2H), 1.36 (s, 3H), 1.35 (s, 3H). | 788.4, 790.4 |
| 198 | 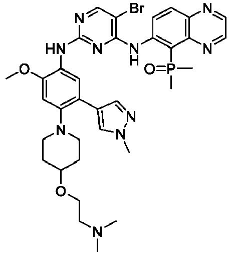 | ¹H NMR (300 MHz, CDsOD) δ 8.89-8.78 (m, 3H), 8.27 (s, 1H), 7.93 (s, 1H), 7.81 (s, 1H), 7.56 (s, 1H), 7.53 (s, 1H), 6.85 (s, 1H), 3.92 (s, 3H), 3.86-3.80 (m, 2H), 3.68 (s, 3H), 3.64-3.52 (m, 1H), 3.29-3.26 (m, 2H), 3.15-3.11 (m, 2H), 2.86 (s, 6H), 2.79-2.72 (m, 2H), 2.17 (d, *J* = 14.4 Hz, 6H), 2.09-2.05 (m, 2H), 1.79-1.70 (m, 2H). | 748.9 ,750 .9 |
| 199 | 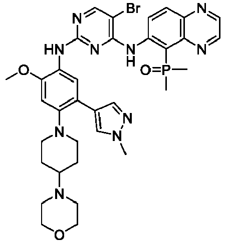 | ¹H NMR (300 MHz, CDsOD) δ 8.82-8.81 (m, 1H), 8.67 (d*, J* = 8.7 Hz, 1H), 8.48 (dd, *J* = 9.3, 4.2 Hz, 1H), 8.24 (s, 1H), 7.87 (s, 1H), 7.70-7.60 (m, 3H), 7.48 (s, 1H), 6.81 (s, 1H), 3.91-3.87 (m, 7H), 3.64 (s, 3H), 3.23-3.19 (m, 2H), 3.09 (s, 4H), 2.90-2.79 (m, 1H), 2.71-2.64 (m, 2H), 2.18-2.11 (m, 8H), 1.79-1.68 (m, 2H). | 746.3,748. 3 |
| 201 | 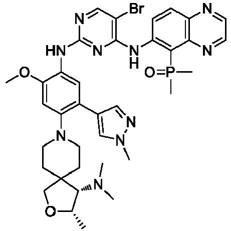 | ¹H NMR (300 MHz, CDsOD) *δ* 8.93-8.85 (m, 3H), 8.33 (s, 1H), 7.97 (s, 1H), 7.90 (s, 1H), 7.60 (s, 2H), 6.90 (s, 1H), 4.44-4.35 (m, 1H), 4.08-4.05 (m, 1H),3.99 (s, 3H), 3.97-3.94 (m, 1H), 3.76 (s, 3H), 3.14-3.07 (m, 2H), 2.83-2.80 (m, 2H), 2.74 (s, 6H), 2.70-2.64 (m, 1H), 2.26 (s, 3H), 2.21 (s, 3H), 2.16-2.07 (m, 2H), 1.90-1.85 (m, 1H), 1.74-1.70 (m, 1H), 1.44 (d, *J* = 6.6 Hz, 3H). | 775.4, 777.4 |
| | | [α]_{D}²⁵ = -7.5° (c = 0.18 , CHCl₃). | |
| 202 | 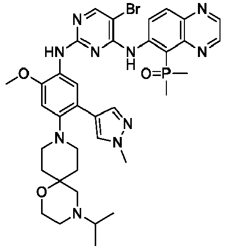 | ¹H NMR (300 MHz, CDCl₃) δ 12.57 (s, 1H), 9.01 (dd, *J* = 9.6, 4.2 Hz, 1H), 8.78-8.68 (m, 2H), 8.30 (s, 1H), 8.20 (s, 1H), 7.70 (s, 1H), 7.63 (s, 2H), 7.37 (s, 1H), 6.78 (s, 1H), 3.92 (s, 3H), 3.88-3.83 (m, 2H), 3.76 (s, 3H), 2.98-2.83 (m, 7H), 2.67-2.64 (m, 2H), 2.17 (s, 3H), 2.12-2.07 (m, 5H), 1.74-1.66 (m, 2H), 1.13 (d, *J* = 6.5 Hz, 6H). | 774.9, 776.9 |
| 203 | 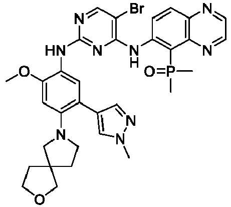 | ¹H NMR (300 MHz, DMSO-*d₆*) δ 12.69 (s, 1H), 8.85-8.82 (m, 3H), 8.42 (s, 1H), 8.25 (s, 1H), 7.75 (s, 1H), 7.55 (s, 2H), 7.36 (s, 1H), 6.70 (s, 1H), 3.84-3.71 (m, 8H), 3.64-3.56 (m, 2H), 3.08-2.97 (m, 4H), 2.02 (d, *J* = 14.4 Hz, 6H), 1.96-1.86 (m, 4H). | 704.4, 706.4 |
| 204 | 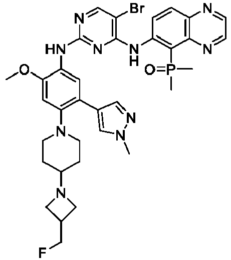 | ¹H NMR (400 MHz, DMSO-*d₆*) δ 12.68 (s, 1H), 8.87-8.74 (m, 3H), 8.27 (s, 1H), 8.18 (s, 1H), 7.97 (s, 1H), 7.74 (s, 1H), 7.57 (s, 2H), 6.82 (s, 1H), 4.59 (d, *J* = 6.4 Hz, 1H), 4.47 (d, *J* = 6.0 Hz, 1H), 3.80 (s, 3H), 3.78 (s, 3H), 3.31-3.28 (m, 2H), 3.05-2.94 (m, 4H), 2.77-2.58 (m, 3H), 2.19-2.13 (m, 1H), 2.01 (d, *J* = 14.4 Hz, 6H), 1.73-1.70 (m, 2H), 1.37-1.30 (m, 2H). | 749.4, 751.4 |
| 206 | 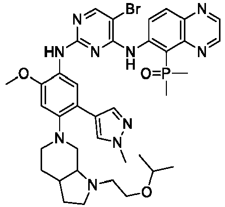 | ¹H NMR (300 MHz, CDsOD) δ 8.89-8.80 (m, 3H), 8.28 (s, 1H), 7.96 (s, 1H), 7.93 (s, 1H), 7.55 (s, 2H), 6.90 (s, 1H), 3.95 (s, 3H), 3.72 (s, 3H), 3.60-3.54 (m, 3H), 3.47-3.40 (m, 2H), 3.17-3.05 (m, 3H), 2.95-2.80 (m, 4H), 2.55-2.48 (m, 1H), 2.17 (d, *J* = 14.4 Hz, 6H), 2.06-1.93 (m, 3H), 1.82-1.75 (m, 1H), 1.11 (t, *J* = 6.6 Hz, 6H). | 789.4, 791.4 |
| 207 | 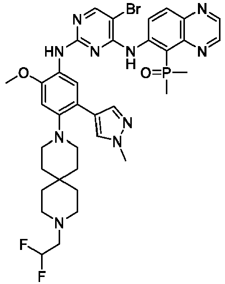 | ¹H NMR (300 MHz, CDCl₃) δ 12.31 (s, 1H), 8.86 (dd, *J* = 4.2, 1.5 Hz, 1H), 8.79 (dd, *J* = 9.6, 4.2 Hz, 1H), 8.28 (s, 1H), 8.21 (s, 1H), 8.09 (d, *J* = 8.7 Hz, 1H), 7.81 (d, *J* = 9.3 Hz, 1H), 7.66 (s, 1H), 7.59 (s, 1H), 7.45 (dd, *J* = 9.0, 4.2 Hz, 1H), 7.35 (s, 1H), 6.72 (s, 1H), 6.15-5.86 (m, 1H), 3.92 (s, 3H), 3.70 (s, 3H), 2.84-2.66 (m, 10H), 2.18 (s, 3H), 2.13 (s, 3H), 1.66-1.62 (m, 8H). ¹⁹F NMR (282 MHz, CDCl₃) δ -118.13. | 794.0, 796.0 |
| 208 | 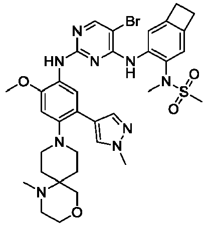 | ¹H NMR (300 MHz, CDCl₃) δ 8.26 (s, 2H), 8.17 (s, 1H), 7.98 (s, 1H), 7.78 (s, 1H), 7.54 (s, 1H), 7.33 (s, 1H), 7.06 (s, 1H), 6.68 (s, 1H), 3.89-3.85 (m, 9H), 3.29 (s, 3H), 3.10-3.02 (m, 8H), 2.85-2.56 (m, 8H), 2.15-2.03 (m, 3H), 1.73-1.67 (m, 2H). | 752.4, 754.4 |
| 209 | 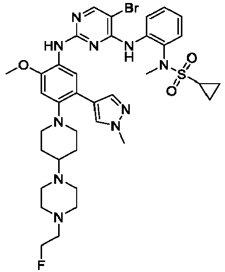 | ¹H NMR (300 MHz, CDCl₃) δ 8.40-8.35 (m, 2H), 8.20 (s, 2H), 7.77 (s, 1H), 7.66 (s, 1H), 7.47 (dd, *J* = 8.1, 1.5 Hz, 1H), 7.30 (s, 1H), 7.04-6.99 (m, 1H), 6.91-6.84 (m, 1H), 6.70 (s, 1H), 4.69 (t, *J* = 4.8 Hz, 1H), 4.53 (t, *J* = 4.8 Hz, 1H), 3.91 (s, 6H), 3.34 (s, 3H), 3.27-3.23 (m, 2H), 2.81-2.42 (m, 13H), 2.35-2.27 (m, 1H), 2.05-1.95 (m, 2H), 1.69-1.64 (m, 2H), 1.20-1.04 (m, 4H). | 797.4, 799.4 |
| 210 | 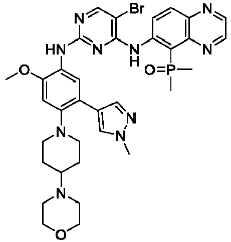 | ¹H NMR (300 MHz, CDCl₃) δ 12.58 (s, 1H), 9.07 (dd, *J* = 9.6, 4.2 Hz, 1H), 8.71-8.69 (m, 2H), 8.64 (s, 1H), 8.29 (s, 1H), 8.24 (s, 1H), 7.71 (s, 1H), 7.43 (dd, *J* = 9.6 Hz, 1H), 7.15 (s, 1H), 6.75 (s, 1H), 3.91 (s, 7H), 3.78 (s, 3H), 3.32-3.28 (m, 2H), 2.84-2.67 (m, 7H), 2.15-2.10 (m, 8H), 1.92-1.85 (m, 2H). | 747.0, 749.0 |
| 211 | 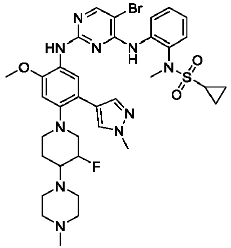 | ¹H NMR (300 MHz, CDCl₃) δ 8.42 (s, 1H), 8.37-8.34 (m, 1H), 8.25 (s, 1H), 8.21 (s, 1H), 7.86 (s, 1H), 7.69 (s, 1H), 7.49-7.46 (m, 1H), 7.33 (s, 1H), 7.05-7.00 (m, 1H), 6.93-6.90 (m, 1H), 6.67 (s, 1H), 5.09-4.92 (m, 1H), 3.91 (s, 3H), 3.90 (s, 3H), 3.56-3.46 (m, 1H), 3.34 (s, 3H), 3.26-3.22 (s, 1H), 3.10-2.86 (m, 8H), 2.78-2.50 (m, 7H), 2.11-2.05 (m, 2H), 1.20-1.05 (m, 4H). | 783.0, 785.0 |
| 212 | 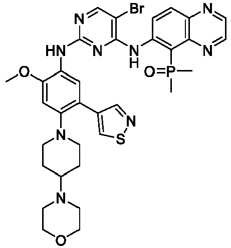 | ¹H NMR: (300 MHz, CDCl₃) δ 12.50 (s, 1H), 8.86 (dd, *J* = 9.6, 4.2 Hz, 1H), 8.78 (d, *J* = 1.8 Hz, 1H), 8.74 (d, *J* = 1.8 Hz, 1H), 8.67 (s, 1H), 8.61 (s, 1H), 8.32-8.28 (m, 2H), 7.69 (d, *J* = 9.6 Hz, 1H), 7.47 (s, 1H), 6.72 (s, 1H), 3.96 (s, 3H), 3.91-3.86 (m, 4H), 3.16-3.12 (m, 2H), 2.83-2.76 (m, 4H), 2.71-2.59 (m, 3H), 2.18 (s, 3H), 2.13 (s, 3H), 2.02-1.93 (m, 2H), 1.72-1.55 (m, 2H). | 749.9, 751.9 |
| 213 | 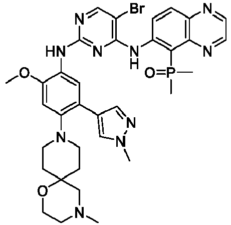 | ¹H NMR (300 MHz, CDCl₃) δ 12.27 (s, 1H), 8.85 (dd, *J* = 4.2, 1.5 Hz, 1H), 8.78 (dd, *J* = 9.6, 4.2 Hz, 1H), 8.28 (s, 1H), 8.23 (s, 1H), 8.09 (d, *J* = 8.7 Hz, 1H), 7.80 (d, *J* = 9.6 Hz, 1H), 7.65 (s, 1H), 7.61 (s, 1H), 7.44 (dd, *J* = 8.7, 4.2 Hz, 1H), 7.33 (s, 1H), 6.76 (s, 1H), 3.91 (s, 3H), 3.83-3.79 (m, 2H), 3.71 (s, 3H), 2.98-2.82 (m, 4H), 2.44 (s, 2H), 2.34-2.31 (m, 5H), 2.15 (d, *J* = 12.9 Hz, 6H), 2.06-2.01 (m, 2H), 1.74-1.64 (m, 2H). | 745.9, 747.9 |
| 214 | 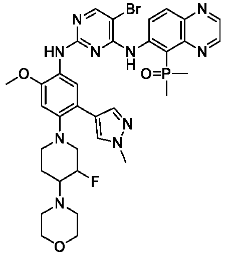 | ¹H NMR (300 MHz, DMSO-*d₆*) δ 12.68 (s, 1H), 8.85-8.78 (m, 3H), 8.41 (s, 1H), 8.29 (s, 1H), 8.03 (s, 1H), 7.81 (s, 1H), 7.64 (s, 1H), 7.58-7.54 (m, 1H), 6.87 (s, 1H), 5.20-5.04 (m, 1H), 3.81 (s, 3H), 3.74 (s, 3H), 3.62 (s, 4H), 3.08-2.91 (m, 2H), 2.70-2.62 (m, 4H), 2.47-2.40 (m, 2H), 2.05-1.88 (m, 8H), 1.74-1.70 (m, 1H). | 765.3, 767.3 |
| | | ¹⁹F NMR (282 MHz, DMSO-*d₆*) δ -199.90. | |
| 215 | 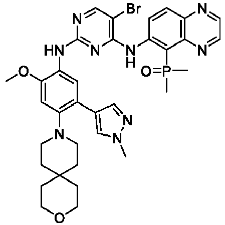 | ¹H NMR (300 MHz, CDsOD) δ 8.86-8.78 (m, 3H), 8.26 (s, 1H), 7.90 (s, 1H), 7.84 (s, 1H), 7.55-7.52 (m, 2H), 6.87 (s, 1H), 3.92 (s, 3H), 3.74-3.69 (m,7H), 2.90-2.86 (m, 4H), 2.16 (d, *J* = 14.4 Hz, 6H), 1.72-1.60 (m, 8H). | 732.3, 734.3 |
| 216 | 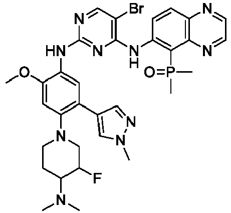 | ¹H NMR (300 MHz, CDsOD) δ 8.88-8.78 (m, 3H), 8.28 (s, 1H), 8.04 (s, 1H), 8.02 (s, 1H), 7.52 (d, *J* = 9.6 Hz, 1H), 7.35 (s, 1H), 6.87 (s, 1H), 5.19-5.03 (m, 1H), 3.93 (s, 3H), 3.71 (s, 3H), 3.47-3.39 (m, 1H), 3.17-3.13 (m, 1H), 3.03-2.87 (m, 1H), 2.76-2.68 (m, 1H), 2.45 (s, 6H), 2.41-2.26 (m, 1H), 2.20-2.15 (m, 6H), 2.04-1.84 (m, 2H). | 723.3, 725.3 |
| | | ¹⁹F NMR (282 MHz, CD₃OD) δ -200.84. | |
| 218 | 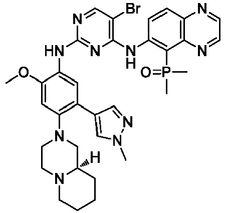 | ¹H NMR (300 MHz, CDCl₃) δ 12.37 (s, 1H), 9.28 (dd, *J* = 9.6, 4.5 Hz, 1H), 8.73-8.69 (m, 2H), 8.29 (s, 1H), 8.02 (s, 1H), 7.81 (s, 1H), 7.64-7.57 (m, 3H), 6.77 (s, 1H), 3.94 (s, 3H), 3.74 (s, 3H), 3.23-2.90 (m, 6H), 2.44-2.36 (m, 3H), 2.29 (s, 3H), 2.16 (s, 3H), 2.11 (s, 3H), 1.92-1.77 (m, 3H), 1.66-1.63 (m, 2H), 1.50-1.39 (m, 1H). [α]_{D}²⁵ = +20.2° (c = 0.17 , CHCl₃). | 653.3 |
| 220 | 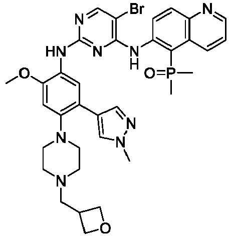 | ¹H NMR (300 MHz, CD₃OD) δ 8.81 (dd, *J* = 4.5, 1.5 Hz, 1H), 8.66 (d, *J* = 8.7 Hz, 1H), 8.47 (dd, *J* = 9.3, 4.2 Hz, 1H), 8.23 (s, 1H), 7.87 (s, 1H), 7.68-7.59 (m, 3H), 7.45 (s, 1H), 6.79 (s, 1H), 4.86-4.75 (m, 2H), 4.48 (t, *J* = 6.3 Hz, 2H), 3.90 (s, 3H), 3.63 (s, 3H), 3.39-3.30 (m, 1H), 2.90-2.81 (m, 6H), 2.57 (s, 4H), 2.16 (d, *J* = 13.2 Hz, 6H). | 732.3, 734.3 |
| 225 | 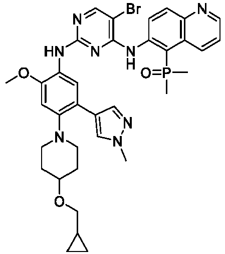 | ¹H NMR (300 MHz, CDCl₃) δ 12.25 (s, 1H), 8.83-8.80 (m, 1H), 8.74 (dd, *J* = 9.6, 4.2 Hz, 1H), 8.26 (s, 1H), 8.22 (s, 1H), 8.07 (d, *J* = 8.7 Hz, 1H), 7.79-7.83 (m, 2H), 7.45-7.40 (m, 2H), 7.32 (s, 1H), 6.70 (s, 1H), 3.88 (s, 3H), 3.74 (s, 3H), 3.44-3.77 (m, 1H), 3.33 (d, *J* = 6.6 Hz, 2H), 3.12-3.08 (m, 2H), 2.67-2.59 (m, 2H), 2.15 (s, 3H), 2.11 (s, 3H), 1.99-1.95 (m, 2H), 1.72-1.63 (m, 2H), 1.26-1.04 (m, 1H), 0.59-0.53 (m, 2H), 0.23-0.20 (m, 2H). | 731.3, 733.3 |
| 226 | 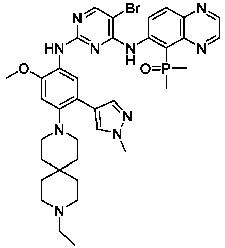 | ¹H NMR (300 MHz, CDCl₃) δ 12.58 (s, 1H), 9.01 (dd, *J* = 9.3, 4.2 Hz, 1H), 8.74-8.70 (m, 2H), 8.29 (s, 1H), 8.19 (s, 1H), 7.65-7.60 (m, 3H), 7.32 (s, 1H), 6.70 (s, 1H), 3.91 (s, 3H), 3.72 (s, 3H), 2.97-2.82 (m, 10H), 2.14 (s, 3H), 2.01 (s, 3H), 1.93-1.89 (m, 4H),1.65-1.62 (m,4H), 1.34 (t, *J* = 7.5 Hz, 3H). | 759.4, 761.4 |
| 227 | 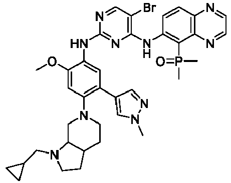 | ¹H NMR (300 MHz, CDsOD) δ 8.89-8.70 (m, 3H), 8.54 (s, 1H), 8.29 (s, 1H), 7.96 (s, 1H), 7.79 (s, 1H), 7.56 (s, 1H), 7.53 (s, 1H), 6.92 (s, 1H), 3.96 (s, 3H), 3.82-3.66 (m, 5H), 3.25-3.19 (m, 1H), 2.96-2.69 (m, 6H), 2.19-2.06 (m, 9H), 1.87-1.84 (m, 1H), 0.97-0.88 (m, 1H), 0.68-0.64 (s, 2H), 0.38-0.34 (m, 2H). | 757.3, 579.3 |
| 228 | 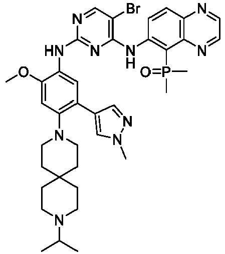 | ¹H NMR (300 MHz, CDCl₃) δ 12.59 (s, 1H), 9.01 (dd, *J* = 9.6, 4.2 Hz, 1H), 8.74-8.70 (m, 2H), 8.29 (s, 1H), 8.19 (s, 1H), 7.65-7.60 (m, 3H), 7.31 (s, 1H), 6.70 (s, 1H), 3.91 (s, 3H), 3.72 (s, 3H), 3.39-3.35 (m, 1H), 2.97 (s, 4H), 2.85-2.82 (m, 4H), 2.15 (s, 3H), 2.10 (s, 3H), 1.97 (s, 4H), 1.65-1.61 (m, 4H), 1.34 (s, 3H), 1.32 (s, 3H). | 773.4, 775.4 |
| 229 | 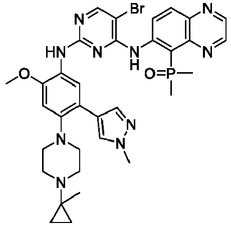 | ¹H NMR (400 MHz, CDCl₃) δ 10.66 (s, 1H), 8.48 (dd, *J* = 9.2, 4.4 Hz, 1H), 8.21 (s, 1H), 8.19 (s, 1H), 7.81 (s, 1H), 7.53 (s, 1H), 7.31 (s, 1H), 7.29-7.23 (m, 1H), 6.99-6.95 (m, 2H), 6.70 (s, 1H), 4.73 (t, *J* = 4.8 Hz, 1H), 4.62 (t, *J* = 4.8 Hz, 1H), 3.90 (s, 3H), 3.85 (s, 3H), 2.95-2.72 (m, 10H), 1.86 (s, 3H), 1.83 (s, 3H), 1.71 (t, *J* = 5.6 Hz, 4H), 1.61 (t, *J* = 5.6 Hz, 4H). | 725.4, 727.4 |
| | | ¹⁹F NMR (377 MHz, CDCl₃) δ -217.12 | |
| 230 | 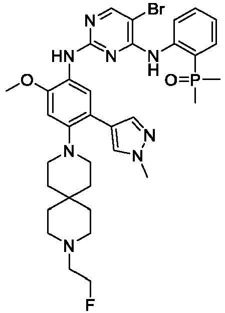 | ¹H NMR (400 MHz, CDCl₃) δ 10.66 (s, 1H), 8.48 (dd, *J* = 9.2, 4.4 Hz, 1H), 8.21 (s, 1H), 8.19 (s, 1H), 7.81 (s, 1H), 7.53 (s, 1H), 7.31 (s, 1H), 7.29-7.23 (m, 1H), 6.99-6.95 (m, 2H), 6.70 (s, 1H), 4.73 (t, *J* = 4.8 Hz, 1H), 4.62 (t, *J* = 4.8 Hz, 1H), 3.90 (s, 3H), 3.85 (s, 3H), 2.95-2.72 (m, 10H), 1.86 (s, 3H), 1.83 (s, 3H), 1.71 (t, *J* = 5.6 Hz, 4H), 1.61 (t, *J* = 5.6 Hz, 4H). | 725.4, 727.4 |
| | | ¹⁹F NMR (377 MHz, CDCl₃) δ -217.12 | |
| 231 | 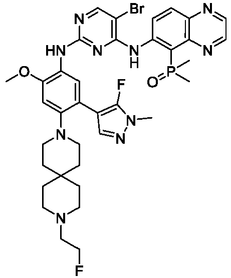 | ¹H NMR (300 MHz, CDCl₃) δ 12.52 (s, 1H), 8.92 (dd, *J* = 9.3, 4.2 Hz, 1H), 8.75-8.73 (m, 2H), 8.35 (s, 1H), 8.30 (s, 1H), 7.73 (d, *J* = 2.1 Hz, 1H), 7.64 (d, *J* = 9.3 Hz, 1H), 7.46 (s, 1H), 6.73 (s, 1H), 4.90-4.87 (m, 1H), 4.74-4.72 (m, 1H), 3.93 (s, 3H), 3.72 (s, 3H), 3.19-3.16 (m, 1H), 3.09-3.06 (m, 1H), 2.97-2.95 (m, 4H), 2.85-2.82 (m, 4H), 2.18 (s, 3H), 2.13 (s, 3H), 1.85-1.81 (m, 4H), 1.62-1.60 (m, 4H). | 795.2, 797.2 |
| | | ¹⁹F NMR (282 MHz, CDCl₃) δ -216.32, -131.24. | |
| 233 | 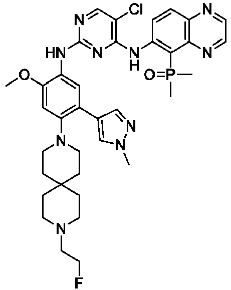 | ¹H NMR (300 MHz, CDCl₃) δ 12.78 (s, 1H), 9.16 (dd, *J* = 9.3, 4.2 Hz, 1H), 8.73 (d, *J* = 2.1 Hz, 1H), 8.69 (d, *J* = 2.1 Hz, 1H), 8.20 (s, 1H), 8.18 (s, 1H), 7.71 (s, 1H), 7.69 (s, 1H), 7.60 (d, *J* = 9.6 Hz, 1H), 7.30 (s, 1H), 6.73 (s, 1H), 4.80-4.64 (m, 2H), 3.91 (s, 3H), 3.76 (s, 3H), 2.94-2.73 (m, 10H), 2.14 (s, 3H), 2.10 (s, 3H), 1.78-1.73 (m, 4H), 1.63-1.60 (m, 4H). | 733.4, 735.4 |
| 235 | 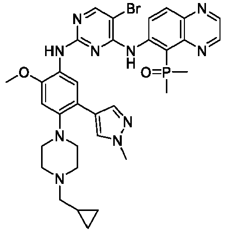 | ¹H NMR (300 MHz, CD₃OD) δ 9.96 (s, 1H), 9.23 (s, 1H), 8.52-8.48 (m, 1H), 8.27 (s, 1H), 7.80 (s, 1H), 7.73 (s, 1H), 7.63 (d, *J* = 8.7 Hz, 1H), 7.34 (s, 1H), 6.82 (s, 1H), 3.92 (s, 3H), 3.75 (s, 3H), 2.96-2.86 (m, 8H), 2.53 (d, *J* = 3.6 Hz, 2H), 2.20 (s, 3H), 2.16 (s, 3H), 1.00-0.95 (m, 1H), 0.67-0.62 (m, 2H), 0.29-0.24 (m, 2H). | 717.3, 719.3 |
| 236 | 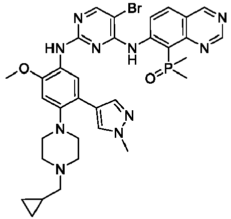 | ¹H NMR (300 MHz, CDsOD) δ 9.20 (s, 1H), 9.08 (s, 1H), 8.72 (dd, *J* = 9.3, 3.6 Hz, 1H), 8.34 (s, 1H), 8.10 (s, 1H), 7.73 (s, 1H), 7.62 (s, 1H), 7.48 (d, *J* = 9.6 Hz, 1H), 6.88 (s, 1H), 3.98 (s, 3H), 3.60 (s, 3H), 3.23-3.12 (m, 8H), 2.93-2.91 (d, *J* = 7.2 Hz, 2H), 2.23 (s, 3H), 2.18 (s, 3H), 1.15-1.06 (m, 1H), 0.79-0.73 (m, 2H), 0.43-0.40 (m, 2H). | 717.2, 719.2 |
| 238 | 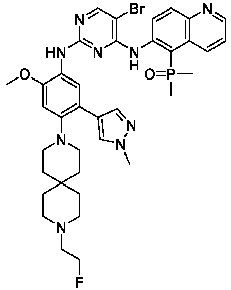 | ¹H NMR (300 MHz, CDCl₃) δ 12.26 (s, 1H), 8.84-8.82 (m, 1H), 8.79-8.84 (m, 1H), 8.26 (s, 1H), 8.19 (s, 1H), 8.07 (d, *J* = 8.7 Hz, 1H), 7.78 (d, *J* = 9.3 Hz, 1H), 7.63 (s, 1H), 7.58 (s, 1H), 7.45-7.41 (m, 1H), 7.30 (s, 1H), 6.70 (s, 1H), 4.80-4.63 (m, 2H), 3.90 (s, 3H), 3.68 (s, 3H), 2.96-2.74 (m, 10H), 2.16 (s, 3H), 2.11 (s, 3H), 1.77-1.74 (m, 4H), 1.62-1.58 (m, 4H). | 776.3, 778.3 |
| 239 | 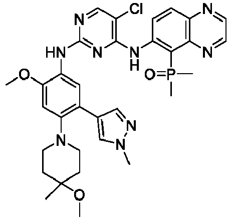 | ¹H NMR (300 MHz, CDCl₃) δ 12.90 (s, 1H), 9.13-9.08 (m, 1H), 8.74 (s, 1H), 8.70 (s, 1H), 8.14 (s, 2H), 7.76 (s, 1H), 7.65-7.50 (m, 2H), 7.26-7.23 (m, 1H), 6.77 (s, 1H), 3.92 (s, 3H), 3.83 (s, 3H), 3.23 (s, 3H), 2.97-2.83 (m, 4H), 2.14 (s, 3H), 2.10 (s, 3H), 1.86-1.82 (m, 2H), 1.72-1.66 (m, 2H), 1.22 (s, 3H). | 662.4, 666.4 |
| 240 | 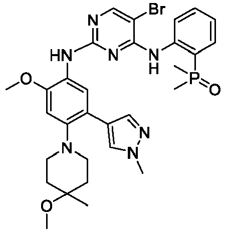 | ¹H NMR: (400 MHz, CDCl₃) δ 10.63 (s, 1H), 8.45 (dd, *J* = 8.8, 4.4 Hz, 1H), 8.20 (s, 1H), 8.15 (d, *J* = 7.6 Hz, 1H), 7.77 (s, 1H), 7.58 (s, 1H), 7.40 (s, 1H), 7.26-7.23 (m, 1H), 6.99-6.95 (m, 2H), 6.75 (s, 1H), 3.90 (s, 3H), 3.86 (s, 3H), 3.23 (s, 3H), 2.93-2.81 (m, 5H), 1.86-1.81 (m, 7H), 1.67-1.60 (m, 2H), 1.22 (s, 3H). | 654.3, 656.3 |
| 241 | 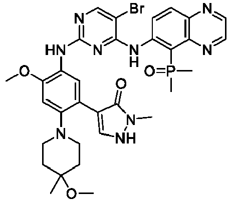 | ¹H NMR (300 MHz, CDCl₃) δ 15.33 (s, 1H), 12.65 (s, 1H), 8.96 (dd, *J* = 9.6, 4.5 Hz, 1H), 8.75 (d, *J* = 5.7 Hz, 2H), 8.39 (s, 1H), 8.34 (s, 1H), 7.71 (d, *J* = 9.6 Hz, 1H), 7.48 (s, 1H), 7.00 (s, 1H), 6.85 (s, 1H), 3.96 (s, 3H), 3.68 (s, 3H), 3.27 (s, 3H), 3.17 (s, 2H), 3.06-3.03 (m, 2H), 2.19 (s, 3H), 2.14 (s, 3H), 1.95 (s, 4H), 1.30 (s, 3H). | 722.3, 724.3 |
| 245 | 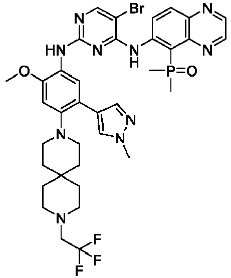 | ¹H NMR: (300 MHz, CDCl₃) δ 12.64 (s, 1H), 8.98 (dd, *J* = 9.6, 4.2 Hz, 1H), 8.73 (d, *J* = 2.1 Hz, 1H), 8.70 (d, *J* = 2.1 Hz, 1H), 8.27 (s, 1H), 8.15 (s, 1H), 7.69 (s, 1H), 7.62 (s, 2H), 7.44 (s, 1H), 6.73 (s, 1H), 3.91 (s, 3H), 3.74 (s, 3H), 3.03-2.94 (m, 2H), 2.85-2.81 (m, 4H), 2.67-2.64 (m, 4H), 2.15 (s, 3H), 2.10 (s, 3H), 1.62-1.59 (m, 8H). | 813.2, 815.2 |
| | | ¹⁹F NMR: (282 MHz, CDCl₃) δ -68.76. | |
| 246 | 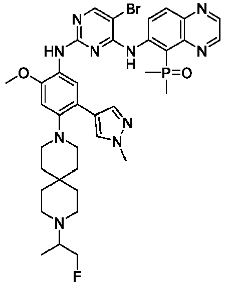 | ¹H NMR: (300 MHz, CDCl₃) δ 12.56 (s, 1H), 8.98 (dd, *J* = 9.3, 4.2 Hz, 1H), 8.73 (d, *J* = 2.1 Hz, 1H), 8.70 (d, *J* = 2.1 Hz, 1H), 8.28 (s, 1H), 8.17 (s, 1H), 7.64-7.62 (m, 3H), 7.38 (s, 1H), 6.70 (s, 1H), 4.90-4.48 (m, 2H), 3.91 (s, 3H), 3.73 (s, 3H), 3.55-3.46 (m, 1H), 3.07-3.03 (m, 4H), 2.86-2.83 (m, 4H), 2.15 (s, 3H), 2.11 (s, 3H), 1.89-1.86 (m, 4H), 1.64-1.61 (m, 4H), 1.34 (d, *J* = 6.9 Hz, 3H). | 791.2, 793.2 |
| 248 | 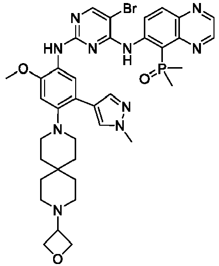 | ¹H NMR (300 MHz, DMSO-*d₆*) δ 12.66 (s, 1H), 8.84-8.77 (m, 3H), 8.41 (s, 1H), 8.27 (s, 1H), 8.00 (s, 1H), 7.80 (s, 1H), 7.55 (s, 2H), 6.88 (s, 1H), 4.55-4.40 (m, 4H), 3.81 (s, 3H), 3.76 (s, 3H), 3.43-3.39 (m, 1H), 2.83-2.80 (m, 4H), 2.22-2.19 (m, 4H), 2.04 (s, 3H), 1.99 (s, 3H), 1.58-1.54 (m, 8H). | 787.0, 789.0 |
| 249 | 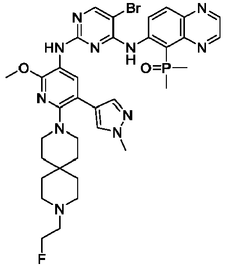 | ¹H NMR (300 MHz, CDCl₃) δ 12.59 (s, 1H), 8.97 (dd, J = 9.6, 4.2 Hz, 1H), 8.76-8.73 (m, 2H), 8.34 (s, 1H), 8.30 (s, 1H), 7.74 (d, *J* = 9.6 Hz, 1H), 7.60 (s, 1H), 7.58 (s, 1H), 7.17 (s, 1H), 4.86-4.83 (m, 1H), 4.70-4.67 (m, 1H), 4.02 (s, 3H), 3.80 (s, 3H), 3.09-3.06 (m, 5H), 3.01-3.29 (m, 1H), 2.92-2.82 (m, 4H), 2.18 (s, 3H), 2.13 (s, 3H), 1.81-1.77 (m, 4H), 1.63-1.60 (m, 4H). | 778.2, 780.2 |
| | | ¹⁹F NMR (282 MHz, CDCl₃) δ -216.61. | |

### Biological test evaluation:

### (I) enzymology experiments in vitro

In this experiment, the method of fluorescence resonance energy transfer was used to test the inhibitory effect of the compounds on the kinase activity of wild-type EGFR and L858R/T790M/C797S triple mutant EGFR, and the half inhibitory concentration IC₅₀ of the compounds on the kinase activity of the wild-type EGFR and the L858R/T790M/C797S triple mutant EGFR were obtained.

### 1. Experimental materials

Wild-type EGFR and L858R/T790M/C797S triple mutant EGFR recombinase, purchased from Signalchem.

HTRF KinEASE-TK kit detection reagent, purchased from Cisbio

Brigatinib, purchased from Selleck.

### 2. Experimental methods

1) 10 nL of gradient diluted compound was transferred into a 384-well experimental plate using Echo 550 (Labcyte).
2) 5 µL of 2 × EGFR enzyme solution was added to the 384-well experimental plate and incubated at room temperature for 10 minutes.
3) 5 µL of 2 × substrate solution containing polypeptide and ATP was added to the 384-well experimental plate and incubated at room temperature for 40 minutes.
4) 10 µL of detection solution containing EDTA, XL665-labeled streptavidin and Eu³⁺-labeled antibody was added thereto and incubated at room temperature for 1 hour.
5) Envision microplate reader (PerkinElmer) detected the fluorescence signal values at 615 nm and 665 nm of each well.
6) The ratio of fluorescence signal 665nm/615nm per well was calculated.
7) GraphPad Prism 8 software was used for data analysis to obtain the IC₅₀ of the compounds of the present disclosure and the comparative embodiment Brigatinib.

The results of kinase activity inhibition of wild-type EGFR and L858R/T790M/C797S triple mutant EGFR are shown in Table 2.

It can be seen from Table 2 that the compounds of the present disclosure have a good inhibitory effect on EGFR (L858R/T790M/C797S) kinase, a weak inhibitory effect on wild-type EGFR, and good selectivity.

**Table 2. Enzymology inhibition results**

| Compound | EGFR (WT) IC₅₀(nM) | EGFR (L858R/T790M/C797S) IC₅₀(nM) |
|---|---|---|
| 1 | 0.94 | 0.12 |
| 2 | 17.98 | 0.09 |
| 3 | 1252 | 21.96 |
| 4 | 1.41 | 0.28 |
| 5 | 8.13 | 0.03 |
| 6 | 0.17 | <0.05 |
| 7 | 9.88 | 0.23 |
| 8 | 357.60 | 3.19 |
| 9 | 75.73 | 0.88 |
| 10 | 4.55 | 0.10 |
| 11 | 12.09 | 0.07 |
| 12 | 1.59 | 0.05 |
| 13 | 7.62 | 0.890 |
| 14 | 1.060 | 0.15 |
| 15 | 12.15 | <0.05 |
| 16 | 0.51 | 0.05 |
| 17 | 3.61 | 0.08 |
| 18 | 1.26 | 0.08 |
| 19 | 39.34 | 0.17 |
| 20 | 178.70 | 9.21 |
| 21 | 1.57 | 0.06 |
| 23 | 40.34 | 1.92 |
| 24 | 346.30 | 11.38 |
| 25 | 18.67 | 0.19 |
| 26 | 5.75 | 0.49 |
| 28 | 15.41 | 0.13 |
| 29 | 3.37 | 0.06 |
| 30 | 55.73 | 1.01 |
| 31 | 0.38 | 0.25 |
| 32 | 3.07 | 0.22 |
| 33 | 50.19 | 0.26 |
| 34 | 15.16 | 0.27 |
| 35 | 383.90 | 11.88 |
| 36 | 21.91 | 0.54 |
| 37 | 268.20 | 3.18 |
| 38 | 17.77 | 0.23 |
| 39 | 7.45 | 0.06 |
| 40 | 55.26 | 2.85 |
| 41 | 21.12 | 0.31 |
| 42 | 165.7 | 1.242 |
| 43 | 95.29 | 0.47 |
| 44 | 1558 | 22.0 |
| 45 | n.d. | n.d. |
| 46 | n.d. | n.d. |
| 47 | 20.46 | 0.37 |
| 48 | n.d. | n.d. |
| 49 | 21.47 | 0.12 |
| 50 | n.d. | n.d. |
| 51 | 111.10 | 0.11 |
| 52 | n.d. | n.d. |
| 53 | 87.03 | 3.60 |
| 54 | n.d. | n.d. |
| 55 | n.d. | n.d. |
| 59 | n.d. | n.d. |
| 60 | n.d. | n.d. |
| 61 | n.d. | n.d. |
| 62 | n.d. | n.d. |
| 63 | n.d. | n.d. |
| 65 | n.d. | n.d. |
| 66 | n.d. | n.d. |
| 67 | n.d. | n.d. |
| 68 | n.d. | n.d. |
| 69 | 17.36 | 0.17 |
| 70 | n.d. | n.d. |
| 71 | 38.86 | 1.70 |
| 72 | 4.69 | 0.20 |
| 73 | n.d. | n.d. |
| 76 | n.d. | n.d. |
| 77 | 113.70 | 1.24 |
| 78 | 45.53 | 0.53 |
| 79 | 29.85 | 0.45 |
| 80 | 8.85 | 0.01 |
| 81 | 7.68 | 0.34 |
| 82 | 18.19 | 0.28 |
| 83 | 1000.00 | 3.95 |
| 84 | 16.56 | 0.33 |
| 85 | 9.06 | 0.11 |
| 86 | 344.60 | 15.62 |
| 87 | 3.47 | 0.35 |
| 88 | 4.26 | 0.09 |
| 89 | 2.11 | 0.11 |
| 90 | n.d. | n.d. |
| 91 | n.d. | n.d. |
| 92 | 2.34 | 0.03 |
| 93 | 23.11 | 0.18 |
| 94 | 2.78 | 0.15 |
| 95 | n.d. | n.d. |
| 96 | n.d. | n.d. |
| 97 | 1.02 | 0.15 |
| 98 | 16.21 | 0.82 |
| 99 | n.d. | n.d. |
| 100 | n.d. | n.d. |
| 101 | 0.41 | 0.18 |
| 102 | 1.49 | 0.23 |
| 103 | 1.57 | 0.13 |
| 104 | 27.21 | 0.95 |
| 105 | 5.62 | 0.16 |
| 107 | 26.37 | 1.30 |
| 108 | n.d. | n.d. |
| 109 | n.d. | n.d. |
| 110 | 1.54 | 0.57 |
| 111 | 5.65 | 1.38 |
| 112 | 0.92 | 0.26 |
| 113 | n.d. | n.d. |
| 114 | 5.41 | 0.56 |
| 115 | 1.65 | 0.40 |
| 116 | 3.13 | 1.31 |
| 117 | n.d. | n.d. |
| 119 | 4.07 | 1.30 |
| 120 | 9.84 | 1.17 |
| 121 | 0.67 | 0.25 |
| 122 | 3.42 | 0.62 |
| 123 | 120.70 | 1.12 |
| 124 | 8.35 | 1.20 |
| 125 | 23.77 | 0.53 |
| 126 | 1.55 | 0.15 |
| 127 | 2.83 | 0.24 |
| 128 | 6.83 | 0.53 |
| 129 | 1.11 | 0.27 |
| 130 | 1.44 | 0.05 |
| 131 | 2.57 | 0.12 |
| 132 | 5.71 | 0.07 |
| 133 | 0.78 | 0.15 |
| 134 | 3.57 | 0.21 |
| 135 | 15.58 | 0.47 |
| 136 | 99.50 | 5.3 |
| 137 | 170.40 | 9.81 |
| 138 | 30.70 | 0.53 |
| 139 | 0.57 | 0.24 |
| 140 | 94.56 | 5.44 |
| 141 | 1.14 | 0.18 |
| 142 | 1.16 | 0.25 |
| 143 | 1.75 | 0.41 |
| 144 | 2.77 | 0.66 |
| 145 | 3.48 | 0.31 |
| 146 | 8.43 | 0.38 |
| 147 | 53.02 | 1.16 |
| 148 | 5.04 | 0.64 |
| 149 | 78.50 | 3.36 |
| 150 | 2.45 | 0.29 |
| 151 | 38.16 | 2.20 |
| 152 | 4.24 | 0.35 |
| 153 | 3.02 | 0.22 |
| 154 | 36.87 | 0.86 |
| 155a | 27.73 | 0.67 |
| 155b | 21.63 | 0.80 |
| 156 | 33.49 | 3.81 |
| 157 | 3.60 | 0.33 |
| 158 | 2.48 | 0.81 |
| 159 | 1.54 | 0.70 |
| 160 | 2.32 | 0.26 |
| 161 | 2.71 | 0.39 |
| 162 | 20.55 | 2.28 |
| 163 | 1.89 | 0.28 |
| 164 | 5.30 | 0.73 |
| 165 | 1.36 | 0.69 |
| 166 | 3.81 | 0.48 |
| 167 | 1.95 | 0.79 |
| 168 | 2.57 | 0.35 |
| 169 | 4.39 | 0.73 |
| 170 | 4.94 | 0.45 |
| 171 | 4.59 | 0.29 |
| 172 | 0.69 | 0.14 |
| 173 | 50.66 | 6.00 |
| 174 | 2.39 | 0.60 |
| 175 | 3.10 | 0.33 |
| 176 | 3.26 | 0.34 |
| 177 | n.d. | n.d. |
| 178 | 7.68 | 1.29 |
| 179 | 1.48 | 0.40 |
| 180 | 1.40 | 0.36 |
| 181 | 3.53 | 0.16 |
| 182 | 8.49 | 0.40 |
| 183 | 12.19 | 1.12 |
| 184 | 2.23 | 0.24 |
| 185 | 2.42 | 0.36 |
| 186 | 1.19 | 0.37 |
| 187 | 5.73 | 0.04 |
| 188 | 5.62 | 0.43 |
| 189 | 1.08 | 0.16 |
| 190 | 4.37 | 0.53 |
| 191 | 2.44 | 0.23 |
| 193 | 0.99 | 0.27 |
| 194 | 2.85 | 0.17 |
| 195 | 8.52 | 2.33 |
| 196 | 3.35 | 0.59 |
| 197 | 0.62 | 0.25 |
| 198 | 0.62 | 0.22 |
| 199 | 2.57 | 0.20 |
| 200 | 7.56 | 0.19 |
| 201 | 6.92 | 0.74 |
| 202 | 3.74 | 0.59 |
| 203 | 21.21 | 0.47 |
| 204 | 1.57 | 0.25 |
| 205 | 3.08 | 0.35 |
| 206 | 8.46 | 0.62 |
| 207 | 8.43 | 1.14 |
| 208 | 5.79 | 0.57 |
| 209 | 9.74 | 0.73 |
| 210 | 8.41 | 0.11 |
| 211 | 10.93 | 0.61 |
| 212 | 18.95 | 0.33 |
| 213 | 1.02 | 0.18 |
| 214 | 1.86 | 0.36 |
| 215 | 7.3 | 0.25 |
| 216 | 1.71 | 0.29 |
| 217 | 3.87 | 0.95 |
| 218 | 1.82 | 0.29 |
| 219 | 1.66 | 0.12 |
| 220 | 2.29 | 0.12 |
| 221 | 1.37 | 0.09 |
| 222 | 4.22 | 0.30 |
| 223 | 7 | 0.17 |
| 224 | 10.66 | 4.31 |
| 225 | 10.77 | 1.96 |
| 226 | 1.40 | 0.19 |
| 227 | 2.69 | 0.59 |
| 228 | 1.04 | 0.48 |
| 229 | 8.53 | 0.42 |
| 230 | 12 | 0.29 |
| 231 | 1.12 | 0.37 |
| 232 | 4.11 | 0.58 |
| 233 | 2.49 | 0.26 |
| 234a | 5.04 | 0.32 |
| 234b | 5.44 | 0.37 |
| 235 | 10.49 | 0.27 |
| 236 | 10.95 | 0.21 |
| 237 | 5.20 | 0.52 |
| 238 | 3.02 | 0.30 |
| 239 | 7.57 | 1.60 |
| 240 | 29.93 | 0.51 |
| 241 | 30.06 | 0.13 |
| 242 | 2.40 | 0.40 |
| 243 | 4.91 | 0.54 |
| 244 | 2.74 | 0.78 |
| 245 | 105.4 | 2.80 |
| 246 | 1.05 | 0.40 |
| 247 | 5.81 | 0.12 |
| 248 | 1.91 | 0.29 |
| 249 | 3.31 | 0.40 |
| Brigatinib | 166.3 | 11.5 |

| | | |
|---|---|---|
| n.d. stands for not determined. | | |

### (II) Cell proliferation inhibition experiment

In this experiment, the method of CellTiter-Glo was used to test the inhibitory effect of compounds on cell proliferation of EGFR wild-type cell line A431 and Ba/F3 Del19/T790M/C797S EGFR triple mutant cell line and Ba/F3 L858R/T790M/C797S EGFR triple mutant cell line, and the half concentration IC₅₀ of the compounds inhibiting cell growth were obtained.

### 1 Experimental materials

A431 cells, purchased from ATCC.
Ba/F3 Del19/T790M/C797S EGFR triple mutant cells, purchased from KYinno Biotechnology (Beijing) Co., Ltd.;
Ba/F3 L858R/T790M/C797S EGFR triple mutant cells, purchased from Pharmaron (Beijing) New Drug Technology Co., Ltd.;
DMEM medium, purchased from Thermo Fisher.
RPMI 1640 medium, purchased from Thermo Fisher.
Fetal bovine serum (FBS), purchased from Thermo Fisher.
CellTiter-Glo reagent, purchased from Promega.
Brigatinib, purchased from Selleck.

### 2 Experimental methods

1) A431 cells were inoculated in a 384-well culture plate at a density of 800 cells per well, 30 µL per well, placed in a cell incubator, and cultured for 24 hours (37 °C, 5 % CO₂).
2) Ba/F3 Del19/T790M/C797S EGFR triple mutant cells and Ba/F3 L858R/T790M/C797S EGFR triple mutant cells were inoculated in the 384-well culture plate at a density of 700 cells per well, 30 µL per well, and placed in a cell incubator.
3) 30 nL of gradient diluted compound was transferred into the 384-well experimental plate using Echo 550 (Labcyte) at a DMSO final concentration of 0.1 %, and the culture plate was incubated in a cell incubator for 72 hours (37 °C, 5 % CO₂).
4) 30 µL of Cell Titer-Glo reagent was added to each well and left for 30 minutes at room temperature and protected from light.
5) Envision microplate reader (PerkinElmer) detected chemiluminescence signals.
6) GraphPad Prism 8 software was used for data analysis to obtain the IC₅₀ of the compounds of the present disclosure and the positive control.

The cell viability inhibition results are shown in Table 3.

It can be seen from the experimental results in Table 3 that, compared with the comparative embodiment Brigatinib, the compounds of the present disclosure have a good inhibitory effect on the cell proliferation of Ba/F3 Del19/T790M/C797S EGFR triple mutant cell line and Ba/F3 L858R/T790M/C797S EGFR triple mutant cell line, and the IC₅₀ of some compounds are lower than 1.0 nM, showing a very good inhibitory effect; showing a weak inhibitory effect on EGFR wild-type cell line A431, and good selectivity.

**Table 3. Results of cell proliferation inhibition test data**

| **Compound** | **A431 EGFR WT IC₅₀(nM)** | **Ba/F3 (Del19/T790M/C797S) IC₅₀(nM)** | **Ba/F3 (L858R/T790M/C797S) IC₅₀(nM)** |
|---|---|---|---|
| 1 | 136.70 | 0.58 | 5.61 |
| 2 | 315.50 | 4.21 | 17.49 |
| 3 | 2236 | 281.1 | 443.6 |
| 4 | 193.50 | 2.38 | 13.58 |
| 5 | 140.00 | 3.71 | 12.66 |
| 6 | 24.51 | 1.99 | 5.96 |
| 7 | 158.90 | 4.43 | 14.77 |
| 8 | 769.20 | 68.95 | 136.70 |
| 9 | 450.30 | 55.04 | 110.00 |
| 10 | 47.03 | 6.06 | 10.71 |
| 11 | 159.25 | 83.42 | 223.6 |
| 12 | 76.14 | 6.32 | 45.85 |
| 13 | 398.40 | 43.35 | 84.28 |
| 14 | 150.10 | 5.98 | 13.61 |
| 15 | 177.00 | 4.62 | 15.59 |
| 16 | 51.32 | 13.18 | 23.46 |
| 17 | 67.82 | 1.28 | 2.13 |
| 18 | 45.44 | 2.87 | 20.91 |
| 19 | 488.00 | 6.71 | 68.85 |
| 20 | 1586.27 | 276.7 | 629.09 |
| 21 | 63.91 | 4.03 | 18.07 |
| 23 | 194.80 | 8.67 | 30.30 |
| 24 | 567.20 | 45.24 | 139.20 |
| 25 | 159.00 | 2.41 | 8.64 |
| 26 | 152.10 | 1.22 | 4.25 |
| 28 | 361.50 | 4.73 | 13.73 |
| 29 | 121.30 | 1.29 | 4.25 |
| 30 | 284.50 | 12.12 | 62.60 |
| 31 | 194.30 | 4.58 | 12.81 |
| 32 | 208.10 | 0.98 | 5.45 |
| 33 | 1424.00 | 10.10 | 32.45 |
| 34 | 462.50 | 2.04 | 8.94 |
| 35 | 752.1 | 93.23 | 326.8 |
| 36 | 320.30 | 2.63 | 17.73 |
| 37 | 1141.00 | 32.61 | 65.56 |
| 38 | 295.40 | 10.21 | 32.60 |
| 39 | 41.27 | 4.07 | 9.69 |
| 40 | 1180 | 1131.1 | 542.4 |
| 41 | 371.00 | 8.64 | 34.48 |
| 42 | 5000.00 | 6.76 | 69.16 |
| 43 | 880.20 | 16.42 | 29.70 |
| 44 | 5000 | 93.45 | n.d. |
| 45 | 102.40 | 7.65 | n.d. |
| 46 | 67.2 | 6.71 | 11.68 |
| 47 | 126.40 | 4.31 | n.d. |
| 48 | 51.07 | 3.0235 | 6.03 |
| 49 | 233.60 | 4.88 | n.d. |
| 50 | 507.70 | 34.13 | n.d. |
| 51 | 292.10 | 911.56 | 36.84 |
| 52 | 158.40 | 26.73 | n.d. |
| 53 | 293.60 | 62.24 | n.d. |
| 54 | 638.1 | 102.6 | n.d. |
| 55 | 5000 | 1228 | n.d. |
| 59 | 345.00 | 26.24 | n.d. |
| 60 | 658.2 | 89.35 | n.d. |
| 61 | 6580.5 | 312.05 | 794.15 |
| 62 | 3380.00 | 73.60 | 195.10 |
| 63 | 331.30 | 14.30 | 24.95 |
| 65 | 72.44 | 6.68 | 17.32 |
| 66 | 575 | 3.04 | 10.78 |
| 67 | 622.05 | 42.26 | 59.16 |
| 68 | 1393.00 | 113.90 | 372.50 |
| 69 | 303.30 | 5.91 | 19.18 |
| 70 | 156.85 | 116.80 | 348.40 |
| 71 | >10000.00 | 99.38 | 316.20 |
| 72 | 369.50 | 4.54 | 13.47 |
| 73 | 3255.00 | 326.20 | 536.40 |
| 76 | 20.71 | 5.40 | 9.96 |
| 77 | 479.50 | 14.42 | 26.39 |
| 78 | 549.60 | 6.76 | 14.44 |
| 79 | 632.90 | 9.89 | 13.53 |
| 80 | 414.90 | 5.37 | 10.13 |
| 81 | 10000.00 | 19.12 | 32.35 |
| 82 | 179.90 | 7.85 | 21.72 |
| 83 | 2418.00 | 34.05 | 58.88 |
| 84 | 392.10 | 12.54 | 20.74 |
| 85 | 380.80 | 6.15 | 9.29 |
| 86 | 3065.00 | 87.77 | 136.10 |
| 87 | 178.10 | 5.89 | 14.25 |
| 88 | 40.85 | 2.18 | 4.45 |
| 89 | 40.88 | 2.33 | 4.16 |
| 90 | 4389.00 | 250.70 | 446.10 |
| 91 | 3549.00 | 278.20 | 430.10 |
| 92 | 31.84 | 0.54 | 2.85 |
| 93 | 178.20 | 12.49 | 27.03 |
| 94 | 167.70 | 6.99 | 28.82 |
| 95 | 96.72 | 15.70 | 40.93 |
| 96 | 44.21 | 3.52 | 9.39 |
| 97 | 405.50 | 6.64 | 25.48 |
| 98 | 177.10 | 10.95 | 27.75 |
| 99 | 208.80 | 40.61 | 103.50 |
| 100 | 29.06 | 2.87 | 6.20 |
| 101 | 46.11 | 2.87 | 4.53 |
| 102 | 107.80 | 0.76 | 2.02 |
| 103 | 152.40 | 0.51 | 5.02 |
| 104 | 191.90 | 4.07 | 17.71 |
| 105 | 1187.00 | 20.91 | 61.88 |
| 107 | 380.10 | 11.20 | 30.92 |
| 108 | 92.75 | 12.48 | 24.48 |
| 109 | 546.90 | 46.28 | 62.79 |
| 110 | 161.80 | 3.70 | 10.88 |
| 111 | 209.60 | 3.22 | 4.79 |
| 112 | 1096.00 | 57.03 | 161.00 |
| 113 | 189.20 | 74.47 | 175.70 |
| 114 | 270.60 | 5.86 | 11.20 |
| 115 | 159.00 | 6.53 | 16.06 |
| 116 | 169.40 | 2.25 | 8.15 |
| 117 | 157.10 | 41.00 | 120.40 |
| 119 | 639.10 | 36.56 | 239.40 |
| 120 | 196.80 | 2.62 | 8.67 |
| 121 | 150.50 | 1.61 | 6.16 |
| 122 | 37.22 | 2.07 | 9.96 |
| 123 | 8835.00 | 24.32 | 82.00 |
| 124 | 515.70 | 3.93 | 7.93 |
| 125 | 489.50 | 5.87 | 14.45 |
| 126 | 285.90 | 0.93 | 3.81 |
| 127 | 170.30 | 0.59 | 4.18 |
| 128 | 397.40 | 2.86 | 11.88 |
| 129 | 86.24 | 0.72 | 2.71 |
| 130 | 73.46 | 1.00 | 9.00 |
| 131 | 67.77 | 0.60 | 7.17 |
| 132 | 270.40 | 2.45 | 30.03 |
| 133 | 82.75 | 0.64 | 2.85 |
| 134 | 315.90 | 1.02 | 7.82 |
| 135 | 402.00 | 3.68 | 13.34 |
| 136 | 1748.00 | 13.67 | 57.09 |
| 137 | 1495.00 | 27.09 | 77.82 |
| 138 | 629.50 | 9.31 | 11.90 |
| 139 | 61.00 | 1.59 | 2.33 |
| 140 | 3670.00 | 29.76 | 61.99 |
| 141 | 194.00 | 2.26 | 5.16 |
| 142 | 196.40 | 0.34 | 2.89 |
| 143 | 420.40 | 3.23 | 10.16 |
| 144 | 84.04 | 0.35 | 1.91 |
| 145 | 202.70 | 1.70 | 2.41 |
| 146 | 178.80 | 1.44 | 5.47 |
| 147 | 475.30 | 1.30 | 2.93 |
| 148 | 123.80 | 0.70 | 2.78 |
| 149 | 376.40 | 1.62 | 3.21 |
| 150 | 138.60 | 1.57 | 3.34 |
| 151 | 318.40 | 1.84 | 7.47 |
| 152 | 133.30 | 1.06 | 4.59 |
| 153 | 61.85 | 1.03 | 2.89 |
| 154 | 469.20 | 5.26 | 15.39 |
| 155a | 320.20 | 2.98 | 15.56 |
| 155b | 212.80 | 1.63 | 7.55 |
| 156 | 372.00 | 1.05 | 7.29 |
| 157 | 49.38 | 1.28 | 7.05 |
| 158 | 165.50 | 3.28 | 6.76 |
| 159 | 113.90 | 0.93 | 4.11 |
| 160 | 207.90 | 1.56 | 9.04 |
| 161 | 153.70 | 2.36 | 6.39 |
| 162 | 517.90 | 6.20 | 17.96 |
| 163 | 71.81 | 0.62 | 1.97 |
| 164 | 195.00 | 3.22 | 8.26 |
| 165 | 70.17 | 0.80 | 2.62 |
| 166 | 92.07 | 1.58 | 5.05 |
| 167 | 47.44 | 2.97 | 4.34 |
| 168 | 67.39 | 0.71 | 4.20 |
| 169 | 175.00 | 2.36 | 4.24 |
| 170 | 280.30 | 1.80 | 8.14 |
| 171 | 622.60 | 2.13 | 7.23 |
| 172 | 329.10 | 101.70 | 328.10 |
| 173 | 737.70 | 11.20 | 24.52 |
| 174 | 307.40 | 4.63 | 7.85 |
| 175 | 41.28 | 1.70 | 5.38 |
| 176 | 61.85 | 2.97 | 5.96 |
| 177 | 59.26 | 25.62 | 35 |
| 178 | 168.50 | 2.86 | 13.02 |
| 179 | 54.33 | 1.37 | 6.84 |
| 180 | 64.75 | 4.13 | 6.53 |
| 181 | 88.93 | 3.52 | 9.67 |
| 182 | 100.60 | 1.71 | 4.43 |
| 183 | 246.40 | 14.41 | 27.05 |
| 184 | 218.10 | 2.28 | 5.67 |
| 185 | 127.00 | 1.77 | 4.02 |
| 186 | 61.99 | 1.39 | 3.47 |
| 187 | 245.30 | 2.11 | 8.73 |
| 188 | 56.60 | 0.61 | 0.87 |
| 189 | 128.10 | 9.18 | 23.62 |
| 190 | 193.70 | 4.16 | 8.69 |
| 191 | 70.02 | 3.47 | 11.37 |
| 193 | 59.93 | 4.48 | 9.83 |
| 194 | 79.47 | 5.56 | 16.36 |
| 195 | 415.10 | 7.70 | 12.46 |
| 196 | 355.60 | 12.32 | 20.80 |
| 197 | 95.59 | 7.70 | 14.29 |
| 198 | 37.99 | 4.26 | 9.49 |
| 199 | 154.10 | 7.08 | 18.43 |
| 200 | 69.61 | 7.47 | 21.99 |
| 201 | 120.20 | 2.22 | 3.70 |
| 202 | 91.26 | 2.93 | 3.75 |
| 203 | 351.40 | 10.56 | 19.98 |
| 204 | 66.88 | 5.19 | 11.18 |
| 205 | 116.30 | 3.20 | 8.90 |
| 206 | 297.70 | 7.62 | 30.22 |
| 207 | 435.80 | 12.34 | 37.50 |
| 208 | 348.00 | 9.45 | 14.57 |
| 209 | 171.40 | 7.18 | 12.57 |
| 210 | 274.60 | 4.42 | 7.23 |
| 211 | 354.10 | 14.26 | 24.85 |
| 212 | 179.20 | 9.29 | 25.05 |
| 213 | 162.90 | 3.36 | 8.39 |
| 214 | 138.60 | 1.47 | 4.87 |
| 215 | 226.80 | 2.23 | 5.58 |
| 216 | 45.67 | 2.06 | 7.32 |
| 217 | 60.55 | 0.94 | 3.29 |
| 218 | 69.22 | 5.20 | 20.12 |
| 219 | 32.07 | 1.10 | 1.08 |
| 220 | 77.68 | 4.04 | 4.37 |
| 221 | 26.21 | 2.41 | 2.79 |
| 222 | 70.41 | 1.22 | 2.02 |
| 223 | 76.90 | 2.88 | 3.56 |
| 224 | 336.00 | 8.88 | 13.18 |
| 225 | 370.20 | 3.54 | 11.32 |
| 226 | 47.36 | 1.55 | 4.34 |
| 227 | 34.70 | 2.78 | 6.64 |
| 228 | 44.84 | 4.58 | 8.50 |
| 229 | 163.80 | 3.76 | 12.11 |
| 230 | 126.20 | 4.47 | 10.14 |
| 231 | 40.13 | 1.08 | 1.12 |
| 232 | 126.20 | 3.16 | 8.59 |
| 233 | 79.55 | 1.74 | 3.54 |
| 234a | 42.36 | 2.05 | 3.91 |
| 234b | 38.37 | 2.52 | 5.65 |
| 235 | 88.97 | 8.53 | 24.80 |
| 236 | 91.49 | 5.45 | 12.72 |
| 237 | 128.80 | 1.26 | 3.05 |
| 238 | 61.91 | 1.83 | 3.59 |
| 239 | 141.10 | 2.10 | 4.61 |
| 240 | 303.60 | 8.20 | 19.19 |
| 241 | 181.80 | 2.92 | 12.34 |
| 242 | 75.72 | 1.36 | 3.07 |
| 243 | 140.90 | 1.73 | 3.95 |
| 244 | 124.80 | 1.19 | 2.43 |
| 245 | 747.30 | 5.35 | 11.59 |
| 246 | 128.30 | 2.59 | 3.64 |
| 247 | 66.41 | 3.76 | 7.76 |
| 248 | 84.71 | 1.17 | 2.37 |
| 249 | 84.97 | 2.55 | 8.95 |
| Brigatinib | 771.10 | 103.00 | 319.80 |

### (III) In vivo efficacy research experiment A

### 1. Experimental objectives

To evaluate the antitumor activity and toxic side effects of compound 242 orally administered for 21 days in PC9 (Del19/T790M/C797S) nude mice xenograft model.

### 2. Experimental Materials

BALB/c-nu mice, female, SPF grade, purchased from Beijing Vital River Laboratory Animal Technology Co., Ltd.

PC9 (Del19/T790M/C797S) cells, constructed by Qilu Pharmaceutical Co., Ltd.

### 3. Experimental steps

### 3.1 Cell culture

PC9 (Del19/T790M/C797S) cells were cultured in RPMI 1640 medium containing 10 % FBS at 37 °C in a 5 % carbon dioxide incubator; cells in exponential growth phase were collected and inoculated.

### 3.2 Cell inoculation

Under sterile conditions, PC9 (Del19/T790M/C797S) cell suspension cultured *in vitro* was taken, the cell concentration was adjusted to 5×10⁷ cells /mL after centrifugation, and subcutaneously inoculated under the right armpit of mice (0.1 mL/ mouse). The day of inoculation was set as day 0.

### 3.3 Tumor grouping, administration and measurement

a, When the average tumor volume was about 180 mm³, 24 mice with moderate tumor volume were selected and randomly divided into 3 groups according to the tumor volume: G1: solvent control group, G2: compound 242 (25 mg/kg) and G3 : compound 242 (75/50 mg/kg), 8 mice/group.
b, After animals were grouped, administration was started, and the administration volume was 10 mL/kg, administered orally (po); administered by weight once a day for 21 consecutive days; tumor diameter was measured twice a week.
c, Tumor volume (TV): the tumor volume was measured twice a week to observe the volume change and growth rate of the tumor. Tumor volume V=1/2×a×b², where a and b represent the long and short diameters of the tumor, respectively. The growth inhibition effect of the compounds on tumor tissue was evaluated by tumor growth inhibition rate TGI (%). TGI (%)=[1-(average tumor volume of a certain administration group - average tumor volume of the administration group on the day of grouping) / (average tumor volume of a negative control group - average tumor volume of the negative control group on the day of grouping)] ×100 %. The data of the administration group and the negative control group were taken on the same day.
d, During the experiment, the living conditions of the mice, including appearance signs, general behavior, mental state, food intake, respiratory state, feces and urine characteristics, local injection site and other toxicity manifestations, were closely observed.
e, After the experiment reached the end point, the mice were euthanized, and the animal carcasses were frozen in a freezer and handed over to a qualified medical waste disposal unit for disposal.

### 4 Experimental results

**Table 4. Tumor inhibition experiment results of compound 242**

| Grouping | Dose (mg/kg) | Tumor volume^{a} (mm3) | TGI (%) | Weight change (%) |
|---|---|---|---|---|
| G1: solvent control group | - | 2489.2±314.0 | - | 11.7 |
| G2: Compound 242 | 25 | 139.8±13.7^{∗∗} | 102 | 2.4 |
| G3: Compound 242 | 75 (D1-D6), 50(D7-D21) | 64.0±11.9^{∗∗} | 105 | -3.4 |

| | | | | |
|---|---|---|---|---|
| a, mean ± standard error; b, P value was statistically analyzed according to tumor volume, compared with G1 group, *P < 0.05; **P < 0.01. | | | | |

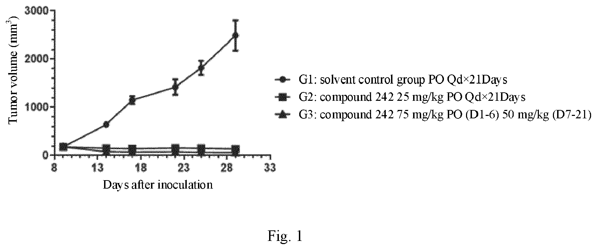

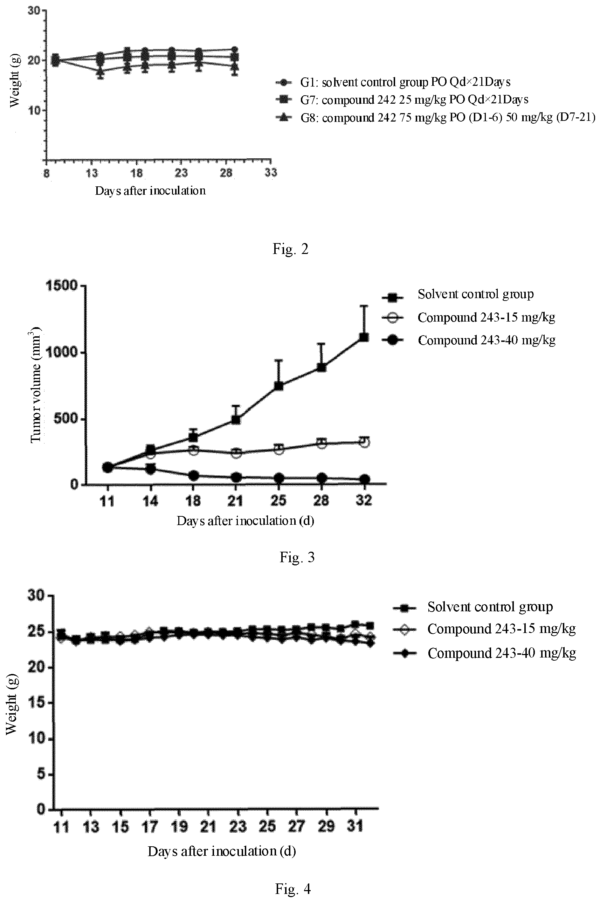

The graph of tumor growth curves of animals in each group is shown in Fig. 1, and the graph of weight curves of animals in each group is shown in Fig. 2.

### 5 Experimental conclusions

It can be seen from the above results that compound 242 (25, 75/50 mg/kg) can significantly inhibit tumor growth, showing good anti-tumor efficacy. The mice are well tolerated at 25 mg/kg and 50 mg/kg doses.

### (IV) In vivo efficacy research experiment B

### 1. Experimental objectives

To evaluate the antitumor activity and toxic side effects of compound 243 orally administered for 21 days in PC9 (Del19/T790M/C797S) nude mice xenograft model.

### 2. Experimental Materials

BALB/c-nu mice, female, SPF grade, purchased from Beijing Vital River Laboratory Animal Technology Co., Ltd.

PC9 (Del19/T790M/C797S) cells, constructed by Qilu Pharmaceutical Co., Ltd.

### 3. Experimental steps

### 3.1 Cell culture

PC9 (Del19/T790M/C797S) cells were cultured in RPMI 1640 medium containing 10 % FBS at 37 °C in a 5 % carbon dioxide incubator; cells in exponential growth phase were collected and inoculated.

### 3.2 Cell inoculation

Under sterile conditions, PC9 (Del19/T790M/C797S) cell suspension cultured *in vitro* was taken, the cell concentration was adjusted to 3×10⁷ cells/mL after centrifugation, and subcutaneously inoculated under the right armpit of mice (0.1 mL/ mouse). The day of inoculation was set as day 0.

### 3.3 Tumor grouping, administration and measurement

a, When the average tumor volume was about 140 mm³, 21 mice with moderate tumor volume were selected and randomly divided into 5 groups according to the tumor volume: G1: solvent control group, G2: compound 243 (15 mg/kg) and G3: compound 243 (40 mg/kg), 7 mice/group.
b, After animals were grouped, administration was started, and the administration volume was 10 mL/kg, administered orally (po); administered by weight once a day for 21 consecutive days; tumor diameter was measured twice a week.
c, Tumor volume (TV): the tumor volume was measured twice a week to observe the volume change and growth rate of the tumor. Tumor volume V=1/2×a×b², where a and b represent the long and short diameters of the tumor, respectively. The growth inhibition effect of the compounds on tumor tissue was evaluated by tumor growth inhibition rate TGI (%). TGI (%)=[1-(average tumor volume of a certain administration group - average tumor volume of the administration group on the day of grouping) / (average tumor volume of a negative control group - average tumor volume of the negative control group on the day of grouping)] ×100 %. The data of the administration group and the negative control group were taken on the same day.
d, During the experiment, the living conditions of the mice, including appearance signs, general behavior, mental state, food intake, respiratory state, feces and urine characteristics, local injection site and other toxicity manifestations, were closely observed.
e, After the experiment reached the end point, the mice were euthanized, and the animal carcasses were frozen in a freezer and handed over to a qualified medical waste disposal unit for disposal.

### 4 Experimental results

**Table 5. Tumor inhibition experimental data of compound 243**

| Grouping | Dose (mg/kg) | Tumor volume^{a} (mm3) | TGI (%) | Weight change (%) |
|---|---|---|---|---|
| G1: solvent control group | - | 1110±235.7 | - | 5.3 |
| G2: Compound 243 | 15 | 318.6±38.63^{∗∗} | 81.4 | -0.4 |
| G3: Compound 243 | 40 | 38.6±5.19^{∗∗} | 110.1 | -6.1 |

| | | | | |
|---|---|---|---|---|
| a, mean ± standard error; b, P value was statistically analyzed according to tumor volume, compared with G1 group, *P < 0.05; **P < 0.01. | | | | |

The graph of tumor growth curves of animals in each group is shown in Fig. 3, and the graph of weight curves of animals in each group is shown in Fig. 4.

### 5 Experimental conclusions

It can be seen from the above results that compound 243 (15, 40 mg/kg) can significantly inhibit tumor growth, and shows a significant dose-effect relationship, showing good anti-tumor efficacy. Mice are well tolerated.

## Claims

1. A compound represented by formula (I), a stereoisomer thereof, a tautomer thereof, a pharmaceutically acceptable salt thereof, a prodrug thereof, a hydrate thereof, a solvate thereof or an isotope-labeled derivative thereof, 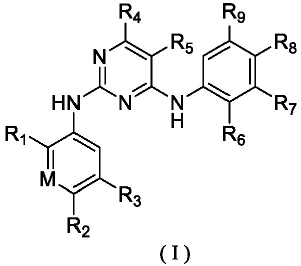 wherein,
R₁ is selected from H, halogen, -CN, C₁₋₆ alkyl, C₁₋₆ heteroalkyl, C₁₋₆ haloalkyl, C₁₋₆ haloalkoxy, C₃₋₆ cycloalkyl, 3-6-membered heterocycloalkyl, C₃₋₆ cycloalkyloxy, 3-6-membered heterocycloalkyloxy, and C₂₋₆ alkenyloxy;
M is selected from N or CR₁₀;
R₁₀ and R₁ may form a 5-8-membered heterocycloalkyl or a 5-7-membered heteroaryl; the 5-8-membered heterocycloalkyl and the 5-7-membered heteroaryl are optionally substituted by one or more R₁₁ groups;
R₂ is selected from H, halogen, -CN, -OH, -NH₂, phosphonyl, sulfonyl, aminosulfonyl, aminocarbonyl, carbonylamino, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, C₃₋₆ heterocycloalkenyl, phenyl and 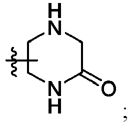 wherein, the -OH, -NH₂, phosphonyl, sulfonyl, aminosulfonyl, aminocarbonyl, carbonylamino, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, C₃₋₆ heterocycloalkenyl, phenyl and 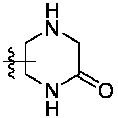 are optionally substituted by one or more R₁₂ groups;
R₃ is selected from -CN, sulfoximino, -L₁-C₆₋₁₀ aryl, -L₁-5-12-membered heteroaryl, -L₁-C₃₋₆ cycloalkenyl and 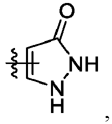 wherein, the -CN, sulfoximino, -L₁-C₆₋₁₀ aryl, -L₁-5-12-membered heteroaryl, -L₁-C₃₋₆ cycloalkenyl and 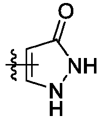 are optionally substituted by one or more R₁₃ groups;
L₁ is independently selected from a linking bond, C₁₋₄ alkyl and C₁₋₄ heteroalkyl, wherein the C₁₋₄ alkyl and C₁₋₄ heteroalkyl are optionally substituted by one or more groups selected from -OH, -NH₂ and halogen;
R₄ and R₅ are each independently selected from H, halogen, -CN, C₁₋₆ alkyl, C₁₋₆ alkoxy, C₁₋₆ haloalkyl, C₁₋₆ haloalkoxy, C₃₋₈ cycloalkyl and 3-8-membered heterocycloalkyl;
or, R₄ and R₅ are cyclized to 4-6-membered cycloalkyl, 4-6-membered heterocycloalkyl, 4-6-membered aryl or 4-6-membered heteroaryl;
R₆ is selected from amino, amido, aminocarbonyl, sulfonyl, thiophosphonyl, phosphonyl, phosphonoamino, sulfonylamino, aminosulfonyl and sulfoximino, wherein the amino, amido, aminocarbonyl, sulfonyl, thiophosphonyl, phosphonyl, phosphonoamino, sulfonylamino, aminosulfonyl and sulfoximino are optionally substituted by one or more R₁₄ groups;
R₇, R₈ and R₉ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6-membered heterocycloalkyl and 5-7-membered heteroaryl;
or, R₇ and R₈ are cyclized to C₄₋₆ cycloalkyl, 4-6-membered heterocycloalkyl, C₅₋₆ aryl or 5-7-membered heteroaryl;
or, R₈ and R₉ are cyclized to C₄₋₆ cycloalkyl, 4-6-membered heterocycloalkyl, C₅₋₆ aryl or 5-7-membered heteroaryl;
R₁₀ is selected from H, halogen, C₁₋₆ alkyl, C₁₋₆ alkoxy, C₁₋₆ haloalkyl and C₁₋₆ haloalkoxy;
R₁₁ is selected from H, halogen, C₁₋₆ alkyl, C₁₋₆ alkoxy, C₁₋₆ haloalkyl, C₁₋₆ haloalkoxy, -C₀₋₄ alkyl-NRₐR_{b}, -C₁₋₄ alkyl-O-C₁₋₄ alkyl, -C₁₋₄ alkyl-OH, -O-C₁₋₄ alkyl, -C₀₋₄ alkyl-C₃₋₆ cycloalkyl and -C₀₋₄ alkyl-3-6-member heterocycloalkyl;
R₁₂ is selected from H, halogen, -CN, -OH, -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₈ cycloalkyl, 3-14-membered heterocycloalkyl, hydroxy-C₁₋₆ alkyl-, C₃₋₈ cycloalkylalkyl-, C₃₋₈ cycloalkyloxy-, C₃₋₈ heterocycloalkylalkyl-, C₃₋₈ heterocycloalkyloxy-, C₃₋₈ cycloalkyl-C₁₋₆ alkoxy-, C₃₋₈ heterocycloalkyl-C₁₋₆ alkoxy-, C₁₋₆ alkylsulfonyl, C₃₋₆ cycloalkylsulfonyl, NRₐR_{b}CO-, C₁₋₆ alkylcarbonyl, C₃₋₈ cycloalkylcarbonyl, C₁₋₆ alkoxy-C₁₋₆ alkyl-, C₃₋₆ cycloalkenyl, 5-12-membered heteroaryl, C₆₋₁₀ aryl, NRₐR_{b}S(O)₂-, -(CH₂)ₘNRₐR_{b}, -(CH₂)ₘO(CH₂)ₙCH₃ and -O(CH₂)ₘNRₐR_{b}; wherein the Rₐ and R_{b} are independently H, C₁₋₆ alkyl or C₁₋₆ alkoxy, or the Rₐ and R_{b} together form a C₃₋₈ cycloalkyl or 3-8-membered heterocycloalkyl; wherein the m and n are independently optionally 0, 1, 2 or 3, and the C₃₋₈ cycloalkyl and 3-8-membered heterocycloalkyl are optionally substituted by a group selected from halogen, -OH, -NH₂, -CN, C₁₋₆ alkyl, C₁₋₆ alkoxy, C₁₋₆ haloalkyl and -C₀₋₄ alkyl-O-C₁₋₄ alkyl;
R₁₃ is selected from H, halogen, -CN, C₁₋₆ alkyl, C₁₋₆ heteroalkyl, C₁₋₆ haloalkyl, C₁₋₆ haloalkoxy, C₃₋₈ cycloalkyl, 3-8-membered heterocycloalkyl, C₃₋₆ cycloalkylsulfonyl, 5-6-membered heteroaryl and phenyl;
R₁₄ is selected from H, C₁₋₆ alkyl, C₁₋₆ alkoxy, C₁₋₆ haloalkyl, C₁₋₆ haloalkoxy, C₃₋₈ cycloalkyl and 3-8-membered heterocycloalkyl.

2. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in claim 1, wherein,
R₁ is selected from H, C₁₋₄ alkyl, C₁₋₄ alkoxy, C₁₋₄ haloalkyl and C₁₋₄ haloalkoxy;
M is selected from N or CR₁₀;
R₁₀ and R₁ may form a 5-8-membered heterocycloalkyl or a 5-7-membered heteroaryl; the 5-8-membered heterocycloalkyl and the 5-7-membered heteroaryl are optionally substituted by one or more R₁₁ groups;
R₂ is selected from H, halogen, -NH₂, phosphonyl, sulfonyl, aminosulfonyl, aminocarbonyl, C₃₋₁₄ cycloalkyl, 3-14-membered heterocycloalkyl, C₃₋₆ cycloalkenyl, C₃₋₆ heterocycloalkenyl and 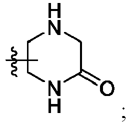 wherein, the -NH₂, phosphonyl, sulfonyl, aminosulfonyl, aminocarbonyl, C₃₋₁₄ cycloalkyl, 3-14-membered heterocycloalkyl, C₃₋₆ cycloalkenyl, C₃₋₆ heterocycloalkenyl and 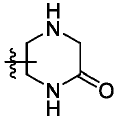 are optionally substituted by one or more R₁₂ groups;
R₃ is selected from -CN, 5-12-membered heteroaryl and 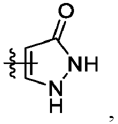 wherein, the -CN, 5-12-membered heteroaryl and 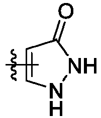 are optionally substituted by one or more R₁₃ groups;
R₄ and R₅ are each independently selected from H, halogen, -CN, C₁₋₄ alkyl, C₁₋₄ haloalkyl, and C₃₋₆ cycloalkyl;
or, R₄ and R₅ are cyclized to 4-6-membered aryl or 4-6-membered heteroaryl;
R₆ is selected from amino, amido, sulfonyl, thiophosphonyl, phosphonyl, sulfonylamino and aminosulfonyl, wherein the amino, amido, sulfonyl, thiophosphonyl, phosphonyl, sulfonylamino and aminosulfonyl are optionally substituted by one or more R₁₄ groups;
R₇, R₈ and R₉ are each independently selected from H and C₁₋₄ alkyl;
or, R₇ and R₈ are cyclized to C₄₋₆ cycloalkyl or 5-7-membered heteroaryl;
or, R₈ and R₉ are cyclized to C₄₋₆ cycloalkyl or 5-7-membered heteroaryl;
R₁₀ is selected from H, halogen and C₁₋₄ alkyl;
R₁₁ is selected from H, C₁₋₄ alkyl, -C₁₋₄ alkyl-NRₐR_{b} and 3-6-membered heterocycloalkyl;
R₁₂ is selected from H, halogen, -OH, -NH₂, C₁₋₆ alkyl, C₁₋₆ haloalkyl, C₃₋₈ cycloalkyl, 3-8-membered heterocycloalkyl, hydroxy-C₁₋₆ alkyl, 6-14-membered spiro heterocyclyl, -C₁₋₄ alkyl-O-C₁₋₄ alkyl, -O-C₁₋₄ alkyl, -C₁₋₄ alkyl-C₃₋₆ cycloalkyl, -O-C₀₋₄ alkyl-C₃₋₆ cycloalkyl, -C₁₋₄ alkyl-3-6-membered heterocycloalkyl, -O-C₀₋₄ alkyl-3-6-membered heterocycloalkyl, -(CH₂)ₘNRₐR_{b}, -O(CH₂)ₘNRₐR_{b}, C₁₋₆ alkylsulfonyl, C₃₋₆ cycloalkylsulfonyl, NRₐR_{b}CO-, C₁₋₆ alkylcarbonyl and C₃₋₆ cycloalkylcarbonyl, wherein the cycloalkyl and heterocycloalkyl are optionally substituted by R_{ab}, and the R_{ab} is selected from H, halogen, -OH, -NH₂, -CN, C₁₋₄ alkyl, C₁₋₄ alkoxy, C₁₋₄ haloalkyl and -C₀₋₄ alkyl-O-C₁₋₄ alkyl;
Rₐ and R_{b} are independently H or C₁₋₄ alkyl;
m is optionally 0, 1, 2 or 3;
R₁₃ is selected from H, halogen, C₁₋₄ alkyl, C₁₋₄ haloalkyl, hydroxy-C₁₋₆ alkyl and C₃₋₆ cycloalkylsulfonyl;
R₁₄ is selected from H, C₁₋₄ alkyl, C₁₋₄ alkoxy and C₃₋₈ cycloalkyl.

3. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in claim 1, wherein,
wherein,
R₁ is selected from H, C₁₋₄ alkyl, C₁₋₄ alkoxy, C₁₋₄ haloalkyl and C₁₋₄ haloalkoxy;
M is selected from N or CR₁₀;
R₁₀ and R₁ may form a 5-8-membered heterocycloalkyl, and the 5-8-membered heterocycloalkyl is optionally substituted by one or more R₁₁ groups;
R₂ is selected from H, halogen, -NH₂, phosphonyl, sulfonyl, C₃₋₁₄ cycloalkyl, 3-14-membered heterocycloalkyl, C₃₋₆ cycloalkenyl and C₃₋₆ heterocycloalkenyl; wherein, the -NH₂, phosphonyl, sulfonyl, C₃₋₁₄ cycloalkyl, 3-14-membered heterocycloalkyl, C₃₋₆ cycloalkenyl and C₃₋₆ heterocycloalkenyl are optionally substituted by one or more R₁₂ groups;
R₃ is selected from 5-12-membered heteroaryl optionally substituted by one or more R₁₃ groups;
R₄ and R₅ are each independently selected from H, halogen, -CN, C₁₋₄ alkyl, C₁₋₄ haloalkyl, and C₃₋₆ cycloalkyl;
or, R₄ and R₅ are cyclized to aryl or 4-6-membered heteroaryl;
R₆ is selected from amido, sulfonyl, thiophosphonyl, phosphonyl, sulfonylamino and aminosulfonyl, wherein the amino, amido, sulfonyl, thiophosphonyl, phosphonyl, sulfonylamino and aminosulfonyl are optionally substituted by one or more R₁₄ groups;
R₇, R₈ and R₉ are each independently selected from H and C₁₋₄ alkyl;
or, R₇ and R₈ are cyclized to C₄₋₆ cycloalkyl or 5-7-membered heteroaryl;
or, R₈ and R₉ are cyclized to C₄₋₆ cycloalkyl or 5-7-membered heteroaryl;
R₁₀ is selected from H, halogen and C₁₋₄ alkyl;
R₁₁ is selected from H, C₁₋₄ alkyl, -C₁₋₄ alkyl-NRₐR_{b} and 3-6-membered heterocycloalkyl;
R₁₂ is selected from H, halogen, -OH, -NH₂, C₁₋₆ alkyl, C₁₋₆ haloalkyl, C₃₋₈ cycloalkyl, 3-8-membered heterocycloalkyl, hydroxy-C₁₋₆ alkyl, 6-14-membered spiro heterocyclyl, -C₁₋₄ alkyl-O-C₁₋₄ alkyl, -O-C₁₋₄ alkyl, -C₁₋₄ alkyl-C₃₋₆ cycloalkyl, -O-C₀₋₄ alkyl-C₃₋₆ cycloalkyl, -C₁₋₄ alkyl-3-6-membered heterocycloalkyl, -O-C₀₋₄ alkyl-3-6-membered heterocycloalkyl, -(CH₂)ₘNRₐR_{b}, -O(CH₂)ₘNRₐR_{b}, C₁₋₆ alkylsulfonyl, C₃₋₆ cycloalkylsulfonyl, NRₐR_{b}CO-, C₁₋₆ alkylcarbonyl and C₃₋₆ cycloalkylcarbonyl, wherein the cycloalkyl and heterocycloalkyl are optionally substituted by R_{ab}, and the R_{ab} is selected from H, halogen, -OH, -NH₂, -CN, C₁₋₄ alkyl, C₁₋₄ alkoxy, C₁₋₄ haloalkyl and -C₀₋₄ alkyl-O-C₁₋₄ alkyl; Rₐ and R_{b} are H or C₁₋₄ alkyl;
Rₐ and R_{b} are H or C₁₋₄ alkyl;
m is optionally 0, 1, 2 or 3;
R₁₃ is selected from H, halogen, C₁₋₄ alkyl, C₁₋₄ haloalkyl and C₃₋₆ cycloalkylsulfonyl;
R₁₄ is selected from H, C₁₋₄ alkyl, C₁₋₄ alkoxy and C₃₋₈ cycloalkyl.

4. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 1-3, wherein, M is selected from CR₁₀, and R₁₀ is defined in any one of claims 1-3.

5. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 1-4, wherein, R₁₀ is optionally and independently selected from H, fluorine, and methyl.

6. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 1-5, wherein, R₁₁ is independently selected from H, methyl, -CH₂CH₂N(CH₃)₂ and

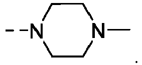

7. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 1-6, wherein, R₁₂ is selected from H, F, - OH, -NH₂, C₁₋₄ alkyl, C₁₋₄ fluoroalkyl, C₁₋₄ alkoxy, -O-C₁₋₄ alkyl-C₃₋₆ cycloalkyl, -O-C₁₋₄ alkyl-NRₐR_{b}, -C₁₋₄ alkyl-O-C₀₋₄ alkyl, 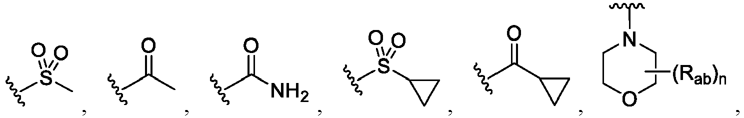 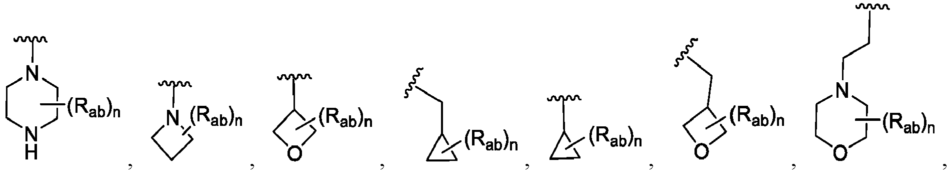 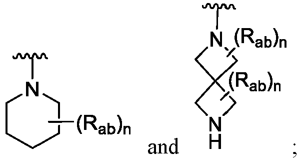 preferably, R₁₂ is selected from H, F, -OH, -NH₂, methyl, ethyl, isopropyl, -CH₂F, -CH₂CH₂F, - CH₂CHF₂, -CH₂CF₃, -CH₂CH₂CHF₂, -CH₂CH₂CF₃, -CH(CH₃)CF₃, -CH(CH₃)CH₂F, -C(CH₃)₂CH₂F, 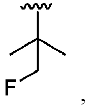 -OCH₃, -OCH(CH₃)CH₃, 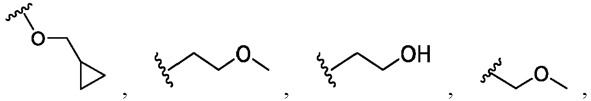 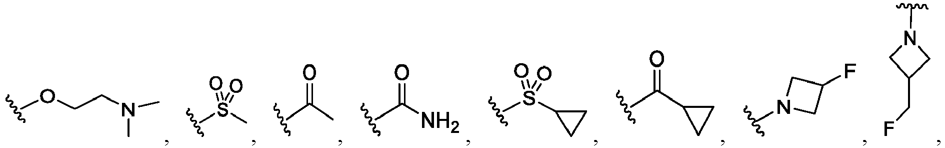 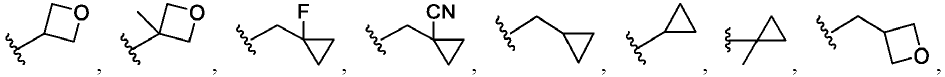 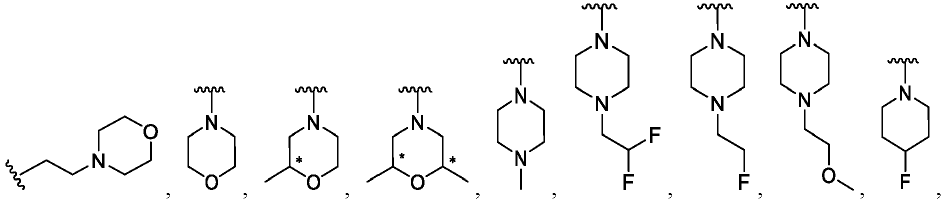 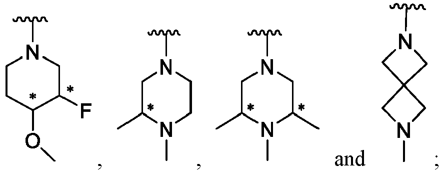 more preferably, R₁₂ is selected from H, methyl, -CH₂CH₂F, -CH₂CH₂OCH₃, -CH₂CHF₂, -CH₂CF₃, 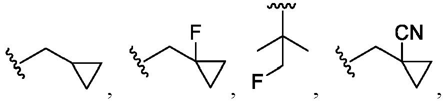 -CH(CH₃)CH₂F, -CH₂CH₂F and

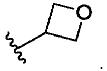

8. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 1-7, wherein, R₁₃ is selected from H, fluorine, chlorine, bromine, methyl, ethyl, n-propyl, isopropyl, difluoromethyl, trifluoromethyl, trichloromethyl, cyclopropyl and 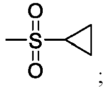
preferably, R₁₃ is selected from H, fluorine, methyl, ethyl, difluoromethyl and 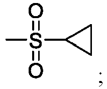
more preferably, R₁₃ is selected from H, fluorine, methyl, ethyl and difluoromethyl;
further more preferably, R₁₃ is selected from methyl.

9. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 1-8, wherein, R₁₄ is selected from methyl, ethyl, n-propyl, isopropyl, n-butyl, methoxy, ethoxy, n-propoxy, isopropoxy and cyclopropyl;
preferably, R₁₄ is selected from methyl, isopropyl, cyclopropyl and ethoxy;
more preferably, R₁₄ is selected from methyl.

10. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 1-9, wherein, R₁ is selected from H, halogen, -CN, C₁₋₃ alkyl, C₁₋₃ alkoxy, C₁₋₃ haloalkyl, C₁₋₃ haloalkoxy and 5-7-membered heteroaryl;
preferably, R₁ is selected from H, methyl, ethyl, *n*-propyl, isopropyl, *n*-butyl, methoxy, ethoxy, *n-*propoxy, isopropoxy, *n*-butoxy, trifluoromethyl, trifluoromethoxy, trichloromethyl, trichloromethoxy and 2,2,2-trifluoroethoxy;
more preferably, R₁ is selected from H, methyl, methoxy, ethoxy and 2,2,2-trifluoroethoxy;
further more preferably, R₁ is selected from methoxy.

11. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 1-10, wherein, R₂ is selected from H, halogen, -CN, -OH, -NH₂, phosphonyl, sulfonyl, C₃₋₇ cycloalkyl, 3-7-membered heterocycloalkyl, C₆₋₁₄ spiro cyclyl, C₆₋₁₄ fused cyclyl, C₆₋₁₄ bridged cyclyl, 6-14-membered spiro heterocyclyl, 6-14-membered bridged heterocyclyl, 6-14-membered fused heterocyclyl and 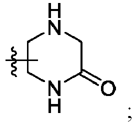 wherein, the -NH₂, phosphonyl, sulfonyl, C₃₋₇ cycloalkyl, 3-7-membered heterocycloalkyl, C₆₋₁₄ spiro cyclyl, C₆₋₁₄ fused cyclyl, C₆₋₁₄ bridged cyclyl, 6-14-membered spiro heterocyclyl, 6-14-membered bridged heterocyclyl, 6-14-membered fused heterocyclyl and 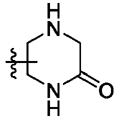 are optionally substituted by one or more R₁₂ groups;
preferably, R₂ is selected from H, halogen, -CN, -OH, -NH₂, -NHR₁₂, -NR₁₂R₁₂, 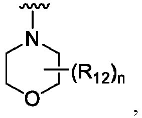 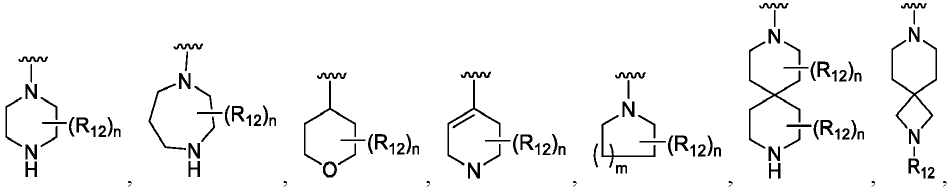 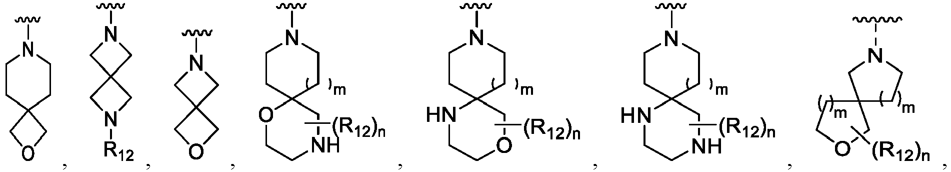 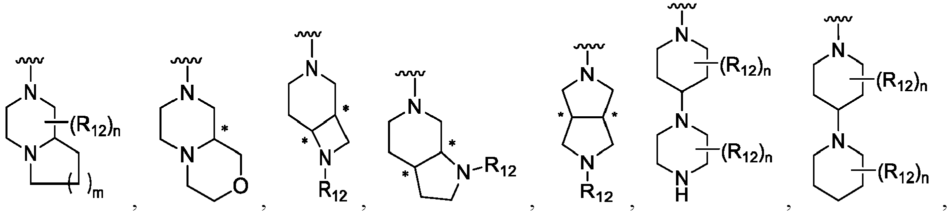 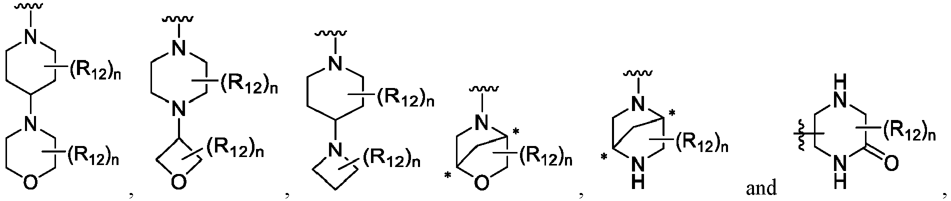 wherein the R₁₂, m and n are defined in any one of claims 1-10;
more preferably, R₂ is selected from H, fluorine, chlorine, bromine, iodine, -NH₂,

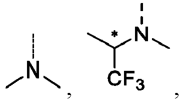

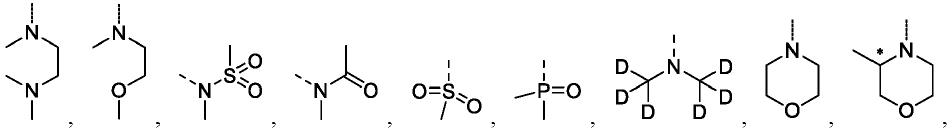

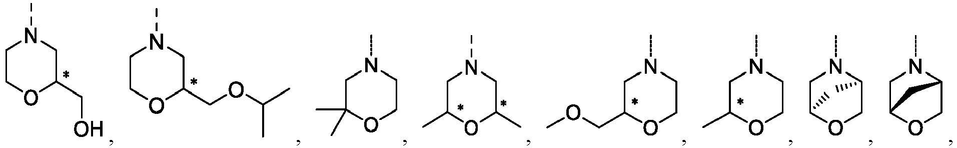

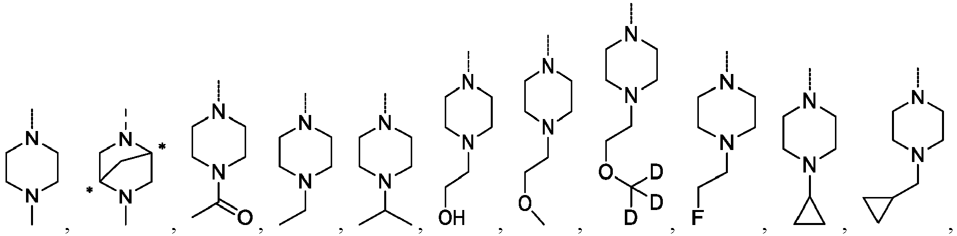

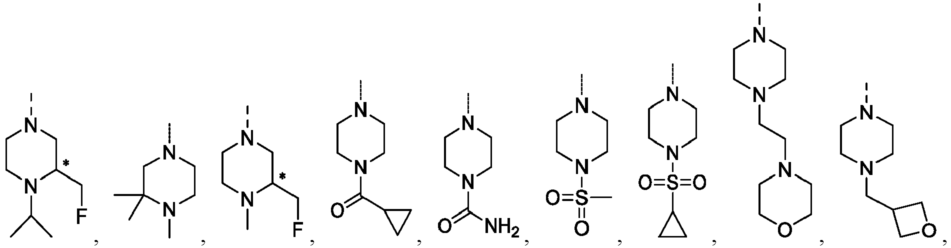

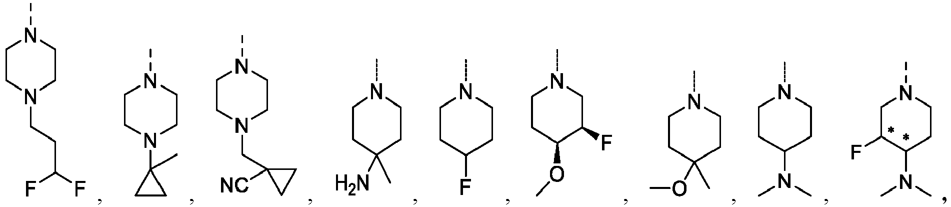

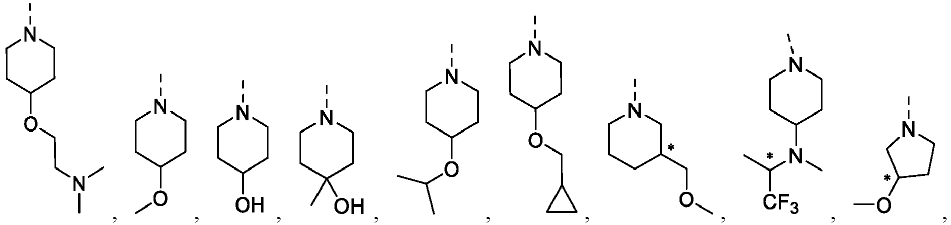

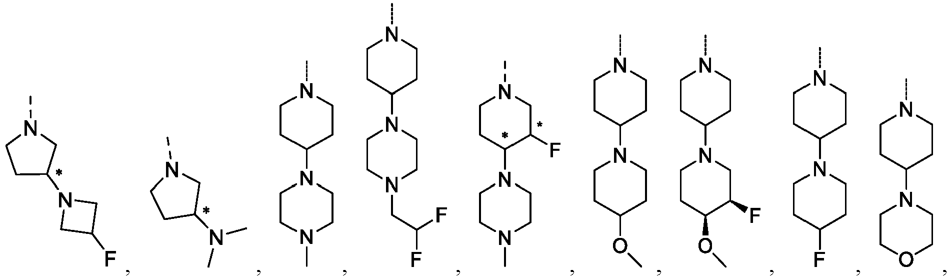

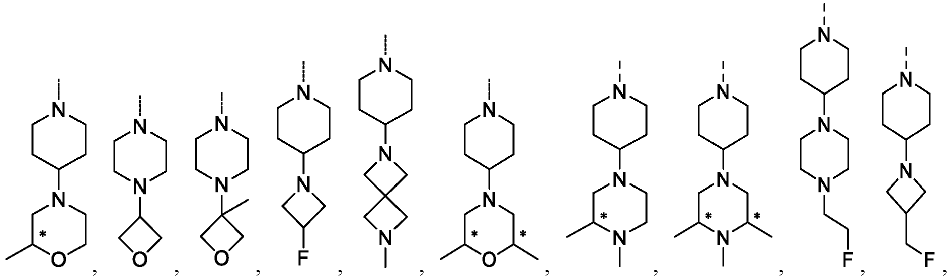

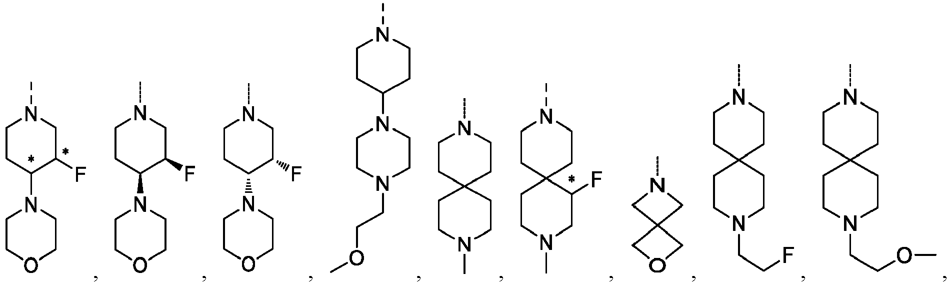

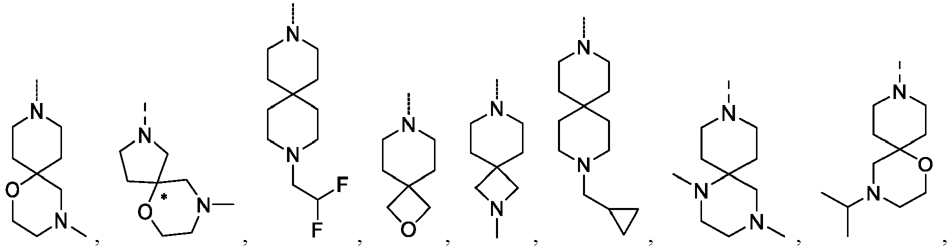

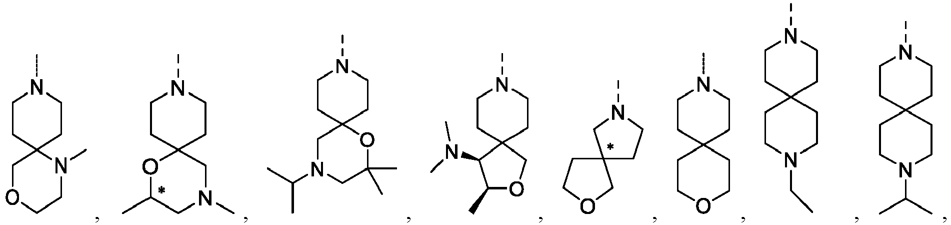

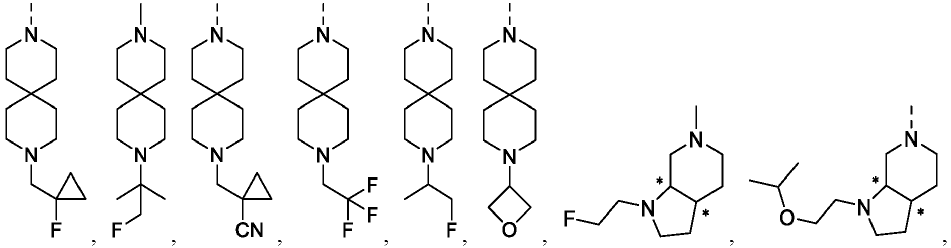

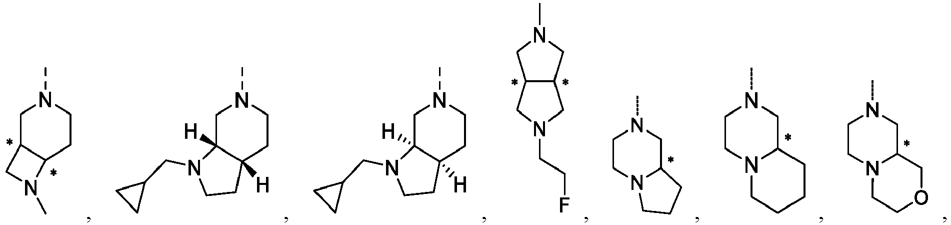

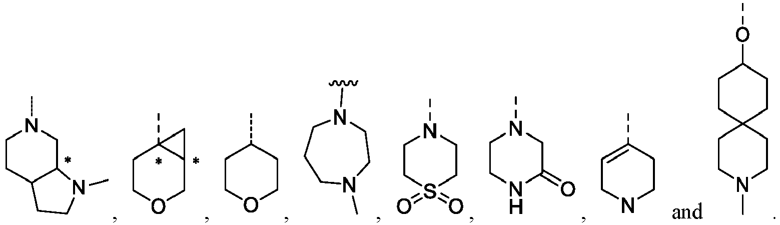

12. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 1-11, wherein,
R₃ is selected from -CN, phenyl, 5-6-membered heteroaryl and 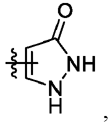 and the phenyl, 5-6-membered heteroaryl and 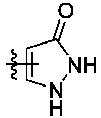 are optionally substituted by one or more R₁₃ groups;
preferably, R₃ is selected from -CN, phenyl, pyranyl, pyridyl, pyrimidinyl, pyridazinyl, pyrazinyl, triazinyl, furanyl, thienyl, pyrrolyl, pyrazolyl, thiazolyl, imidazolyl, benzofuranyl, benzimidazolyl, benzothienyl, benzoxazolyl, benzothiazolyl, indolyl, pyrazolo[1,5-α]pyridyl, quinolinyl, isoquinolinyl, tetrahydropyrrolyl, piperidinyl, piperazinyl, morpholinyl, tetrahydrofuranyl, tetrahydropyranyl, cyclobutyl, cyclopentyl, cyclohexyl and 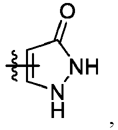 and the phenyl, pyranyl, pyridyl, pyrimidinyl, pyridazinyl, pyrazinyl, triazinyl, furanyl, thienyl, pyrrolyl, pyrazolyl, thiazolyl, imidazolyl, benzofuranyl, benzimidazolyl, benzothienyl, benzoxazolyl, benzothiazolyl, indolyl, pyrazolo[1,5-α]pyridyl, quinolinyl, isoquinolinyl, tetrahydropyrrolyl, piperidinyl, piperazinyl, morpholinyl, tetrahydrofuranyl, tetrahydropyranyl, cyclobutyl, cyclopentyl, cyclohexyl and 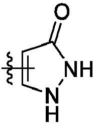 are optionally substituted by one or more R₁₃ groups;
more preferably, R₃ is selected from -CN, 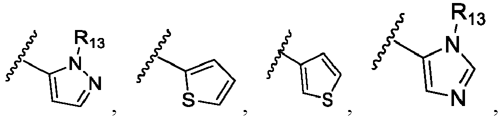 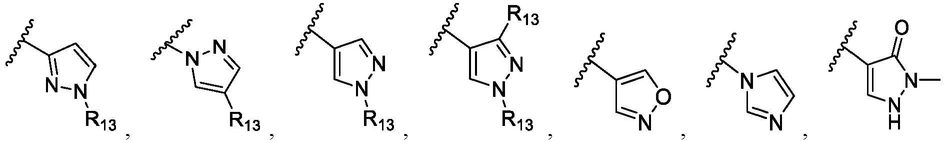 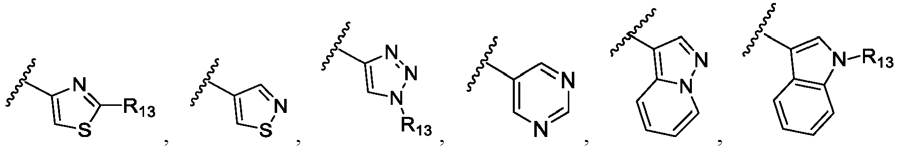 and 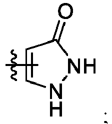
further more preferably, R₃ is selected from -CN, 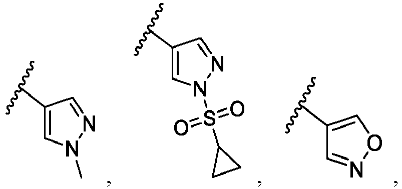 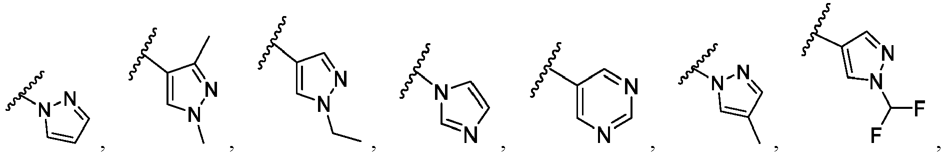 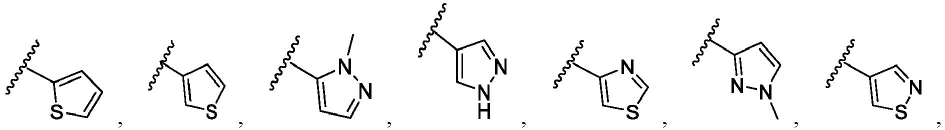 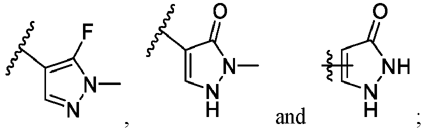
further more preferably, R₃ is selected from

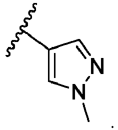

13. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 1-12, wherein, R₄ and R₅ are each independently selected from H, F, Cl, Br, CN, methyl, ethyl, isopropyl, methoxy, ethoxy, isopropoxy, trifluoromethyl, 2,2,2-trifluoroethyl and cyclopropyl, or, R₄ and R₅ form C₅₋₆ aryl or 5-6-membered heteroaryl;
preferably, R₄ is selected from H, and R₅ is selected from H, F, Cl, Br, CN, methyl, ethyl, trifluoromethyl and cyclopropyl;
further more preferably, R₄ is selected from H, and R₅ is selected from Br.

14. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 1-13, wherein, R₆ is selected from 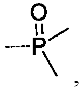 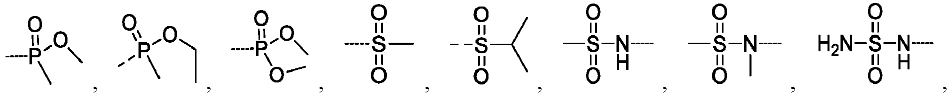 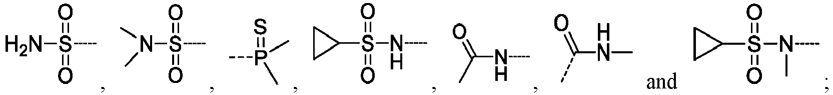 more preferably, R₆ is selected from 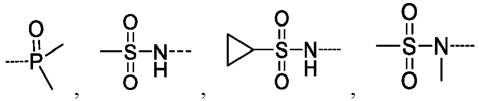 and 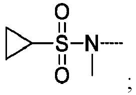 further more preferably, R₆ is selected from

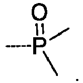

15. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 1-14, wherein, R₇, R₈ and R₉ are all H, or R₇ and R₈ are cyclized to C₄₋₆ cycloalkyl, 4-6-membered heterocycloalkyl, C₅₋₆ aryl or 5-7-membered heteroaryl, or R₈ and R₉ are cyclized to C₄₋₆ cycloalkyl, 4-6-membered heterocycloalkyl, C₅₋₆ aryl or 5-7-membered heteroaryl;
preferably, R₇ and R₈ or R₈ and R₉ are independently cyclized to cyclobutane, cyclopentane, tetrahydropyrrole ring, tetrahydrofuran ring, tetrahydropyran ring, thiophene ring, imidazole ring, pyrazole ring, pyrrole ring, oxazole ring, thiazole ring, isoxazole ring, piperazine ring, isothiazole ring, benzene ring, pyridine ring, piperidine ring, pyrimidine ring, pyridazine ring or pyrazine ring;
more preferably, R₇ and R₈ or R₈ and R₉ are independently cyclized to cyclobutane, pyridine ring or pyrazine ring;
further more preferably, R₇ and R₈ are independently cyclized to pyrazine ring, or R₉ and R₈ are independently cyclized to cyclobutane.

16. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 1-15, wherein, which is selected from, 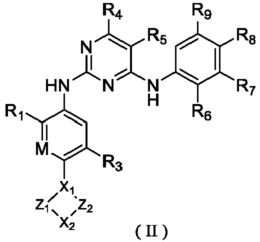 wherein, X₁ is independently selected from CR_{c} and N;
X₂ is independently selected from -CR_{c}R_{d}-, -NR_{c}- and -O-;
Z₁ and Z₂ are each independently selected from -(CRₑR_{f})ₘ(CRₑR_{f})ₙ-;
the R_{c}, R_{d}, Rₑ, and R_{f} are each independently selected from H, halogen, -CN, -OH, -NRₐR_{b}, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, phenyl, -(CH₂)ₘNRₐR_{b}, hydroxy-C₁₋₆ alkyl-, -O(CH₂)ₘNRₐR_{b}, C₃₋₈ cycloalkylalkyl-, C₃₋₈ heterocycloalkylalkyl-, C₃₋₈ cycloalkyl-C₁₋₆ alkoxy-, C₃₋₈ heterocycloalkyl-C₁₋₆ alkoxy-, C₁₋₆ alkylcarbonyl-, C₃₋₈ cycloalkylcarbonyl-, NRₐR_{b}CO-, C₁₋₆ alkylcarbonylsulfonyl-, C₃₋₈ cycloalkylsulfonyl-, C₁₋₆ alkyl-O-C₁₋₆ alkyl-, 6-14-membered spiro heterocyclyl, wherein the Rₐ, R_{b}, m and n are defined in claims 1-3;
or, R_{c} and R_{d} together form C₃₋₈ cycloalkyl or 3-8-membered heterocycloalkyl;
or, Rₑ and R_{f} together form C₃₋₈ cycloalkyl or 3-8-membered heterocycloalkyl;
or, R_{c} and Rₑ, or R_{c} and R_{f}, or R_{d} and Rₑ, or R_{d} and R_{f} together form C₃₋₈ cycloalkyl or 3-8-membered heterocycloalkyl;
wherein, a ring formed by X₁, X₂, Z₁ and Z₂ and a ring further formed by substituent groups R_{c}, R_{d}, Rₑ and R_{f} thereof are optionally substituted by one or more R₁₂ groups;
R₁, R₃, R₄, R₅, R₆, R₇, R₈, R₉, R₁₂ and M are defined in any one of claims 1-15.

17. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in claim 16, wherein, which is selected from, 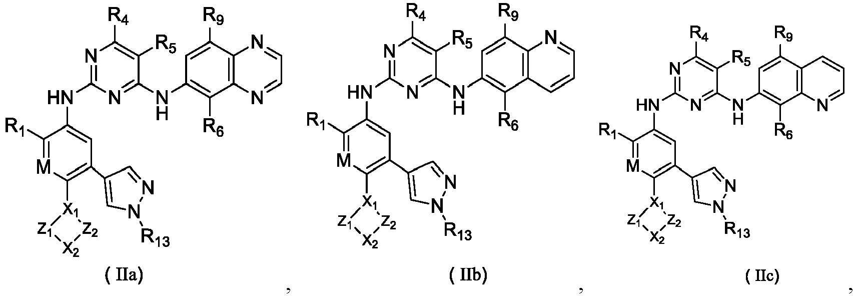 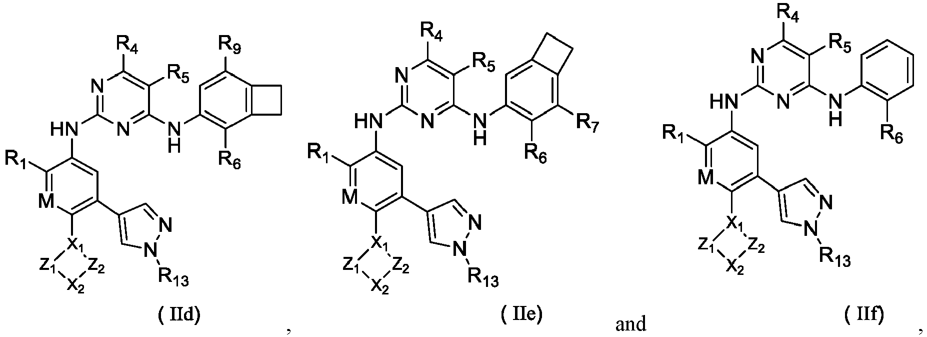 wherein, the ring formed by X₁, X₂, Z₁ and Z₂ and the ring further formed by substituent groups thereof are optionally substituted by one or more R₁₂ groups;
X₁, X₂, Z₁, Z₂, R₁, R₄, R₅, R₆, R₇, R₉, R₁₂ and M are defined in claim 16;
R₁₃ is defined in any one of claims 1-15.

18. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in claim 17, wherein, which is selected from, 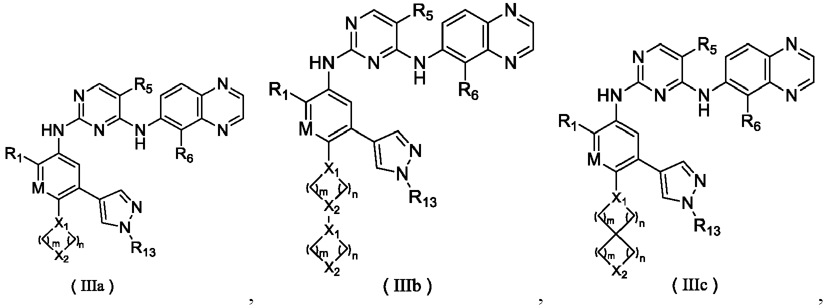 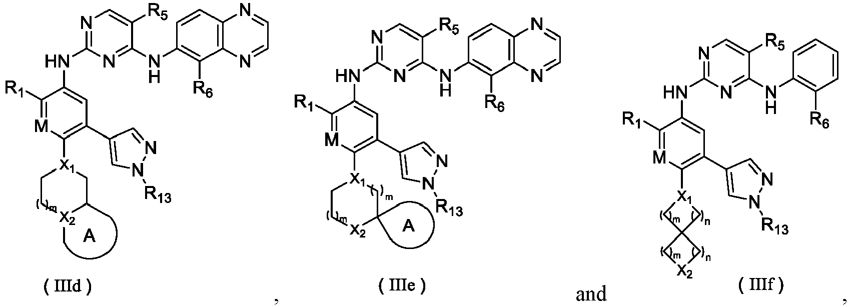 wherein, ring A is selected from C₅₋₇ cycloalkyl and 5-7-membered heterocycloalkyl;
a monocyclic ring, a bicyclic ring, a spiro ring, a fused ring formed by X₁ and X₂ and ring A are optionally substituted by one or more R₁₂ groups;
X₁, X₂, R₁, R₅, R₆, m, n, M, R₁₂ and R₁₃ are defined in claim 17.

19. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in claim 18, wherein, which is selected from, 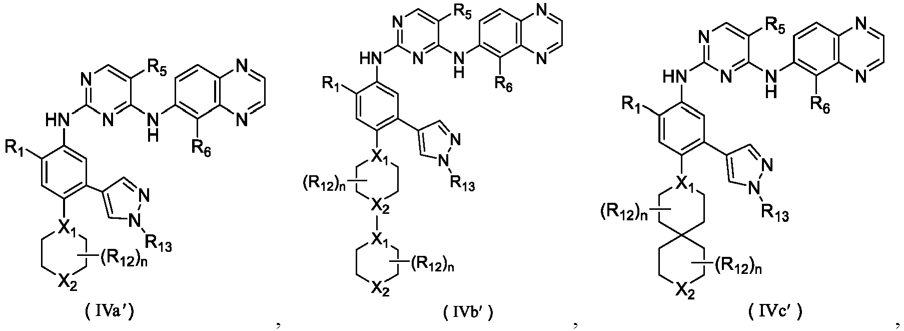 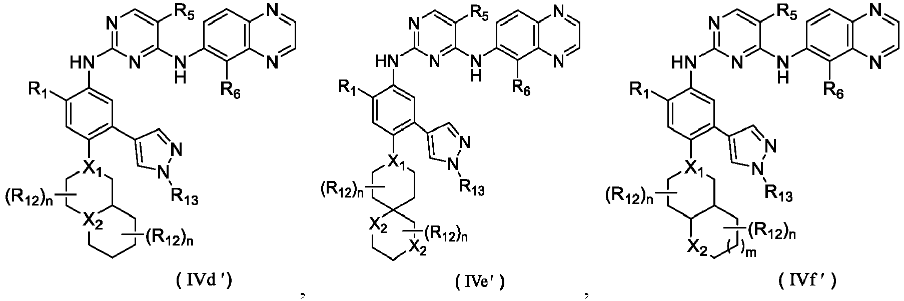 and 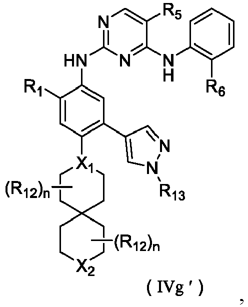
X₁, X₂, R₁, R₅, R₆, R₁₂ and R₁₃ are defined in claim 18;
m and n are defined in claim 1.

20. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in claim 19, wherein, which is selected from, 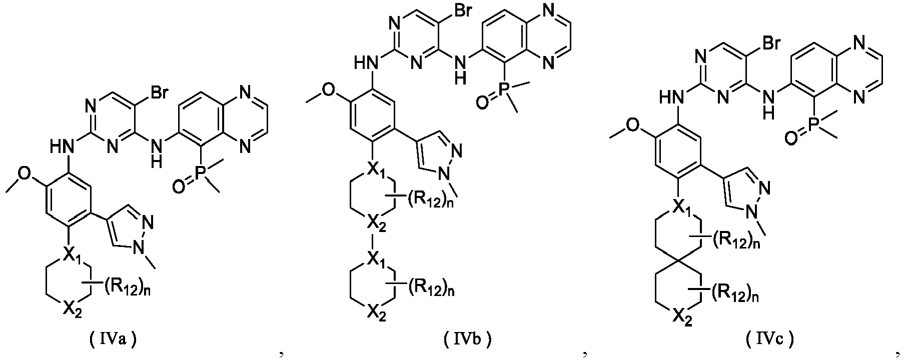 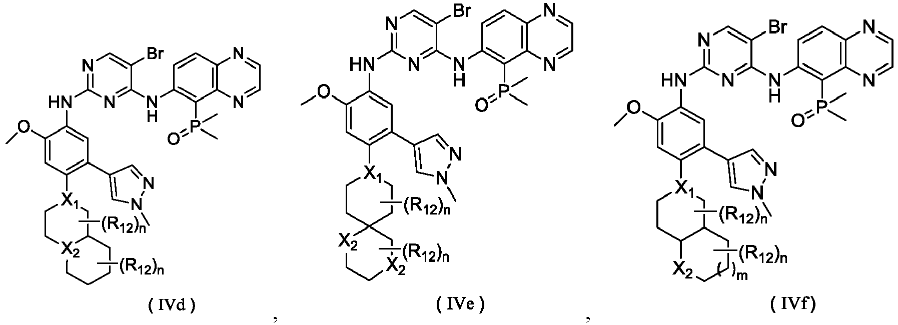 and 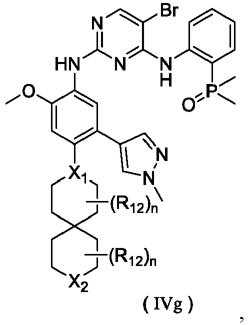 wherein, X₁, X₂, R₁₂, m and n are defined in claim 19.

21. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in claim 20, wherein, which is selected from, 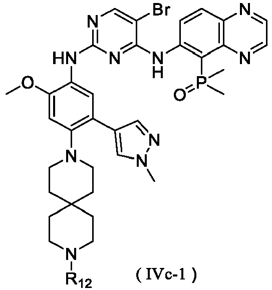 wherein, R₁₂ is defined in claim 20.

22. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in claim 20, wherein, which is selected from, 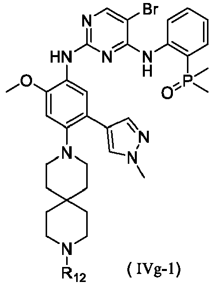 wherein, R₁₂ is defined in claim 20.

23. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in claim 1, wherein,
wherein,
R₁ is selected from H, halogen, -CN, C₁₋₆ alkyl, C₁₋₆ alkoxy, C₁₋₆ haloalkyl, C₁₋₆ haloalkoxy, C₃₋₆ cycloalkyl, 3-6-membered heterocycloalkyl, C₃₋₆ cycloalkyloxy, 3-6-membered heterocycloalkyloxy and C₂₋₆ alkenyloxy;
M is selected from N or CR₁₀;
R₁₀ and R₁ may form a 5-8-membered heterocycloalkyl, and the 5-8-membered heterocycloalkyl is optionally substituted by one or more R₁₁ groups;
R₂ is selected from H, halogen, -CN, -OH, -NH₂, phosphonyl, sulfonyl, aminosulfonyl, aminocarbonyl, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, C₃₋₆ heterocycloalkenyl and phenyl; wherein, the -OH, -NH₂, phosphonyl, sulfonyl, aminosulfonyl, aminocarbonyl, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, C₃₋₆ heterocycloalkenyl and phenyl are optionally substituted by one or more R₁₂ groups;
R₃ is selected from C₆₋₁₀ aryl, 5-12-membered heteroaryl and C₃₋₆ cycloalkenyl, wherein the C₆₋₁₀ aryl, 5-12-membered heteroaryl and C₃₋₆ cycloalkenyl are optionally substituted by one or more R₁₃ groups;
R₄ and R₅ are each independently selected from H, halogen, -CN, C₁₋₆ alkyl, C₁₋₆ alkoxy, C₁₋₆ haloalkyl, C₁₋₆ haloalkoxy, C₃₋₈ cycloalkyl and 3-8-membered heterocycloalkyl;
or, R₄ and R₅ are cyclized to 4-6-membered cycloalkyl, 4-6-membered heterocycloalkyl, 4-6-membered aryl or 4-6-membered heteroaryl;
R₆ is selected from amino, amido, sulfonyl, thiophosphonyl, phosphonyl, sulfonylamino and aminosulfonyl, wherein the amino, amido, sulfonyl, phosphonyl, sulfonylamino and aminosulfonyl are optionally substituted by one or more R₁₄ groups;
R₇, R₈ and R₉ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6-membered heterocycloalkyl and 5-7-membered heteroaryl;
or, R₇ and R₈ are cyclized to C₄₋₆ cycloalkyl, 4-6-membered heterocycloalkyl, C₅₋₆ aryl, or 5-7-membered heteroaryl;
or, R₈ and R₉ are cyclized to C₄₋₆ cycloalkyl, 4-6-membered heterocycloalkyl, C₅₋₆ aryl, or 5-7-membered heteroaryl;
R₁₀ and R₁₁ are each independently selected from H, halogen, C₁₋₆ alkyl, C₁₋₆ alkoxy, C₁₋₆ haloalkyl and C₁₋₆ haloalkoxy;
R₁₂ is selected from H, halogen, -CN, -OH, -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₈ cycloalkyl, 3-8-membered heterocycloalkyl, hydroxy-C₁₋₆ alkyl-, C₃₋₈ cycloalkylalkyl-, C₃₋₈ heterocycloalkylalkyl-, C₃₋₈ cycloalkyl-C₁₋₆ alkoxy-, C₁₋₆ alkylsulfonyl, C₃₋₆ cycloalkylsulfonyl, NRₐR_{b}CO-, C₁₋₆ alkylcarbonyl, C₃₋₈ cycloalkylcarbonyl, C₁₋₆ alkoxy-C₁₋₆ alkyl-, C₃₋₆ cycloalkenyl, phenyl, NRₐR_{b}S(O)₂, -(CH₂)ₘNRₐR_{b}, -(CH₂)ₘO(CH₂)ₙCH₃ and -O(CH₂)ₘNRₐR_{b}; wherein the Rₐ and R_{b} are H, C₁₋₆ alkyl or C₁₋₆ alkoxy, or Rₐ and R_{b} together form C₃₋₈ cycloalkyl or 3-8-membered heterocycloalkyl; wherein the m and n are independently optionally 0, 1, 2 or 3, and C₃₋₈ cycloalkyl and 3-8-membered heterocycloalkyl are optionally substituted by halogen, -OH, -NH₂, -CN, C₁₋₆ alkyl, C₁₋₆ alkoxy or C₁₋₆ haloalkyl;
R₁₃ is selected from H, halogen, -CN, C₁₋₆ alkyl, C₁₋₆ alkoxy, C₁₋₆ haloalkyl, C₁₋₆ haloalkoxy, C₃₋₈ cycloalkyl and 3-8-membered heterocycloalkyl;
R₁₄ is selected from H, C₁₋₆ alkyl, C₁₋₆ alkoxy, C₁₋₆ haloalkyl, C₁₋₆ haloalkoxy, C₃₋₈ cycloalkyl and 3-8-membered heterocycloalkyl.

24. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in claim 23, wherein,
R₁₀ and R₁₁ are each independently selected from H, fluorine, chlorine, bromine, methyl, ethyl, n-propyl, isopropyl, *n*-butyl, methoxy, ethoxy, *n*-propoxy, isopropoxy, *n*-butoxy, trifluoromethyl, trifluoromethoxy, trichloromethyl, trichloromethoxy and 2,2,2-trifluoroethoxy.

25. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in claim 23 or 24, wherein,
R₁₃ is selected from H, fluorine, chlorine, bromine, methyl, ethyl, n-propyl, isopropyl, difluoromethyl, trifluoromethyl, trichloromethyl and cyclopropyl;
preferably, R₁₃ is selected from H, fluorine, methyl, ethyl and difluoromethyl.

26. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 23-25, wherein,
R₁₄ is selected from methyl, ethyl, *n*-propyl, isopropyl, *n*-butyl, methoxy, ethoxy, *n*-propoxy, isopropoxy and cyclopropyl.

27. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 23-26, wherein,
R₁ is selected from H, halogen, -CN, C₁₋₃ alkyl, C₁₋₃ alkoxy, C₁₋₃ haloalkyl and C₁₋₃ haloalkoxy;
preferably, R₁ is selected from H, methyl, ethyl, *n*-propyl, isopropyl, *n*-butyl, methoxy, ethoxy, *n-*propoxy, isopropoxy, *n*-butoxy, trifluoromethyl, trifluoromethoxy, trichloromethyl, trichloromethoxy and 2,2,2-trifluoroethoxy.

28. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 23-27, wherein,
R₂ is selected from H, halogen, -CN, -OH, -NH₂, C₃₋₇ cycloalkyl, 3-7-membered heterocycloalkyl, C₆₋₁₄ spiro cyclyl, C₆₋₁₄ fused cyclyl, C₆₋₁₄ bridged cyclyl, 6-14-membered spiro heterocyclyl, 6-14-membered bridged heterocyclyl and 6-14-membered fused heterocyclyl; wherein, the -NH₂, C₃₋₇ cycloalkyl, 3-7-membered heterocycloalkyl, C₆₋₁₄ spiro cyclyl, C₆₋₁₄ fused cyclyl, C₆₋₁₄ bridged cyclyl, 6-14-membered spiro heterocyclyl, 6-14-membered bridged heterocyclyl and 6-14-membered fused heterocyclyl are optionally substituted by one or more R₁₂ groups, and the R₁₂ group is defined in claim 23;
preferably, R₂ is selected from H, halogen, -CN, -OH, -NH₂, -NHR₁₂, -NR₁₂R₁₂, 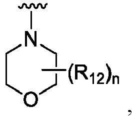 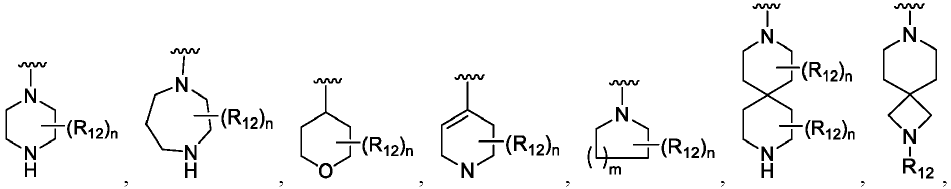 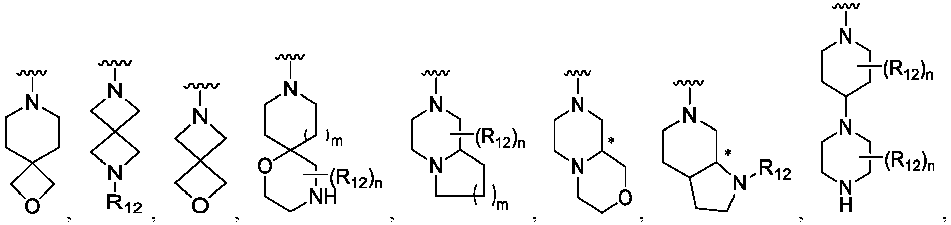 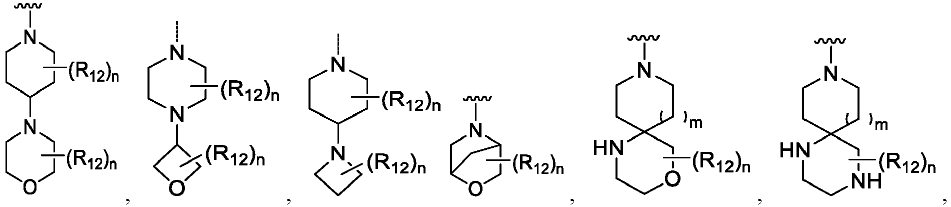 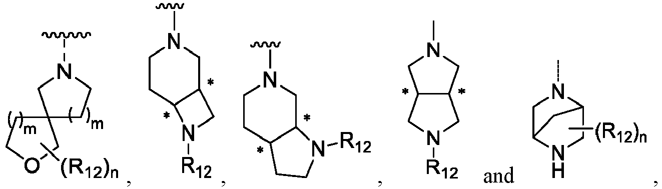 wherein the R₁₂, m and n are defined in claim 23;
more preferably, R₂ is selected from H, fluorine, chlorine, bromine, iodine, -NH₂, 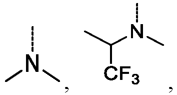 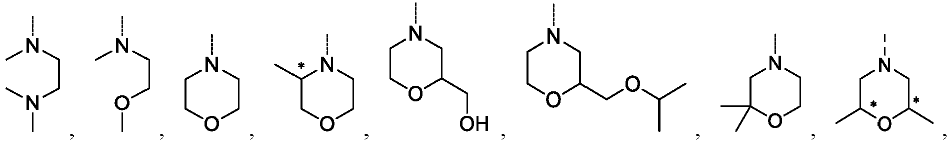 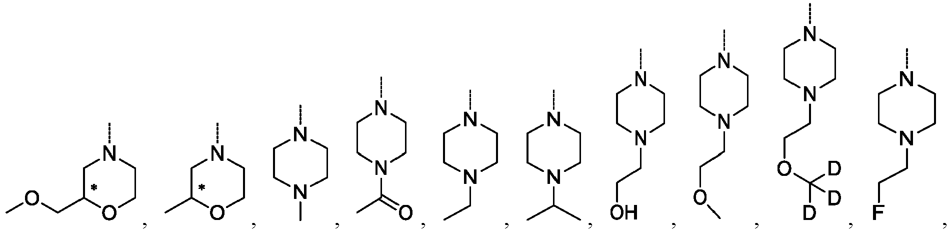 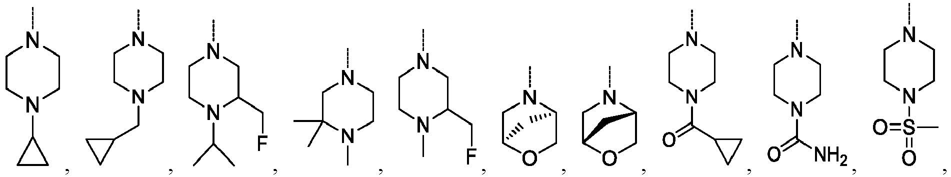 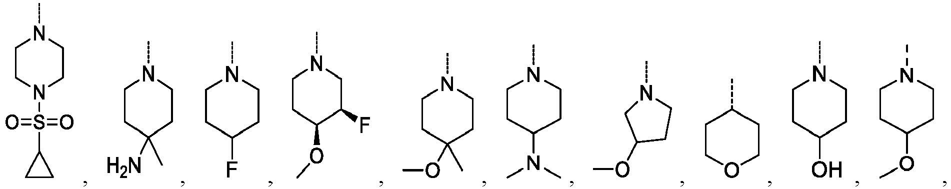 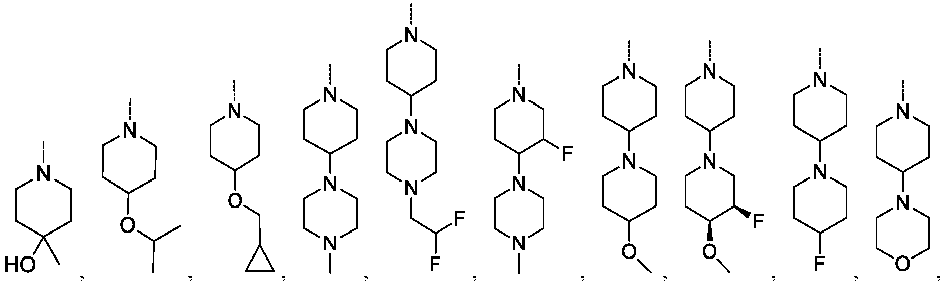 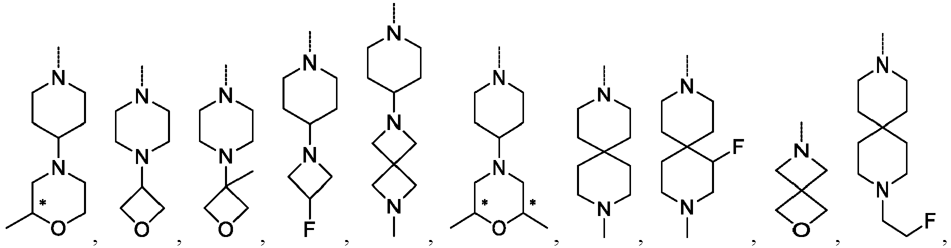 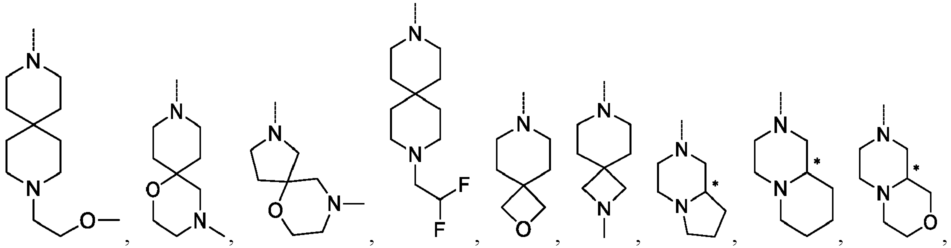 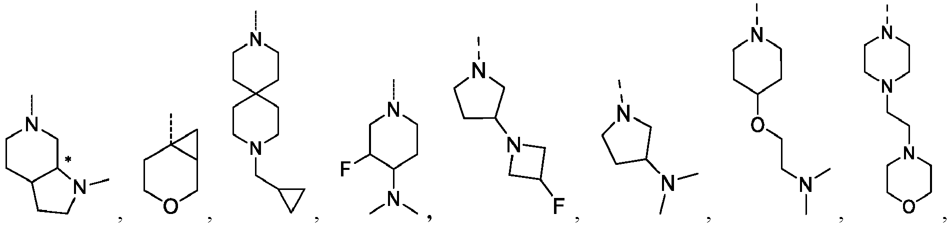 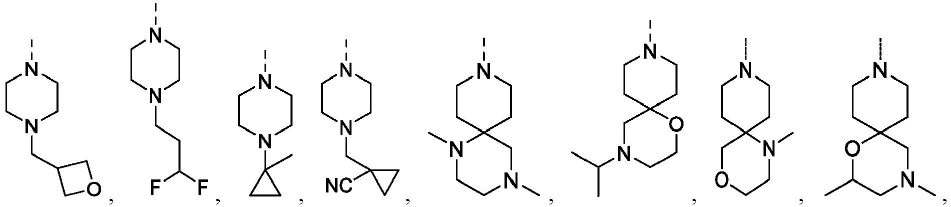 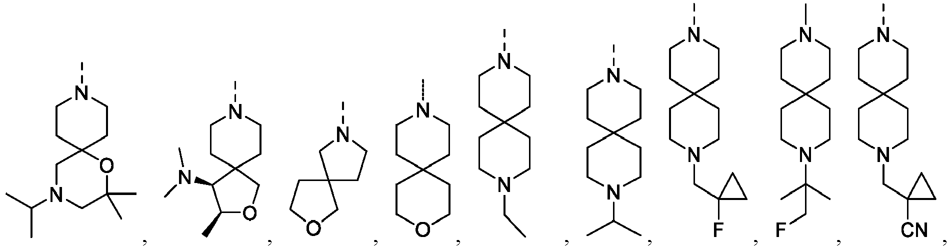 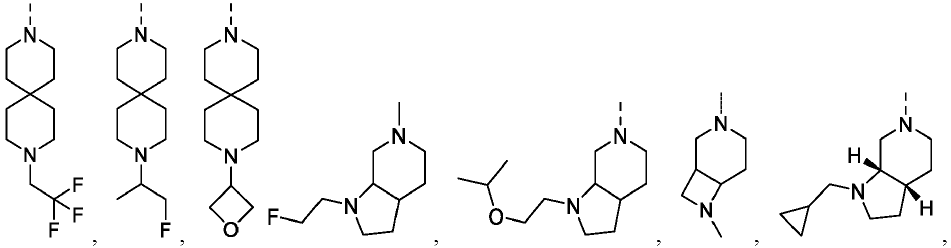 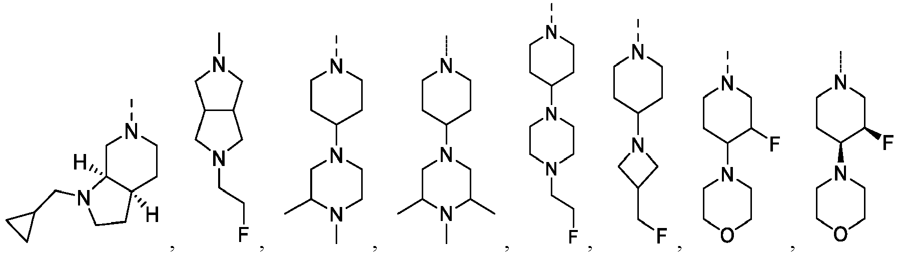 and 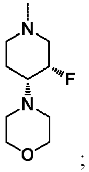
further more preferably, R₂ is selected from 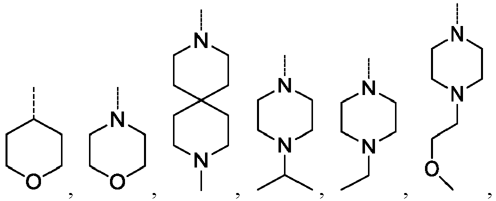 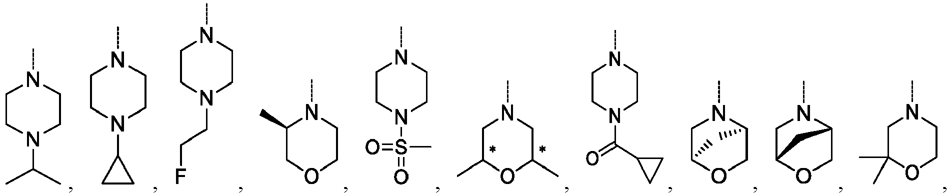 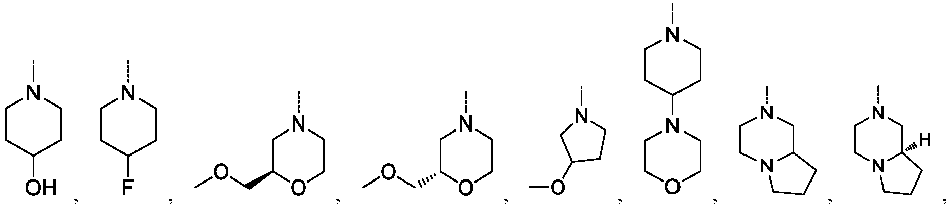 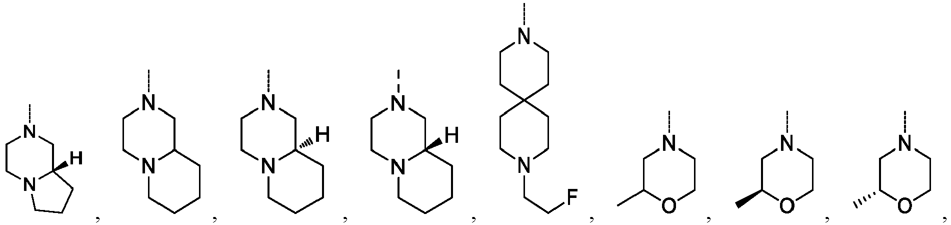 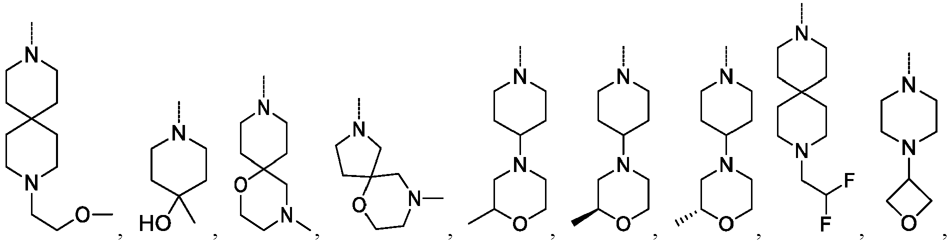 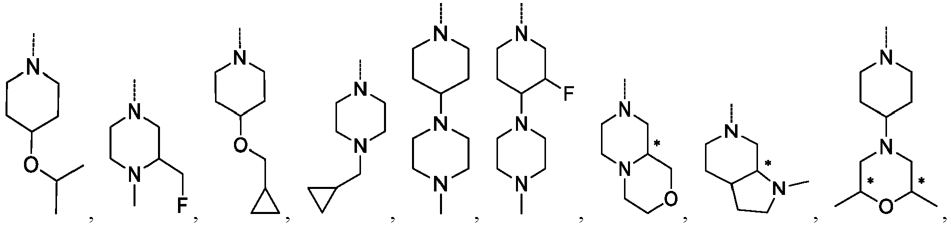 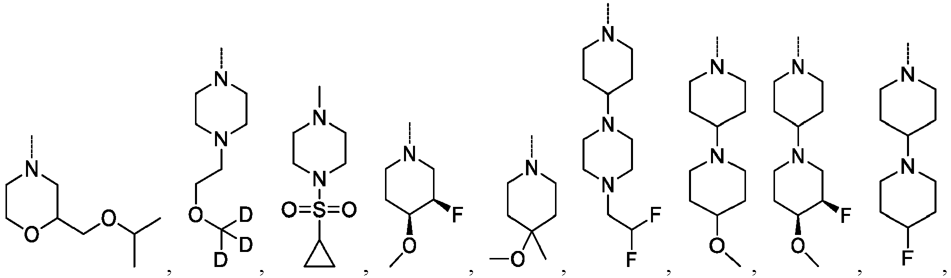 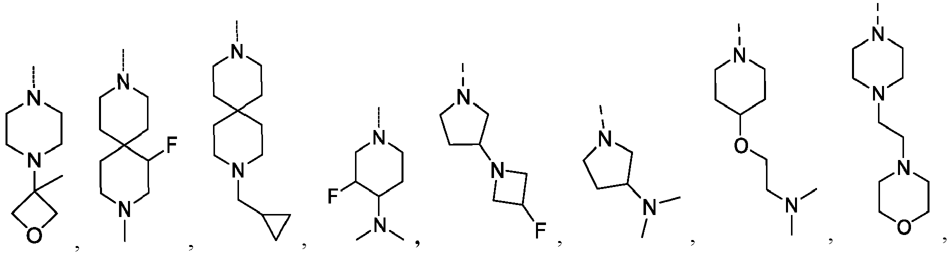 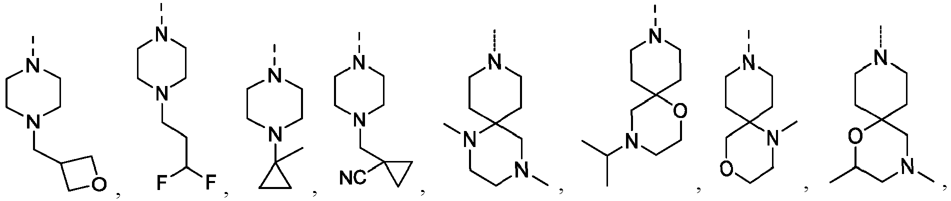 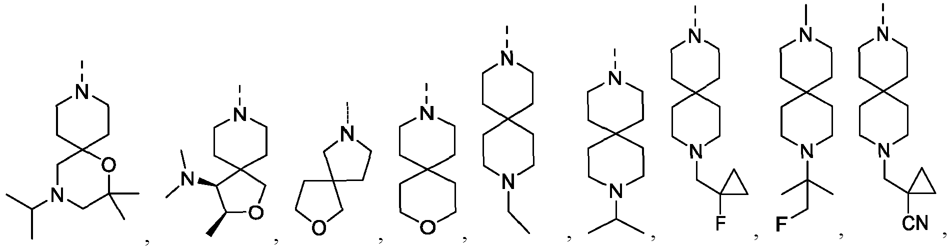 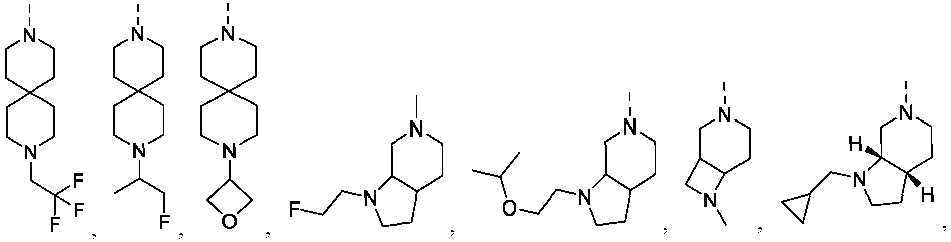 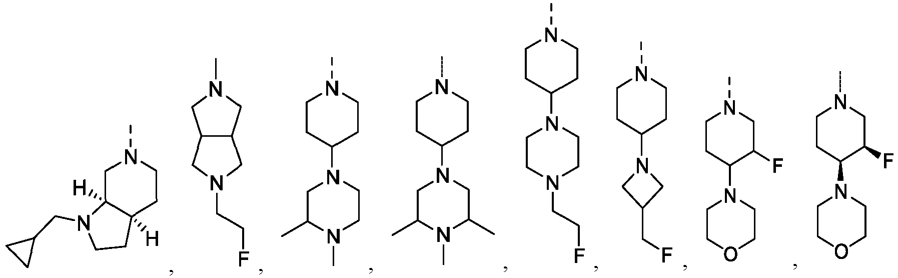 and

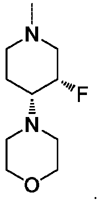

29. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 23-28, wherein,
R₃ is selected from phenyl, pyranyl, pyridyl, pyrimidinyl, pyridazinyl, pyrazinyl, furanyl, thienyl, pyrrolyl, pyrazolyl, thiazolyl, imidazolyl, benzofuranyl, benzimidazolyl, benzothienyl, benzoxazolyl, benzothiazolyl, indolyl, pyrazolo[1,5-*α*]pyridyl, quinolinyl, isoquinolinyl, tetrahydropyrrolyl, piperidinyl, piperazinyl, morpholinyl, tetrahydrofuranyl, tetrahydropyranyl, cyclobutyl, cyclopentyl and cyclohexyl, and the phenyl, pyranyl, pyridyl, pyrimidinyl, pyridazinyl, pyrazinyl, furanyl, thienyl, pyrrolyl, pyrazolyl, thiazolyl, imidazolyl, benzofuranyl, benzimidazolyl, benzothienyl, benzoxazolyl, benzothiazolyl, indolyl, pyrazolo[1,5-*α*]pyridyl, quinolinyl, isoquinolinyl, tetrahydropyrrolyl, piperidinyl, piperazinyl, morpholinyl, tetrahydrofuranyl, tetrahydropyranyl, cyclobutyl, cyclopentyl and cyclohexyl are optionally substituted by one or more R₁₃ groups;
preferably, R₃ is selected from 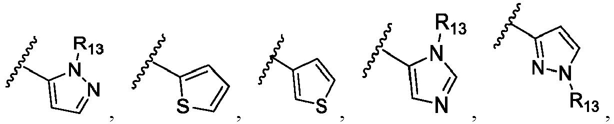 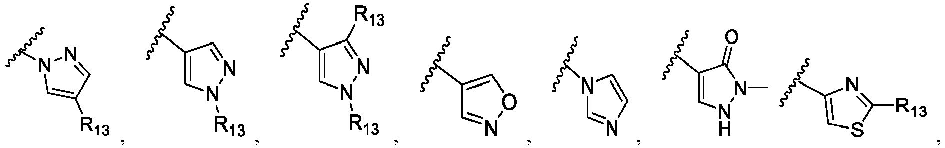 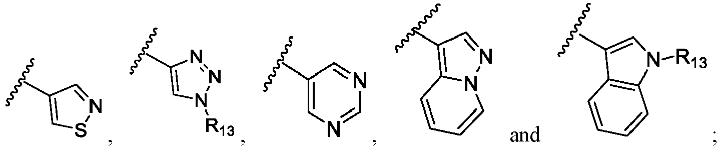
more preferably, R₃ is selected from 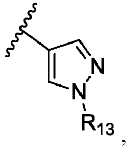 wherein the R₁₃ is defined in claim 23 or 25.

30. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 23-29, wherein,
R₄ and R₅ are each independently selected from H, F, Cl, Br, CN, methyl, ethyl, isopropyl, methoxy, ethoxy, isopropoxy, trifluoromethyl, 2,2,2-trifluoroethyl and cyclopropyl.

31. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 23-30, wherein,
R₆ is selected from 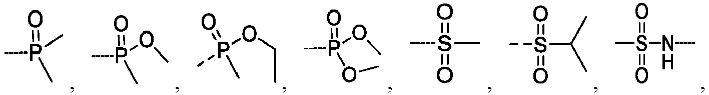 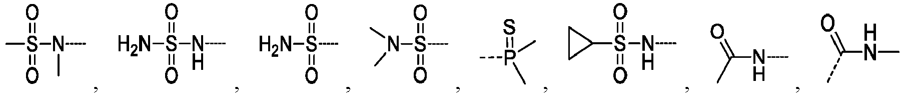 and 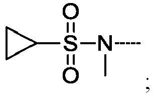
more preferably, the R₆ is selected from 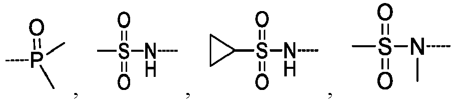 and

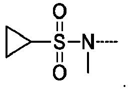

32. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 23-31, wherein,
R₇ and R₈ are cyclized to C₄₋₆ cycloalkyl, 4-6-membered heterocycloalkyl, C₅₋₆ aryl or 5-7-membered heteroaryl, or R₈ and R₉ are cyclized to C₄₋₆ cycloalkyl, 4-6-membered heterocycloalkyl, C₅₋₆ aryl or 5-7-membered heteroaryl;
preferably, R₇ and R₈ or R₈ and R₉ are independently cyclized to cyclobutane, cyclopentane, tetrahydropyrrole ring, tetrahydrofuran ring, tetrahydropyran ring, thiophene ring, imidazole ring, pyrazole ring, pyrrole ring, oxazole ring, thiazole ring, isoxazole ring, piperazine ring, isothiazole ring, benzene ring, pyridine ring, piperidine ring, pyrimidine ring, pyridazine ring or pyrazine ring;
more preferably, R₇ and R₈ or R₈ and R₉ are independently cyclized to cyclobutane, pyridine ring or pyrazine ring.

33. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in claim 23, wherein, which is selected from, 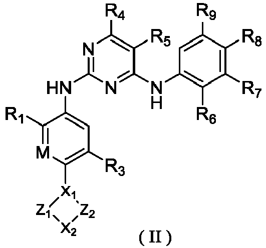 wherein, X₁ is independently selected from CR_{c} and N;
X₂ is independently selected from -CR_{c}R_{d}-, -NR_{c}- and -O-;
Z₁ and Z₂ are each independently selected from -(CRₑR_{f})ₘ(CRₑR_{f})ₙ-;
the R_{c}, R_{d}, Rₑ, and R_{f} are each independently selected from H, halogen, -CN, -OH, -NRₐR_{b}, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, phenyl, -(CH₂)ₘNRₐR_{b}, hydroxy-C₁₋₆ alkyl-O(CH₂)ₘNRₐR_{b}, C₃₋₈ cycloalkylalkyl-, C₃₋₈ heterocycloalkylalkyl-, C₃₋₈ cycloalkyl-C₁₋₆ alkoxy-, C₃₋₈ heterocycloalkyl-C₁₋₆ alkoxy-, C₁₋₆ alkylcarbonyl-, C₃₋₈ cycloalkylcarbonyl-, NRₐR_{b}CO-, C₁₋₆ alkylcarbonylsulfonyl- and C₃₋₈ cycloalkylsulfonyl-, wherein the Rₐ, R_{b}, m and n are defined in claim 23;
or, R_{c} and R_{d} together form C₃₋₈ cycloalkyl or 3-8-membered heterocycloalkyl;
or, Rₑ and R_{f} together form C₃₋₈ cycloalkyl or 3-8-membered heterocycloalkyl;
or, R_{c} and Rₑ, or R_{c} and R_{f}, or R_{d} and Rₑ, or R_{d} and R_{f} together form C₃₋₈ cycloalkyl or 3-8-membered heterocycloalkyl;
wherein, a ring formed by X₁, X₂, Z₁ and Z₂ and a ring further formed by substituent groups R_{c}, R_{d}, Rₑ and R_{f} thereof are optionally substituted by one or more R₁₂ groups;
R₁, R₃, R₄, R₅, R₆, R₇, R₈, R₉, R₁₂ and M are defined in any one of claims 23-32.

34. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in claim 33, wherein, which is selected from, 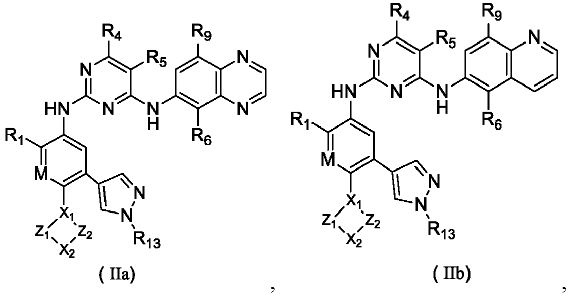 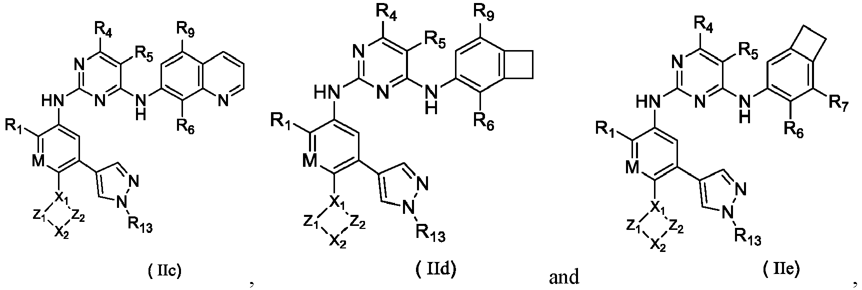 wherein, the ring formed by X₁, X₂, Z₁ and Z₂ and the ring further formed by substituent groups thereof are optionally substituted by one or more R₁₂ groups;
X₁, X₂, Z₁, Z₂, R₁, R₄, R₅, R₆, R₇, R₉, R₁₂ and M are defined in claim 33;
R₁₃ is defined in any one of claims 23-32.

35. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in claim 34, wherein, which is selected from, 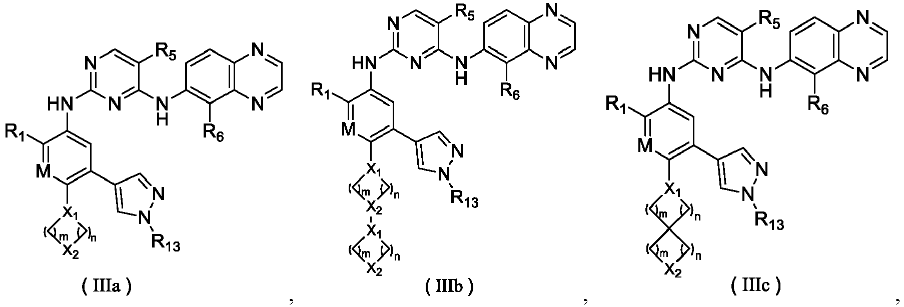 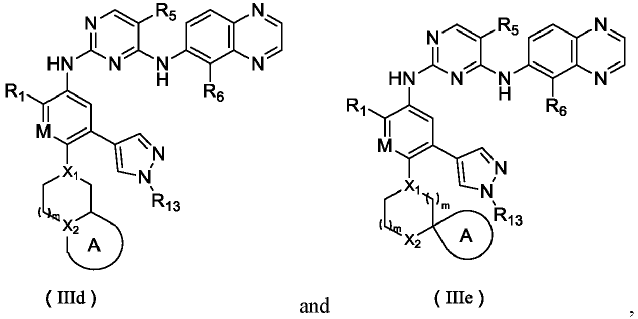 wherein, ring A is selected from C₅₋₇ cycloalkyl and 5-7-membered heterocycloalkyl;
a monocyclic ring, a bicyclic ring, a spiro ring, a fused ring formed by X₁ and X₂ and ring A are optionally substituted by one or more R₁₂ groups;
X₁, X₂, R₁, R₅, R₆, m, n, M, R₁₂ and R₁₃ are defined in claim 34.

36. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in claim 35, wherein, which is selected from, 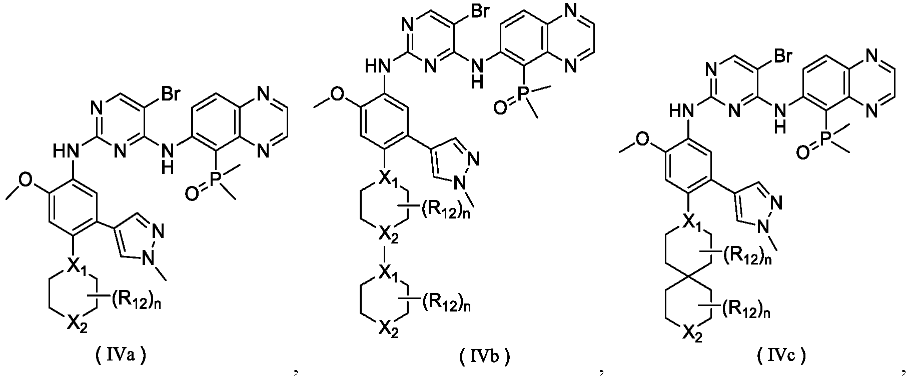 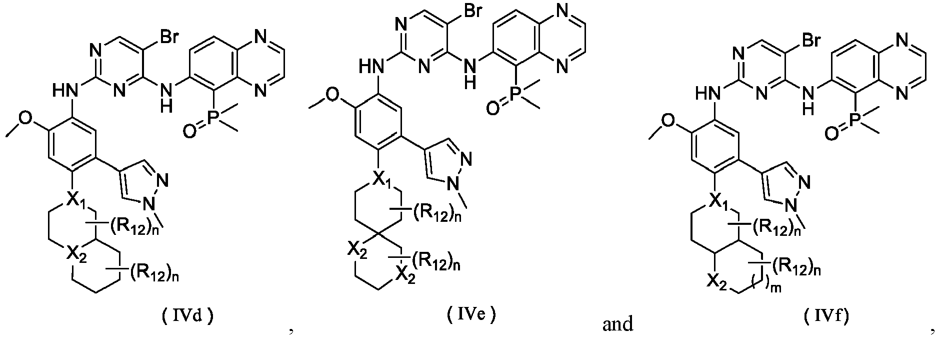 wherein X₁, X₂, R₁₂, m and n are defined in claim 35.

37. The compound represented by formula (I), the stereoisomer thereof, the tautomer thereof, the pharmaceutically acceptable salt thereof, the prodrug thereof, the hydrate thereof, the solvate thereof or the isotope-labeled derivative thereof as claimed in claim 36, wherein, which is selected from, 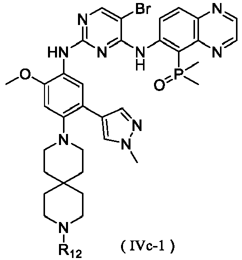 wherein R₁₂ is defined in claim 36.

38. A compound represented by formula (I-A), a stereoisomer thereof, a tautomer thereof, a pharmaceutically acceptable salt thereof, a prodrug thereof, a hydrate thereof, a solvate thereof or an isotope-labeled derivative thereof, 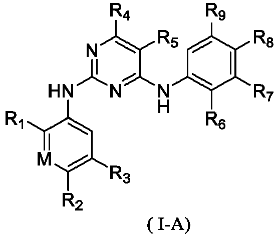 wherein,
R₁ is selected from H, halogen, -CN, C₁₋₆ alkyl, C₁₋₆ alkoxy, C₁₋₆ haloalkyl, C₁₋₆ haloalkoxy, C₃₋₆ cycloalkyl, 3-6-membered heterocycloalkyl, C₃₋₆ cycloalkyloxy, 3-6-membered heterocycloalkyloxy and C₂₋₆ alkenyloxy;
M is selected from N or CR₁₀;
R₁₀ and R₁ may form a 5-8-membered heterocycloalkyl, and the 5-8-membered heterocycloalkyl is optionally substituted by one or more R₁₁ groups;
R₂ is selected from H, halogen, -CN, -OH, -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, C₃₋₆ heterocycloalkenyl and phenyl; wherein, the -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, C₃₋₆ heterocycloalkenyl and phenyl are optionally substituted by one or more R₁₂ groups;
R₃ is selected from C₆₋₁₀ aryl, 5-12-membered heteroaryl and C₃₋₆ cycloalkenyl, wherein the C₆₋₁₀ aryl, 5-12-membered heteroaryl and C₃₋₆ cycloalkenyl are optionally substituted by one or more R₁₃ groups;
R₄ and R₅ are each independently selected from H, halogen, -CN, C₁₋₆ alkyl, C₁₋₆ alkoxy, C₁₋₆ haloalkyl, C₁₋₆ haloalkoxy, C₃₋₈ cycloalkyl and 3-8-membered heterocycloalkyl;
R₆ is selected from amino, amido, sulfonyl, phosphonyl, sulfonylamino and aminosulfonyl, wherein the amino, amido, sulfonyl, phosphonyl, sulfonylamino and aminosulfonyl are optionally substituted by one or more R₁₄ groups;
R₇, R₈ and R₉ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6-membered heterocycloalkyl and 5-7-membered heteroaryl;
or, R₇ and R₈ are cyclized to C₄₋₆ cycloalkyl, 4-6-membered heterocycloalkyl, C₅₋₆ aryl, or 5-7-membered heteroaryl;
or, R₈ and R₉ are cyclized to C₄₋₆ cycloalkyl, 4-6-membered heterocycloalkyl, C₅₋₆ aryl, or 5-7-membered heteroaryl;
R₁₀, R₁₁, R₁₂, R₁₃ and R₁₄ are each independently selected from H, halogen, -CN, -OH, -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₈ cycloalkyl, 3-8-membered heterocycloalkyl, hydroxy-C₁₋₆ alkyl, C₃₋₈ cycloalkylalkyl, C₃₋₈ cycloalkyl-C₁₋₆ alkoxy, C₁₋₆ alkylsulfonyl, C₃₋₆ cycloalkylsulfonyl, aminocarbonyl, C₃₋₈ cycloalkylcarbonyl, C₁₋₆ alkoxy-C₁₋₆ alkyl, C₃₋₆ cycloalkenyl, phenyl, -(CH₂)ₘNRₐR_{b} and -(CH₂)ₘO(CH₂)ₙCH₃; wherein Rₐ and R_{b} are H, C₁₋₆ alkyl or C₁₋₆ alkoxy, or Rₐ and R_{b} together form C₃₋₈ cycloalkyl or 3-8-membered heterocycloalkyl; wherein m and n are independently optionally 0, 1, 2 or 3, C₃₋₈ cycloalkyl and 3-8-membered heterocycloalkyl are optionally substituted by halogen, -OH, -NH₂, C₁₋₆ alkyl, C₁₋₆ alkoxy and C₁₋₆ haloalkyl.

39. The compound represented by formula (I-A), the stereoisomer thereof, the tautomer thereof, the pharmaceutically acceptable salt thereof, the prodrug thereof, the hydrate thereof, the solvate thereof or the isotope-labeled derivative thereof as claimed in claim 38, wherein,
R₁ is selected from H, halogen, -CN, C₁₋₆ alkyl, C₁₋₆ alkoxy, C₁₋₆ haloalkyl, C₁₋₆ haloalkoxy, C₃₋₆ cycloalkyl, 3-6-membered heterocycloalkyl, C₃₋₆ cycloalkoxy, 3-6-membered heterocycloalkyloxy and C₂₋₆ alkenyloxy;
M is selected from N or CR₁₀;
R₁₀ and R₁ may form a 5-8-membered heterocycloalkyl, and the 5-8-membered heterocycloalkyl is optionally substituted by one or more R₁₁ groups;
R₂ is selected from H, halogen, -CN, -OH, -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, C₃₋₆ heterocycloalkenyl and phenyl; wherein, the -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, C₃₋₆ heterocycloalkenyl and phenyl are optionally substituted by one or more R₁₂ groups;
R₃ is selected from C₆₋₁₀ aryl, 5-12-membered heteroaryl and C₃₋₆ cycloalkenyl, wherein the C₆₋₁₀ aryl, 5-12-membered heteroaryl and C₃₋₆ cycloalkenyl are optionally substituted by one or more R₁₃ groups;
R₄ and R₅ are each independently selected from H, halogen, -CN, C₁₋₆ alkyl, C₁₋₆ alkoxy, C₁₋₆ haloalkyl, C₁₋₆ haloalkoxy, C₃₋₈ cycloalkyl and 3-8-membered heterocycloalkyl;
R₆ is selected from amino, amido, sulfonyl, phosphonyl, sulfonylamino and aminosulfonyl, wherein the amino, amido, sulfonyl, phosphonyl, sulfonylamino and aminosulfonyl are optionally substituted by one or more R₁₄ groups;
R₇, R₈ and R₉ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6-membered heterocycloalkyl and 5-7-membered heteroaryl;
or, R₇ and R₈ are cyclized to C₄₋₆ cycloalkyl, 4-6-membered heterocycloalkyl, C₅₋₆ aryl, or 5-7-membered heteroaryl;
or, R₈ and R₉ are cyclized to C₄₋₆ cycloalkyl, 4-6-membered heterocycloalkyl, C₅₋₆ aryl, or 5-7-membered heteroaryl;
R₁₀ and R₁₁ are each independently selected from H, halogen, C₁₋₆ alkyl, C₁₋₆ alkoxy, C₁₋₆ haloalkyl and C₁₋₆ haloalkoxy;
R₁₂ is selected from H, halogen, -CN, -OH, -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₈ cycloalkyl, 3-8-membered heterocycloalkyl, hydroxy-C₁₋₆ alkyl, C₃₋₈ cycloalkylalkyl, C₃₋₈ cycloalkyl-C₁₋₆ alkoxy, C₁₋₆ alkylsulfonyl, C₃₋₆ cycloalkylsulfonyl, aminocarbonyl, C₃₋₈ cycloalkylcarbonyl, C₁₋₆ alkoxy-C₁₋₆ alkyl, C₃₋₆ cycloalkenyl, phenyl, -(CH₂)ₘNRₐR_{b} and-(CH₂)ₘO(CH₂)ₙCH₃; wherein Rₐ and R_{b} are H, C₁₋₆ alkyl or C₁₋₆ alkoxy, or Rₐ and R_{b} together form C₃₋₈ cycloalkyl or 3-8-membered heterocycloalkyl; wherein m and n are independently optionally 0, 1, 2 or 3, C₃₋₈ cycloalkyl and 3-8-membered heterocycloalkyl are optionally substituted by halogen, -OH, -NH₂, C₁₋₆ alkyl, C₁₋₆ alkoxy and C₁₋₆ haloalkyl;
R₁₃ is selected from H, halogen, -CN, C₁₋₆ alkyl, C₁₋₆ alkoxy, C₁₋₆ haloalkyl, hydroxy-C₁₋₆ alkyl, C₁₋₆ haloalkoxy, C₃₋₈ cycloalkyl and 3-8-membered heterocycloalkyl;
R₁₄ is selected from H, C₁₋₆ alkyl, C₁₋₆ alkoxy, C₁₋₆ haloalkyl, C₁₋₆ haloalkoxy, C₃₋₈ cycloalkyl and 3-8-membered heterocycloalkyl.

40. The compound represented by formula (I-A), the stereoisomer thereof, the tautomer thereof, the pharmaceutically acceptable salt thereof, the prodrug thereof, the hydrate thereof, the solvate thereof or the isotope-labeled derivative thereof as claimed in claim 38 or 39, wherein,
R₁ is selected from H, halogen, -CN, C₁₋₃ alkyl, C₁₋₃ alkoxy, C₁₋₃ haloalkyl and C₁₋₃ haloalkoxy;
preferably, R₁ is selected from H, methyl, ethyl, n-propyl, isopropyl, n-butyl, methoxy, ethoxy, n-propoxy, isopropoxy, n-butoxy, trifluoromethyl, trifluoromethoxy, trichloromethyl, trichloromethoxy and 2,2,2-trifluoroethoxy.

41. The compound represented by formula (I-A), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 38-40, wherein,
R₂ is selected from H, halogen, -NH₂, C₃₋₇ cycloalkyl, 3-7-membered heterocycloalkyl, C₆₋₁₄ spiro cyclyl, C₆₋₁₄ fused cyclyl, C₆₋₁₄ bridged cyclyl, 6-14-membered spiro heterocyclyl, 6-14-membered bridged heterocyclyl and 6-14-membered fused heterocyclyl; wherein, the -NH₂, C₃₋₇ cycloalkyl, 3-7-membered heterocycloalkyl, C₆₋₁₄ spiro cyclyl, C₆₋₁₄ fused cyclyl, C₆₋₁₄ bridged cyclyl, 6-14-membered spiro heterocyclyl, 6-14-membered bridged heterocyclyl and 6-14-membered fused heterocyclyl are optionally substituted by one or more R₁₂ groups, and the R₁₂ group is defined in claim 38;
preferably, R₂ is selected from H, halogen, -CN, -OH, -NH₂, -NHR₁₂, -NR₁₂R₁₂, 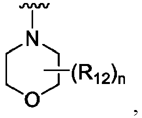 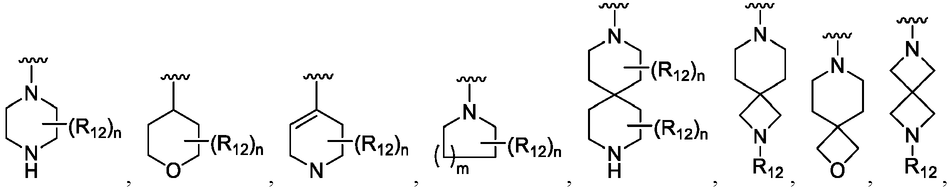 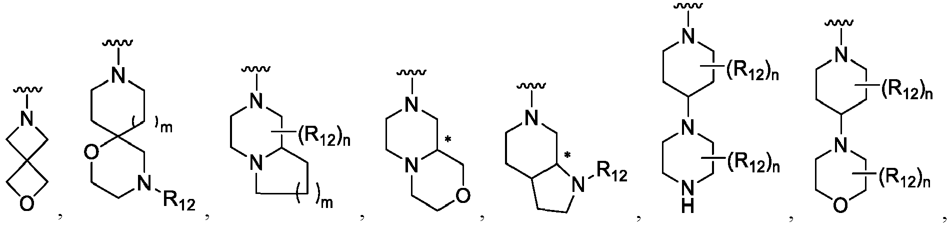 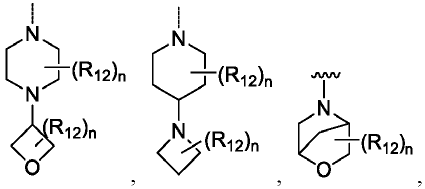 wherein the R₁₂, m and n are defined in claim 38 or 39;
more preferably, R₂ is selected from H, fluorine, chlorine, bromine, iodine, -NH₂, 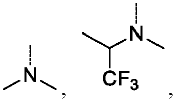 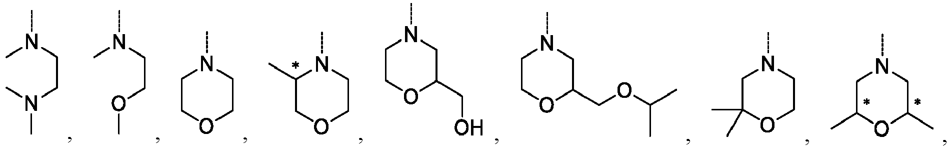 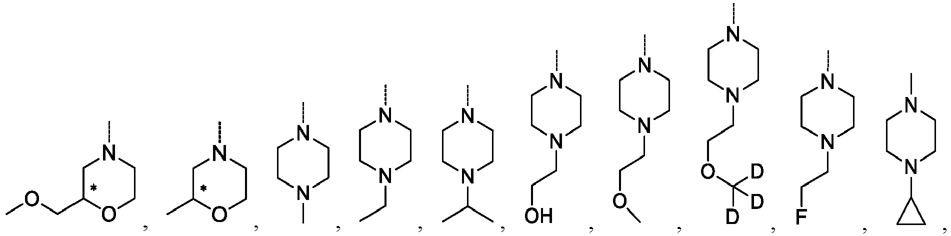 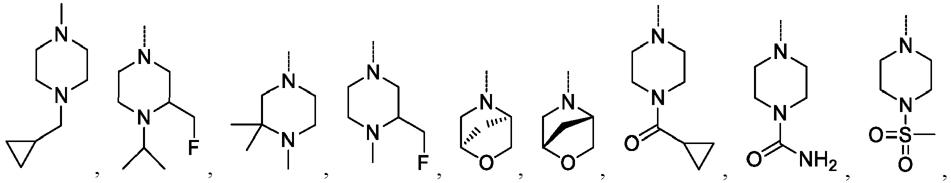 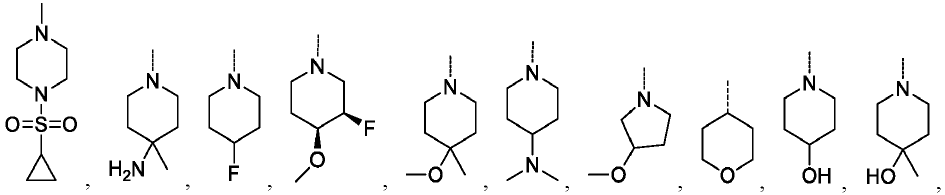 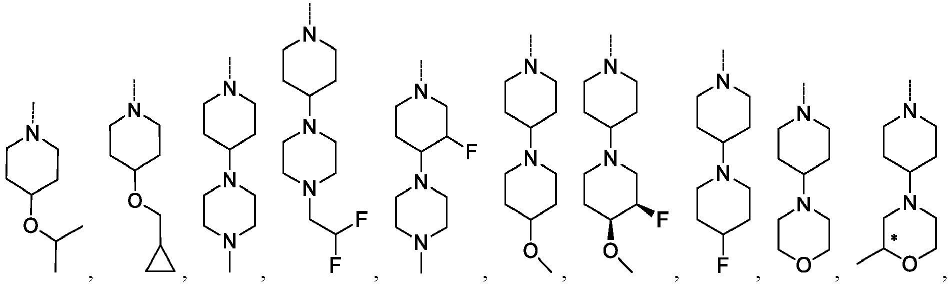 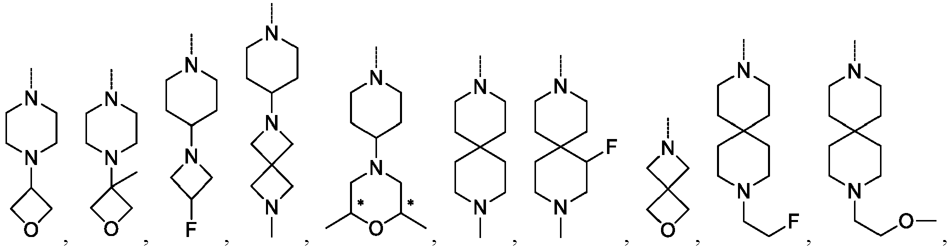 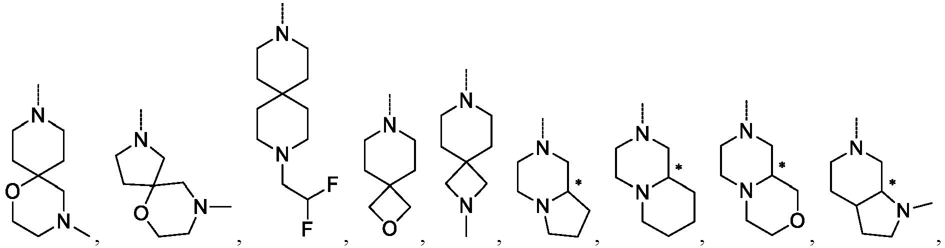 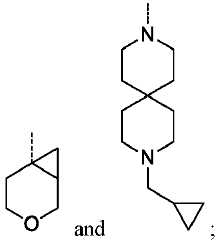
further more preferably, R₂ is selected from

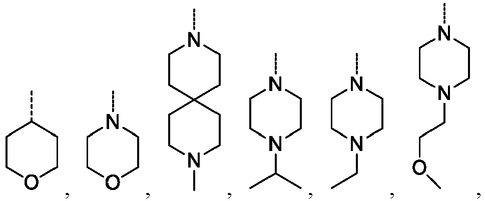

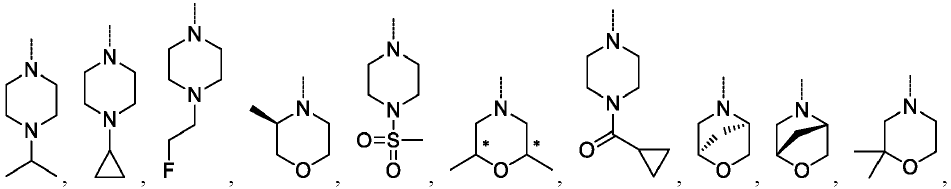

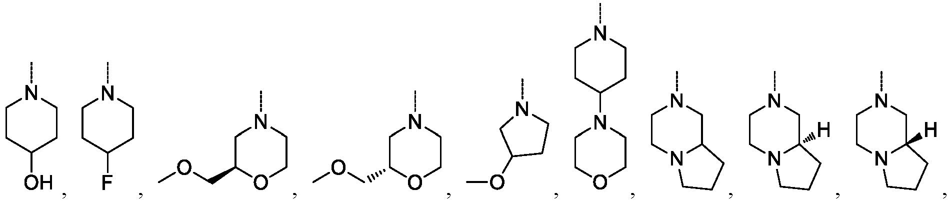

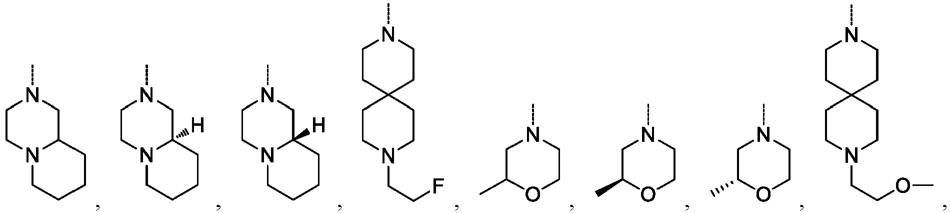

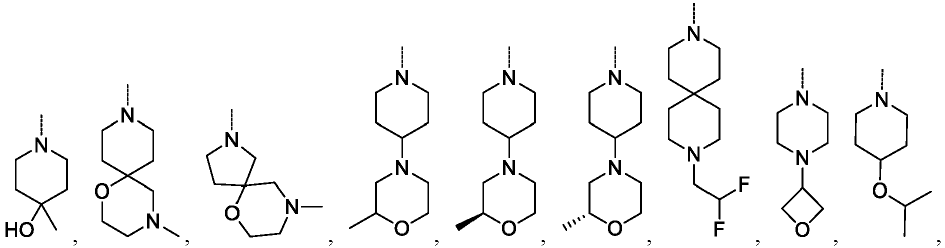

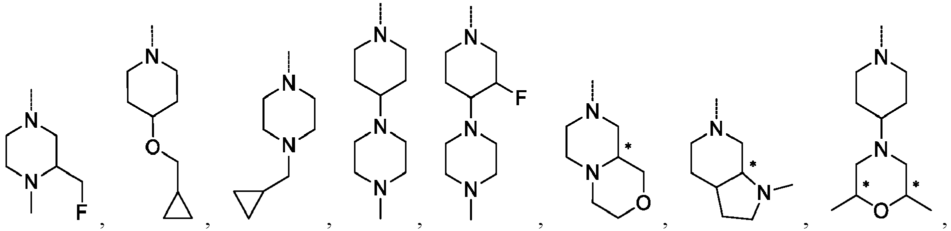

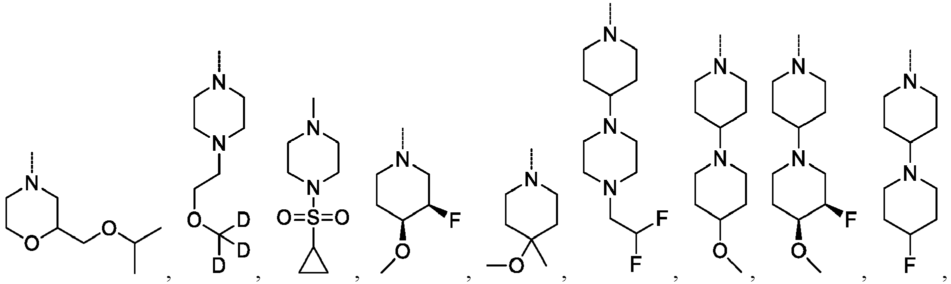

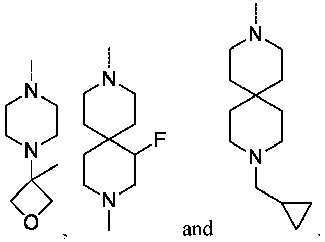

42. The compound represented by formula (I-A), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 38-41, wherein,
R₃ is selected from phenyl, pyranyl, pyridyl, pyrimidinyl, pyridazinyl, pyrazinyl, furanyl, thienyl, pyrrolyl, pyrazolyl, thiazolyl, imidazolyl, benzofuranyl, benzimidazolyl, benzothienyl, benzoxazolyl, benzothiazolyl, indolyl, pyrazolo[1,5-*α*]pyridyl, quinolinyl, isoquinolinyl, tetrahydropyrrolyl, piperidinyl, piperazinyl, morpholinyl, tetrahydrofuranyl, tetrahydropyranyl, cyclobutyl, cyclopentyl and cyclohexyl, and the phenyl, pyranyl, pyridyl, pyrimidinyl, pyridazinyl, pyrazinyl, furanyl, thienyl, pyrrolyl, pyrazolyl, thiazolyl, imidazolyl, benzofuranyl, benzimidazolyl, benzothienyl, benzoxazolyl, benzothiazolyl, indolyl, pyrazolo[1,5-*α*]pyridyl, quinolinyl, isoquinolinyl, tetrahydropyrrolyl, piperidinyl, piperazinyl, morpholinyl, tetrahydrofuranyl, tetrahydropyranyl, cyclobutyl, cyclopentyl and cyclohexyl are optionally substituted by one or more R₁₃ groups;
preferably, R₃ is selected from 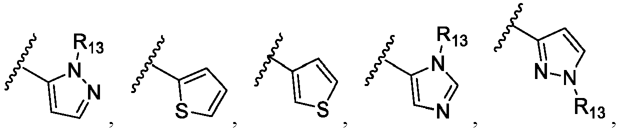 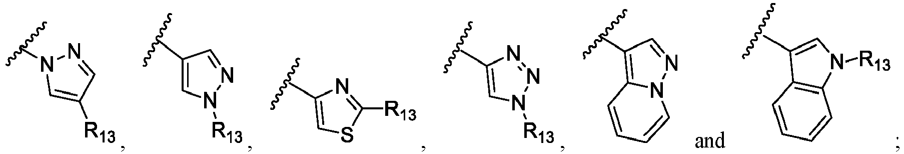
more preferably, R₃ is selected from 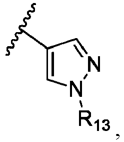 wherein the R₁₃ is defined in claim 38 or 39.

43. The compound represented by formula (I-A), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 38-42, wherein,
R₄ and R₅ are each independently selected from H, F, Cl, Br, CN, methyl, ethyl, isopropyl, methoxy, ethoxy, isopropoxy, trifluoromethyl, 2,2,2-trifluoroethyl and cyclopropyl.

44. The compound represented by formula (I-A), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 38-43, wherein,
R₆ is selected from 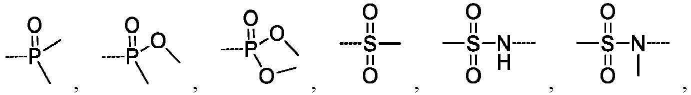 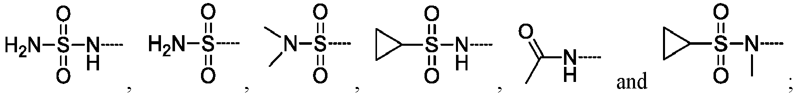
more preferably, the R₆ is selected from 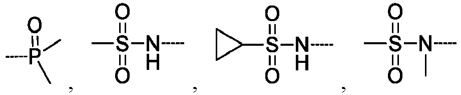 and

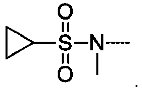

45. The compound represented by formula (I-A), the stereoisomer thereof, the tautomer thereof, the pharmaceutically acceptable salt thereof, the prodrug thereof, the hydrate thereof, the solvate thereof or the isotope-labeled derivative thereof as claimed in any one of claims 38-44, wherein,
R₇ and R₈ are cyclized to C₄₋₆ cycloalkyl, 4-6-membered heterocycloalkyl, C₅₋₆ aryl or 5-7-membered heteroaryl, or R₈ and R₉ are cyclized to C₄₋₆ cycloalkyl, 4-6-membered heterocycloalkyl, C₅₋₆ aryl or 5-7-membered heteroaryl;
preferably, R₇ and R₈ or R₈ and R₉ are independently cyclized to cyclobutane, cyclopentane, tetrahydropyrrole ring, tetrahydrofuran ring, tetrahydropyran ring, thiophene ring, imidazole ring, pyrazole ring, pyrrole ring, oxazole ring, thiazole ring, isoxazole ring, piperazine ring, isothiazole ring, benzene ring, pyridine ring, piperidine ring, pyrimidine ring, pyridazine ring or pyrazine ring;
more preferably, R₇ and R₈ or R₈ and R₉ are independently cyclized to cyclobutane, pyridine ring or pyrazine ring.

46. The compound represented by formula (I-A), the stereoisomer thereof, the tautomer thereof, the pharmaceutically acceptable salt thereof, the prodrug thereof, the hydrate thereof, the solvate thereof or the isotope-labeled derivative thereof as claimed in claim 38, wherein, which is selected from, 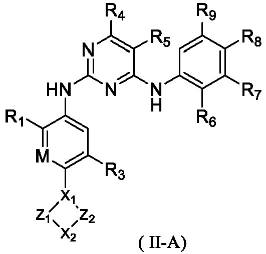 wherein, X₁ is independently selected from CR_{c} and N;
X₂ is independently selected from -CR_{c}R_{d}-, -NR_{c}- and -O-;
Z₁ and Z₂ are each independently selected from -(CRₑR_{f})ₘ(CRₑR_{f})ₙ-;
the R_{c}, R_{d}, Rₑ, and R_{f} are each independently selected from H, halogen, -CN, -OH, -NH₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₁₄ cycloalkyl, 3-14-membered heterocycloalkyl, C₃₋₆ cycloalkenyl, phenyl, -(CH₂)ₘNRₐR_{b} and -(CH₂)ₘO(CH₂)ₙCH₃; wherein the Rₐ, R_{b}, m and n are defined in claim 38;
or, R_{c} and R_{d} together form C₃₋₈ cycloalkyl or 3-8-membered heterocycloalkyl;
or, Rₑ and R_{f} together form C₃₋₈ cycloalkyl or 3-8-membered heterocycloalkyl;
or, R_{c} and Rₑ, or R_{c} and R_{f}, or R_{d} and Rₑ, or R_{d} and R_{f} together form C₃₋₈ cycloalkyl or 3-8-membered heterocycloalkyl;
wherein, a ring formed by X₁, X₂, Z₁ and Z₂ and a ring further formed by substituent groups R_{c}, R_{d}, Rₑ and R_{f} thereof are optionally substituted by one or more R₁₂ groups;
R₁, R₃, R₄, R₅, R₆, R₇, R₈, R₉, R₁₂ and M are defined in any one of claims 38-45.

47. The compound represented by formula (I-A), the stereoisomer thereof, the tautomer thereof, the pharmaceutically acceptable salt thereof, the prodrug thereof, the hydrate thereof, the solvate thereof or the isotope-labeled derivative thereof as claimed in claim 46, wherein, which is selected from, 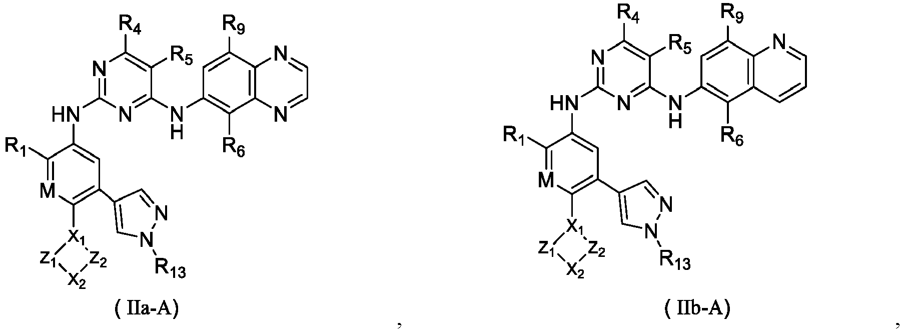 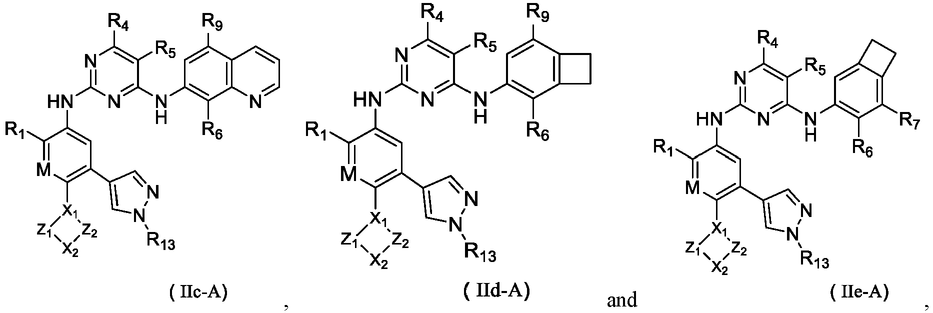 wherein, the ring formed by X₁, X₂, Z₁ and Z₂ and the ring further formed by substituent groups thereof are optionally substituted by one or more R₁₂ groups;
X₁, X₂, Z₁, Z₂, R₁, R₄, R₅, R₆, R₇, R₉, R₁₂ and M are defined in claim 46;
R₁₃ is defined in any one of claims 38-45.

48. The compound represented by formula (I-A), the stereoisomer thereof, the tautomer thereof, the pharmaceutically acceptable salt thereof, the prodrug thereof, the hydrate thereof, the solvate thereof or the isotope-labeled derivative thereof as claimed in claim 47, wherein, which is selected from, 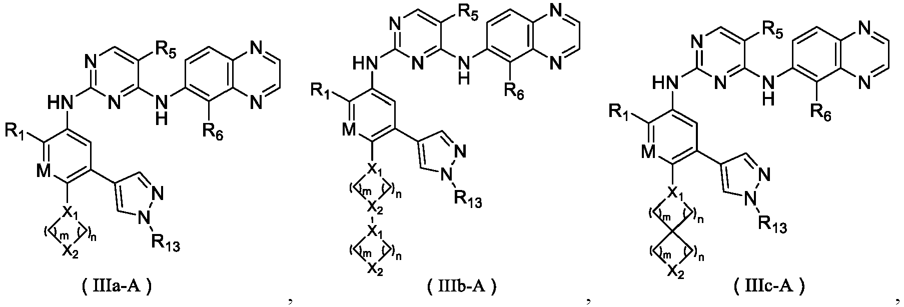 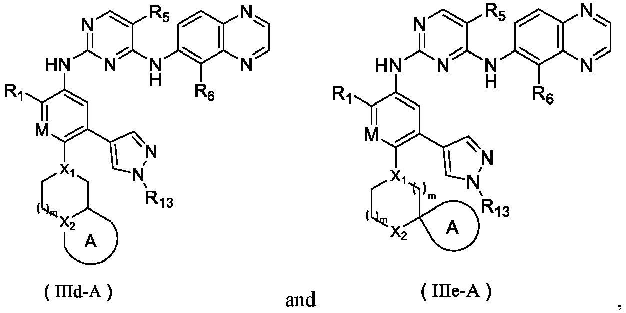 wherein, ring A is selected from C₅₋₇ cycloalkyl and 5-7-membered heterocycloalkyl;
a monocyclic ring, a bicyclic ring, a spiro ring, a fused ring formed by X₁ and X₂ and ring A are optionally substituted by one or more R₁₂ groups;
X₁, X₂, R₁, R₅, R₆, m, n, M, R₁₂ and R₁₃ are defined in claim 47.

49. The compound represented by formula (I-A), the stereoisomer thereof, the tautomer thereof, the pharmaceutically acceptable salt thereof, the prodrug thereof, the hydrate thereof, the solvate thereof or the isotope-labeled derivative thereof as claimed in claim 48, wherein, which is selected from, 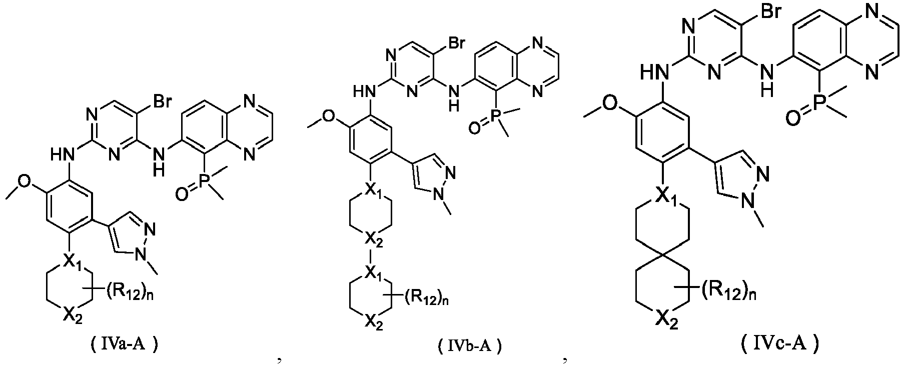 and 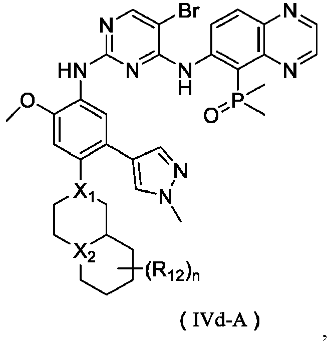 wherein X₁, X₂, R₁₂ and n are defined in claim 48.

50. A compound, a stereoisomer thereof, a tautomer thereof, a pharmaceutically acceptable salt thereof, a prodrug thereof, a hydrate thereof, a solvate thereof or an isotope-labeled derivative thereof, which is selected from ,

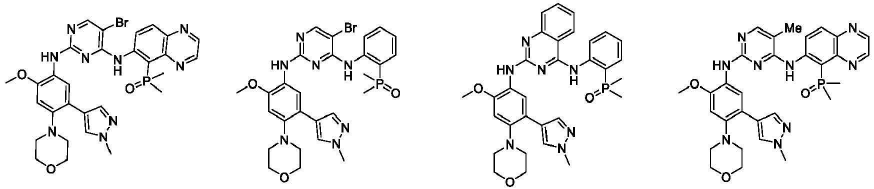

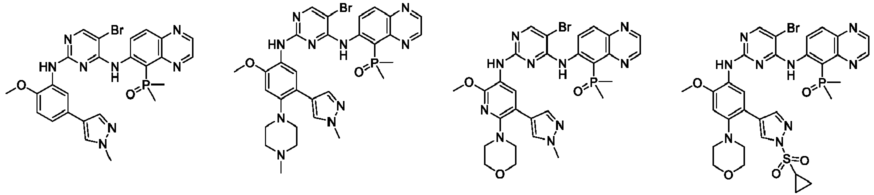

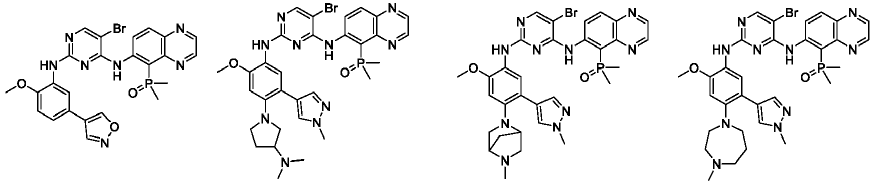

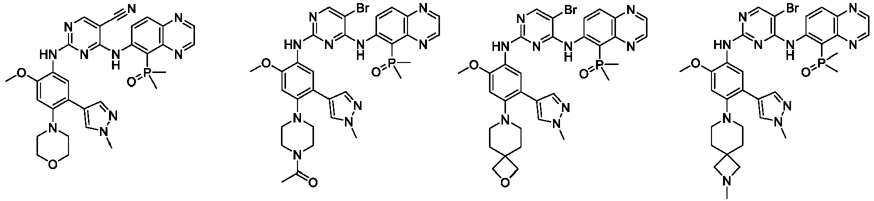

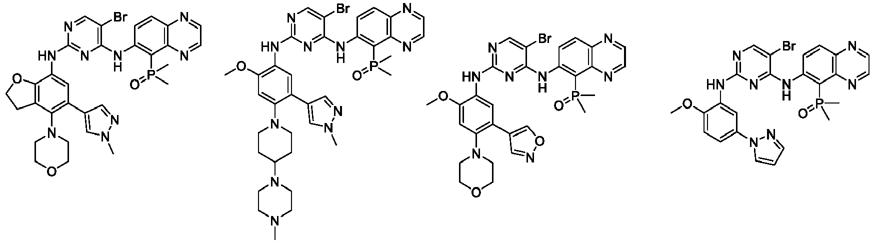

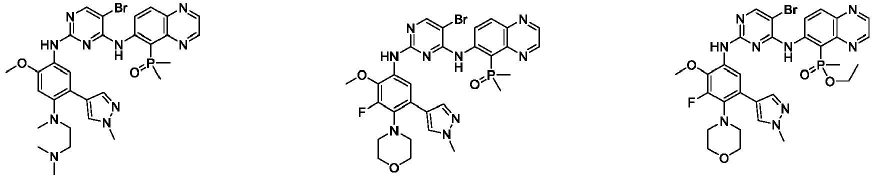

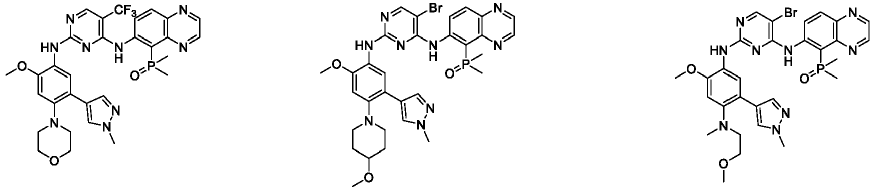

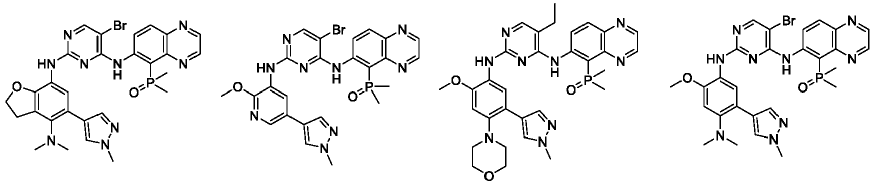

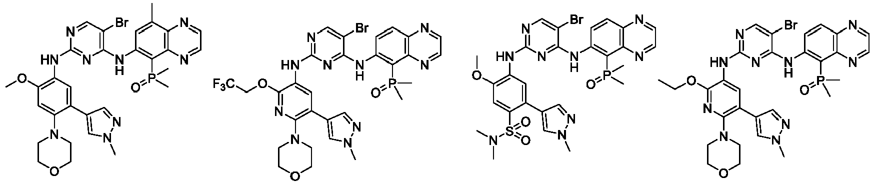

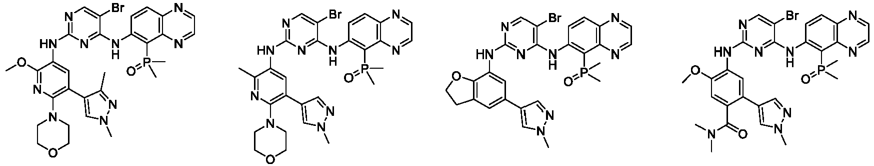

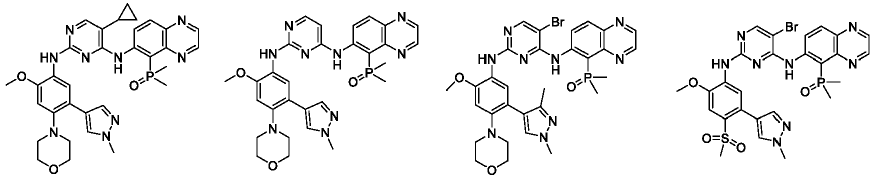

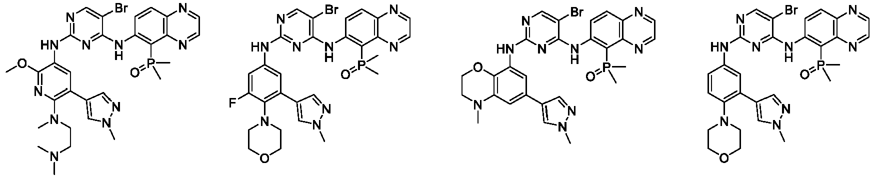

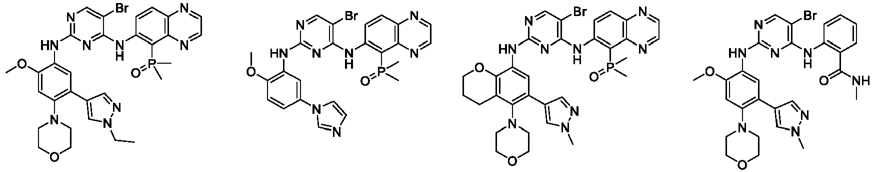

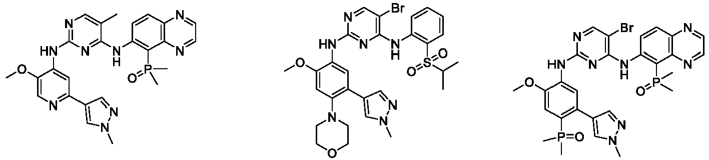

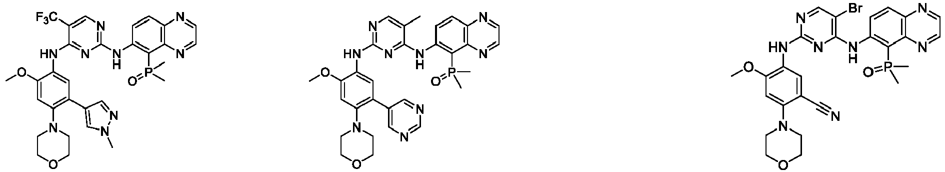

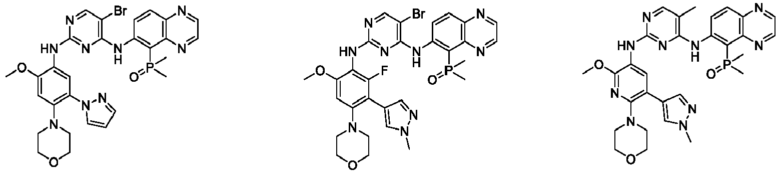

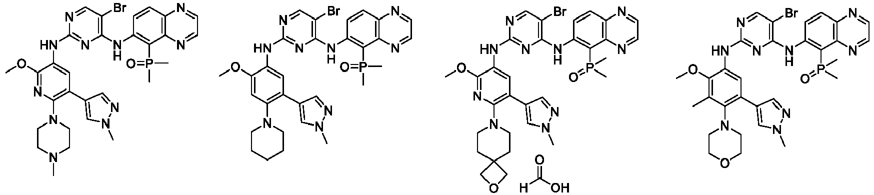

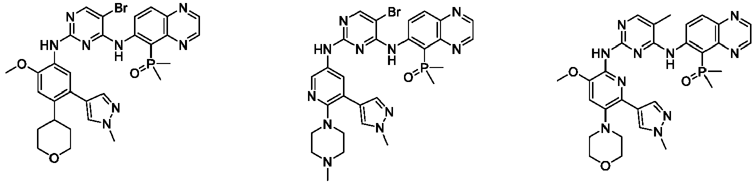

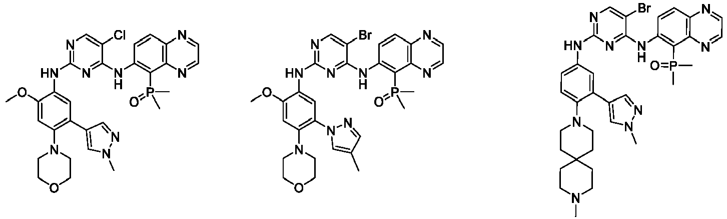

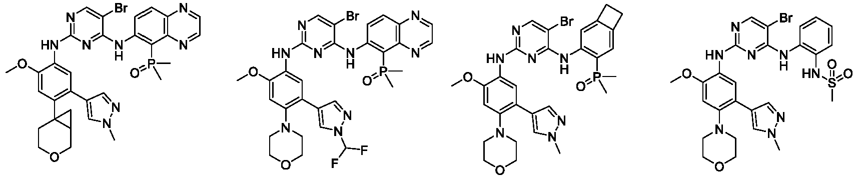

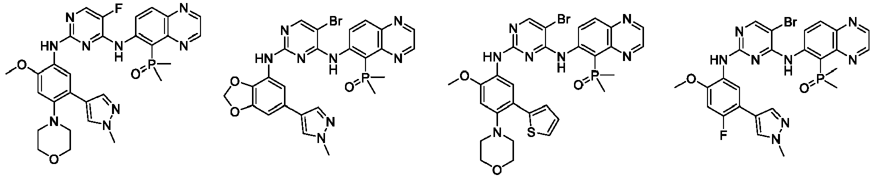

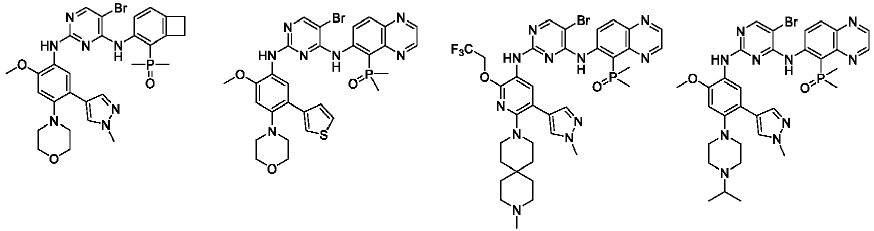

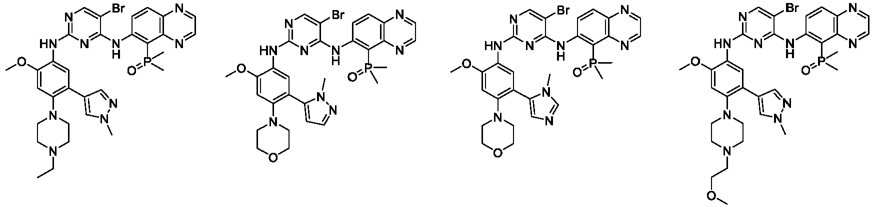

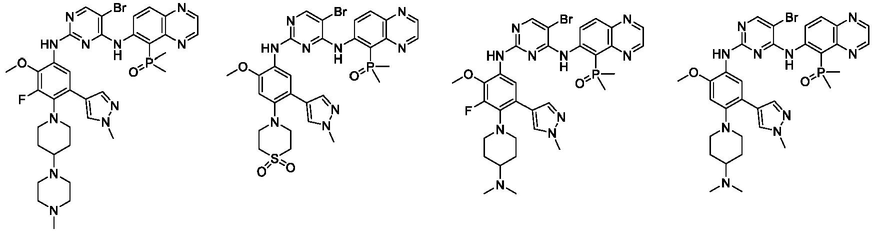

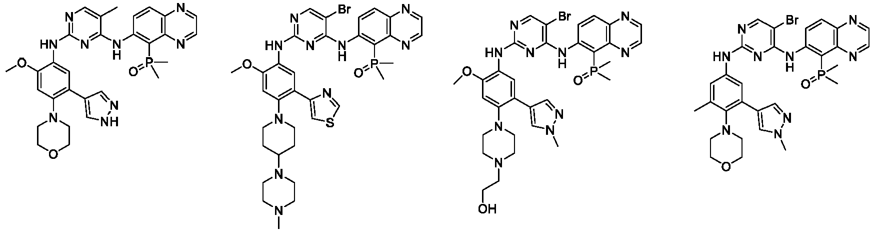

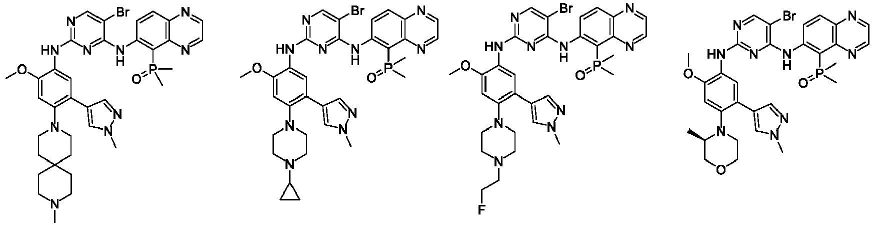

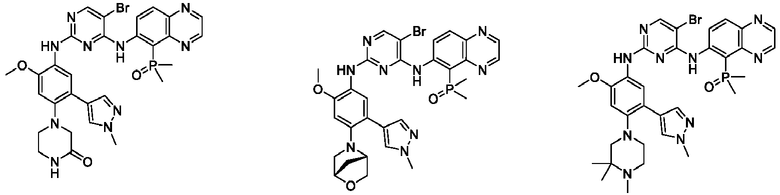

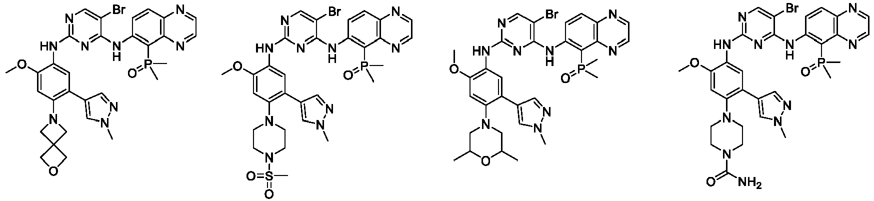

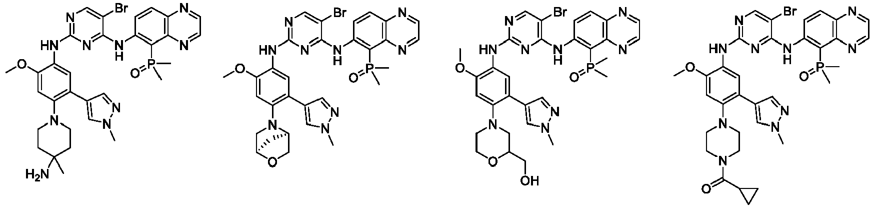

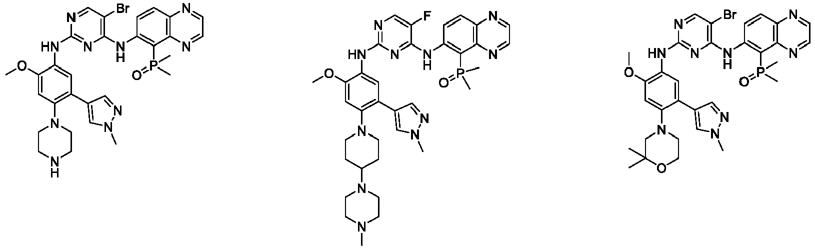

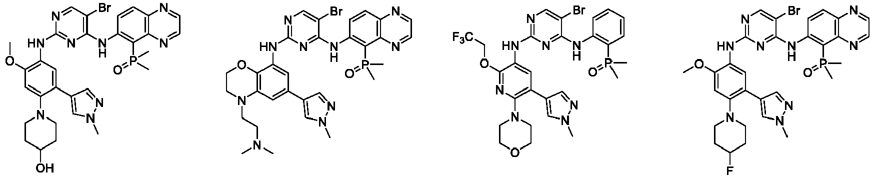

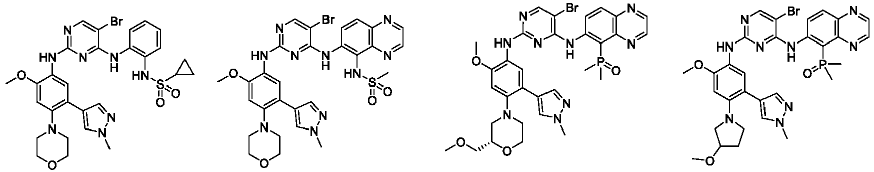

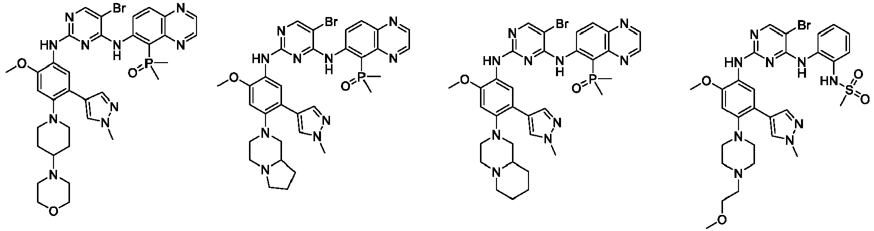

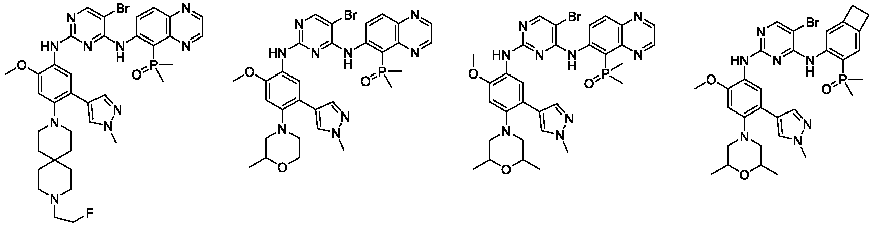

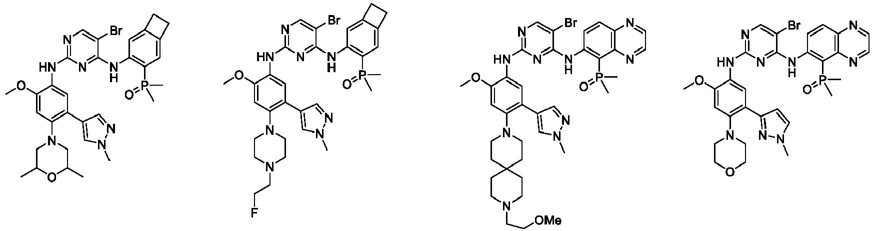

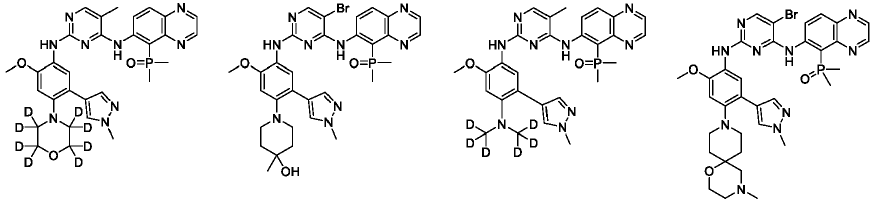

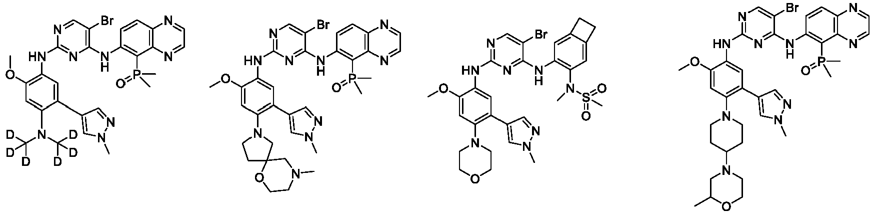

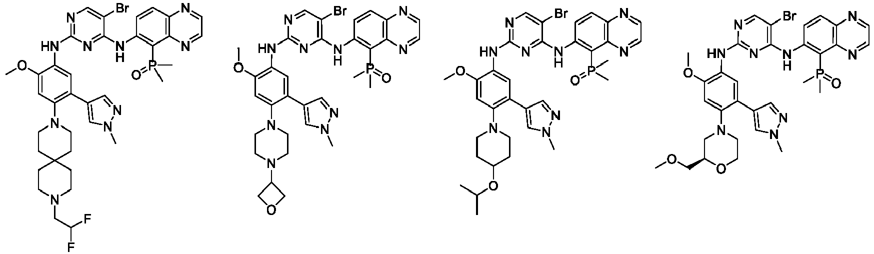

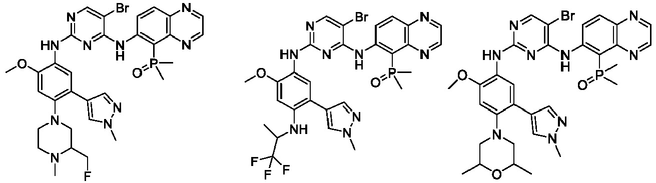

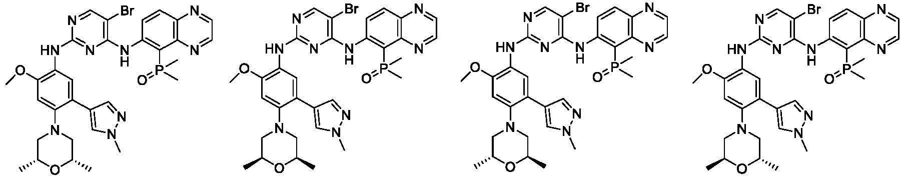

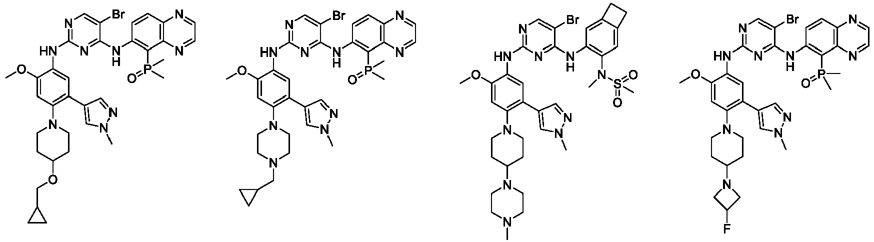

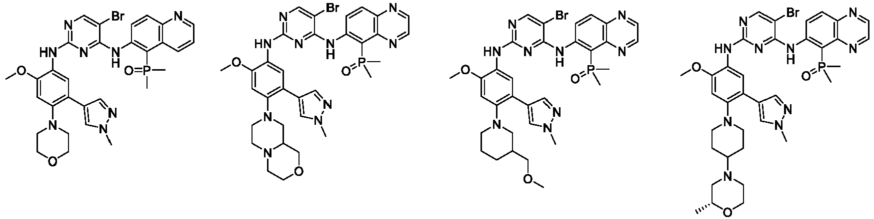

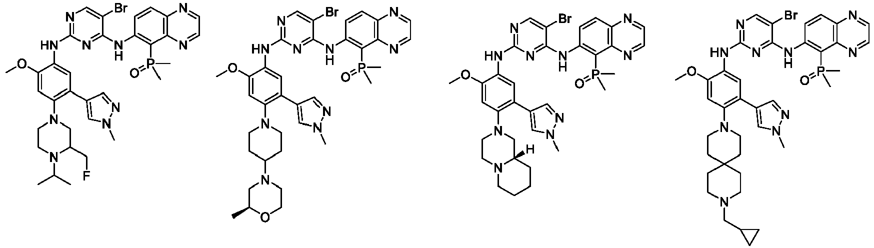

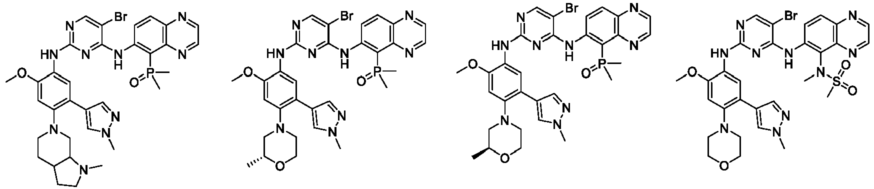

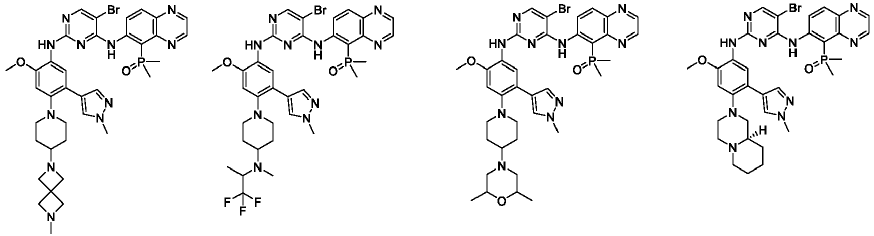

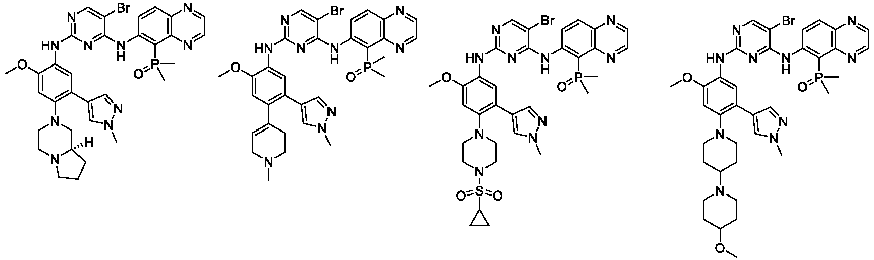

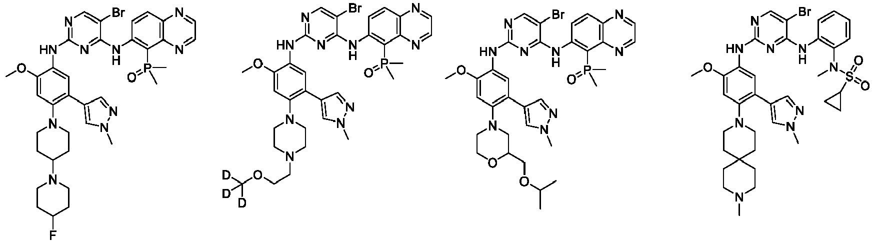

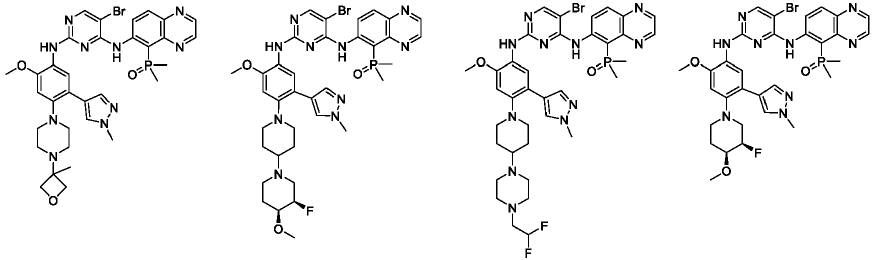

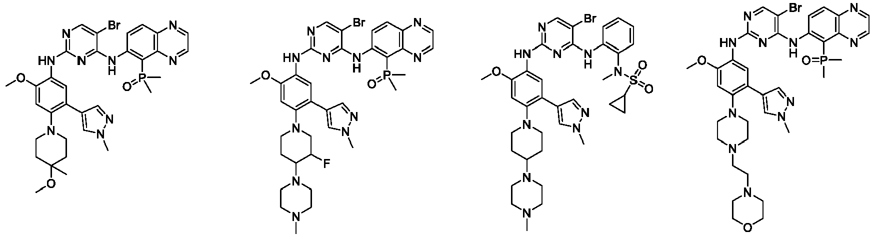

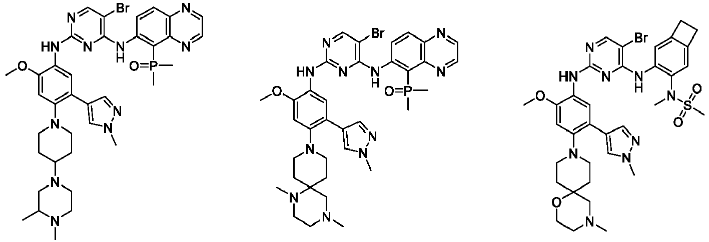

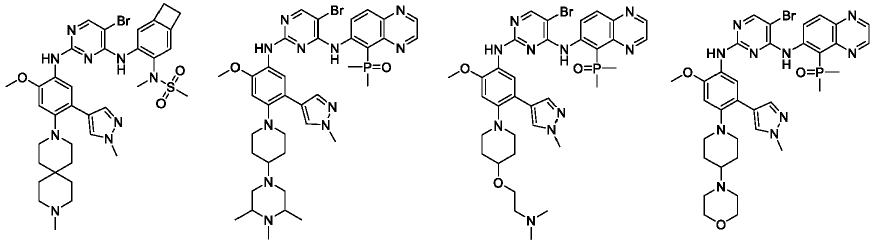

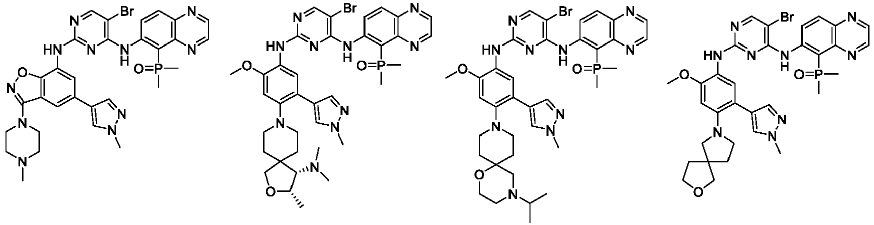

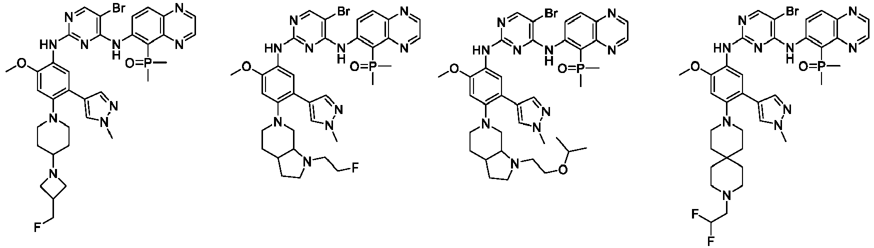

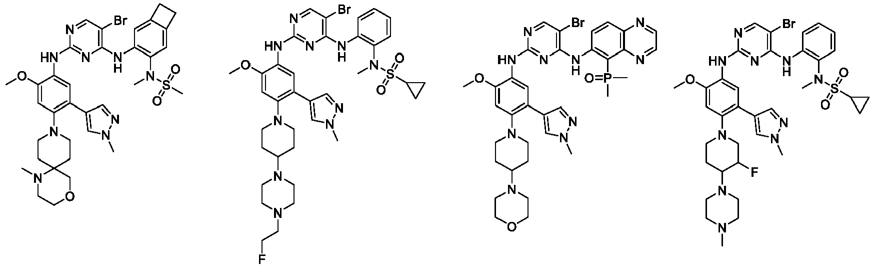

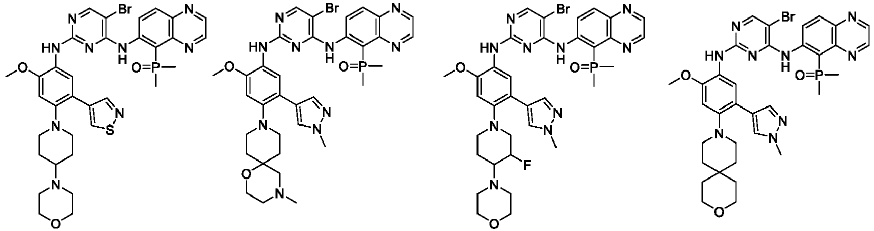

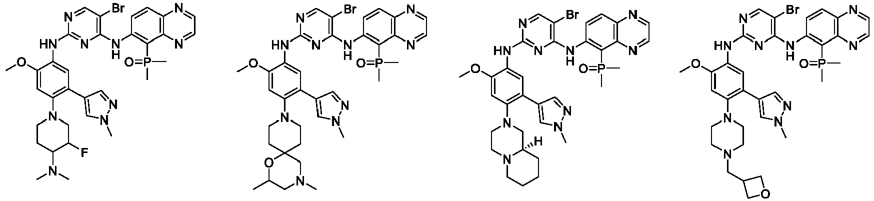

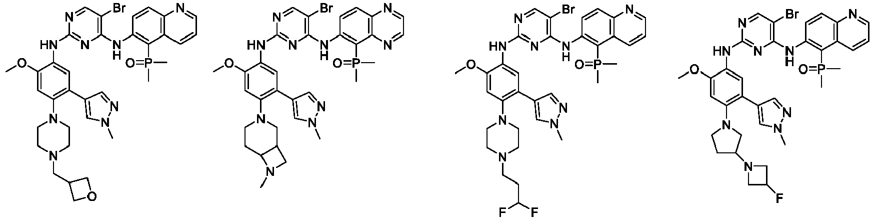

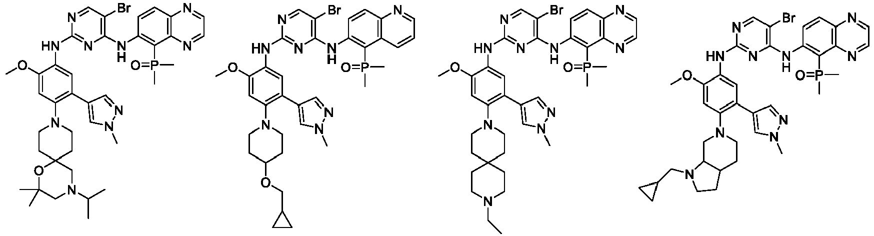

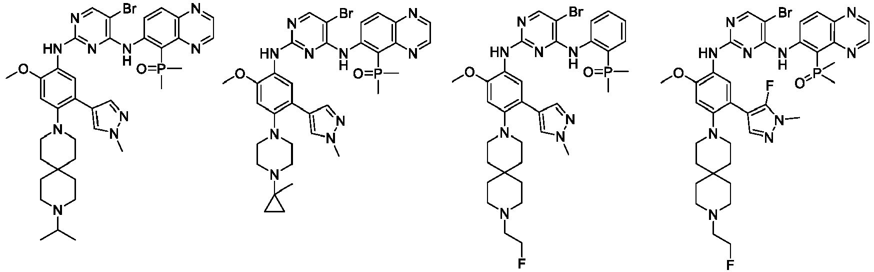

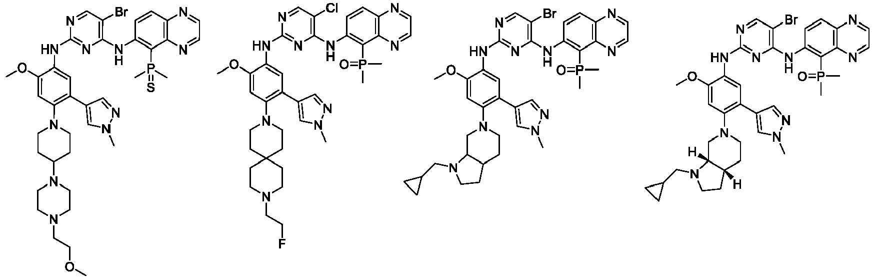

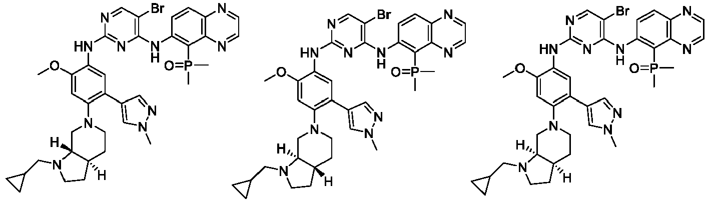

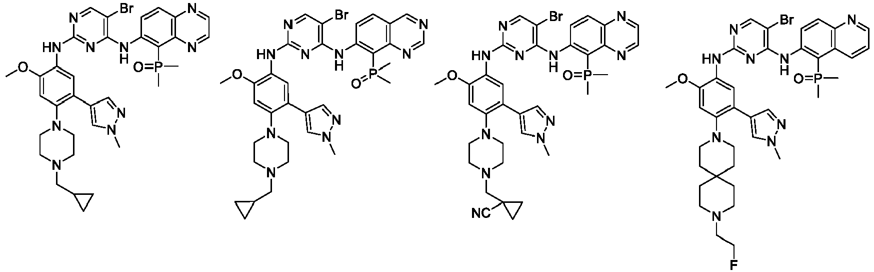

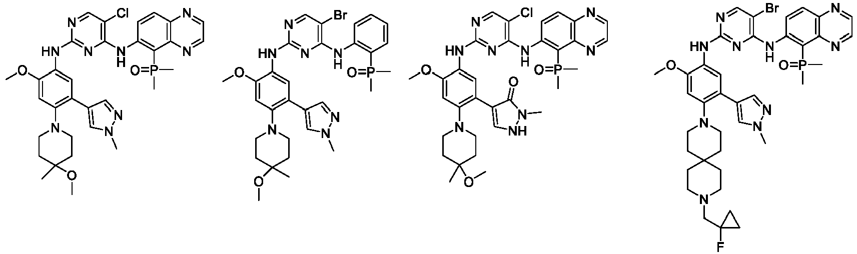

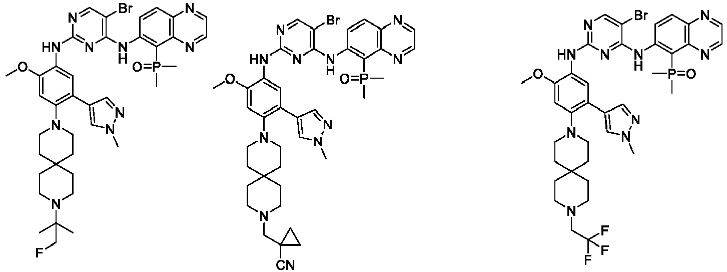

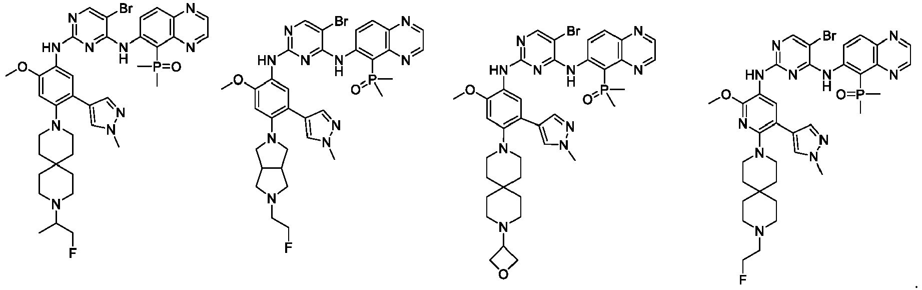

51. A pharmaceutical composition comprising a therapeutically effective amount of a substance A, and a pharmaceutically acceptable carrier, a diluent and an excipient;
the substance A is the compound represented by formula (I) or the pharmaceutically acceptable salt as claimed in any one of claims 1-37, or the compound represented by formula (I-A) or the pharmaceutically acceptable salt as claimed in any one of claims 38-49, or the compound or the pharmaceutically acceptable salt as claimed in claim 50.

52. Use of a substance A or the pharmaceutical composition as claimed in claim 51 in the manufacture of a medicament for treating cancer;
the substance A is the compound represented by formula (I) or the pharmaceutically acceptable salt as claimed in any one of claims 1-37, or the compound represented by formula (I-A) or the pharmaceutically acceptable salt as claimed in any one of claims 38-49, or the compound or the pharmaceutically acceptable salt as claimed in claim 50;
preferably, the cancer comprises lymphoma, non-Hodgkin lymphoma, ovarian cancer, cervical cancer, prostate cancer, colorectal cancer, breast cancer, pancreatic cancer, glioma, glioblastoma, melanoma, leukemia, gastric cancer, endometrial cancer, lung cancer, hepatocellular carcinoma, gastric cancer, gastrointestinal stromal tumor, acute myeloid leukemia, cholangiocarcinoma, renal carcinoma, thyroid carcinoma, anaplastic large cell lymphoma, mesothelioma, multiple myeloma and melanoma;
more preferably, the cancer is lung cancer.

53. A method for treating cancer, comprising administering to a patient a therapeutically effective amount of a substance A or the pharmaceutical composition as claimed in claim 51;
the substance A is the compound represented by formula (I) or the pharmaceutically acceptable salt as claimed in any one of claims 1-37, or the compound represented by formula (I-A) or the pharmaceutically acceptable salt as claimed in any one of claims 38-49, or the compound or the pharmaceutically acceptable salt as claimed in claim 50;
preferably, the cancer comprises lymphoma, non-Hodgkin lymphoma, ovarian cancer, cervical cancer, prostate cancer, colorectal cancer, breast cancer, pancreatic cancer, glioma, glioblastoma, melanoma, leukemia, gastric cancer, endometrial cancer, lung cancer, hepatocellular carcinoma, gastric cancer, gastrointestinal stromal tumor, acute myeloid leukemia, cholangiocarcinoma, renal carcinoma, thyroid carcinoma, anaplastic large cell lymphoma, mesothelioma, multiple myeloma and melanoma;
more preferably, the cancer is lung cancer.

54. A compound represented by formula (V), a stereoisomer thereof or a pharmaceutically acceptable salt thereof, 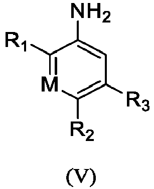 wherein, R₁, R₂, R₃ and M are defined in any one of claims 1-15, 23-32 and 38-45.

55. The compound represented by formula (V), the stereoisomer thereof or the pharmaceutically acceptable salt thereof as claimed in claim 54, wherein the compound represented by formula (V) is selected from, 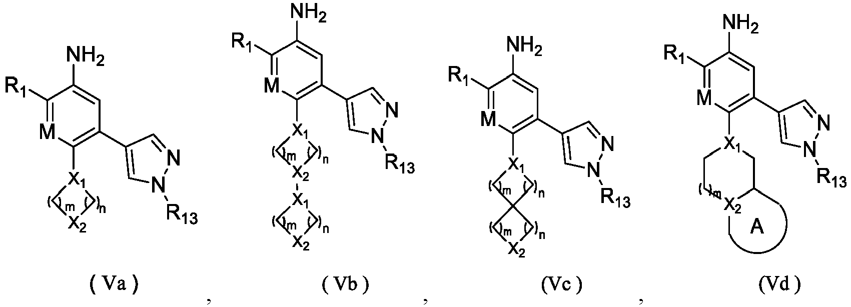 and 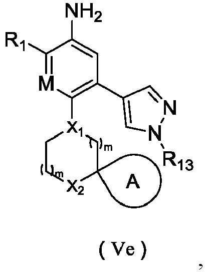 wherein, R₁ and M are defined in claim 54;
R₁₃, m and n are defined in any one of claims 1-15, 23-32 and 38-45;
X₁, X₂ and ring A are defined in any one of claims 18, 35 and 48.

56. The compound represented by formula (V), the stereoisomer thereof or the pharmaceutically acceptable salt thereof as claimed in claim 55, wherein the compound represented by formula (V) is selected from, 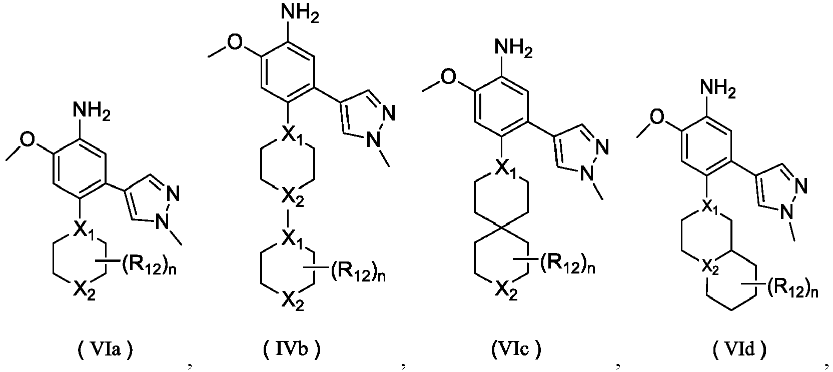 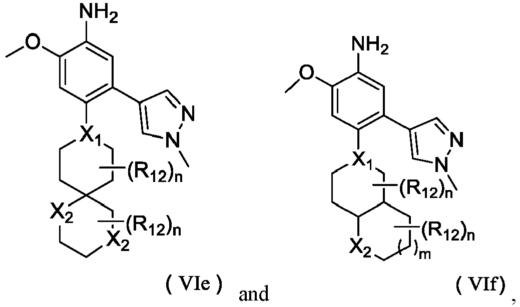 wherein X₁, X₂ and n are defined in claim 55;
R₁₂ is defined in any one of claims 1-15, 23-32 and 38-45.

57. The compound represented by formula (V), the stereoisomer thereof or the pharmaceutically acceptable salt thereof as claimed in claim 56, wherein the compound represented by formula (V) is selected from, 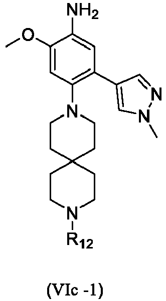 wherein, R₁₂ is defined in claim 56.

58. Use of the compound as claimed in any one of claims 54-57 in the manufacture of the compound, the stereoisomer thereof, the tautomer thereof, the pharmaceutically acceptable salt thereof, the prodrug thereof, the hydrate thereof, the solvate thereof or the isotope-labeled derivative thereof as claimed in any one of claims 1-50.
